# EUROPEAN PATENT APPLICATION

(11) **EP 4 209 485 A1**
(43) Date of publication of application: **12.07.2023**
(21) Application number: 21863693.4
(22) Date of filing: 03.09.2021
(51) Int. Cl.: C07D 217/00, C07D 401/06, C07D 401/14, C07D 405/04, A61K 31/4725, A61P 35/00

(54) **COMPOUND HAVING ANTITUMOR ACTIVITY AND USE THEREOF**

(30) Priority: 04.09.2020 CN 202010921153
(71) Applicant: Innovstone Therapeutics Limited, Shanghai 201318 (CN)
(72) Inventor: DANG, Qun, Shanghai 201318 (CN); YANG, Hanyu, Shanghai 201318 (CN); LI, Pan, Shanghai 201318 (CN); JIANG, Chunhua, Shanghai 201318 (CN); YIN, Zhou, Shanghai 201318 (CN); MA, Jianbin, Shanghai 201318 (CN); FU, Xiaodan, Shanghai 201318 (CN); CAI, Xin, Shanghai 201318 (CN)
(74) Representative: Patentanwälte Olbricht Buchhold Keulertz
(86) International application number: PCT/CN2021/116418
(87) International publication number: WO 2022/048631

(57) **Abstract**

The present disclosure provides a compound having a novel structure as a PRMT5 inhibitor, a method for preparing the compound, and use thereof in the treatment of a disease mediated by a PRMT5 inhibitor. Experiments have shown that these compounds have a strong inhibitory effect on PRMT5, and can be used as promising candidate compounds for treatment of diseases mediated by PRMT5 inhibitors. In addition, a specific synthetic method is developed in the present disclosure, which has a simple process and convenient operation, and is beneficial to industrial mass production and application.

## Description

### Technical field

The present disclosure relates to the field of pharmaceutical technology, in particular, to a compound as a PRMT5 inhibitor and a preparation method and use of the compound.

### Background of Art

PRMT5 is a kind of protein arginine methyltransferase, an abbreviation for Protein arginine N-methyltransferase 5, and a novel anti-tumor target related with epigenetic modification. It has several aliases, namely Hsl7, Jbp1, Skb 1, Capsuleen, or Dart5. PRMT5 is the main enzyme responsible for monomethylation and symmetric dimethylation of arginine. More and more literatures demonstrate that protein arginine methyltransferases (PRMTs) play a key role in different biological processes, such as cell growth and proliferation, apoptosis, metastasis, and the like.

The protein arginine methyltransferase (PRMTs) functions to transfer a methyl group from S-adenosylmethionine (or AdoMet or SAM) to an arginine residue on histones or other proteins, forming methylarginine and S-adenosylhomocysteine (or SAH). Currently, 9 members of the family (PRMT1-9) have been identified, and can be classified into three types according to the difference in the way it catalyzes arginine methylation: Type I PRMTs, including PRMT1, PRMI2, PRMT3, PRMT4, PRMT6 and PRMT8, which catalyze monomethylarginine (MMA) and asymmetric dimethylarginine (aDMA); Type II PRMTs, including PRMT5 and PRMT9, which catalyze MMA and symmetric dimethylarginine (sDMA); Type III PRMTs, including PRMT7, which can only catalyze monomethylation. As an epigenetic enzyme, PRMT5 can symmetrically methylate arginine residues of histone or non-histone substrates, affect multiple target genes and multiple signaling pathways, and play an important role in protein methylation, such as participating in alternative splicing, post-transcriptional regulation, RNA processing, cell proliferation, cell differentiation, apoptosis, and tumor formation. The selective inhibition of PRMT5 can act as a potentially powerful new drug against cancer. The development of new drugs based on the study of PRMT5 as the target has a positive role to fill in the gaps in solving unsatisfied clinical needs.

In the past few years, there have been many reports about PRMT5 Inhibitor, referring to: WO2014100719A, WO2019102494A, WO2015200677A, WO2015200680A, WO2014100764A, WO2014100730A, WO2014100716A, WO2014100695A, WO2019173804A, CN108570059A, WO2018167269A and the like. And there are already two examples of compounds, JNJ-64619178 and GSK-3326595, entering the clinical treatment of solid tumors and mantle cell lymphoma.

JNJ-64619178 is a selective PRMT5 inhibitor developed by Johnson & Johnson, and has an inhibitory effect on the growth of various tumor cells *in vitro.* A number of xenograft animal models were selected by Johnson & Johnson to demonstrate its effective anti-tumor effect, for example, Xenograft models of small cell lung cancer (SCLC), non-small cell lung cancer (NSCLC), acute myeloid leukemia (AML) and non-Hodgkin's lymphoma (non-Hodgkin lymphoma) were selected for antitumor experiments. It was observed in the models that the tumor growth was inhibited significantly up to 99%, and continuously inhibited for several weeks after discontinuance of the drug. JNJ-64619178 could inhibit Sym-Arg dimethylation of SMD1/3 protein, a core component of the tumor spliceosome, and inhibit Sym-Arg dimethylation of serum protein. These can be used as pharmacodynamic markers for the tumor growth inhibition of PRMT5 in the Xenograft models. In the SCLC models, it was observed that PRMT5 had an effective and long-term inhibitory effect on SMD1/3 dimethylation both during the administration and after the administration. Based on these high selectivity and high efficiency, good pharmacokinetics and safety, and significant preclinical efficacy and pharmacodynamic results, Phase I clinical trials of JNJ-64619178 began in 2018.

GSK-3326595 is optimized from EPZ015666 (having a structure as follows), and it is a small molecule which is highly selective and orally administrated, and is the first generation PRMT5 Inhibitor. EPZ015666 showed a significant activity in vitro and in vivo in mantle cell lymphoma, and Glaxo Smith Kline (GSK) announced in September 2016 that GSK-3326595 firstly entered the clinic trials, after two years of optimization and preclinical research. At the ESMO conference of 2019, Glaxo Smith Kline announced Phase I clinical data of GSK-3326595. In Phase I clinical trials of GSK-3326595, adult patients with solid tumors were selected, with the main purpose of testing the safety, tolerance and PK/PD, and collecting drug efficacy data (ORR and DCR). The data showed that GSK3326595 PK is dose-dependent in plasma.

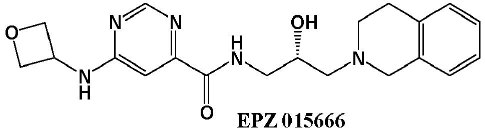

Although some PRMT5 inhibitor small molecules have been published, but there is no PRMT5 Inhibitor developed and marketed, so there is still an urgent need to develop new compounds with market potential, better efficacy and pharmacokinetic results. The present disclosure designs a series of compounds with novel structures represented by the general formula, and finds that the compounds with such a structure exhibit excellent effects, which has a positive significance for the development of PRMT5 inhibitors.

### Summary of the invention

An object of the present disclosure is to provide a compound having a novel structure as a PRMT5 inhibitor, a preparation method thereof and use thereof in the treatment of a disease mediated by the PRMT5 inhibitor.

The first aspect of the present disclosure provides a compound represented by formula (I) below, and stereoisomers, geometric isomers, tautomers, pharmaceutically acceptable salts, prodrugs, hydrates, solvates or isotope labeled analogues thereof, 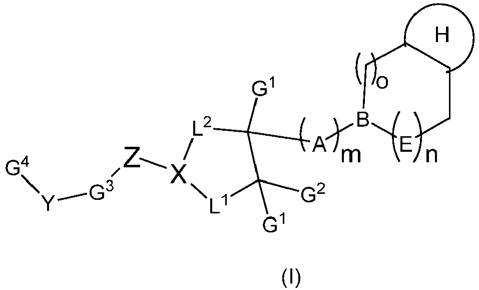
wherein L¹ and L² are each independently one selected from -C(R¹)(R²)-, -C(R¹)(R²)C(R¹)(R²)-, and -C(R¹)(R²)C(R¹)(R²)C(R¹)(R²)-; wherein R¹ and R² are independently one selected from hydrogen, halogen, hydroxy, mercapto, amino, cyano, C₁₋₆ alkyl, C₃₋₆ cycloalkyl, 4- to 6-membered heterocyclyl, -OR³, -NHR³, and -NR³R⁴ in each occurrence; R³ and R⁴ are independently selected from C₁₋₆ alkyl, C₂₋₆ alkenyl, C₂₋₆ alkynyl, C₃₋₆ cycloalkyl, 4- to 6-membered heterocyclyl, aryl, and 5- to 6-membered heteroaryl in each occurrence.
X is selected from C(R⁵) or N; wherein R⁵ is independently one selected from hydrogen, halogen, hydroxyl, mercapto, amino, and cyano in each occurrence;
Y is one selected from - (chemical bond), -H, -OH, -NH₂, halogen, -O-, -S-, -CO-, -C(R⁶)F-, -CF₂-, -SO-, -SO₂-, -(CH₂)ₚN(R⁶)-, -N(R⁶)(CH₂)ₚ-, -S(O)N(R⁶)-, -S(O)₂N(R⁶)-, -N(R⁶)SO-, -N(R⁶)S(O)₂-, -C(O)N(R⁶)-, -N(R⁶)C(O)-, and -CH(R⁶)-; wherein p=0, 1, 2 or 3; R⁶ may be one selected from hydrogen, optionally substituted C₁₋₆ alkyl, optionally substituted C₃₋₆ cycloalkyl and optionally substituted 4- to 6-membered heterocyclyl; the term "optionally substituted" means that the hydrogen on the group to be substituted is unsubstituted or one or more substitutable sites of the group to be substituted are independently substituted by a substituent selected from halogen, hydroxyl, mercapto, amino, cyano, C₁₋₆ alkyl, C₂₋₆ alkenyl, C₂₋₆ alkynyl, C₃₋₆ cycloalkyl, 4- to 6-membered heterocyclyl, aryl, and 5- to 6-membered heteroaryl; when Y is one selected from -H, -OH, -NH₂ and halogen, G⁴ does not exist;
Z is one selected from • (chemical bond), -O-, -S-, -CO-, -N(R⁷)-, -S(O)N(R⁷)-, -S(O)₂N(R⁷)-, -N(R⁷)SO-, -N(R⁷)S(O)₂-, -C(O)N(R⁷)-, -N(R⁷)C(O)-, -N(R⁷)C(O)N(R⁷)-, and -CH(R⁷)-; wherein R⁷ is independently one selected from hydrogen, optionally substituted C₁₋₆ alkyl, optionally substituted C₃₋₆ cycloalkyl and optionally substituted 4- to 6-membered heterocyclyl in each occurrence; the term "optionally substituted" means that the hydrogen on the group to be substituted is unsubstituted or one or more substitutable sites of the group to be substituted are independently substituted by a substituent selected from halogen, hydroxyl, mercapto, amino, cyano, C₁₋₆ alkyl, C₂₋₆ alkenyl, C₂₋₆ alkynyl, C₃₋₆ cycloalkyl, 4- to 6-membered heterocyclyl, aryl, and 5- to 6-membered heteroaryl;
G¹ is independently one selected from hydrogen, halogen, hydroxy, mercapto, amino, cyano, and C₁₋₅ linear or branched alkyl in each occurrence;
G² is one selected from hydrogen, halogen, hydroxyl, mercapto, amino, cyano, optionally substituted R⁸, optionally substituted -O(R⁸), optionally substituted -S(R⁸), optionally substituted -NH(R⁸), and optionally substituted -N(R⁸)(R⁸); wherein R⁸ is independently one selected from C₁₋₆ alkyl, C₃₋₆ cycloalkyl and 4- to 6-membered heterocyclyl in each occurrence; the term "optionally substituted" means that the hydrogen on the group to be substituted is unsubstituted or one or more substitutable sites of the group to be substituted are independently substituted by a substituent selected from halogen, hydroxyl, mercapto, amino, cyano, C₁₋₆ alkyl, C₂₋₆ alkenyl, C₂₋₆ alkynyl, C₃₋₆ cycloalkyl, 4- to 6-membered heterocyclyl, aryl, and 5- to 6-membered heteroaryl;
G³ is one selected from optionally substituted C₆₋₁₀ aryl, optionally substituted 5- to 10-membered heteroaryl, optionally substituted C₃₋₇ cycloalkyl, and optionally substituted 4-to 10-membered heterocyclyl; the term "optionally substituted" means that the hydrogen on the group to be substituted is unsubstituted or one or more substitutable sites of the group to be substituted are independently substituted by R¹⁶, wherein R¹⁶ is independently selected from hydrogen, halogen, hydroxy, mercapto, amino, cyano, -R⁹, -OR⁹, -SR⁹, -SO(R⁹), -SO₂(R⁹), -COOR⁹, -NH(R⁹), -N(R⁹)(R¹⁰), -CONHR⁹, -CON(R⁹)(R¹⁰), -SONH(R⁹), -SON(R⁹)(R¹⁰), -SO₂NH(R⁹), and -SO₂N(R⁹)(R¹⁰) in each occurrence; wherein R⁹ and R¹⁰ are independently selected from C₁₋₆ alkyl, C₂₋₆ alkenyl, C₂₋₆ alkynyl, C₃₋₆ cycloalkyl, 4- to 6-membered heterocyclyl, aryl, and 5- to 6-membered heteroaryl, which may be substituted with one or more of hydrogen, halogen, hydroxy, mercapto, amino, cyano, C₁₋₆ alkyl, C₃₋₆ cycloalkyl, 4- to 6-membered heterocyclyl, aryl, and 5- to 6-membered heteroaryl, in each occurrence;
G⁴ is one selected from optionally substituted C₁₋₁₂ alkyl, optionally substituted C₂₋₁₂ alkenyl, optionally substituted C₂₋₁₂ alkynyl, optionally substituted C₃₋₁₂ cycloalkyl, optionally substituted 4- to 10-membered heterocyclyl, optionally substituted C₆₋₁₀ aryl, and optionally substituted 5- to 10-membered heteroaryl; the term "optionally substituted" means that the hydrogen on the group to be substituted is unsubstituted or one or more substitutable sites of the group to be substituted are independently substituted by R¹⁷, R¹⁷ is independently selected from hydrogen, halogen, hydroxyl, mercapto, amino, cyano, carbonyl, 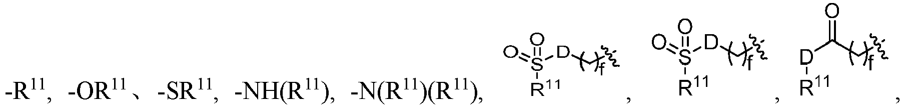 and
in each occurrence,wherein R¹¹ is independently selected from C₁₋₆ alkyl, C₂₋₆ alkenyl, C₂₋₆ alkynyl, C₃₋₆ cycloalkyl, 4- to 6-membered heterocyclyl, aryl, and 5- to 6-membered heteroaryl, which may be substituted with one or more of hydrogen, halogen, hydroxy, mercapto, amino, cyano, C₁₋₆ alkyl, C₃₋₆ cycloalkyl, 4- to 6-membered heterocyclyl, aryl, and 5- to 6-membered heteroaryl, in each occurrence; D is independently one selected from a bond, -CH₂-, -C(=O)-, -NH-, -N(CH₃)-, -O-, and -S- in each occurrence, and f is independently selected from 0, 1, 2, 3, 4, 5, 6, 7 or 8 in each occurrence;
m=0 or 1, and when m=1, A may be selected from -N(R¹²)-, -CH(R¹²)- or -CH(NHR¹²)-, where R¹² may be one selected from hydrogen, hydroxyl, halogen, C₁₋₆ alkyl, C₃₋₆ cycloalkyl and 4- to 6-membered heterocyclyl; when m=0, A does not exist;
B may be selected from -N- or -CH-;
n=0 or 1, and when n=1, E may be selected from -NR¹³- or -C(R¹³)R¹³-, where R¹³ is independently one selected from hydrogen, hydroxyl, halogen, C₁₋₆ alkyl, C₃₋₆ cycloalkyl and 4- to 6-membered heterocyclyl in each occurrence; when n=0, E does not exist;
at least one of A, B, and E is an N atom that meets the respective definitions;
o=0, 1, 2 or 3;
H ring is one selected from C₆₋₁₀ aryl ring and 5- to 10-membered heteroaryl ring; the aryl ring or heteroaryl ring may be independently substituted with one or more R¹⁵, where R¹⁵ is independently selected from hydrogen, halogen, hydroxy, mercapto, amino, cyano, optionally substituted -R¹⁴, optionally substituted -OR¹⁴, optionally substituted -NHR¹⁴, and optionally substituted -N(R¹⁴)(R¹⁴) in each occurrence; wherein R¹⁴ is independently selected from C₁₋₆ alkyl, C₂₋₆ alkenyl, C₂₋₆ alkynyl, C₃₋₆ cycloalkyl, 4- to 6-membered heterocyclyl, aryl, and 5- to 6-membered heteroaryl in each occurrence; the term "optionally substituted" means that the hydrogen on the group to be substituted is unsubstituted or one or more substitutable sites of the group to be substituted are independently substituted by a substituent selected from halogen, hydroxyl, mercapto, amino, cyano, C₁₋₆ alkyl, C₂₋₆ alkenyl, C₂₋₆ alkynyl, C₃₋₆ cycloalkyl, 4- to 6-membered heterocyclyl, aryl, and 5- to 6-membered heteroaryl.

In a preferable embodiment of the present disclosure, the compound represented by formula (I) further has a structure represented by formula (II)A, (II)B or (II)C: 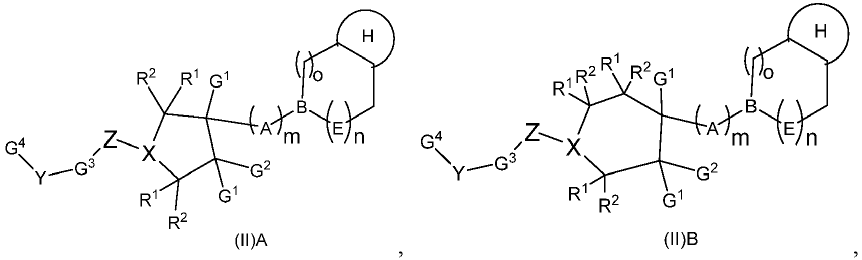 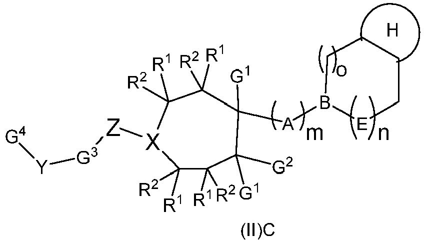 wherein each substituent in the formula (II)A, (II)B, or (II)C is as defined in the formula (I).

In a further preferable embodiment, R¹ and R² are independently one selected from hydrogen, halogen, hydroxy, mercapto, amino, cyano, C₁₋₆ alkyl, C₃₋₆ cycloalkyl, 4- to 6-membered heterocyclyl, -OR³, -NH(R³), and -NR³R⁴ in each occurrence; R³ and R⁴ are independently selected from C₁₋₆ alkyl, C₂₋₆ alkenyl, C₂₋₆ alkynyl, C₃₋₆ cycloalkyl, 4- to 6-membered heterocyclyl, aryl, and 5- to 6-membered heteroaryl in each occurrence. Still further preferably, R¹ and R² are independently one of hydrogen, halogen, hydroxy, amino, methyl, methylamino, and dimethylamino, most preferably hydrogen in each occurrence.

In some preferable embodiments of the present disclosure, X is one selected from CH, C(OH), and N.

Preferably, Y is one selected from - (chemical bond), -H, -OH, -NH₂, halogen, -O-, -S-, -CO-, -C(R⁶)F-, -CF₂-, -SO-, -SO₂-, -(CH₂)ₚN(R⁶)-, -N(R⁶)(CH₂)ₚ-, -S(O)N(R⁶)-, -S(O)₂N(R⁶)-, -N(R⁶)SO-, -N(R⁶)S(O)₂-, -C(O)N(R⁶)-, -N(R⁶)C(O)-, and -CH(R⁶)-, where p=0, 1, 2 or 3; R⁶ may be one selected from hydrogen, C₁₋₆ alkyl, C₃₋₆ cycloalkyl, and 4- to 6-membered heterocyclyl, and when Y is one selected from -H, -OH, -NH₂ and halogen, G⁴does not exist.

More preferably, Y is one selected from - (chemical bond), -H, -OH, -NH₂, -NH-, -O-, -S-, -CO-, -CHF-, -CF₂-, -SO-, -SO₂-, -(CH₂)ₚNH-, -N(CH₃)-, -S(O)NH-, -S(O)₂NH-,-NHSO-, -NHS(O)₂-, -C(O)NH-, -NHC(O)-, and -CH₂-, where p=1, 2 or 3.

Most preferably, Y is one selected from - (chemical bond), -H, -OH, -NH₂, -NH-, -CH₂NH-, -(CH₂)₂NH-, -N(CH₃)-, -O-, and -S-.

In some preferable embodiments of the present disclosure, Z is one selected from-(chemical bond), -O-, -S-, -CO-, -N(R⁷)-, -S(O)N(R⁷)-, -S(O)₂N(R⁷)-, -N(R⁷)SO-, -N(R⁷)S(O)₂-, -C(O)N(R⁷)-, -N(R⁷)C(O)-, -N(R⁷)C(O)N(R⁷)-, and -CH(R⁷)-, where R7 is independently one selected from hydrogen, C₁₋₆ alkyl, C₃₋₆ cycloalkyl and 4- to 6-membered heterocyclyl in each occurrence.

More preferably, Z is one selected from - (chemical bond), -O-, -S-, -CO-, -NH-, -S(O)NH-, -S(O)₂NH-, -NHSO-, -NHS(O)₂-, -C(O)NH-, -NHC(O)-, -NHC(O)NH-, and -CH₂-.

Most preferably, Z is one selected from - (chemical bond), -O-, -S-, -CO-, -NH-, -C(O)NH-, and -CH₂-.

In some preferable embodiments of the present disclosure, G¹ is independently one selected from hydrogen, halogen, hydroxyl, mercapto, amino, cyano and methyl in each occurrence; more preferably, G¹ is selected from hydrogen.

In some preferable embodiments of the present disclosure, G² is one selected from hydrogen, halogen, hydroxyl, mercapto, amino, cyano, -R⁸, -O(R⁸), -S(R⁸), -NH(R⁸), and -N(R⁸)(R⁸), where R⁸ is independently one selected from C₁₋₆ alkyl, C₃₋₆ cycloalkyl and 4- to 6-membered heterocyclyl in each occurrence; more preferably, G² is one selected from hydrogen, halogen, hydroxyl, mercapto, amino, cyano, -CH₃, cyclopropyl, -OCH₃, -SCH₃, -NHCH₃, -N(CH₃)(CH₃), and -NH(CH₃); most preferably, G² is selected from hydrogen, fluorine, hydroxyl, and amino.

In some preferable embodiments of the present disclosure, G³ is selected from optionally substituted C₆₋₁₀ aryl, and optionally substituted 5- to 10-membered heteroaryl, wherein in the 5- to 10-membered heteroaryl, the heteroatom is N, O, or S, and the number of heteroatoms is 1, 2, 3 or 4, the term "optionally substituted" means that the hydrogen on the group to be substituted is unsubstituted or one or more substitutable sites of the group to be substituted are independently substituted by R¹⁶.

In a still further preferable embodiment of the present disclosure, G³ is one of optionally substituted 6- to 10-membered aryl and optionally substituted 5- to 10-membered heteroaryl, and the 6- to 10-membered aryl or 5- to 10-membered heteroaryl is one selected from: 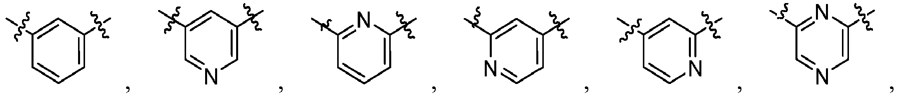 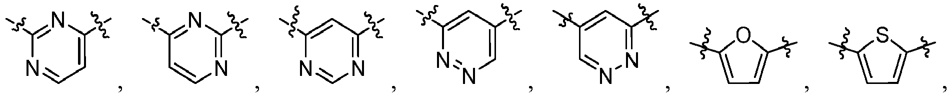 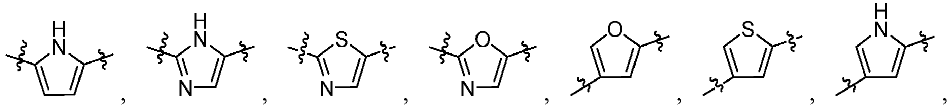 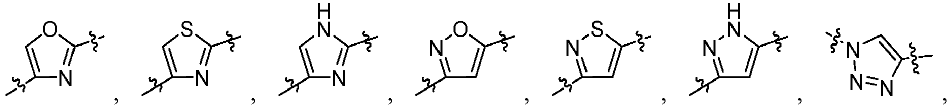 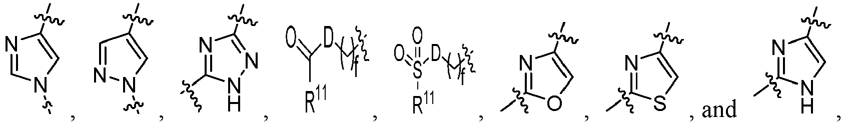 and the term "optionally substituted" means that the hydrogen on the group to be substituted is unsubstituted or one or more substitutable sites of the group to be substituted are independently substituted by R¹⁶.

More preferably, R¹⁶ is independently selected from hydrogen, halogen, hydroxy, mercapto, amino, cyano, methyl and methoxy in each occurrence.

In some preferable embodiments of the present disclosure, G⁴ is one selected from optionally substituted C₃₋₁₂ cycloalkyl, optionally substituted 4- to 10-membered heterocyclyl, optionally substituted C₆₋₁₀ aryl, and optionally substituted 5- to 10-membered heteroaryl, the term "optionally substituted" means that the hydrogen on the group to be substituted is unsubstituted or one or more substitutable sites of the group to be substituted are independently substituted by R¹⁷, where R¹⁷ is independently selected from hydrogen, halogen, hydroxyl, mercapto, amino, cyano, carbonyl, -R¹¹, -OR¹¹ , -SR¹¹, -NH(R¹¹), -N(R¹¹)(R¹¹), 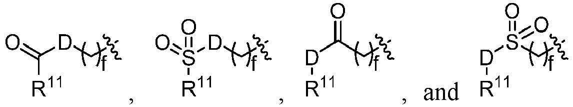 in each occurrence, wherein R¹¹ is independently selected from C₁₋₆ alkyl, C₂₋₆ alkenyl, C₂₋₆ alkynyl, C₃₋₆ cycloalkyl, 4- to 6-membered heterocyclyl, aryl, and 5- to 6-membered heteroaryl, which may be substituted with one or more of hydrogen, halogen, hydroxy, mercapto, amino, cyano, C₁₋₆ alkyl, C₃₋₆ cycloalkyl, 4- to 6-membered heterocyclyl, aryl, and 5- to 6-membered heteroaryl, in each occurrence; D is independently one selected from a bond, -CH₂-, -C(=O)-, -NH-, -N(CH₃)-, -O-, and -S- in each occurrence, and f is independently selected from 0, 1, or 2 in each occurrence.

In a still further preferable embodiment of the present disclosure, G⁴ is one selected from: 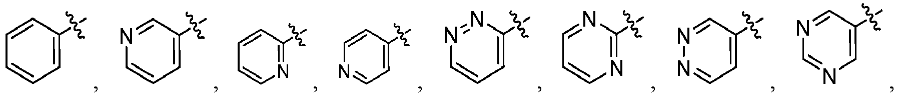 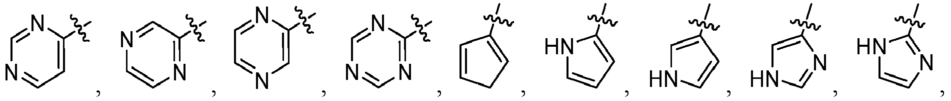 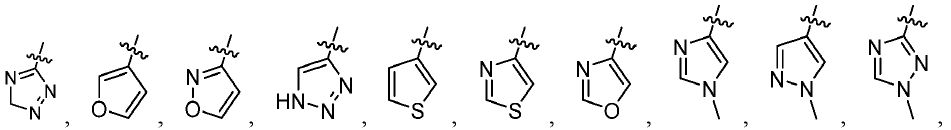 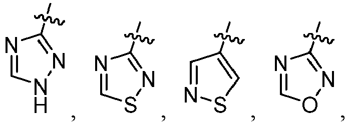 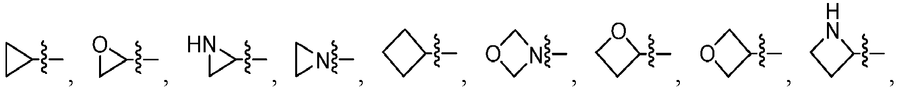 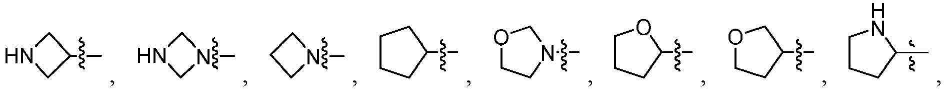 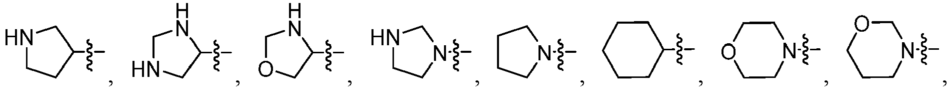 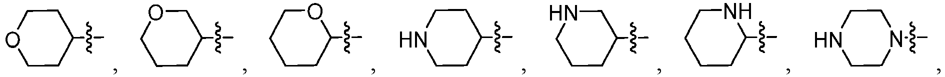 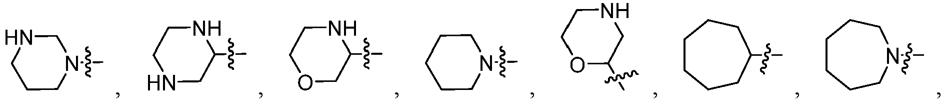 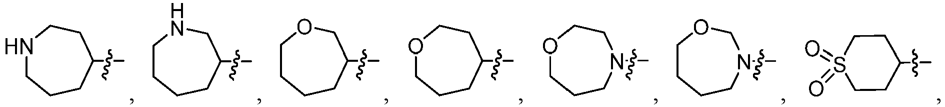 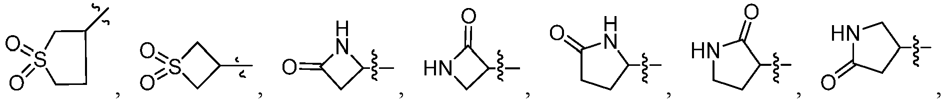 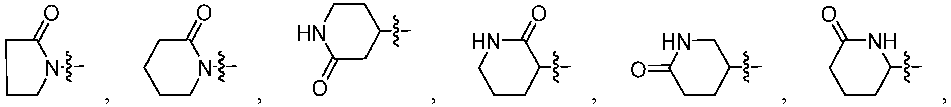 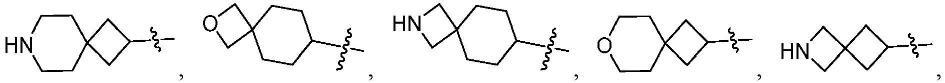 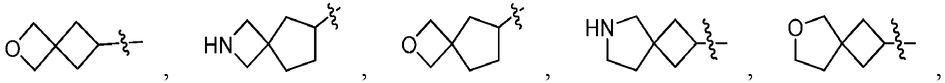 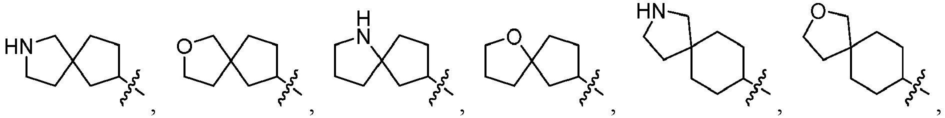 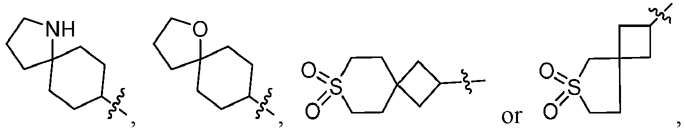 wherein the above groups are substituted with one or more R¹⁷, located at any substitutable site of the groups.

Preferably, R¹⁷ is independently selected from hydrogen, halogen, hydroxy, mercapto, amino, cyano, carbonyl, sulfoxide, sulfone, -R¹¹, -OR¹¹, -SR¹¹, -NH(R¹¹), -N(R¹¹)(R¹¹), 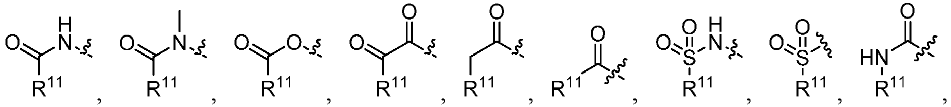 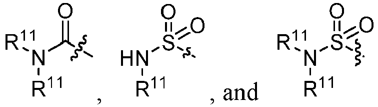 in each occurrence.

Preferably, R¹¹ is independently one selected from hydrogen, hydroxy, cyano, amino, trifluoromethyl, difluoromethyl, trifluoroethyl, difluoroethyl, methyl, ethyl, n-propyl, isopropyl, cyclopropyl, n-butyl, isobutyl, tert-butyl, sec-butyl, methoxy, ethoxy, n-propoxy, isopropoxy, cyclopropoxy, n-butoxy, isobutoxy, tert-butoxy, sec-butoxy, 4- to 6-membered heterocyclyl, aryl and 5- to 6-membered heteroaryl in each occurrence.

In some preferable embodiments of the present disclosure, A does not exist.

In some preferable embodiments of the present disclosure, B is selected from N.

In some preferable embodiments of the present disclosure, n=1, and E is selected from -CH(R¹³)- or -N(R¹³)-, where R¹³ is one selected from hydrogen, hydroxy, halogen, and methyl; more preferably, n=1, and E is selected from -CH₂- or -NH-.

In some preferable embodiments of the present disclosure, o=0, 1 or 2, more preferably, o=1.

In some preferable embodiments of the present disclosure, the H ring is one selected from phenyl ring, 5- to 6-membered heteroaryl ring, 5-membered fused to 6-membered heteroaryl ring, and 6-membered fused to 5-membered heteroaryl ring, which may be independently substituted with one or more R¹⁵, wherein R¹⁵ is selected from hydrogen, halogen, hydroxyl, mercapto, amino, cyano, C₁₋₆ alkyl, C₂₋₆ alkenyl, C₂₋₆ alkynyl, C₃₋₆ cycloalkyl, 4- to 6-membered heterocyclyl, aryl, and 5- to 6-membered heteroaryl.

As one preferable embodiment, the H ring is a phenyl ring, which may be independently substituted with one or more R¹⁵.

As one preferable embodiment, the H ring is 5- to 6-membered heteroaryl ring selected from: 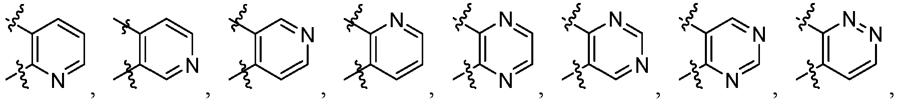 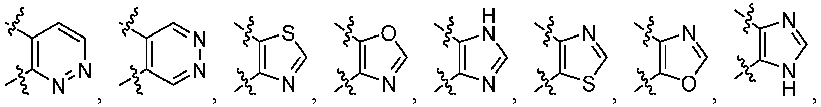 and the 5-to 6-membered heteroaryl ring may be independently substituted with one or more R¹⁵.

As one preferable embodiment, the H ring is 5-membered fused to 6-membered heteroaryl ring selected from: 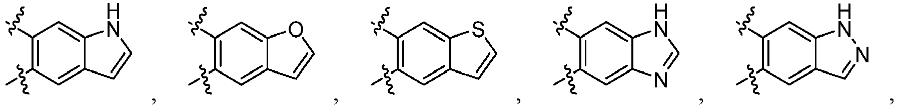 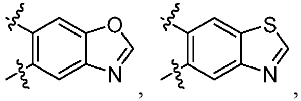 and the 5-membered fused to 6-membered heteroaryl ring may be independently substituted with one or more R¹⁵.

As one preferable embodiment, the H ring is 6-membered fused to 5-membered heteroaryl ring selected from: 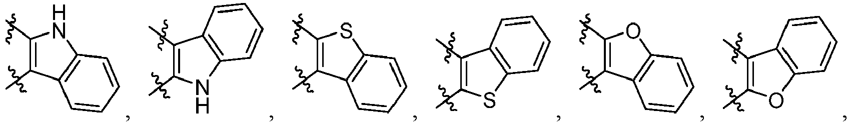 and the 6-membered fused to 5-membered heteroaryl ring may be independently substituted with one or more R¹⁵.

Further preferably, R¹⁵ is selected from hydrogen, halogen, hydroxyl, mercapto, amino, cyano, C₁₋₆ alkyl, C₂₋₆ alkenyl, C₂₋₆ alkynyl, C₃₋₆ cycloalkyl, 4- to 6-membered heterocyclyl, aryl, and 5- to 6-membered heteroaryl.

In a preferable embodiment of the present disclosure, a compound represented by formula (II)A, formula (II)B or formula (II)C, and stereoisomers, geometric isomers, tautomers, pharmaceutically acceptable salts, prodrugs, hydrates, solvates or isotope labeled analogues thereof are provided, 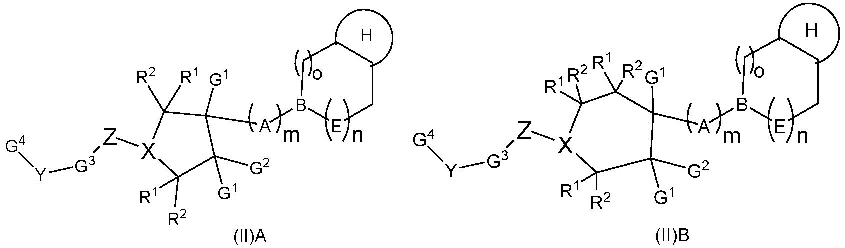 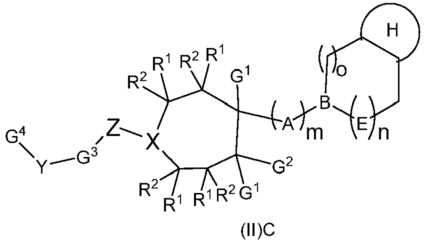 wherein X is N, B is N, m=0, o=1, n=1, E is -C(R¹³)(R¹³)-, where R¹³ is independently selected from hydrogen, halogen, C₁₋₆ alkyl and C₃₋₆ cycloalkyl, preferably hydrogen and halogen, more preferably hydrogen in each occurrence; the H ring is a phenyl ring, and may be independently substituted with one or more R¹⁵, where R¹⁵ is selected from hydrogen, halogen, C₁₋₆ alkyl and C₃₋₆ cycloalkyl, preferably hydrogen and halogen, more preferably hydrogen; G¹ is independently selected from hydrogen, halogen, and C₁₋₅ linear or branched alkyl, preferably hydrogen and halogen, more preferably hydrogen in each occurrence; G² is selected from hydroxy, mercapto, amino and cyano, preferably hydroxy, mercapto and amino; more preferably hydroxy; the groups R¹, R², Z, G³, Y, and G⁴ are as defined above in the formula (I), preferably as described in the following embodiments.

In a preferable embodiment of the present disclosure, a compound represented by formula (II)A, formula (II)B or formula (II)C, and stereoisomers, geometric isomers, tautomers, pharmaceutically acceptable salts, prodrugs, hydrates, solvates or isotope labeled analogues thereof are provided, wherein R¹ and R² are independently selected from hydrogen, halogen, hydroxy, mercapto, amino, cyano, C₁₋₆ alkyl, C₃₋₆ cycloalkyl, 4- to 6-membered heterocyclyl, -OR³, -NH(R³), and -NR³R⁴ in each occurrence, R³ and R⁴ are independently selected from C₁₋₆ alkyl, C₂₋₆ alkenyl, C₂₋₆ alkynyl, C₃₋₆ cycloalkyl, 4- to 6-membered heterocyclyl, aryl, and 5- to 6-membered heteroaryl in each occurrence; preferably, R¹ and R² are independently selected from hydrogen, halogen, C₁₋₆ alkyl, C₃₋₆ cycloalkyl, and -OC₁₋₆ alkyl, preferably hydrogen, halogen and methyl, most preferably hydrogen in each occurrence.

In a preferable embodiment of the present disclosure, a compound represented by formula (II)A, formula (II)B or formula (II)C, and stereoisomers, geometric isomers, tautomers, pharmaceutically acceptable salts, prodrugs, hydrates, solvates or isotope labeled analogues thereof are provided, wherein Z is selected from - (chemical bond) and -CO-, preferably -CO-.

In a preferable embodiment of the present disclosure, a compound represented by formula (II)A, formula (II)B or formula (II)C, and stereoisomers, geometric isomers, tautomers, pharmaceutically acceptable salts, prodrugs, hydrates, solvates or isotope labeled analogues thereof are provided, wherein G³ is selected from optionally substituted C₆₋₁₀ aryl, and optionally substituted 5- to 10-membered heteroaryl, wherein in the 5- to 10-membered heteroaryl, the heteroatom is N, O, or S, and the number of heteroatoms is 1, 2, 3 or 4, preferably 1 or 2, the term "optionally substituted" means that the hydrogen on the group to be substituted is unsubstituted or one or more substitutable sites of the group to be substituted are independently substituted by R¹⁶, R¹⁶ is as defined above in the formula (I), preferably as described in the following embodiments.

In a preferable embodiment of the present disclosure, a compound represented by formula (II)A, formula (II)B or formula (II)C, and stereoisomers, geometric isomers, tautomers, pharmaceutically acceptable salts, prodrugs, hydrates, solvates or isotope labeled analogues thereof are provided, wherein G³ is selected from optionally substituted 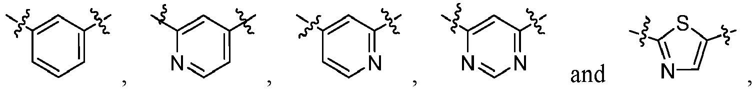 more preferably optionally substituted 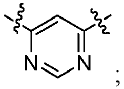 the term "optionally substituted" means that the hydrogen on the group to be substituted is unsubstituted or one or more substitutable sites of the group to be substituted are independently substituted by R¹⁶, where R¹⁶ is as defined above in the formula (I); further preferably, R¹⁶ is independently selected from hydrogen, halogen, -R⁹, -OR⁹, -SR⁹, -SO(R⁹), -NH(R⁹), and -N(R⁹)(R¹⁰) in each occurrence, wherein R⁹ and R¹⁰ are independently selected from C₁₋₆ alkyl, C₂₋₆ alkenyl, C₂₋₆ alkynyl, C₃₋₆ cycloalkyl, aryl, and 5-to 6-membered heteroaryl, which may be optionally substituted with one or more of hydrogen, halogen, C₁₋₆ alkyl, and C₃₋₆ cycloalkyl, in each occurrence.

More preferably, R¹⁶ is independently selected from hydrogen, halogen, methyl and methoxy in each occurrence.

More preferably, R¹⁶ is independently selected from ethyl, n-propyl, isopropyl, n-butyl, isobutyl, n-pentyl, isopentyl, ethoxy, n-propoxy, isopropoxy, n-butoxy, isobutoxy, n-pentyloxy, isopentyloxy, trifluoromethyl, methoxy, amino, methylamino, dimethylamino, phenyl, pyridyl, vinyl, ethynyl, -OCF₃, -SCH(CH₃)₂, -OCH(CH₂CH₃)₂, -S(O)CH(CH₃)₂, 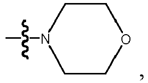 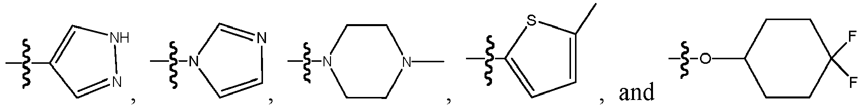 in each occurrence.

In a preferable embodiment of the present disclosure, a compound represented by formula (II)A, formula (II)B or formula (II)C, and stereoisomers, geometric isomers, tautomers, pharmaceutically acceptable salts, prodrugs, hydrates, solvates or isotope labeled analogues thereof are provided, wherein Y is selected from - (chemical bond), -NH-, -O-, -S-, -SO-, -SO₂-, -N(CH₃)-, -S(O)NH-, -S(O)₂NH-, -NHSO- and -NHS(O)₂-; more preferably, Y is selected from - (chemical bond), -S-, -O- and -NH-; further preferably, Y is -NH-.

In a preferable embodiment of the present disclosure, a compound represented by formula (II)A, formula (II)B or formula (II)C, and stereoisomers, geometric isomers, tautomers, pharmaceutically acceptable salts, prodrugs, hydrates, solvates or isotope labeled analogues thereof are provided, wherein G⁴ is as described above in each embodiment, more preferably, G⁴ is selected from 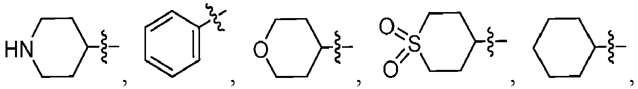 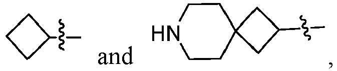 most preferably 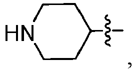 where the above groups are substituted with one or more R¹⁷, located at any substitutable site of the groups.

In a preferable embodiment of the present disclosure, a compound represented by formula (II)A, formula (II)B or formula (II)C, and stereoisomers, geometric isomers, tautomers, pharmaceutically acceptable salts, prodrugs, hydrates, solvates or isotope labeled analogues thereof are provided, wherein G⁴ is 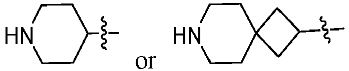 substituted with one or more R¹⁷, and preferably at least one R¹⁷ is on N atom.

In a preferable embodiment of the present disclosure, a compound represented by formula (II)A, formula (II)B or formula (II)C, and stereoisomers, geometric isomers, tautomers, pharmaceutically acceptable salts, prodrugs, hydrates, solvates or isotope labeled analogues thereof are provided, wherein R¹⁷ is independently selected from hydrogen, halogen, hydroxy, mercapto, amino, cyano, carbonyl, -R¹¹, -OR¹¹, -SR¹¹, 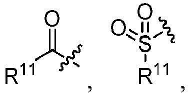 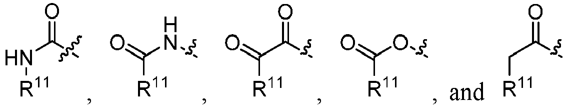 in each occurrence, preferably, R¹⁷ is -OR¹¹ or 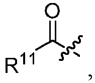 most preferably R¹⁷ is 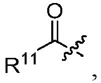 wherein R¹¹ is as defined above in the formula (I), preferably, R¹¹ is independently selected from C₁₋₆ alkyl, C₃₋₆ cycloalkyl, 4- to 6-membered heterocyclyl, aryl, and 5- to 6-membered heteroaryl, which may be optionally substituted with one or more of hydrogen, halogen, hydroxy, mercapto, amino, cyano, and C₁₋₆ alkyl, in each occurrence; more preferably, R¹¹ is independently one selected from hydrogen, hydroxy, cyano, amino, trifluoromethyl, difluoromethyl, trifluoroethyl, difluoroethyl, methyl, ethyl, n-propyl, isopropyl, cyclopropyl, n-butyl, isobutyl, tert-butyl, sec-butyl, methoxy, ethoxy, n-propoxy, isopropoxy, cyclopropoxy, n-butoxy, isobutoxy, tert-butoxy, sec-butoxy, 4- to 6-membered heterocyclyl, aryl and 5- to 6-membered heteroaryl in each occurrence.

In a preferable embodiment of the present disclosure, a compound represented by formula (II)A, formula (II)B or formula (II)C, and stereoisomers, geometric isomers, tautomers, pharmaceutically acceptable salts, prodrugs, hydrates, solvates or isotope labeled analogues thereof are provided, wherein G⁴ is 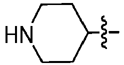 substituted with 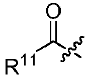 on N atom, where R¹¹ is selected from C₁₋₆ alkyl or aryl, which may be optionally substituted with one or more of hydrogen, halogen, hydroxy, and C₁₋₆ alkyl, or R¹¹ is selected from trifluoromethyl, difluoromethyl, trifluoroethyl, difluoroethyl, methyl, ethyl, n-propyl, isopropyl, cyclopropyl, n-butyl, isobutyl, tert-butyl, sec-butyl, methoxy, ethoxy, n-propoxy, isopropoxy, cyclopropoxy, n-butoxy, isobutoxy, tert-butoxy, sec-butoxy, difluorocyclobutyl, 4- to 6-membered heterocyclyl and phenyl.

In a preferable embodiment of the present disclosure, a compound represented by formula (II)A, formula (II)B or formula (II)C, and stereoisomers, geometric isomers, tautomers, pharmaceutically acceptable salts, prodrugs, hydrates, solvates or isotope labeled analogues thereof are provided, wherein G⁴ is C₁₋₆ alkyl optionally substituted with R¹⁷, preferably C₁₋₄ alkyl optionally substituted with R¹⁷, more preferably ethyl, n-propyl or n-butyl optionally substituted with R¹⁷; R¹⁷ is selected from halogen and methoxy.

In a preferable embodiment of the present disclosure, the compound represented by formula (I), and stereoisomers, geometric isomers, tautomers, pharmaceutically acceptable salts, prodrugs, hydrates, solvates or isotope labeled analogues thereof are selected from the following compounds:

| | | | |
|---|---|---|---|
| 1 | 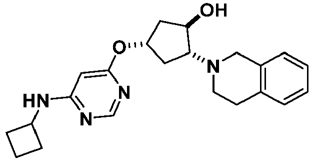 | 2 | 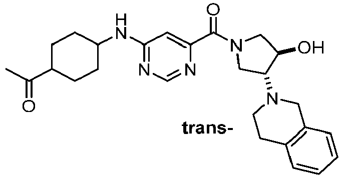 |
| 3 | 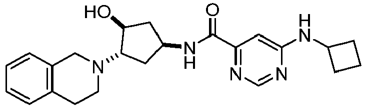 | 4 | 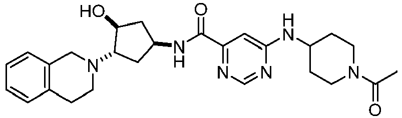 |
| 5 | 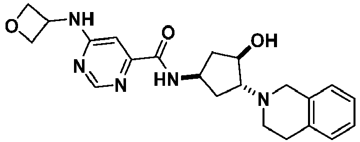 | 6 | 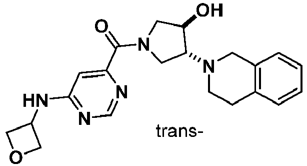 |
| 7 | 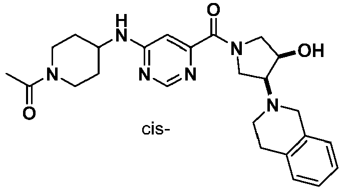 | 8 | 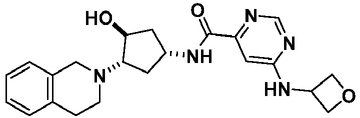 |
| 9 | 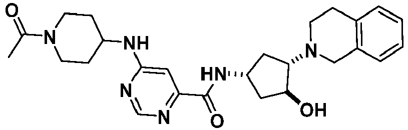 | 10 | 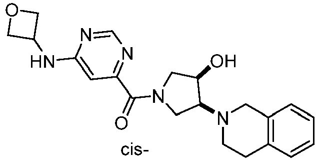 |
| 11 | 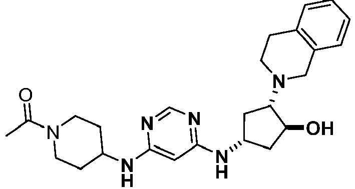 | 12 | 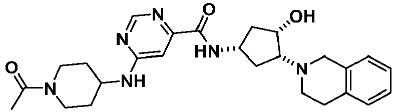 |
| 13 | 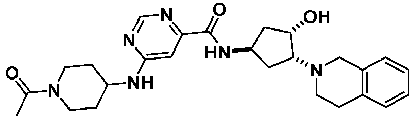 | 14 | 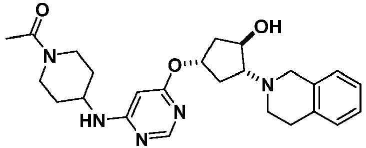 |
| 15 | 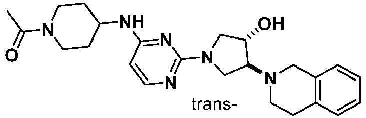 | 16 | 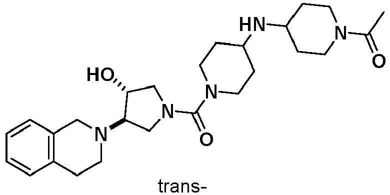 |
| 17 | 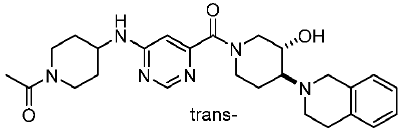 | 18 | 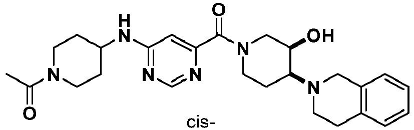 |
| 19 | 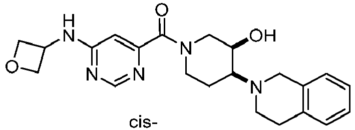 | 20 | 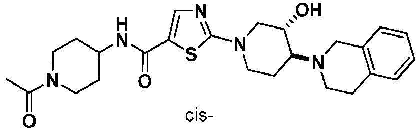 |
| 21 | 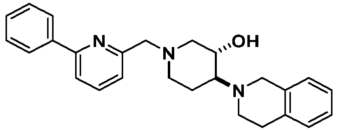 | 22 | 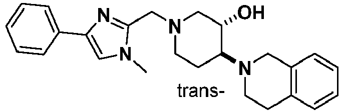 |
| 23 | 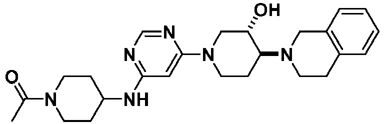 | 24 | 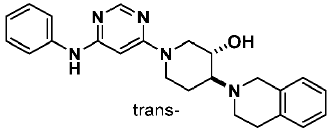 |
| 25 | 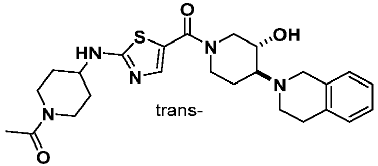 | 26 | 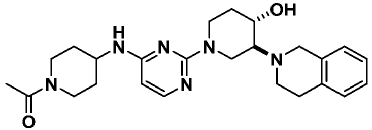 |
| 27 | 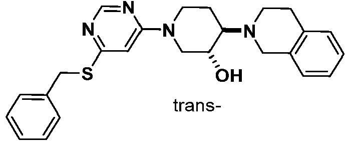 | 28 | 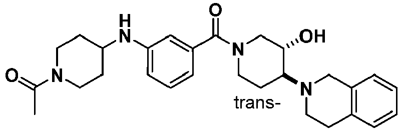 |
| 29 | 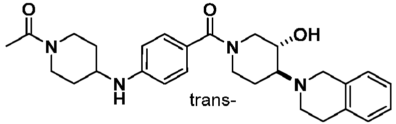 | 30 | 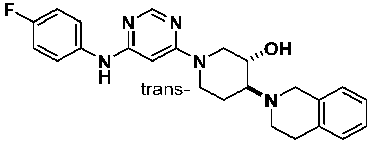 |
| 31 | 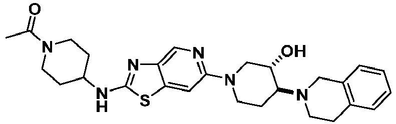 | 32 | 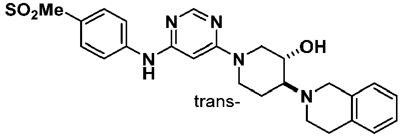 |
| 33 | 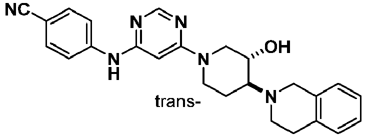 | 34 | 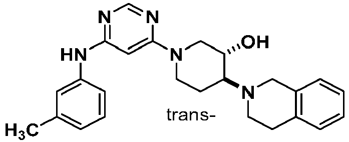 |
| 35 | 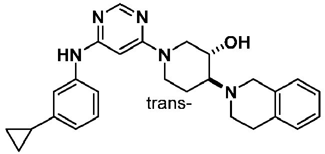 | 36 | 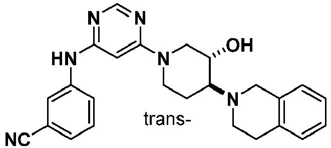 |
| 37 | 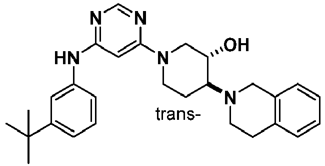 | 38 | 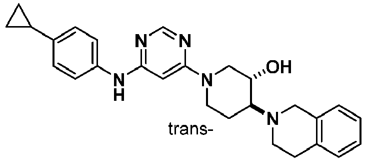 |
| 39 | 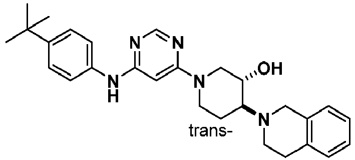 | 40 | 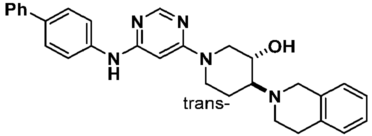 |
| 41 | 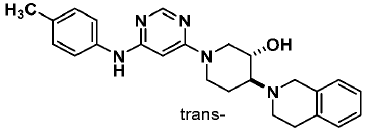 | 42 | 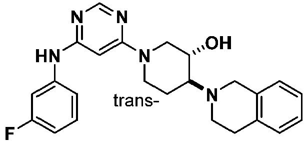 |
| 43 | 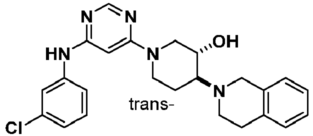 | 44 | 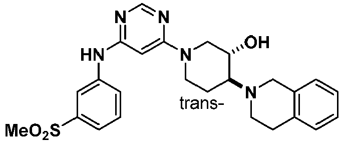 |
| 45 | 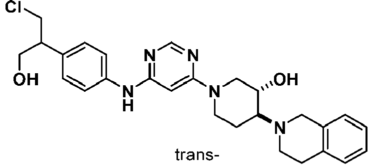 | 46 | 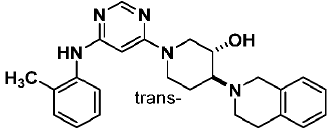 |
| 47 | 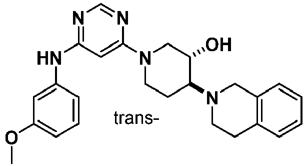 | 48 | 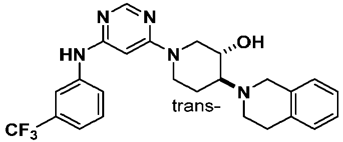 |
| 49 | 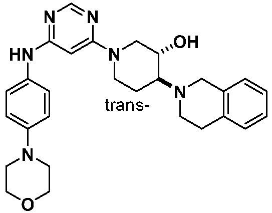 | 50 | 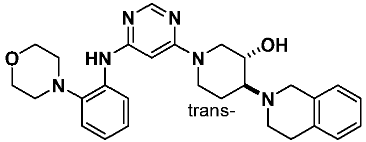 |
| 51 | 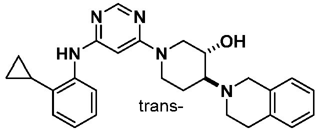 | 52 | 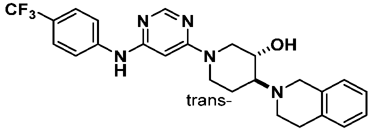 |
| 53 | 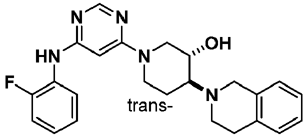 | 54 | 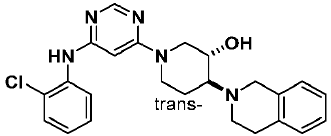 |
| 55 | 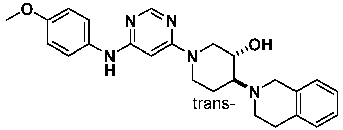 | 56 | 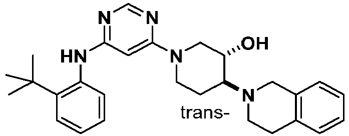 |
| 57 | 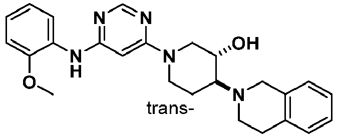 | 58 | 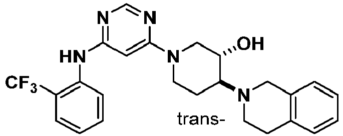 |
| 59 | 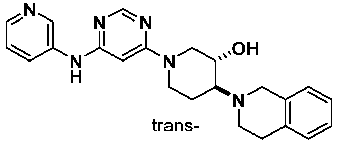 | 60 | 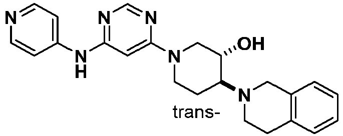 |
| 61 | 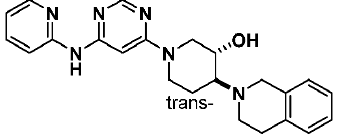 | 62 | 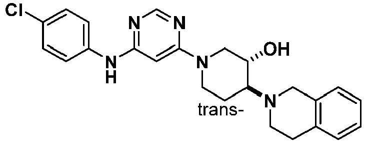 |
| 63 | 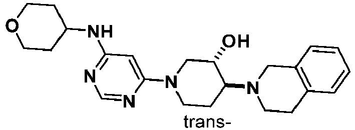 | 64 | 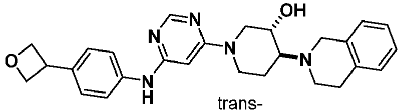 |
| 65 | 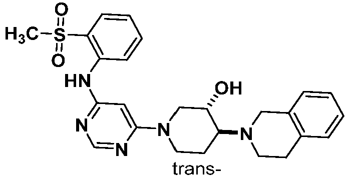 | 66 | 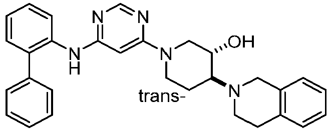 |
| 67 | 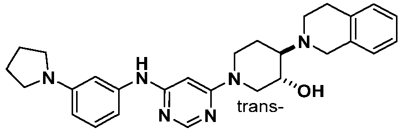 | 68 | 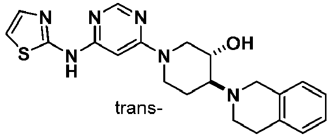 |
| 69 | 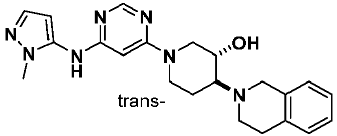 | 70 | 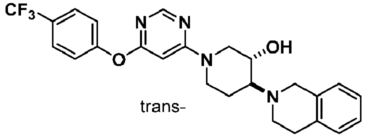 |
| 71 | 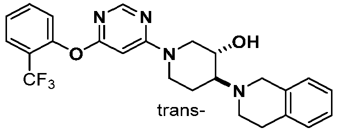 | 72 | 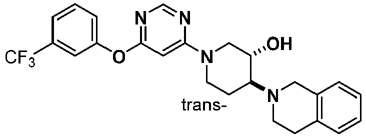 |
| 73 | 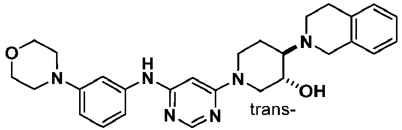 | 74 | 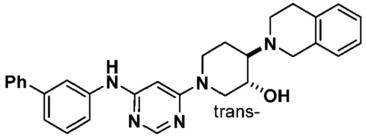 |
| 75 | 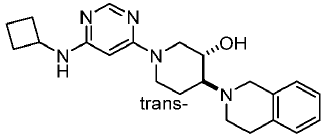 | 76 | 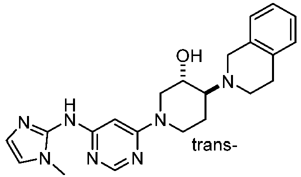 |
| 77 | 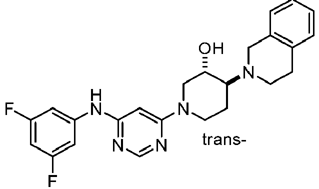 | 78 | 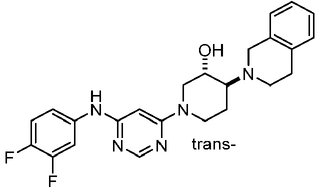 |
| 79 | 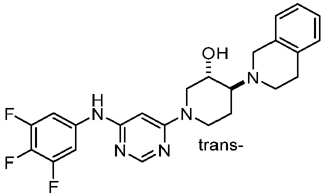 | 80 | 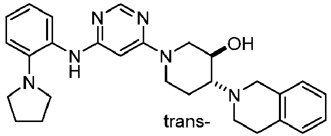 |
| 81 | 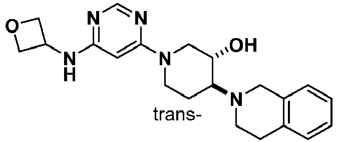 | 82 | 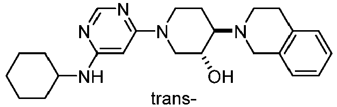 |
| 83 | 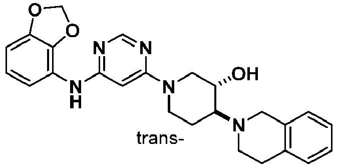 | 84 | 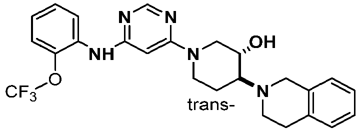 |
| 85 | 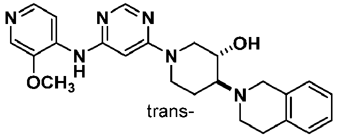 | 86 | 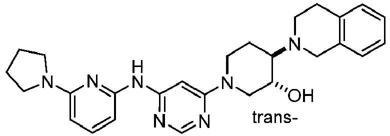 |
| 87 | 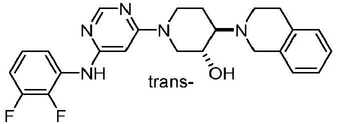 | 88 | 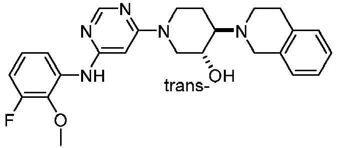 |
| 89 | 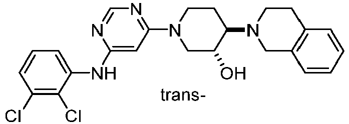 | 90 | 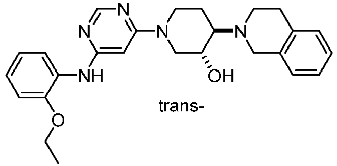 |
| 91 | 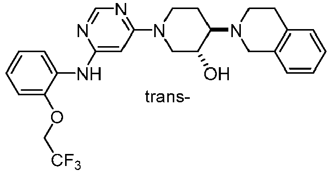 | 92 | 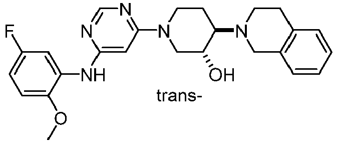 |
| 93 | 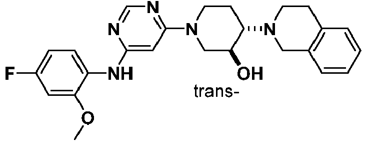 | 94 | 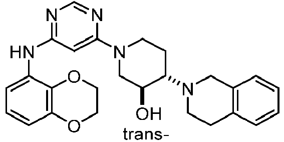 |
| 95 | 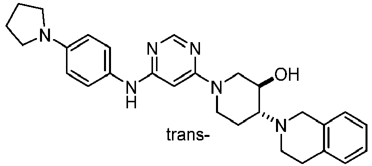 | 96 | 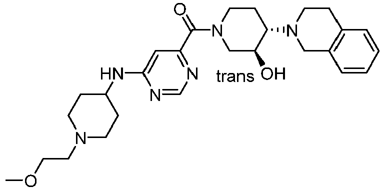 |
| 97 | 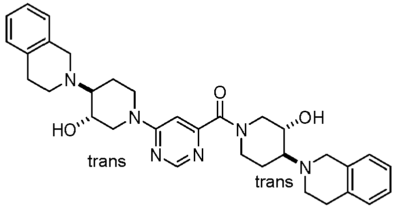 | 98 | 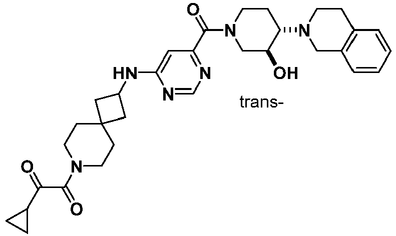 |
| 99 | 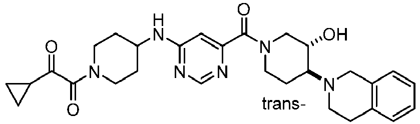 | 10 0 | 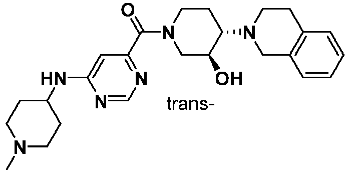 |
| 101 | 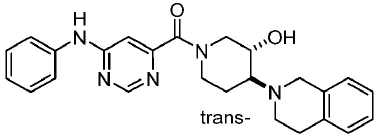 | 10 2 | 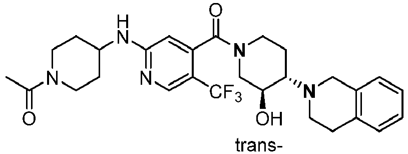 |
| 103 | 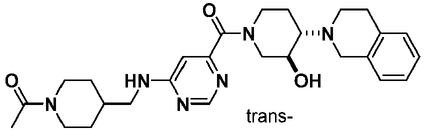 | 10 4 | 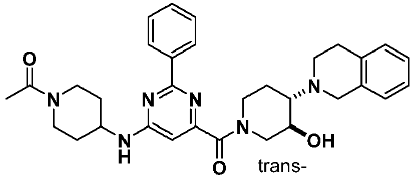 |
| 105 | 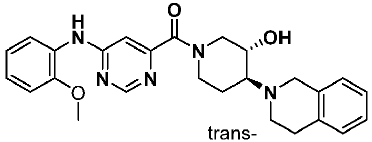 | 10 6 | 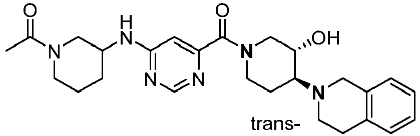 |
| 107 | 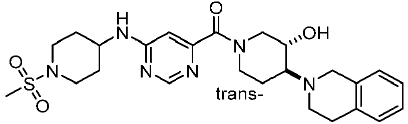 | 10 8 | 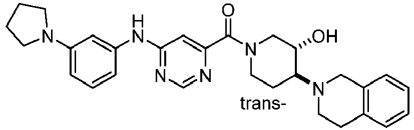 |
| 109 | 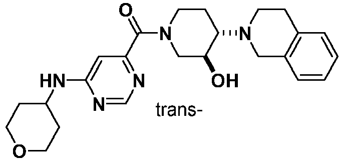 | 11 0 | 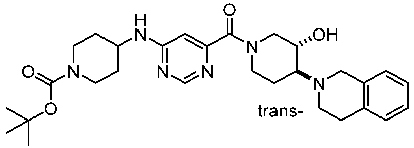 |
| 111 | 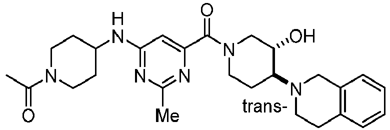 | 11 2 | 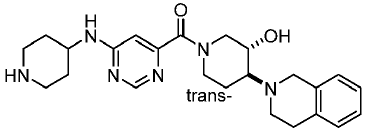 |
| 113 | 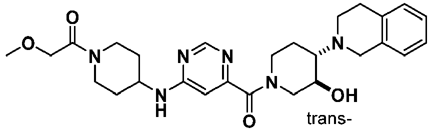 | 11 4 | 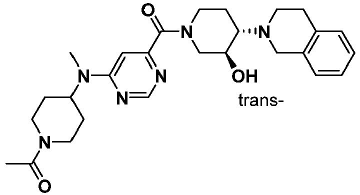 |
| 115 | 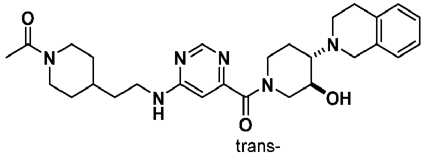 | 11 6 | 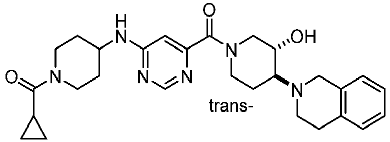 |
| 117 | 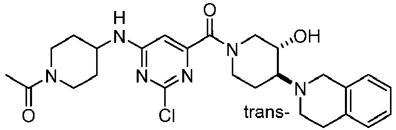 | 11 8 | 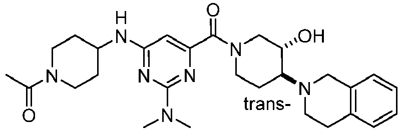 |
| 119 | 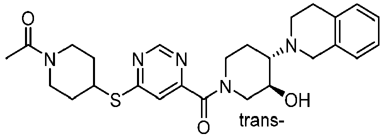 | 12 0 | 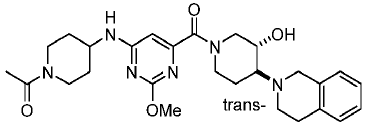 |
| 121 | 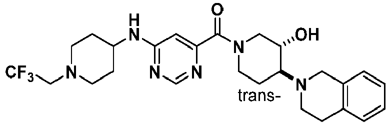 | 12 2 | 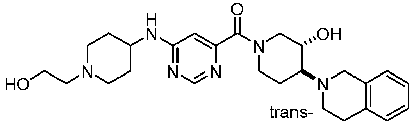 |
| 123 | 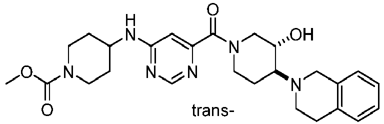 | 12 4 | 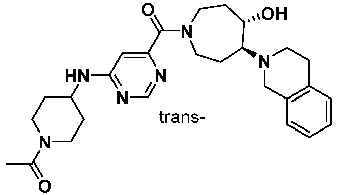 |
| 125 | 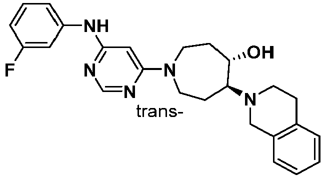 | 12 6 | 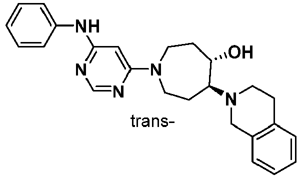 |
| 127 | 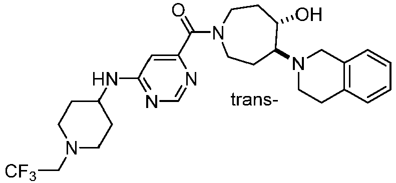 | 12 8 | 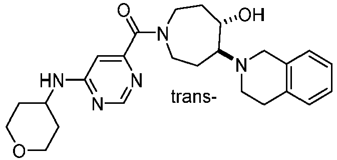 |
| 129 | 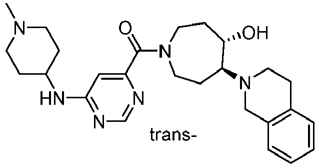 | 13 0 | 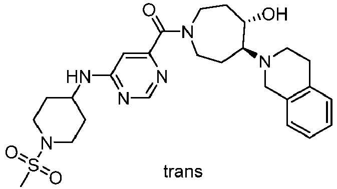 |
| 131 | 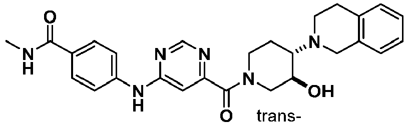 | 13 2 | 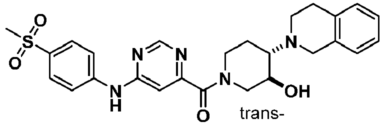 |
| 133 | 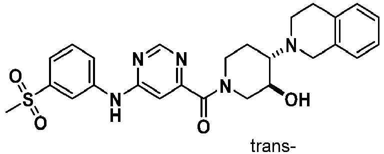 | 13 4 | 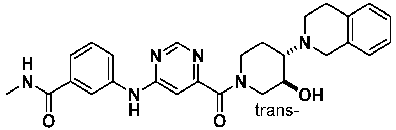 |
| 135 | 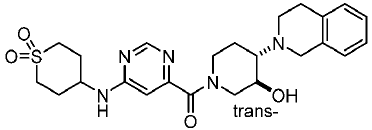 | 13 6 | 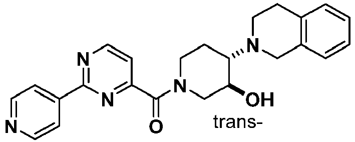 |
| 137 | 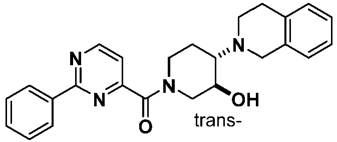 | 13 8 | 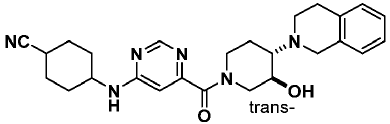 |
| 139 | 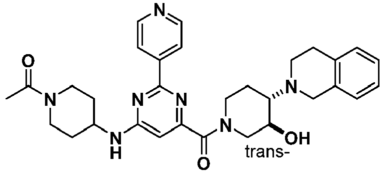 | 14 0 | 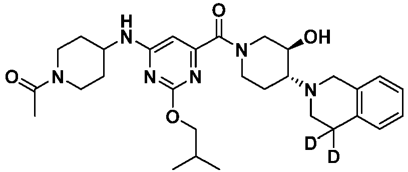 |
| 141 | 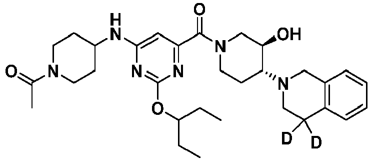 | 14 2 | 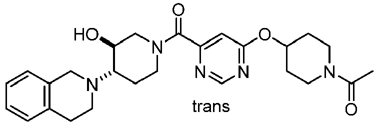 |
| 143 | 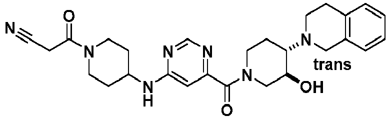 | 14 4 | 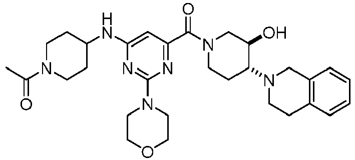 |
| 145 | 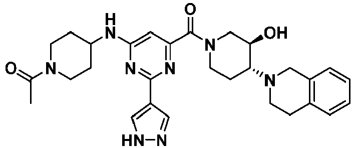 | 14 6 | 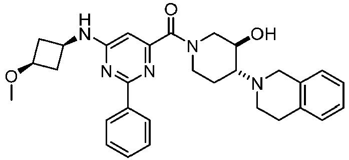 |
| 147 | 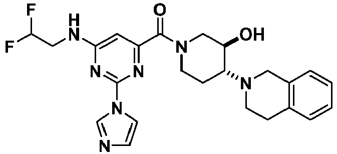 | 14 8 | 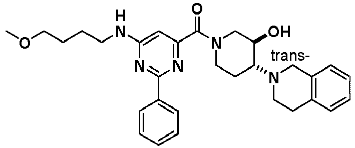 |
| 149 | 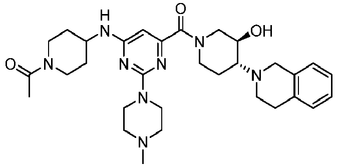 | 15 0 | 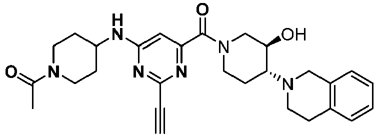 |
| 151 | 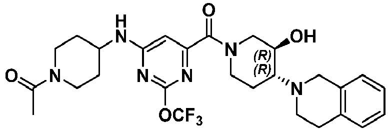 | 15 2 | 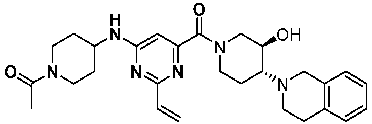 |
| 153 | 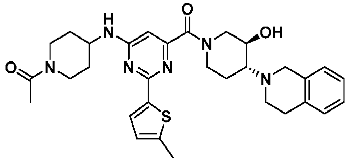 | 15 4 | 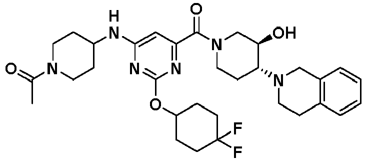 |
| 155 | 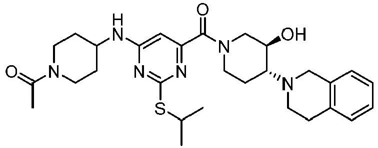 | 15 6 | 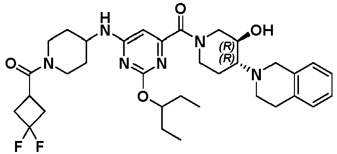 |
| 157 | 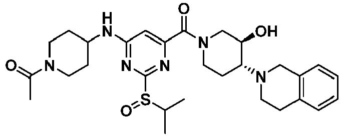 | | |

An object of the present disclosure also includes a method for preparing the compound represented by formula (I), and stereoisomers, geometric isomers, tautomers, pharmaceutically acceptable salts, prodrugs, hydrates, solvates or isotope labeled analogues thereof.

The method can be, for example, the method shown in Scheme 1: firstly LG^{a} (leaving group a) on the left chain of the G³-Z structure is linked to G⁴-YH, and then LG^{c} (leaving group c) of a ring structure is linked to LG^{b} (leaving group b) on the right chain of the G³-Z structure, so that the target compound is synthesized. Scheme 1 is as follows:

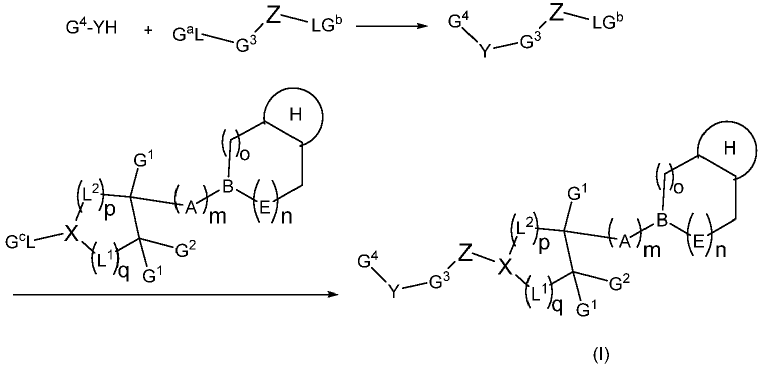

The method can be, for example, the method shown in Scheme 2: firstly LG^{c} (leaving group c) of a ring structure is linked to LG^{b} (leaving group b) on the right chain of the G³-Z structure, and then LG^{a} (leaving group a) on the left chain of the G³-Z structure is linked to G⁴-YH, so that the target compound is synthesized. Scheme 2 is as follows: 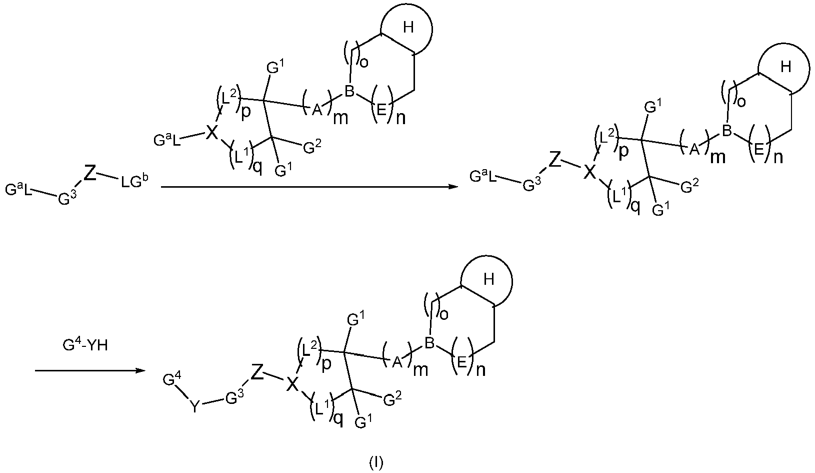 wherein in the above-mentioned preparation method, each substituent in the shown compound is as defined above.

The present disclosure further provides a pharmaceutical composition comprising the compound represented by formula (I), (II)A, (II)B or (II)C according to the present disclosure, or stereoisomers, geometric isomers, tautomers, pharmaceutically acceptable salts, prodrugs, hydrates, solvates or isotope labeled analogues thereof, and a pharmaceutically acceptable excipient.

An object of the present disclosure is further to provide use of the compound represented by formula (I), (II)A, (II)B or (II)C according to the present disclosure, or stereoisomers, geometric isomers, tautomers, pharmaceutically acceptable salts, prodrugs, hydrates, solvates or isotope labeled analogues thereof, in the preparation of a medicament for treating a disease mediated by PRMT5 inhibitor.

In some examples, the disease mediated by PRMT5 inhibitor is a disease related with cancer or tumor, and the representative examples of the cancer or tumor include but are not limited to skin cancer, bladder cancer, ovarian cancer, breast cancer, stomach cancer, pancreatic cancer, prostate cancer, colon cancer, lung cancer, bone cancer, brain cancer, neuroblastoma, rectal cancer, colon cancer, familial adenomatous polyposis cancer, hereditary nonpolyposis colorectal cancer, esophageal cancer, lip cancer, laryngeal cancer, hypopharyngeal cancer, tongue cancer, salivary gland cancer, gastric cancer, adenocarcinoma, medullary thyroid cancer, papillary thyroid cancer, kidney cancer, carcinoma of renal parenchyma, ovarian cancer, cervical cancer, corpus carcinoma, endometrial cancer, choriocarcinoma, pancreatic cancer, prostate cancer, testicular cancer, urinary cancer, melanoma, brain tumors such as glioblastoma, astrocytoma, meningioma, medulloblastoma and peripheral primitive neuroectodermal tumor, Hodgkin's lymphoma, non-Hodgkin's lymphoma, Burkitt's lymphoma, acute lymphoblastic leukemia (ALL), chronic lymphocytic leukemia (CLL), acute myelogenous leukemia (AML), chronic myelogenous leukemia (CML), adult T-cell leukemia lymphoma, diffuse large B-cell lymphoma (DLBCL), hepatocellular carcinoma, gallbladder carcinoma, bronchial carcinoma, small cell lung cancer, non-small cell lung cancer, multiple myeloma, basal cell tumor, teratoma, retinoblastoma, choroidal melanoma, seminoma, rhabdomyosarcoma, craniopharyngioma, osteosarcoma, chondrosarcoma, myoma, liposarcoma, fibrosarcoma, Ewing sarcoma, or plasmacytoma.

An object of the present disclosure is also to provide a method for preventing or treating a disease mediated by PRMT5 inhibitor, comprising administrating to a subject a therapeutically effective amount of the compound represented by formula (I), (II)A, (II)B or (II)C, or stereoisomers, geometric isomers, tautomers, pharmaceutically acceptable salts, prodrugs, hydrates, solvates or isotope labeled analogues thereof, or a pharmaceutical composition according to the present disclosure.

In some examples, the disease mediated by PRMT5 inhibitor is a disease related with cancer or tumor, and the representative examples of the cancer or tumor include but are not limited to skin cancer, bladder cancer, ovarian cancer, breast cancer, stomach cancer, pancreatic cancer, prostate cancer, colon cancer, lung cancer, bone cancer, brain cancer, neuroblastoma, rectal cancer, colon cancer, familial adenomatous polyposis cancer, hereditary nonpolyposis colorectal cancer, esophageal cancer, lip cancer, laryngeal cancer, hypopharyngeal cancer, tongue cancer, salivary gland cancer, gastric cancer, adenocarcinoma, medullary thyroid cancer, papillary thyroid cancer, kidney cancer, carcinoma of renal parenchyma, ovarian cancer, cervical cancer, corpus carcinoma, endometrial cancer, choriocarcinoma, pancreatic cancer, prostate cancer, testicular cancer, urinary cancer, melanoma, brain tumors such as glioblastoma, astrocytoma, meningioma, medulloblastoma and peripheral primitive neuroectodermal tumor, Hodgkin's lymphoma, non-Hodgkin's lymphoma, Burkitt's lymphoma, acute lymphoblastic leukemia (ALL), chronic lymphocytic leukemia (CLL), acute myelogenous leukemia (AML), chronic myelogenous leukemia (CML), adult T-cell leukemia lymphoma, diffuse large B-cell lymphoma (DLBCL), hepatocellular carcinoma, gallbladder carcinoma, bronchial carcinoma, small cell lung cancer, non-small cell lung cancer, multiple myeloma, basal cell tumor, teratoma, retinoblastoma, choroidal melanoma, seminoma, rhabdomyosarcoma, craniopharyngioma, osteosarcoma, chondrosarcoma, myoma, liposarcoma, fibrosarcoma, Ewing sarcoma, or plasmacytoma.

When the compound according to the present disclosure or a pharmaceutically acceptable salt thereof is administered in combination with another anticancer agent for the treatment of cancer or tumor or immune checkpoint inhibitor, the compound according to the present disclosure or a pharmaceutically acceptable salt thereof can provide an enhanced anticancer effect.

Representative examples of the anticancer agent for the treatment of cancer or tumor may include, but are not limited to, signal transduction inhibitor, chlorambucil, melphalan, cyclophosphamide, ifosfamide, busulfan, carmustine, lomustine, streptozotocin, cisplatin, carboplatin, oxaliplatin, dacarbazine, temozolomide, procarbazine, methotrexate, fluorouracil, cytarabine, gemcitabine, mercaptopurine, fludarabine, vinblastine, vincristine, vinorelbine, paclitaxel, docetaxel, topotecan, irinotecan, etoposide, trabectedin, dactinomycin, doxorubicin, epirubicin, daunorubicin, mitoxantrone, bleomycin, mitomycin C, ixabepilone, tamoxifen, flutamide, gonadorelin analogues, megestrol, prednisone, dexamethasone, methylprednisolone, thalidomide, interferon alpha, calcium leucovorin, sirolimus, sirolimus ester, everolimus, afatinib, alisertib, amuvatinib, apatinib, axitinib, bortezomib, bosutinib, brivanib, cabozantinib, cediranib, crenolanib, crizotinib, dabrafenib, dacomitinib, danusertib, dasatinib, dovitinib, erlotinib, foretinib, ganetespib, gefitinib, ibrutinib, icotinib, imatinib, iniparib, lapatinib, lenvatinib, linifanib, linsitinib, masitinib, momelotinib, motisanib, neratinib, nilotinib, niraparib, oprozomib, olaparib, pazopanib, pictilisib, ponatinib, quizartinib, regorafenib, rigosertib, rucaparib, ruxolitinib, saracatinib, saridegib, sorafenib, sunitinib, tilatinib, tivantinib, tivozanib, tofacitinib, trametinib, vandetanib, veliparib, vemurafenib, erivedge, volasertib, alemtuzumab, bevacizumab, berentuzumab vedotin, cartuxomab, cetuximab, denosumab, gemtuzumab, ipilimumab, nimotuzumab, ofatumumab, panitumumab, rituximab, tositumumab, trastuzumab, PI3K inhibitor, CSF1R inhibitor, A2A and/or A2B receptor antagonist, IDO inhibitor, anti-PD-1 antibody, anti-PD-L1 antibody, LAG3 antibody, TIM-3 antibody and anti-CTLA-4 antibody, anti-OX40 antibody and anti-OX40L antibody or any combination thereof.

In some embodiments, the pharmaceutical composition according to the present disclosure may further comprise the other anticancer agent or immune checkpoint inhibitor as described above.

In some embodiments, the method for preventing or treating a disease mediated by PRMT5 inhibitor according to the present disclosure may further include administrating to the subject the other anticancer agent or immune checkpoint inhibitor as described above.

### Definition

Unless specified otherwise, the term "alkyl" refers to a monovalent saturated aliphatic hydrocarbyl, including a linear or branched group containing 1 to 20 carbon atoms, preferably 1 to 10 carbon atoms (i.e. C₁₋₁₀ alkyl), further preferably 1 to 8 carbon atoms (C₁₋₈ alkyl), still further preferably 1 to 6 carbon atoms (i.e. C₁₋₆ alkyl). For example "C₁₋₆ alkyl" means that the group is an alkyl group, and the number of carbon atoms in the carbon chain is between 1 and 6 (specifically 1, 2, 3, 4, 5 or 6). Examples include, but are not limited to, methyl, ethyl, n-propyl, isopropyl, n-butyl, isobutyl, tert-butyl, sec-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl , n-octyl, and the like.

Unless specified otherwise, the term "cycloalkyl" refers to a monocyclic saturated aliphatic hydrocarbyl having a specified number of carbon atoms, preferably containing 3 to 12 carbon atoms (i.e. C₃₋₁₂ cycloalkyl), further preferably 3 to 10 carbon atoms (C₃₋₁₀ cycloalkyl), still further preferably 3 to 6 carbon atoms (C₃₋₆ cycloalkyl), 4 to 6 carbon atoms (C₄₋₆ cycloalkyl), or 5 to 6 carbon atoms (C₅₋₆ cycloalkyl). Examples include, but are not limited to, cyclopropyl, cyclobutyl, cyclopentyl, cyclohexyl, methylcyclopropyl, 2-ethyl-cyclopentyl, dimethylcyclobutyl, and the like.

Unless specified otherwise, the term "alkoxy" refers to -O-alkyl where the alkyl is as defined above, that is, containing 1 to 20 carbon atoms, preferably 1 to 10 carbon atoms, further preferably 1 to 8 carbon atoms, still further preferably 1 to 6 carbon atoms (specifically 1, 2, 3, 4, 5 or 6). Representative examples include, but are not limited to, methoxy, ethoxy, propoxy, isopropoxy, butoxy, 1-methylpropoxy, 2-methylpropoxy, t-butoxy, pentyloxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, and the like.

The term "halogen" or "halo" refer to F, Cl, Br, or I, unless specified otherwise. The term "haloalkyl" refers to an alkyl group as defined above, in which one, two or more hydrogen atoms or all hydrogen atoms are substituted by halogen. Representative examples of haloalkyl include CCl₃, CF₃, CHCl₂, CH₂Cl, CH₂Br, CH₂I, CH₂CF₃, CF₂CF₃ and the like.

Unless specified otherwise, the term "heterocyclyl" refers to a saturated or partially unsaturated monocyclic, bicyclic or polycyclic hydrocarbyl substituent, having a non-aromatic structure and containing 3 to 20 ring atoms, of which 1, 2, 3 or more ring atoms are selected from N, O or S, and the remaining ring atoms are C. Preferably, it contains 3 to 12 ring atoms, more preferably 3 to 10 ring atoms, or 3 to 8 ring atoms, or 3 to 6 ring atoms, or 4 to 6 ring atoms, or 5 to 6 ring atoms. The number of heteroatoms is preferably 1 to 4, more preferably 1 to 3 (i.e. 1, 2 or 3). Examples of monocyclic heterocyclyl include pyrrolidinyl, imidazolidinyl, tetrahydrofuranyl, dihydropyrrolyl, piperidinyl, piperazinyl, pyranyl, and the like. Polycyclic heterocyclyls include spiro, fused and bridged heterocyclyls.

Unless specified otherwise, the term "carbocyclyl" or "carbocycle" refers to a non-aromatic cyclic hydrocarbyl having from 3 to 14 ring carbon atoms ("C₃₋₁₄ carbocyclyl") and having no heteroatom in the non-aromatic ring system. In some embodiments, the carbocyclyl has 3 to 12 ring carbon atoms ("C₃₋₁₂ carbocyclyl"), or 4 to 12 ring carbon atoms ("C₄₋₁₂ carbocyclyl"), or 3 to 10 ring carbon atoms ("C₃₋₁₀ carbocyclyl"). In some embodiments, the carbocyclyl group has 3 to 8 ring carbon atoms ("C₃₋₈ carbocyclyl"). In some embodiments, the carbocyclyl group has 3 to 7 ring carbon atoms ("C₃₋₇ carbocyclyl"). In some embodiments, the carbocyclyl group has 4 to 6 ring carbon atoms ("C₄₋₆ carbocyclyl"). In some embodiments, the carbocyclyl group has 5 to 10 ring carbon atoms ("C₅₋₁₀ carbocyclyl") or 5 to 7 ring carbon atoms ("C₅₋₇ carbocyclyl"). Exemplary C₃₋₆ carbocyclyl groups include, but are not limited to, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆) and the like. Exemplary C₃₋₈ carbocyclyl groups include, but are not limited to, the aforementioned C₃₋₆ carbocyclyl groups and cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptyl (C₇), bicyclo[2.2.2]octyl (C₈) and the like. Exemplary C₃₋₁₀ carbocyclyl groups include, but are not limited to, the aforementioned C₃₋₈ carbocyclyl groups and cyclononyl (C₉), cyclononenyl (C₉), cyclodecyl (C₁₀), cyclodecenyl (C₁₀), octahydro-1H-indenyl (C₉), decalinyl (C₁₀), spiro[4.5]decyl (C₁₀) and the like. As in the above examples, in certain embodiments, the carbocyclyl group is monocyclic ("monocyclic carbocyclyl") or a fused (fused carbocyclyl), bridged (bridged carbocyclyl) or spiro-fused (spirocyclyl) ring system, such as a bicyclic ring system ("bicyclic carbocyclyl"), and may be saturated or partially unsaturated. The "carbocyclyl" also includes ring systems, in which the carbocyclyl ring as defined above is fused by one or more aryl or heteroaryl groups, wherein the attachment point is on the carbocyclyl ring and in such cases, the number of carbon still indicates the number of carbon in the carbocyclic ring system. In certain embodiments, each instance of the carbocyclyl group is independently optionally substituted, e.g., unsubstituted (an "unsubstituted carbocyclyl") or substituted with one or more substituents (a "substituted carbocyclyl"). In certain embodiments, the carbocyclyl group is unsubstituted C₃₋₁₀ carbocyclyl. In certain embodiments, the carbocyclyl group is substituted C₃₋₁₀ carbocyclyl.

Unless specified otherwise, the term "aryl" denotes monocyclic, bicyclic and tricyclic aromatic carbocyclic ring systems containing 6 to 16 carbon atoms, or 6 to 14 carbon atoms, or 6 to 12 carbon atoms, or 6 to 10 carbon atoms, preferably 6-10 carbon atoms. The term "aryl" may be used interchangeably with the term "aromatic ring". Examples of aryl groups may include, but are not limited to, phenyl, naphthyl, anthracenyl, phenanthrenyl, pyrenyl, or the like.

Unless specified otherwise, the term "heteroaryl" means an aromatic monocyclic or polycyclic ring system having a 5-12 membered structure, or preferably a 5- to 10-membered structure, or a 5-8 membered structure, more preferably a 5- to 6-membered structure, in which 1, 2, 3 or more ring atoms are heteroatoms and the remaining atoms are carbon, the heteroatoms are independently selected from O, N or S, and the number of heteroatoms is preferably 1, 2 or 3. Examples of heteroaryl include, but are not limited to, furyl, thienyl, oxazolyl, thiazolyl, isoxazolyl, oxadiazolyl, thiadiazolyl, pyrrolyl, pyrazolyl, imidazolyl, triazolyl , tetrazolyl, pyridyl, pyrimidyl, pyrazinyl, pyridazinyl, thiodiazolyl, triazinyl, phthalazinyl, quinolinyl, isoquinolinyl, pteridyl, purinyl, indolyl, isoindolyl, indazolyl, benzofuryl, benzothienyl, benzopyridyl, benzopyrimidinyl, benzopyrazinyl, benzimidazolyl, benzophthalazinyl, pyrrolo[2,3-b]pyridyl, imidazo[1,2-a]pyridyl, pyrazolo[1,5-a]pyridyl, pyrazolo[1,5-a]pyrimidinyl, imidazo[1,2-b]pyridazinyl, [1,2,4]triazolo[4,3 -b]pyridazinyl, [1,2,4]triazolo[1,5-a]pyrimidyl, [1,2,4]triazolo[1,5-a]pyridyl, and the like.

Unless specified otherwise, the term "pharmaceutically acceptable salt" means those suitable for use in contact with the tissues of mammals, especially humans, without undue toxicity, irritation, allergic response or the like, and commensurate with a reasonable benefit/risk ratio within the range of reasonable medical judgment. For example, the pharmaceutically acceptable salts of amines, carboxylic acids and other types of compounds are well known in the art. The salts can be prepared in situ during the final isolation and purification of the compounds according to the present disclosure, or separately by reacting the free base or acid with a suitable reagent, as outlined below. For example, the functionality of the free base can be reacted with a suitable acid. In addition, when the compound according to the present disclosure bears an acidic moiety, suitable pharmaceutically acceptable salts thereof may include metal salts, such as alkali metal salts (such as sodium or potassium salts); and alkaline earth metal salts (such as calcium or magnesium salts). Examples of pharmaceutically acceptable non-toxic acid addition salts are those formed from amino with inorganic acids (e.g., hydrochloric acid, hydrobromic acid, phosphoric acid, sulfuric acid, and perchloric acid) or organic acids (e.g., acetic acid, oxalic acid, maleic acid, tartaric acid, citric acid, succinic acid or malonic acid), or by using other methods known in the art such as ion exchange. Other pharmaceutically acceptable salts include adipate, sodium alginate, ascorbate, aspartate, besylate, benzoate, bisulfate, borate, butyrate, camphorate, camphorsulfonate, citrate, cyclopentanepropionate, digluconate, lauryl sulfate, ethanesulfonate, formate, fumarate, glucoheptonate, glycerin phosphate, gluconate, hemisulfate, heptanoate, hexanoate, hydroiodide, 2-Hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectate, persulfate, 3-phenylpropionate, phosphate, picrate, pivalate, propionate, stearate, succinate, sulfate, tartrate, thiocyanate, p-toluenesulfonate, undecanoate, pentanoate, and the like. Representative alkali or alkaline earth metal salts include those of sodium, lithium, potassium, calcium, magnesium, and the like. Other pharmaceutically acceptable salts include, where appropriate, nontoxic ammonium salts, quaternary ammonium salts, and amine cations formed with counterions, for example, halides, hydroxides, carboxylates, sulfates, phosphates, nitrates, lower alkylsulfonates and arylsulfonates.

Unless specified otherwise, the term "solvate" refers to a physical association of a compound according to the present disclosure with one or more solvent molecules, regardless of organic or inorganic. This physical association includes hydrogen bonding. In some cases, for example, when one or more solvent molecules are incorporated into the crystal lattice of a crystalline solid, the solvates can be isolated. Solvent molecules in the solvates may exist in regular and/or disordered arrangements. The solvates may contain stoichiometric or non-stoichiometric amounts of solvent molecules. The "solvate" encompasses both solution phase and isolatable solvates. Exemplary solvates include, but are not limited to, hydrates, ethanolates, methanolates, and isopropanolates. Solvation methods are well known in the art.

Unless specified otherwise, the term "isotope labeled analogues" refers to isotope labeled molecules of the compounds of Formulae I and II, thereby providing isotope labeled analogues that may have improved pharmacological activity. Isotopes commonly used as isotopic labels are: hydrogen isotopes: ²H and ³H; carbon isotope: ¹¹C, ¹³C and ¹⁴C; chlorine isotopes: ³⁵Cl and ³⁷Cl; fluorine isotopes: ¹⁸F; iodine isotopes: ¹²³I and ¹²⁵I; nitrogen isotopes: ¹³N and ¹⁵N; oxygen isotopes: ¹⁵O, ¹⁷O and ¹⁸O, and sulfur isotope ³⁵S. These isotope labeled compounds can be used to study the distribution of pharmaceutical molecules in tissues. In particular, deuterium (D or ²H), ³H and carbon ¹³C are more widely used due to their ease of labeling and ease of detection. The substitution with certain heavy isotopes, such as deuterium (²H), can enhance the stability of metabolism, and prolong the half-life, so as to achieve the purpose of reducing dosage and providing therapeutic advantages. Isotope labeled compounds are generally synthesized starting from labeled starting materials in the same way as non-isotope labeled compounds using known synthetic techniques.

Unless specified otherwise, the term "prodrug" refers to a drug that is converted into the parent drug in vivo. The prodrugs are often useful, because they may be more easily administered than the parent drug in some cases. For example, they are bioavailable via oral administration, whereas the parent drug can not. The prodrugs also have improved solubility in pharmaceutical compositions compared to the parent drug. An example of the prodrug may be, but not limited to, any compound of formula I administered as an ester ("prodrug") to facilitate delivery across cell membranes where water solubility is detrimental to mobility but once into the cells the water solubility is beneficial, which is then metabolically hydrolyzed to carboxylic acids, the active entities. Another example of the prodrug may be a short peptide (polyamino acid) bound to an acid group, where the peptide is metabolized to reveal the active moiety.

In the compounds according to the present disclosure, "trans-" means that -G² and -(A)ₘ-B are respectively linked to both sides of X-L²-C-C-L¹ ring plane in the formula(I), and "cis-"means that -G² and -(A)ₘ-B are linked to the same side of X-L²-C-C-L¹ ring plane in the formula(I).

Among the compounds according to the present disclosure, bonds of equal width, 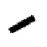 and 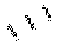 , represent a mixture of two orientations, e.g., 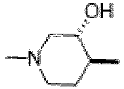 represents 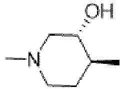 and

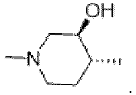

Unless specified otherwise, the term "optionally substituted" means that the hydrogen at the substitutable site of the group is unsubstituted, or substituted by one or more substituents, which are preferably selected from the group consisting of halogen, hydroxy, mercapto, cyano, nitro, amino, azido, oxo, carboxyl, C₂₋₆ alkenyl, C₂₋₆ alkynyl, C₁₋₆ alkyl, C₁₋₆ alkoxy, C₃₋₁₀ cycloalkyl, C₃₋₁₀ cycloalkylsulfonyl, 3-10 membered heterocycloalkyl, C₆₋₁₄ aryl or 5- to 10-membered heteroaryl, wherein the C₂₋₆ alkenyl, C₂₋₆ alkynyl, C₁₋₆ alkyl, C₁₋₆ alkoxy, C₃₋₁₀ cycloalkyl, C₃₋₁₀ cycloalkylsulfonyl, 3-10 membered heterocycloalkyl, C₆₋₁₄ aryl or 5- to 10-membered heteroaryl may be optionally substituted with one or more selected from halogen, hydroxy, amino, cyano, C₁₋₆ alkyl or C₁₋₆ alkoxy, and the oxo group means that two Hs in the same substitutable site are substituted by the single O to form a double bond.

The beneficial effects of the present disclosure are as follows.

The invention designs a class of compounds with novel structures, which provides a new direction for the development of PRMT5 inhibitor drugs. Enzyme inhibitory activity studies in vitro have shown that these compounds have a strong inhibitory effect on PRMT5 enzymes, and can be used as promising compounds for the treatment of diseases mediated by PRMT5 inhibitors. In addition, a specific synthetic method is studied in the present disclosure, which has a simple process and convenient operation, and is beneficial to industrial mass production and application.

### Detailed Description of the Invention

The present disclosure is further described below in connection with specific examples. It should be understood that these examples are intended to illustrate the invention only and are not intended to limit the scope of the invention. Experimental methods for which no specific conditions are indicated in the following examples are generally in accordance with conventional conditions or in accordance with the conditions recommended by the manufacturers. Unless otherwise defined, all professional and scientific terms used herein have the same meaning as those familiar to those skilled in the art. In addition, any methods and materials similar or equivalent to those described herein may be used in the methods of the present disclosure. The preferred embodiments and materials shown herein are illustrative only.

The structures of the compounds according to the present disclosure were determined by nuclear magnetic resonance (NMR) or/and liquid chromatograph mass spectrometry (LC-MS) or/and liquid chromatography (HPLC). The instrument used for NMR was Bruker AVANCE NEO 400 MHz; the instrument used for LC-MS was LCMS WATERS ACQUITY UPLC H-Class PLUS and/or SQD2; the instrument used for HPLC was WATERS ACQUITYUPLC and/or Agilent 1260.

The starting materials in the examples of the present disclosure are known and commercially available, or can be synthesised using or according to methods known in the art.

### Example 1

### Preparation of 4-((6-(cyclobutylamino)pyrimidin-4-yl)oxy)-2-(3,4-dihydroisoquinolin-2(1H)-yl)cyclopentan-1-ol:

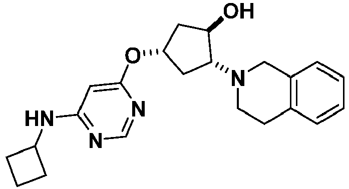

### Step I: preparation of t-butyl (cyclopent-3-en-1-oxy)diphenylsilane:

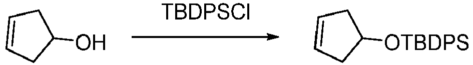

The compounds cyclopent-3-en-1-ol (2.0 g, 23.78 mmol) and imidazole (3.24 g, 45.76 mmol) were dissolved in 12 mL anhydrous DMF (*N,N*-dimethylformamide), followed by addition of TBDPSCl (t-butyl diphenylchlorosilane) (7.19 g, 26.16 mmol) in portions on an ice bath, and then warmed to room temperature (20-25°C). The reaction mixture were stirred for 18 h, and the reaction was completed as indicated by TLC. 20 mL water and 50 mL ethyl acetate were added to the reaction mixture, and the aqueous phase was extracted three times with ethyl acetate (3×30 mL). The organic phases were combined, washed with 50 mL saturated sodium chloride solution solution and dried over anhydrous sodium sulfate, suction filtrated, and concentrated. Purification was conducted by silica gel column chromatography (petroleum ether:ethyl acetate=15:1), and 7.1 g of a colorless oily product was obtained, i.e., t-butyl (cyclopent-3-en-1-oxy)diphenylsilane, yield: 92.5%. ¹H NMR (400 MHz, CDCl₃) δ 7.66 (ddd, J = 13.9, 7.6, 1.5 Hz, 4H), 7.44-7.30 (m, 6H), 5.65-5.56 (m, 2H), 4.55 (tt, J = 6.6, 4.3 Hz, 1H), 2.47-2.33 (m, 4H), 1.06 (d, J = 5.8 Hz, 9H).

### Step II: preparation of ((6-oxabicyclo[3.1.0]hexan-3-yl)oxy)(t-butyl)diphenylsilane:

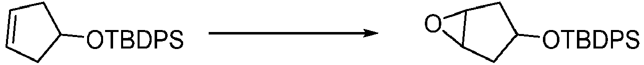

The compound t-butyl (cyclopent-3-en-1-oxy)diphenylsilane (6.5 g, 20.18 mmol) was dissolved in 100 mL dichloromethane, and m-chloroperbenzoic acid (1.9 g, 26.23 mmol, 85%) was added thereto in portions on an ice bath. After completion of the addition of the raw materials, the ice bath was removed, and the reaction was continued at room temperature (20-25°C) for 18 h. TLC indicated the reaction of the raw materials was complete. 20 mL of saturated NaHCO₃ solution was added to the reaction system, which was allowed to stand still before the organic phase was separated. The organic phase was washed three times with saturated NaHCO₃ solution, then dried over anhydrous sodium sulfate, suction filtrated, and concentrated, and concentrated. Purification was conducted by silica gel column chromatography (petroleum ether: ethyl acetate=15:1), and 2.2 g of an oily product was obtained, i.e., the compound ((6-oxabicyclo[3.1.0]hexan-3-yl)oxy)(t-butyl)diphenylsilane, yield: 32.2%. ¹H NMR (400 MHz, CDCl₃) δ 7.69-7.63 (m, 4H), 7.42-7.33 (m, 6H), 4.41 (ddd, J = 6.7, 4.1, 0.7 Hz, 1H), 3.42 (d, J = 8.6 Hz, 2H), 2.09-1.98 (m, 2H), 1.86 (dd, J = 15.2, 7.4 Hz, 2H), 1.06-1.02 (m, 9H).

### Step III: preparation of 4-((t-butyldiphenylsilyl)oxy)-2-(3,4-dihydroisoquinolin-2(1H)-yl)cyclopentan-1-ol:

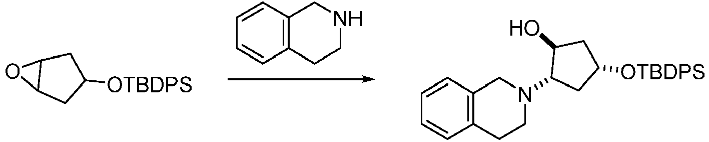

The compound ((6-oxabicyclo[3.1.0]hexan-3-yl)oxy)(t-butyl)diphenylsilane (86 mg, 0.254 mmol) was dissolved in 1.27 mL ethanol, and triethylamine (128 mg, 1.28 mmol) and 1,2,3,4-tetrahydroisoquinoline (40.5 mg, 0.3048 mmol) were added. The reaction system was refluxed for 48 h, and the reaction was stopped and cooled to room temperature. Then 5 mL water and 20 mL dichloromethane were added to the reaction system, which was allowed to stand still before the organic phase was separated. The organic phase was washed with saturated sodium chloride solution, then dried over anhydrous sodium sulfate, suction filtrated, and concentrated. Purification was conducted by silica gel column chromatography (petroleum ether: ethyl acetate=10:1), and 55 mg product 4-((5-butyldiphenylsilyl)oxy)-2-(3,4-dihydroisoquinolin-2(1H)-yl)cyclopentan-1-ol was obtained, yield: 46.2%. LC-MS: 472.38 [M+H]⁺; ¹H NMR (400 MHz, CDCl₃) δ 7.7-7.60 (m, 4H), 7.48-7.35 (m, 6H), 7.16-7.06 (m, 3H), 7.05-6.99 (m, 1H), 4.40 (s, 1H), 4.23 (s, 1H), 3.16 (s, 1H), 2.91 (d, J = 5.1 Hz, 3H), 2.66 (d, J = 63.2 Hz, 2H), 2.15-1.99 (m, 2H), 1.93-1.82 (m, 2H), 1.58 (d, J = 7.9 Hz, 2H), 1.26 (ddd, J = 9.6, 6.5, 2.7 Hz, 1H), 1.07 (d, J = 6.7 Hz, 9H).

### Step IV: preparation of 4-((t-butyldiphenylsilyl)oxy)-2-(3,4-dihydroisoquinolin-2(1H)-yl)cyclopentyl acetate:

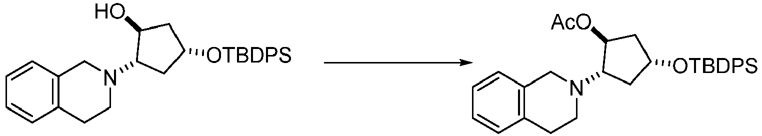

Under the conditions of an ice bath and nitrogen protection, sodium hydride (17 mg, 0.424 mmol) was dissolved in 1 mL DMF (*N,N*-dimethylformamide), and then the compound 4-((t-butyldiphenylsilyl)oxy)-2-(3,4-dihydroisoquinolin-2(1H)-yl)cyclopentan-1-ol (100 mg, 0.212 mmol) was added. After half an hour, acetylchloride (17 mg, 0.212 mmol) was added. After two hours, TLC indicated the reaction of the raw materials was complete, and 1 mL of saturated ammonium chloride solution was added to the system to quench the reaction. The reaction mixture was extracted with ethyl acetate, the organic phase was washed with saturated sodium chloride solution, then dried over anhydrous sodium sulfate, suction filtrated, and concentrated. Purification was conducted by silica gel column chromatography (petroleum ether: ethyl acetate=2:1), and 88 mg product 4-((t-butyldiphenylsilyl)oxy)-2-(3,4-dihydroisoquinolin-2(1H)-yl)cyclopentyl acetate was obtained, yield: 81.4%. LC-MS: 514.45 [M+H]⁺.

### Step V: preparation of 2-(3,4-dihydroisoquinolin-2(1H)-yl)-4-hydroxylcyclopentyl acetate:

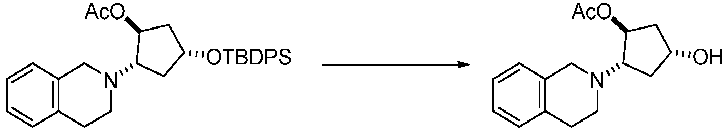

The compound 4-((t-butyldiphenylsilyl)oxy)-2-(3,4-dihydroisoquinolin-2(1H)-yl)-cyclopentyl acetate (315 mg, 0.613 mmol) was dissolved in 3 mL THF (tetrahydrofuran), to which 0.674 mL of 1 mol/L tetrabutylammonium fluoride in THF was added, and stirred at room temperature overnight. 5 mL water and 20 mL dichloromethane were added to the reaction system, which was allowed to stand still before the organic phase was separated. The organic phase was washed with saturated sodium chloride solution, then dried over anhydrous sodium sulfate, suction filtrated, and concentrated. Purification was conducted by column chromatography (dichloromethane:methanol=20:1), and 90 mg product 2-(3,4-dihydroisoquinolin-2(1*H*)-yl)-4-hydroxylcyclopentyl acetate was obtained, yield: 54.5%. LC-MS: 276.24 [M+H]⁺; ¹H NMR (400 MHz, CDCl₃) δ 7.31-7.21 (m, 2H), 7.18 (d, J = 7.4 Hz, 1H), 7.07 (d, J = 7.4 Hz, 1H), 5.59 (ddd, J = 8.7, 5.4, 3.3 Hz, 1H), 4.90-4.46 (m, 1H), 4.37 (s, 2H), 4.10-4.00 (m, 1H), 3.91-3.32 (m, 2H), 3.16 (s, 2H), 2.60-2.49 (m, 1H), 2.38-2.26 (m, 1H), 2.27-2.14 (m, 1H), 2.11-1.98 (m, 3H), 1.75 (d, J = 15.1 Hz, 1H).

### Step VI: preparation of 4-((6-((t-butoxycarbonyl)(cyclobutyl)amino)pyrimidin-4-yl)oxy)-2-(3,4-dihydroisoquinolin-2(1H)-yl)cyclopentyl acetate:

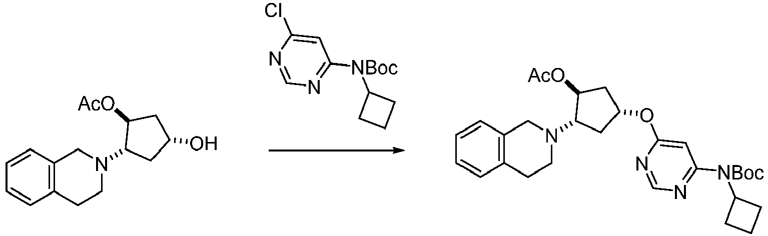

Under the conditions of an ice bath and nitrogen protection, tcompound 6 (50 mg, 0.181 mmol) and compound 7 (51mg, 0.181 mmol) were dissolved in 0.9 mL anhydrous tetrahydrofuran, and 0.18 mL of 1 mol/L potassium t-butoxide in tetrahydrofuran was further added. After two hours, 1 mL of saturated ammonium chloride was added dropwise, and 5 mL water and 20 mL ethyl acetate were added into the reaction system, which was allowed to stand still before the organic phase was separated. The organic phase was washed with saturated sodium chloride solution, then dried over anhydrous sodium sulfate, suction filtrated, and concentrated. Purification was conducted by column chromatography (dichloromethane:methanol=20:1), and 32 mg product 4-((6-((t-butoxycarbonyl)(cyclobutyl)amino)pyrimidin-4-yl)oxy)-2-(3,4-dihydroisoquinolin-2 (1H)-yl)cyclopentyl acetate was obtained, yield: 54.5%. LC-MS: 523.42 [M+H]⁺.

### Step VII: preparation of 4-((6-((cyclobutylamino))pyrimidin-4-yl)oxy)-2-(3,4-dihydroisoquinolin-2(1H)-yl)cyclopentyl acetate:

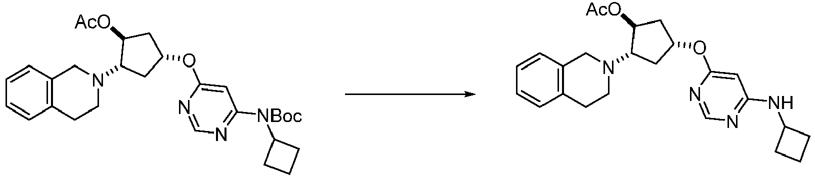

The compound 4-((6-((t-butoxycarbonyl)(cyclobutyl)amino)pyrimidin-4-yl)oxy)-2-(3,4-dihydroisoquinolin-2(1H)-yl)cyclopentyl acetate (90 mg, 0.212 mmol) was dissolved in 1 mL dichloromethane, and 0.25 mL trifluoroacetic acid was added. After two hours, the reaction of the raw materials was complete as indicated by TLC, 5 mL 1,2-dichloroethane was added, and the solvent was rotary evaporated. The crude product was directly used for the next reaction step.

### Step VIII: preparation of 4-((6-((cyclobutylamino))pyrimidin-4-yl)oxy)-2-(3,4-dihydroisoquinolin-2(1H)-yl)cyclopentan-1-ol:

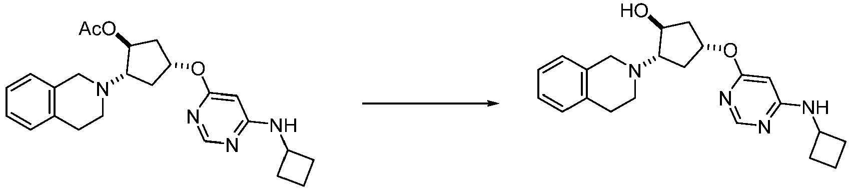

The crude product of the compound 4-((6-((cyclobutylamino))pyrimidin-4-yl)oxy)-2-(3,4-dihydroisoquinolin-2(1H)-yl)cyclopentyl acetate (70 mg, 0.165 mmol) was dissolved in 1 mL methanol, and a potassium carbonate solution (57 mg, 0.414 mmol, in 1.5 mL water) was added. After two hours, 5 mL water and 20 mL ethyl acetate were added to the reaction system, which was allowed to stand still before the organic phase was separated. The organic phase was washed with saturated sodium chloride solution, then dried over anhydrous sodium sulfate, suction filtrated, and concentrated. The product was subjected to preparative purification under medium pressure. LC-MS: 381.38 [M+H]⁺; ¹H NMR (400 MHz, CDCl₃) δ 10.21 (s, 1H), 8.16 (d, J = 20.7 Hz, 1H), 7.36-7.29 (m, 1H), 7.21 (d, J = 7.3 Hz, 1H), 7.11 (d, J = 7.1 Hz, 1H), 5.71 (s, 1H), 5.48 (s, 1H), 4.94 (s, 1H), 4.56 (d, J = 14.1 Hz, 2H), 3.93-3.59 (m, 4H), 3.26 (s, 3H), 2.62 (d, J = 75.0 Hz, 2H), 2.45-2.33 (m, 3H), 2.16 (d, J = 8.2 Hz, 2H), 2.05-1.93 (m, 2H), 1.91-1.73 (m, 2H).

### Example 2

### Preparation of trans-1-(4-((6-(3-(3,4-dihydroisoquinolin-2(1H)-yl)-4-hydroxypyrrolidine-1-carbonyl)pyrimidin-4-yl)amino)piperidin-1-yl)ethan-1-one:

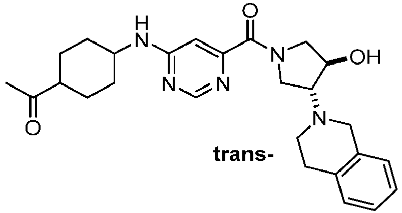

### Step I: t-butyl trans-3-(3,4-dihydroisoquinolin-2(1H)-yl)-4-hydroxypyrrolidine-1-carboxylate:

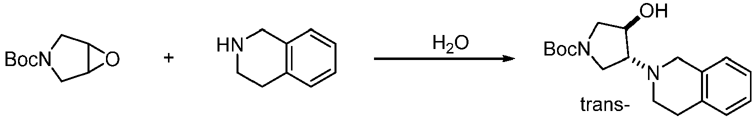

The compound 3-Boc-6-oxa-3-azabicyclo[3.1.0]hexane (1.50 g, 8.10 mmol, 1.0 eq) was dissolved in 40 mL water, then 1,2,3,4-tetrahydroisoquinoline (1.62 g, 12.15 mmol, 1.5 eq) was added thereto, and the reaction was carried out at 20°C for 16 h. After the reaction was complete as indicated by TLC, the reaction mixture was extracted with ethyl acetate, and dried over anhydrous sodium sulfate. The organic phase was concentrated and purified by column chromatography to obtain 1.20 g t-butyl (±)-3-(3,4-dihydroisoquinolin-2(1*H*)-yl)-4-hydroxypyrrolidine-1-carboxylate, yield: 46.5%; LCMS (ESI) [M+H]⁺ = 319.11.

### Step II: preparation of trans-4-(3,4-dihydroisoquinolin-2(1H)-yl)pyrrolidin-3-ol:

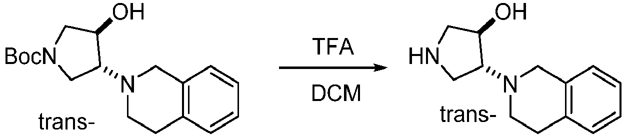

The compound t-butyl (±)-3-(3,4-dihydroisoquinolin-2(1*H*)-yl)-4-hydroxypyrrolidine-1-carboxylate (400 mg, 1.26 mmol, 1.0 eq) was dissolved in DCM (dichloromethane) (20 mL), followed by addtion of TFA (trifluoroacetic acid) (5 mL), and a reaction was carried out at 20°C overnight. After the reaction was complete as indicated by TLC, the reaction mixture was concentrated to obtain (±)-4-(3,4-dihydroisoquinolin-2(1*H*)-yl)pyrrolidin-3-ol crude product; LCMS (ESI) [M+H]⁺ = 219.06.

### Step III: preparation of trans-1-(4-((6-(3-(3,4-dihydroisoquinolin-2(1H)-yl)-4-hydroxypyrrolidine-1 -carbonyl)pyrimidin-4-yl)amino)piperidin-1 -yl)ethan-1 -one:

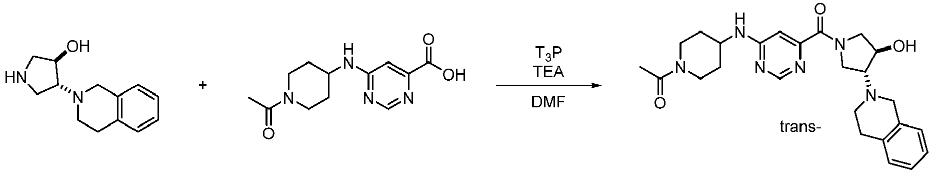

The compound (±)-4-(3,4-dihydroisoquinolin-2(1*H*)-yl)pyrrolidin-3-ol (274 mg, 1.26mmol, 1.0 eq) was dissolved in DMF (*N,N*-dimethylformamide) (6 mL), then the compound 6-((1-acetylpiperidin-4-yl)amino)pyrimidine-4-carboxylic acid (332 mg, 1.26 mmol, 1.0 eq), T₃P (1-propylphosphoric anhydride) (50wt% in ethyl acetate, 1.60 g, 2.51 mmol, 2.0 eq) and TEA (triethylamine) (0.87 mL, 6.28 mmol, 5.0 eq) were added, and the reaction was carried out at 20°C for 1 h. After the reaction was complete as indicated by TLC, the reaction mixture was concentrated and purified by preparative HPLC to obtain 150 mg of the target compound, yield: 25.7%. LCMS (ESI) [M+H]⁺ = 465.20; ¹H NMR (400 MHz, Chloroform-*d₃*) δ 8.52 (d, *J* = 8.6 Hz, 1H), 7.09 (dtt, *J* = 9.1, 6.2, 3.0 Hz, 3H), 6.99 (dt, *J* = 6.7, 3.3 Hz, 1H), 6.84 (d, *J* = 17.6 Hz, 1H), 6.30 (d, *J* = 145.5 Hz, 1H), 4.47 (ddq, *J* = 16.7, 11.3, 5.8, 4.5 Hz, 2H), 4.26 - 4.04 (m, 2H), 3.98 (td, *J* = 12.8, 7.0 Hz, 1H), 3.78 (dddd, *J* = 53.3, 20.7, 12.8, 7.2 Hz, 5H), 3.53 (dd, *J* = 12.8, 5.6 Hz, 1H), 3.21 (tq, *J* = 14.4, 4.0 Hz, 1H), 3.08 (dq, *J* = 12.9, 6.7 Hz, 1H), 2.94 (dt, *J* = 8.4, 4.1 Hz, 1H), 2.82 (ddt, *J* = 25.1, 11.0, 5.7 Hz, 4H), 2.09 (d, *J* = 2.2 Hz, 3H), 2.02 - 1.95 (m, 2H), 1.56 - 1.36 (m, 2H).

### Example 3

### Preparation of 6-((cyclobutylamino))-N-((3-(3,4-dihydroisoquinolin-2(1H)-yl)-4-hydroxycyclopentyl)pyrimidine-4-carboxamide:

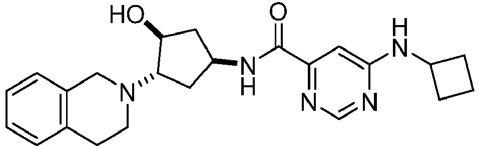

### Step I: preparation of 2-(3,4-dihydroisoquinolin-2(1H)-yl)-4-(1,3-dioxoisoindolin-2-yl)cyclopentyl acetate:

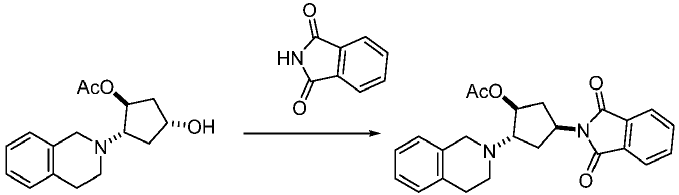

The compound 2-(3,4-dihydroisoquinolin-2(*1H*)-yl)-4-hydroxycyclopentyl acetate (50 mg, 0.182 mmol, 1.0 eq), phthalimide (26.80 mg, 0.182 mmol, 1.0 eq) and triphenylphosphine (71.50 mg, 0.273 mmol, 1.5 eq) were dissolved in anhydrous tetrahydrofuran (1 mL), and the reaction mixture was stirred under the conditions of an ice bath and nitrogen protection for 0.5 h. Then, diethyl azodicarboxylate (47.5 mg, 0.273 mmol, 1.5 eq) was added to the reaction mixture, the ice bath was removed, and the reaction mixture was further stirred for 2 h. The reaction of the raw materials was complete as indicated by TLC, water (10 mL) and ethyl acetate (30 mL) were added to the reaction mixture, and the aqueous phase was extracted three times with ethyl acetate (3×30 mL). The organic phases were combined, washed with saturated sodium chloride solution (50 mL) and dried over anhydrous sodium sulfate, suction filtrated, and concentrated. Purification was conducted by column chromatography (silica gel, dichloromethane: methanol = 100: 0 to 96 : 4), to obtain 65 mg 2-(3,4-dihydroisoquinolin-2(*1H*)-yl)-4-(1,3-dioxoisoindolin- 2-yl)cyclopentyl acetate as a white solid, yield: 89.2%. ¹H NMR (400 MHz, CDCl₃) δ 7.87-7.80 (m, 2H), 7.75-7.68 (m, 2H), 7.15-7.07 (m, 3H), 7.06-6.98 (m, 1H), 5.47 (ddd, J = 7.7, 4.4, 3.0 Hz, 1H), 4.81 (tt, J = 10.9, 7.7 Hz, 1H), 3.88-3.75 (m, 2H), 3.29-3.17 (m, 1H), 3.02-2.94 (m, 1H), 2.91 (dd, J = 11.3, 5.9 Hz, 2H), 2.88-2.83 (m, 1H), 2.83-2.78 (m, 1H), 2.56 (dd, J = 22.8, 11.5 Hz, 1H), 2.30-2.18 (m, 1H), 2.09 (s, 3H), 1.92 (dd, J = 14.0, 7.9 Hz, 1H); LC-MS (ESI) [M+H]⁺ =405.27.38.

### Step II: preparation of 4-amino-2-(3,4-dihydroisoquinolin-2(1H)-yl)cyclopentan-1-ol:

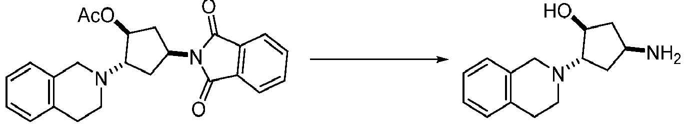

2-(3,4-dihydroisoquinolin-2(*1H*)-yl)-4-(1,3-dioxoisoindolin-2-yl)cyclopentyl acetate (160 mg, 0.398 mmol, 1.0 eq) was dissolved in ethanol (2 mL), and hydrazine hydrate (200 mg, 4 mmol, 10 eq) was added. The temperature was raised to 85 °C, and the reaction mixture was stirred for half an hour to allow solid to precipitate. The reaction mixture was cooled to room temperature, filtered to obtain a filtrate, and then concentrated. Seperation and purification were conducted by preparative HPLC (C18, 10 mmol/L NH₄HCO₃ (aqueous), acetonitrile). 50 mg 4-amino-2-(3,4-dihydroisoquinolin-2(1*H*)-yl)cyclopentan-1-ol was obtained as a white solid, yield: 54.3%. LC-MS (ESI) [M+H]⁺= 232.28.

### Step III: preparation of 6-((cyclobutylamino))-N-((3-(3,4-dihydroisoquinolin-2(1H)-yl)-4-hydroxycyclopentyl)pyrimidine-4-carboxamide:

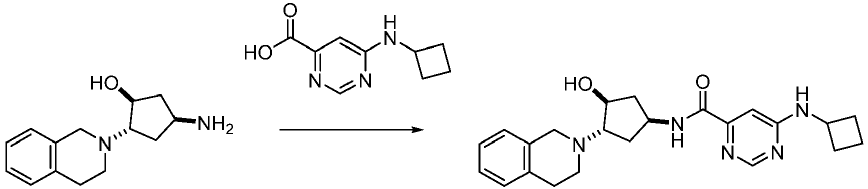

4-amino-2-(3,4-dihydroisoquinolin-2(*1H*)-yl)cyclopentan-1-ol (20 mg, 0.086 mmol, 1.0 eq), 6-((cyclobutylamino))pyrimidine-4-carboxylic acid (16.6 mg, 0.086 mmol, 1.0 eq) and HATU (2-(7-azabenzotriazole)-*N,N,N',N'*-tetramethylurea hexafluorophosphate) (47 mg, 0.1245 mmol, 1.5 eq) were dissolved in *N,N-*dimethylformamide (0.4 mL), and *N,N-*diisopropylethylamine (33.0 mg, 0.258 mmol, 3.0 eq) was added, followed by stirring for 2 h under nitrogen protection. Water (10 mL) and ethyl acetate (20 mL) were added to the reaction system, and the aqueous phase was extracted three times with ethyl acetate (3×20 mL). The organic phases were combined, washed with saturated sodium chloride solution (50 mL), dried over anhydrous sodium sulfate, suction filtrated, and concentrated. Seperation and purification was conducted by preparative HPLC (C18, 10 mmol/L aqueous NH₄HCO₃, acetonitrile) to obtain 4.4 mg 6-(cyclobutyl-amino)-*N*-((3-(3,4-dihydroisoquinolin-2(1*H*)-yl)-4-hydroxycyclopentyl)pyrimidine-4-carboxa mide, yield: 12.5%. LC-MS (ESI) [M+H]⁺=408.20; ¹H NMR (400 MHz, CDCl₃) δ 8.40 (s, 1H), 8.09 (d, J = 8.1 Hz, 1H), 7.14-7.09 (m, 3H), 7.07 (s, 1H), 7.02 (d, J = 5.5 Hz, 1H), 5.43 (s, 1H), 4.61 (dd, J = 15.3, 7.9 Hz, 1H), 4.43 (d, J = 3.3 Hz, 1H), 3.79 (s, 2H), 2.93 (s, 2H), 2.91 (d, J = 4.0 Hz, 1H), 2.83 (dd, J = 14.7, 5.8 Hz, 2H), 2.48 (dd, J = 12.1, 6.4 Hz, 3H), 2.19-2.11 (m, 1H), 2.03 (dt, J = 7.5, 5.5 Hz, 2H), 1.96-1.90 (m, 2H), 1.84 (d, J = 7.2 Hz, 2H), 1.68 (dd, J = 21.6, 9.2 Hz, 2H).

### Example 4

### Preparation of 6-(((1-acetylpiperidin-4-yl)amino)-N-(3-(3,4-dihydroisoquinolin-2(1H)-yl)-4-hydroxycyclopentyl)pyrimidine-4-carboxamide:

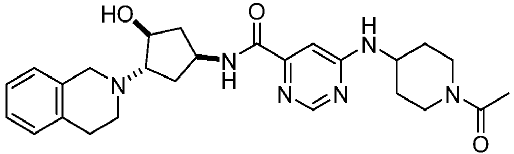

### Step I: preparation of 6-(((1-acetylpiperidin-4-yl)amino)-N-(3-(3,4-dihydro-isoquinolin-2(1H)-yl)-4-hydroxycyclopentyl)pyrimidine-4-carboxamide:

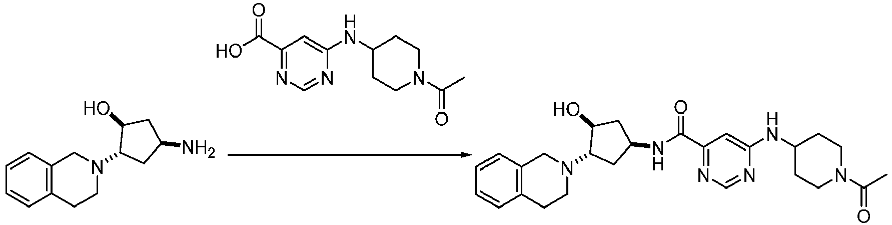

4-amino-2-(3,4-dihydroisoquinolin-2(*1H*)-yl)cyclopentan-1-ol (38 mg, 0.164 mmol, 1.0 eq), 6-((1-acetylpiperidin-4-yl)amino)pyrimidine-4-carboxylic acid (43.2 mg, 0.164 mmol, 1.0 eq) and HATU (2-(7-azabenzotriazole)-*N,N,N',N'*-tetramethyluronium hexafluorophosphate) (94 mg, 0.246 mmol, 1.5 eq) were dissolved in N,N-dimethylformamide (0.8 mL), and *N,N*-diisopropylethylamine (64 mg, 0.492 mmol, 3.0 eq) was added, followed by stirring for 2 h under nitrogen protection. Water (10 mL) and ethyl acetate (20 mL) were added to the reaction system, and the aqueous phase was extracted three times with ethyl acetate (3×20 mL). The organic phases were combined, washed with saturated sodium chloride solution (50 mL), dried over anhydrous sodium sulfate, suction filtrated, and concentrated. Seperation and purification was conducted by preparative HPLC (C18, 10 mmol/L aqueous NH₄HCO₃, acetonitrile) to obtain 6 mg 6-(((1-acetylpiperidin-4-yl)amino)-*N*-(3-(3,4-dihydroisoquinolin-2(*1H*)-yl)-4-hydroxycyclope ntyl)pyrimidine-4-carboxamide, yield: 7.6%. LC-MS (ESI) [M+H]⁺=479.36; ¹H NMR (400 MHz, CDCl₃) δ 8.46 (s, 1H), 8.17 (d, J = 8.4 Hz, 1H), 7.24-7.18 (m, 2H), 7.18-7.11 (m, 3H), 7.04 (d, J = 6.6 Hz, 1H), 4.65-4.54 (m, 3H), 4.12 (d, J = 7.1 Hz, 1H), 3.96 (s, 1H), 3.83 (d, J = 13.9 Hz, 1H), 3.22 (d, J = 11.8 Hz, 1H), 3.05 (s, 2H), 2.79 (d, J = 10.6 Hz, 1H), 2.55-2.47 (m, 1H), 2.14 (s, 2H), 2.11 (s, 3H), 1.86 (s, 1H), 1.57 (s, 4H), 1.46 (s, 2H), 1.41 (d, J = 8.0 Hz, 2H).

### Example 5

### Preparation of N-(3-(3,4-dihydroisoquinolin-2(1H)-yl)-4-hydroxycyclopentyl)-6-(oxetan-3-ylamino)pyrimidine-4-carboxamide:

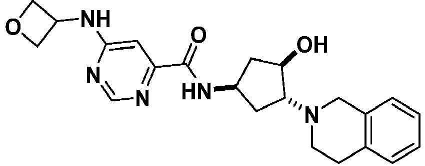

### Step I: preparation of N-(3-(3,4-dihydroisoquinolin-2(1H)-yl)-4-hydroxycyclopentyl)-6-(oxetan-3-ylamino)pyrimidine-4-carboxamide:

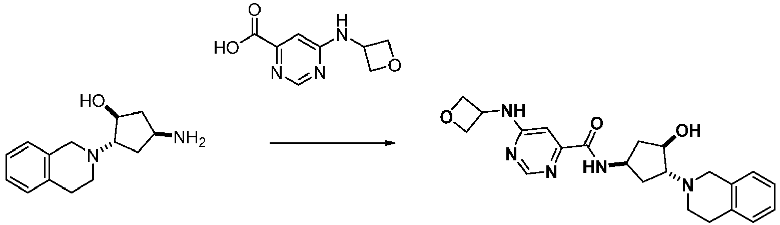

The compounds 4-amino-2-(3,4-dihydroisoquinolin-2(*1H*)-yl)cyclopentan-1-ol (5 mg, 0.0216 mmol, 1.0eq), 6-(cyclobutylamino)pyrimidine-4-carboxylic acid (4.2 mg, 0.0216 mmol, 1.0eq) and 2-(7-azabenzotriazole)-*N,N,N',N'*-tetramethyluronium hexafluorophosphate (12.3 mg, 0.0324 mmol, 1.5eq) were dissolved in 0.1 mL DMF, and N,N-diisopropylethylamine (8.4 mg, 0.0648 mmol, 3.0eq) was added thereto, followed by stirring for 2 h under nitrogen. 2 mL water and 10 mL ethyl acetate were added to the reaction system, and the aqueous phase was extracted three times with ethyl acetate (3×10 mL). The organic phases were combined, washed with 20 mL saturated sodium chloride solution solution, dried over anhydrous sodium sulfate, suction filtrated, and concentrated. Purification was conducted by preparative HPLC to obtain 1.2 mg *N*-(3-(3,4-dihydroisoquinolin-2(1*H*)-yl)-4-hydroxycyclopentyl)-6-(oxetan-3-ylamino)-pyrimi dine-4-carboxamide, yield: 13.6%. ¹H NMR (400 MHz, CD₃OD) δ 8.41 (d, J = 1.1 Hz, 1H), 7.15-7.08 (m, 4H), 7.05 (d, J = 7.8 Hz, 1H), 5.10 (s, 1H), 4.95 (t, J = 6.9 Hz, 2H), 4.60 (t, J = 6.4 Hz, 2H), 4.53 (dd, J = 15.9, 8.0 Hz, 1H), 4.32 (dd, J = 10.0, 5.3 Hz, 1H), 3.84-3.74 (m, 2H), 3.08-3.00 (m, 1H), 2.94 (t, J = 5.8 Hz, 2H), 2.82 (dd, J = 11.8, 5.8 Hz, 1H), 2.79-2.73 (m, 1H), 2.48 (dt, J = 13.2, 6.8 Hz, 1H), 2.05 (dd, J = 8.2, 5.7 Hz, 2H), 1.68 (dt, J = 12.4, 9.5 Hz, 1H); LC-MS: 410.16 [M+H]⁺.

### Example 6

### Preparation of trans-(3-(3,4-dihydroisoquinolin-2(1H)-yl)-4-hydroxypyrrolidin-1-yl)(6-(oxetan-3-ylamino)pyrimidin-4-yl)ketone:

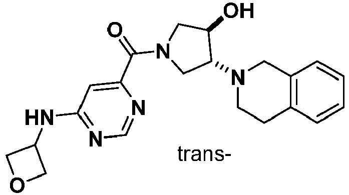

### Step I: preparation of trans-(3-(3,4-dihydroisoquinolin-2(1H)-yl)-4-hydroxy-pyrrolidin-1-yl)(6-(oxetan-3-ylamino)pyrimidin-4-yl)ketone:

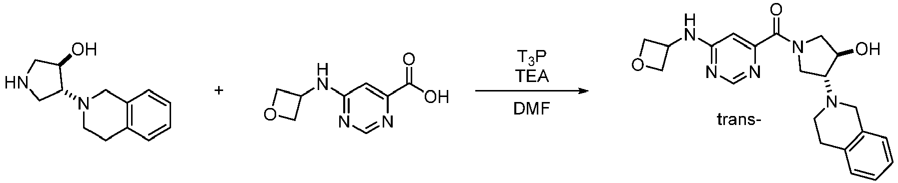

The compound trans-4-(3,4-dihydroisoquinolin-2(1*H*)-yl)pyrrolidin-3-ol (206 mg, 0.94 mmol, 1.0 eq) was dissolved in DMF (N,N-dimethylformamide) (5 mL), then the compounds 6-(oxetan-3-ylamino)pyrimidine-4-carboxylic acid (184 mg, 0.94 mmol, 1.0 eq), T₃P (1-propylphosphoric anhydride) (50wt% in ethyl acetate, 1.20 g, 1.88 mmol, 2.0 eq) and TEA (triethylamine) (0.66 mL, 4.71 mmol, 5.0 eq) were added thereto, and the reaction was carried out at 20°C for 1 h. After the reaction was complete as indicated by TLC, the reaction mixture was concentrated and subjected to purification by preparative HPLC to obtain 36 mg of the target compound, yield: 9.7%. LCMS (ESI) [M+H]⁺ = 396.13; ¹H NMR (400 MHz, CDCl₃) δ 8.53 (dd, *J =* 4.6, 1.1 Hz, 1H), 7.21 - 7.09 (m, 3H), 7.06 - 6.99 (m, 1H), 6.92 (d, *J* = 14.3 Hz, 1H), 6.41 (d, *J =* 50.9 Hz, 1H), 5.08 (s, 1H), 4.99 (t, *J* = 6.8 Hz, 2H), 4.64 (dd, *J* = 6.5, 3.0 Hz, 1H), 4.57 (td, *J* = 6.4, 2.2 Hz, 2H), 4.23 (ddd, *J* = 18.8, 12.2, 7.1 Hz, 1H), 4.14 - 3.90 (m, 4H), 3.78 (ddd, *J* = 18.4, 12.5, 7.0 Hz, 1H), 3.56 (dd, *J =* 13.0, 6.0 Hz, 1H), 3.30 (q, *J =* 7.1 Hz, 1H), 3.13 (dp, *J =* 17.5, 5.8 Hz, 2H), 2.99 (dq, *J =* 14.6, 6.9, 6.5 Hz, 2H).

### Example 7

### Preparation of cis-1-(4-((6-(3-(3,4-dihydroisoquinolin-2(1H)-yl)-4-hydroxypyrrolidin-1-carbonyl)pyrimidin-4-yl)amino)piperidin-1-yl)ethan-1-one:

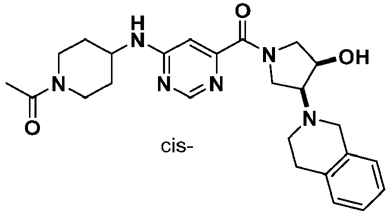

### Step I: preparation of t-butyl trans-3-(3,4-dihydroisoquinolin-2(1H)-yl)-4-oxopyrrolidine-1-carboxylate:

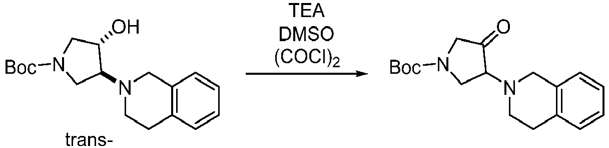

(COCl)₂ (oxalyl chloride) (951 mg, 7.56 mmol, 1.2 eq) was dissolved in dichloromethane (10 mL). Under nitrogen protection, at -78°C, DMSO (dimethylsulfoxide) (540 mg, 6.93 mmol, 1.1 eq) was dissolved in dichloromethane (10 mL), and then slowly added dropwise to the above solution. The reaction mixture was allowed to react at -78°C for 25 min. The compound t-butyl trans-3-(3,4-dihydroisoquinolin-2(1*H*)-yl)-4-hydroxypyrrolidine-1-carboxylate (2 g, 6.3 mmol, 1.0 eq) was dissolved in dichloromethane (10 ml), and then slowly added dropwise to the reaction mixture. The reaction mixture was allowed to react at -78°C for 25 mins, and TEA (triethylamine) (3.2 g, 31.5 mmol, 5 eq) was then added under nitrogen protection. The reaction mixture was allowed to react at -78°C for 2 h. After the reaction was complete as indicated by TLC and LCMS, the reaction mixture was naturally warmed to 0°C, and quenched by addition of saturated ammonium chloride solution (10 mL). The reaction mixture was naturally warmed to room temperature, poured into 20 mL water, and extracted three times with dichloromethane (20 mL each). The organic phases were combined, washed once with 15 mL saturated sodium chloride solution, dried over anhydrous sodium sulfate, and filtrated and concentrated. After seperation by column chromatography, t-butyl 3-(3,4-dihydroisoquinolin-2(1*H*)-yl)-4-oxopyrrolidine-1-carboxylate (720 mg, yield: 36%) was obtained. LCMS(ESI)[M+1]⁺ = 317.12.

### Step II: preparation of t-butyl cis-3-(3,4-dihydroisoquinolin-2(1H)-yl)-4-hydroxypyrrolidine-1 -carboxylate

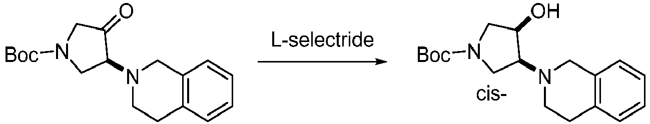

Tert-butyl 3-(3,4-dihydroisoquinolin-2(1*H*)-yl)-oxopyrrolidine-1-carboxylate (700 mg, 2.21 mmol, 1.0 eq) was dissolved in tetrahydrofuran (11 ml), and L-selectride (lithium tri-sec-butylborohydride) (3.31 mL, 3.31 mmol, 1.5 eq) was slowly added dropwise under nitrogen protection at -78°C. The reaction was conducted under stirring at -78°C for 3 h. After the reaction was complete as indicated by TLC and LCMS, the reaction was quenched by slowly adding saturated ammonium chloride solution (10 mL) at -78°C. The reaction mixture was naturally warmed to room temperature, to which 20 mL water was added, and extracted three times with ethyl acetate (20 mL each). The ethyl acetate phases were combined, washed once with 10 mL saturated sodium chloride solution, dried over anhydrous sodium sulfate, filtrated and concentrated. After seperation by column chromatography, 410 mg t-butyl cis-3-(3,4-dihydroisoquinolin-2(1*H*)-yl)-4-hydroxypyrrolidine-1-carboxylate (410 mg, yield: 58%) was obtained. LCMS(ESI)[M+1]⁺ = 319.11.

### Step III: preparation of cis-4-(3,4-dihydroisoquinolin-2(1H)-yl)pyrrolidin-3-ol

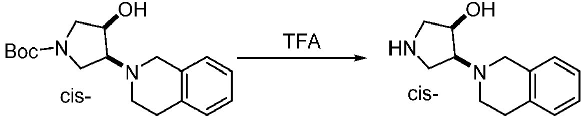

Tert-butyl cis-3-(3,4-dihydroisoquinolin-2(1*H*)-yl)-4-hydroxypyrrolidine-1-carboxylate (400 mg, 1.26 mmol, 1.0 eq) was dissolved in dichloromethane (6 mL), and TFA (trifluoroacetic acid) (1.5 mL) was added to the reaction mixture under nitrogen protection. The reaction was conducted under stirring at room temperature for 2 h. After the reaction was complete as indicated by TLC and LCMS, the reaction mixture was diluted with dichloromethane, and then adjusted to around pH=8 by adding saturated ammonium bicarbonate solution. 10 mL water was added to the reaction mixture, which was extracted three times with dichloromethane (15 mL each). The dichloromethane phases were combined, washed once with 10 mL saturated sodium chloride solution, dried over anhydrous sodium sulfate, filtrated and concentrated to obtain a yellow oily product cis-4-(3,4-dihydroisoquinolin-2(1*H*)-yl)pyrrolidin-3-ol (182 mg, yield: 66%). LCMS(ESI)[M+1]⁺ = 219.02.

### Step IV: preparation of cis-1-(4-((6-(3-(3,4-dihydroisoquinolin-2(1H)-yl)-4-hydroxypyrrolidine-1-carbonyl)pyrimidin-4-yl)amino)piperidin-1-ylethanone:

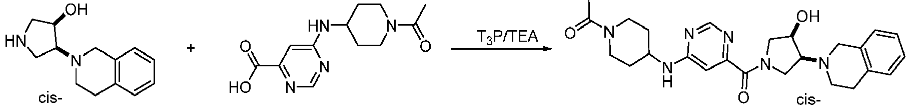

Cis-4-(3,4-dihydroisoquinolin-2(1*H*)-yl)pyrrolidin-3-ol (90 mg, 0.41 mmol, 1.0 eq) was dissolved in *N,N*-dimethylformamide (2 mL), and 6-((1-acetylpiperidin-4-yl)amino)pyrimidine- 4-carboxylic acid (87 mg, 0.33 mmol, 0.8 eq), TEA (triethylamine) (207 mg, 2.05 mmol, 5.0 eq) and T₃P (1-propylphosphoric anhydride) (50wt% in ethyl acetate, 1.044 g, 0.82 mmol, 2.0 eq) were added to the reaction mixture under nitrogen protection. The reaction was carried out under stirring at room temperature (20-25°C) for 3 h. After the reaction was complete as indicated by TLC and LCMS, 10 mL water was added to the reaction mixture, which was extracted three times with ethyl acetate (20 mL for each). The ethyl acetate phases were combined, washed once with 10 mL saturated sodium chloride solution, dried over anhydrous sodium sulfate, filtrated and concentrated, and then purified by preparative HPLC (C18, 10 mmol/L aqueous NH₄HCO₃, acetonitrile) to obtain cis-1-(4-((6-(3-(3,4-dihydroisoquinolin-2(1*H*)-yl)-4-hydroxypyrrolidine-1-carbonyl)pyrimidi n-4-yl)amino)piperidin-1-ylethanone (2.5 mg, yield: 1.3%), LCMS(ESI)[M+1]⁺ = 465.24; ¹H NMR (400 MHz, CDCl₃) δ 8.57 (m, 1H), 7.22 - 7.08 (m, 3H), 7.07 - 6.99 (m, 1H), 6.86 (d, *J* = 11.2 Hz, 1H), 5.32 (m, 1H), 4.55 (d, *J* = 13.2 Hz, 1H), 4.46 - 4.27 (m, 1H), 4.15 - 3.54 (m, 8H), 3.23 (t, *J* = 12.7 Hz, 1H), 3.10 - 2.89 (m, 4H), 2.89 - 2.70 (m, 2H), 2.18 - 2.09 (m, 4H), 2.05 (d, *J* = 12.5 Hz, 1H), 1.51 - 1.33 (m, 2H).

### Example 8

### Preparation of N-(3-(3,4-dihydroisoquinolin-2(1H)-yl)-4-hydroxycyclopentyl)-6-(oxetan-3-ylamino)pyrimidine-4-carboxamide :

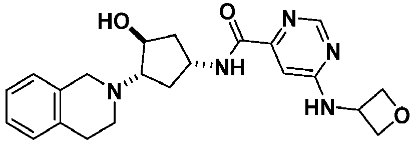

### Step I: preparation of t-butyl cyclopent-3-en-1-ylcarbamate:

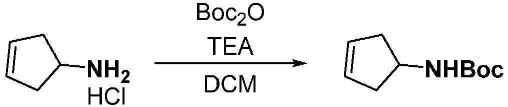

The compound 3-cyclopentenylamine hydrochloride (4.5 g, 37.625 mmol, 1.0 eq) was dissolved in dichloromethane (188 ml), and under nitrogen protection, di-t-butyl dicarbonate (9.85 g, 45.151 mmol, 1.2 eq) and triethylamine (23 mL, 165.552 mmol, 4.4 eq) were added, and the reaction mixture was allowed to react at room temperature (18°C) overnight (10 h). After the reaction was complete as indicated by TLC and LCMS, water (30 mL) was added to the reaction mixture, which was extracted three times with dichloromethane (100 mL for each), dried over anhydrous sodium sulfate, filtrated, and concentrated. The crude product was purified by column chromatography (petroleum ether: ethyl acetate=5: 1) to obtain 5.85 g t-butyl cyclopent-3-en-1-ylcarbamate as an intermediate, yield: 85.0%. LCMS: [M+1]⁺ = 184.17; ¹H NMR (400 MHz, DMSO-d₆) δ 6.97 (d, J = 7.2 Hz, 1H), 5.64 (s, 2H), 4.04 (dt, J = 8.0, 4.6 Hz, 1H), 2.54 (d, J = 8.2, 1.9 Hz, 1H), 2.20 - 2.05 (m, 2H), 1.47 (s, 1H), 1.38 (s, 9H).

### Step II: preparation of t-butyl (6-oxabicyclo[3.1.0]hex-3-yl)carbamate

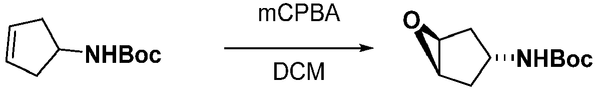

The compound t-butyl cyclopent-3-en-1-ylcarbamate (5.85 g, 31.924 mmol, 1.0 eq) was dissolved in dichloromethane (160 ml), and under nitrogen protection, m-chloroperbenzoic acid (9.72 g, 47.885 mmol, 1.5 eq) was added, and the reaction mixture was allowed to react at room temperature (18°C) overnight (10h). After the reaction was complete as indicated by TLC and LCMS, water (30 mL) was added to the reaction mixture, which was extracted three times with dichloromethane (100 mL each), dried over anhydrous sodium sulfate, filtrated, and concentrated. The crude product was purified by column chromatography (dichloromethane) to obtain 800 mg light yellow solid product t-butyl (6-oxabicyclo[3.1.0]hex-3-yl)carbamate as an intermediate,, as a, yield: 12.6%. LCMS: [M+1]⁺ = 184.17; ¹H NMR (400 MHz, DMSO-d₆) δ 6.79 (d, J = 8.5 Hz, 1H), 3.59 - 3.49 (m, 1H), 3.44 (s, 2H), 2.17 (dd, J = 13.9, 7.6 Hz, 2H), 1.48 (dd, J = 13.8, 8.9 Hz, 2H), 1.36 (s, 9H).

### Step III: preparation of t-butyl (3-(3,4-dihydroisoquinolin-2(1H)-yl)-4-hydroxycyclopentyl)carbamate:

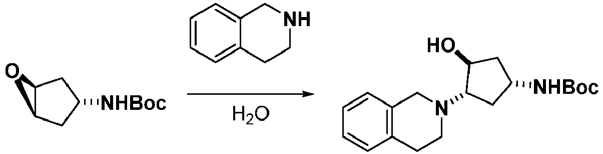

The raw material t-butyl (6-oxabicyclo[3.1.0]hex-3-yl)carbamate (150 mg, 0.754 mmol, 1.0 eq) was dissolved in water (4 mL), and under nitrogen protection, 1,2,3,4-tetrahydroisoquinoline (110 mg, 0.829 mmol, 1.1 eq) was added, and the reaction mixture was allowed to react at 80°C overnight (10 h). After the reaction was complete as indicated by TLC and LCMS, the reaction mixture was extracted three times with dichloromethane (30 mL for each), dried over anhydrous sodium sulfate, filtrated, and concentrated. The crude product was purified by column chromatography (dichloromethane/methanol=15/1) to obtain 210 mg light yellow solid product t-butyl (3-(3,4-dihydroisoquinolin-2(1H)-yl)-4-hydroxycyclopentyl)carbamate as an intermediate, yield: 84.0%. LCMS: [M+1]⁺ = 333.14; 1H NMR (400 MHz, DMSO-d6) δ 7.16 - 6.96 (m, 4H), 6.82 (d, J = 8.0 Hz, 1H), 4.73 (d, J = 5.0 Hz, 1H), 4.06 - 3.98 (m, 1H), 3.96 - 3.85 (m, 1H), 3.61 (s, 2H), 2.93 - 2.83 (m, 1H), 2.81 - 2.73 (m, 2H), 2.68 - 2.51 (m, 3H), 2.13 (dt, J = 12.6, 6.6 Hz, 1H), 1.79 - 1.61 (m, 2H), 1.38 (s, 9H).

### Step IV: preparation of 4-amino-2-(3,4-dihydroisoquinolin-2(1H)-yl)cyclopentan-1-ol:

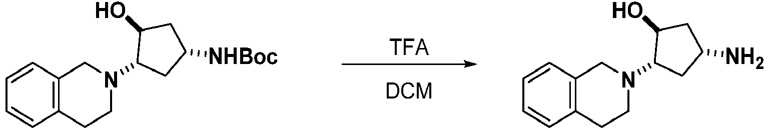

The raw material t-butyl (3-(3,4-dihydroisoquinolin-2(1H)-yl)-4-hydroxycyclopentyl)carbamate (100 mg, 0.301 mmol, 1.0 eq) was dissolved in dichloromethane (4 ml), and under nitrogen protection, trifluoroacetic acid (1 ml) was added, and the reaction mixture was allowed to react at room temperature (18°C) for 2 h. After the reaction was complete as indicated by TLC and LCMS, 1,2-dichloroethane (5mL) was added to the reaction mixture, which was concentrated to obtain 84 mg, yellow oily liquid crude product (4-amino-2-(3,4-dihydroisoquinolin-2(1H)-yl)cyclopentan-1-ol, as an intermediate, yield: 100%. LCMS: [M+1]⁺ = 233.00.

### Step V: preparation of N-(3-(3,4-dihydroisoquinolin-2(1H)-yl)-4-hydroxycyclopentyl)-6-(oxetan-3-ylamino)pyrimidine-4-carboxamide

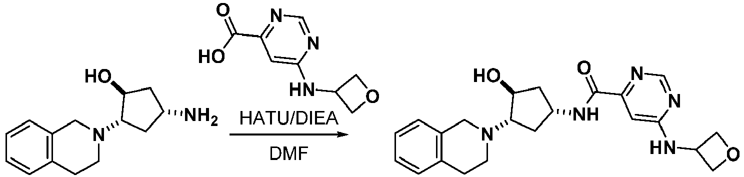

(4-amino-2-(3,4-dihydroisoquinolin-2(1H)-yl)cyclopentan-1-ol (35 mg, 0.150 mmol, 1.0 eq), 6-(oxetan-3-ylamino)pyrimidine-4-carboxylic acid (30 mg, 0.150 mmol, 1.0 eq) and HATU (2-(7-azabenzotriazole)-*N,N,N',N'*-tetramethyluronium hexafluorophosphate) (86 mg, 0.226 mmol, 1.5 eq) were dissolved in DMF (N,N-dimethylformamide) (1 mL), then DIEA (*N,N-*diisopropylethylamine) (0.13 ml, 0.754 mmol, 5.0 eq) was added, and the reaction mixture was allowed to react under nitrogen protection at room temperature (18°C) for 10h. After the reaction was complete as indicated by TLC and LCMS, the reaction mixture was poured into 50 mL water, and extracted three times with ethyl acetate (15 mL each). The ethyl acetate phases were combined, washed once with 20 mL water and once with 20 mL saturated sodium chloride solution, dried over anhydrous sodium sulfate, filtrated, and concentrated. Upon reverse phase preparation, 5.54 mg N-(3-(3,4-dihydroisoquinolin-2(1H)-yl)-4-hydroxycyclopentyl)-6-(oxetan-3-ylamino)pyrimid ine-4-carboxamide was obtained, yield: 9.0%. LCMS: [M+H]⁺ = 410.20; ¹H NMR (400 MHz, CDCl₃) δ 8.47 (d, J = 1.2 Hz, 1H), 8.21 (d, J = 8.0 Hz, 1H), 7.38 (brs, 1H), 7.22 - 7.12 (m, 3H), 7.07 - 7.00 (m, 1H), 6.88 (brs, 1H), 5.21 (brs, 1H), 5.05 (td, J = 7.0, 1.8 Hz, 2H), 4.62 (ddd, J = 12.4, 6.4, 4.2 Hz, 4H), 4.02 (s, 2H), 3.24 - 2.98 (m, 6H), 2.55 (dt, J = 12.9, 6.6 Hz, 1H), 2.21 (ddd, J = 11.1, 7.1, 3.5 Hz, 1H), 2.08 (dt, J = 13.8, 8.2 Hz, 1H), 1.88 (q, J = 10.4 Hz, 1H).

### Example 9

### Preparation of 6-((1-acetylpiperidin-4-yl)amino)-N-(3-(3,4-dihydroisoquinolin-2(1H)-yl)-4-hydroxycyclopentyl)pyrimidine-4-carboxamide:

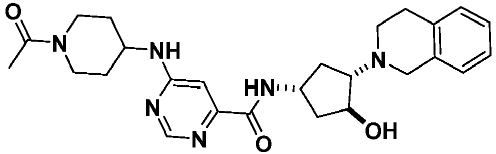

### Step I: preparation of 6-((1-acetylpiperidin-4-yl)amino)-N-(3-(3,4-dihydroisoquinolin-2(1H)-yl)-4-hydroxycyclopentyl)pyrimidine-4-carboxamide:

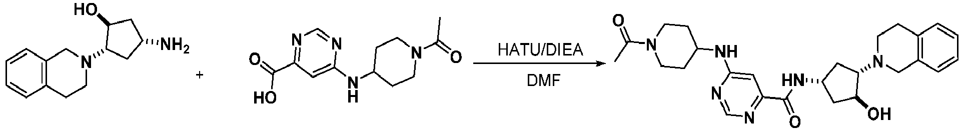

(4-amino-2-(3,4-dihydroisoquinolin-2(1*H*)-yl)cyclopentan-1-ol (35 mg, 0.150 mmol, 1.0 eq), 6-((1-acetylpiperidin-4-yl)amino)-pyrimidine-4-carboxylic acid (40 mg, 0.150 mmol, 1.0 eq) and HATU (2-(7-azabenzotriazole)-N,N,N',N'-tetramethyluronium hexafluorophosphate) (86 mg, 0.226 mmol, 1.5 eq) were dissolved in DMF (*N,N*-dimethylformamide) (1 mL), then DIEA (*N,N*-diisopropylethylamine) (0.13 ml, 0.754 mmol, 5.0 eq) was added, and under nitrogen protection, the reaction mixture was allowed to react at room temperature (18°C) for 10h. After the reaction was complete as indicated by TLC and LCMS, the reaction mixture was poured into 50 mL water, and extracted three times with ethyl acetate (15 mL for each). The ethyl acetate phases were combined, washed once with 20 mL water and once with 20 mL saturated sodium chloride solution, dried over anhydrous sodium sulfate for 10 min, filtrated, and concentrated. Upon reverse phase preparation, 4.89 mg 6-((1-acetylpiperidin-4-yl)amino)-N-(3-(3,4-dihydroisoquinolin-2(1H)-yl)-4-hydroxy-cyclop entyl)pyrimidine-4-carboxamide was obtained, yield: 6.8%. LCMS: [M+H]⁺ = 479.27; ¹H NMR (400 MHz, CD3Cl) δ 8.50 (s, 1H), 8.26 (s, 1H), 7.30 (d, J = 7.2 Hz, 1H), 7.24 (d, J = 7.6 Hz, 1H), 7.22 - 7.13 (m, 2H), 7.09 (d, J = 7.5 Hz, 1H), 4.93 (s, 1H), 4.49 (d, J = 16.8 Hz, 3H), 4.20 (brs, 1H), 3.81 (d, J = 13.5 Hz, 1H), 3.60 (s, 1H), 3.50 (s, 1H), 3.30 - 3.19 (m, 2H), 2.76 (s, 1H), 2.58 (s, 1H), 2.17-2.11 (m, 4H), 2.09 (d, J = 3.5 Hz, 3H), 1.99-1.91 (m, 2H), 1.88-1.55 (m, 4H), 1.43 - 1.24 (m, 2H).

### Example 10

### Preparation of cis-(3-(3,4-dihydroisoquinolin-2(1H)-yl)-4-hydroxypyrrolidin-1-yl)(6-(oxetan-3-ylamino)pyrimidin-4-yl)ketone:

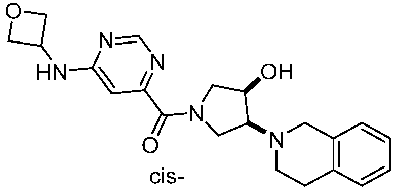

### Step I: preparation of cis-(3-(3,4-dihydroisoquinolin-2(1H)-yl)-4-hydroxypyrrolidin-1-yl)(6-(oxetan-3-ylamino)pyrimidin-4-yl)ketone:

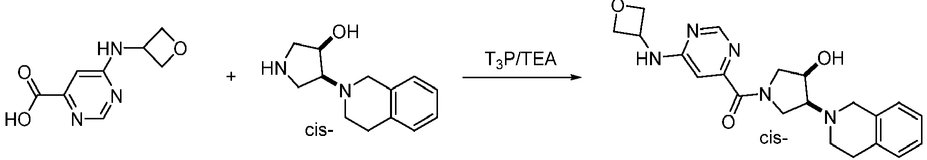

Cis-4-(3,4-dihydroisoquinolin-2(1*H*)-yl)pyrrolidin-3-ol (90 mg, 0.41 mmol, 1.0 eq) was dissolved in DMF (*N,N*-dimethylformamide) (2 mL), and under nitrogen protection, 6-(oxetan-3-ylamino)pyrimidine-4-carboxylic acid (72 mg, 0.37 mmol, 0.9 eq), TEA (triethylamine) (207 mg, 2.05 mmol, 5.0 eq) and T₃P (1-propylphosphoric anhydride) (50wt% in ethyl acetate, 261 mg, 0.82 mmol, 2.0 eq) were added to the reaction mixture. The reaction was conducted under stirring at room temperature (25-30°C) for 3 h. After the reaction was complete as indicated by TLC and LCMS, the reaction mixture was poured into 10mL water, and extracted three times with ethyl acetate (20 mL for each). The ethyl acetate phases were combined, washed once with 10 mL saturated sodium chloride solution, dried over anhydrous sodium sulfate for 5 min, filtrated, and concentrated. Upon purification by preparative HPLC (C18, 10 mmol/L aqueous solution, acetonitrile), cis-(3-(3,4-dihydroisoquinolin-2(1*H*)-yl)-4-hydroxypyrrolidin-1-yl)(6-(oxetan-3-ylamino)-py rimidin-4-yl)ketone (2.2 mg, yield: 1.3%) was obtained. LCMS(ESI)[M+1]⁺ = 396.18; ¹H NMR (400 MHz, CDCl₃) δ 8.59 (m, 1H), 7.22 - 7.09 (m, 3H), 7.07 - 6.99 (m, 1H), 6.96 - 6.86 (m, 1H), 5.83 (m, 1H), 5.20 - 4.93 (m, 3H), 4.59 (q, *J* = 5.8 Hz, 2H), 4.46 - 4.28 (m, 1H), 4.14 - 3.46 (m, 7H), 3.11 - 2.89 (m, 4H), 2.88 - 2.69 (m, 1H).

### Example 11

### Preparation of 1-(4-((6-((3-(3,4-dihydroisoquinolin-2(1H)-yl)-4-hydroxycyclopentyl)-amino)pyrimidin-4-yl)amino)piperidin-1-yl)ethan-1-one:

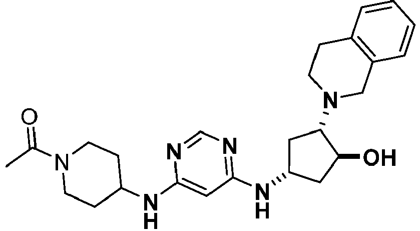

### Step I: preparation of 1-(4-((6-((3-(3,4-dihydroisoquinolin-2(1H)-yl)-4-hydroxycyclopentyl)-amino)pyrimidin-4-yl)amino)piperidin-1 -yl)ethan-1 -one:

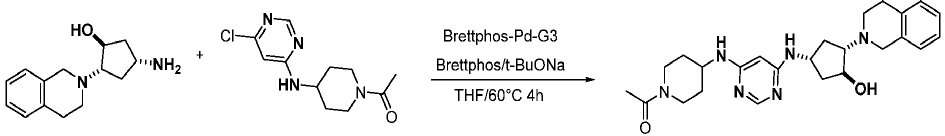

(4-amino-2-(3,4-dihydroisoquinolin-2(1*H*)-yl)cyclopentan-1-ol (50 mg, 0.216 mmol, 1.0 eq), 1-(4-((6-chloropyrimidin-4-yl)amino)piperidin-1-yl)ethan-1-one (66 mg, 0.259 mmol, 1.2 eq), Brettphos (bicyclohexyl[3,6-dimethoxy-2',4',6'-triisopropyl[1,1'-biphenyl]-2-yl]phosphine) (12 mg, 0.022 mmol, 0.1 eq), Brettphos-Pd-G3 ((2-bicyclohexylphosphino-3,6-dimethoxy-2',4',6'-triisopropyl-1,1'-biphenyl)(2-amino-1,1'-biphen-2-yl) palladium (II) methanesulfonate) (20 mg, 0.022 mmol, 0.1 eq) and *t*-BuONa (sodium t-butoxide) (31 mg, 0.323 mmol, 1.5 eq) were added to a sealed microwave tube, and THF (tetrahydrofuran) (1.1 mL) was added under nitrogen protection. The reaction was conducted for 4 h under nitrogen protection by raising the temperature to 60°C. After the reaction was complete as indicated by TLC and LCMS, the reaction mixture was poured into 5 mL water, and extracted three times with ethyl acetate (10 mL each). The ethyl acetate phases were combined, washed once with 10 mL water and once with 10 mL saturated sodium chloride solution, dried over anhydrous sodium sulfate for 10 mins, filtrated, and concentrated. Upon purification by preparative HPLC, 18.1 mg 1-(4-((6-((3-(3,4-dihydroisoquinolin-2(1H)-yl)-4-hydroxycyclopentyl)amino)pyrimidin-4-yl)amino)piperidin-1-yl)ethan-1-one was obtained, yield: 18.6%. LCMS: [M+H]⁺ = 451.26; ¹H NMR (400 MHz, CDCl₃) δ 8.06 (s, 1H), 7.24 - 7.12 (m, 3H), 7.05 (d, J = 7.2 Hz, 1H), 6.04 (brs, 1H), 5.59 (brs, 1H), 5.35 (s, 1H), 4.58 (s, 1H), 4.46 (d, J = 13.7 Hz, 1H), 4.29 (s, 1H), 4.10 - 3.95 (m, 2H), 3.80 (d, J = 14.9 Hz, 2H), 3.23 (d, J = 12.7 Hz, 1H), 3.14 (s, 1H), 3.06 - 3.00 (m, 2H), 2.86 (t, J = 12.5 Hz, 1H), 2.61 - 2.51 (m, 2H), 2.11 (s, 3H), 2.08 - 1.96 (m, 5H), 1.87 - 1.74 (m, 1H), 1.51 - 1.33 (m, 3H).

### Example 12

### Preparation of 6-((1-acetylpiperidin-4-yl)amino)-N-(3-(3,4-dihydroisoquinolin-2(1H)-yl)-4-hydroxycyclopentyl)pyrimidine-4-carboxamide:

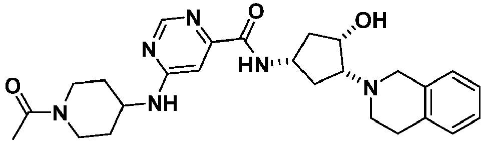

### Step I: preparation of t-butyl (3-(3,4-dihydroisoquinolin-2(1H)-yl)-4-carbonylcyclopentyl) carbamate:

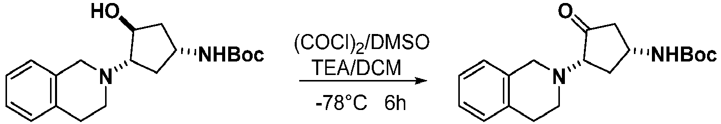

Oxalyl chloride (0.38 mL, 4.52 mmol, 3.0 eq) was dissolved in DCM (dichloromethane) (1 mL) at -78°C, and slowly added dropwise to a 1 ml solution of dichloromethane in dimethyl sulfoxide (DMSO) (0.44 mL, 6.02 mmol, 4.0 eq). Under a water- and oxygen-free condition at -78°C, the reaction mixture was stirred for 1 h. Then, the raw material t-butyl (3-(3,4-dihydroisoquinolin-2(1*H*)-yl)-4-hydroxycyclopentyl)carbamate (500 mg, 1.51 mmol, 1.0 eq) was dissolved in dichloromethane (5.5 mL) and added dropwise to the reaction system under nitrogen protection, and the reaction was conducted for 4.5 h at -78°C. Triethylamine (1.68 mL, 12.05 mmol, 8.0 eq) was further added, and the reaction was conducted at -78°C for 0.5 h. After the reaction was complete as indicated by TLC and LCMS, the reaction was quenched by adding aqueous ammonium chloride (5 mL). Dichloromethane (30 mL) and water (5 mL) were added to the reaction mixture, which was extracted three times (30 mL for each), dried over anhydrous sodium sulfate, filtrated, and concentrated. The crude product was separated by column chromatography (DCM/MeOH=15/1) to obtain t-butyl (3-(3,4-dihydroisoquinolin-2(1*H*)-yl)-4-carbonylcyclopentyl)carbamate (200 mg, yield: 40.2%) as an intermediate. LCMS(ESI)[M+1]⁺ = 331.32.

### Step II: preparation of t-butyl (3-(3,4-dihydroisoquinolin-2(1H)-yl)-4-hydroxycyclopentyl) carbamate:

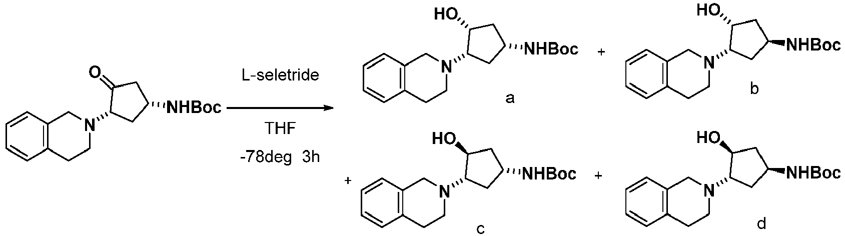

Under a water- and oxygen-free condition at -78°C, the raw material t-butyl (3-(3,4-dihydroisoquinolin-2(1*H*)-yl)-4-carbonylcyclopentyl) carbamate (200 mg, 0.61 mmol, 1.0 eq) was dissolved in tetrahydrofuran (3 mL), and under nitrogen protection L-seletride (lithium tri-sec-butylborohydride) (0.91 mL, 0.91 mmol, 1.5 eq) was added dropwise thereto, and the reaction was carried out at -78°C for 5 h. After the reaction was complete as indicated by TLC and LCMS, the reaction was quenched by adding aqueous ammonium chloride (5 mL). Dichloromethane (30 mL) and water (5 mL) were added to the reaction mixture, which was extracted three times (30 mL for each), dried over anhydrous sodium sulfate, filtrated, and concentrated. The crude product was separated and purified by preparative HPLC (C18, 0.08% aqueous ammonium bicarbonate, acetonitrile) to obtain four compounds:
Compound a: t-butyl (3-(3,4-dihydroisoquinolin-2(1*H*)-yl)-4-hydroxycyclopentyl) carbamate (34 mg, yield: 16.9%). LCMS(ESI)[M+1]⁺ = 333.20; 1H NMR (400 MHz, DMSO-*d₆*) δ 7.15 - 7.00 (m, 4H), 6.65 (d, J = 7.8 Hz, 1H), 4.09 (d, J = 5.3 Hz, 1H), 4.01 - 3.94 (m, 1H), 3.87 - 3.77 (m, 1H), 3.70 (d, J = 15.1 Hz, 1H), 3.59 (d, J = 15.1 Hz, 1H), 2.91 - 2.73 (m, 3H), 2.69 - 2.60 (m, 1H), 2.39 (ddd, J = 11.2, 6.5, 4.0 Hz, 1H), 2.23 - 2.06 (m, 2H), 1.60 (td, J = 11.8, 8.6 Hz, 1H), 1.48 (dd, J = 14.4, 4.9 Hz, 1H), 1.38 (s,9H).
Compound b: t-butyl (3-(3,4-dihydroisoquinolin-2(1*H*)-yl)-4-hydroxycyclopentyl) carbamate (8.0 mg, yield: 4.0%). LCMS(ESI)[M+1]⁺ = 333.20; ¹H NMR (400 MHz, DMSO-*d₆*) δ 7.13 - 7.01 (m, 4H), 6.93 (d, J = 7.7 Hz, 1H), 4.17 (s, 1H), 4.08 - 3.96 (m, 1H), 3.87 (s, 1H), 3.68 (d, J = 15.1 Hz, 1H), 3.58 (d, J = 15.1 Hz, 1H), 2.90 - 2.74 (m, 3H), 2.69 - 2.57 (m, 2H), 2.04 - 1.92 (m, 2H), 1.71 - 1.62 (m, 1H), 1.57 (ddd, J = 13.0, 7.7, 4.2 Hz, 1H), 1.38 (s, 9H).
Compounds c and d: LCMS(ESI)[M+1]⁺ = 333.20, not seperated.

### Step III: preparation of 4-amino-2-(3,4-dihydroisoquinolin-2(1H)-yl)cyclopentan-1-ol:

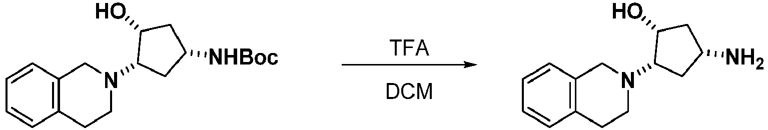

The raw material t-butyl (3-(3,4-dihydroisoquinolin-2(1*H*)-yl)-4-hydroxycyclopentyl) carbamate (34 mg, 0.10 mmol, 1.0 eq) was dissolved in dichloromethane (4 mL), and under nitrogen protection, trifluoroacetic acid (1 mL) was added, and the reaction mixture was allowed to react at room temperature for 1 h. After the reaction was complete as indicated by TLC and LCMS, 1,2-dichloroethane (5 mL) was added to the reaction mixture, which was concentrated to give an intermediate crude product 4-amino-2-(3,4-dihydroisoquinolin-2(1*H*)-yl)cyclopentan-1-ol. LCMS(ESI)[M+1]⁺ = 233.24. Step IV: preparation of 6-((1-acetylpiperidin-4-yl)amino)-N-(3-(3,4-dihydroisoquinolin-2(1H)-yl)-4-hydroxycyclopentyl)pyrimidine-4-carboxamide:

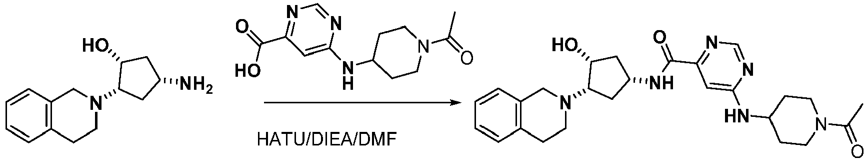

4-amino-2-(3,4-dihydroisoquinolin-2(1*H*)-yl)cyclopentan-1-ol (23.7 mg, 0.10 mmol, 1.0 eq), 6-((1-acetylpiperidin-4-yl)amino)pyrimidine-4-carboxylic acid (27 mg, 0.10 mmol, 1.0 eq) and HATU (2-(7-azabenzotriazole)-N,N,N',N'-tetramethyluronium hexafluorophosphate) (59 mg, 0.15 mmol, 1.5 eq) were dissolved in DMF (*N,N*-dimethylformamide) (1 mL), and DIEA (*N,N*-diisopropylethylamine) (0.09 ml, 0.51 mmol, 5.0 eq) was added. Under nitrogen protection, the reaction mixture was allowed to react at room temperature for 3 h. After the reaction was complete as indicated by TLC and LCMS, the reaction mixture was poured into 10 mL water, and extracted three times with ethyl acetate (10 mL each). The ethyl acetate phases were combined, washed once with 20 mL water and once with 20 mL saturated sodium chloride solution, dried over anhydrous sodium sulfate for 3 mins, filtrated, and concentrated. The crude product was separated and purified by preparative HPLC (C18, 0.08% aqueous NH₄HCO₃, acetonitrile) to obtain 6-((1-acetylpiperidin-4-yl)amino)-*N*-(3-(3,4-dihydroisoquinolin-2(1*H*-yl)-4-hydroxycyclope ntyl)pyrimidine-4-carboxamide (14.6 mg, yield: 29.9%). LCMS(ESI)[M+H]⁺ = 479.43; ¹H NMR (400 MHz, CD₃Cl) δ 8.50 (d, J = 1.1 Hz, 1H), 8.43 (d, J = 9.0 Hz, 1H), 7.20 - 7.09 (m, 4H), 7.05 - 6.98 (m, 1H), 5.21 (d, J = 7.8 Hz, 1H), 4.66 - 4.50 (m, 2H), 4.31 (d, J = 4.0 Hz, 1H), 4.07 (brs, 1H), 3.91 - 3.80 (m, 2H), 3.75 (d, J = 14.9 Hz, 1H), 3.23 (ddd, J = 14.2, 11.8, 2.8 Hz, 1H), 2.99 (s, 1H), 2.93 (s, 2H), 2.89 - 2.70 (m, 3H), 2.56 (dt, J = 12.8, 7.7 Hz, 1H), 2.26 - 2.18 (m, 1H), 2.12 (s, 3H), 2.06 (d, J = 13.3 Hz, 1H), 1.94 (dd, J = 14.6, 3.1 Hz, 1H), 1.85 (s, 1H), 1.62 (s, 2H), 1.50 - 1.37 (m, 2H).

### Example 13

### Preparation of 6-((1-acetylpiperidin-4-yl)amino)-N-(3-(3,4-dihydroisoquinolin-2(1H)-yl)-4-hydroxycyclopentyl)pyrimidine-4-carboxamide:

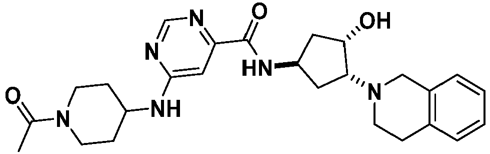

### Step I: preparation of 4-amino-2-(3,4-dihydroisoquinolin-2(1H)-yl)cyclopentan-1-ol:

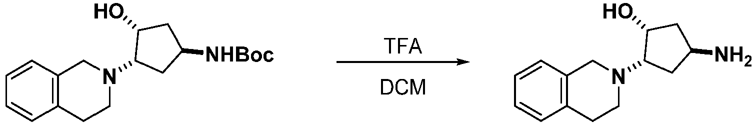

The raw material t-butyl (3-(3,4-dihydroisoquinolin-2(1*H*)-yl)-4-hydroxycyclopentyl) carbamate (8 mg, 0.02 mmol, 1.0 eq) was dissolved in dichloromethane (2 mL), and under nitrogen protection, trifluoroacetic acid (0.5 mL) was added, and the reaction mixture was allowed to react at room temperature (20-25°C) for 1 h. After the reaction was complete as indicated by TLC and LCMS, 1,2-dichloroethane (3 mL) was added to the mixture which was then concentrated to give an intermediate crude product (1*R*,2*S*,4*S*)-4-amino-2-(3,4-dihydroisoquinolin-2(1*H*)-yl)cyclopentan-1-ol.
LCMS(ESI)[M+1]⁺ = 233.16.

### Step II: preparation of 6-((1-acetylpiperidin-4-yl)amino)-N-(3-(3,4-dihydroisoquinolin-2(1H)-yl)-4-hydroxycyclopentyl)pyrimidine-4-carboxamide:

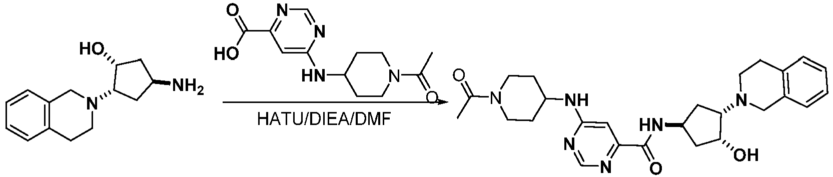

4-amino-2-(3,4-dihydroisoquinolin-2(1*H*)-yl)cyclopentan-1-ol (5.6 mg, 0.02 mmol, 1.0 eq), 6-((1-acetylpiperidin-4-yl)amino)pyrimidine-4-carboxylic acid (6.4 mg, 0.02 mmol, 1.0 eq) and HATU (2-(7-azabenzotriazole)-N,N,N',N'-tetramethyluronium hexafluorophosphate) (14 mg, 0.036 mmol, 1.5 eq) were dissolved in DMF (*N,N*-dimethylformamide) (1 mL), then DIEA (*N,N*-diisopropylethylamine) (16 mg, 0.120 mmol, 5.0 eq) was added, and under nitrogen protection, the reaction mixture was allowed to react at room temperature (18°C) for 3 h. After the reaction was complete as indicated by TLC and LCMS, the reaction mixture was poured into 10 mL water, and extracted three times with ethyl acetate (10 mL for each). The ethyl acetate phases were combined, washed once with 20 mL water and once with 20 mL saturated sodium chloride solution, dried over anhydrous sodium sulfate for 1 min, filtrated, and concentrated. The crude product was separated and purified by preparative HPLC (C18, 0.08% aqueous NH₄HCO₃, acetonitrile) to obtain 6-((1-acetylpiperidin-4-yl)amino)-*N-*((1*S*,3*S*,4*R*)-3-(3,4-dihydroisoquinolin-2(1*H*)-yl)-4-hydroxycyclopentyl)pyrimidine-4-carbox amide (3.1 mg, yield: 27.0%). LCMS(ESI)[M+1]⁺ = 479.23; ¹H NMR (400 MHz, CD3Cl) δ 8.49 (s, 1H), 8.01 (d, J = 7.5 Hz, 1H), 7.22 - 7.10 (m, 4H), 7.05 (d, J = 7.0 Hz, 1H), 5.49 (brs, 1H), 4.72 - 4.63 (m, 1H), 4.56 (d, J = 13.6 Hz, 1H), 4.47 (s, 1H), 4.07 - 3.90 (m, 2H), 3.84 (d, J = 14.1 Hz, 1H), 3.29 - 3.08 (m, 3H), 3.00 (s, 2H), 2.83 (t, J = 12.5 Hz, 1H), 2.51 (dd, J = 14.6, 8.2 Hz, 2H), 2.12 (s, 3H), 2.08 - 1.97 (m, 3H), 1.88 - 1.80 (m, 2H), 1.72 - 1.63 (m, 2H), 1.46 (d, J = 11.7 Hz, 2H).

### Example 14

### Preparation of 1-(4-((6-((3-(3,4-dihydroisoquinolin-2(1H)-yl)-4-hydroxycyclopentyl)oxy)pyrimidin-4-yl) amino)piperidin-1-yl)ethan-1-one:

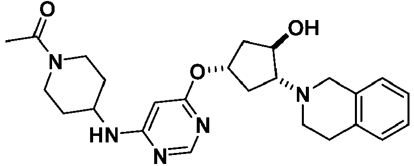

### Step I: preparation of 4-((5-butyldiphenylsilyl)oxy)-2-(3,4-dihydroisoquinolin-2(1H)-yl)cyclopentyl pivalate:

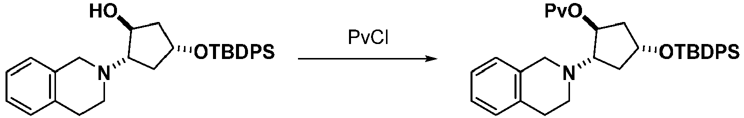

4-((5-butyldiphenylsilyl)oxy)-2-(3,4-dihydroisoquinolin-2(1*H*)-yl)cyclopentanol and DMAP (4-dimethylaminopyridine) (16 mg, 0.13 mmol, 0.1 eq) were dissolved in dry acetonitrile (6 ml), and triethylamine (258 mg, 2.55 mmol, 2.0 eq) was added under nitrogen protection. The solution was heated to 40°C, and PvCl (trimethylacetyl chloride) (306 mg, 2.55 mmol, 2.0 eq) was added. After reacting at 60°C for 1 h, the reaction was complete as indicated by TLC. Water was added to the reaction mixture which was then extracted with ethyl acetate, dried over sodium sulfate, and rotary evaporated till dryness. The crude product was separated and purified by flash chromatography (silica gel, EA: PE = 0 to 8%), and 4-((5-butyldiphenylsilyl)oxy)-2-(3,4-dihydroisoquinolin-2(1*H*)-yl)cyclopentyl pivalate was obtained (680 mg, yield: 96.3%).

### Step II: preparation of 2-(3,4-dihydroisoquinolin-2(1H)-yl)-4-hydroxycyclopentyl pivalate:

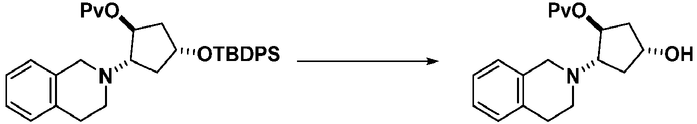

4-((5-butyldiphenylsilyl)oxy)-2-(3,4-dihydroisoquinolin-2(1*H*)-yl)cyclopentyl pivalate (430 mg, 0.78 mmol, 1.0 eq) was dissolved in tetrahydrofuran (5 ml), and a tetrabutylammonium fluoride solution (1.0 M in tetrahydrofuran, 1.6 ml, 1.55 mmol, 2.0 eq) was added. The reaction was carried out at room temperature overnight (16 h), and the reaction was complete as monitored by TLC and LCMS. Ethyl acetate was added to the reaction mixture, which was then washed with water, and the aqueous phase was back-extracted with ethyl acetate. The organic phases were combined, dried over sodium sulfate, and rotary evaporated till dryness to obtain a crude product 2-(3,4-dihydroisoquinolin-2(1*H*)-yl)-4-hydroxycyclopentyl pivalate (513 mg) as a brown oily product. The crude product was directly used in the next step. ¹H NMR (400 MHz, CDCl₃) δ 7.15 - 7.06 (m, 3H), 7.01 - 6.96 (m, 1H), 5.20 (ddd, *J* = 8.3, 5.0, 3.5 Hz, 1H), 4.37 (tt, *J* = 5.2, 2.3 Hz, 1H), 3.69 (s, 2H), 3.45 (s, 2H), 3.32 (ddd, *J* = 9.8, 7.4, 4.9 Hz, 1H), 2.85 (dq, *J* = 22.6, 5.8 Hz, 3H), 2.78 - 2.71 (m, 1H), 2.48 (ddd, *J =* 15.1, 8.3, 5.4 Hz, 1H), 2.19 (ddt, *J* = 13.5, 7.4, 2.4 Hz, 1H), 1.86 (ddd, *J* = 13.3, 9.8, 5.1 Hz, 1H), 1.66 (dq, *J=* 15.0, 2.7 Hz, 1H), 1.23 (s, 9H).

### Step III: preparation of 4-((6-((1-acetylpiperidin-4-yl)(t-butoxycarbonyl)amino)pyrimidin-4-yl)oxy)-2-(3,4-dihydroisoquinolin-2(1H)-yl)cyclopentyl pivalate:

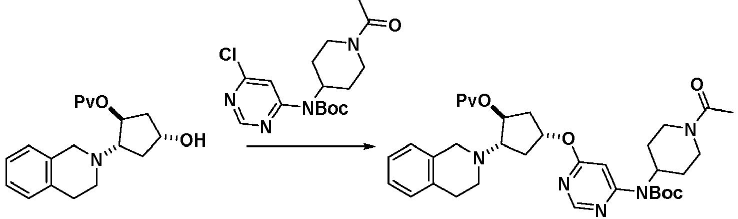

2-(3,4-dihydroisoquinolin-2(1H)-yl)-4-hydroxycyclopentyl pivalate (452 mg crude product, 1.43 mmol, 1.0 eq) was dissolved in tetrahydrofuran (3 ml), and a suspension (2 ml) of sodium hydride in tetrahydrofuran (a suspension formed by adding 63 mg solution of sodium hydride at 60wt% to 2 ml tetrahydrofuran, 1.1 eq) was added dropwise at 0°C under nitrogen protection. After stirring for half an hour, t-butyl (1-acetylpiperidin-4-yl)(6-chloropyrimidin-4-yl)carbamate (253 mg, 0.71 mmol, 0.5 eq) in tetrahydrofuran (4 ml) was added. The reaction mixture was warmed to room temperature and stirred for 3 h, and quenched by adding saturated ammonium chloride solution. The mixture was extracted with ethyl acetate and rotary evaporated to dryness. Seperation and purification were carried out by flash chromatography (silica gel, MeOH: DCM = 0 to 1%) to obtain 140 mg 4-((6-((1-acetylpiperidin-4-yl)(t-butoxycarbonyl)amino)pyrimidin-4-yl)oxy)-2-(3,4-dihydroisoquinolin-2(1H)-yl)cyclopentyl pivalate. Two-step yield: 28.5%. LCMS (ESI) [M+H]⁺ = 636.17.

### Step IV: preparation of 4-((6-((1-acetylpiperidin-4-yl)amino)pyrimidin-4-yl)oxy)-2-(3,4-dihydroisoquinolin-2(1H)-yl)cyclopentyl pivalate:

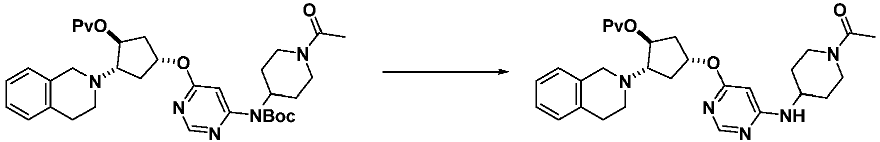

4-((6-((1-acetylpiperidin-4-yl)(t-butoxycarbonyl)amino)pyrimidin-4-yl)oxy)-2-(3,4-dihydroisoquinolin-2(1*H*)-yl)cyclopentyl pivalate (55 mg, 0.087 mmol) was dissolved in dichloromethane (2 ml), and trifluoroacetic acid (0.5 ml) was added. The reaction was carried out at room temperature for 2 h, and was complete as monitored by TLC and LCMS. The reaction mixture was rotary evaporated to dryness to obtain a crude product 4-((6-((1-acetylpiperidin-4-yl)amino)pyrimidin-4-yl)oxy)-2-(3,4-dihydroisoquinolin-2(1*H*)-yl )cyclopentyl pivalate (80 mg) as a light yellow oil, which was directly used in the next step. LCMS (ESI) [M+H]⁺ = 536.48.

### Step V: preparation of 1-(4-((6-(((3-(3,4-dihydroisoquinolin-2(1H)-yl)-4-hydroxycyclopentyl)oxy)pyrimidin-4-yl)amino)piperidin-1-yl)ethanone:

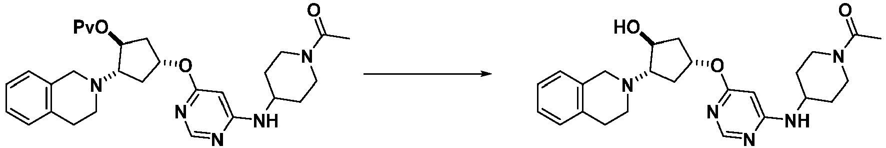

4-((6-((1-acetylpiperidin-4-yl)amino)pyrimidin-4-yl)oxy)-2-(3,4-dihydroisoquinolin-2(1*H*)-yl)cyclopentyl pivalate (80 mg crude product, 0.087 mmol, 1.0 eq) was dissolved in anhydrous methanol (2 ml), and sodium methoxide (122 mg, 2.27 mmol, 26 eq) was added. The reaction was conducted at 50°C for 2 h, and monitored by LCMS. After the reaction was complete, 1 N HCl aqueous solution was added to adjust the pH to 7. The crude product was concentrated and reverse phase preparation was conducted, to obtain 1-(4-((6-((3-(3,4-dihydroisoquinolin-2(1H)-yl)-4-hydroxycyclopentyl)oxy)pyrimidin-4-yl)am ino)piperidin-1-yl)ethan-1-one (10 mg). LCMS: LCMS (ESI) [M+H]⁺ = 452.24; ¹H NMR (400 MHz, CDCl₃) δ 8.25 (d, *J* = 0.8 Hz, 1H), 7.18 - 7.07 (m, 3H), 7.06 - 7.00 (m, 1H), 5.65 (d, *J* = 0.9 Hz, 1H), 5.45 (s, 1H), 4.74 (d, *J* = 7.9 Hz, 1H), 4.51 (d, *J* = 13.6 Hz, 1H), 4.35 (s, 1H), 3.94 - 3.69 (m, 4H), 3.22 (td, *J* = 13.9, 12.7, 2.8 Hz, 1H), 3.11 (dt, *J* = 11.7, 6.3 Hz, 1H), 3.04 - 2.76 (m, 5H), 2.51 (ddd, *J* = 14.2, 8.0, 5.6 Hz, 1H), 2.39 - 2.17 (m, 1H), 2.15 - 1.88 (m, 8H), 1.40 (qd, *J* = 11.4, 4.3 Hz, 2H).

### Example 15

### Preparation of trans-1-(4-((2-(3-(3,4-dihydroisoquinolin-2(1H)-yl)-4-hydroxypyrrolidin-1-yl)pyrimidin-4-yl)amino)piperidin-1-yl)ethan-1-one:

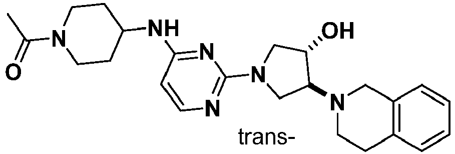

### Step I: preparation of trans-1-(4-((2-(3-(3,4-dihydroisoquinolin-2(1H)-yl)-4-hydroxypyrrolidin-1-yl)pyrimidin-4-yl)amino)piperidin-1-yl)ethanone:

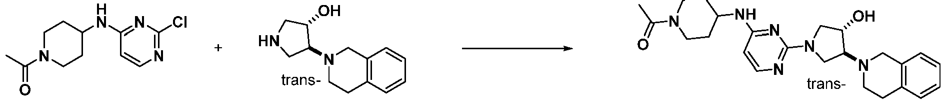

1-(4-((2-chloropyrimidin-4-yl)amino)piperidin-1-yl)ethanone (30 mg, 0.118 mmol, 1.0 eq) and trans-4-(3,4-dihydroisoquinolin-2(1*H*)-yl)pyrrolidin-3-ol (26 mg, 0.118 mmol, 1.0 eq) were dissolved in isopropanol (1 mL), followed by addition of N,N-diisopropylethylamine (31 mg, 0.236 mmol, 2.0 eq), and the reaction mixture was allowed to reacted at 100°C for 16 h. The reaction mixture was rotary evaporated to dryness, and reverse phase HPLC preparation was conducted to obtain 19 mg trans-1-(4-((2-(3-(3,4-dihydroisoquinolin-2(1H)-yl)-4-hydroxypyrrolidin-1-yl)pyrimidin-4-yl )amino)piperidin-1-yl)ethanone, yield: 36.9%. LCMS: [M+1]⁺ = 437.3; ¹H NMR (400 MHz, CD₃Cl) δ 7.87 (d, *J* = 5.7 Hz, 1H), 7.17 - 7.08 (m, 3H), 7.06 - 7.00 (m, 1H), 5.74 (d, *J* = 6.0 Hz, 1H), 4.81 (brs, 1H), 4.59 - 4.45 (m, 2H), 4.09 - 3.76 (m, 6H), 3.58 (t, *J* = 9.6 Hz, 1H), 3.50 - 3.39 (m, 1H), 3.26 - 3.15 (m, 2H), 3.04 - 2.79 (m, 5H), 2.20 - 2.01 (m, 5H), 1.49 - 1.34 (m, 2H).

### Example 16

### Preparation of trans-1-(4-((1-(3-(3,4-dihydroisoquinolin-2(1H)-yl)-4-hydroxy-pyrrolidine-1-carbonyl)piperidin-4-yl)amino)piperidin-1-yl)ethan-1-one:

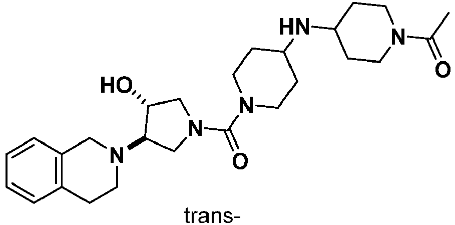

### Step I: t-butyl trans-(1-(3-(3,4-dihydroisoquinolin-2(1H)-yl)-4-hydroxypyrrolidine-1-carbonyl)piperidin-4-yl)carbamate:

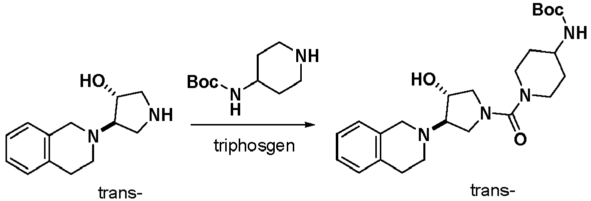

The compound trans-4-(3,4-dihydroisoquinolin-2(1*H*)-yl)pyrrolidin-3-ol (500 mg, 2.30 mmol, 1.0 eq) and bis(trichloromethyl)carbonate (273 mg, 0.40 mmol, 0.4 eq) were dissolved in dichloromethane (10 mL) and cooled to 0°C. Under nitrogen protection, t-butyl (piperidin-4-yl)carbamate (459 mg, 2.30 mmol, 1.0 eq) and *N,N*-diisopropylethylamine (890 mg, 6.90 mmol, 3.0 eq) was added, and a reaction was conducted at 0°C for 2h. After the reaction was complete as indicated by LCMS, the reaction mixture was extracted three times with dichloromethane (15 mL for each). The dichloromethane phases were combined, washed once with 20 mL water and once with 20 mL saturated sodium chloride solution, dried over anhydrous sodium sulfate for 10 min, filtrated, and concentrated. The crude product was separated and purified by flash chromatography (silica gel, DCM: MeOH = 19: 1) to give the target compound (420 mg, yield: 41.2%) as a white solid. LCMS (ESI) [M+H]⁺ =445.43; ¹H NMR (400 MHz, DMSO-d₆) δ 8.87 (s, 1H), 7.06 (d, *J* = 20.3 Hz, 4H), 6.84 (d, *J* = 7.6 Hz, 1H), 5.19 (d, *J* = 4.0 Hz, 1H), 4.30 - 4.07 (m, 1H), 3.74 (t, *J* = 11.4 Hz, 1H), 3.64 - 3.48 (m, 6H), 3.38 (s, 1H), 3.15 - 3.09 (m, 2H), 2.92 (s, 1H), 2.72 (dd, *J* = 23.5, 11.3 Hz, 6H), 1.69 (d, *J* = 11.2 Hz, 2H), 1.38 (s, 9H).

### Step II: preparation of trans-(4-aminopiperidin-1-yl)(3-(3,4-dihydroisoquinolin-2(1H)-yl)-4-hydroxypyrrolidin-1 -yl)ketone:

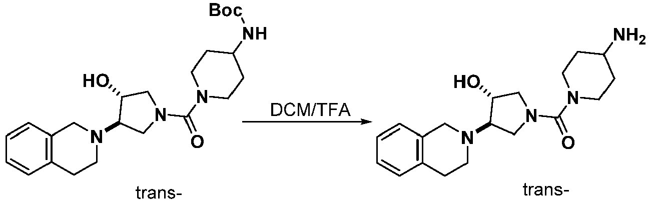

The compound t-butyl trans-(1-(3-(3,4-dihydroisoquinolin-2(1*H*)-yl)-4-hydroxypyrrolidine-1-carbonyl)piperidin-4-yl)carbamate (280 mg, 0.63 mmol, 1.0 eq) was dissolved in dichloromethane (3.2 mL), followed by addition of trifluoroacetic acid (0.8 mL), and stirred at room temperature (25°C) for 1h. After the reaction was complete as indicated by TLC, the reaction mixture was adjusted to an alkaline pH by adding saturated sodium bicarbonate solution, extracted three times with dichloromethane (10 mL for each), dried over anhydrous sodium sulfate for 10 min, filtrated, and concentrated to obtain a crude target product (191 mg, yield: 88.2%) as a yellow solid.
LCMS (ESI) [M+H]⁺ =345.21.

### Step III: preparation of trans-1-(4-((1-(3-(3,4-dihydroisoquinolin-2(1H)-yl)-4-hydroxypyrrolidine-1 -carbonyl)piperidin-4-yl)amino)piperidin-1 -yl)ethan-1 -one:

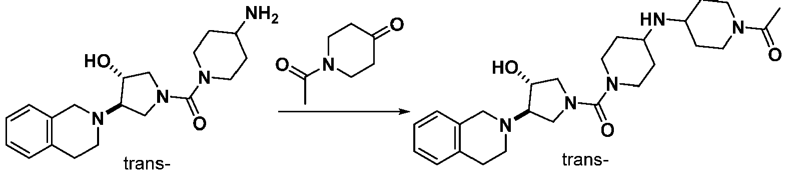

The intermediate trans-(4-aminopiperidin-1-yl)(3-(3,4-dihydroisoquinolin-2(1*H*)-yl)-4-hydroxypyrrolidin-1-yl)ketone (190 mg, 0.55 mmol, 1.0 eq) and 1-acetylpiperidin-4-one (78 mg, 0.55 mmol, 1.0 eq) were dissolved in tetrahydrofuran (2.5 mL), and stirred at room temperature (25°C) for 1 h. Then, sodium triacetoxyborohydride (140 mg, 0.66 mmol, 1.1 eq) was added. After the reaction was complete as indicated by TLC, the reaction was quenched by adding 2 mL water, and extracted three times with ethyl acetate (15 mL for each). The ethyl acetate phases were combined, washed once with 20 mL water and once with 20 mL saturated sodium chloride solution, dried over anhydrous sodium sulfate for 10 min, and filtrated. The crude product was separated and purified by preparative HPLC (C18, 10 mmol/L aqueous NH₄HCO₃, acetonitrile) to obtain the target compound (98 mg, yield: 37.8%). LCMS (ESI) [M+H]⁺ =470.39; ¹H NMR (400 MHz, CDCl₃) δ 7.15 - 7.06 (m, 3H), 7.02 - 6.98 (m, 1H), 4.47 (d, *J* = 13.3 Hz, 1H), 4.34 (q, *J* = 6.2 Hz, 1H), 3.84 (dd, *J* = 20.3, 12.2 Hz, 2H), 3.78 - 3.59 (m, 6H), 3.43 (dt, *J=* 16.1, 8.0 Hz, 1H), 3.39 - 3.30 (m, 1H), 3.13 - 3.04 (m, 1H), 3.00 (dd, *J* = 13.8, 7.4 Hz, 1H), 2.96 - 2.91 (m, 1H), 2.91 - 2.62 (m, 9H), 2.08 (s, 3H), 1.88 (s, 4H), 1.35 - 1.16 (m, 4H).

### Example 17

### Preparation of trans-1-(4-((6-(4-(3,4-dihydroisoquinolin-2(1H)-yl)-3-hydroxypiperidine-1-carbonyl)pyrimidin-4-yl)amino)piperidin-1-yl)ethanone:

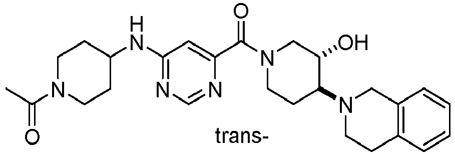

### Step I: preparation of 6-chloropyrimidine-4-carbonyl chloride:

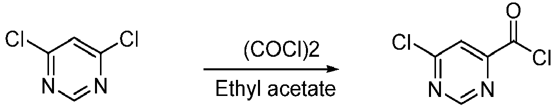

The compound 4,6-dichloropyrimidine (570 mg, 3.83 mmol, 1.0 eq) was dissolved in 18 mL EA, and (COCl)₂ (oxalyl chloride) (2.43 g, 19.13 mmol, 5.0 eq) and N,N-dimethylformamide (1.8 ml) were added. The reaction was carried out at 85°C for 2 h. After the reaction was complete as monitored by TLC and LCMS, the reaction mixture was rapidly evaporated to dryness with a rotary evaporator, and sealed for direct use in the following step.

### Step II: preparation of t-butyl trans-4-(3,4-dihydroisoquinolin-2(1H)-yl)-3-hydroxypiperidine-1-carboxylate:

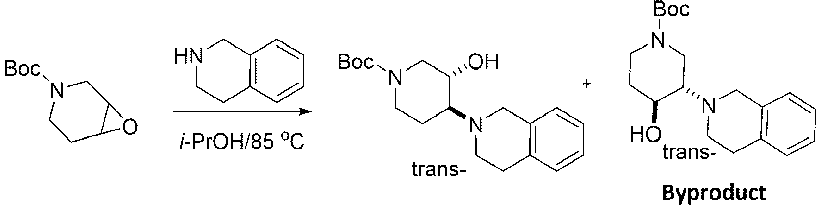

The compound t-butyl 7-oxa-3-azabicyclo[4.1.0]heptane-3-carboxylate (2.50 g, 12.56 mmol, 1.0 eq) was dissolved in *i*-PrOH (isopropanol, 63 mL), followed by addition of the compound 1,2,3,4-tetrahydroisoquinoline (1.67 g, 12.56 mmol, 1.0 eq), and the reaction was conducted under nitrogen protection at 85°C for 18 h. After the reaction was complete as monitored by TLC and LCMS, the reaction mixture was rotary evaporated for solvent removal, followed by addition of water (200 ml), extracted with dichloromethane (3 times, 200 ml for each), dried over anhydrous sodium sulfate, suction filtrated and rotary evaporated to dryness. In this step, regioisomers including the compund t-butyl trans-4-(3,4-dihydroisoquinolin-2(1*H*)-yl)-3-hydroxypiperidine-1-carboxylate and the compound t-butyl trans-3-(3,4-dihydroisoquinolin-2(1H)-yl)-4-hydroxypiperidine-1-carboxylate were present as a crude mixture product (3.40 g, yield: 81.5%). The crude product was seperated 2 to 3 times by chromatography (silica gel, ethyl acetate: petroleum ether = 15:85) and purified to obtain 1.7 g t-butyl trans-4-(3,4-dihydroisoquinolin-2(1*H*)-yl)-3-hydroxypiperidine-1-carboxylate, yield: 41%. LCMS (ESI) [M+H]⁺ = 333.3; ¹H NMR (400 MHz, CDCl₃) δ 7.20 - 7.08 (m, 3H), 7.05 - 6.98 (m, 1H), 4.59 - 4.17 (m, 2H), 3.94 (d, *J* = 14.6 Hz, 1H), 3.68 (d, *J* = 14.5 Hz, 2H), 3.54 (td, *J* = 10.0, 5.0 Hz, 1H), 3.03 (dt, *J* = 10.9, 5.3 Hz, 1H), 2.91 (t, *J* = 5.6 Hz, 2H), 2.80 - 2.47 (m, 4H), 1.82 (dd, *J* = 12.7, 2.5 Hz, 1H), 1.59 - 1.38 (m, 10H).

### Step III: preparation of trans-4-(3,4-dihydroisoquinolin-2(1H)-yl)piperidin-3-ol:

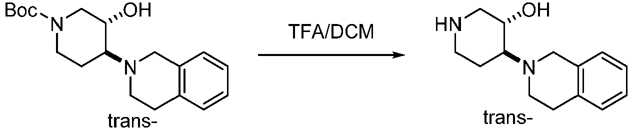

The compound t-butyl trans-4-(3,4-dihydroisoquinolin-2(1*H*)-yl)-3-hydroxypiperidine-1-carboxylate (1.00 g, 3.01 mmol, 1.0 eq) was dissolved in DCM (dichloromethane) (15mL) and TFA (trifluoroacetic acid) (3.75 mL). The reaction was carried out under stirring at room temperature (20-25°C) for 2 h. After the reaction was complete as monitored by TLC and LCMS, the reaction mixture was evaporated to dryness with a rotary evaporator, and 1,2-dichloroethane was added to remove extra TFA (repeated 3 times). It was dried upon rotary evaporation and sealed, which was directly used in Step IV LCMS (ESI) [M+H]⁺ = 233.2.

### Step IV: preparation of trans-(6-chloropyrimidin-4-yl)(4-(3,4-dihydroisoquinolin-2(1H)-yl)-3-hydroxypiperidin-1-yl)ketone:

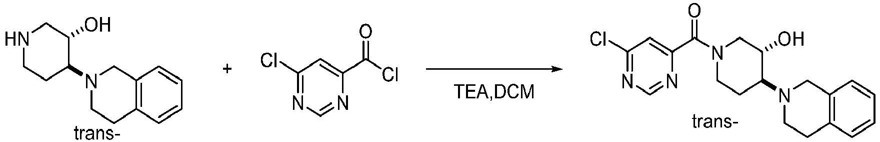

The compound trans-4-(3,4-dihydroisoquinolin-2(1*H*)-yl)piperidin-3-ol (570 mg, 3.23 mmol, 1.0 eq) was dissolved in DCM (dichloromethane) (8 ml) and TEA (triethylamine) (653 mg, 6.46 mmol, 2.0 eq), and the compound 6-chloropyrimidine-4-carbonyl chloride was dissolved in dichloromethane (8 ml). They were slowly added under nitrogen protection to a reaction mixture of the compound trans-4-(3,4-dihydroisoquinolin-2(1*H*)-yl)piperidin-3-ol. The reaction mixture was allowed to react in an ice bath for 2 h, and slowly warmed to room temperature (20-25°C) and reacted for 1 h. After the reaction was complete as monitored by TLC and LCMS, 100 mL water was added to the reaction mixture, which was extracted with ethyl acetate, and dried over anhydrous sodium sulfate. The organic phase was concentrated, and seperated and purified by reverse phase HPLC (C18, 0.08% aqueous NH₄HCO₃, acetonitrile) to obtain 900 mg of the compound trans-(6-chloropyrimidin-4-yl)(4-(3,4-dihydroisoquinolin-2(1*H*)-yl)-3-hydroxypiperidin-1-yl) ketone (900 mg, yield: 56.3%). LCMS (ESI) [M+H]⁺ = 373.

### Step V: preparation of trans-1-(4-((6-(4-(3,4-dihydroisoquinolin-2(1H)-yl)-3-hydroxypiperidine-1-carbonyl)pyrimidin-4-yl)amino)piperidin-1-yl)ethanone:

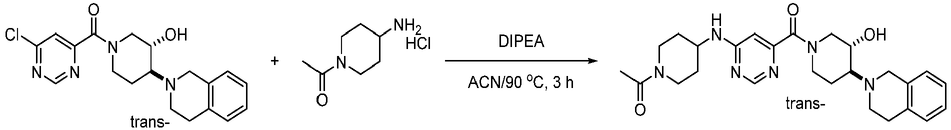

Trans-(6-chloropyrimidin-4-yl)(4-(3,4-dihydroisoquinolin-2(1*H*)-yl)-3-hydroxypiperidin-1-yl )ketone (110 mg, 0.30 mmol, 1.0 eq) and 1-acetylpiperidin-4-amine hydrochloride (64 mg, 0.36 mmol, 1.2 eq) were dissolved in ACN (acetonitrile) (2 ml), and DIPEA (*N,N*-diisopropylethylamine) (153 mg, 1.18 mmol, 3.9 eq) was added under nitrogen protection. The reaction mixture was allowed to react at 90°C for 3 h, and slowly cooled to room temperature (20-25°C). After the reaction was complete, 20 mL water was added to the reaction mixture, which was extracted with ethyl acetate, and dried over anhydrous sodium sulfate. The organic phase was concentrated, and seperated and purified by preparative HPLC (C18, 0.08% aqueous NH₄HCO₃, acetonitrile) to obtain trans-1-(4-((6-(4-(3,4-dihydroisoquinolin-2(1*H*)-yl)-3-hydroxypiperidine-1-carbonyl)pyrimid in-4-yl)amino)piperidin-1-yl)ethanone (17 mg, yield: 11.8%). ¹H NMR (400 MHz, CDCl₃) δ 8.54 (s, 1H), 7.20 - 7.07 (m, 3H), 7.06 - 6.96 (m, 1H), 6.70 - 6.57 (m, 1H), 5.50 - 5.30 (m, 1H), 4.82 (m, 1H), 4.55 (d, *J* = 13.6 Hz, 1H), 4.37 - 3.92 (m, 3H), 3.90 - 3.72 (m, 3H), 3.31 - 3.16 (m, 1.5H), 3.16 - 3.03 (m, 1H), 3.03 - 2.58 (m, 6.5H), 2.23 (d, *J* = 13.0 Hz, 0.5H), 2.16 - 1.97 (m, 5.5H), 1.72 - 1.51 (m, 1H), 1.49 - 1.34 (m, 2H); LCMS (ESI) [M+H]⁺ = 479.26.

### Example 18

### Preparation of cis-1-(4-((6-(4-(3,4-dihydroisoquinolin-2(1H-yl)-3-hydroxypiperidine-1-carbonyl)pyrimidin-4-yl)amino)piperidin-1-yl)ethanone

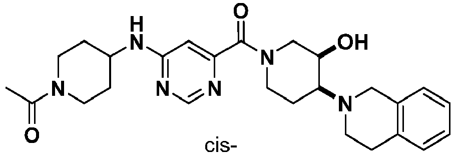

### Step I: preparation of t-butyl 4-(3,4-dihydroisoquinolin-2(1H)-yl)-3-oxypiperidine-1-carboxylate:

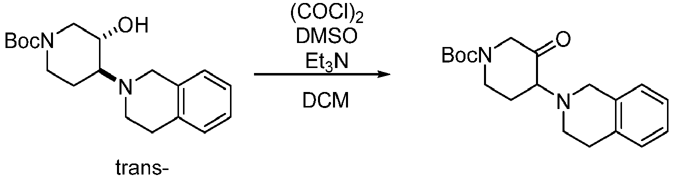

(COCl)₂ (oxalyl chloride) (688 mg, 5.40 mmol, 1.2 eq) was dissolved in DCM (dichloromethane) (8 mL) and cooled to -78°C. Under nitrogen protection, DMSO (dimethylsulfoxide) (387 mg, 4.95 mmol, 1.1 eq) was dissolved in DCM (8 mL), and slowly added dropwise to the above reaction mixture, and then stirred at -78°C for 30 min. Then, a solution of the compound t-butyl trans-4-(3,4-dihydroisoquinolin-2(1*H*)-yl)-3-hydroxy-piperidine-1-carboxylate (1.5 g, 4.5 mmol, 1.0 eq) in DCM (8 mL) was slowly added to the reaction mixture and stirred for 30 min. Thereafter, Et₃N (triethylamine) (2.27 g, 22.5 mmol, 5.0 eq) was added and stirred for 30 min, and the mixture was naturally warmed to room temperature (15°C). The reaction mixture was quenched with saturated ammonium chloride solution and extracted three times with dichloromethane (15 mL for each). The dichloromethane phases were combined, washed once with 20 mL water and once with 20 mL saturated sodium chloride solution, dried over anhydrous sodium sulfate for 10 min, filtrated, and concentrated. The crude product was separated and purified by flash chromatography (silica gel, DCM: MeOH = 49: 1) to obtain the target compound (1.00 g, yield: 67.1%) as a yellow oily product. LCMS (ESI) [M+H]⁺ = 331.

### Step II: preparation of t-butyl cis-4-(3,4-dihydroisoquinolin-2(1H)-yl)-3-hydroxypiperidine-1-carboxylate

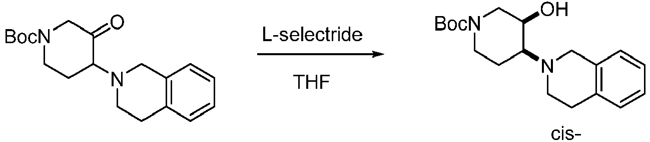

The compound t-butyl 4-(3,4-dihydroisoquinolin-2(1*H*)-yl)-3-oxypiperidine-1-carboxylate (1.00 g, 3.00 mmol, 1.0 eq) was dissolved in THF (tetrahydrofuran) (15 mL) and cooled to -78°C. Under nitrogen protection, a solution of L-selectride (lithium tri-sec-butylborohydride) in tetrahydrofuran (1M, 3.7 mL, 3.70 mmol, 1.2 eq) was slowly added dropwise to the reaction mixture, and then stirred at -78°C for 3 h. The reaction mixture was quenched with saturated ammonium chloride solution and extracted three times with ethyl acetate (15 mL each). The ethyl acetate phases were combined, washed once with 20 mL water and once with 20 mL saturated sodium chloride solution, dried over anhydrous sodium sulfate for 10 min, filtrated, and concentrated. The crude product was separated and purified by flash chromatography (silica gel, DCM: MeOH = 49: 1) to obtain the target intermediate (220 mg, yield: 21.9%) as a yellow oily product. LCMS (ESI) [M+H]⁺ = 333.18; ¹H NMR (400 MHz, CDCl₃) δ 7.15 (dd, *J* = 6.3, 3.2 Hz, 2H), 7.13 - 7.09 (m, 1H), 7.02 (dd, *J* = 9.6, 7.2 Hz, 1H), 5.30 (s, 1H), 4.12 (m, 1H), 3.89 (s, 2H), 3.49 (s, 4H), 3.11 - 3.01 (m, 1H), 2.92 (s, 3H), 2.66 (s, 1H), 2.50 (s, 1H), 2.03 (d, *J* = 10.6 Hz, 1H), 1.47 (s, 9H).

### Step III: preparation of cis-4-(3,4-dihydroisoquinolin-2(1H)-yl)-3-hydroxypiperidin-3-ol:

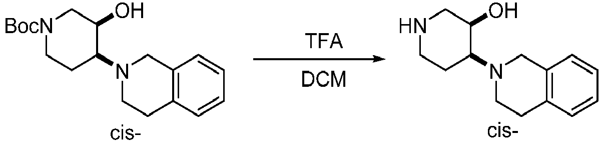

TFA (trifluoroacetic acid) (0.25 mL) was slowly added dropwise to a solution of the compound t-butyl cis-4-(3,4-dihydroisoquinolin-2(1*H*)-yl)-3-hydroxypiperidine-1-carboxylate (200 mg, 0.60 mmol, 1.0 eq) in DCM (dichloromethane) (1 mL), and then stirred at room temperature (15°C) for 1 h. The reaction mixture was adjusted to basic with saturated sodium bicarbonate solution and extracted three times with dichloromethane (5 mL for each). The dichloromethane phases were combined, washed once with 5 mL water and once with 5 mL saturated sodium chloride solution, dried over anhydrous sodium sulfate for 10 min, filtrated, and concentrated. The crude product was separated and purified by flash chromatography (silica gel, DCM: MeOH = 49: 1) to obtain the target intermediate (110 mg, yield: 79.1%) as a yellow oily product. LCMS (ESI) [M+H]⁺ =233.04.

### Step IV: preparation of cis-1-(4-((6-(4-(3,4-dihydroisoquinolin-2(1H)-yl)-3-hydroxypiperidine-1-carbonyl)pyrimidin-4-yl)amino)piperidin-1-yl)ethan-1-one:

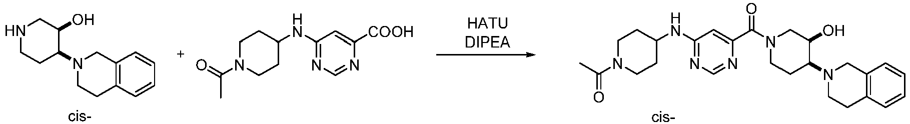

After the compounds cis-4-(3,4-dihydroisoquinolin-2(1*H*)-yl)-3-hydroxypiperidin-3-ol (51 mg, 0.22 mmol, 1.0 eq), 6-((1-acetylpiperidin-4-yl)amino)pyrimidine-4-carboxylic acid (58 mg, 0.22 mmol, 1.0 eq), and HATU (2-(7-azabenzotriazole)-N,N,N',N'-tetramethyluronium hexafluorophosphate) (125 mg, 0.33 mmol, 1.5 eq) were dissolved in DMF (*N,N-*dimethylformamide) (1.5 mL), DIPEA (*N,N-*diisopropylethylamine) (85 mg, 0.66 mmol, 3.0 eq) was added and stirred at room temperature (10-15°C) for 1 h. The reaction mixture was poured into 10 mL water and extracted three times with ethyl acetate (6 mL for each). The ethyl acetate phases were combined, washed once with 10 mL water and once with 10 mL saturated sodium chloride solution, dried over anhydrous sodium sulfate for 5 min, filtrated, and concentrated. The crude product was separated and purified by preparative HPLC (C18, 10 mmol/L aqueous NH₄HCO₃, acetonitrile) to obtain the target compound (12 mg, yield: 11.4%). LCMS (ESI) [M+H]⁺ = 479.27; ¹H NMR (400 MHz, DMSO-d₆) δ 8.63 (d, *J* = 5.3 Hz, 1H), 8.38 (s, 1H), 7.88 (s, 1H), 7.43 (t, *J* = 5.5 Hz, 1H), 7.18 (d, *J* = 6.0 Hz, 1H), 7.12 - 7.06 (m, 3H), 7.04 - 6.97 (m, 1H), 5.22 - 5.09 (m, 1H), 5.09 (s, 1H), 4.86 (t, *J* = 6.9 Hz, 2H), 4.65 (t, *J* = 6.4 Hz, 2H), 4.09 - 3.89 (m, 1H), 3.73 - 3.55 (m, 3H), 3.49 - 3.38 (m, 1H), 2.84 (t, *J* = 5.4 Hz, 2H), 2.76 (dt, *J* = 11.2, 5.5 Hz, 1H), 2.68 (dd, *J* = 11.3, 6.1 Hz, 1H), 2.58 - 2.52 (m, 2H).

### Example 19

### Preparation of cis-(4-(3,4-dihydroisoquinolin-2(1H)-yl)-3-hydroxypiperidin-1-yl)(6-(oxetan-3-ylamino)pyrimidin-4-yl)ketone:

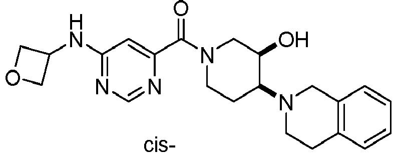

Step I: preparation of cis-(4-(3,4-dihydroisoquinolin-2(1H)-yl)-3-hydroxypiperidin-1-yl)(6-(oxetan-3-ylamino)pyrimidin-4-yl)ketone:
After the compounds cis-4-(3,4-dihydroisoquinolin-2(1*H*)-yl)-3-hydroxypiperidin-3-ol (60 mg, 0.258 mmol, 1.0 eq), 6-(oxetan-3-ylamino)pyrimidine-4-carboxylic acid (50 mg, 0.258 mmol, 1.0 eq), and HATU (2-(7-azabenzotriazole)-N,N,N',N'-tetramethyluronium hexafluorophosphate) (147 mg, 0.387 mmol, 1.5 eq) were dissolved in DMF (*N,N*-dimethylformamide) (2 mL), DIPEA (*N,N*-diisopropylethylamine) (100 mg, 0.774 mmol, 3 eq) was added and then stirred at room temperature (10-15°C) for 1 h. The reaction mixture was poured into 10 mL water and extracted three times with ethyl acetate (6 mL for each). The ethyl acetate phases were combined, washed once with 10 mL water and once with 10 mL saturated sodium chloride solution, dried over anhydrous sodium sulfate for 5 min, filtrated and concentrated, and purified by preparative HPLC to obtain 9 mg target compound, yield: 8.57%. LCMS: [M+H] = 410.16; ¹H NMR (400 MHz, CDCl₃) δ 8.53 (d, *J* = 4.3 Hz, 1H), 7.18 - 7.07 (m, 3H), 7.02 (d, *J* = 5.9 Hz, 1H), 6.75 (d, *J* = 15.1 Hz, 1H), 6.12 (m , 1H), 5.00 (m, 3H), 4.77 (d, *J* = 13.3 Hz, 1H), 4.56 (t, *J* = 6.1 Hz, 2H), 4.25 - 4.07 (m, 2H), 3.95 - 3.75 (m, 2H), 3.17 (d, *J* = 14.0 Hz, 1H), 3.00 - 2.94 (m, 1H), 2.89 (dd, *J* = 11.3, 5.6 Hz, 2H), 2.75 (td, *J* = 13.0, 2.7 Hz, 1H), 2.56 (dd, *J* = 8.4, 3.2 Hz, 1H), 2.17 - 1.83 (m, 2H), 1.72 (m, 2H).

### Example 20

### Preparation of trans-N-(1-acetylpiperidin-4-yl)-2-(4-(3,4-dihydroisoquinolin-2(1H)-yl) -3-hydroxypiperidin-1-yl)thiazole-5-carboxamide:

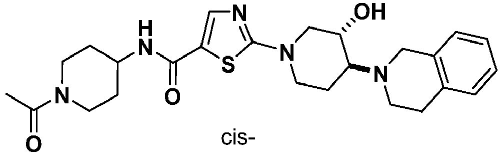

### Step I: preparation of N-(1-acetylpiperidin-4-yl)-2-chlorothiazole-5-carboxamide:

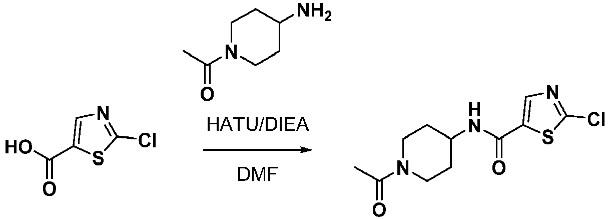

2-chlorothiazole-5-carboxylic acid (200 mg, 1.23 mmol, 1.0 eq), 1-(4-aminopiperidin-1-yl)ethan-1-one (218 mg, 1.23 mmol, 1.0 eq) and HATU (2-(7-azabenzotriazole)-N,N,N',N'-tetramethyluronium hexafluorophosphate) (699 mg, 1.85 mmol, 1.5 eq) were dissolved in DMF (*N,N*-dimethylformamide) (1 mL), DIEA (*N,N-*diisopropylethylamine) (475 mg, 3.69 mmol, 3.0 eq) was added, and the reaction mixture was allowed to react under nitrogen protection at room temperature (20-25°C) for 4 h. After the reaction was completed as indicated by TLC and LCMS, the reaction mixture was poured into 50 mL water and extracted three times with ethyl acetate (50 mL for each). The ethyl acetate phases were combined, washed once with 30 mL water and once with 30 mL saturated sodium chloride solution, dried over anhydrous sodium sulfate for 10 min, filtrated, and concentrated. The crude product was separated and purified by preparative HPLC (C18, 0.08% aqueous NH₄HCO₃, acetonitrile) to obtain *N*-(1-acetylpiperidin-4-yl)-2-chlorothiazole-5-carboxamide (180 mg, yield: 51.0%). LCMS(ESI)[M+1]⁺ = 288.18.

### Step II: preparation of trans-N-(1-acetylpiperidin-4-yl)-2-(4-(3,4-dihydroisoquinolin-2(1H)-yl)-3-hydroxypiperidin-1-yl)thiazole-5-carboxamide:

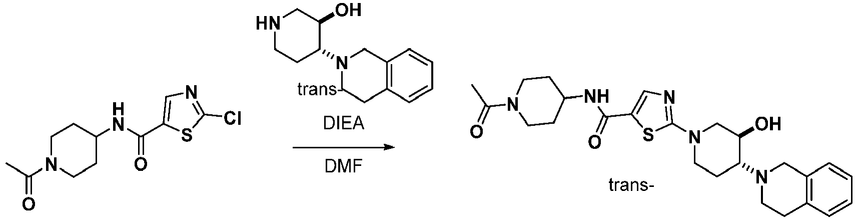

The compound *N*-(1-acetylpiperidin-4-yl)-2-chlorothiazole-5-carboxamide (40 mg, 0.14 mmol, 1.0 eq) was dissolved in DMF (N,N-dimethylformamide) (0.7 mL), and under nitrogen protection, trans-4-(3,4-dihydroisoquinolin-2(1*H*)-yl)piperidin-3-ol (39 mg, 0.17 mmol, 1.2 eq) and DIEA (*N,N*-diisopropylethylamine) (0.05 mL, 0.28 mmol, 2.0 eq) were added. Under nitrogen protection, the reaction mixture was allowed to react at 60°C overnight. After the reaction was completed as indicated by TLC and LCMS, 30 mL water was added, and the reaction mixture was extracted three times with ethyl acetate (10 mL each), dried over anhydrous sodium sulfate, filtrated, and concentrated. The crude product was separated and purified by preparative HPLC (C18, 0.08% aqueous NH₄HCO₃, acetonitrile) to obtain trans-*N*-(1-acetylpiperidin-4-yl)-2-(4-(3,4-dihydroisoquinolin-2(1*H*-yl)-3-hydroxypiperidin-1-yl)thiazole-5-carboxamide (23.69 mg, yield: 35.3%). LCMS(ESI)[M+1]⁺ = 484.20; ¹H NMR (400 MHz, CDCL₃) δ 7.60 (s, 1H), 7.20 - 7.10 (m, 3H), 7.07 - 7.01 (m, 1H), 5.77 (d, J = 7.8 Hz, 1H), 4.60 (d, J = 13.6 Hz, 1H), 4.30 (dt, J = 15.1, 7.7 Hz, 2H), 4.19 - 4.10 (m, 1H), 4.01 (d, J = 14.6 Hz, 1H), 3.85 - 3.71 (m, 3H), 3.24 - 3.14 (m, 1H), 3.07 (td, J = 12.8, 12.1, 4.3 Hz, 2H), 2.97 (dd, J = 13.7, 9.0 Hz, 3H), 2.80 - 2.67 (m, 3H), 2.11 (s, 3H), 2.04 - 1.94 (m, 3H), 1.55 (dqd, J = 120.3, 12.1, 11.6, 4.3 Hz, 4H).

### Example 21

### Preparation of trans-4-(3,4-dihydroisoquinolin-2(1H)-yl)-1-((6-phenylpyridin-2-yl)methyl)piperidin-3-ol:

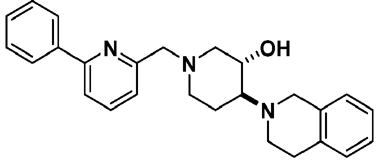

### Step I: preparation of trans-4-(3,4-dihydroisoquinolin-2(1H)-yl)-1-((6-phenylpyridin-2-yl)methyl)piperidin-3-ol:

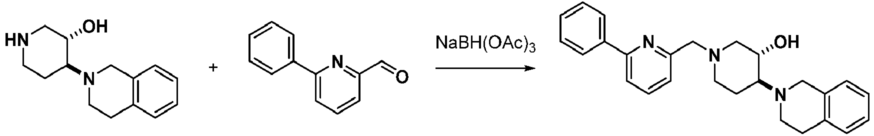

Trans-4-(3,4-dihydroisoquinolin-2(1*H*)-yl)piperidin-3-ol (50 mg, 0.215 mmol, 1.0 eq) and 6-phenylpyridinecarbaldehyde (59 mg, 0.323 mmol, 1.5 eq) were dissolved in dry tetrahydrofuran (1 ml) and stirred at room temperature for 40 min, and then sodium acetate borohydride (137 mg, 0.646 mmol, 3.0 eq) was added. The reaction was conducted at room temperature (20-25°C) for 1 h, and then quenched by adding water. After extraction with ethyl acetate, the organic phase was rotary evaporated to dryness, and purified by preparative HPLC to obtain 45 mg trans-4-(3,4-dihydroisoquinolin-2(1*H*)-yl)-1-((6-phenylpyridin-2-yl)methyl)piperidin-3-ol, yield: 52.3%. LCMS: [M+1]+ = 400.26; ¹H NMR (400 MHz, CDCl₃) δ 8.03 - 7.96 (m, 2H), 7.72 (t, *J* = 7.7 Hz, 1H), 7.60 (dd, *J* = 7.8, 1.1 Hz, 1H), 7.50 - 7.44 (m, 2H), 7.43 - 7.36 (m, 2H), 7.17 - 7.09 (m, 3H), 7.05 - 6.99 (m, 1H), 3.96 (d, *J* = 14.6 Hz, 1H), 3.89 - 3.73 (m, 3H), 3.69 (d, *J* = 14.6 Hz, 1H), 3.54 (s, 1H), 3.34 (ddd, *J* = 10.5, 4.6, 2.1 Hz, 1H), 3.14 - 3.02 (m, 2H), 2.91 (t, *J=* 5.8 Hz, 2H), 2.66 (dt, *J* = 11.9, 6.2 Hz, 1H), 2.53 - 2.41 (m, 1H), 2.24 (t, *J* = 11.3 Hz, 1H), 2.12 (t, *J* = 10.1 Hz, 1H), 1.83 (dd, *J* = 12.3, 3.4 Hz, 1H), 1.70 (qd, *J* = 12.0, 3.9 Hz, 1H).

### Example 22

### Preparation of trans-4-(3,4-dihydroisoquinolin-2(1H)-yl)-1-((1-methyl-4-phenyl-1H-imidazol-2-yl)methyl)piperidin-3-ol:

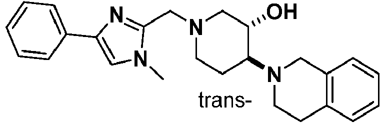

### Step I: preparation of trans-4-(3,4-dihydroisoquinolin-2(1H)-yl)-1-((1-methyl-4-phenyl-1H-imidazol-2-yl)methyl)piperidin-3-ol:

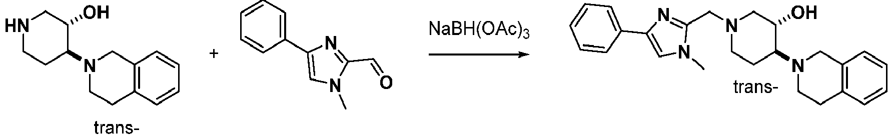

Trans-4-(3,4-dihydroisoquinolin-2(1*H*)-yl)piperidin-3-ol (50 mg, 0.215 mmol, 1.0 eq) and 1-methyl-4-phenyl-1*H*-imidazole-2-carbaldehyde (60 mg, 0.323 mmol, 1.5 eq) was dissolved in dry tetrahydrofuran (1 ml) and stirred at room temperature (20-25°C) for 40 min, and then sodium acetate borohydride (137 mg, 0.646 mmol, 3.0 eq) was added. The reaction was conducted at room temperature for 1 h, and then quenched by adding water. After extraction with ethyl acetate, the organic phase was rotary evaporated to dryness, and purified by preparative HPLC to obtain 38.3 mg trans-4-(3,4-dihydroisoquinolin-2(1*H*)-yl)-1-((1-methyl-4-phenyl-1H-imidazol-2-yl)methyl)piperidin-3-ol, yield: 44.2%. LCMS: [M+1]⁺ = 403.27; ¹H NMR (600 MHz, CDCl₃) δ 7.75 - 7.70 (m, 2H), 7.37 - 7.33 (m, 2H), 7.21 (tt, *J* = 7.2, 1.2 Hz, 1H), 7.16 - 7.09 (m, 4H), 7.02 (dd, *J* = 7.0, 1.9 Hz, 1H), 3.93 (d, *J* = 14.6 Hz, 1H), 3.74 (s, 3H), 3.73 - 3.66 (m, 4H), 3.58 (brs, 1H), 3.24 (ddd, *J* = 10.7, 4.6, 2.1 Hz, 1H), 3.04 (dt, *J* = 11.2, 5.4 Hz, 1H), 2.96 (ddt, *J* = 11.5, 4.5, 2.5 Hz, 1H), 2.90 (t, *J* = 5.9 Hz, 2H), 2.66 (dt, *J* = 11.8, 6.1 Hz, 1H), 2.50 - 2.44 (m, 1H), 2.17 (td, *J* = 11.8, 2.6 Hz, 1H), 2.10 (t, *J* = 10.1 Hz, 1H), 1.80 (dq, *J* = 12.7, 3.0 Hz, 1H), 1.57 (qd, *J* = 12.2, 4.1 Hz, 1H).

### Example 23

### Preparation of trans-1-(4-((6-((3,4-dihydroisoquinolin-2(1H)-yl)-3-hydroxypiperidin-1-yl)pyrimidin-4-yl)amino)piperidin-1-yl)ethanone:

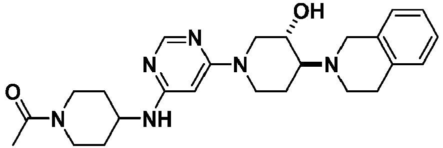

### Step I: preparation of 1-(4-((6-chloropyrimidin-4-yl)amino)piperidin-1-yl)ethanone:

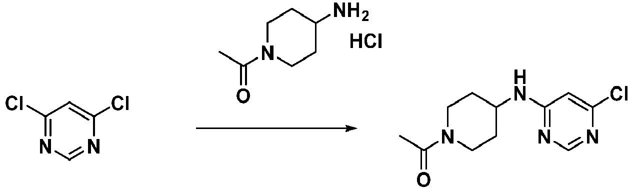

4,6-dichloropyrimidine (500 mg, 3.356 mmol, 1.0 eq) and 1-(4-aminopiperidin-1-yl)ethanone hydrochloride (600 mg, 3.356 mmol, 1.0 eq) was dissolved in isopropanol (15 ml), N,N-diisopropylethylamine (1.3 g, 10.068 mmol, 3.0 eq) was added, and the mixture was stirred at 100°C for 1 h. The reaction was complete as monitored by LCMS. After drying the solvent by rotary evaporation and purification with a normal phase silica gel column (0-3% methanol/dichloromethane), 850 mg 1-(4-((6-chloropyrimidin-4-yl)amino)piperidin-1-yl)ethanone was obtained. LCMS: [M+1]⁺ = 345.10.

### Step II: preparation of trans-1-(4-((6-((3,4-dihydroisoquinolin-2(1H)-yl)-3-hydroxypiperidin-1-yl)pyrimidin-4-yl)amino)piperidin-1-yl)ethanone:

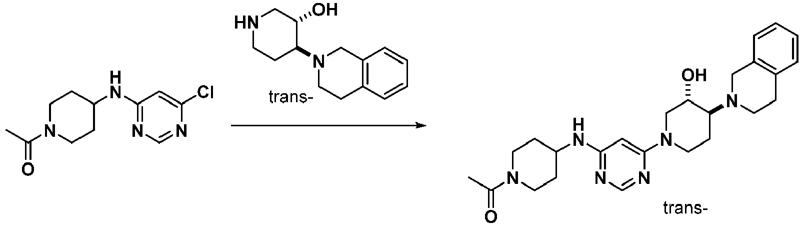

1-(4-((6-chloropyrimidin-4-yl)amino)piperidin-1-yl)ethanone (50 mg, 0.197 mmol, 1.0 eq) and trans-4-(3,4-dihydroisoquinolin-2(1H)-yl)piperidin-3-ol (46 mg, 0.197 mmol, 1.0 eq) were dissolved in isopropanol (1 mL), followed by addition of N,N-diisopropylethylamine (51 mg, 0.394 mmol, 2.0 eq), and heated under stirring at 100°C overnight (16 h). After drying the solvent by rotary evaporation and preparative HPLC, 11 mg trans-1-(4-((6-((3,4-dihydroisoquinolin-2(1*H*)-yl)-3-hydroxypiperidin-1-yl)-pyrimidin-4-yl)-amino)piperidin-1-yl)ethanone was obtained, yield: 12.4%. LCMS: [M+1]+ = 451.31; ¹H NMR (400 MHz, CDCl₃) δ 8.18 (d, *J* = 0.9 Hz, 1H), 7.19 - 7.09 (m, 3H), 7.06 - 6.98 (m, 1H), 5.51 (d, *J* = 1.1 Hz, 1H), 4.76 (d, *J* = 13.4 Hz, 1H), 4.57 - 4.38 (m, 3H), 3.94 (d, *J* = 14.6 Hz, 1H), 3.90 - 3.77 (m, 2H), 3.77 - 3.65 (m, 2H), 3.59 (td, *J* = 10.0, 4.8 Hz, 1H), 3.24 (dd, *J* = 14.0, 11.2 Hz, 1H), 3.02 (dt, *J* = 11.0, 5.3 Hz, 1H), 2.91 (t, *J* = 5.8 Hz, 2H), 2.89 - 2.83 (m, 1H), 2.81 - 2.62 (m, 4H), 2.20 - 1.99 (m, 5H), 1.91 (dd, *J* = 12.9, 3.4 Hz, 1H), 1.63 - 1.50 (m, 1H), 1.45 - 1.33 (m, 2H).

### Example 24

### Preparation of trans-4-(3,4-dihydroisoquinolin-2(1H)-yl)-1-(6-(phenylamino)-pyrimidin-4-yl)piperidin-3-ol:

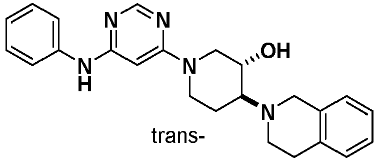

### Step I: preparation of trans-1-(6-chloropyrimidin-4-yl)-4-(3,4-dihydroisoquinolin-2(1H)-yl)piperidin-3-ol:

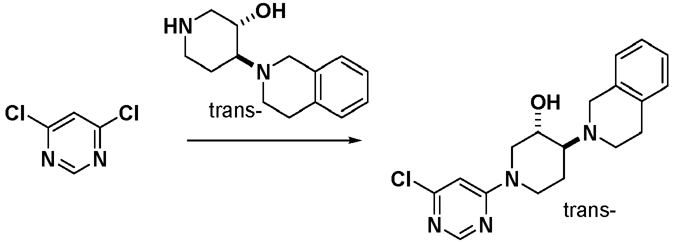

4,6-dichloropyrimidine (150 mg, 1.007 mmol, 1.5 eq) and trans-4-(3,4-dihydroisoquinolin-2(1H)-yl)piperidin-3-ol (156 mg, 0.671 mmol, 1.0 eq) were dissolved in isopropanol (3.5 ml), followed by addition of N,N-diisopropylethylamine (173 mg, 1.342 mmol, 2.0 eq), and the mixture was stirred at 100°C for 30 min. The reaction was complete as monitored by LCMS. After drying the solvent by rotary evaporation and purification with a normal phase silica gel column (0-3 vol% methanol/dichloromethane), 187 mg trans-1-(6-chloropyrimidin-4-yl)-4-(3,4-dihydroisoquinolin-2(1*H*)-yl)piperidin-3-ol was obtained, yield: 54.0%. LCMS: [M+1]⁺ = 345.10.

### Step II: preparation of trans-4-(3,4-dihydroisoquinolin-2(1H)-yl)-1-(6-(phenylamino)-pyrimidin-4-yl)piperidin-3-ol:

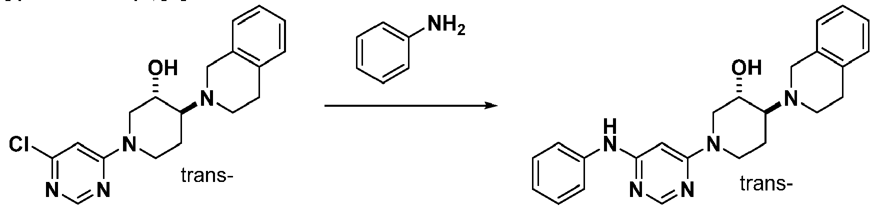

(3 S,4S)-1-(6-chloropyrimidin-4-yl)-4-(3,4-dihydroisoquinolin-2(1H)-yl)piperidin-3-ol (racemic) (113 mg, 0.328 mmol, 1.0 eq) was dissolved in isopropanol (2 ml), followed by addition of phenylamine (46 mg, 0.493 mmol, 1.5 eq) and concentrated hydrochloric acid (36wt%, 0.2 ml), and the mixture was heated under stirring at 100°C overnight (12h). 1N aqueous sodium hydroxide was added to adjust to pH 7, followed by drying the solvent by rotary evaporation and reverse phase preparative HPLC, to obtain 38.7 mg trans-4-(3,4-dihydroisoquinolin-2(1*H*)-yl)-1-(6-(phenylamino)pyrimidin-4-yl)piperidin-3-ol, yield: 29.4%. LCMS: [M+1]+ = 403.27; ¹H NMR (600 MHz, CDCl₃) δ 8.26 (d, *J* = 1.0 Hz, 1H), 7.41 - 7.35 (m, 2H), 7.30 - 7.26 (m, 2H), 7.17 - 7.09 (m, 4H), 7.04 - 7.00 (m, 1H), 6.87 - 6.77 (m, 1H), 6.00 (d, *J* = 1.1 Hz, 1H), 4.71 (d, *J* = 13.4 Hz, 1H), 4.48 - 4.38 (m, 1H), 3.93 (d, *J* = 14.5 Hz, 1H), 3.74 - 3.64 (m, 2H), 3.57 (td, *J* = 10.0, 4.9 Hz, 1H), 3.01 (dt, *J* = 11.1, 5.4 Hz, 1H), 2.90 (t, *J* = 5.8 Hz, 2H), 2.77 (td, *J* = 12.9, 2.6 Hz, 1H), 2.71 (dd, *J* = 12.7, 10.2 Hz, 1H), 2.68 - 2.62 (m, 2H), 1.90 (dq, *J* = 13.0, 2.8 Hz, 1H), 1.54 (qd, *J* = 12.5, 4.4 Hz, 1H).

### Example 25

### Preparation of trans-1-(4-((5-(4-(3,4-dihydroisoquinolin-2(1H)-yl)-3-hydroxypiperidin-1-carbonyl)thiazol-2-yl)amino)piperidin-1-yl)ethan-1-one:

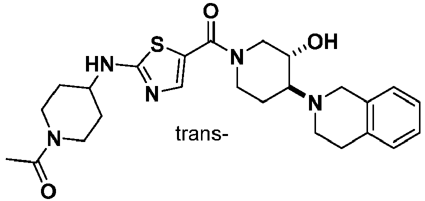

### Step I: preparation of trans-(2-chlorothiazol-5-yl)(4-(3,4-dihydroisoquinolin-2(1H)-yl)-3-hydroxypiperidin-1-yl)ket one:

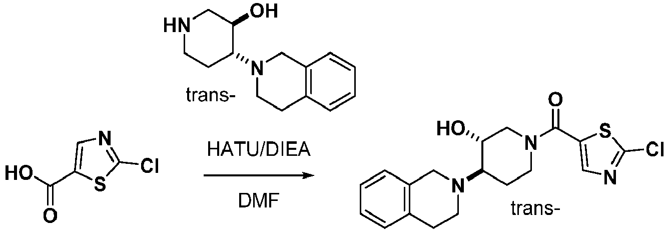

2-chlorothiazole-5-carboxylic acid (200 mg, 1.223 mmol, 1.0 eq), trans-4-(3,4-dihydroisoquinolin-2(1*H*)-yl)piperidin-3-ol (340 mg, 1.467 mmol, 1.2 eq) and HATU (2-(7-azabenzotriazole)-N,N,N',N'-tetramethyluronium hexafluorophosphate) (697 mg, 1.834 mmol, 1.5 eq) were dissolved in *N,N*-dimethylformamide (6.2 mL), followed by addition of N,N-diisopropylethylamine (1.06 mL, 6.113 mmol, 5.0 eq), and, under nitrogen protection, the reaction mixture was allowed to react at room temperature (18°C) overnight. After the reaction was complete as indicated by TLC and LCMS, the reaction mixture was poured into 50 mL water and extracted three times with ethyl acetate (50 mL for each). The ethyl acetate phases were combined, washed once with 30 mL water and once with 30 mL saturated sodium chloride solution, dried over anhydrous sodium sulfate for 10 min, filtrated and concentrated, and purified by column chromatography to obtain 300 mg trans-(2-chlorothiazol-5-yl)(4-(3,4-dihydroisoquinolin-2(1*H*)-yl)-3-hydroxypiperidin-1-yl)ket one, yield: 65.1%. LCMS: [M+H]⁺ = 378.07.

### Step II: preparation of trans-1-(4-((5-(4-(3,4-dihydroisoquinolin-2(1H)-yl)-3-hydroxypiperidine-1-carbonyl)thiazol-2-yl)amino)piperidin-1-yl)ethan-1-one:

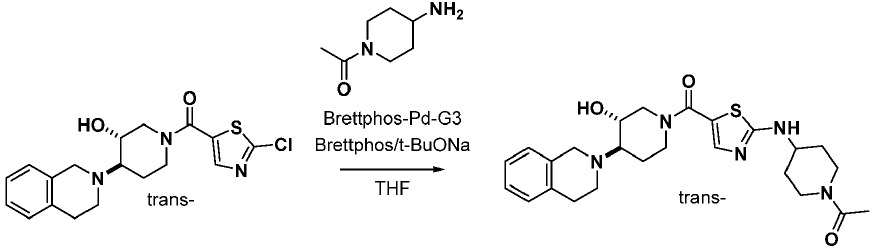

The compounds trans-(2-chlorothiazol-5-yl)(4-(3,4-dihydroisoquinolin-2(1*H*)-yl)-3-hydroxypiperidin-1-yl)ketone (60 mg, 0.159 mmol, 1.0 eq), 1-(4-aminopiperidin-1-yl)ethan-1-one (34 mg, 0.191 mmol, 1.2 eq), Brettphos (bicyclohexyl[3,6-dimethoxy-2',4',6'-triisopropyl[1,1'-biphenyl]-2-yl]phosphine) (8.6 mg, 0.016 mmol, 0.1 eq), and Brettphos-Pd-G3 ((2-bicyclohexylphosphino-3,6-dimethoxy-2',4',6'-triisopropyl-1,1'-biphenyl)(2-amino-1,1'-biphen-2-yl) palladium (II) methanesulfonate) (14.5 mg, 0.016 mmol, 0.1 eq) were dissolved in THF (tetrahydrofuran) (0.8 mL), followed by addition of t-BuONa (sodium t-butoxide) (46 mg, 0.477 mmol, 3.0 eq), and the temperature was raised to 60°C at which the reaction was allowed to proceed for 2.5 h. After the reaction was complete, the reaction mixture was poured into 10 mL water and extracted three times with ethyl acetate (5 mL for each). The ethyl acetate phases were combined, washed once with 10 mL saturated sodium chloride solution, dried over anhydrous sodium sulfate for 10 min, filtrated and concentrated to obtain a crude product which was subjected to preparative HPLC to obtain 2.1 mg of the target compound, yield: 2.7%. LCMS: [M+1]⁺ = 484.33; ¹H NMR (400 MHz, CDCl₃) δ 7.44 (s, 1H), 7.21 - 7.10 (m, 3H), 7.07 - 7.02 (m, 1H), 5.55 (brs, 1H), 4.73 (d, J = 13.1 Hz, 1H), 4.64 (d, J = 13.5 Hz, 1H), 4.50 (d, J = 13.7 Hz, 1H), 4.16 - 4.00 (m, 1H), 3.89 - 3.77 (m, 2H), 3.72 (s, 2H), 3.28 - 3.18 (m, 1H), 3.15 (s, 1H), 3.02 (s, 2H), 2.94 - 2.81 (m, 4H), 2.20 (d, J = 13.0 Hz, 1H), 2.12 (s, 4H), 1.99 (d, J = 12.7 Hz, 1H), 1.71 - 1.57 (m, 3H), 1.53 - 1.41 (m, 2H).

### Example 26

### Preparation of trans-1-(4-((2-(3-(3,4-dihydroisoquinolin-2(1H)-yl)-4-hydroxypiperidin-1-yl)pyrimidin-4-yl)amino)piperidin-1-yl)ethanone:

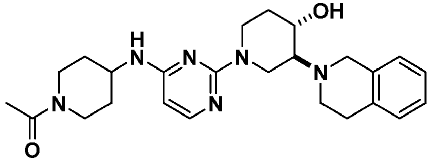

### Step I: preparation of 1-(4-((2-chloropyrimidin-4-yl)amino)piperidin-1-yl)ethanone:

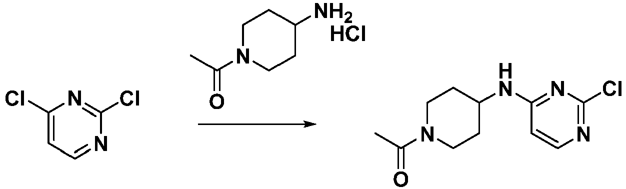

2,4-dichloropyrimidine (300 mg, 2.014 mmol, 1.0 eq) and 1-(4-aminopiperidin-1-yl)ethanone hydrochloride (395 mg, 2.215 mmol, 1.1 eq) were dissolved in acetonitrile (9 mL), followed by addition of N,N-diisopropylethylamine (780 mg, 6.042 mmol, 3.0 eq), and the reaction mixture was allowed to react at 90°C for 1 h. After the reaction was complete as indicated by LCMS, the reaction mixture was rotary evaporated to dryness and subjected to column chromatography to obtain 197 mg 1-(4-((2-chloropyrimidin-4-yl)amino)piperidin-1-yl)ethanone, yield: 38.5%. LCMS: [M+H]⁺ = 255.10, 257.10; ¹H NMR (400 MHz, CDCl₃) δ 8.00 (d, *J* = 5.8 Hz, 1H), 6.30 (d, *J* = 5.9 Hz, 1H), 5.37 (d, *J* = 7.5 Hz, 1H), 4.54 (d, *J* = 13.7 Hz, 1H), 4.06 (s, 1H), 3.90 - 3.78 (m, 1H), 3.25 (ddd, *J* = 14.2, 11.7, 2.9 Hz, 1H), 2.85 (t, *J* = 12.2 Hz, 1H), 2.19 - 2.08 (m, 4H), 2.08 - 1.99 (m, 1H), 1.47 - 1.35 (m, 2H).

### Step II: preparation of trans-1-(4-((2-(3-(3,4-dihydroisoquinolin-2(1H)-yl)-4-hydroxypiperidin-1-yl)pyrimidin-4-yl)amino)piperidin-1-yl)ethanone:

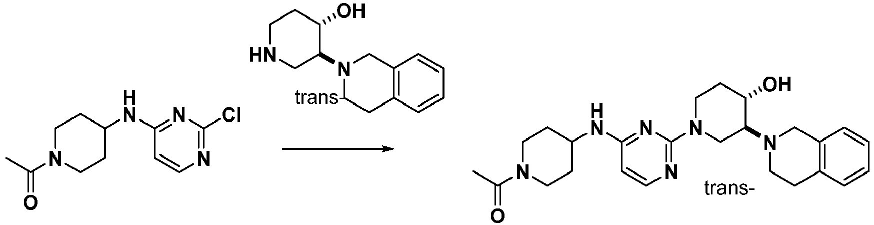

1-(4-((2-chloropyrimidin-4-yl)amino)piperidin-1-yl)ethanone (30 mg, 0.118 mmol, 1.0 eq) and trans-3-(3,4-dihydroisoquinolin-2(1*H*)-yl)piperidin-4-ol (28 mg, 0.118 mmol, 1.0 eq) were dissolved in isopropanol (1 mL), followed by addition of N,N-diisopropylethylamine (31 mg, 0.236 mmol, 2.0 eq), and the reaction mixture was allowed to react at 100°C for 22 h. The reaction mixture was rotary evaporated to dryness and subjected to reverse phase preparative HPLC to obtain 18 mg trans-1-(4-((2-((3-(3,4-dihydroisoquinolin-2(1*H*)-yl)-4-hydroxypiperidin-1-yl)pyrimidin-4-yl)amino)piperidin-1-yl)ethanone, yield: 33.9%. LCMS: [M+1]⁺ = 451.3; ¹H NMR (400 MHz, CDCl₃) δ 7.88 (d, *J* = 5.8 Hz, 1H), 7.18 - 7.09 (m, 3H), 7.05 - 6.99 (m, 1H), 5.67 (d, *J* = 5.8 Hz, 1H), 5.12 (dd, *J* = 12.7, 4.9 Hz, 1H), 4.90 (d, *J* = 13.3 Hz, 1H), 4.56 - 4.43 (m, 1H), 4.07 - 3.90 (m, 2H), 3.86 - 3.76 (m, 1H), 3.76 - 3.68 (m, 2H), 3.59 (tt, *J* = 9.7, 4.7 Hz, 1H), 3.29 - 3.18 (m, 1H), 3.04 (dt, *J* = 11.0, 5.3 Hz, 1H), 2.94 - 2.76 (m, 4H), 2.66 (t, *J* = 11.5 Hz, 3H), 2.18 - 1.99 (m, 5H), 1.89 (dd, *J* = 12.6, 3.3 Hz, 1H), 1.55 (qd, *J* = 12.3, 4.4 Hz, 1H), 1.46 - 1.33 (m, 2H).

### Example 27

### Preparation of trans-(6-(benzylthio)pyrimidin-4-yl)-4-(3,4-dihydroisoquinolin-2(1H)-yl)piperidin-3-ol:

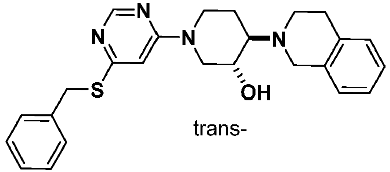

### Step I: preparation of trans-(6-(benzylthio)pyrimidin-4-yl)-4-(3,4-dihydroisoquinolin-2(1H)-yl)piperidin-3 -ol:

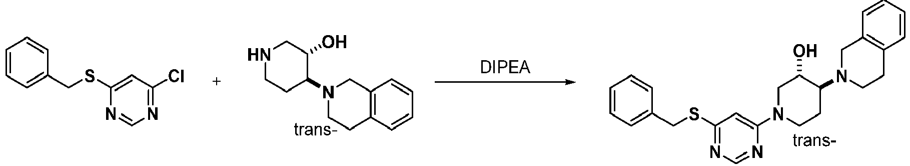

4-(benzylthio)-6-chloropyrimidine (60 mg, 0.254 mmol, 1.2 eq) and trans-4-(3,4-dihydroisoquinolin-2(1*H*)-yl)piperidin-3-ol (49 mg, 0.212 mmol, 1.0 eq) were dissolved in isopropanol (1 mL), followed by addition of DIEA (*N,N*-diisopropylethylamine) (55 mg, 0.424 mmol, 2.0 eq), and the reaction mixture was allowed to react at 100°C for 1 h. The reaction mixture was rotary evaporated to dryness and subjected to reverse phase preparative HPLC to obtain 40 mg trans-1-(6-(benzylthio)pyrimidin-4-yl)-4-(3,4-dihydroisoquinolin-2(1*H*)-yl)piperidin-3-ol, yield: 43.7%. LCMS: [M+1]⁺ = 433.21; ¹H NMR (400 MHz, CDCl₃) δ 8.45 (d, *J* = 1.1 Hz, 1H), 7.45 - 7.37 (m, 2H), 7.34 - 7.28 (m, 2H), 7.28 - 7.21 (m, 1H), 7.18 - 7.08 (m, 3H), 7.05 - 6.97 (m, 1H), 6.41 (d, *J* = 1.2 Hz, 1H), 4.59 (dd, *J* = 37.2, 12.8 Hz, 2H), 4.41 (s, 2H), 3.92 (d, *J* = 14.5 Hz, 1H), 3.75 - 3.62 (m, 2H), 3.54 (td, *J* = 10.1, 4.9 Hz, 1H), 3.00 (dt, *J* = 10.9, 5.3 Hz, 1H), 2.90 (t, *J* = 5.7 Hz, 2H), 2.81 (td, *J* = 13.1, 2.6 Hz, 1H), 2.73 (dd, *J* = 12.8, 10.3 Hz, 1H), 2.66 (qd, *J* = 8.6, 6.0 Hz, 2H), 1.90 (*dq, J* = 12.9, 2.8 Hz, 1H), 1.51 (qd*, J* = 12.5, 4.4 Hz, 1H).

### Example 28

### Preparation of trans-1-(4-((3-(4-(3,4-dihydroisoquinolin-2(1H)-yl)-3-hydroxypiperidine-1-carbonyl)phenyl)amino)piperidin-1-yl)ethan-1-one:

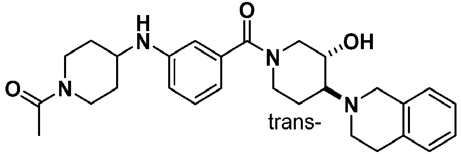

### Step I: preparation of methyl 3-((1-acetylpiperidin-4-yl)amino)benzoate:

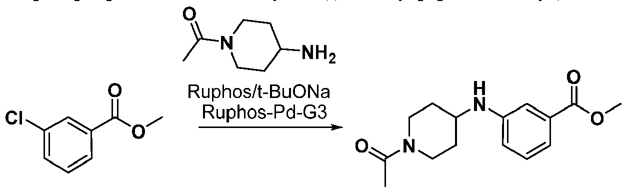

The compounds methyl m-chlorobenzoate (500 mg, 2.93 mmol, 1.0 eq), 1-acetyl-4-aminopiperidine (626 mg, 3.52 mmol, 1.2 eq), Ruphos (2-dicyclohexylphosphino-2',6'-diisopropoxy-1,1'-biphenyl) (41 mg, 0.09 mmol, 0.03 eq), and Ruphos-Pd-G3 ((2-dicyclohexylphosphino-2',6'-diisopropoxy-1,1'-biphenyl) (2-amino-1,1 '-biphen-2-yl)-palladium(II) methanesulfonate) (74 mg, 0.09 mmol, 0.03 eq) were dissolved in THF (tetrahydrofuran) (14 mL), followed by addition of t-BuONa (sodium t-butoxide) (563 mg, 5.86 mmol, 2.0 eq), and the temperature was raised to 60°C at which the reaction was allowed to proceed for 1.5 h. After the reaction was complete, the reaction mixture was poured into 20 mL water and extracted three times with ethyl acetate (15 mL each). The ethyl acetate phases were combined, washed once with 20 mL saturated sodium chloride solution, dried over anhydrous sodium sulfate for 10 min, filtrated and concentrated to obtain a crude product. The crude product was separated by column chromatography (silica gel, DCM: MeOH = 20:1 to 15:1) to obtain 180 mg of the intermediate methyl 3-((1-acetylpiperidin-4-yl)amino)benzoate, yield: 22.2%. LCMS (ESI+) [M+1]⁺ = 277.1.

### Step II: preparation of 3-((1-acetylpiperidin-4-yl)amino)benzoic acid:

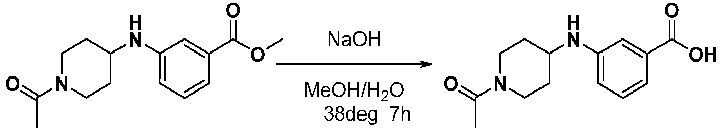

Methyl 3-((1-acetylpiperidin-4-yl)amino)benzoate (180 mg, 0.65 mmol, 1.0 eq) was dissolved in MeOH (methanol) (3.2 mL), followed by addition of a 3.5 M (molar concentration) aqueous sodium hydroxide solution (29 mg, 0.72 mmol, 1.1 eq), and under nitrogen protection, the reaction mixture was allowed to react at rt (room temperature, 24°C) for 3 h. The reaction was incomplete as indicated by TLC and LCMS, and the temperature was raised to 38°C to allow the reaction to proceed for 4 h. After the reaction was complete as indicated by TLC and LCMS, the reaction mixture was poured into 5 mL water and extracted with ethyl acetate. The aqueous phase was adjusted to about pH 4 with 1M (molar concentration) aqueous hydrogen chloride, and rotary evaporated to remove water and dried to obtain 154 mg 3-((1-acetylpiperidin-4-yl)amino)benzoic acid, yield: 90%.

### Step III: preparation of trans-1-(4-((3-(4-(3,4-dihydroisoquinolin-2(1H)-yl)-3-hydroxypiperidine-1-carbonyl)phenyl)amino)piperidin-1-yl)ethan-1-one:

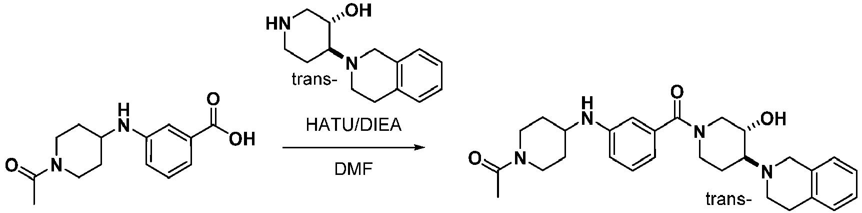

3-((1-acetylpiperidin-4-yl)amino)benzoic acid (100 mg, 0.38 mmol, 1.0 eq), trans-4-(3,4-dihydroisoquinolin-2(1*H*)-yl)piperidin-3-ol (89 mg, 0.38 mmol, 1.0 eq) and HATU (2-(7-azabenzotriazole)-N,N,N',N'-tetramethyluronium hexafluorophosphate) (218 mg, 0.57 mmol, 1.5 eq) were dissolved in DMF (N,N-dimethylformamide) (1.9 mL), followed by addition of DIEA (*N,N*-diisopropylethylamine) (0.34 mL, 1.91 mmol, 5.0 eq), and under nitrogen protection, the reaction mixture was allowed to react at room temperature (24°C) for 1.5 h. After the reaction was complete as indicated by TLC and LCMS, the reaction mixture was poured into 5 mL water and extracted three times with ethyl acetate (5 mL for each). The ethyl acetate phases were combined, washed once with 10 mL water and once with 10 mL saturated sodium chloride solution, dried over anhydrous sodium sulfate for 2 min, filtrated and concentrated. The crude product was separated and purified by preparative HPLC (C18, 0.08% aqueous NH₄HCO₃, MeCN) to obtain 25.4 mg of the product trans-1-(4-((3-(4-(3,4-dihydroisoquinolin-2(1*H*)-yl)-3-hydroxypiperidine-1-carbonyl)phenyl) amino)piperidin-1-yl)ethan-1-one, yield: 14.0%. LCMS (ESI) [M+H]⁺=477.4; ¹H NMR (400 MHz, CDCl₃) δ 7.21 - 7.10 (m, 4H), 7.06 - 7.01 (m, 1H), 6.69 (d, J = 7.4 Hz, 1H), 6.63 (d, J = 6.2 Hz, 2H), 5.17 - 4.80 (m, 1H), 4.50 (d, J = 13.6 Hz, 1H), 4.27 - 3.93 (m, 2H), 3.87 - 3.63 (m, 3H), 3.60 - 3.44 (m, 2H), 3.26 - 3.15 (m, 1H), 3.08 (s, 1H), 2.96 (s, 2H), 2.85 (t, J = 12.5 Hz, 2H), 2.73 (s, 2H), 2.15 (s, 1H), 2.12 (s, 3H), 2.08 - 2.04 (m,1H), 2.00 - 1.91 (m, 1H), 1.71 - 1.50 (m, 3H), 1.42 - 1.30 (m, 2H).

### Example 29

### Preparation of trans-1-(4-((4-(4-(3,4-dihydroisoquinolin-2(1H)-yl)-3-hydroxypiperidin-1-carbonyl)phenyl)amino)piperidin-1-yl)ethan-1-one:

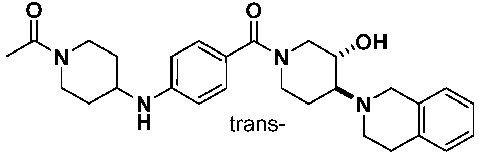

### Step I: preparation of trans-(4-bromophenyl)(4-(3,4-dihydroisoquinolin-2(1H)-yl)-3-hydroxypiperidin-1-yl)ketone:

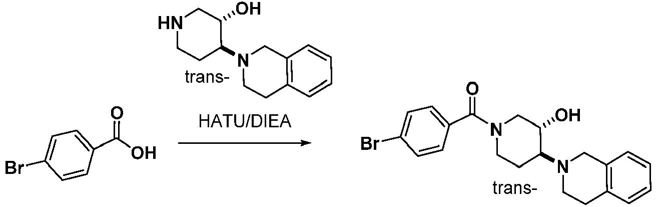

The compounds 4-bromobenzoic acid (200 mg, 1.00 mmol, 1.0 eq), trans-4-(3,4-dihydroisoquinolin-2(1H)-yl)piperidin-3-ol (277 mg, 1.19 mmol, 1.2 eq) and HATU (2-(7-azabenzotriazole)-N,N,N',N'-tetramethyluronium hexafluorophosphate) (568 mg, 1.49 mmol, 1.5 eq) were dissolved in DMF (N,N-dimethylformamide) (9 mL), followed by addition of DIEA (N,N-diisopropylethylamine) (0.86 mL, 4.98 mmol, 5.0 eq), and under nitrogen protection, the reaction mixture was allowed to react at room temperature (24°C) for 1.5 h. After the reaction was complete as indicated by TLC and LCMS, the reaction mixture was poured into 15 mL water and extracted three times with ethyl acetate (15 mL each). The ethyl acetate phases were combined, washed once with 20 mL water and once with 10 mL saturated sodium chloride solution, dried over anhydrous sodium sulfate for 2 min, filtrated and concentrated. The crude product was separated by column chromatography (DCM: MeOH = 20:1 to 10:1) to obtain the intermediate trans-(4-bromophenyl)(4-(3,4-dihydroisoquinolin-2(1H)-yl)-3-hydroxypiperidin-1-yl)ketone (410 mg, yield: 99.3%). LCMS (ESI) [M+H]⁺ = 415.3.

### Step II: preparation of trans-1-(4-((4-(4-(3,4-dihydroisoquinolin-2(1H)-yl)-3-hydroxypiperidine-1-carbonyl)phenyl)amino)piperidin-1-yl)ethan-1-one:

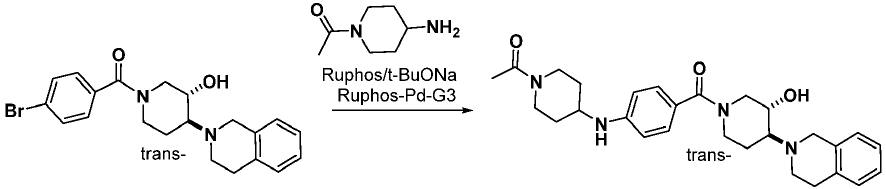

Trans-(4-bromophenyl)(4-(3,4-dihydroisoquinolin-2(1*H*)-yl)-3-hydroxypiperidin-1-yl)ke tone (340 mg, 0.82 mmol, 1.0 eq), 1-acetyl-4-aminopiperidine (175 mg, 0.98 mmol, 1.2 eq), Ruphos (2-dicyclohexylphosphino-2',6'-diisopropoxy-1,1'-biphenyl) (12 mg, 0.03 mmol, 0.03 eq), Ruphos-Pd-G3 ((2-dicyclohexylphosphino-2',6'-diisopropoxy-1,1'-biphenyl) (2-amino-1,1'-biphen-2-yl)palladium(II) methanesulfonate) (21 mg, 0.03 mmol, 0.03 eq) were dissolved in THF (tetrahydrofuran) (4 mL), followed by addition of t-BuONa (sodium t-butoxide) (158 mg, 1.64 mmol, 2.0 eq), and the temperature was raised to 80°C to allow the reaction to proceed overnight. After the reaction was complete, the reaction mixture was poured into 20 mL water and extracted three times with ethyl acetate (15 mL for each). The ethyl acetate phases were combined, washed once with 20 mL saturated sodium chloride solution, dried over anhydrous sodium sulfate for 10 min, filtrated and concentrated obtain a crude product. The crude product was separated and purified by Prep-HPLC (C18, 0.08% aqueous NH₄HCO₃, acetonitrile) to obtain the product trans-1-(4-((4-(4-(3,4-dihydroisoquinolin-2(1*H*)-yl)-3-hydroxypiperidine-1-carbonyl)phenyl)amino)piperidin-1-yl)ethan-1-one (20.5 mg, yield: 5.2%). LCMS (ESI) [M+H]⁺ = 477.4; 1H NMR (400 MHz, CDCl₃) δ 7.21 - 7.10 (m, 4H), 7.06 - 7.01 (m, 1H), 6.69 (d, J = 7.4 Hz, 1H), 6.63 (d, J = 6.2 Hz, 2H), 5.17 - 4.80 (m, 1H), 4.50 (d, J = 13.6 Hz, 1H), 4.27 - 3.93 (m, 2H), 3.87 - 3.63 (m, 3H), 3.60 - 3.44 (m, 2H), 3.26 - 3.15 (m, 1H), 3.08 (s, 1H), 2.96 (s, 2H), 2.85 (t, J = 12.5 Hz, 2H), 2.73 (s, 2H), 2.15 (s, 1H), 2.12 (s, 3H), 2.08 - 2.04 (m,1H), 2.00 - 1.91 (m, 1H), 1.71 - 1.50 (m, 3H), 1.42 - 1.30 (m, 2H).

### Example 30

### Preparation of trans-4-(3,4-dihydroisoquinolin-2(1H)-yl)-1-(6-((4-fluorophenyl)amino)-pyrimidin-4-yl)piperidin-3-ol:

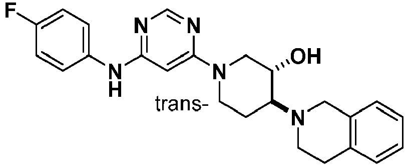

### Step I: preparation of trans-4-(3,4-dihydroisoquinolin-2(1H)-yl)-1-(6-((4-fluorophenyl)-amino)-pyrimidin-4-yl)piperidin-3-ol:

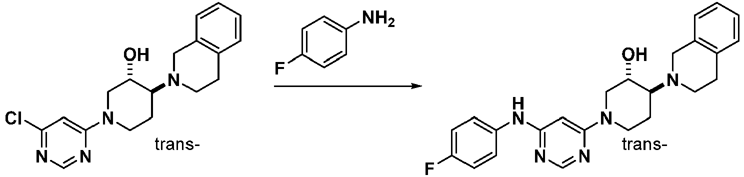

Trans-1-(6-chloropyrimidin-4-yl)-4-(3,4-dihydroisoquinolin-2(1*H*)-yl)piperidin-3-ol (100 mg, 0.29 mmol, 1.0 eq) was dissolved in isopropanol (1.5 mL), and 4-fluoroaniline (64 mg, 0.58 mmol, 2.0 eq) and concentrated hydrochloric acid (36wt%, 0.15 mL) were added thereto. The mixture was heated under stirring at 100°C overnight (16 h). The reaction mixture was adjusted to pH 7 with 1 M (molar concentration) aqueous sodium hydroxide, and extracted three times with ethyl acetate (5 mL for each). The ethyl acetate phases were combined, washed once with 10 mL water and once with 10 mL saturated sodium chloride solution, dried over anhydrous sodium sulfate for 10 min, and filtrated. The crude product was separated and purified by preparative HPLC (C18, 10 mmol/L aqueous NH₄HCO₃, MeCN) to obtain trans-4-(3,4-dihydroisoquinolin-2(1*H*)-yl)-1-(6-((4-fluorophenyl)amino)pyrimidin-4-yl)-piper idin-3-ol (17.8 mg, yield: 14.7%). LCMS (ESI) [M+H]⁺ =420.36; ¹H NMR (400 MHz, CDCl₃) δ 8.24 (s, 1H), 7.27 - 7.23 (m, 2H), 7.18 - 7.04 (m, 5H), 7.03 - 6.99 (m, 1H), 6.85 (s, 1H), 5.81 (s, 1H), 4.70 (d, *J* = 12.7 Hz, 1H), 4.40 (d, *J* = 10.0 Hz, 1H), 3.93 (d, *J* = 14.5 Hz, 1H), 3.68 (d, *J* = 14.6 Hz, 2H), 3.56 (td, *J* = 10.0, 4.9 Hz, 1H), 3.01 (dt, *J* = 10.8, 5.3 Hz, 1H), 2.90 (t, *J* = 5.6 Hz, 2H), 2.82 - 2.61 (m, 4H), 1.89 (dd, *J* = 12.8, 3.1 Hz, 1H), 1.53 (qd, *J* = 12.5, 4.3 Hz, 1H).

### Example 31

### Preparation of trans-1-(4-((6-(4-(3,4-dihydroisoquinolin-2(1H)-yl)-3-hydroxypiperidin-1-yl)thiazolo[4,5-c]pyridin-2-yl)amino)piperidin-1-yl)ethanone:

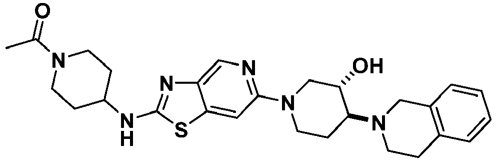

### Step I: preparation of 6-chlorothiazolo[4,5-c]pyridine-2(3H)-thione:

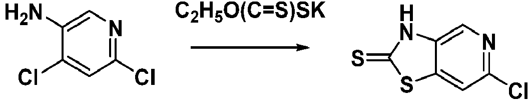

A three-neck bottle containing 4,6-dichloropyridin-3-amine (1.0 g, 6.14 mmol, 1.0 eq) and C₂H₅O(C=S)SK (potassium ethyl xanthogenate) (1.47 g, 9.20 mmol, 1.5 eq) was purged with nitrogen, then anhydrous NMP (N-methylpyrrolidone) (40 mL) was added thereto, and the reaction was conducted at 145°C overnight (16 h). The reaction was complete as monitored by LCMS and TLC. After the reaction mixture was cooled to room temperature, 1.2 mL acetic acid was added. The reaction mixture was poured into 200 mL water and filtrated. The filter cake was washed with a small amount of water, and then the solid was dried under vacuum to obtain a crude product of 6-chlorothiazolo[4,5-c]pyridine-2(3H)-thione (1.17 g, crude product yield: 94.4%) as a brown solid. LCMS (ESI) [M+H]⁺ = 203.02; ¹H NMR (400 MHz, DMSO-d₆) δ 14.14 (s, 2H), 8.28 (d, J = 0.6 Hz, 1H), 7.94 (d, J = 0.6 Hz, 1H).

### Step II: preparation of 2,6-dichlorothiazolo[4,5-c]pyridine:

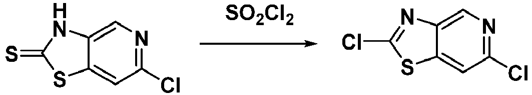

A three-neck bottle containing the 6-chlorothiazolo[4,5-c]pyridine-2(3H)-thione crude product (937 mg, 4.64 mmol, 1.0 eq) was purged with nitrogen, and SO₂Cl₂ (sulfonyl chloride) (10 mL) was added, followed by stirring at room temperature for 2 h. The reaction was complete as monitored by LCMS. The reaction mixture was poured into ice water, adjusted to pH=7 with 1 M (molar concentration) aqueous sodium hydroxide, extracted with ethyl acetate, and rotary evaporated to dryness. Seperation and purification were conducted by flash chromatography (silica gel, EA: PE = 0 to 1:20) to obtain a pure product of 2,6-dichlorothiazolo[4,5-c]pyridine (501 mg, two-step yield: 50.0%) as a white solid. LCMS (ESI) [M+H]⁺ = 204.84, 206.83; ¹H NMR (400 MHz, CDCl₃) δ 9.00 (d, *J* = 0.8 Hz, 1H), 7.79 (d, *J* = 0.8 Hz, 1H).

### Step III: preparation of 1-(4-((6-chlorothiazolo[4,5-c]pyridin-2-yl)amino)-piperidin-1 -yl)ethanone:

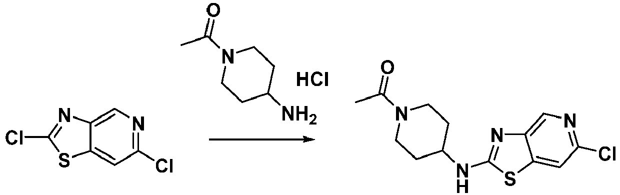

2,6-dichlorothiazolo[4,5-c]pyridine (1.00 g, 4.90 mmol, 1.0 eq) and 1-acetylpiperidin-4-amine hydrochloride (960 mg, 5.39 mmol, 1.1 eq) were dissolved in anhydrous acetonitrile (40 ml), and potassium carbonate (2.37 g, 17.16 mmol, 3.5 eq) was added. The reaction was conducted under nitrogen protection at 50°C for 20 h. The reaction mixture was filtrated, added to water, and extracted with dichloromethane. The organic phase was dried and rotary evaporated. Seperation and purification were conducted by flash chromatography (silica gel, MeOH: DCM = 0 to 1:24) to obtain a pure product of 1-(4-((6-chlorothiazolo[4,5-c]pyridin-2-yl)amino)piperidin-1-yl)ethanone (425 mg, yield: 28.0%) as a white solid. 424 mg of raw materials were recovered. LCMS (ESI) [M+H]⁺ = 311.28, 313.30.

### Step IV: preparation of trans-1-(4-((6-(4-(3,4-dihydroisoquinolin-2(1H)-yl)-3-hydroxypiperidin-1-yl)thiazolo[4,5-c]pyridin-2-yl)amino)piperidin-1-yl)ethanone:

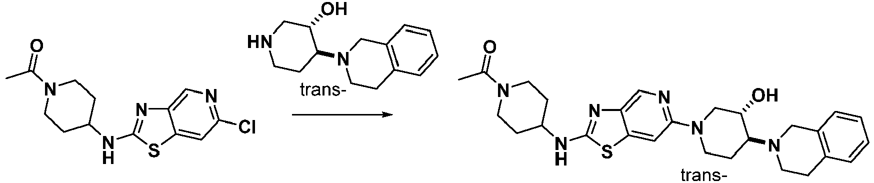

1-(4-((6-chlorothiazolo[4,5-c]pyridin-2-yl)amino)piperidin-1-yl)ethanone (400 mg, 1.29 mmol, 1.0 eq), trans-4-(3,4-dihydroisoquinolin-2(1*H*)-yl)piperidin-3-ol (309 mg, 1.42 mmol, 1.1 eq), RuPhos (2-dicyclohexylphosphino-2',6'-diisopropoxy-1,1'-biphenyl) (120 mg, 0.26 mmol, 0.2 eq), RuPhosPdG3 ((2-dicyclohexylphosphino-2',6'-diisopropoxy-1,1'-biphenyl) (2-amino-1,1'-biphen-2-yl)palladium(II) methanesulfonate) (216 mg, 0.26 mmol, 0.2 eq) and sodium t-butoxide (496 mg, 5.16 mmol, 4.0 eq) were put into a microwave tube, which was purged with nitrogen. Anhydrous dioxane (6.5 ml) was added, and the reaction was conducted by heating at 100°C for 40 h. The reaction mixture was filtrated, added to water, and extracted with dichloromethane. The organic phase was dried and rotary evaporated. Seperation and purification were conducted by flash chromatography (silica gel, MeOH: DCM = 0 to 1:24) to obtain a crude product. Upon further separation and purification by preparative HPLC (C18, 10 mmol/L aqueous NH₄HCO₃, MeCN), a pure product of trans-1-(4-((6-(4-(3,4-dihydroisoquinolin-2(1*H*)-yl)-3-hydroxypiperidin-1-yl)thiazolo[4,5-*c*]-pyridin-2-yl)amino)piperidin-1-yl)ethanone (35 mg, yield: 5.4%) was obtained. LCMS (ESI) [M+H]⁺ = 507.41; ¹H NMR (400 MHz, CDCl₃) δ 8.43 (s, 1H), 7.18 - 7.09 (m, 3H), 7.05 - 6.99 (m, 1H), 6.96 (s, 1H), 5.06 (s, 1H), 4.54 (d, *J* = 13.5 Hz, 2H), 4.42 (ddd, *J* = 12.6, 5.0, 2.0 Hz, 1H), 4.09 - 3.92 (m, 2H), 3.89 - 3.79 (m, 1H), 3.77 - 3.66 (m, 3H), 3.26 (ddd, *J* = 14.1, 11.6, 2.9 Hz, 1H), 3.04 (dt, *J* = 10.9, 5.3 Hz, 1H), 2.91 (t, *J* = 5.9 Hz, 3H), 2.88 - 2.71 (m, 3H), 2.71 - 2.61 (m, 2H), 2.34 - 2.23 (m, 1H), 2.22 - 2.07 (m, 4H), 1.96 - 1.88 (m, 1H), 1.72 - 1.65 (m, 1H), 1.53 - 1.39 (m, 2H).

### Example 32

### Preparation of trans-4-(3,4-dihydroisoquinolin-2(1H)-yl)-1-(6-((4-(methylsulfonyl)-phenyl)amino)pyrimidin-4-yl)piperidin-3-ol:

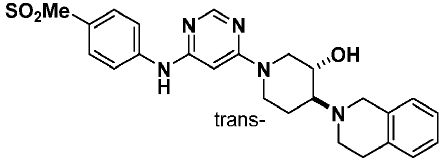

### Step I: preparation of trans-4-(3,4-dihydroisoquinolin-2(1H)-yl)-1-(6-((4-(methylsulfonyl)-phenyl)amino)pyrimidin-4-yl)piperidin-3-ol:

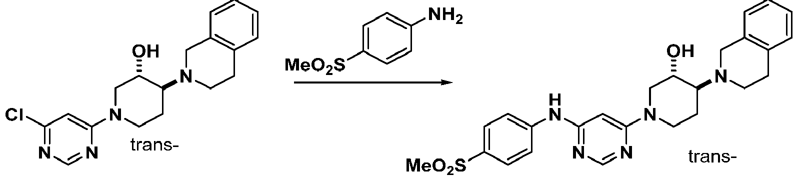

Trans-1-(6-chloropyrimidin-4-yl)-4-(3,4-dihydroisoquinolin-2(1*H*)-yl)piperidin-3-ol (60 mg, 0.17 mmol, 1.0 eq) was dissolved in isopropanol (1 mL), followed by addition of methylsulfonylanilide (60 mg, 0.35 mmol, 2.0 eq) and concentrated hydrochloric acid (36wt%, 0.1 mL). The mixture was heated under stirring at 100°C overnight, then adjusted to pH 8 with saturated sodium bicarbonate aqueous solution, and extracted three times with ethyl acetate (5 mL for each). The ethyl acetate phases were combined, washed once with 10 mL water and once with 10 mL saturated sodium chloride solution, dried over anhydrous sodium sulfate for 2 min, and filtrated. The crude product was purified by preparative HPLC (C18, 10 mmol/L aqueous NH₄HCO₃, acetonitrile) to obtain trans-4-(3,4-dihydroisoquinolin-2(1*H*)-yl)-1-(6-((4-(methylsulfonyl)phenyl)amino)pyrimidin-4-yl)piperidin-3-ol (15.6 mg, yield: 18.7%). LCMS(ESI)[M+H]⁺ =480.3; ¹H NMR (400 MHz, CDCl₃) δ 8.35 (s, 1H), 7.93 - 7.85 (m, 2H), 7.62 - 7.55 (m, 2H), 7.19 - 7.08 (m, 3H), 7.05 - 7.01 (m, 1H), 6.90 (s, 1H), 6.05 (s, 1H), 4.76 (d, J = 13.4 Hz, 1H), 4.48 (d, J = 12.9 Hz, 1H), 3.98 (d, J = 14.6 Hz, 1H), 3.89 - 3.66 (m, 2H), 3.63 (td, J = 10.0, 4.8 Hz, 1H), 3.13 - 3.01 (m, 4H), 2.94 (t, J = 5.8 Hz, 2H), 2.88 - 2.68 (m, 4H), 1.95 (dd, J = 12.8, 3.3 Hz, 1H), 1.63 - 1.52 (m, 1H).

### Example 33

### Preparation of trans-4-((6-(4-(3,4-dihydroisoquinolin-2(1H)-yl)-3-hydroxypiperidin-1-yl)pyrimidin-4-yl)amino)benzonitrile:

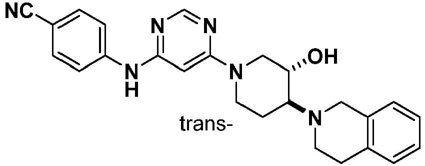

### Step I: preparation of trans-4-((6-(4-(3,4-dihydroisoquinolin-2(1H)-yl)-3-hydroxypiperidin-1-yl)pyrimidin-4-yl)amino)benzonitrile:

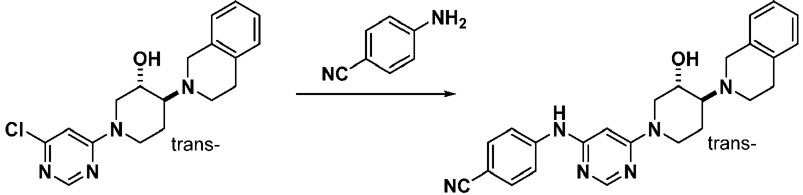

Trans-1-(6-chloropyrimidin-4-yl)-4-(3,4-dihydroisoquinolin-2(1*H*)-yl)piperidin-3-ol (70 mg, 0.20 mmol, 1.0 eq) was dissolved in isopropanol (1 mL), followed by addition of p-aminobenzonitrile (48 mg, 0.41 mmol, 2.0 eq) and concentrated hydrochloric acid (0.1 mL). The mixture was heated under stirring at 100°C overnight, then adjusted to pH 8 with saturated sodium bicarbonate aqueous solution, and extracted three times with ethyl acetate (5 mL for each). The ethyl acetate phases were combined, washed once with 10 mL water and once with 10 mL saturated sodium chloride solution, dried over anhydrous sodium sulfate for 2 min, and filtrated. The crude product was separated and purified by Prep-HPLC (C18, 10 mmol/L aqueous NH₄HCO₃, acetonitrile) to obtain trans-4-((6-(4-(3,4-dihydroisoquinolin-2(1*H*)-yl)-3-hydroxypiperidin-1-yl)pyrimidin-4-yl)amino)benzonitrile (11.3 mg, yield: 13.1%). LCMS(ESI)[M+H]⁺ =427.3; ¹H NMR (400 MHz, CDCl₃) δ 8.35 (s, 1H), 7.61 (d, J = 8.3 Hz, 2H), 7.52 (d, J = 8.3 Hz, 2H), 7.20 - 7.10 (m, 3H), 7.02 (d, J = 6.9 Hz, 1H), 6.74 (s, 1H), 6.02 (s, 1H), 4.76 (d, J = 13.4 Hz, 1H), 4.47 (d, J = 13.0 Hz, 1H), 3.98 (d, J = 14.6 Hz, 1H), 3.89 - 3.68 (m, 2H), 3.62 (td, J = 10.0, 4.9 Hz, 1H), 3.05 (dd, J = 11.1, 5.5 Hz, 1H), 2.94 (t, J = 5.7 Hz, 2H), 2.89 - 2.67 (m, 4H), 1.95 (d, J = 12.4 Hz, 1H), 1.58 - 1.52 (m, 1H).

### Example 34

### Preparation of trans-4-(3,4-dihydroisoquinolin-2(1H)-yl)-1-(6-(m-methylphenylamino)-pyrimidin-4-yl)piperidin-3-ol:

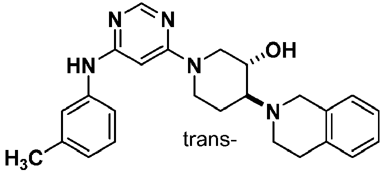

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3-methylaniline to obtain the target compound trans-4-(3,4-dihydroisoquinolin-2(1*H*)-yl)-1-(6-(m-methylphenylamino)pyrimidin-4-yl)-piperidin-3-ol, yield: 63.0%. LCMS(ESI)[M+H]⁺ =417.4; ¹H NMR (400 MHz, CDCl₃) δ 8.25 (d, J = 0.9 Hz, 1H), 7.32 - 7.21 (m, 1H), 7.19 - 7.05 (m, 5H), 7.04 - 6.93 (m, 2H), 6.75 (s, 1H), 5.96 (d, J = 1.0 Hz, 1H), 4.71 (d, J = 13.5 Hz, 1H), 4.42 (d, J = 11.3 Hz, 1H), 3.94 (d, J = 14.6 Hz, 1H), 3.69 (d, J = 14.6 Hz, 2H), 3.57 (td, J = 10.0, 4.9 Hz, 1H), 3.02 (dt, J = 11.0, 5.3 Hz, 1H), 2.91 (t, J = 5.8 Hz, 2H), 2.84 - 2.62 (m, 4H), 2.37 (s, 3H), 1.90 (dd, J = 12.8, 3.5 Hz, 1H), 1.54 (qd, J = 12.4, 4.3 Hz, 1H).

### Example 35

### Preparation of trans-1-(6-((3-cyclopropylphenyl)amino)pyrimidin-4-yl)-4-(3,4-dihydroisoquinolin-2(1H)-yl)piperidin-3-ol:

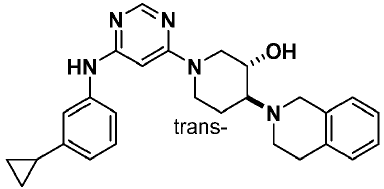

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3-cyclopropylaniline to obtain the target compound trans-1-(6-((3-cyclopropylphenyl)amino)pyrimidin-4-yl)-4-(3,4-dihydroisoquinolin-2(1*H*)-yl) piperidin-3-ol, yield: 45.8%. LCMS(ESI)[M+H]⁺ =442.3; ¹H NMR (400 MHz, Chloroform-*d*) δ 8.25 (d, J = 0.9 Hz, 1H), 7.28 - 7.22 (m, 1H), 7.17 - 7.09 (m, 3H), 7.09 - 7.05 (m, 1H), 7.03 - 6.99 (m, 1H), 6.94 (t, J = 2.0 Hz, 1H), 6.87 (dt, J = 7.7, 1.4 Hz, 1H), 6.70 (s, 1H), 5.97 (d, J = 1.1 Hz, 1H), 4.69 (d, J = 13.5 Hz, 1H), 4.43 (d, J = 13.0 Hz, 1H), 3.94 (d, J = 14.5 Hz, 1H), 3.75 - 3.65 (m, 2H), 3.57 (td, J = 10.0, 4.9 Hz, 1H), 3.02 (dt, J = 11.0, 5.3 Hz, 1H), 2.91 (t, J = 5.8 Hz, 2H), 2.83 - 2.61 (m, 4H), 1.94 - 1.85 (m, 2H), 1.54 (qd, J = 12.5, 4.4 Hz, 1H), 1.04 - 0.95 (m, 2H), 0.73 - 0.67 (m, 2H).

### Example 36

### Preparation of trans-3-((6-(4-(3,4-dihydroisoquinolin-2(1H)-yl)-3-hydroxypiperidin-1-yl)pyrimidin-4-yl)amino)benzonitrile:

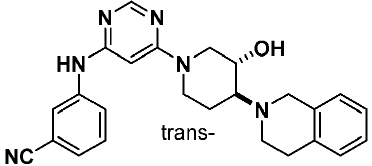

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3-cyanoaniline to obtain the target compound trans-3-((6-(4-(3,4-dihydroisoquinolin-2(1*H*)-yl)-3-hydroxypiperidin-1-yl)pyrimidin-4-yl)-amino)benzonitrile, yield: 61.2%. LCMS(ESI)[M+H]⁺ =428.4; ¹H NMR (400 MHz, CDCl₃) δ 8.32 (s, 1H), 7.80 (t, J = 1.9 Hz, 1H), 7.66 - 7.57 (m, 1H), 7.44 (t, J = 7.9 Hz, 1H), 7.36 (dt, J = 7.6, 1.5 Hz, 1H), 7.14 (dt, J = 9.4, 3.3 Hz, 3H), 7.07 - 6.99 (m, 1H), 6.67 (s, 1H), 5.90 (s, 1H), 4.73 (d, J = 13.2 Hz, 1H), 4.46 (d, J = 12.6 Hz, 1H), 3.95 (d, J = 14.6 Hz, 1H), 3.87 - 3.66 (m, 2H), 3.60 (td, J = 10.0, 4.9 Hz, 1H), 3.03 (dt, J = 11.0, 5.4 Hz, 1H), 2.92 (t, J = 5.7 Hz, 2H), 2.87 - 2.64 (m, 4H), 1.93 (dd, J = 12.9, 3.3 Hz, 1H), 1.56 (qd, J = 12.5, 4.3 Hz, 1H).

### Example 37

### Preparation of trans-1-(6-((3-t-butylphenyl)amino)pyrimidin-4-yl)-4-(3,4-dihydroisoquinolin-2(1H)-yl)piperidin-3-ol:

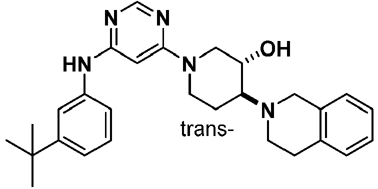

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3-t-butylaniline to obtain the target compound trans-1-(6-((3-t-butylphenyl)amino)pyrimidin-4-yl)-4-(3,4-dihydroisoquinolin-2(1*H*)-yl)-piperidin-3-ol, yield: 23%. LCMS(ESI)[M+H]⁺ =458.4; ¹H NMR (400 MHz, CDCl₃) δ 8.25 (d, J = 0.9 Hz, 1H), 7.32 (t, J = 7.9 Hz, 1H), 7.26 - 7.24 (m, 1H), 7.21 - 7.17 (m, 1H), 7.16 - 7.08 (m, 4H), 7.06 - 6.97 (m, 1H), 6.84 (s, 1H), 6.01 (s, 1H), 4.67 (d, J = 13.5 Hz, 1H), 4.44 (d, J = 12.4 Hz, 1H), 3.93 (d, J = 14.5 Hz, 1H), 3.83 - 3.62 (m, 2H), 3.56 (td, J = 10.1, 4.9 Hz, 1H), 3.01 (dt, J = 11.1, 5.3 Hz, 1H), 2.90 (t, J = 5.8 Hz, 2H), 2.86 - 2.58 (m, 4H), 1.89 (dd, J = 12.8, 3.4 Hz, 1H), 1.54 (qd, J = 11.8, 11.1, 3.6 Hz, 1H), 1.33 (s, 9H).

### Example 38

### Preparation of trans-1-(6-((4-cyclopropylphenyl)amino)pyrimidin-4-yl)-4-(3,4-dihydroisoquinolin-2(1H)-yl)piperidin-3-ol:

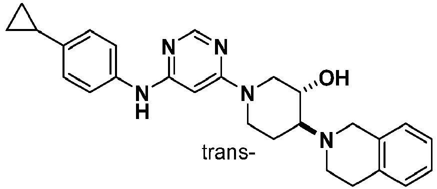

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 4-cyclopropylaniline to obtain the target compound trans-1-(6-((4-cyclopropylphenyl)amino)pyrimidin-4-yl)-4-(3,4-dihydroisoquinolin-2(1*H*)-yl) piperidin-3-ol, yield: 22.3%. LCMS(ESI)[M+H]⁺ =442.4; ¹H NMR (400 MHz, CDCl₃) δ 11.53 (s, 1H), 8.11 (s, 1H), 7.34 - 7.27 (m, 2H), 7.26 - 7.23 (m, 1H), 7.20 (dd, J = 7.4, 1.7 Hz, 1H), 7.16 - 7.11 (m, 3H), 7.07 (d, J = 7.4 Hz, 1H), 5.77 (s, 1H), 4.48 (d, J = 14.8 Hz, 1H), 4.25 (d, J = 14.8 Hz, 2H), 3.90 (s, 2H), 3.58 - 3.26 (m, 4H), 3.22 - 2.79 (m, 4H), 2.11 (d, J = 12.5 Hz, 1H), 1.91 (td, J = 8.6, 4.4 Hz, 1H), 1.68 (d, J = 12.6 Hz, 1H), 1.07 - 0.96 (m, 2H), 0.72 (dt, J = 6.6, 4.7 Hz, 2H).

### Example 39

### Preparation of trans-1-(6-((4-t-butylphenyl)amino)pyrimidin-4-yl)-4-(3,4-dihydroisoquinolin-2(1H)-yl)piperidin-3-ol:

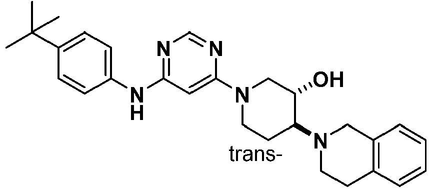

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 4-t-butylaniline to obtain the target compound trans-1-(6-((4-t-butylphenyl)amino)pyrimidin-4-yl)-4-(3,4-dihydroisoquinolin-2(1*H*)-yl)-piperidin-3-ol, yield: 47.4%. LCMS (ESI) [M+H]⁺ =458.4; ¹H NMR (400 MHz, DMSO-*d6*) δ 8.94 (s, 1H), 8.16 (s, 1H), 7.47 - 7.44 (m, 2H), 7.31 - 7.27 (m, 2H), 7.07 - 7.03 (m, 4H), 5.98 (s, 1H), 4.70 (s, 1H), 4.27 (dd, J =39.2. Hz, 12.0 Hz, 2H), 3.81 (dd, *J* = 26.8 Hz, 14.8 Hz, 2H), 3.59 - 3.57 (m, 1H), 2.90 - 2.79 (m, 5H), 2.69 - 2.64 (m, 2H), 1.81 (d, *J=* 10.4 Hz, 1H), 1.49 - 1.42 (m, 1H), 1.27 (s, 9H).

### Example 40

### Preparation of trans-1-(6-([1,1'-biphenyl]-4-ylamino)pyrimidin-4-yl)-4-(3,4-dihydroisoquinolin-2(1H)-yl)piperidin-3-ol:

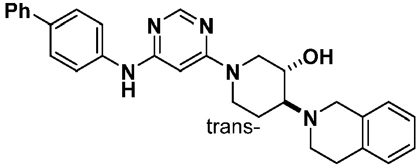

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 4-aminobiphenyl to obtain the target compound trans-1-(6-([1,1'-biphenyl]-4-ylamino)pyrimidin-4-yl)-4-(3,4-dihydroisoquinolin-2(1*H*)-yl)-piperidin-3-ol, yield: 64.9%. LCMS(ESI)[M+H]⁺ =478.4; ¹H NMR (400 MHz, CDCl₃) δ 11.77 (s, 1H), 8.14 (s, 1H), 7.66 (d, J = 8.1 Hz, 2H), 7.62 - 7.57 (m, 2H), 7.46 (dd, J = 8.3, 6.8 Hz, 2H), 7.40 - 7.32 (m, 3H), 7.30 - 7.23 (m, 2H), 7.22 - 7.16 (m, 1H), 7.07 (d, J = 7.4 Hz, 1H), 5.92 (s, 1H), 4.52 (d, J = 14.8 Hz, 1H), 4.44 - 3.76 (m, 4H), 3.68 - 3.30 (m, 4H), 3.22 - 2.79 (m, 4H), 2.13 (d, J = 12.4 Hz, 1H), 1.79 - 1.60 (m, 1H).

### Example 41

### Preparation of trans-1-(6-((4-methylphenyl)amino)pyrimidin-4-yl)-4-(3,4-dihydroisoquinolin-2(1H)-yl)piperidin-3-ol:

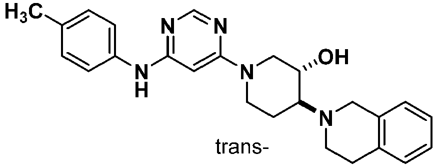

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 4-methylaniline to obtain the target compound trans-1-(6-((4-methylphenyl)amino)pyrimidin-4-yl)-4-(3,4-dihydroisoquinolin-2(1*H*)-yl)piper idin-3-ol, yield: 45.6%. LCMS (ESI) [M+H]⁺ =416.45; ¹H NMR (400 MHz, Chloroform-*d*) δ 8.24 (d, *J* = 1.0 Hz, 1H), 7.21 - 7.08 (m, 7H), 7.05 - 6.96 (m, 1H), 6.66 (s, 1H), 5.89 (d, *J* = 1.1 Hz, 1H), 4.72 (d, *J* = 13.4 Hz, 1H), 4.39 (d, *J* = 11.9 Hz, 1H), 3.93 (d, *J* = 14.6 Hz, 1H), 3.69 (d, *J* = 14.0 Hz, 2H), 3.56 (td, *J* = 10.0, 4.9 Hz, 1H), 3.01 (dt, *J* = 10.9, 5.3 Hz, 1H), 2.91 (t*, J* = 5.7 Hz, 2H), 2.81 - 2.60 (m, 4H), 2.36 (s, 3H), 1.89 (dd, *J* = 13.0, 3.3 Hz, 1H), 1.53 (qd, *J* = 12.5, 4.4 Hz, 1H).

### Example 42

### Preparation of trans-1-(6-((3-fluorophenyl)amino)pyrimidin-4-yl)-4-(3,4-dihydroisoquinolin-2(1H)-yl)piperidin-3-ol:

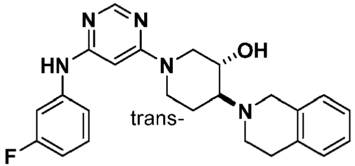

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3-fluoroaniline to obtain the target compound trans-1-(6-((3-fluorophenyl)amino)pyrimidin-4-yl)-4-(3,4-dihydroisoquinolin-2(1*H*)-yl)-piperidin-3-ol, yield: 49.6%. LCMS (ESI): [M+H]⁺ = 420.32; ¹H NMR (400 MHz, DMSO-d₆) δ 9.28 (s, 1H), 8.25 (s, 1H), 7.78-7.71 (m, 1H), 7.32-7.23 (m, 2H), 7.11-7.05 (m, 3H), 7.03 (dd, J = 5.9, 2.2 Hz, 1H), 6.76-6.68 (m, 1H), 6.02 (s, 1H), 4.72 (d, J = 4.0 Hz, 1H), 4.33 (d, J = 10.3 Hz, 1H), 4.22 (d, J = 12.4 Hz, 1H), 3.80 (q, J = 15.0 Hz, 2H), 3.64-3.55 (m, 1H), 2.88 (dd, J = 15.4, 8.4 Hz, 2H), 2.83-2.74 (m, 3H), 2.74-2.62 (m, 2H), 1.87-1.77 (m, 1H), 1.48 (qd, J = 12.4, 4.1 Hz, 1H).

### Example 43

### Preparation of trans-1-(6-((3-chlorophenyl)amino)pyrimidin-4-yl)-4-(3,4-dihydroisoquinolin-2(1H)-yl)piperidin-3-ol:

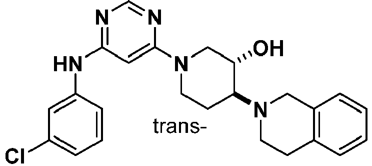

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3-chloroaniline to obtain the target compound trans-1-(6-((3-chlorophenyl)amino)pyrimidin-4-yl)-4-(3,4-dihydroisoquinolin-2(1*H*)-yl)-piperidin-3-ol, yield: 49.6%. LCMS(ESI) [M+H]⁺ = 436.30, 438.31; ¹H NMR (400 MHz, DMSO-*d₆*) δ 9.26 (s, 1H), 8.25 (s, 1H), 7.95 (t, J = 2.0 Hz, 1H), 7.48-7.39 (m, 1H), 7.28 (t, J = 8.1 Hz, 1H), 7.13-6.99 (m, 4H), 6.95 (ddd, J = 7.9, 2.0, 0.8 Hz, 1H), 6.01 (s, 1H), 4.71 (d, J = 4.0 Hz, 1H), 4.33 (d, J = 10.8 Hz, 1H), 4.22 (d, J = 11.4 Hz, 1H), 3.80 (q, J = 15.0 Hz, 2H), 3.64-3.54 (m, 1H), 2.98-2.84 (m, 2H), 2.76 (dt, J = 9.7, 6.4 Hz, 3H), 2.74-2.62 (m, 2H), 1.88-1.76 (m, 1H), 1.48 (qd, J = 12.4, 4.1 Hz, 1H).

### Example 44

### Preparation of trans-1-(6-((3-(methylsulfonyl)phenyl)amino)pyrimidin-4-yl)-4-(3,4-dihydroisoquinolin-2(1H)-yl)piperidin-3-ol:

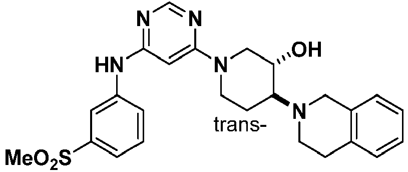

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3-(methylsulfonyl)aniline to obtain the target compound trans-1-(6-((3-(methylsulfonyl)phenyl)amino)pyrimidin-4-yl)-4-(3,4-dihydroisoquinolin-2(1*H*)-yl)piperidin-3-ol, yield: 7.8%. LCMS (ESI) [M+H]⁺ =480.29; ¹H NMR (400 MHz, CDCl₃) δ 8.31 (d, J = 8.0 Hz, 1H), 8.00 (s, 1H), 7.74 (d, J = 8.0 Hz, 1H), 7.62 (d, J = 7.9 Hz, 1H), 7.54 (t, J = 7.9 Hz, 1H), 7.19 - 7.09 (m, 3H), 7.06 - 7.00 (m, 1H), 6.95 (s, 1H), 5.98 (s, 1H), 4.70 (d, J = 12.7 Hz, 1H), 4.48 (d, J = 10.2 Hz, 1H), 3.87 (dd, J = 94.3, 14.6 Hz, 3H), 3.63 (td, J = 9.9, 4.8 Hz, 1H), 3.09 (s, 3H), 3.06 - 2.99 (m, 1H), 2.94 (t, J = 5.5 Hz, 2H), 2.78 (ddd, J = 20.9, 16.4, 7.8 Hz, 4H), 1.94 (d, J = 9.8 Hz, 1H), 1.60 - 1.52 (m, 1H).

### Example 45

### Preparation of trans-1-(6-((4-(1-chloro-3-hydroxypropan-2-yl)phenyl)amino)-pyrimidin-4-yl)-4-(3,4-dihydroisoquinolin-2(1H)-yl)piperidin-3-ol:

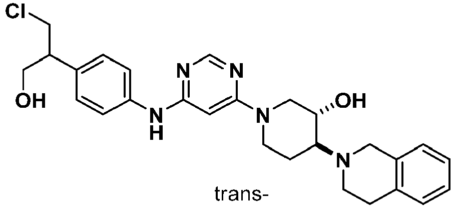

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 4-(oxetan-3-yl)aniline to obtain the target compound trans-1-(6-((4-(1-chloro-3-hydroxypropan-2-yl)phenyl)amino)pyrimidin-4-yl)-4-(3,4-dihydroisoquinolin-2(1*H*)-yl)piperidin-3-ol, yield: 23.7%. LCMS (ESI) [M+H]⁺ =494.32; ¹H NMR (400 MHz, CDCl₃) δ 8.25 (s, 1H), 7.26 (s, 4H), 7.19 - 7.07 (m, 3H), 7.02 (d, J = 6.0 Hz, 1H), 6.76 (s, 1H), 5.99 (s, 1H), 4.74 (d, J = 12.4 Hz, 1H), 4.42 (d, J = 10.3 Hz, 1H), 3.99 (d, J = 5.9 Hz, 2H), 3.94 (d, J = 14.8 Hz, 1H), 3.90 - 3.85 (m, 1H), 3.81 (dd, J = 11.0, 6.3 Hz, 1H), 3.69 (d, J = 14.5 Hz, 1H), 3.58 (td, J = 10.0, 4.9 Hz, 1H), 3.24 - 3.15 (m, 1H), 3.01 (dd, J = 10.8, 5.2 Hz, 1H), 2.91 (t, J = 5.3 Hz, 2H), 2.83 - 2.72 (m, 2H), 2.71 - 2.62 (m, 2H), 1.91 (d, J = 10.2 Hz, 1H), 1.62 - 1.50 (m, 1H).

### Example 46

### Preparation of trans-1-(6-((2-methylphenyl)amino)pyrimidin-4-yl)-4-(3,4-dihydroisoquinolin-2(1H)-yl)piperidin-3-ol:

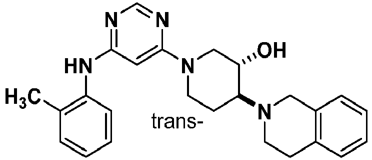

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2-methylaniline to obtain the target compound trans-1-(6-((2-methylphenyl)amino)pyrimidin-4-yl)-4-(3,4-dihydroisoquinolin-2(1*H*)-yl)-piperidin-3-ol, yield: 17.2%. LCMS (ESI) [M+H]⁺ =416.36; ¹H NMR (400 MHz, CDCl₃) δ 8.24 (s, 1H), 7.33 (d, *J=* 7.3 Hz, 1H), 7.28 (d, *J=* 7.8 Hz, 1H), 7.23 (d, *J* = 7.7 Hz, 1H), 7.20 - 7.08 (m, 4H), 7.06 - 6.98 (m, 1H), 6.41 (s, 1H), 5.64 (s, 1H), 4.69 (d, *J* = 12.8 Hz, 1H), 4.36 (d, *J* = 9.6 Hz, 1H), 3.92 (d, *J* = 14.5 Hz, 1H), 3.68 (d, *J* = 14.4 Hz, 2H), 3.55 (td, *J* = 10.0, 4.9 Hz, 1H), 3.01 (dt, *J* = 10.9, 5.3 Hz, 1H), 2.90 (t, *J* = 5.6 Hz, 2H), 2.80 - 2.58 (m, 4H), 2.27 (s, 3H), 1.88 (dd, *J* = 12.8, 3.2 Hz, 1H), 1.52 (qd, *J* = 12.4, 4.3 Hz, 1H).

### Example 47

### Preparation of trans-1-(6-((3-methoxyphenyl)amino)pyrimidin-4-yl)-4-(3,4-dihydroisoquinolin-2(1H)-yl)piperidin-3-ol:

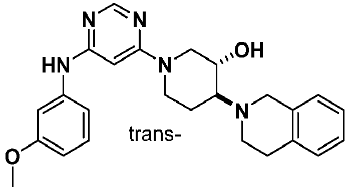

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3-methoxyaniline to obtain the target compound trans-1-(6-((3-methoxyphenyl)amino)pyrimidin-4-yl)-4-(3,4-dihydroisoquinolin-2(1*H*)-yl)-piperidin-3-ol, yield: 30.2%. LCMS (ESI) [M+H]⁺ =432.4; ¹H NMR (400 MHz, CDCl₃) δ 8.28 (s, 1H), 7.33-7.28 (m, 1H), 7.19 -7.12 (m, 3H), 7.05 -7.03 (m, 1H), 6.92 - 6.87 (m, 3H), 6.75-6.72 (d, *J* = 7.4 Hz, 1H), 6.03 (s, 1H), 4.76-4.73 (d, *J* = 13.6 Hz, 1H), 4.48-4.45 (d, *J* = 10.0 Hz, 1H), 4.00-3.96 (d, *J* = 14.4 Hz, 1H), 3.84 (s, 3H), 3.76-3.72 (d, *J* = 14.8 Hz, 1H), 3.64-3.58 (m, 1H), 3.07-3.03 (m, 1H), 2.96-2.94 (t, *J* = 5.6 Hz, 2H), 2.85-2.70 (m, 4H),196-1.92 (dd, *J=* 3.2 Hz,12.8 Hz,1H), 1.63-1.53 (m, 1H).

### Example 48

### Preparation of trans-1-(6-((3-trifluoromethylphenyl)amino)pyrimidin-4-yl)-4-(3,4-dihydroisoquinolin-2(1H)-yl)piperidin-3-ol:

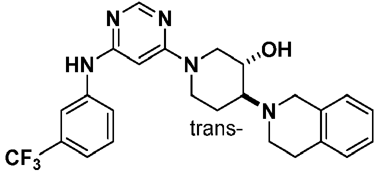

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3-aminotrifluorotoluene to obtain the target compound trans-1-(6-((3-trifluoromethylphenyl)amino)pyrimidin-4-yl)-4-(3,4-dihydroisoquinolin-2(1*H*) -yl)piperidin-3-ol, yield: 20.6%. LCMS (ESI) [M+H]⁺=470.3; ¹H NMR (400 MHz, CDCl₃) δ 8.33 (s, 1H), 7.64 (s, 1H),7.58-7.56 (d, J = 8.4 Hz, 1H), 7.52-7.48 (t, J = 7.6 Hz, 1H), 7.39-7.38 (d, J = 7.6 Hz, 1H),7.19 -7.12 (m, 3H), 7.05-7.03 (m, 1H), 6.78 (s, 1H), 5.97 (s, 1H),4.74-4.71 (d, J = 13.6 Hz, 1H), 4.50-4.48 (d, J = 9.6 Hz, 1H), 4.00-3.96 (d, *J* = 14.4 Hz, 1H), 3.75-3.72 (d, *J* = 14.8 Hz, 1H), 3.65-3.59 (m, 1H), 3.09-3.03 (m, 1H), 2.96-2.93 (t, *J* = 5.6 Hz, 2H), 2.88-2.68 (m, 4H),1.97-1.93 (dd, *J=* 3.2 Hz,12.8 Hz,1H), 1.63-1.54 (m, 1H).

### Example 49

### Preparation of trans-4-(3,4-dihydroisoquinolin-2(1H)-yl)-1-(6-((4-morpholinylphenyl) amino)pyrimidin-4-yl)piperidin-3-ol:

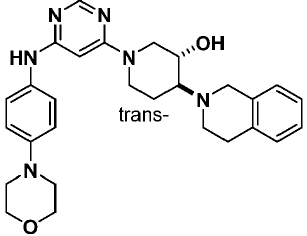

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 4-morpholinylaniline to obtain the target compound trans-4-(3,4-dihydroisoquinolin-2(1*H*)-yl)-1-(6-((4-morpholinylphenyl)amino)pyrimidin-4-yl)piperidin-3-ol, yield: 69.8%. LCMS (ESI) [M+H]⁺ =487.5; ¹H NMR (400 MHz, DMSO-*d*₆) δ 8.77(s, 1H), 8.12 (s, 1H), 7.39-7.37 (d, *J=* 9.2 Hz, 1H), 7.10 -7.07 (m, 3H), 7.04 -7.02 (m, 1H), 6.91-6.88 (d, J = 9.2 Hz, 1H), 5.88 (s, 1H), 4.69-4.68 (d, *J* = 4.0 Hz, 1H), 4.31-4.28 (d, J = 3.6 Hz, 1H), 4.22-4.19 (d, *J* = 12.4 Hz, 1H), 3.86-3.79 (m, 2H), 3.75-3.73 (t, *J* = 4.8 Hz, 4H),3.60-3.55 (m, 1H), 3.05-3.03 (t, *J* = 4.8 Hz, 4H), 2.91-2.78 (m, 5H), 2.68-2.61(t, *J* = 10.0 Hz, 2H), 1.81-1.78 (d, *J* = 3.2 Hz, 1H), 1.48-1.44(m, 1H).

### Example 50

### Preparation of trans-4-(3,4-dihydroisoquinolin-2(1H)-yl)-1-(6-((2-morpholinylphenyl)-amino)pyrimidin-4-yl)piperidin-3-ol:

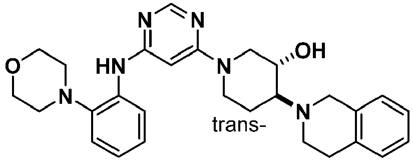

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2-(morpholinyl)aniline to obtain the target compound trans-4-(3,4-dihydroisoquinolin-2(1*H*)-yl)-1-(6-((2-morpholinylphenyl)amino)pyrimidin-4-yl)piperidin-3-ol, yield: 20.57%. LCMS (ESI) [M+H]⁺=487.4; ¹H NMR (400 MHz, CDCl₃) δ 8.33 (s, 1H), 7.77-7.75 (d, *J* = 7.6 Hz, 1H), 7.33 (s, 1H), 7.18 -7.15 (m, 5H), 7.10 -7.03 (m, 2H), 6.11 (s, 1H), 4.80-4.77 (d, *J* = 13.2 Hz, 1H), 4.52-4.49 (dd, *J* = 2.8 Hz,12.8 Hz, 1H), 3.99-3.95 (d, *J* = 14.4 Hz, 1H), 3.90-3.88 (t, *J* = 4.4 Hz, 4H), 3.76-3.71 (m, 2H), 3.66-3.60 (m, 1H), 3.08-3.03 (m,1H), 2.95-2.91(m, 6H), 2.86-2.67 (m, 4H), 1.97-1.93 (dd, *J* = 3.2 Hz, 12.8 Hz, 1H), 1.64-1.57(m, 1H).

### Example 51

### Preparation of trans-4-(3,4-dihydroisoquinolin-2(1H)-yl)-1-(6-((2-cyclopropylphenyl) amino)pyrimidin-4-yl)piperidin-3-ol:

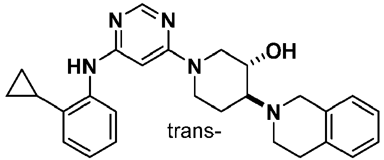

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2-cyclopropylaniline to obtain the target compound trans-4-(3,4-dihydroisoquinolin-2(1*H*)-yl)-1-(6-((2-cyclopropylphenyl)amino)pyrimidin-4-yl) piperidin-3-ol, yield: 28.2%. LCMS (ESI) [M+H]⁺ =442.3; ¹H NMR (400 MHz, CDCl₃) δ 8.29 (s, 1H), 7.46-7.44 (d, *J* =0.8Hz, 8.0Hz, 1H), 7.27-7.23 (td, *J* =1.6Hz, 7.2Hz, 1H), 7.17-7.08 (m, 5H), 7.05-7.02 (m, 1H), 6.80 (s, 1H), 5.89 (s, 1H), 4.74-4.71 (d, *J* = 13.2 Hz, 1H), 4.45-4.42 (dd, *J* = 2.4 Hz, 12.4 Hz, 1H), 3.97-3.93 (d, *J* = 14.4 Hz, 1H), 3.72-3.69 (m, 2H), 3.62-3.56 (m, 1H), 3.05-3.01 (m, 1H), 2.94-2.91(t, *J* = 6.0 Hz, 2H), 2.82-2.64 (m, 4H), 1.95-1.88 (m, 2H), 1.58-1.54 (m, 1H), 1.02-0.98 (m, 2H), 0.72-0.68 (m, 2H).

### Example 52

### Preparation of trans-4-(3,4-dihydroisoquinolin-2(1H)-yl)-1-(6-((4-(trifluoromethyl) phenyl)amino)pyrimidin-4-yl)piperidin-3-ol:

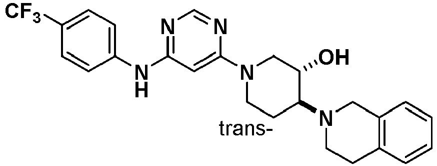

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 4-trifluoromethylaniline to obtain the target compound trans-4-(3,4-dihydroisoquinolin-2(1*H*)-yl)-1-(6-((4-(trifluoromethyl)phenyl)amino)pyrimidin-4-yl)piperidin-3-ol, yield: 74%. LCMS (ESI) [M+H]⁺=442.3; ¹H NMR (400 MHz, TFA-*d*₆) δ 8.52 (s, 1H), 8.01-7.99 (d, *J* =8.0Hz, 2H), 7.67-7.65 (d, *J* =8.0Hz, 2H), 7.57-7.48 (m, 2H), 7.44-7.43 (d, *J* =7.6Hz, 1H), 7.34-7.29 (m, 1H), 6.35 (s, 1H), 5.10 (s, 1H), 4.95-4.70 (m, 3H), 4.60-4.53 (m, 1H), 4.12-4.10 (d, *J* =11.2 Hz, 2H), 3.91-3.38 (m, 5H), 2.66-2.58 (t, *J* =16.8Hz, 1H), 2.26-2.21(t, *J* =10.4Hz, 1H).

### Example 53

### Preparation of trans-4-(3,4-dihydroisoquinolin-2(1H)-yl)-1-(6-((2-fluorophenyl)amino) pyrimidin-4-yl)piperidin-3-ol:

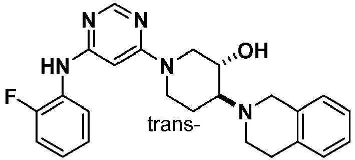

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2-fluoroaniline to obtain the target compound trans-4-(3,4-dihydroisoquinolin-2(1*H*)-yl)-1-(6-((2-fluorophenyl)amino)pyrimidin-4-yl) piperidin-3-ol, yield: 43.4%. LCMS (ESI) [M+H]⁺ =420.32; ¹H NMR (400 MHz, CDCl₃) δ 8.30 (s, 1H), 7.76 (td, *J* = 8.0, 1.5 Hz, 1H), 7.21 - 6.97 (m, 7H), 6.63 (s, 1H), 5.91 (s, 1H), 4.73 (d, *J* = 13.5 Hz, 1H), 4.53 - 4.38 (m, 1H), 3.94 (d, *J* = 14.5 Hz, 1H), 3.85 - 3.65 (m, 2H), 3.59 (td, *J* = 10.0, 4.9 Hz, 1H), 3.02 (dt, *J* = 11.0, 5.3 Hz, 1H), 2.91 (t, *J* = 5.8 Hz, 2H), 2.86 - 2.61 (m, 4H), 1.91 (dq, *J* = 12.9, 2.9 Hz, 1H), 1.55 (qd, *J* = 12.5, 4.4 Hz, 1H).

### Example 54

### Preparation of trans-4-(3,4-dihydroisoquinolin-2(1H)-yl)-1-(6-((2-chlorophenyl) amino)pyrimidin-4-yl)piperidin-3-ol:

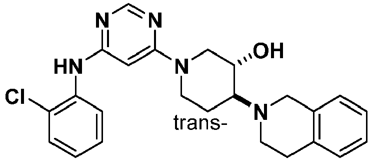

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2-chloroaniline to obtain the target compound trans-4-(3,4-dihydroisoquinolin-2(1*H*)-yl)-1-(6-((2-chlorophenyl)amino)pyrimidin-4-yl) piperidin-3-ol, yield: 17.1%. LCMS (ESI) [M+H]⁺ =436.30,438.30; ¹H NMR (400 MHz, CDCl₃) δ 8.32 (d, *J* = 1.0 Hz, 1H), 7.85 (dd, *J* = 8.2, 1.5 Hz, 1H), 7.43 (dd, *J* = 8.0, 1.5 Hz, 1H), 7.32 - 7.26 (m, 1H), 7.13 (dtd, *J* = 9.0, 5.9, 2.3 Hz, 3H), 7.08 - 6.98 (m, 2H), 6.79 (s, 1H), 5.96 (d, *J* = 1.0 Hz, 1H), 4.73 (d, *J* = 13.4 Hz, 1H), 4.45 (d, *J* = 12.8 Hz, 1H), 3.95 (d, *J* = 14.5 Hz, 1H), 3.86 - 3.66 (m, 2H), 3.59 (td, *J* = 10.0, 4.9 Hz, 1H), 3.03 (dt, *J* = 11.0, 5.3 Hz, 1H), 2.91 (t, *J* = 5.8 Hz, 2H), 2.85 - 2.63 (m, 4H), 1.92 (dq, *J* = 12.8, 2.8 Hz, 1H), 1.56 (qd, *J* = 12.5, 4.4 Hz, 1H).

### Example 55

### Preparation of trans-4-(3,4-dihydroisoquinolin-2(1H)-yl)-1-(6-((4-methoxyphenyl) amino)pyrimidin-4-yl)piperidin-3-ol:

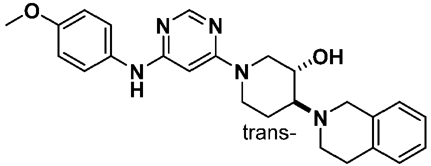

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 4-methoxyaniline to obtain the target compound trans-4-(3,4-dihydroisoquinolin-2(1*H*)-yl)-1-(6-((4-methoxyphenyl)amino)pyrimidin-4-yl) piperidin-3-ol, yield: 14%. LCMS (ESI) [M+H]⁺ =432.4; ¹H NMR (400 MHz, CDCl₃) δ 8.21 (d, *J* = 0.9 Hz, 1H), 7.23 - 7.07 (m, 5H), 7.05 - 6.98 (m, 1H), 6.98 - 6.88 (m, 2H), 6.75 (s, 1H), 5.72 (d, *J* = 1.0 Hz, 1H), 4.72 (d, *J* = 13.4 Hz, 1H), 4.36 (d, *J* = 12.6 Hz, 1H), 3.94 (d, *J* = 14.6 Hz, 1H), 3.84 (s, 3H), 3.75 - 3.65 (m, 2H), 3.56 (td, *J* = 10.0, 5.0 Hz, 1H), 3.02 (dt, *J* = 10.9, 5.2 Hz, 1H), 2.91 (t, *J=* 5.7 Hz, 2H), 2.81 - 2.62 (m, 4H), 1.90 (dd, *J=* 12.9, 3.5 Hz, 1H), 1.55 (td, *J* = 12.5, 4.3 Hz, 1H).

### Example 56

### Preparation of trans-4-(3,4-dihydroisoquinolin-2(1H)-yl)-1-(6-((2-t-butylphenyl) amino)pyrimidin-4-yl)piperidin-3-ol:

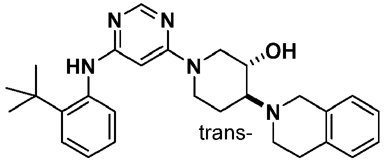

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2-t-butylaniline to obtain the target compound trans-4-(3,4-dihydroisoquinolin-2(1*H*)-yl)-1-(6-((2-t-butylphenyl)amino)pyrimidin-4-yl) piperidin-3-ol, yield: 13.7%. LCMS (ESI) [M+H]⁺ =458.4; ¹H NMR (400 MHz, CDCl₃) δ 8.22 (d, *J* = 0.9 Hz, 1H), 7.53 - 7.44 (m, 1H), 7.25 (d, *J* = 2.9 Hz, 3H), 7.17 - 7.08 (m, 3H), 7.01 (d, *J* = 6.0 Hz, 1H), 5.48 (s, 1H), 4.69 (s, 1H), 4.29 (s, 1H), 3.94 (d, *J* = 14.6 Hz, 1H), 3.69 (d, *J* = 14.8 Hz, 2H), 3.55 (td, *J* = 10.0, 4.9 Hz, 1H), 3.02 (dt, *J* = 10.9, 5.3 Hz, 1H), 2.91 (t, *J=* 5.7 Hz, 2H), 2.81 - 2.57 (m, 4H), 1.89 (dd, *J=* 12.8, 3.3 Hz, 1H), 1.67 - 1.45 (m, 1H).

### Example 57

### Preparation of trans-4-(3,4-dihydroisoquinolin-2(1H)-yl)-1-(6-((2-methoxyphenyl) amino)pyrimidin-4-yl)piperidin-3-ol:

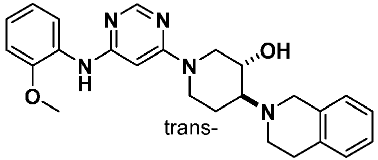

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2-methoxyaniline to obtain the target compound trans-4-(3,4-dihydroisoquinolin-2(1*H*)-yl)-1-(6-((2-methoxyphenyl)amino)pyrimidin-4-yl) piperidin-3-ol, yield: 35.16%. LCMS (ESI) [M+H]⁺ =432.4; ¹H NMR (400 MHz, Chloroform-*d*) *δ* 8.30 (s, 1H), 7.75 (d, *J=* 7.8 Hz, 1H), 7.17 - 7.10 (m, 3H), 7.09 - 6.96 (m, 4H), 6.93 (dd, *J=* 8.0, 1.5 Hz, 1H), 6.00 (s, 1H), 4.74 (d, *J=* 13.6 Hz, 1H), 4.45 (d, *J=* 12.9 Hz, 1H), 4.04 - 3.92 (m, 1H), 3.88 (s, 3H), 3.82 (br s, 1H), 3.73 (d, *J* = 14.7 Hz, 1H), 3.62 (td, *J* = 10.0, 4.9 Hz, 1H), 3.05 (dt, *J* = 10.9, 5.3 Hz, 1H), 2.96-2.94 (m, 2H), 2.83-2.73 (m, 4H), 1.92 (dd, *J* = 13.0, 3.4 Hz, 1H), 1.56 (qd, *J* = 12.4, 4.4 Hz, 1H).

### Example 58

### Preparation of trans-4-(3,4-dihydroisoquinolin-2(1H)-yl)-1-(6-((2-trifluoromethylphenyl)amino)pyrimidin-4-yl)piperidin-3-ol:

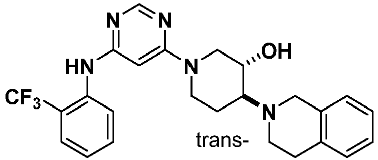

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2-trifluoromethylaniline to obtain the target compound trans-4-(3,4-dihydroisoquinolin-2(1*H*)-yl)-1-(6-((2-trifluoromethylphenyl)amino)pyrimidin-4-yl)piperidin-3-ol, yield: 14.7%. LCMS (ESI) [M+H]⁺=470.3; ¹H NMR (400 MHz, CDCl₃) δ 8.32 (s, 1H), 7.77-7.75 (d, *J* = 8.0Hz, 1H), 7.71-7.69 (d, *J* = 7.60Hz, 1H), 7.61-7.57 (t, *J* = 8.0 Hz, 1H), 7.29-7.26 (m, 1H), 7.18-7.12 (m, 3H), 7.05-7.03 (m, 1H), 6.66 (s, 1H), 5.91 (s, 1H), 4.79-4.75 (d, *J=* 14.0 Hz, 1H), 4.43-4.41(d, *J=* 10.0 Hz, 1H), 4.0-3.96 (d, *J* = 14.8 Hz, 1H), 3.79-3.72 (m, 2H), 3.64-3.58 (m, 1H), 3.08-3.03 (m, 1H), 2.85-2.70 (m, 4H), 1.96-1.92 (dd, *J* = 3.6 Hz, 12.8 Hz,1H), 1.65-1.53 (m, 2H).

### Example 59

### Preparation of trans-4-(3,4-dihydroisoquinolin-2(1H)-yl)-1-(6-(pyridin-3-ylamino) pyrimidin-4-yl)piperidin-3-ol:

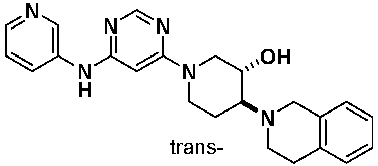

### Step I: preparation of trans-4-(3,4-dihydroisoquinolin-2(1H)-yl)-1-(6-(pyridin-3-ylamino)pyrimidin-4-yl)piperidin-3-ol:

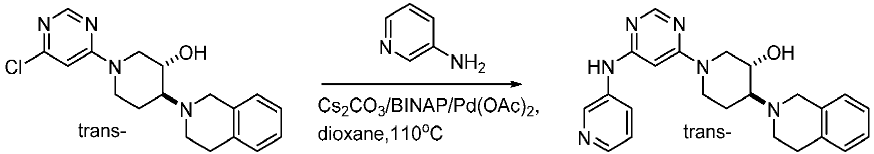

Under nitrogen protection, trans-1-(6-chloropyrimidin-4-yl)-4-(3,4-dihydroisoquinolin-2(1*H*)-yl)piperidin-3-ol (50 mg, 0.15 mmol, 1.0 eq) as a raw material was dissolved in dioxane (1,4-dioxane) (3 mL) at room temperature (20°C), and 3-aminopyridine (68 mg, 0.73 mmol, 5.0 eq), Pd(OAc) (palladium acetate) (6.0 mg, 0.029 mmol, 0.2 eq), BINAP (1,1'-binaphthyl-2,2'-bis(diphenylphosphine)) (36.0 mg, 0.058 mmol, 0.4 eq), and Cs₂CO₃ (cesium carbonate) (94.0 mg, 0.29 mmol, 2.0 eq) were added. The mixture was heated under stirring at 110°C for 12 h. The reaction was complete as indicated by LCMS, and cooled to room temperature. The reaction mixture was diluted with ethyl acetate (5 mL), filtrated, and concentrated. The crude product was separated and purified by preparative HPLC (C18, 10 mmol/L aqueous NH₄HCO₃, acetonitrile) to obtain the target compound trans-4-(3,4-dihydroisoquinolin-2(1*H*)-yl)-1-(6-(pyridin-3-ylamino)pyrimidin-4-yl)piperidin-3-ol (20 mg , yield: 34.3%). LCMS (ESI) [M+H]⁺=403.3; ¹H NMR (400 MHz, CDCl₃) δ 8.60-8.59 (d, *J* = 2.4Hz, 1H), 8.40-8.39 (dd, *J* = 1.2Hz, 4.8Hz, 1H), 8.32 (s, 1H), 7.89-7.86 (m, 1H), 7.36-7.28 (m, 1H), 7.19-7.13 (m, 3H), 7.06-7.03 (m, 1H), 6.71 (s, 1H), 5.93 (s, 1H), 4.75-4.72 (d, *J=* 13.2Hz, 1H), 4.75-4.72 (d, *J=* 12.4Hz, 1H), 4.03-3.99 (d, *J* = 14.8Hz, 1H), 3.79-3.75 (d, *J* = 10.0Hz, 1H), 3.67-3.61 (m, 1H), 3.1-3.06 (m, 1H), 2.99-2.96 (t, *J* = 5.6 Hz, 1H), 2.87-2.76 (m, 4H), 1.98-1.94 (dd, *J=* 3.6 Hz, 16.8Hz, 1H), 1.64-1.57(m, 1H).

### Example 60

### Preparation of trans-4-(3,4-dihydroisoquinolin-2(1H)-yl)-1-(6-(pyridin-4-ylamino) pyrimidin-4-yl)piperidin-3-ol:

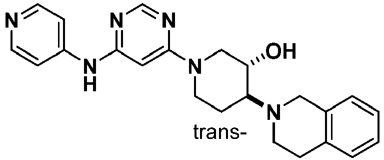

According to the method of Example 59, trans-1-(6-chloropyrimidin-4-yl)-4-(3,4-dihydroisoquinolin-2(1*H*)-yl)piperidin-3-ol was used as a raw material to undergo a substitution reaction with 4-aminopyridine to obtain the target compound trans-4-(3,4-dihydroisoquinolin-2(1*H*)-yl)-1-(6-(pyridin-4-ylamino)pyrimidin-4-yl)piperidin-3-ol, yield: 51.4%. LCMS (ESI) [M+H]⁺=403.3; ¹H NMR (400 MHz, CDCl₃) δ 8.47-8.46 (d, *J* = 6.4Hz, 2H), 8.39 (s, 1H), 7.44-7.42 (d, *J=* 6.4Hz, 2H), 7.20-7.13(m, 3H), 7.05-6.96 (m, 2H), 6.14 (s, 1H), 4.78-4.75 (d, *J* = 13.2Hz, 1H), 4.57-4.54(d, *J* = 10.4 Hz, 1H), 3.99-3.95 (d, *J =* 14.4 Hz, 1H), 3.75-3.71 (d, *J =* 14.8 Hz, 1H),3.67-3.60 (m, 1H), 3.08-3.02 (m, 1H), 2.95-2.92(t, *J* = 5.6 Hz, 1H), 2.91-2.79 (m, 2H), 2.76-2.67(m, 2H),1.99-1.95(m, 1H),1.64-1.54(m, 1H).

### Example 61

### Preparation of trans-4-(3,4-dihydroisoquinolin-2(1H)-yl)-1-(6-(pyridin-2-ylamino) pyrimidin-4-yl)piperidin-3-ol:

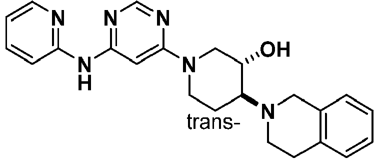

According to the method of Example 59, trans-1-(6-chloropyrimidin-4-yl)-4-(3,4-dihydroisoquinolin-2(1*H*)-yl)piperidin-3-ol was used as a raw material to undergo a substitution reaction with 2-aminopyridine to obtain the target compound trans-4-(3,4-dihydroisoquinolin-2(1*H*)-yl)-1-(6-(pyridin-2-ylamino)pyrimidin-4-yl)piperidin-3-ol, yield: 51.4%. LCMS (ESI) [M+H]⁺=403.36; ¹H NMR (400 MHz, CDCl₃) δ 8.47-8.46 (d, *J* = 6.4Hz, 2H), 8.39 (s, 1H), 7.44-7.42 (d, *J* = 6.4Hz, 2H), 7.20-7.13(m, 3H), 7.05-6.96 (m, 2H), 6.14 (s, 1H), 4.78-4.75 (d, *J=* 13.2Hz, 1H), 4.57-4.54(d, *J=* 10.4 Hz, 1H), 3.99-3.95 (d, *J* = 14.4 Hz, 1H), 3.75-3.71 (d, *J* = 14.8 Hz, 1H),3.67-3.60 (m, 1H), 3.08-3.02 (m, 1H), 2.95-2.92(t, *J* = 5.6 Hz, 1H), 2.91-2.79 (m, 2H), 2.76-2.67(m, 2H),1.99-1.95(m, 1H),1.64-1.54(m, 1H).

### Example 62

### Preparation of trans-4-(3,4-dihydroisoquinolin-2(1H)-yl)-1-(6-((4-chlorophenyl)amino) pyrimidin-4-yl)piperidin-3-ol:

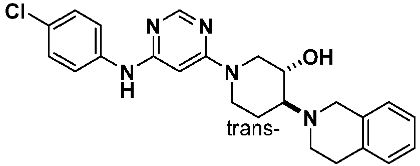

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 4-chloroaniline to obtain the target compound trans-4-(3,4-dihydroisoquinolin-2(1*H*)-yl)-1-(6-((4-chlorophenyl)amino)pyrimidin-4-yl)piper idin-3-ol, yield: 88.9%. LCMS(ESI)[M+H]⁺ =436.3; ¹H NMR (400 MHz, CDCl₃) *δ* 11.69 (s, 1H), 8.14 (s, 1H), 7.41 (d, *J=* 8.4 Hz, 2H), 7.29 (t, *J=* 7.2 Hz, 1H), 7.25 - 7.12 (m, 4H), 7.06 (d, *J=* 7.5 Hz, 1H), 5.78 (s, 1H), 4.53 (d, *J=* 14.9 Hz, 1H), 4.44 - 3.74 (m, 4H), 3.66 - 3.25 (m, 4H), 3.19 - 2.78 (m, 4H), 2.14 (d, *J=* 11.5 Hz, 1H), 1.80 - 1.58 (m, 1H).

### Example 63

### Preparation of trans-4-(3,4-dihydroisoquinolin-2(1H)-yl)-1-(6-((tetrahydro-2H-pyran-4-yl)amino)pyrim idin-4-yl)piperidin-3-ol:

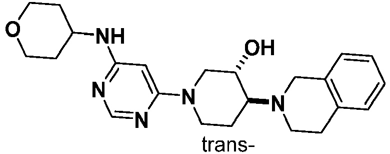

According to the method of Example 59, trans-1-(6-chloropyrimidin-4-yl)-4-(3,4-dihydroisoquinolin-2(1*H*)-yl)piperidin-3-ol was used as a raw material to undergo a substitution reaction with 4-aminotetrahydropyran to obtain the target compound trans-4-(3,4-dihydroisoquinolin-2(1*H*)-yl)-1-(6-((tetrahydro-2*H*-pyran-4-yl)amino)pyrimidin-4-yl)piperidin-3-ol, yield: 10.2%. LCMS (ESI) [M+H]⁺ =410.33; ¹H NMR (400 MHz, CDCl₃) *δ* 8.16 (s, 1H), 7.19 - 7.08 (m, 3H), 7.06 - 6.98 (m, 1H), 5.50 (s, 1H), 4.72 (dd, *J* = 24.8, 10.6 Hz, 2H), 4.43 (dd, *J* = 12.6, 2.8 Hz, 1H), 3.97 (dd, *J* = 20.8, 13.3 Hz, 3H), 3.74 (dd, *J* = 35.1, 11.2 Hz, 3H), 3.71-3.51 (m, 3H), 3.08 - 2.97 (m, 1H), 2.91 (t, *J* = 5.6 Hz, 2H), 2.83 - 2.60 (m, 4H), 2.01 (d, *J* = 11.9 Hz, 2H), 1.91 (dd, *J* = 12.8, 2.8 Hz, 1H), 1.62 - 1.43 (m, 3H).

### Example 64

### Preparation of trans-4-(3,4-dihydroisoquinolin-2(1H)-yl)-1-(6-((4-(oxetan-3-yl)phenyl) amino)pyrimidin-4-yl)piperidin-3-ol:

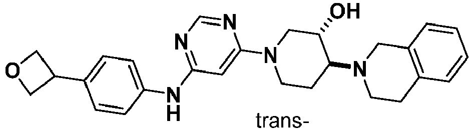

According to the method of Example 59, trans-1-(6-chloropyrimidin-4-yl)-4-(3,4-dihydroisoquinolin-2(1*H*)-yl)piperidin-3-ol was used as a raw material to undergo a substitution reaction with 4-(oxetan-3-yl)aniline to obtain the target compound trans-4-(3,4-dihydroisoquinolin-2(1*H*)-yl)-1-(6-((4-(oxetan-3-yl)phenyl)amino)pyrimidin-4-y l)piperidin-3-ol, yield: 17.6%. LCMS (ESI) [M+H]⁺=458.36; ¹H NMR (400 MHz, CDCl₃) *δ* 8.26 (s, 1H), 7.41 (d, *J=* 8.3 Hz, 2H), 7.28 (d, *J=* 8.4 Hz, 2H), 7.17 - 7.08 (m, 3H), 7.01 (d, *J* = 5.9 Hz, 1H), 6.83 (s, 1H), 5.96 (s, 1H), 5.09 (dd, *J* = 8.3, 6.0 Hz, 2H), 4.78 (dd, *J* = 17.3, 10.9 Hz, 3H), 4.39 (d, *J* = 9.8 Hz, 1H), 4.29 - 4.19 (m, 1H), 3.93 (d, *J* = 14.6 Hz, 1H), 3.68 (d, *J=* 14.6 Hz, 2H), 3.57 (td, *J=* 10.0, 4.9 Hz, 1H), 3.05 - 2.96 (m, 1H), 2.90 (t, *J=* 5.5 Hz, 2H), 2.80-2.62 (m, 4H), 1.95 - 1.86 (m, 1H), 1.61 - 1.45 (m, 1H).

### Example 65

### Preparation of trans-4-(3,4-dihydroisoquinolin-2(1H)-yl)-1-(6-((2-(methylsulfonyl) phenyl)amino)pyrimidin-4-yl)piperidin-3-ol:

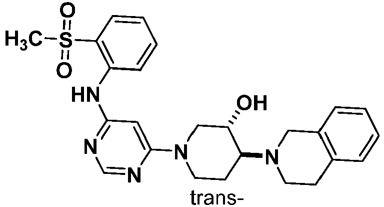

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2-(methylsulfonyl)aniline to obtain the target compound trans-4-(3,4-dihydroisoquinolin-2(1*H*-yl)-1-(6-((2-(methylsulfonyl)phenyl)amino)pyrimidin-4-yl)piperidin-3-ol, yield: 7.9%. LCMS (ESI) [M+H]⁺ =480.33; ¹H NMR (400 MHz, CDCl₃) *δ* 8.38 (d, *J* = 17.0 Hz, 2H), 8.24 (d, *J* = 8.1 Hz, 1H), 7.94 (dd, *J* = 8.0, 1.5 Hz, 1H), 7.67 - 7.53 (m, 1H), 7.22 - 7.10 (m, 4H), 7.01 (dd, *J* = 8.0, 5.6 Hz, 1H), 5.98 (s, 1H), 4.52-4.72 (m, 2H), 3.95 (d, *J* = 14.5 Hz, 1H), 3.71 (d, *J* = 14.5 Hz, 2H), 3.61 (td, *J =* 10.1, 4.9 Hz, 1H), 3.07 (s, 3H), 3.03 (dd, *J* = 11.0, 5.3 Hz, 1H), 2.91 (dd, *J* = 11.2, 5.7 Hz, 2H), 2.88 - 2.80 (m, 1H), 2.79 - 2.72 (m, 1H), 2.71 - 2.62 (m, 2H), 1.95 (dd, *J* = 12.9, 3.1 Hz, 1H), 1.25 (m, 1H).

### Example 66

### Preparation of trans-1-(6-([1,1'-biphenyl]-2-ylamino)pyrimidin-4-yl)-4-(3,4-dihydro-isoquinolin-2(1H)-yl)piperidin-3-ol:

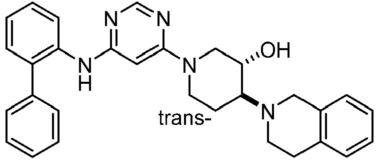

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with [1,1'-biphenyl]-2-amine to obtain the target compound trans-1-(6-([1,1'-biphenyl]-2-ylamino)pyrimidin-4-yl)-4-(3,4-dihydroisoquinolin-2(1H)-yl) piperidin-3-ol, yield: 52.8%. LCMS(ESI): [M+H]⁺= 478.40; ¹H NMR (400 MHz, DMSO-*d*₆) *δ* 8.27 (s, 1H), 7.99 (s, 1H), 7.48 (d, *J=* 7.8 Hz, 1H), 7.40-7.34 (m, 6H), 7.33-7.23 (m, 2H), 7.13-6.99 (m, 4H), 5.58 (s, 1H), 4.66 (s, 1H), 4.14 (dd, *J* = 29.9, 10.3 Hz, 2H), 3.79 (q, *J* = 14.8 Hz, 2H), 3.51-3.44 (m, 1H), 2.95-2.68 (m, 5H), 2.67-2.52 (m, 2H), 1.74 (d, *J=* 10.6 Hz, 1H), 1.36 (dd, *J=* 20.8, 11.8 Hz, 1H).

### Example 67

### Preparation of trans-4-(3,4-dihydroisoquinolin-2(1H)-yl)-1-(6-((3-(pyrrolidin-1-yl) phenyl)amino)pyrimidin-4-yl)piperidin-3-ol:

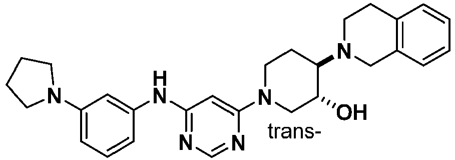

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3-pyrrolidin-1-ylaniline to obtain the target compound trans-4-(3,4-dihydroisoquinolin-2(1*H*)-yl)-1-(6-((3-(pyrrolidin-1-yl)phenyl)amino)pyrimidin-4-yl)piperidin-3-ol, yield: 44.8%. LCMS (ESI) [M+H]⁺ = 471.37; ¹H NMR (400 MHz, Chloroform-*d*) *δ* 11.32 (s, 1H), 8.12 (s, 1H), 7.27 (dt, *J* = 16.3, 7.5 Hz, 3H), 7.19 (d, *J* = 7.4 Hz, 1H), 7.07 (d, *J=* 7.5 Hz, 1H), 6.60 - 6.49 (m, 2H), 6.44 (s, 1H), 5.91 (s, 1H), 4.53 (d, *J=* 14.8 Hz, 1H), 4.34 (d, *J* = 14.8 Hz, 1H), 3.97 (s, 2H), 3.68 - 3.23 (m, 9H), 3.14 (d, *J* = 16.8 Hz, 1H), 2.96 (dd, *J* = 25.8, 13.3 Hz, 2H), 2.14 (d, *J* = 12.7 Hz, 1H), 2.08 - 2.00 (m, 4H), 1.80 - 1.62 (m, 1H).

### Example 68

### Preparation of trans-4-(3,4-dihydroisoquinolin-2(1H)-yl)-1-(6-(thiazol-2-ylamino) pyrimidin-4-yl)piperidin-3-ol:

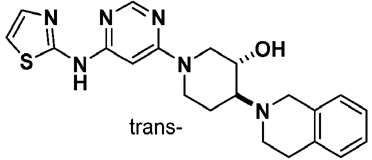

According to the method of Example 59, trans-1-(6-chloropyrimidin-4-yl)-4-(3,4-dihydroisoquinolin-2(1*H*)-yl)piperidin-3-ol was used as a raw material to undergo a substitution reaction with 2-aminothiazole to obtain the target compound trans-4-(3,4-dihydroisoquinolin-2(1*H*)-yl)-1-(6-(thiazol-2-ylamino)pyrimidin-4-yl)piperidin-3-ol, yield: 48.96%. LCMS (ESI) [M+H]⁺ =409.3; ¹H NMR (400 MHz, Chloroform-*d*) *δ* 8.43 (s, 1H), 7.41 (d, *J* = 3.3 Hz, 1H), 7.15 (dt, *J* = 10.3, 3.6 Hz, 3H), 7.03 (d, *J* = 6.9 Hz, 1H), 6.88 (d, *J* = 3.7 Hz, 1H), 6.23 (s, 1H), 4.74 (d, *J* = 13.5 Hz, 1H), 4.58 (d, *J* = 12.8 Hz, 1H), 4.03 (d, *J* = 14.8 Hz, 1H), 3.79 (d, *J* = 14.7 Hz, 1H), 3.70-3.67 (m, 1H), 3.10-3.08 (m, 1H), 2.98 (s, 2H), 2.93 - 2.74 (m, 4H), 2.02 - 1.94 (m, 1H), 1.66 - 1.54 (m, 1H).

### Example 69

### Preparation of trans-4-(3,4-dihydroisoquinolin-2(1H)-yl)-1-(6-((1-methyl-1H-pyrazol-5-yl)amino)pyrimidin-4-yl)piperidin-3-ol:

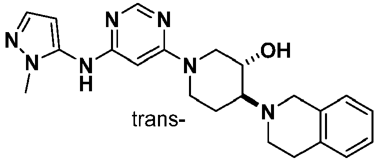

According to the method of Example 59, trans-1-(6-chloropyrimidin-4-yl)-4-(3,4-dihydroisoquinolin-2(1*H*)-yl)piperidin-3-ol was used as a raw material to undergo a substitution reaction with 1-methyl-1H-pyrazole-5-amine to obtain the target compound trans-4-(3,4-dihydroisoquinolin-2(1*H*)-yl)-1-(6-((1-methyl-1*H*-pyrazol-5-yl)amino) pyrimidin-4-yl)piperidin-3-ol, yield: 42.8%. LCMS (ESI) [M+H]⁺ =407.6; ¹H NMR (400 MHz, Chloroform-*d*) *δ* 8.24 (d, *J* = 1.0 Hz, 1H), 7.51 (d, *J=* 2.0 Hz, 1H), 7.15-7.10 (m, 3H), 7.06 - 6.97 (m, 1H), 6.39 (br s, 1H), 6.16 (d, *J* = 1.9 Hz, 1H), 5.57 (d, *J* = 1.1 Hz, 1H), 4.62 (d, *J* = 13.4 Hz, 1H), 4.46 (d, *J* = 12.6 Hz, 1H), 3.94 (d, *J* = 14.5 Hz, 1H), 3.76 (s, 3H), 3.70 (d, *J* = 14.9 Hz, 2H), 3.56 (td, *J* = 10.0, 4.8 Hz, 1H), 3.02 (dt, *J* = 11.0, 5.2 Hz, 1H), 2.91 (t, *J* = 5.8 Hz, 2H), 2.85 - 2.58 (m, 4H), 1.90 (dd, *J* = 12.9, 3.3 Hz, 1H), 1.52 (qd, *J* = 12.5, 4.4 Hz, 1H).

### Example 70

### Preparation of trans-4-(3,4-dihydroisoquinolin-2(1H)-yl)-1-(6-(4-(trifluoromethyl)-phenoxy)pyrimidin-4-yl)piperidin-3-ol:

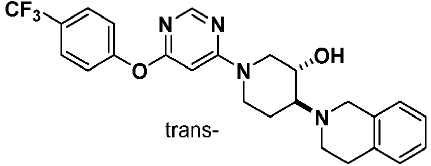

### Step I: preparation of 4-chloro-6-(4-(trifluoromethyl)phenoxy)pyrimidine:

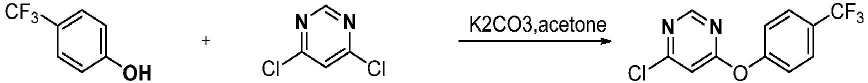

*p*-trifluoromethylphenol (0.326 g, 2.01 mmol), 4,6-dichloropyrimidine (0.3 g, 2.01 mmol) and potassium carbonate (0.292 g, 2.11 mmol) were dissolved in acetone (5 mL), and the reaction mixture was stirred at room temperature (20-25°C) for 3 h. The reaction was complete as indicated by TLC (pure PE). The reaction mixture was extracted three times with ethyl acetate (20 mL for each), and the ethyl acetate phases were combined, washed once with 10 mL water and once with 10 mL saturated sodium chloride solution, dried over anhydrous sodium sulfate for 10 min, and filtrated. The crude product was recrystallized in petroleum ether at 0°C to obtain 4-chloro-6-(4-(trifluoromethyl)phenoxy)pyrimidine (0.37 g, yield: 66.9%) as a white solid. ¹H NMR (400 MHz, CDCl₃) *δ* 8.59 (s, 1H), 7.73 (d, *J =* 8.6 Hz, 2H), 7.28 (d, *J=* 8.5 Hz, 2H), 7.02 (s, 1H).

### Step II: preparation of trans-4-(3,4-dihydroisoquinolin-2(1H)-yl)-1-(6-(4-(trifluoromethyl) phenoxy)pyrimidin-4-yl)piperidin-3 -ol:

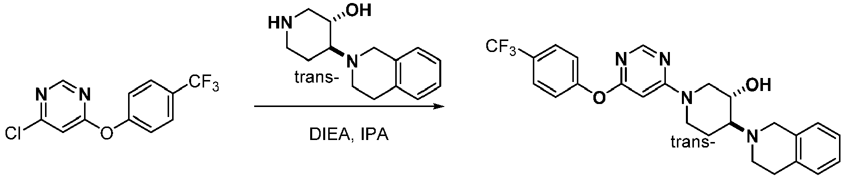

4-chloro-6-(4-(trifluoromethyl)phenoxy)pyrimidine (40 mg, 0.145 mmol), trans-4-(3,4-dihydroisoquinolin-2(1H)-yl)piperidin-3-ol (37 mg, 0.16 mmol) and 2-isopropylethylamine (37 mg, 0.291 mmol) were dissolved in isopropanol (0.8 mL), and the reaction mixture was stirred at 100°C for 3 h. The reaction mixture was extracted three times with ethyl acetate (10 mL for each), and the ethyl acetate phases were combined, washed once with 10 mL water and once with 10 mL saturated sodium chloride solution, dried over anhydrous sodium sulfate for 10 min, and filtrated. The crude product was separated and purified by preparative HPLC (C18, 10 mmol/L aqueous NH₄HCO₃, acetonitrile) to obtain trans-4-(3,4-dihydroisoquinolin-2(1*H*)-yl)-1-(6-(4-(trifluoromethyl)phenoxy)pyrimidin-4-yl) piperidin-3-ol (45.8 mg, yield: 66%). LCMS (ESI) [M+H]⁺ =471.4; ¹H NMR (400 MHz, CDCl₃) *δ* 8.31 (s, 1H), 7.67 (d, *J* = 8.4 Hz, 2H), 7.25 (d, *J* = 11.2 Hz, 2H), 7.19 - 7.09 (m, 3H), 7.03 (d, *J* = 6.7 Hz, 1H), 6.11 (s, 1H), 4.74 (d, *J* = 13.5 Hz, 1H), 4.57 (d, *J* = 12.7 Hz, 1H), 3.99 (d, *J* = 14.5 Hz, 1H), 3.75 (d, *J* = 14.7 Hz, 1H), 3.64-3.62 (m, 1H), 3.08-3.05 (m, 1H), 2.95 (s, 2H), 2.88-2.72 (m, 4H), 1.97 (d, *J* = 12.8 Hz, 1H), 1.60 (td, *J* = 12.4, 4.4 Hz, 1H).

### Example 71

### Preparation of trans-4-(3,4-dihydroisoquinolin-2(1H)-yl)-1-(6-(2-(trifluoromethyl) phenoxy)pyrimidin-4-yl)piperidin-3-ol:

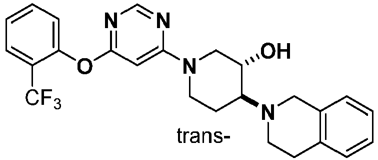

According to the method of Example 70, after o-trifluoromethylphenol reacted with 4,6-dichloropyrimidine in a substitution reaction, the product was subjected to a further substitution reaction with trans-1-(6-chloropyrimidin-4-yl)-4-(3,4-dihydroisoquinolin-2(1*H*)-yl)piperidin-3-ol to obtain the target compound trans-4-(3,4-dihydroisoquinolin-2(1*H*)-yl)-1-(6-(2-(trifluoromethyl)phenoxy)pyrimidin-4-yl)piperidin-3-ol, yield: 60.1%. LCMS (ESI) [M+H]⁺ =471.4; ¹H NMR (400 MHz, CDCl₃) *δ* 8.28 (s, 1H), 7.70 (d, *J* = 7.8 Hz, 1H), 7.59 (t, *J* = 7.9 Hz, 1H), 7.33 (t, *J* = 7.7 Hz, 1H), 7.24 (s, 1H), 7.16-7.11 (m, 3H), 7.07 - 7.00 (m, 1H), 6.15 (s, 1H), 4.72 (d, *J* = 13.1 Hz, 1H), 4.61 (d, *J* = 12.6 Hz, 1H), 3.99 (d, *J* = 14.5 Hz, 1H), 3.74 (d, *J* = 14.6 Hz, 2H), 3.64 (td, *J* = 10.0, 4.9 Hz, 1H), 3.06 (dd, *J* = 11.1, 5.5 Hz, 1H), 2.95-2.91 (m, 2H), 2.90 - 2.64 (m, 4H), 2.04 - 1.91 (m, 1H), 1.60 (qd, *J* = 12.5, 4.4 Hz, 1H).

### Example 72

### Preparation of trans-4-(3,4-dihydroisoquinolin-2(1H)-yl)-1-(6-(3-(trifluoromethyl) phenoxy)pyrimidin-4-yl)piperidin-3-ol:

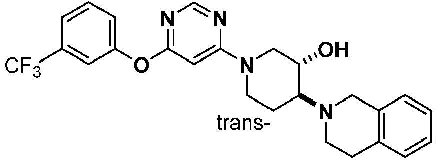

According to the method of Example 70, after m-trifluoromethylphenol reacted with 4,6-dichloropyrimidine in a substitution reaction, the product was subjected to a further substitution reaction with trans-1-(6-chloropyrimidin-4-yl)-4-(3,4-dihydroisoquinolin-2(1*H*)-yl)-piperidin-3-ol to obtain the target compound trans-4-(3,4-dihydroisoquinolin-2(1*H*)-yl)-1-(6-(3-(trifluoromethyl)phenoxy)pyrimidin-4-yl)piperidin-3-ol, yield: 52.53%. LCMS (ESI) [M+H]⁺ =471.4; ¹H NMR (400 MHz, CDCl₃) δ 8.30 (s, 1H), 7.58 - 7.45 (m, 2H), 7.40 (s, 1H), 7.33 (d, *J* = 7.9 Hz, 1H), 7.18-7.14 (m, 3H), 7.04 (d, *J* = 6.6 Hz, 1H), 6.10 (s, 1H), 4.74 (d, *J* = 13.2 Hz, 1H), 4.56 (d, *J* = 12.9 Hz, 1H), 4.04 (d, *J* = 13.6 Hz, 1H), 3.80 (d, *J* = 10.0 Hz, 1H), 3.67 (s, 1H), 3.10 (s, 1H), 2.99 (s, 2H), 2.91-2.80 (m, 4H), 1.98 (d, *J* = 12.8 Hz, 1H), 1.69 - 1.53 (m, 1H).

### Example 73

### Preparation of trans-4-(3,4-dihydroisoquinolin-2(1H)-yl)-1-(6-((3-morpholinophenyl) amino)pyrimidin-4-yl)piperidin-3-ol:

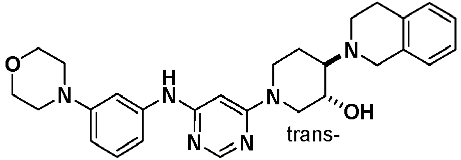

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3-morpholinoaniline to obtain the target compound trans-4-(3,4-dihydroisoquinolin-2(1*H*)-yl)-1-(6-((3-morpholinophenyl)amino)pyrimidin-4-yl) piperidin-3-ol, yield: 21.5%. LCMS (ESI) [M+H]⁺ = 487.39; ¹H NMR (400 MHz, CDCl₃) *δ* 11.20 (s, 1H), 8.12 (s, 1H), 7.34 (t, *J* = 8.1 Hz, 1H), 7.26 - 7.18 (m, 2H), 7.15 (d, *J* = 7.4 Hz, 1H), 7.05 (d, *J* = 7.5 Hz, 1H), 6.94 - 6.79 (m, 3H), 5.86 (s, 1H), 4.68 - 4.25 (m, 2H), 4.06 - 3.77 (m, 6H), 3.71 - 3.45 (m, 3H), 3.39 - 3.08 (m, 7H), 2.95 (s, 2H), 2.16 (d, *J* = 12.2 Hz, 1H), 1.72 (d, *J* = 12.6 Hz, 1H).

### Example 74

### Preparation of trans-1-(6-([1,1'-biphenyl]-3-ylamino)pyrimidin-4-yl)-4-(3,4-dihydroisoquinolin-2(1H)-yl)piperidin-3-ol:

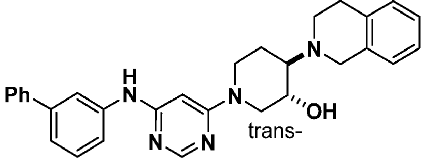

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3-aminobiphenyl to obtain the target compound trans-1-(6-([1,1'-biphenyl]-3-ylamino)pyrimidin-4-yl)-4-(3,4-dihydroisoquinolin-2(1*H*)-yl) piperidin-3-ol, yield: 45.1%. LCMS (ESI) [M+H]⁺ = 478.38; ¹H NMR (400 MHz, CDCl₃) δ 8.28 (s, 1H), 7.61 - 7.57 (m, 2H), 7.50 - 7.42 (m, 4H), 7.40 - 7.33 (m, 2H), 7.33 - 7.23 (m, 2H), 7.15-7.02 (m, 3H), 7.04 - 6.96 (m, 1H), 6.05 (s, 1H), 4.68 (d, *J* = 13.4 Hz, 1H), 4.46 (dd, *J* = 13.4, 4.9 Hz, 1H), 3.92 (d, *J* = 14.5 Hz, 1H), 3.68 (d, *J* = 14.6 Hz, 1H), 3.58 (td, *J* = 10.0, 4.8 Hz, 1H), 3.00 (dt, *J* = 10.9, 5.3 Hz, 1H), 2.89 (t, *J* = 5.8 Hz, 2H), 2.83 - 2.60 (m, 4H), 2.00 - 1.80 (m, 2H), 1.54 (qd, *J* = 12.5, 4.3 Hz, 1H).

### Example 75

### Preparation of trans-1-(6-((cyclobutylamino))pyrimidin-4-yl)-4-(3,4-dihydroisoquinolin-2(1H)-yl)piperidin-3-ol:

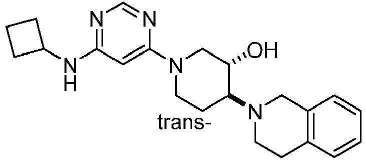

### Step I: preparation of 6-chloro-N-cyclobutylpyrimidinyl-4-amine:

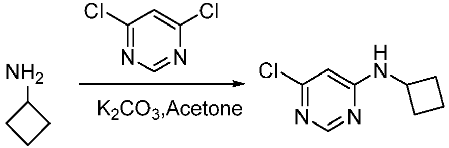

Cyclobutylamine (200 mg, 2.81 mmol, 1.0 eq) was dissolved in acetone (5 mL), and 4,6-dichloropyrimidine (420 mg, 2.81 mmol, 1.0 eq) and potassium carbonate (580 mg, 4.21 mmol, 2.0 eq) were added thereto. The mixture was stirred at room temperature overnight (16 h). The compounds were filtrated, and the filtrate was concentrated. 20 mL water was added to the reaction mixture, which was extracted three times with ethyl acetate (5 mL for each). The ethyl acetate phases were combined, washed once with 10 mL water and once with 10 mL saturated sodium chloride solution, dried over anhydrous sodium sulfate for 10 min, filtrated and concentrated. The crude product was separated and purified by column chromatography (PE:EA=0-20%) to obtain 6-chloro-N-cyclobutylpyrimidin-4-amine (208 mg, yield: 40.3%) as a yellow solid. LCMS (ESI) [M+H]⁺ =184.1.

### Step II: preparation of trans-1-(6-((cyclobutylamino))pyrimidin-4-yl)-4-(3,4-dihydroisoquinolin-2(1H)-yl)piperidin-3-ol:

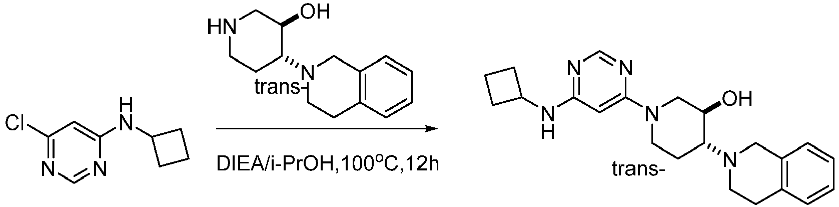

6-chloro-N-cyclobutylpyrimidin-4-amine (50 mg, 0.272 mmol, 1.0 eq) was dissolved in isopropanol (2 mL), and trans-4-(3,4-dihydroisoquinolin-2(1H)-yl)piperidin-3-ol (69 mg, 0.299 mmol, 1.1 eq) and N,N-diisopropylethylamine (70 mg, 0.544 mmol, 2.0 eq) were added thereto. The mixture was heated under stirring at 100°C overnight (16 h). The reaction mixture was adjusted to pH 7 with 1 N aqueous sodium hydroxide, and extracted three times with ethyl acetate (5 mL for each). The ethyl acetate phases were combined, washed once with 10 mL water and once with 10 mL saturated sodium chloride solution, dried over anhydrous sodium sulfate for 10 min, and filtrated. The crude product was separated and purified by Prep-HPLC (C18, 10 mmol/L aqueous NH₄HCO₃, acetonitrile) to obtain trans-1-(6-((cyclobutylamino))pyrimidin-4-yl)-4-(3,4-dihydroisoquinolin-2(1*H*)-yl)piperidin-3-ol (16.8 mg, yield: 16.3%). LCMS (ESI) [M+H]⁺ =380.4; ¹H NMR (400 MHz, CDCl₃) *δ* 7.97 (s, 1H), 7.08-7.01 (m, 4H), 6.94 (d, *J=* 6.8 Hz, 1H), 5.54 (s, 1H), 4.64 (s, 1H), 4.22 (d, *J* = 10.8 Hz, 2H), 3.81 - 3.73 (m, 2H), 3.55 (s, 1H), 2.89 - 2.74 (m, 5H), 2.67 - 2.55 (m, 2H), 2.25 (d, *J=* 8.0 Hz, 2H), 1.91 - 1.76 (m, 3H), 1.68 - 1.59 (m, 2H), 1.45 - 1.42 (m, 1H).

### Example 76

### Preparation of trans-4-(3.4-dihydroisoquinolin-2(1H)-yl)-1-(6-((1-methyl-1H-pyrazol-5-yl)amino)pyrimidin-4-yl)piperidin-3-ol:

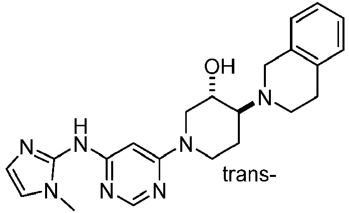

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 1-methyl-1H-pyrazole-5-amine to obtain the target compound trans-4-(3,4-dihydroisoquinolin-2(1*H*)-yl)-1-(6-((1-methyl-1*H*-pyrazol-5-yl)amino)pyrimidin -4-yl)piperidin-3-ol, yield: 3.6%. LCMS (ESI) [M+H]⁺ = 406.33; ¹H NMR (400 MHz, Chloroform-*d*) *δ* 8.25 (s, 1H), 7.17 - 7.09 (m, 3H), 7.04 - 6.99 (m, 1H), 6.82 (s, 1H), 6.66 (s, 1H), 6.43 (s, 1H), 4.64 (t, *J* = 12.8 Hz, 2H), 3.93 (d, *J* = 14.5 Hz, 1H), 3.69 (d, *J* = 14.6 Hz, 1H), 3.61 (dd, *J* = 9.9, 5.0 Hz, 1H), 3.56 (s, 3H), 3.01 (dt, *J* = 10.7, 5.3 Hz, 1H), 2.90 (t, *J* = 5.8 Hz, 2H), 2.86 - 2.62 (m, 5H), 1.90 (dd, *J* = 13.2, 3.4 Hz, 1H), 1.55 (qd, *J* = 12.5, 4.3 Hz, 1H).

### Example 77

### Preparation of trans-1-(6-((3,5-difluorophenyl)amino)pyrimidin-4-yl)-4-(3,4-dihydroisoquinolin-2(1H)-yl)piperidin-3-ol:

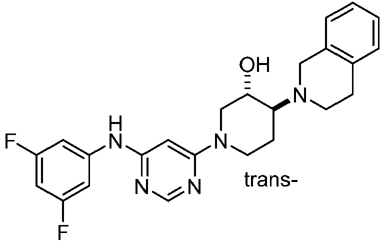

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3,5-difluoroaniline to obtain the target compound trans-1-(6-((3,5-difluorophenyl)amino)pyrimidin-4-yl)-4-(3,4-dihydroisoquinolin-2(1*H*)-yl)-piperidin-3-ol, yield: 46.8%. LCMS (ESI) [M+H]⁺ = 438.3; ¹H NMR (400 MHz, CDCl₃) *δ* 8.31 (s, 1H), 7.22 - 7.09 (m, 4H), 7.04 - 7.00 (m, 1H), 6.98 - 6.92 (m, 2H), 6.51 (td, *J* = 8.9, 2.1 Hz, 1H), 5.98 (s, 1H), 4.71 (d, *J* = 13.4 Hz, 1H), 4.48 (dd, *J* = 11.9, 4.8 Hz, 1H), 3.94 (d, *J* = 14.5 Hz, 1H), 3.70 (d, *J* = 14.6 Hz, 1H), 3.59 (td, *J* = 10.0, 4.8 Hz, 1H), 3.02 (dt, *J* = 11.1, 5.4 Hz, 1H), 2.91 (t, *J* = 5.8 Hz, 2H), 2.81 (ddd, *J* = 21.7, 11.0, 2.4 Hz, 2H), 2.74 - 2.64 (m, 2H), 1.92 (dd, *J* = 12.9, 3.4 Hz, 1H), 1.55 (qd, *J* = 12.5, 4.3 Hz, 1H).

### Example 78

### Preparation of trans-1-(6-((3,4-difluorophenyl)amino)pyrimidin-4-yl)-4-(3,4-dihydroisoquinolin-2(1H)-yl)piperidin-3-ol:

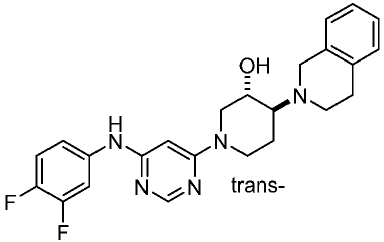

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3,4-difluoroaniline to obtain the target compound trans-1-(6-((3,4-difluorophenyl)amino)pyrimidin-4-yl)-4-(3,4-dihydroisoquinolin-2(1*H*)-yl)-piperidin-3-ol, yield: 29.3%. LCMS (ESI) [M+H]⁺ = 438.34; ¹H NMR (400 MHz, CDCl₃) δ 8.26 (s, 1H), 7.32 - 7.22 (m, 1H), 7.19 - 7.09 (m, 4H), 7.04 - 6.92 (m, 3H), 5.85 (s, 1H), 4.69 (d, *J* = 13.4 Hz, 1H), 4.49 - 4.39 (m, 1H), 3.94 (d, *J* = 14.5 Hz, 1H), 3.69 (d, *J* = 14.5 Hz, 1H), 3.58 (td, *J* = 10.0, 4.8 Hz, 1H), 3.02 (dt, *J* = 11.0, 5.4 Hz, 1H), 2.91 (t, *J* = 5.8 Hz, 2H), 2.83 - 2.73 (m, 2H), 2.67 (td, *J* = 9.9, 9.3, 3.3 Hz, 2H), 1.91 (dd, *J* = 12.9, 3.4 Hz, 1H), 1.54 (qd, *J* = 12.5, 4.4 Hz, 1H).

### Example 79

### Preparation of trans-4-(3,4-dihydroisoquinolin-2(1H)-yl)-1-(6-((3,4,5-trifluorophenyl) amino)pyrimidin-4-yl)piperidin-3-ol:

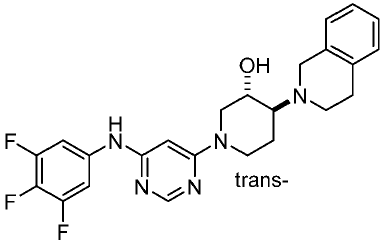

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3,4,5-trifluoroaniline to obtain the target compound trans-4-(3,4-dihydroisoquinolin-2(1*H*)-yl)-1-(6-((3,4,5-trifluorophenyl)amino)pyrimidin-4-yl) piperidin-3-ol, yield: 33.9%. LCMS (ESI) [M+H]⁺ = 456.32; ¹H NMR (400 MHz, CDCl₃) *δ* 8.29 (s, 1H), 7.18 - 7.05 (m, 5H), 7.03 - 7.00 (m, 1H), 6.89 (s, 1H), 5.87 (s, 1H), 4.71 (d, *J* = 13.4 Hz, 1H), 4.50 - 4.42 (m, 1H), 3.95 (d, *J* = 14.5 Hz, 1H), 3.71 (d, *J* = 14.6 Hz, 1H), 3.59 (td, *J* = 10.0, 4.9 Hz, 1H), 3.03 (dt, *J* = 11.0, 5.3 Hz, 1H), 2.92 (t, *J* = 5.8 Hz, 2H), 2.86 - 2.75 (m, 2H), 2.75 - 2.64 (m, 2H), 1.93 (dd, *J* = 12.9, 3.3 Hz, 1H), 1.55 (qd, *J* = 12.5, 4.3 Hz, 1H).

### Example 80

### Preparation of trans-4-(3,4-dihydroisoquinolin-2(1H)-yl)-1-(6-((2-(pyrrolidin-1-yl) phenyl)amino)pyrimidin-4-yl)piperidin-3-ol

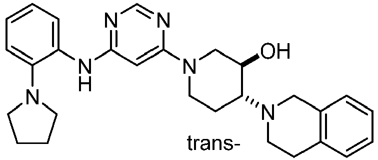

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2-(pyrrolidin-1-yl)aniline to obtain the target compound trans-4-(3,4-dihydroisoquinolin-2(1*H*)-yl)-1-(6-((2-(pyrrolidin-1-yl)phenyl)amino)pyrimidin-4-yl)piperidin-3-ol, yield: 4.2%. LCMS (ESI) [M+H]⁺ =471.5; ¹H NMR (400 MHz, CDCl₃) *δ* 8.25 (s, 1H), 7.37 (dd, *J* = 8.0 Hz, 0.8 Hz, 1H), 7.17 - 7.08 (m, 4H), 7.01 (dd, *J* = 7.2 Hz, 2.4 Hz, 1H), 6.97 (d, *J=* 7.2 Hz, 1H), 6.91 (t, *J=* 7.2 Hz, 1H), 6.65 (s, 1H), 5.79 (s, 1H), 4.69 (d, *J* = 12.8 Hz, 1H), 4.42 (d, *J* = 9.6 Hz, 1H), 3.93 (d, *J* = 14.8 Hz, 1H), 3.70-3.66 (m, 1H), 3.60 - 3.54 (m, 1H), 3.16-3.14 (m, 4H), 3.04 - 2.99 (m, 1H), 2.90 (t, *J=* 5.6 Hz, 2H), 2.79 - 2.61 (m, 4H), 1.91 - 1.88 (m, 5H), 1.60 - 1.49 (m, 1H).

### Example 81

### Preparation of trans-4-(3,4-dihydroisoquinolin-2(1H)-yl)-1-(6-(oxetan-3-ylamino) pyrimidin-4-yl)piperidin-3-ol

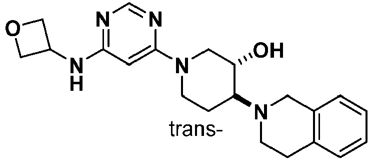

According to the method of Example 59, trans-1-(6-chloropyrimidin-4-yl)-4-(3,4-dihydroisoquinolin-2(1*H*)-yl)piperidin-3-ol was used as a raw material to undergo a substitution reaction with 3-oxetanamine to obtain the target compound trans-4-(3,4-dihydroisoquinolin-2(1*H*)-yl)-1-(6-(oxetan-3-ylamino)pyrimidin-4-yl)piperidin-3 -ol, yield: 11.2%. LCMS (ESI) [M+H]⁺ =382.40; ¹H NMR (400 MHz, Chloroform-*d*) *δ* 8.17 (s, 1H), 7.19 - 7.09 (m, 3H), 7.06 - 6.99 (m, 1H), 5.42 (s, 1H), 5.21 (d, *J* = 6.9 Hz, 1H), 5.06 - 4.95 (m, 2H), 4.91 - 4.89 (m, 1H), 4.72 (d, *J* = 13.5 Hz, 1H), 4.57 - 4.54 (m, 2H), 4.48 - 4.41 (m, 1H), 3.95 (d, *J=* 14.5 Hz, 1H), 3.84 - 3.66 (m, 2H), 3.60 - 3.59 (m, 1H), 3.04 - 3.01 (m, 1H), 2.93 - 2.90 (m, 2H), 2.84 - 2.63 (m, 4H), 1.96 - 1.87 (m, 1H), 1.57 - 1.53 (m, 1H).

### Example 82

### Preparation of trans-1-(6-(cyclohexylamino)pyrimidin-4-yl)-4-(3,4-dihydroisoquinolin-2(1H)-yl)piperidin-3-ol:

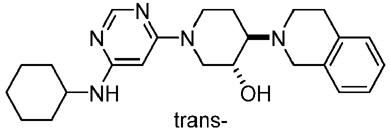

According to the method of Example 59, trans-1-(6-chloropyrimidin-4-yl)-4-(3,4-dihydroisoquinolin-2(1*H*)-yl)piperidin-3-ol was used as a raw material to undergo a substitution reaction with cyclohexylamine to obtain the target compound trans-1-(6-(cyclohexylamino)pyrimidin-4-yl)-4-(3,4-dihydroisoquinolin-2(1H)-yl)piperidin-3 -ol, yield: 23.7%. LCMS (ESI) [M+H]⁺ = 408.37; ¹H NMR (400 MHz, CDCl₃) *δ* 8.13 (s, 1H), 7.18 - 7.09 (m, 3H), 7.05 - 7.00 (m, 1H), 5.47 (s, 1H), 4.88 (bs, 1H), 4.76 (d, *J* = 13.5 Hz, 1H), 4.49 - 4.40 (m, 1H), 3.95 (d, *J* = 14.5 Hz, 1H), 3.77 - 3.66 (m, 2H), 3.68 - 3.59 (m, 1H), 3.44 (bs, 1H), 3.04 - 3.01 (m, 1H), 2.91 (t, *J* = 5.8 Hz, 2H), 2.81 - 2.71 (m, 2H), 2.71 - 2.63 (m, 2H), 2.04 - 1.96 (m, 2H), 1.92 - 1.89 (m, 1H), 1.78 - 1.74 (m, 3H), 1.59 - 1.57 (m, 1H), 1.47 - 1.34 (m, 2H), 1.30 - 1.18 (m, 3H).

### Example 83

### Preparation of trans-1-(6-(benzo[d][1,3]dioxazol-4-ylamino)pyrimidin-4-yl)-4-(3,4-dihydroisoquinolin-2(1H)-yl)piperidin-3-ol

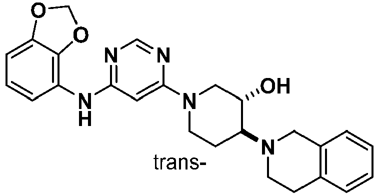

According to the method of Example 59, trans-1-(6-chloropyrimidin-4-yl)-4-(3,4-dihydroisoquinolin-2(1*H*)-yl)piperidin-3-ol was used as a raw material to undergo a substitution reaction with benzo[*d*][1,3]dioxazol-4-ylamine to obtain the target compound trans-1-(6-(benzo[*d*][1,3]dioxazol-4-ylamino)pyrimidin-4-yl)-4-(3,4-dihydroisoquinolin-2(1*H* )-yl)piperidin-3-ol, yield: 28.2%. LCMS (ESI): [M+H]⁺= 446.5; ¹H NMR (400 MHz, DMSO-*d*₆) *δ* 8.69 (s, 1H), 8.12 (s, 1H), 7.18 (d, *J* = 8.2 Hz, 1H), 7.11-7.00 (m, 4H), 6.80 (t, *J* = 8.0 Hz, 1H), 6.68 (d, *J* = 7.6 Hz, 1H), 6.02 (s, 2H), 5.94 (s, 1H), 4.69 (d, *J* = 2.5 Hz, 1H), 4.30 (d, *J* = 9.0 Hz, 1H), 4.21 (d, *J* = 11.4 Hz, 1H), 3.80 (q, *J* = 15.0 Hz, 2H), 3.5 8-3.5 4 (m, 1H), 2.92-2.73 (m, 5H), 2.69-2.60 (m, 2H), 1.79 (dd, *J* = 10.2 Hz, 1H), 1.49-1.40 (m, 1H).

### Example 84

### Preparation of trans-4-(3,4-dihydroisoquinolin-2(1H)-yl)-1-(6-((2-(trifluoromethoxy)-phenyl)amino)pyrimidin-4-yl)piperidin-3-ol:

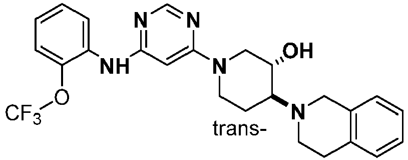

According to the method of Example 59, trans-1-(6-chloropyrimidin-4-yl)-4-(3,4-dihydroisoquinolin-2(1*H*)-yl)piperidin-3-ol was used as a raw material to undergo a substitution reaction with 2-(trifluoromethoxy)aniline to obtain the target compound trans-4-(3,4-dihydroisoquinolin-2(1*H*)-yl)-1-(6-((2-(trifluoromethoxy)phenyl)amino)pyrimidi n-4-yl)piperidin-3-ol, yield: 31.3%. LCMS (ESI) [M+H]⁺ =486.5; ¹H NMR (400 MHz, Chloroform-*d*) *δ* 8.32 (d, *J* = 1.0 Hz, 1H), 7.92 (dd, *J* = 8.5, 1.6 Hz, 1H), 7.35 - 7.28 (m, 2H), 7.18 - 7.07 (m, 4H), 7.06 - 6.99 (m, 1H), 6.59 (s, 1H), 5.97 (d, *J* = 1.0 Hz, 1H), 4.75 (d, *J* = 13.5 Hz, 1H), 4.46 (d, *J* = 12.5 Hz, 1H), 3.95 (d, *J* = 14.5 Hz, 1H), 3.72-3.68 (m, 2H), 3.60 (td, *J* = 10.0, 4.9 Hz, 1H), 3.03 (dt, *J* = 11.0, 5.3 Hz, 1H), 2.92 (t, *J* = 5.8 Hz, 2H), 2.86 - 2.75 (m, 2H), 2.73-2.65 (m, 2H), 1.94-1.90 (m, 1H), 1.65 - 1.52 (m, 1H).

### Example 85

### Preparation of trans-4-(3,4-dihydroisoquinolin-2(1H)-yl)-1-(6-((3-methoxypyridin-4-yl) amino)pyrimidin-4-yl)piperidin-3-ol:

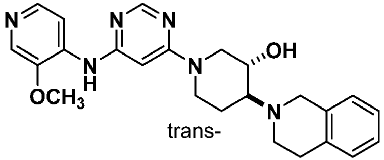

According to the method of Example 59, trans-1-(6-chloropyrimidin-4-yl)-4-(3,4-dihydroisoquinolin-2(1*H*)-yl)piperidin-3-ol was used as a raw material to undergo a substitution reaction with 4-amino-3-methoxypyridine to obtain the target compound trans-4-(3,4-dihydroisoquinolin-2(*1H*)-yl)-1-(6-((3-methoxypyridin-4-yl)amino)pyrimidin-4-yl)piperidin-3-ol, yield: 39.3%. LCMS(ESI): [M+H]⁺= 433.4; ¹H NMR (400 MHz, DMSO-*d*₆) *δ* 8.54 (s, 1H), 8.43 (d, *J* = 5.4 Hz, 1H), 8.29 (s, 1H), 8.21 (s, 1H), 8.05 (d, *J* = 5.4 Hz, 1H), 7.13-6.99 (m, 4H), 6.62 (s, 1H), 4.71 (d, *J* = 4.1 Hz, 1H), 4.37 (d, *J* = 10.4 Hz, 1H), 4.25 (d, *J* = 14.0 Hz, 1H), 3.95 (s, 3H), 3.80 (q, *J* = 15.0 Hz, 2H), 3.57 (dt, *J* = 13.7, 4.5 Hz, 1H), 2.96-2.84 (m, 2H), 2.83-2.63 (m, 5H), 1.82 (d, *J=* 10.0 Hz, 1H), 1.48 (dt, *J=* 12.1, 8.4 Hz, 1H).

### Example 86

### Preparation of trans-4-(3,4-dihydroisoquinolin-2(1H)-yl)-1-(6-((6-(pyrrolidin-1-yl) pyridin-2-yl)amino)pyrimidin-4-yl)piperidin-3-ol:

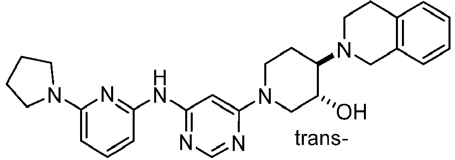

### Step I: preparation of 6-(pyrrolidin-1-yl)pyridinyl-2-amine:

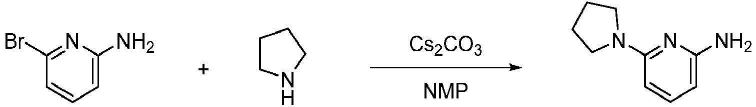

2-amino-6-bromopyridine (200 mg, 1.16 mmol, 1.0 eq), tetrahydropyrrole (164 mg, 2.31 mmol, 2.0 eq) and Cs₂CO₃ (cesium carbonate) (565 mg, 1.73 mmol, 1.5 eq) were dissolved in NMP (N-methylpyrrolidone) (8 mL), and allowed to react at 200°C under a microwave condition for 0.5 h. The reaction mixture was poured into 20 mL water, extracted with ethyl acetate, and dried over sodium sulfate. The organic phase was concentrated, and the crude product was seperated and purified by flash chromatography (silica gel, dichloromethane) to obtain the target compound 6-(pyrrolidin-1-yl)pyridin-2-amine (406 mg, crude product) as a brown liquid. LCMS (ESI) [M+H]⁺ = 164.10.

### Step II: preparation of trans-4-(3,4-dihydroisoquinolin-2(1H)-yl)-1-(6-((6-(pyrrolidin-1-yl) pyridin-2-yl)amino)pyrimidin-4-yl)piperidin-3-ol:

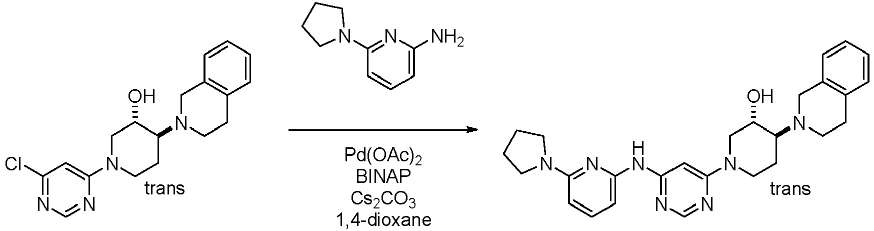

Trans-1-(6-chloropyrimidin-4-yl)-4-(3,4-dihydroisoquinolin-2(1H)-yl)piperidin-3-ol (50 mg, 0.14 mmol, 1.0 eq), 6-(pyrrolidin-1-yl)pyridin-2-amine (71 mg, 0.43 mmol, 3.0 eq), Pd(OAc)₂ (palladium acetate) (7 mg, 0.03 mmol, 0.2 eq), BINAP (1,1'-binaphthyl-2,2'-bis(diphenylphosphine)) (36 mg, 0.06 mmol, 0.4 eq) and Cs₂CO₃ (cesium carbonate) (94 mg, 0.29 mmol, 2.0 eq) were dissolved in 1,4-dioxane (1 mL), and allowed to react at 110°C under nitrogen protection for 17 h. The reaction mixture was filtrated, and the filtrate was extracted with ethyl acetate. The organic phase was concentrated, and the crude product was seperated and purified by preparative HPLC (C18, 10 mmol/L aqueous NH₄HCO₃, acetonitrile) to obtain the target compound trans-4-(3,4-dihydroisoquinolin-2(1*H*)-yl)-1-(6-((6-(pyrrolidin-1-yl)pyridin-2-yl)amino)pyrimidin-4-yl)piperidin-3-ol (12.4 mg, 18.1%). LCMS (ESI) [M+H]⁺ = 472.48; ¹H NMR (400 MHz, DMSO-*d*₆) δ 9.32 (s, 1H), 8.16 (s, 1H), 7.78 (s, 1H), 7.34 (t, *J* = 8.0 Hz, 1H), 7.10 - 7.05 (m, 3H), 7.05 - 7.01 (m, 1H), 6.38 (d, *J* = 7.8 Hz, 1H), 5.92 (d, *J* = 8.0 Hz, 1H), 4.66-4.33 (m, 2H), 3.80 (q, *J* = 15.0 Hz, 2H), 3.57 (tt, *J* = 9.4, 4.5 Hz, 1H), 3.43 (t, *J* = 6.0 Hz, 4H), 2.94 - 2.85 (m, 2H), 2.82 - 2.64 (m, 5H), 1.98 - 1.90 (m, 4H), 1.79 (d, *J=* 12.8 Hz, 1H), 1.48 (qd, *J=* 12.2, 4.2 Hz, 1H).

### Example 87

### Preparation of trans-1-(6-((2,3-difluorophenyl)amino)pyrimidin-4-yl)-4-(3,4-dihydroisoquinolin-2(1H)-yl)piperidin-3-ol:

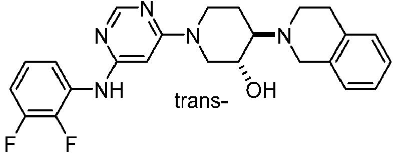

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2,3-difluoroaniline to obtain the target compound trans-1-(6-((2,3-difluorophenyl)amino)pyrimidin-4-yl)-4-(3,4-dihydroisoquinolin-2(1*H*)-yl)pi peridin-3-ol, yield: 21.8%. LCMS (ESI) [M+H]⁺ = 438.4; ¹H NMR (400 MHz, CDCl₃) δ 8.32 (s, 1H), 7.62 (ddt, *J* = 8.4, 6.8, 1.7 Hz, 1H), 7.17 - 7.00 (m, 5H), 6.91-6.89 (m, 1H), 6.69 (s, 1H), 5.94 (s, 1H), 4.73 (d, *J* = 13.5 Hz, 1H), 4.51 - 4.40 (m, 1H), 3.96 (d, *J* = 14.6 Hz, 1H), 3.81 - 3.69 (m, 2H), 3.60 (td, *J=* 10.1, 4.9 Hz, 1H), 3.03 (dt, *J=* 11.0, 5.4 Hz, 1H), 2.92 (t, *J* = 5.8 Hz, 2H), 2.85 - 2.67 (m, 4H), 1.94-1.91 (m, 1H), 1.56 (qd, *J* = 12.5, 4.4 Hz, 1H).

### Example 88

### Preparation of trans-1-(6-((3-fluoro-2-methoxyphenyl)amino)pyrimidin-4-yl)-4-(3,4-dihydroisoquinolin-2(1H)-yl)piperidin-3-ol:

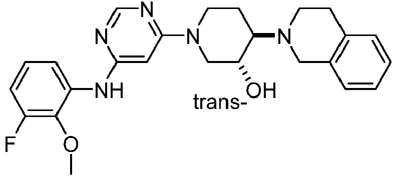

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 3-fluoro-2-methoxyaniline to obtain the target compound trans-1-(6-((3-fluoro-2-methoxyphenyl)amino)pyrimidin-4-yl)-4-(3,4-dihydroisoquinolin-2(1*H*)-yl)piperidin-3-ol, yield: 57.5%. LCMS (ESI) [M+H]⁺ = 450.5; ¹H NMR (400 MHz, DMSO-*d*₆) *δ* 8.43 (s, 1H), 8.20 (s, 1H), 8.05 (d, *J=* 8.4 Hz, 1H), 7.05-7.08 (m, 3H), 7.0-7.05 (m, 2H), 6.90 - 6.83 (m, 1H), 6.38 (s, 1H), 4.69 (d, *J=* 4.0 Hz, 1H), 4.36 - 4.24 (m, 2H), 3.84 - 3.82 (m, 4H), 3.78 - 3.75 (m, 1H), 3.61 - 3.54 (m, 1H), 2.93 - 2.85 (m, 2H), 2.82 - 2.79 (m, 3H), 2.70 - 2.65 (m, 2H), 1.82 (*d, J* = 10.3 Hz, 1H), 1.52 - 1.41 (m, 1H).

### Example 89

### Preparation of trans-1-(6-((2,3-dichlorophenyl))amino)pyrimidin-4-yl)-4-(3,4-dihydroisoquinolin-2(1H)-yl)piperidin-3-ol:

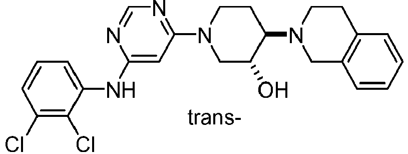

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2,3-dichloroaniline to obtain the target compound trans-1-(6-((2,3-dichlorophenyl))amino)pyrimidin-4-yl)-4-(3,4-dihydroisoquinolin-2(1*H*)-yl) piperidin-3-ol, yield: 44.2%. LCMS (ESI) [M+H]⁺ = 470.4; ¹H NMR (400 MHz, DMSO-*d*₆) *δ* 8.61 (s, 1H), 8.16 (s, 1H), 7.94 (dd, *J=* 6.6, 3.1 Hz, 1H), 7.38 - 7.26 (m, 2H), 7.05 (dd; , *J* = 18.3, 3.8 Hz, 3H), 7.03 (d, *J=* 3.7 Hz, 1H), 6.26 (s, 1H), 4.71 (d, *J=* 4.0 Hz, 1H), 4.35-4.22 (m, 2H), 3.81 (q, *J* = 15.1 Hz, 2H), 3.65 - 3.53 (m, 1H), 3.09 - 2.61 (m, 5H), 2.68 (dd, *J* = 13.5, 8.4 Hz, 2H), 1.82 (d, *J=* 13.7 Hz, 1H), 1.47 (d, *J* = 8.2 Hz, 1H).

### Example 90

### Preparation of trans-1-(6-((2-ethoxyphenyl)amino)pyrimidin-4-yl)-4-(3,4-dihydroisoquinolin-2(1H)-yl)piperidin-3-ol:

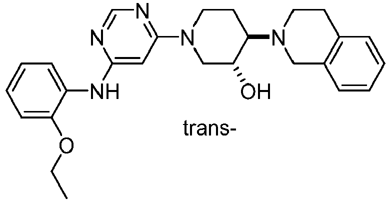

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2-ethoxyaniline to obtain the target compound trans-1-(6-((2-ethoxyphenyl)amino)pyrimidin-4-yl)-4-(3,4-dihydroisoquinolin-2(1*H*)-yl)-piperidin-3-ol, yield: 55.6%. LCMS (ESI) [M+H]⁺ =446.1; ¹H NMR (400 MHz, DMSO-*d*₆) δ 8.15 (s, 1H), 8.04 - 7.92 (m, 2H), 7.13 - 7.05 (m, 3H), 7.04 - 6.99 (m, 2H), 6.98 (ddd, *J =* 9.6, 7.2, 1.6 Hz, 1H), 6.89 (td, *J* = 7.7, 1.6 Hz, 1H), 6.19 (s, 1H), 4.68 (d, *J* = 4.0 Hz, 1H), 4.29 (dd, *J* = 42.2, 10.9 Hz, 2H), 4.09 (q, *J* = 7.0 Hz, 2H), 3.80 (q, *J* = 15.1 Hz, 2H), 3.57 (tt, *J* = 9.3, 4.5 Hz, 1H), 2.90-2.86 (m, 1H), 2.84 - 2.75 (m, 4H), 2.64 (dd, *J=* 14.6, 8.1 Hz, 2H), 1.80 (d, *J=* 9.9 Hz, 1H), 1.46 (td, *J=* 12.1, 8.3 Hz, 1H), 1.36 (t, *J=* 7.0 Hz, 3H).

### Example 91

### Preparation of trans-1-(6-((2-(2,2,2-trifluoroethoxy)phenyl)amino)pyrimidin-4-yl)-4-(3,4-dihydroisoquinolin-2(1H)-yl)piperidin-3-ol:

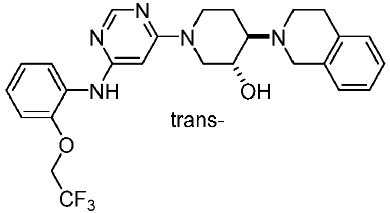

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 2-(2,2,2-trifluoroethoxy)aniline to obtain the target compound trans-1-(6-((2,2,2-trifluoroethoxy)amino)pyrimidin-4-yl)-4-(3,4-dihydroisoquinolin-2(1*H*)-yl) piperidin-3-ol, yield: 48.2%. LCMS (ESI) [M+H]⁺ =500.4; ¹H NMR (400 MHz, DMSO-*d*₆) *δ* 8.11 (d, *J* = 8.9 Hz, 2H), 7.83 (dd, *J* = 7.6, 1.9 Hz, 1H), 7.17 (dd, *J* = 7.8, 1.3 Hz, 1H), 7.10 - 6.99 (m, 6H), 6.07 (s, 1H), 4.77 (q, *J* = 8.9 Hz, 2H), 4.68 (d, *J* = 4.0 Hz, 1H), 4.28 (dd, *J* = 41.2, 11.4 Hz, 2H), 3.80 (q, *J=* 15.0 Hz, 2H), 3.55 (dt, *J=* 14.1, 4.7 Hz, 1H), 2.90-2.84 (m, 1H), 2.82-2.75 (m, 4H), 2.68 - 2.60 (m, 2H), 1.79 (d, *J=* 9.9 Hz, 1H), 1.45 (dt, *J=* 11.7, 8.2 Hz, 1H).

### Example 92

### Preparation of trans-1-(6-((5-fluoro-2-methoxyphenyl)amino)pyrimidin-4-yl)-4-(3,4-dihydroisoquinolin-2(1H)-yl)piperidin-3-ol:

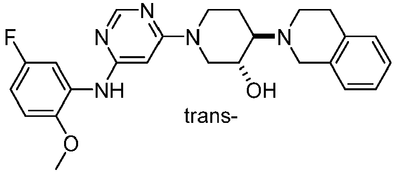

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 5-fluoro-2-methoxyaniline to obtain the target compound trans-4-(3,4-dihydroisoquinolin-2(1*H*)-yl)-1-(6-((5-fluoro-2-methoxyphenyl)amino)pyrimidin -4-yl)piperidin-3-ol, yield: 29.5%. LCMS (ESI) [M+H]⁺ = 450.4; ¹H NMR (400 MHz, CDCl₃) *δ* 8.35 (s, 1H), 7.96 (dd, *J=* 10.7, 3.1 Hz, 1H), 7.19 - 7.09 (m, 3H), 7.05 - 6.98 (m, 2H), 6.80 (dd, *J* = 8.9, 5.0 Hz, 1H), 6.66 (td, *J* = 8.4, 3.0 Hz, 1H), 5.97 (s, 1H), 4.72 (d, *J* = 13.5 Hz, 1H), 4.51 (d, *J=* 11.4 Hz, 1H), 3.95 (d, *J* = 14.6 Hz, 1H), 3.88 (s, 3H), 3.79 - 3.67 (m, 2H), 3.61 (td, *J =* 10.0, 4.8 Hz, 1H), 3.03 (dt, *J* = 11.0, 5.4 Hz, 1H), 2.91 (t, *J* = 5.8 Hz, 2H), 2.87 - 2.64 (m, 4H), 1.92 (dd, *J* = 12.9, 3.2 Hz, 1H), 1.56 (qd, *J* = 12.6, 4.4 Hz, 1H).

### Example 93

### Preparation of trans-4-(3,4-dihydroisoquinolin-2(1H)-yl)-1-(6-((4-fluoro-2-methoxyphenyl)amino)pyrimidin-4-yl)piperidin-3-ol:

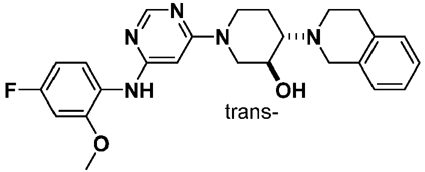

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 4-fluoro-2-methoxyaniline to obtain the target compound trans-4-(3,4-dihydroisoquinolin-2(1H)-yl)-1-(6-((4-fluoro-2-methoxyphenyl)amino)pyrimidin -4-yl)piperidin-3-ol, yield: 39.1%. LCMS (ESI) [M+H]⁺ =450.37; ¹H NMR (400 MHz, CDCl₃) *δ* 8.28 (s, 1H), 7.70 (dd, J = 8.6, 6.1 Hz, 1H), 7.21 - 7.06 (m, 3H), 7.06 - 6.94 (m, 1H), 6.76 - 6.60 (m, 3H), 5.86 (s, 1H), 4.72 (d, *J=* 13.4 Hz, 1H), 4.43 (d, *J=* 10.0 Hz, 1H), 3.94 (d, *J* = 14.5 Hz, 1H), 3.86 (s, 3H), 3.69 (d, *J* = 14.6 Hz, 2H), 3.58 (td, *J* = 10.0, 4.9 Hz, 1H), 3.02 (dt, *J* = 10.9, 5.3 Hz, 1H), 2.91 (t, *J* = 5.6 Hz, 2H), 2.82 - 2.63 (m, 4H), 1.90 (dd, *J* = 12.8, 3.3 Hz, 1H), 1.54 (qd, *J* = 12.4, 4.3 Hz, 1H).

### Example 94

### Preparation of trans-1-(6-((2,3-dihydrobenzo[b][1,4]dioxin-5-yl)amino)pyrimidin-4-yl)-4-(3,4-dihydroisoquinolin-2(1H)-yl)piperidin-3-ol:

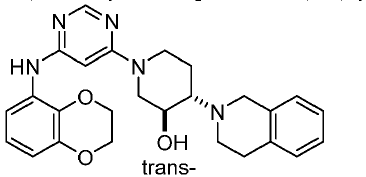

According to the method of Example 59, trans-1-(6-chloropyrimidin-4-yl)-4-(3,4-dihydroisoquinolin-2(1*H*)-yl)piperidin-3-ol was used as a raw material to undergo a substitution reaction with 5-amino-1,4-benzodioxane to obtain the target compound trans-1-(6-((2,3-dihydrobenzo[b][1,4]dioxin-5-yl)amino)pyrimidin-4-yl)-4-(3,4-dihydro-isoquinolin-2(1*H*)-yl)piperidin-3-ol, yield: 6.5%. LCMS (ESI) [M+H]⁺ = 460.4; ¹H NMR (400 MHz, DMSO-*d*₆) *δ* 8.15 (d, *J =* 2.8 Hz, 2H), 7.50 (d, *J =* 7.6 Hz, 1H), 7.08-7.03 (m, 4H), 6.76 (t, *J* = 8.0 Hz, 1H), 6.56 (d, *J* = 7.6 Hz, 1H), 6.16 (s, 1H), 4.69 (d, *J* = 3.6 Hz,1H), 4.30-4.20 (m, 6H), 3.86-3.75 (m, 2H), 3.59-3.54 (m, 1H), 2.90-2.78 (m, 5H), 2.65 (t, *J=* 10.0 Hz, 2H), 1.79 (d, *J* = 10.4 Hz, 1H), 1.47-1.44 (m, 1H).

### Example 95

### Preparation of trans-4-(3,4-dihydroisoquinolin-2(1H)-yl)-1-(6-((4-(pyrrolidin-1-yl) phenyl)amino)pyrimidin-4-yl)piperidin-3-ol:

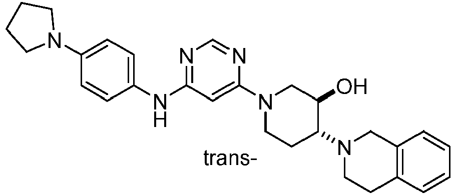

According to the method of Example 33, trans-1-(6-chloropyrimidin-4-yl)-4-(3,4-dihydroisoquinolin-2(1*H*)-yl)piperidin-3-ol was used as a raw material to undergo a substitution reaction with 4-(pyrrolidin-1-yl)aniline to obtain the target compound trans-4-(3,4-dihydroisoquinolin-2(1*H*)-yl)-1-(6-((4-(pyrrolidin-1-yl)phenyl)amino)pyrimidin-4-yl)piperidin-3-ol, yield: 14.7%. LCMS (ESI) [M+H]⁺ =471.6; ¹H NMR (400 MHz, CDCl₃) *δ* 8.18 (s, 1H), 7.33 - 7.26 (m, 3H), 7.21 (d, *J* = 6.8 Hz, 1H), 7.12 (d, *J* = 8.8 Hz, 2H), 6.67 (d, *J* = 8.8 Hz, 2H), 5.87 (s, 1H), 4.76 - 4.42 (m, 4H), 4.02 - 3.96 (m, 1H), 3.76 - 3.61 (m, 3H), 3.33 (s, 3H), 3.24 - 3.10 (m, 4H), 2.98 (td, *J* = 11.4 Hz, 2.0 Hz, 1H), 2.26 - 2.24 (m, 1H), 2.07 - 2.03 (m, 4H), 1.88-1.85 (m, 1H).

### Example 96

### Preparation of trans-(4-(3,4-dihydroisoquinolin-2(1H)-yl)-3-hydroxypiperidin-l-yl)(6-((1-(2-methoxyethyl)piperidin-4-yl)amino)pyrimidin-4-yl)ketone:

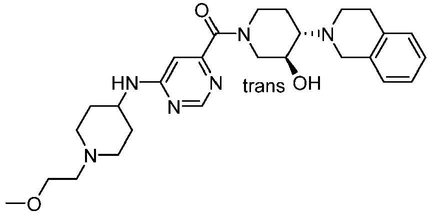

### Step I: preparation of t-butyl (1-(2-methoxyethyl)piperidin-4-yl)carbamate:

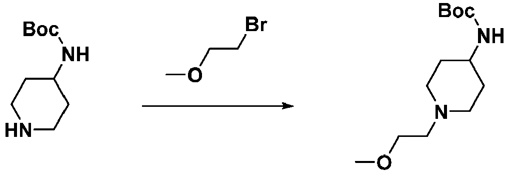

T-butyl piperidin-4-ylcarbamate (500 mg, 2.49 mmol, 1.0 eq) was dissolved in acetonitrile (10 mL) at room temperature (20 °C) under nitrogen protection, then 1-bromo-2-methoxyethane (382 mg, 2.75 mmol, 1.1 eq) and potassium carbonate (1 g, 7.49 mmol, 3.0 eq) were added thereto, and the mixture was heated under stirring at 80°C for 16 h. After the reaction was complete as indicated by LCMS, the reaction mixture was cooled to room temperature, filtrated and concentrated. The crude product was separated by column chromatography (silica gel, gradient elution: from pure DCM to DCM:MeOH=95:5 (vol)) to obtain the title compound t-butyl (1-(2-methoxyethyl)piperidin-4-yl)carbamate (545 mg , yield: 84.5%) as a white solid. LCMS: [M+H]⁺ =1.49.

### Step II: preparation of 1-(2-methoxyethyl)piperidin-4-amine:

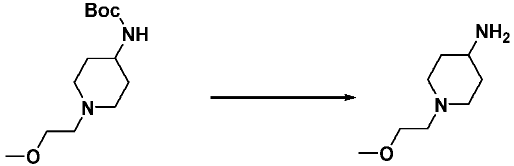

T-butyl (1-(2-methoxyethyl)piperidin-4-yl)carbamate (550 mg, 2.13 mmol, 1.0 eq) was dissolved in a solution of hydrogen chloride in 1,4-dioxane (3 mL, 4M), stirred at room temperature (25 °C) for 1 h, and extracted with dichloromethane (3×20 mL). The organic phase was washed once with water and once with saturated sodium chloride solution, dried over anhydrous sodium sulfate, and concentrated, to obtain a crude product of 1-(2-methoxyethyl)-piperidin-4-amine (520 mg crude product) as a yellow oil. LC-MS(ESI):[M+H]⁺=159.16.

### Step III: preparation of trans-(4-(3,4-dihydroisoquinolin-2(1H)-yl)-3-hydroxypiperidin-1-yl)(6-((1-(2-methoxyethyl)piperidin-4-yl)amino)pyrimidin-4-yl)ketone:

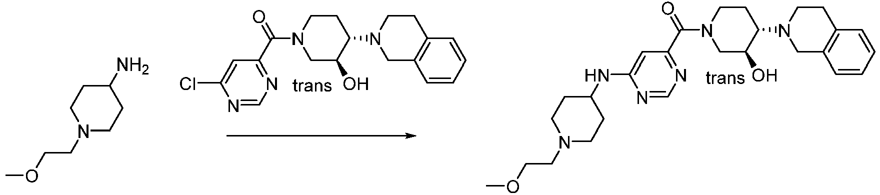

The raw material trans-(6-chloropyrimidin-4-yl)(4-(3,4-dihydroisoquinolin-2(1*H*)-yl)-3-hydroxypiperidin-1-yl)ketone (70 mg, 0.19 mmol, 1.0 eq) was dissolved in 1,4-dioxane (2 mL) at room temperature (20 °C) under nitrogen protection, then 1-(2-methoxyethyl)piperidin-4-amine (89 mg, 0.56 mmol, 3.0 eq), palladium acetate (9 mg, 0.04 mmol, 0.2 eq), BINAP (1,1'-binaphthyl-2,2'-bis(diphenylphosphine)) (47 mg, 0.08 mmol, 0.4 eq), and cesium carbonate (306 mg, 0.94 mmol, 5.0 eq) were added thereto, and the mixture was heated under stirring at 100°C for 16 h. After the reaction was complete as indicated by LCMS, the reaction mixture was cooled to room temperature, diluted with dichloromethane (5 mL), filtrated and concentrated. The crude product was separated and purified by preparative HPLC (C18, 10 mmol/L aqueous NH₄HCO₃, acetonitrile) to obtain the compound trans-(4-(3,4-dihydroisoquinolin-2(1*H*)-yl)-3-hydroxypiperidin-1-yl)(6-((1-(2-methoxyethyl)piperidin-4-yl)amino)pyrimidin-4-yl)ketone (7.2 mg, 7.8%). LCMS: [M+H]⁺ =495.64; ¹H NMR (400 MHz, CDCl₃) *δ* 8.52 (d, *J* = 6.0 Hz, 1H), 7.19 - 7.09 (m, 3H), 7.05 - 6.99 (m, 1H), 6.57 (d, *J* = 16.6 Hz, 1H), 5.03 (d, *J* = 9.3 Hz, 1H), 5.05-5.01 (m, 1H), 4.72 (d, *J=* 14.0 Hz, 1 H), 4.32 - 4.13 (m, 1H), 3.95 (d, *J* = 14.6 Hz, 2H), 3.76 - 3.67 (m, 2H), 3.52 (t, *J* = 5.5 Hz, 2H), 3.36 (s, 3H), 3.12 - 2.81 (m, 7H), 2.73 - 2.64 (m, 2H), 2.61 (t, *J* = 5.4 Hz, 2H), 2.32 - 2.15 (m, 2H), 2.12 - 1.97 (m, 3H), 1.91 - 1.80 (m, 1H), 1.64 - 1.56 (m, 2H).

### Example 97

### Preparation of trans-4-(3,4-dihydroisoquinolin-2(1H)-yl)-3-hydroxypiperidin-1-yl)-(6-(trans-4-(3,4-dihydroisoquinolin-2(1H)-yl)-3-hydroxypiperidin-1-yl)pyrimidin-4-yl) ketone:

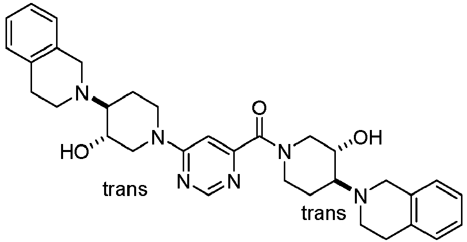

### Step I: preparation of 4-(chlorocarbonyl)-6-chloropyrimidine:

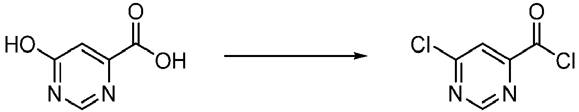

6-hydroxypyrimidine-4-carboxylic acid (100 mg, 0.71 mmol, 1.0 eq) was dissolved in ethyl acetate (3 mL), followed by addition of oxalyl chloride (453 mg, 3.57 mmol, 5.0 eq) and N,N-dimethylformamide (0.001 mL) at 0°C, and the mixture was stirred under nitrogen protection at 80°C for 1 h. The reaction was complete as indicated by TLC, and the mixture was rotary evaporated to dryness to obtain a crude product of 4-(chlorocarbonyl)-6-chloropyrimidine as a black solid.

### Step II: preparation of trans-4-(3,4-dihydroisoquinolin-2(1H)-yl)-3-hydroxypiperidin-1-yl)-(6-(trans-4-(3,4-dihydroisoquinolin-2(1H)-yl)-3-hydroxypiperidin-1-yl)pyrimidin-4-yl)ketone

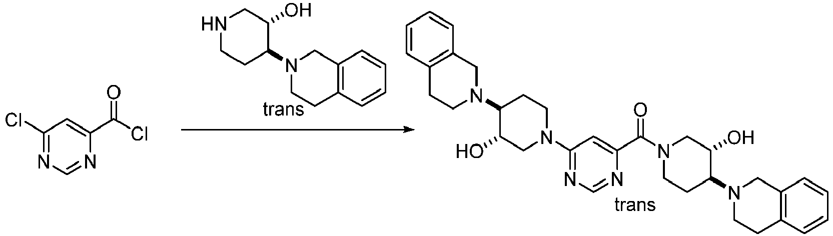

Trans-4-(3,4-dihydroisoquinolin-2(1*H*)-yl)piperidin-3-ol (265 mg, 1.14 mmol, 1.6 eq) and 4-(chlorocarbonyl)-6-chloropyrimidine (126 mg, 0.71 mmol, 1.0 eq) were dissolved in dichloromethane (3 mL) at 0°C, followed by addition of triethylamine (504 mg, 4.98 mmol, 7.0 eq), and then the mixture was stirred at 20°C for 2 h. The reaction mixture was suction filtrated, and the filtrate was concentrated. The crude product was separated and purified by preparative HPLC (C18, 10 mmol/L aqueous NH₄HCO₃, acetonitrile) to obtain trans-4-(3,4-dihydroisoquinolin-2(1*H*)-yl)-3-hydroxypiperidin-1-yl)(6-(trans-4-(3,4-dihydrois oquinolin-2(1*H*)-yl)-3-hydroxypiperidin-1-yl)pyrimidin-4-yl)ketone (26.4 mg, yield: 6.5%). LCMS (ESI) [M+H]⁺ = 569.7; ¹H NMR (400 MHz, CDCl₃) *δ* 8.60 - 8.57 (m, 1H), 7.19 - 7.10 (m, 6H), 7.07 - 7.01 (m, 2H), 6.89 - 6.81 (m, 1H), 5.13 - 4.53 (m, 3H), 4.27 - 3.92 (m, 4H), 3.84 - 3.55 (m, 5H), 3.21 - 2.59 (m, 15H), 2.09 - 1.94 (m, 2H), 1.87 (d, *J* = 12.8 Hz, 1H).

### Example 98

### Preparation of 1-cyclopropyl-2-(2-((6-(trans-4-(3,4-dihydroisoquinolin-2(1H)-yl)-3-hydroxypiperidine-1-carbonyl)pyrimidin-4-yl)amino)-7-azaspiro[3.5]nonan-7-yl)ethane-1,2-dione:

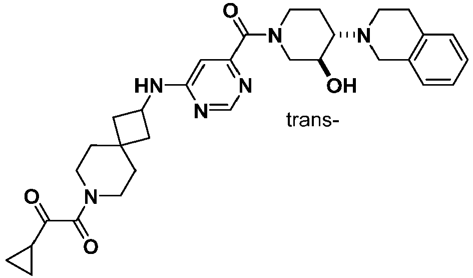

### Step I: preparation of t-butyl (7-(2-cycloproyl-2-oxoacetyl)-7-azaspiro[3.5]nonan-2-yl) carbamate:

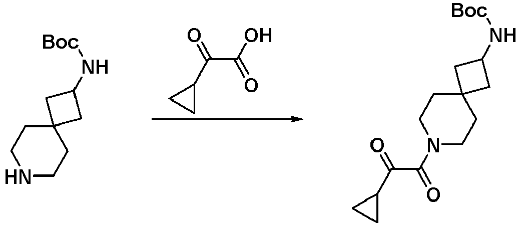

T-butyl 7-azaspiro[3.5]nonane-2-ylcarbamate (220 mg, 0.94 mmol, 1.0 eq), 2-cycloproyl-2-oxoacetic acid (107 mg, 0.94 mmol, 1.0 eq) and 1-propylphosphoric anhydride (1.2 g, 1.88 mmol, 2.0 eq) were dissolved in N,N-dimethylformamide (5 mL) at room temperature (20°C) under nitrogen protection, followed by addition of triethylamine (475 mg, 4.70 mmol, 5.0 eq), and heated to 25°C and stirred for 2 h. After the reaction was complete as indicated by LCMS, the reaction mixture was cooled to room temperature (20-25°C), filtrated, and concentrated. The crude product was separated by column chromatography (silica gel, gradient elution: from pure DCM to DCM:MeOH=20:1) to obtain the title compound t-butyl (7-(2-cycloproyl-2-oxoacetyl)-7-azaspiro[3.5]nonan-2-yl) carbamate (300 mg, yield: 94.8%) as a brown oil. LCMS: [M+H]⁺ =326.20.

### Step II preparation of 1-(2-amino-7-azaspiro[3.5]nonan-7-yl)-2-cyclopropylethane-1,2-dione:

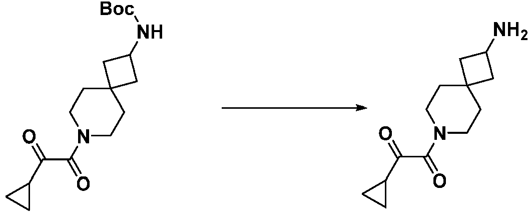

T-butyl (7-(2-cycloproyl-2-oxoacetyl)-7-azaspiro[3.5]nonan-2-yl)-carbamate (300 mg, 0.89 mmol, 1.0 eq) was dissolved in a solution of hydrogen chloride in 1,4-dioxane (4 mL, 4 M), stirred at room temperature (25°C) for 1 h. and extracted with dichloromethane (3×20 mL). The organic phase was washed once with water and once with saturated sodium chloride solution, dried over anhydrous sodium sulfate, and concentrated to obtain a crude product of 1-(2-amino-7-azaspiro[3.5]nonan-7-yl)-2-cycloproylethane-1,2-dione (300 mg, crude product) as a yellow oil. LCSM(ESI):[M+H]⁺=237.23.

### Step III preparation of 1-cycloproyl-2-(2-((6-(trans-4-(3,4-dihydroisoquinolin-2(1H)-yl)-3-hydroxypiperidine-1-carbonyl)pyrimidin-4-yl)amino)-7-azaspiro[3.5]nonan-7-yl)ethane-1,2-dione:

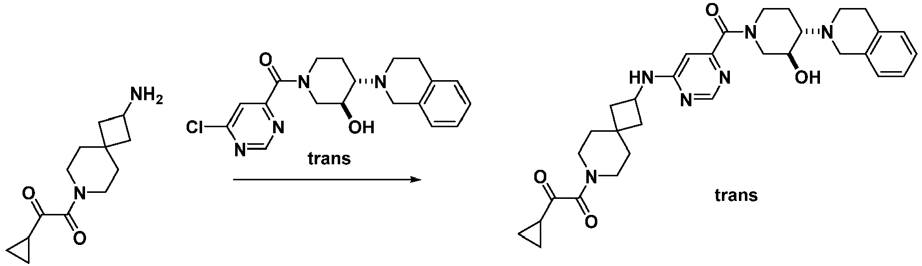

The raw material trans-(6-chloropyrimidin-4-yl)-4-(3,4-dihydroisoquinolin-2(1*H*)-yl)-3-hydroxypiperidin-1-yl)ketone (100 mg, 0.27 mmol, 1.0 eq) was dissolved in 1,4-dioxane (2 mL) at room temperature (20°C) under nitrogen protection, followed by addition of 1-(2-amino-7-azaspiro[3.5]nonan-7-yl)-2-cyclopropylethane-1,2-dione (190 mg, 0.81 mmol, 3.0 eq), palladium acetate (12 mg, 0.05 mmol, 0.2 eq), BINAP (1,1'-binaphthyl-2,2'-bis(diphenylphosphine)) (67 mg, 0.11 mmol, 0.4 eq), and cesium carbonate (437 mg, 1.34 mmol, 5.0 eq), and the mixture was heated at 100°C and stirred for 16 h. After the reaction was complete as indicated by LCMS, the reaction mixture was cooled to room temperature, diluted with dichloromethane (5 mL), filtrated and concentrated. The crude product was separated and purified by preparative HPLC (C18, 10 mmol/L aqueous NH₄HCO₃, acetonitrile) to obtain the title compound 1-cyclopropyl-2-(2-((6-(trans-4-(3,4-dihydroisoquinolin-2(1*H*)-yl)-3-hydroxypiperidine-1-carbonyl)pyrimidin-4-yl) amino)-7-azaspiro[3.5]nonan-7-yl)ethane-1,2-dione (2.7 mg , yield: 1.8%). LCMS: [M+H]⁺ =573.67; ¹H NMR (400 MHz, CDCl₃) δ 8.55 - 8.53 (m, 1H), 7.19 - 7.09 (m, 3H), 7.03 (d, *J* = 5.5 Hz, 1H), 6.55 - 6.51 (m, 1H), 5.34 (s, 1H), 5.09 - 4.65 (m, 1H), 4.45 - 4.11 (m, 2H), 4.03 - 3.81 (m, 2H), 3.78 - 3.45 (m, 5H), 3.40 - 3.35 (m, 1H), 3.33 - 3.26 (m, 1H), 3.07 - 3.01 (m, 1H), 3.01 - 2.78 (m, 4H), 2.74 - 2.62 (m, 2H), 2.54 - 2.43 (m, 2H), 2.36 - 2.25 (m, 1H), 2.04 - 1.94 (m, 1H), 1.89 - 1.79 (m, 1H), 1.81 - 1.71 (m, 5H), 1.30 - 1.21 (m, 2H), 1.17 - 1.09 (m, 2H).

### Example 99

### Preparation of trans-1-cyclopropyl-2-(4-((6-(4-(3,4-dihydroisoquinolin-2(1H)-yl)-3-hydroxypiperidine-1-carbonyl)pyrimidin-4-yl)amino)piperidin-1-yl)ethane-1,2-dione:

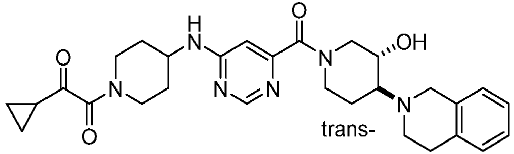

### Step I: preparation of t-butyl (1-(2-cyclopropyl-2-oxoacetyl)piperidin-4-yl)carbamate:

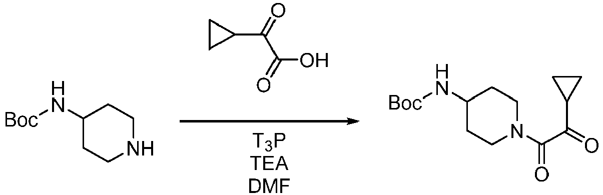

4-Boc-aminopiperidine (500 mg, 2.50 mmol, 1.0 eq), 2-cyclopropyl-2-oxoacetic acid (313 mg, 2.75 mmol, 1.1 eq), T₃P (1-propylphosphoric anhydride) (50% in EA, 3.18 g, 4.99 mmol, 2.0 eq) and TEA (triethylamine) (1.26 g, 12.48 mmol, 5.0 eq) were dissolved in DMF (*N*,*N*-dimethylformamide) (12 mL), and allowed to react at 20°C for 1 h. The reaction mixture was extracted with ethyl acetate, and the organic phase was concentrated. The crude product was separated and purified by flash chromatography (silica gel, DCM: MeOH = 100: 1) to obtain the title compound (703 mg, 95%) as an orange solid. LCMS (ESI) [M+H-56]⁺ = 241.12.

### Step II: preparation of 1-(4-aminopiperidin-1-yl)-2-cyclopropylethane-1,2-dione:

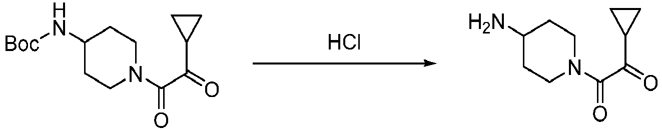

(1-(2-cyclopropyl-2-oxoacetyl)piperidin-4-yl)carbamate (250 mg, 0.84 mmol, 1.0 eq) was dissolved in a solution of hydrogen chloride in 1,4-dioxane (3 mL, 4 M) and allowed to react at 20°C for 1 h. The reaction mixture was concentrated to obtain a crude product of the target compound (168 mg) as a yellow solid. LCMS (ESI) [M+H]⁺ = 197.13;

### Step III: preparation of trans-1-cyclopropyl-2-(4-((6-(4-(3,4-dihydroisoquinolin-2(1H)-yl)-3-hydroxypiperidine-1-car bonyl)pyrimidin-4-yl)amino)piperidin-1 -yl)ethane-1,2-dione:

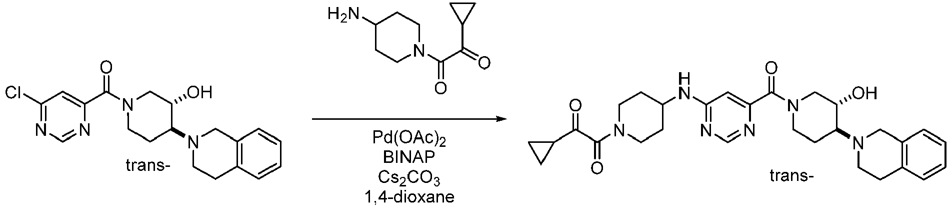

Trans-(6-chloropyrimidin-4-yl)(4-(3,4-dihydroisoquinolin-2(1*H*)-yl)-5-hydroxypiperidin-1-yl )ketone (80 mg, 0.22 mmol, 1.0 eq), 1-(4-aminopiperidin-1-yl)-2-cyclopropylethane-1,2-dione (84 mg, 0.43 mmol, 2.0 eq), Pd(OAc)₂ (palladium acetate) (10 mg, 0.04 mmol, 0.2 eq), BINAP (1,1'- binaphthyl-2,2'-bis(diphenylphosphine)) (53 mg, 0.09 mmol, 0.4 eq) and Cs₂CO₃ (cesium carbonate) (350 mg, 1.07 mmol, 5.0 eq) were dissolved in 1,4-dioxane (2 mL), and allowed to react under nitrogen protection at 110°C for 17 h. The reaction mixture was filtrated, and the filtrate was concentrated. The crude product was separated and purified by preparative HPLC (C18, 10 mmol/L NH₄HCO₃ in water, MeCN) to obtain the title compound (2.0 mg, 1.7%). LCMS (ESI) [M+H]⁺ = 533.6; ¹H NMR (400 MHz, CDCl₃) δ 8.62 - 8.46 (m, 1H), 7.19 - 7.10 (m, 3H), 7.06 - 6.99 (m, 1H), 6.69 - 6.56 (m, 1H), 5.46 - 5.20 (m, 1H), 4.71 (d, *J* = 12.0 Hz, 1H), 4.49 (d, *J* = 13.5 Hz, 1H), 4.33 - 4.08 (m, 2H), 4.00 (d, *J* = 14.6 Hz, 1H), 3.86 - 3.67 (m, 3H), 3.28 - 3.14 (m, 1H), 3.14 - 2.56 (m, 8H), 2.34 (tt, *J* = 7.9, 4.6 Hz, 1H), 2.21 - 2.06 (m, 2H), 2.06 - 1.97 (m, 1H), 1.89 (d, *J* = 12.5 Hz, 1H), 1.51 (pd, *J* = 12.0, 4.1 Hz, 3H), 1.31 - 1.21 (m, 2H), 1.19-1.11 (m, 2H).

### Example 100

### Preparation of (trans-4-(3,4-dihydroisoquinolin-2(1H)-yl)-3-hydroxypiperidin-1-yl)-(6-((1-methylpiperidin-4-yl)amino)pyrimidin-4-yl)ketone:

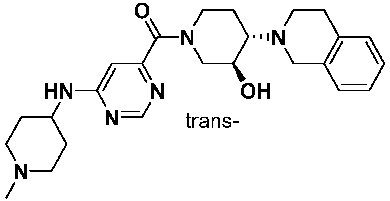

### Step I: preparation of (trans-4-(3,4-dihydroisoquinolin-2(1H)-yl)-3-hydroxypiperidin-1-yl)-(6-((1-methylpiperidin-4-yl)amino)pyrimidin-4-yl)ketone:

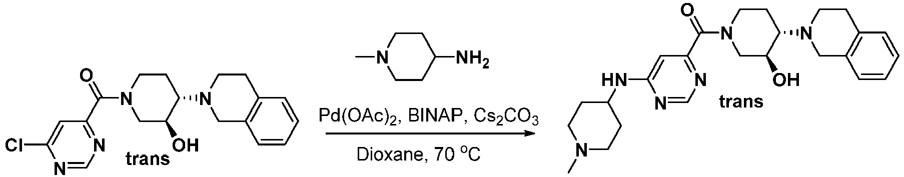

The raw material trans-(6-chloropyrimidin-4-yl)-4-(3,4-dihydroisoquinolin-2(1*H*)-yl)-3-hydroxypiperidin-1-yl)ketone (80 mg, 0.22 mmol, 1.0 eq) was dissolved in 1,4-dioxane (2 mL) at room temperature (20°C) under nitrogen protection, followed by addition of 1-methylpiperidin-4-amine hydrochloride (74 mg, 0.64 mmol, 3.0 eq), palladium acetate (10 mg, 0.04 mmol, 0.2 eq), BINAP (1,1'- binaphthyl-2,2'-bis(diphenylphosphine)) (53 mg, 0.08 mmol, 0.4 eq) and cesium carbonate (350 mg, 1.07 mmol, 5.0 eq), and the mixture was heated at 70°C under stirring for 4 h. After the reaction was complete as indicated by LCMS, the reaction mixture was cooled to room temperature, diluted with dichloromethane (5 mL), filtrated and concentrated. The crude product was separated and purified by preparative HPLC (C18, 10mmol/L aqueous NH₄HCO₃, acetonitrile) to obtain the title compound (7.6 mg , 7.8%). LCMS: [M+H]⁺ =451.37; ¹H NMR (400 MHz, CDCl₃)δ 8.66 - 8.43 (m, 1H), 7.20 - 7.09 (m, 3H), 7.07 - 6.98 (m, 1H), 6.64 - 6.45 (m, 1H), 5.29 - 4.64 (m, 2H), 4.35 - 4.09 (m, 1H), 4.05 - 3.83 (m, 2H), 3.74 - 3.67 (m, 2H), 3.13 - 2.96 (m, 2H), 2.94 - 2.89 (m, 2H), 2.87 - 2.80 (m, 2H), 2.79 - 2.53 (m, 3H), 2.31 (s, 3H), 2.20 - 2.11 (m, 2H), 2.09 - 1.97 (m, 3H), 1.73 - 1.49 (m, 4H).

### Example 101

### Preparation of trans-4-(3,4-dihydroisoquinolin-2(1H)-yl)-3-hydroxypiperidin-1-yl) (6-(anilino)pyrimidin-4-yl)ketone:

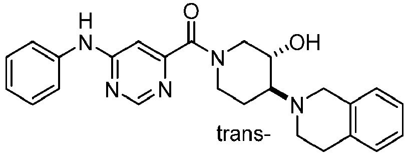

### Step I: preparation of methyl 6-(anilino)pyrimidine-4-carboxylate:

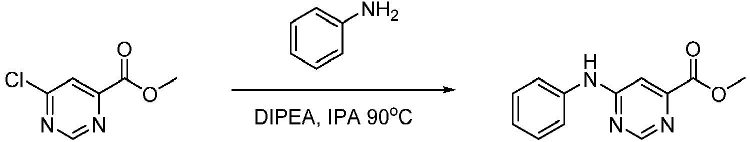

Methyl 6-chloro-pyrimidine-4-carboxylate (200 mg, 1.16 mmol), aniline (109.93 mg, 1.16 mmol) and DIPEA (N,N-diisopropylethylamine) (299.59 mg, 2.32 mmol) were dissolved in IPA (isopropanol) (2.5 mL), and the reaction mixture was stirred at 90°C for 16 h. The reaction was complete as indicated by TLC. The reaction mixture was extracted three times with ethyl acetate (20 mL each). The ethyl acetate phases were combined, washed once with 10 mL water and once with 10 mL saturated sodium chloride solution, dried over anhydrous sodium sulfate for 10 min, and filtrated. The crude product was purified by column chromatography with PE:EA=2:1 to obtain methyl 6-(anilino)pyrimidine-4-carboxylate (0.237 g, 89%) as a white solid. ¹H NMR (400 MHz, CDCl₃) *δ* 8.86 (s, 1H), 7.49 - 7.35 (m, 5H), 7.24 - 7.20 (m, 1H), 3.98 (s, 3H).

### Step II: preparation of 6-(anilino)pyrimidine-4-carboxylic acid:

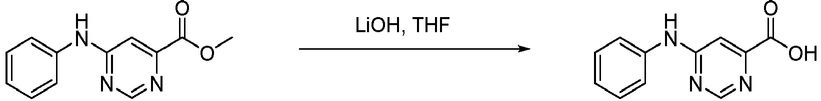

Methyl 6-(anilino)pyrimidine-4-carboxylate (187 mg, 0.815 mmol) was dissolved in THF (4 mL), and 2 M (molar concentration) aqueous lithium hydroxide (0.83 mL, 1.63 mmol) was added. The reaction mixture was stirred at room temperature (20-25°C) for 2 h. The reaction was complete as indicated by TLC. 1 M (molar concentration) hydrochloric acid was added to adjust to pH=4~5, and solid was precipitated. Upon filtration, the filter cake was washed with 2 mL water. A pure product of 6-(anilino)pyrimidine-4-carboxylic acid (136 mg, 78%) was obtained as a white solid. LCMS (ESI) [M+H]⁺ =279.0;

### Step III: preparation of trans-4-(3,4-dihydroisoquinolin-2(1H)-yl)-3-hydroxypiperidin-1-yl) (6-(anilino)pyrimidin-4-yl)ketone:

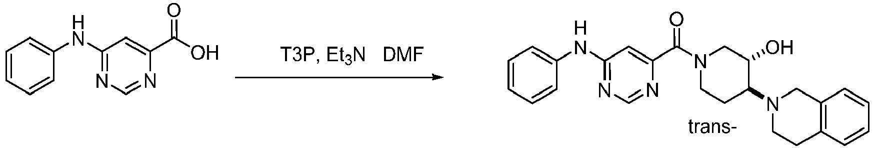

6-(anilino)pyrimidine-4-carboxylic acid (140 mg, 0.65 mmol), T3P (1-propylphosphoric anhydride) (0.82 g, 1.3 mmol, 50% by mass in ethyl acetate), trans-4-(3,4-dihydroisoquinolin-2(1*H*)-yl)piperidin-3-ol (151 mg, 0.65 mmol) and Et₃N (triethylamine) (0.329 g, 3.25 mmol) were dissolved in DMF (3 mL), and the reaction mixture was stirred at room temperature for 2 h. The reaction was complete as indicated by LCMS. The reaction mixture was extracted three times with ethyl acetate (10 mL each). The ethyl acetate phases were combined, washed once with 10 mL water and once with 10 mL saturated sodium chloride solution, dried over anhydrous sodium sulfate for 10 min, and filtrated. The crude product was separated and purified by preparative HPLC (C18, 10 mmol/L aqueous NH₄HCO₃, acetonitrile) to obtain the title compound (144 mg, 75%). LCMS (ESI) [M+H]⁺ =430.4; ¹H NMR (400 MHz, DMSO-*d*₆) δ 9.81 (d, *J=* 7.3 Hz, 1H), 8.64 (dd, *J* = 9.0, 1.2 Hz, 1H), 7.67 (dd, *J* = 8.0, 3.7 Hz, 2H), 7.38 - 7.34 (m, 2H), 7.17 - 7.00 (m, 5H), 6.86 (dd, *J* = 6.2, 1.2 Hz, 1H), 4.79 (dd, *J* = 38.2, 3.9 Hz, 1H), 4.61 - 4.27 (m, 1H), 4.00 - 3.70 (m, 3H), 3.67 - 3.61 (m, 1H), 3.04 (t, *J* = 12.5 Hz, 0.5H), 2.99 - 2.72 (m, 4H), 2.71 - 2.57 (m, 1.5H), 2.01 - 1.69 (m, 1H), 1.54 - 1.50 (m, 1H).

### Example 102

### Preparation of trans-1-(4-((4-(4-(3,4-dihydroisoquinolin-2(1H)-yl)-3-hydroxypiperidine-1-carbonyl)-5-(trifluoromethyl)pyridin-2-yl)amino)piperidin-1-yl)ethan-1-one:

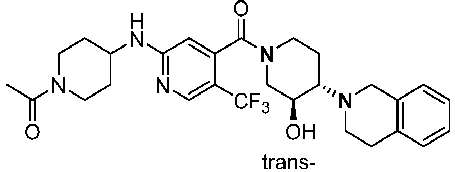

### Step I: preparation of methyl 2-((1-acetylpiperidin-4-yl)amino)-5-(trifluoromethyl)isonicotinate:

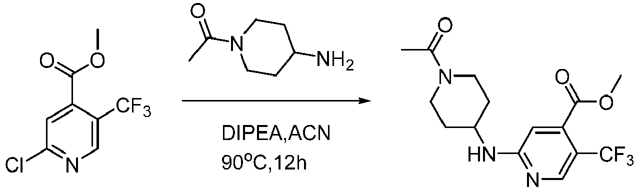

The raw material methyl 2-chloro-5-trifluoromethylisonicotinate (300 mg, 1.26 mmol, 1.0 equiv.) was added into acetonitrile (3 mL), followed by addition of 1-acetylpiperidin-4-amine hydrochloride (268 mg, 1.51 mmol, 1.5 equiv) and DIPEA (N,N-diisopropylethylamine) (487 mg, 3.78 mmol, 3.0 equiv.), and stirred at 90°C for 12h. After the reaction was complete as indicated by LCMS, the reaction mixture was cooled to room temperature (28°C), mixed with water (5 mL), and then extracted three times with dichloromethane:methanol (V/V= 10/1) (5 mL×3). The organic phases were combined, filtrated and concentrated, and the crude product was separated and purified by flash chromatography (silica gel, dichloromethane:methanol (V/V= 8/1)) to obtain the title compound methyl 2-((1-acetylpiperidin-4-yl)amino)-5-(trifluoromethyl)isonicotinate (150 mg, 34 %) as a yellow solid. LCMS (ESI) [M+H]⁺= 346.35.

### Step II: preparation of 2-((1-acetylpiperidin-4-yl)amino)-5-(trifluoromethyl)isonicotinic acid:

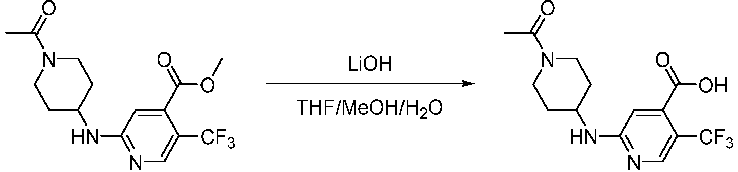

The raw material methyl 2-((1-acetylpiperidin-4-yl)amino)-5-(trifluoromethyl)isonicotinate (150 mg, 0.43 mmol, 1.0 equiv.) was added at room temperature (28°C) to water (1 mL), methanol (1.0 mL) and tetrahydrofuran (1.0 mL), followed by addition of lithium hydroxide (42 mg, 1.74 mmol, 1.1 equiv.), and stirred at room temperature (28°C) for 1h. After the reaction was complete as indicated by TLC analysis (dichloromethane:methanol (V/V= 10/1)), the reaction mixture was concentrated to 1 mL, adjusted to pH=1-2 with 1M (molar concentration) HCl, and the crude product was separated and purified by preparative HPLC (C18, 10 mmol/L aqueous NH₄HCO₃, acetonitrile), and lyophilized to obtain the title compound (30 mg, yield: 21%) as a white solid. LCMS (ESI) [M+H]⁺= 332.3; ¹H NMR (400 MHz, MeOD-d4) δ 8.31 (s, 1H), 6.89 (d, *J=* 4.0 Hz, 1H), 4.46 (dd, *J=* 1.6 Hz, 13.2 Hz, 1H), 4.11(brs, 1H), 3.95 (dd, *J=* 1.2 Hz, 13.6 Hz, 1H), 2.96-2.89 (m, 1H), 2.14 (s, 3H), 2.12-2.03 (m, 2H),1.54-1.43 (m, 2H). Step III: preparation of trans-1-(4-((4-(4-(3,4-dihydroisoquinolin-2(1H)-yl)-3-hydroxy-piperidine-1-carbonyl)-5-(trifluoromethyl)pyridin-2-yl)amino)piperidin-1-yl)ethan-1-one:

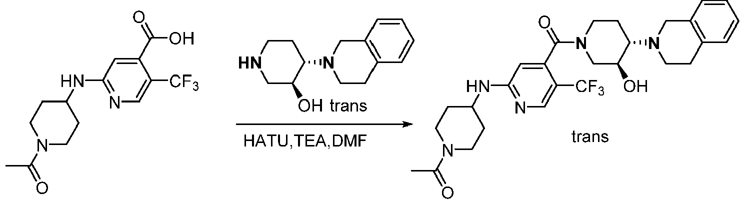

The raw material 2-((1-acetylpiperidin-4-yl)amino)-5-(trifluoromethyl)isonicotinic acid (30 mg, 0.114 mmol, 1.0 equiv.) was added to DMF (N,N-dimethylformamide) (1 mL), followed by addition of HATU (52 mg, 0.137 mmol, 1.2 equiv.) and triethylamine (23 mg, 0.228 mmol, 2.0 equiv.). The mixture was stirred for 5 mins and then a solution of trans-4-(3,4-dihydroisoquinolin-2(1*H*)-yl)piperidin-3-ol (32 mg, 0.137mmol, 1.20 equiv.) in DMF (1 mL) was added. The mixture was then stirred at room temperature (28°C) for 3h. After the reaction was complete as indicated by LCMS, the crude product was separated and purified by preparative HPLC (C18, 10 mmol/L aqueous NH₄HCO₃, acetonitrile), and lyophilized to obtain the title compound (8 mg, yield: 12.8%). LCMS (ESI) [M+H]+ = 546.3. ¹H NMR (400 MHz, CD₃OD) δ 8.35 (t, *J* = 4.8 Hz, 1H), 7.10-7.05 (m, 4H), 6.49-6.40 (m, 1H), 4.78-4.60 (m, 1H), 4.45 (d, *J=* 13.2Hz, 1H), 4.14(brs,1H), 3.98-3.88 (m, 3H), 3.82-3.48 (m, 2H), 3.20-2.98 (m, 2H), 2.95-2.87 (m, 4H), 2.83-2.64 (m, 2H),2.14 (s, 3H), 2.07-1.85(m,3H), 1.68-1.38 (m,3H).

### Example 103

### Preparation of trans-1-(4-((((6-(4-(3,4-dihydroisoquinolin-2(1H)-yl)-3-hydroxy-piperidine-1-carbonyl)pyrimidin-4-yl)amino)methyl)piperidin-1-yl)ethanone:

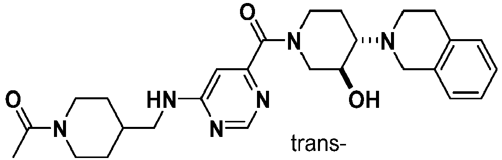

### Step I: preparation of t-butyl ((1-acetylpiperidin-4-yl)methyl)carbamate:

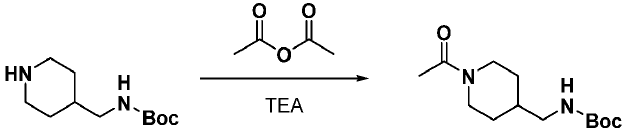

The raw material t-butyl *N*-(4- piperidylmethyl)carbamate (500 mg, 2.33 mmol, 1 equiv.) was dissolved in dichloromethane (10 ml), followed by addition of TEA (triethylamine) (354 mg, 3.50 mmol, 1.5 equiv.) and acetic anhydride (240 mg, 2.35 mmol, 1.01 equiv.) under nitrogen at 0°C. The reaction was stirred at room temperature (20-25°C) for 2 h, and was quenched with water. Upon extraction with dichloromethane, the organic phase was dried and concentrated, and separated and purified by flash chromatography (silica gel, MeOH:DCM = 0-3%) to obtain the product t-butyl ((1-acetylpiperidin-4-yl)methyl)carbamate (580 mg, yield: 97%) as a light yellow oily substance. LCMS(ESI)[M+1]⁺ = 257.31.

### Step II: preparation of 1-(4-(aminomethyl)piperidin-1-yl)ethanone hydrochloride:

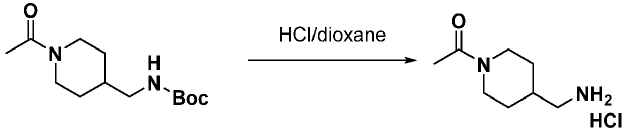

The raw material t-butyl ((1-acetylpiperidin-4-yl)methyl)carbamate (100 mg, 0.390 mmol, 1 equiv.) was dissolved in dioxane (1 ml), and 4M (molar concentration) hydrochloric acid in dioxane (1 ml) was added. The reaction mixture was stirred at room temperature (20-25°C) for 2h, and the reaction was complete as monitored by TLC. The solution was rotary evaporated to dryness to obtain the product 1-(4-(aminomethyl)piperidin-1-yl)ethanone hydrochloride (103 mg crude product) as a white solid. LCMS(ESI)[M+1]⁺ = 157.14.

### Step III: preparation of methyl 6-((((1-acetylpiperidin-4-yl)methyl)amino)-pyrimidine-4-carboxylate:

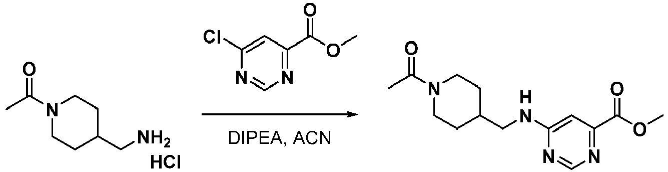

1-(4-(aminomethyl)piperidin-1-yl)ethanone hydrochloride (103 mg crude product, 0.390 mmol, 1.2 equiv.) and methyl 6-chloropyrimidine-4-carboxylate (56 mg, 0.325 mmol, 1 equiv.) were dissolved in acetonitrile (3 ml) (suspension), followed by addition of DIPEA (N,N-diisopropylethylamine) (169 mg, 1.31 mmol, 4.03 equiv.), and stirred at 90°C for 2h. After the reaction was complete as monitored by LCMS, The solution was rotary evaporated to dryness, and was separated and purified by flash chromatography (silica gel, MeOH:DCM = 0-4%) to obtain the product methyl 6-((((1-acetylpiperidin-4-yl)methyl) amino)pyrimidine-4-carboxylate (152 mg) as a light yellow oily substance. LCMS(ESI)[M+1]⁺ = 293.3.

### Step IV: preparation of 6-((((1-acetylpiperidin-4-yl)methyl)amino)pyrimidine-4-carboxylic acid:

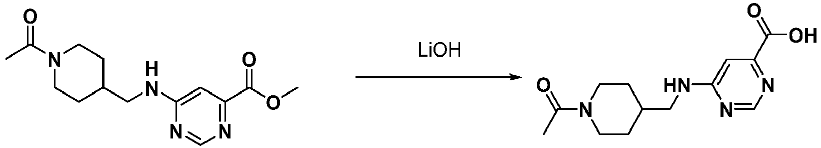

The raw material methyl 6-((((1-acetylpiperidin-4-yl)methyl)amino)pyrimidine-4-carboxylate (152 mg, 0.520 mmol, 1 equiv.) was dissolved in a mixed solution of tetrahydrofuran (2.4 ml) and water (0.6 ml), followed by addition of LiOH (lithium hydroxide) (25 mg, 1.044 mmol, 2.01 equiv.), and the reaction was stirred at room temperature (20-25°C) for 1 h. The reaction was complete as monitored by TLC. 1M (molar concentration) hydrochloric acid was added to adjust to pH 5-6, and the solvent was evaporated to dryness to obtain a crude product of 6-((((1-acetylpiperidin-4-yl)methyl)-amino)pyrimidine-4-carboxylic acid, which was directly used in the following step. LCMS(ESI)[M+1]⁺ = 279.0.

### Step V: preparation of trans-1-(4-((((6-(4-(3,4-dihydroisoquinolin-2(1H)-yl)-3-hydroxypiperidine-1-carbonyl)pyrimidin-4-yl)amino)methyl)piperidin-1-yl)ethanone:

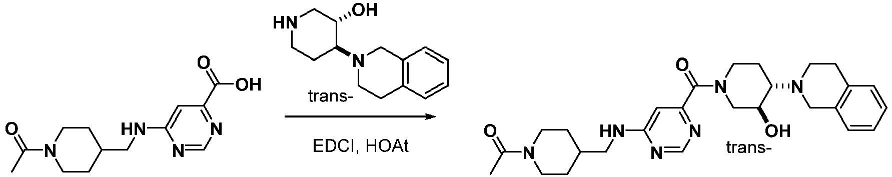

The reactant 6-(((1-acetylpiperidin-4-yl)methyl)amino)pyrimidine-4-carboxylic acid (190 mg, 0.512 mmol, 1 equiv.) was dissolved in DMF (*N*,*N*-dimethylformamide) (2.5 ml), and EDCI (1-(3-dimethylaminopropyl)-3-ethylcarbodiimide hydrochloride) (150 mg, 0.782 mmol, 1.53 equiv.) and HOAt (*N*-hydroxy-7-azabenzotriazole) (106 mg, 0.779 mmol, 1.52 equiv.) were added thereto. After stirring for 5 min, a solution of trans-4-(3,4-dihydroisoquinolin-2(1*H*)-yl)piperidin-3-ol (200 mg, 0.517 mmol, 1.01 equiv.) in DMF (2.5 ml) was added, and the reaction mixture was stirred at room temperature for 1 h. The solvent was evaporated to dryness and the crude product was separated and purified by flash chromatography (silica gel, MeOH: DCM = 0-10%) and then purified on a prep-HPLC reverse phase column (C18, 10 mmol/L aqueous NH₄HCO₃, acetonitrile) to obtain the product (66.5 mg, yield: 26.4%). LCMS(ESI)[M+1]⁺ = 493.5; ¹H NMR (400 MHz, Chloroform-*d*) *δ* 8.59 - 8.46 (m, 1H), 7.21 - 7.08 (m, 3H), 7.07 - 6.98 (m, 1H), 6.68 - 6.56 (m, 1H), 5.77 - 5.38 (m, 1H), 5.09 - 4.54 (m, 2H), 4.31 - 4.10 (m, 1H), 4.02 - 3.59 (m, 5H), 3.44 - 3.17 (m, 2H), 3.11 - 2.79 (m, 6H), 2.77 - 2.45 (m, 3H), 2.12 - 2.08 (m, 3H), 2.03 - 1.58 (m, 5H), 1.31 - 1.08 (m, 2H).

### Example 104

### Preparation of trans-1-(4-((6-(-4-(3,4-dihydroisoquinolin-2(1H)-yl)-3-hydroxy-piperidine-1-carbonyl)-2-phenylpyrimidin-4-yl)amino)piperidin-1-ylethanone:

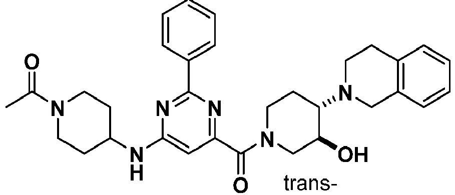

### Step I: preparation of 6-((1-acetylpiperidin-4-yl)amino)-2-phenylpyrimidine-4-carboxylic acid:

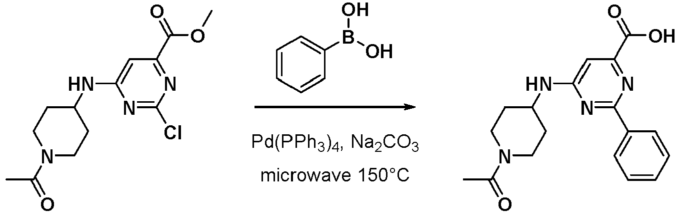

The raw materials methyl 6-(((1-acetylpiperidin-4-yl)amino)-2-chloropyrimidine-4-carboxylate (100 mg, 0.320 mmol, 1 equiv.), phenylboronic acid (35 mg, 0.0303 mmol, 0.09 equiv.), Pd(PPh₃)₄ and Na₂CO₃ (sodium carbonate) (49 mg, 0.462 mmol, 1.45 equiv.) were weighed and put into a microwave tube, and the tube was sealed and purged inside with nitrogen. The solvents acetonitrile (1.2 ml) and water (0.3 ml, purged with nitrogen beforehand to remove oxygen) were added. The reaction was stirred under microwave at 150°C for 30 min. The reaction was complete as monitored by LCMS. The reaction mixture was filtrated and rotary evaporated, and the crude product was purified on a medium-pressure reverse phase column (C18, 0.05% aqueous formic acid, MeCN) to obtain the product 6-((1-acetylpiperidin-4-yl)amino)-2-phenylpyrimidine-4-carboxylic acid (70 mg, yield: 64.3%) as a light yellow solid. LCMS(ESI)[M+1]⁺ = 340.9; ¹H NMR (400 MHz, DMSO-*d*₆) *δ* 8.44 (s, 1H), 8.36 - 8.27 (m, 2H), 7.50 - 7.41 (m, 3H), 7.35 (brs, 1H), 6.85 (s, 1H), 4.24 (d, *J* = 13.1 Hz, 2H), 3.86 - 3.77 (m, 1H), 3.24 - 3.22 (s, 1H), 2.88 (t, *J=* 12.2 Hz, 1H), 2.08 - 1.88 (m, 5H), 1.52 - 1.20 (m, 2H).

### Step II: preparation of trans-1-(4-((6-(-4-(3,4-dihydroisoquinolin-2(1H)-yl)-3-hydroxypiperidine-1-carbonyl)-2-phenylpyrimidin-4-yl)amino)piperidin-1-ylethanone:

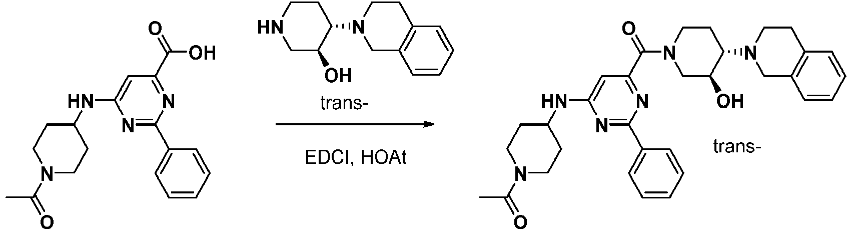

The reactant 6-((1-acetylpiperidin-4-yl)amino)-2-phenylpyrimidine-4-carboxylic acid (70 mg, 0.206 mmol, 1 equiv.) was dissolved in DMF (*N*,*N*-dimethylformamide) (2 ml), and EDCI (1-(3-dimethylaminopropyl)-3-ethylcarbodiimide hydrochloride) (59 mg, 0.308 mmol, 1.5 equiv.) and HOAt (N-hydroxy-7-azabenzotriazole) (42 mg, 0.309 mmol, 1.5 equiv.) were added thereto. After stirring for 5 min, a solution of trans-4-(3,4-dihydroisoquinolin-2(1*H*)-yl)piperidin-3-ol (58 mg, 0.250 mmol, 1.21 equiv.) in DMF (1 ml) was added, and the reaction mixture was stirred at room temperature for 1 h. The reaction mixture was rotary evaporated to dryness, and the crude product was purified on a preparative HPLC reverse phase column (C18, 10 mmol/L aqueous NH₄HCO₃, acetonitrile) to obtain the title compound (37.9 mg, yield: 34.8%). LCMS(ESI)[M+1]⁺ = 555.6; ¹H NMR (400 MHz, CDCl₃) *δ* 8.39 - 8.29 (m, 2H), 7.52 - 7.40 (m, 3H), 7.20 - 7.10 (m, 3H), 7.08 - 7.02 (m, 1H), 6.65 - 6.48 (m, 1H), 5.45 - 5.22 (m, 1H), 5.13 - 4.72 (m, 1H), 4.59 - 4.34 (m, 2H), 4.17 (brs, 1H), 4.05 - 3.94 (m, 1H), 3.93 - 3.69 (m, 4H), 3.31 - 3.17 (m, 1H), 3.17 - 3.00 (m, 2H), 2.98 - 2.63 (m, 6H), 2.29 - 1.98 (m, 5H), 1.92 - 1.69 (m, 2H), 1.53 - 1.38 (m, 2H).

### Example 105

### Preparation of trans-(4-(3,4-dihydroisoquinolin-2(1H)-yl)-3-hydroxypiperidin-1-yl)(6-((2-methoxyphenyl)amino)pyrimidin-4-yl)ketone:

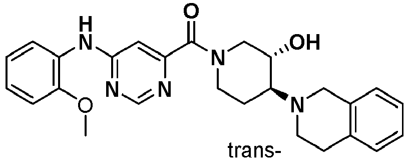

### Step I: preparation of methyl 6-((2-methoxyphenyl)amino)pyrimidine-4-carboxylate:

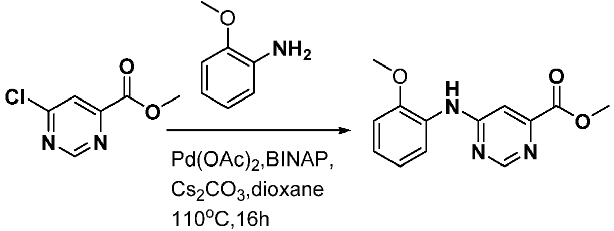

The raw material methyl 6-chloropyrimidine-4-carboxylate (500 mg, 28.97 mmol, 1.0 equiv.) was added to dioxane (10 mL), followed by addition of 2-methoxyaniline (428 mg, 34.77 mmol, 1.2 equiv.), Pd(OAc)₂ (palladium acetate) (65 mg, 2.9 mmol, 0.1 equiv.), BINAP (binaphthyl diphenylphosphine) (361 mg, 5.79 mmol, 0.2 equiv.), and Cs₂CO₃ (cesium carbonate) (2.36 g, 72.43 mmol, 2.5 equiv.), and stirred under nitrogen at 110°C for 16h. After the reaction was complete as indicated by LCMS, the reaction mixture was cooled to room temperature 25°C, mixed with water (20 mL), and extracted three times with dichloromethane (30 mL×3). The organic phases were combined, filtrated and concentrated, and the crude product was separated and purified by flash chromatography (silica gel, petroleum etherethyl acetate(V/V= 1/3)) to obtain the title compound (178 mg, 23.7 %) as a yellow solid. LCMS (ESI) [M+H]⁺= 260.2.

### Step II: preparation of 6-((2-methoxyphenyl)amino)pyrimidine-4-carboxylic acid:

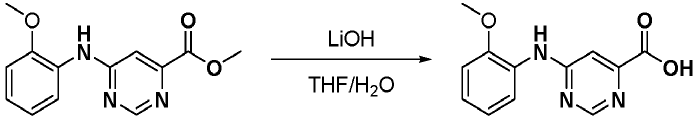

The raw material methyl 6-((2-methoxyphenyl)amino)pyrimidine-4-carboxylate (178 mg, 6.87 mmol, 1.0 equiv.) was added to water (3 mL) and tetrahydrofuran (3.0 mL) at room temperature (25°C), followed by addition of lithium hydroxide (57.7 mg, 13.74 mmol, 2.0 equiv.), and stirred at room temperature for 1 h. After the reaction was complete as indicated by LCMS, the reaction mixture was concentrated to remove the organice solvent, adjusted to pH=3 with 1 M HCl, and the mixture was extracted three times with dichloromethane:methanol (V/V= 10/1) (30 mL×5). The organic phases were combined, dried, filtrated, and concentrated to obtain a crude product 6-((2-methoxyphenyl)amino)pyrimidine-4-carboxylic acid (180 mg, crude) as a yellow solid. LCMS (ESI) [M+H]⁺= 246.1.

### Step III: preparation of trans-(4-(3,4-dihydroisoquinolin-2(1H)-yl)-3-hydroxypiperidin-1-yl)-(6-((2-methoxyphenyl)amino)pyrimidin-4-yl)ketone:

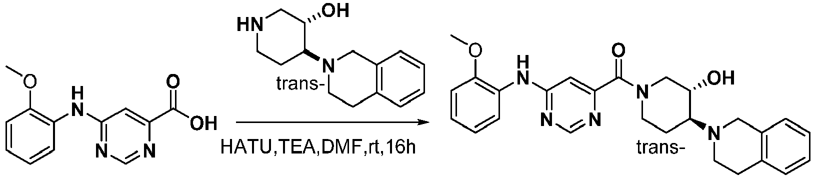

The raw material 6-((2-methoxyphenyl)amino)pyrimidine-4-carboxylic acid (180 mg, 7.34 mmol, 1.0 equiv.) was added to DMF (N,N-dimethylformamide) (3mL), followed by addition of HATU (335 mg, 8.81 mmol, 1.2 equiv.), triethylamine (285 mg, 22 mmol, 3.0 equiv.) and trans-4-(3,4-dihydroisoquinolin-2(1H)-yl)piperidin-3-ol (205 mg, 8.81 mmol, 1.2 equiv.), and stirred at room temperature (25°C) for 16 h. After the reaction was complete as indicated by LCMS, the crude product was separated and purified by Prep-HPLC (C18, 10 mmol/L NH₄HCO₃ in water, MeCN), and lyophilized to obtain the compound trans-(4-(3,4-dihydroisoquinolin-2(1H)-yl)-3-hydroxypiperidin-1-yl)(6-((2-methoxyphenyl)-amino)pyrimidin-4-yl)ketone (60.2 mg, 17.8%). LCMS (ESI) [M+H]+ = 460.4; ¹H NMR (400 MHz, DMSO-d6) *δ* 9.14 (d, *J=* 7.2 Hz, 1H), 8.56 (dd, *J=* 8.4 Hz, 0.8 Hz, 1H), 7.84 (d, *J* = 7.2 Hz, 1H), 7.17-7.03 (m, 6H), 6.99-6.95 (m, 1H), 6.87 (s, 1H), 4.79 (dd, *J =* 34.4 Hz, 4.0 Hz, 1H), 4.50-4.33 (m, 1H), 3.86-3.80 (m, 5H), 3.76-3.60 (m, 2H), 3.06-2.79 (m, 6H), 2.67-2.58 (m, 1H), 1.87-1.73 (m, 1H), 1.55-1.44 (m, 1H).

### Example 106

### Preparation of trans-1-(3-((6-(4-(3,4-dihydroisoquinolin-2(1H)-yl)-3-hydroxypiperidine-1-carbonyl)pyrimidin-4-yl)amino)piperidin-1-yl)ethan-1-one:

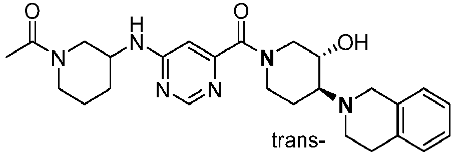

### Step I: preparation of t-butyl (1-acetylpiperidin-3-yl)carbamate:

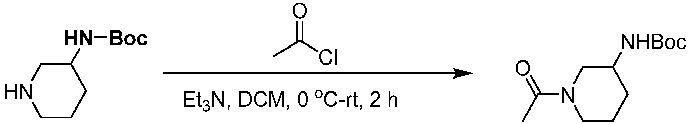

The compound 3-t-butoxycarbonylaminopiperidine (1.2 g, 6.0 mmol, 1.0 equiv.) was dissolved in DCM (dichloromethane) (20 mL), cooled on an ice water bath to 0 °C, followed by addition of Et₃N (triethylamine) (1.5 mL, 10.8 mmol, 1.8 equiv.) and then addition of acetyl chloride (0.6 mL, 8.4 mmol, 1.4 equiv.), and the reaction was stirred under stirring at 0°C to room temperature overnight (15h). After the reaction was complete as indicated by LCMS, the reaction mixture was concentrated, diluted with ethyl acetate (30 mL), washed sequentially with 0.5N HCl (15 mL), water (30 mL) and saturated sodium chloride (30 mL), dried over anhydrous sodium sulfate for 10 mins, filtrated and concentrated to obtain the title compound t-butyl (1-acetylpiperidin-3-yl)carbamate (800 mg, 55%) as a yellow solid. LCMS (ESI) [M+H]⁺= 243.3; ¹H NMR (400 MHz, DMSO-d6) δ 6.96-6.83 (m, 1H), 4.19-3.70 (m, 1H), 3.60 (t, J = 12.4 Hz, 1H), 3.32-3.02(m, 2H), 2.98-2.90(m, 1H), 1.98-1.91(m, 3H), 1.81-1.79(m, 1H), 1.71-1.63(m, 1H), 1.40-1.38(m, 9H), 1.35-1.23(m, 2 H).

### Step II: preparation of 1-(3-aminopiperidin-1-yl)ethan-1-one:

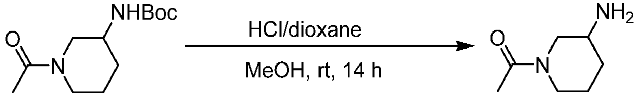

The raw material t-butyl (1-acetylpiperidin-3-yl)carbamate (800 mg, 3.31mmol, 1.0 equiv.) was dissolved at room temperature in methanol (10 mL), followed by addition of 4M HCl/dioxane (10 mL), and the reaction was stirred under stirring at room temperature (25°C) for 14 h. After the reaction was complete as indicated by LCMS, the reaction mixture was concentrated to obtain the title compound 1-(3-aminopiperidin-1-yl)ethan-1-one (700 mg, 94.5%) as a white oily solid.

### Step III: preparation of methyl 6-((1-acetylpiperidin-3-yl)amino)pyrimidine-4-carboxylate:

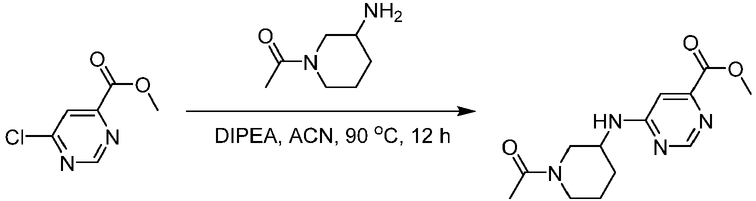

The raw material methyl 6-chloropyrimidine-4-carboxylate (100 mg, 0.58 mmol, 1.0 equiv.) was added to acetonitrile (3 mL), followed by addition of 1-(3-aminopiperidin-1-yl)ethan-1-one (155 mg, 0.87 mmol, 1.5 eq) and DIPEA(N,N-diisopropylethylamine) (224 mg, 1.74mmol, 3.0 equiv.), and stirred at 90°C for 12h. After the reaction was complete as indicated by LCMS, the reaction mixture was cooled to room temperature (18°C), mixed with water (5 mL), and extracted three times with dichloromethane:methanol (V/V= 10/1) (5 mL×3). The organic phases were combined, filtrated and concentrated, and the crude product was separated and purified by flash chromatography (silica gel, dichloromethane:methanol (V/V= 8/1)) to obtain the title compound as a yellow solid (80 mg, 49.6 %). LCMS (ESI) [M+H]+ = 279.28.

### Step IV: preparation of 6-((1-acetylpiperidin-3-yl)amino)pyrimidine-4-carboxylic acid:

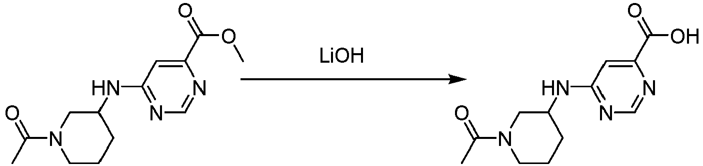

At room temperature (28°C), the raw material methyl 6-((1-acetylpiperidin-3-yl)amino)-pyrimidine-4-carboxylate (150 mg, 0.54 mmol, 1.0 equiv.) was added to water (1 mL) and methanol (1.0 mL), followed by addition of lithium hydroxide (52 mg, 2.16 mmol, 1.1 eq), and stirred at room temperature for 1h. After the reaction was complete as indicated by TLC analysis (dichloromethane:methanol (V/V= 10/1)), the reaction mixture was concentrated to 1 mL, adjusted to pH=1-2 with 1M HCl, and the aqueous phase was lyophilized to obtain the title compound (153 mg, yield>90 %) as a yellow solid.

### Step V: preparation of trans-1-(3-((6-(4-(3,4-dihydroisoquinolin-2(1H)-yl)-3-hydroxypiperidine-1-carbonyl)pyrimidin-4-yl)amino)piperidin-1-yl)ethan-1-one:

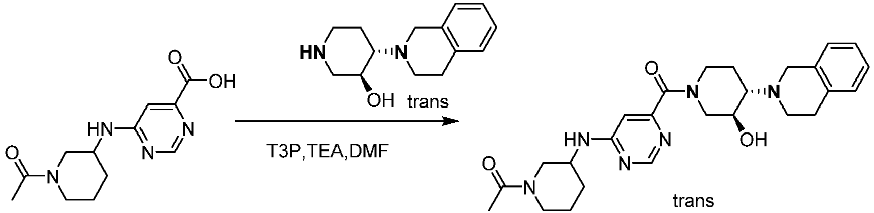

The raw material 6-((1-acetylpiperidin-3-yl)amino)pyrimidine-4-carboxylic acid (130 mg, 0.49 mmol, 1.0 equiv.) was added to DMF (*N*,*N*-dimethylformamide) (3 mL), followed by addition of T₃P (1-propylphosphoric anhydride) (50% by mass in ethyl acetate) (623 mg, 0.98 mmol, 2.0 equiv.), triethylamine (99 mg, 0.98 mmol, 2.0 equiv.), and trans-4-(3,4-dihydroisoquinolin-2(1H)-yl)piperidin-3-ol (136 mg, 0.59 mmol, 1.20 equiv.), and stirred at room temperature (25°C) for 14h. After the reaction was complete as indicated by LCMS, the reaction mixture was mixed with water (5 mL), and extracted three times with dichloromethane:methanol (V/V= 10/1) (5 mL×3). The organic phases were combined, filtrated and concentrated, and the crude product was separated and purified by preparative HPLC (C18, 10 mmol/L aqueous NH₄HCO₃, acetonitrile), and then lyophilized to obtain the title compound (4 mg, yield: 1.7%). LCMS (ESI) [M+H]+ = 479.3; ¹H NMR (400 MHz, MeOD-d4) δ 8.54-8.47 (m, 1H), 7.12-7.06 (m, 4H), 6.66-6.64 (m, 1H), 4.73-4.70 (m, 1H), 4.57-4.53 (m, 1H), 4.26-4.05 (m, 1H), 4.01-3.71(m, 7H), 3.27-3.13 (m, 2H), 3.06-2.87 (m, 6H), 2.77-2.74 (m, 2H), 2.15-2.03 (m, 5H),1.91-1.80 (m, 2H), 1.72-1.58 (m, 3H).

### Example 107

### Preparation of trans-4-(3,4-dihydroisoquinolin-2(1H)-yl)-3-hydroxypiperidin-1-yl)(6-((1-(methylsulfonyl)piperidin-4-yl)amino)pyrimidin-4-yl)ketone:

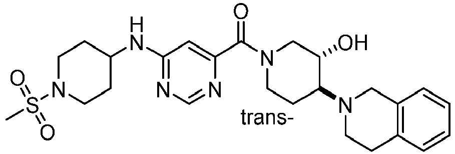

### Step I: preparation of trans-4-(3,4-dihydroisoquinolin-2(1H)-yl)-3-hydroxypiperidin-1-yl)(6-((1-(methylsulfonyl)piperidin-4-yl)amino)pyrimidin-4-yl)ketone:

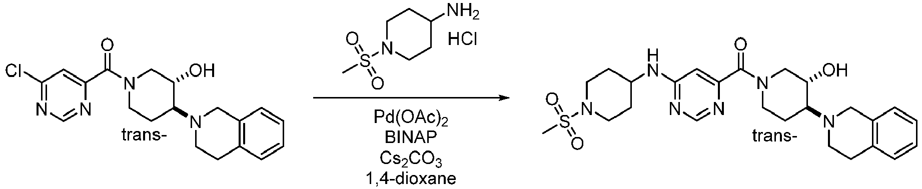

Trans-(6-chloropyrimidin-4-yl)(4-(3,4-dihydroisoquinolin-2(1*H*)-yl)-5-hydroxypiperidin-1-yl)ketone (50 mg, 0.13 mmol, 1.0 equiv.), 1-methanesulfonylpiperidin-4-amine hydrochloride (58 mg, 0.27 mmol, 2.0 equiv.), Pd(OAc)₂ (palladium acetate) (CAS: 462-08-8) (6 mg, 0.03 mmol, 0.2 equiv.), BINAP (1,1'-binaphthyl-2,2'-bis(diphenylphosphine)) (CAS: 98327-87-8) (34 mg, 0.05 mmol, 0.4 equiv.) and Cs₂CO₃ (cesium carbonate) (CAS: 534-17-8) (219 mg, 0.67 mmol, 5.0 equiv.) were dissolved in 1,4-dioxane (1 mL), and a reaction was stirred at 80°C under nitrogen for 16 h. The reaction mixture was filtrated, and the filtrate was concentrated. The crude product was separated and purified by preparative HPLC (C18, 10 mmol/L aqueous NH₄HCO₃, acetonitrile) to obtain the title compound (23.1 mg, 33.5%). LCMS (ESI) [M+H]⁺ = 515.35; ¹H NMR (400 MHz, CDCl₃) δ 8.60 - 8.49 (m, 1H), 7.19 - 7.10 (m, 3H), 7.07 - 6.99 (m, 1H), 6.67 - 6.53 (m, 1H), 5.37 - 5.13 (m, 1H), 4.71 (d, *J* = 13.3 Hz, 1H), 4.34 - 3.66 (m, 8H), 3.13 - 2.86 (m, 7H), 2.82 (s, 3H), 2.79 - 2.60 (m, 2H), 2.15 (d, *J* = 13.0 Hz, 2H), 2.06 - 1.83 (m, 2H), 1.65 - 1.57 (m, 2H).

### Example 108

### Preparation of trans-(4-(3,4-dihydroisoquinolin-2(1H)-yl)-3-hydroxypiperidin-1-yl)(6-((3-(pyrrolidin-1-yl)phenyl)amino)pyrimidin-4-yl)ketone:

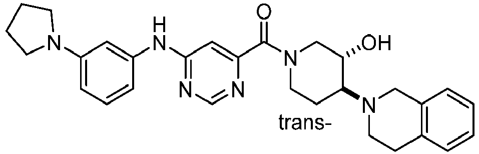

### Step I: preparation of trans-(4-(3,4-dihydroisoquinolin-2(1H)-yl)-3-hydroxypiperidin-1-yl)(6-((3-(pyrrolidin-1-yl)phenyl)amino)pyrimidin-4-yl)ketone:

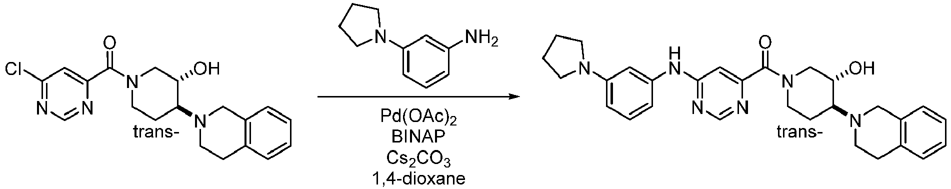

Trans-(6-chloropyrimidin-4-yl)(4-(3,4-dihydroisoquinolin-2(1H)-yl)-3-hydroxypiperidin-1-yl )- ketone (50 mg, 0.13 mmol, 1.0 equiv.), 3-pyrrolidine-1-aniline (44 mg, 0.27 mmol, 2.0 equiv.), Pd(OAc)₂ (palladium acetate) (6 mg, 0.03 mmol, 0.2 equiv.), BINAP (1,1'-binaphthyl-2,2'-bis(diphenylphosphine)) (33 mg, 0.05 mmol, 0.4 equiv.) and Cs₂CO₃ (cesium carbonate) (87 mg, 0.27 mmol, 2.0 equiv.) were dissolved in 1,4-dioxane (1 mL), and a reaction was stirred at 80°C under nitrogen protection for 16 h. The reaction mixture was filtrated, and the filtrate was concentrated. The crude product was separated and purified by preparative HPLC (C18, 10 mmol/L aqueous NH₄HCO₃, acetonitrile) to obtain the title compound (14.0 mg, 21.0%). LCMS (ESI) [M+H]⁺ = 499.42; ¹H NMR (400 MHz, CDCl₃) δ 8.65 - 8.61 (m, 1H), 7.25 - 7.19 (m, 1H), 7.18 - 7.09 (m, 4H), 7.05 - 6.94 (m, 2H), 6.61 - 6.57 (m, 1H), 6.48 - 6.41 (m, 2H), 5.05 - 4.67 (m, 1H), 4.22 - 4.02 (m, 1H), 4.00 - 3.83 (m, 2H), 3.77 - 3.62 (m, 2H), 3.35 - 3.23 (m, 4H), 3.09 - 3.00 (m, 1H), 2.99 - 2.55 (m, 6H), 2.05 - 1.98 (m, 4H), 1.99 - 1.94 (m, 1H), 1.88 - 1.80 (m, 1H).

### Example 109

### Preparation of trans-4-(3,4-dihydroisoquinolin-2(1H)-yl)-3-hydroxypiperidin-1-yl)(6-((tetrahydro-2H-pyran-4-yl)amino)pyrimidin-4-yl)ketone:

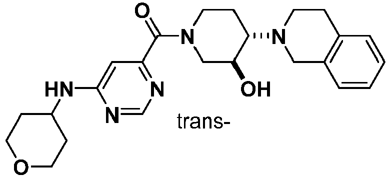

### Step I: preparation of methyl 6-((tetrahydro-2H-pyran-4-yl)amino)pyrimidine-4-carboxylate:

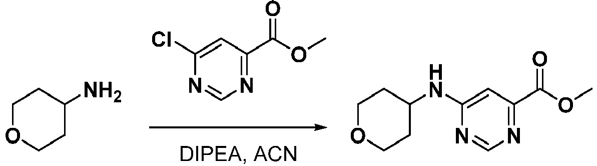

4-aminotetrahydropyran (141 mg, 1.39 mmol, 1.2 equiv.) and methyl 6-chloropyrimidine-4-carboxylate (200 mg, 1.16 mmol, 1 equiv.) were dissolved in acetonitrile (4 mL), followed by addition of DIPEA (N,N-diisopropylethylamine) (600 mg, 4.64 mmol, 4.0 equiv.), and stirred at 70°C for 4 h. After the reaction was complete as monitored by LCMS, the solvent was rotary evaporated to dryness, and upon separation and purification by flash chromatography (silica gel, MeOH:DCM = 0-4%), the product methyl 6-((tetrahydro-2H-pyran-4-yl)amino)pyrimidine-4-carboxylate (180 mg, yield: 65.5%) was obtained as a yellow oily substance. LCMS(ESI)[M+H]⁺ = 238.19.

### Step II: preparation of 6-((tetrahydro-2H-pyran-4-yl)amino)pyrimidine-4-carboxylic acid:

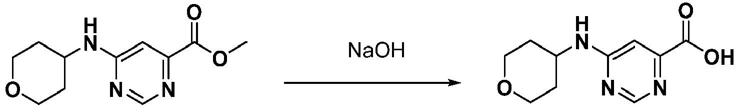

The raw material methyl 6-((tetrahydro-2H-pyran-4-yl)amino)pyrimidine-4-carboxylate (180 mg, 0.76 mmol, 1.0 equiv.) was dissolved in methanol (1.5 mL), followed by addition of 3.5 M (molar concentration) NaOH (sodium hydroxide) aqueous solution (0.15 mL.), and the reaction was stirred under stirring at room temperature (20-25°C) for 2 h. The reaction was complete as monitored by TLC. 1M (molar concentration) HCl was added to adjust to pH 5-6, and the solvent was rotary evaporated to obtain a crude product of 6-((tetrahydro-2H-pyran-4-yl)amino)pyrimidine-4-carboxylic acid, which was directly used in the following step. LCMS(ESI)[M+H]⁺ = 224.16.

### Step III: preparation of trans-4-(3,4-dihydroisoquinolin-2(1H)-yl)-3-hydroxypiperidin-1-yl) (6-((tetrahydro-2H-pyran-4-yl)amino)pyrimidin-4-yl)ketone:

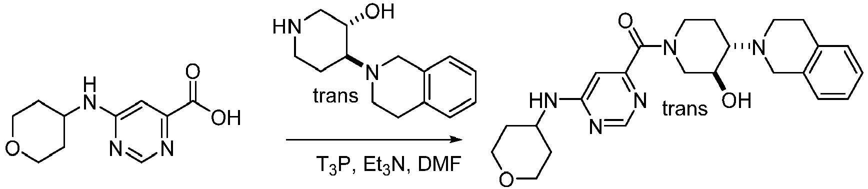

The reactant 6-((tetrahydro-2H-pyran-4-yl)amino)pyrimidine-4-carboxylic acid (70 mg, 0.31 mmol, 1 equiv.) was dissolved in DMF (N,N-dimethylformamide) (1.5 ml), followed by addition of T₃P (1-N-propylphosphoric anhydride) in ethyl acetate (50% by mass) (400 mg, 0.63 mmol, 2.0 equiv.) and Et₃N (triethylamine) (159 mg, 1.57 mmol, 5.0 equiv.). Then trans-4-(3,4-dihydroisoquinolin-2(1H)-yl)piperidin-3-ol (72.8 mg, 0.31 mmol, 1.0 equiv.) was added, and a reaction was stirred under stirring at room temperature (20-25°C) for 1 h. The solvent was rotary evaporated, and the crude product was purified with a preparative HPLC reverse phase column (C18, 10 mmol/L aqueous NH₄HCO₃, acetonitrile) to obtain the product trans-4-(3,4-dihydroisoquinolin-2(1*H*)-yl)-3-hydroxypiperidin-1-yl)(6-((tetrahydro-2H-pyran-4-yl)amino)pyrimidin-4-yl)ketone (55.2 mg, yield: 40.2 %). LCMS(ESI)[M+H]⁺ = 438.47; ¹H NMR (400 MHz, CDCl₃) δ 8.62 - 8.46 (m, 1H), 7.20 - 7.08 (m, 3H), 7.08 - 6.98 (m, 1H), 6.67 - 6.49 (m, 1H), 5.24 - 4.58 (m, 2H), 4.31 - 4.14 (m, 1H), 4.03 - 3.96 (m, 3H), 3.81 - 3.69 (m, 2H), 3.60 - 3.46 (m, 2H), 3.12 - 2.98 (m, 2H), 2.95 - 2.87 (m, 2H), 2.79 - 2.58 (m, 2H), 2.07 - 1.96 (m, 3H), 1.94 - 1.78 (m, 1H), 1.77 - 1.50 (m, 5H).

### Example 110

### Preparation of t-butyl trans-4-((6-(4-(3,4-dihydroisoquinolin-2(1H)-yl)-3-hydroxypiperidine-1-carbonyl)pyrimidin-4-yl)amino)piperidine-1-carboxylate:

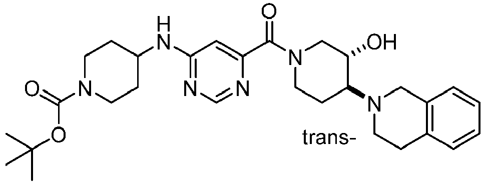

### Step I: preparation of methyl 6-((1-(t-butoxycarbonyl)piperidin-4-yl)amino) pyrimidine-4-carboxylate:

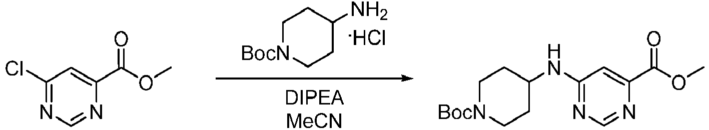

Methyl 6-chloro-pyrimidine-4-carboxylate (1 g, 5.80 mmol, 1.0 equiv.), 1-t-butoxycarbonyl-4-aminopiperidine hydrochloride (1.65 g, 6.95 mmol, 1.2 equiv.) and DIPEA (N,N-diisopropylethylamine) (3.00 g, 23.18 mmol, 4.0 equiv.) were dissolved in MeCN (acetonitrile) (30 mL), and a reaction was stirred at 90°C for 15 h. The reaction mixture was concentrated, and the crude product was separated and purified by flash chromatography (silica gel, PE: EA = 3: 2) to obtain the target compound (1.82 g, 93.4%) as a yellow solid. LCMS (ESI) [M+H]⁺ = 337.31;

### Step II: preparation of 6-((1-(t-butoxycarbonyl)piperidin-4-yl)amino)pyrimidine-4-carboxylic acid:

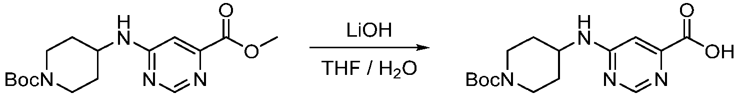

Methyl 6-((1-(t-butoxycarbonyl)piperidin-4-yl)amino)pyrimidine-4-carboxylate (1.8 g, 5.35 mmol, 1.0 equiv.) and LiOH (lithium hydroxide) (0.26 g, 10.70 mmol, 2.0 equiv.) were dissolved in THF (tetrahydrofuran) (27 mL) and H₂O (water) (9 mL), and a reaction was stirred at 20°C for 1 h. The reaction mixture was concentrated, diluted with dichloromethane/methanol (3:1) and filtrated, and the filtrate was concentrated to obtain a crude product of the target compound (2.14 g, yield: N.D.) as a yellow solid. LCMS (ESI) [M+H]⁺ = 323.2.

### Step III: preparation of t-butyl trans-4-((6-(4-(3,4-dihydroisoquinolin-2(1H)-yl)-3-hydroxypiperidine-1-carbonyl)pyrimidin-4-yl)amino)piperidine-1-carboxylate:

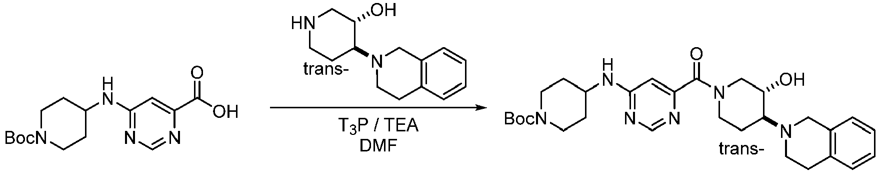

6-((1-(t-butoxycarbonyl)piperidin-4-yl)amino)pyrimidine-4-carboxylic acid (2.1 g, 6.51 mmol, 1.0 equiv.), trans-4-(3,4-dihydroisoquinolin-2(1H)-yl)piperidin-3-ol (2.09 g, 8.99 mmol, 1.38 equiv.), T₃P (1-N-propylphosphoric anhydride) in ethyl acetate (50% by mass) (8.29 g, 13.03 mmol, 2.0 equiv.) and TEA (triethylamine) (3.30 g, 32.57 mmol, 5.0 equiv.) were dissolved in DMF (N,N-dimethylformamide) (30 mL), and a reaction was stirred at 20°C for 17 h. The reaction mixture was extracted with ethyl acetate, the organic phase was concentrated, and the crude product was separated and purified by flash chromatography (silica gel, DCM: MeOH = 20: 1) to obtain a crude product of the title compound (0.98 g, 28.0%) as a yellow solid. 30 mg of the crude product of the title compound was separated and purified by Prep-HPLC (C18, 10 mmol/L aqueous NH₄HCO₃, acetonitrile) to obtain the target compound (19.4 mg). LCMS (ESI) [M+H]⁺ = 537.6; ¹H NMR (400 MHz, CDCl₃) δ 8.57 - 8.51 (m, 1H), 7.19 - 7.08 (m, 3H), 7.06 - 6.99 (m, 1H), 6.63 - 6.54 (m, 1H), 5.17 - 4.66 (m, 2H), 4.32 - 3.84 (m, 6H), 3.77 - 3.62 (m, 2H), 3.17 - 2.80 (m, 7H), 2.79 - 2.54 (m, 2H), 2.08 - 1.81 (m, 3H), 1.76 - 1.65 (m, 1H), 1.47 (s, 9H), 1.44 - 1.35 (m, 2H).

### Example 111

### Preparation of trans-1-(4-((6-(4-(3,4-dihydroisoquinolin-2(1H)-yl)-3-hydroxypiperidine-1-carbonyl)-2-methylpyrimidin-4-yl)amino)piperidin-1-yl)ethan-1-one:

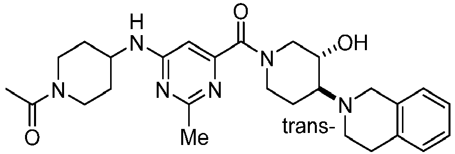

### Step I: preparation of ethyl 6-((1-acetylpiperidin-4-yl)amino)-2-methylpyrimidine-4-carboxylate:

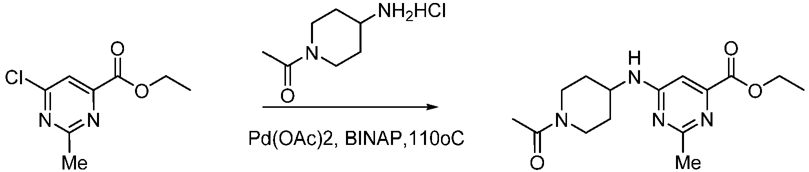

Ethyl 6-chloro-2-methylpyrimidine-4-carboxylate (200 mg, 1 mmol, 1 equiv.), 1-acetylpiperidin-4-amine hydrochloride (240 mg, 1.2 mmol, 1.2 equiv.) and palladium acetate (22.4 mg, 0.1 mmol, 0.1 equiv.), BINAP (1,1'-binaphthyl-2,2'-bis(diphenylphosphine)) (124.5 mg, 0.2 mmol, 0.2 equiv.) and cesium carbonate (814.55 mg, 2.5 mmol, 2.5 equiv.) were dissolved in 1,4-dioxane (5 ml), purged with nitrogen three times, and heated at 110°C under stirring overnight (16 h). Upon extraction with ethyl acetate three times (10 mL each), the ethyl acetate phases were combined, washed once with 10 mL water and once with 10 mL saturated sodium chloride, dried over anhydrous sodium sulfate for 10 min, and filtrated. The crude product was separated and purified on a column with DCM:MeOH=100:1 to obtain ethyl 6-((1-acetylpiperidin-4-yl)amino)-2-methylpyrimidine-4-carboxylate (130 mg, 42.6%), as a white solid. LCMS (ESI) [M+H]⁺ =307.3.

### Step II: preparation of 6-((1-acetylpiperidin-4-yl)amino)-2-methylpyrimidine-4-carboxylic acid:

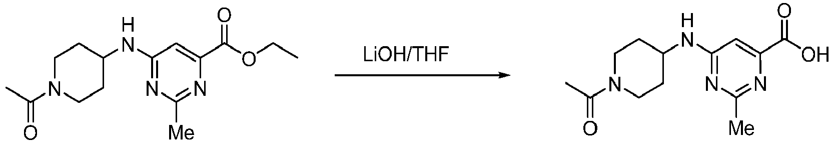

Ethyl 6-((1-acetylpiperidin-4-yl)amino)-2-methylpyrimidine-4-carboxylate (120 mg, 0.392 mmol, 1 equiv.) was dissolved in THF (4 mL), followed by addition of a LiOH (lithium hydroxide) aqueous solution (0.39 mL, 0.78 mmol, 2M (molar concentration), 2 equiv.), and the reaction mixture was stirred at room temperature (20-25°C) for 2 h. The reaction was complete as indicated by TLC. After the mixture was adjusted to PH=6-7 with 1M (molar concentration) HCl and directly rotary evaporated to dryness, to obtain a crude product of 6-((1-acetylpiperidin-4-yl)amino)-2-methylpyrimidine-4-carboxylic acid (200 mg,) as a white solid. LCMS (ESI) [M+H]⁺ =279.0.

### Step III: preparation of trans-1-(4-((6-(4-(3,4-dihydroisoquinolin-2(1H)-yl)-3-hydroxypiperidine-1 -carbonyl)-2-methylpyrimidin-4-yl)amino)piperidin-1 -yl)ethan-1 -one:

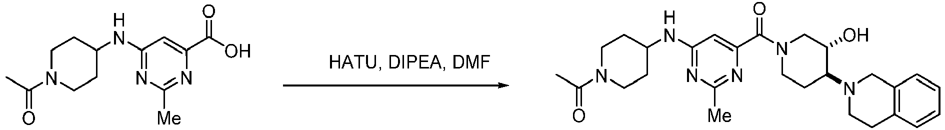

6-((1-acetylpiperidin-4-yl)amino)-2-methylpyrimidine-4-carboxylic acid (200 mg, 0.718 mmol, 1 equiv.), HATU (0.4 g, 1.08 mmol, 1.5 equiv.), trans-4-(3,4-dihydroisoquinolin-2(1H)-yl)piperidin-3-ol (200 mg, 0.862 mmol, 1.2 equiv.) and DIPEA (0.278 g, 2.16 mmol,, 3 equiv.) were dissolved in DMF (3.5 mL), and the reaction mixture was stirred at room temperature (20-25°C) for 2 h. The reaction was complete as indicated by LCMS. Upon extraction with ethyl acetate three times (10 mL each), the ethyl acetate phases were combined, washed once with 10 mL water and once with 10 mL saturated sodium chloride, dried over anhydrous sodium sulfate for 10 min, and filtrated. The crude product was separated and purified by preparative HPLC (C18, 10 mmol/L aqueous NH₄HCO₃, acetonitrile) to obtain the title compound (75.3 mg, 21.3%). LCMS (ESI) [M+H]⁺ =494.0; ¹H NMR (400 MHz, Chloroform-d) δ 7.20 - 7.09 (m, 3H), 7.06 - 6.99 (m, 1H), 6.41 - 6.37 (m, 1H), 5.19 - 4.93 (m, 1H), 4.65 (d, *J=* 13.4 Hz, 1H), 4.53 (d, *J* = 13.7 Hz, 1H), 4.22 - 4.03 (m, 2H), 3.94 (d, *J* = 14.5 Hz, 1H), 3.82 (d, *J* = 14.0 Hz, 1H), 3.71 (d, *J* = 14.9 Hz, 2H), 3.31 - 3.11 (m, 1H), 3.14 - 2.98 (m, 2H), 2.98 - 2.78 (m, 3H), 2.77 - 2.55 (m, 2H), 2.51 (d, *J* = 4.6 Hz, 3H), 2.12 (d, *J* = 1.8 Hz, 4H), 2.07 - 1.94 (m, 2H), 1.668-1.616 (m, 1H), 1.41 (q, *J=* 12.3, 11.7 Hz, 2H).

### Example 112

### Preparation of trans-(4-(3,4-dihydroisoquinolin-2(1H)-yl)-3-hydroxypiperidin-1-yl)(6-(piperidin-4-ylamino)pyrimidin-4-yl)ketone:

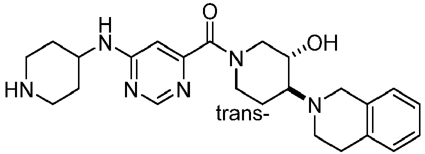

### Step I: preparation of trans-(4-(3,4-dihydroisoquinolin-2(1H)-yl)-3-hydroxypiperidin-1-yl)(6-(piperidin-4-ylamino)pyrimidin-4-yl)ketone:

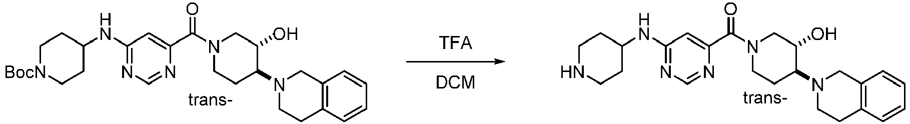

T-butyl trans-4-((6-(4-(3,4-dihydroisoquinolin-2(1*H*)-yl)-3-hydroxypiperidine-1-carbonyl)pyrimidin-4-yl)amino)piperidine-1-carboxylate (30 mg, 0.06 mmol, 1.0 equiv.) was dissolved in DCM (dichloromethane) (0.4 mL), followed by addition of TFA (trifluoroacetic acid) (0.1 mL), and a reaction was stirred at 20°C for 0.5 h. The reaction mixture was quenched with saturated aqueous solution of sodium bicarbonate, and concentrated. The crude product was separated and purified by preparative HPLC (C18, 10 mmol/L aqueous NH₄HCO₃, acetonitrile) to obtain the title compound (17.42 mg, 71.4%). LCMS (ESI) [M+H]⁺ = 437.5; ¹H NMR (400 MHz, DMSO-d₆) δ 8.46 - 8.32 (m, 1H), 7.61 (t, *J* = 7.7 Hz, 1H), 7.13 - 7.01 (m, 4H), 6.51 (s, 1H), 4.79 (dd, *J* = 35.1, 3.9 Hz, 1H), 4.40 (dd, *J* = 56.4, 12.7 Hz, 1H), 3.93 (m, 1H), 3.87 - 3.56 (m, 5H), 2.98 (d, *J* = 12.5 Hz, 2H), 2.93 - 2.87 (m, 1H), 2.85 - 2.75 (m, 4H), 2.69 - 2.53 (m, 4H), 1.92 - 1.69 (m, 3H), 1.57 - 1.43 (m, 1H), 1.41 - 1.27 (m, 2H).

### Example 113

### Preparation of 1-(4-((6-(trans-4-(3,4-dihydroisoquinolin-2(1H)-yl)-3-hydroxypiperidine-1-carbonyl)pyrimidin-4-yl)amino)piperidin-1-yl)-2-methoxyethan-1-one:

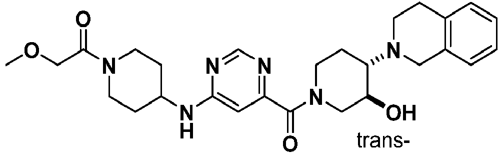

### Step I: preparation of 1-(4-((6-(trans-4-(3,4-dihydroisoquinolin-2(1H)-yl)-3-hydroxypiperidine-1-carbonyl)pyrimidin-4-yl)amino)piperidin-1-yl)-2-methoxyethan-1-one:

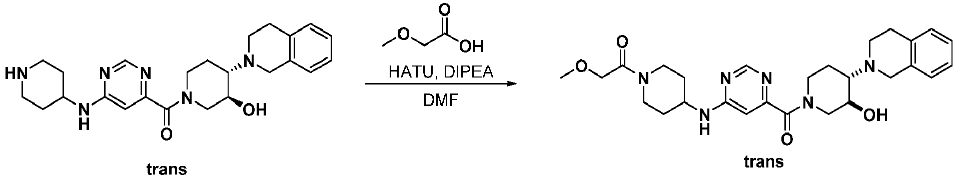

At room temperature (25°C) and under nitrogen, the raw material (trans-4-(3,4-dihydroisoquinolin-2(1*H*)-yl)-3-hydroxypiperidin-1-yl)(6-(piperidin-4-ylamino) pyrimidin-4-yl)ketone (30 mg, 0.07 mmol, 1.0 equiv.) was dissolved in DMF (*N*,*N*-dimethylformamide) (1.5 mL), followed by addition of 2-methoxyacetic acid (6 mg, 0.07 mmol, 1.0 equiv.), HATU (2-(7-azabenzotriazole)-N,N,N',N'-tetramethyluronium hexafluorophosphate) (39 mg, 0.10 mmol, 1.5 equiv.) and DIPEA (N,N-diisopropylethylamine) (27 mg, 0.20 mmol, 3.0 equiv.), and stirred at room temperature (25°C) for 4 h. After the reaction was complete as indicated by LCMS, the reaction mixture was extracted with ethyl acetate (5 mL) and concentrated. The crude product was separated and purified by preparative HPLC (C18, 10 mmol/L aqueous NH₄HCO₃, acetonitrile) to obtain the title compound (12.5 mg, 35.8%). LCMS: [M+H]⁺ =509.62; ¹H NMR (400 MHz, CDCl₃) δ 8.64 - 8.44 (m, 1H), 7.21 - 7.10 (m, 3H), 7.07 - 7.00 (m, 1H), 6.72 - 6.54 (m, 1H), 5.33 - 4.97 (m, 1H), 4.87 - 4.36 (m, 2H), 4.31 - 4.19 (m, 1H), 4.18 - 4.10 (m, 2H), 4.07 - 4.00 (m, 1H), 3.96 - 3.87 (m, 1H), 3.83 - 3.71 (m, 2H), 3.49 - 3.39 (m, 3H), 3.25 - 3.07 (m, 2H), 3.05 - 2.92 (m, 3H), 2.91 - 2.59 (m, 4H), 2.17 - 2.06 (m, 2H), 2.05 - 1.83 (m, 2H), 1.76 - 1.65 (m, 2H), 1.51 - 1.37 (m, 2H).

### Example 114

### Preparation of trans-1-(4-((6-(4-(3,4-dihydroisoquinolin-2(1H)-yl)-3-hydroxypiperidine-1-carbonyl)pyrimidin-4-yl)(methyl)amino)piperidin-1-ylethanone:

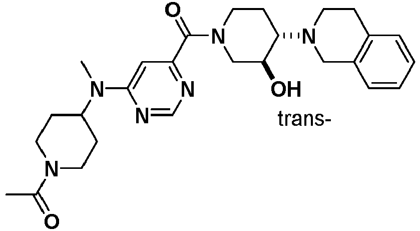

### Step I: preparation of methyl 6-((1-acetylpiperidin-4-yl) (methyl)amino)pyrimidine-4-carboxylate:

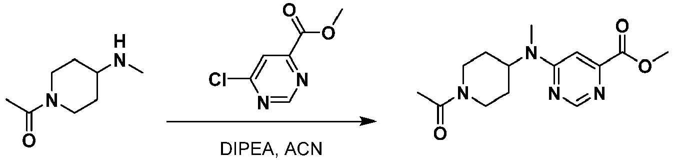

The raw material N-1-acetyl-4-methylaminopiperidine (450 mg, 2.883 mmol, 1 equiv.) and methyl 6-chloropyrimidine-4-carboxylate (597 mg, 3.459 mmol, 1.2 equiv.) was dissolved in acetonitrile (15 ml), followed by addition of DIPEA (N,N-diisopropylethylamine) (1.49 g, 11.53 mmol, 4 equiv.) at 25°C, and then stirred at 90°C for 18 h. After the reaction was complete as monitored by LCMS, the solvent was rotary evaporated, and upon separation and purification by flash chromatography (silica gel, MeOH: DCM = 0-4%) the product methyl 6-((1-acetylpiperidin-4-yl) (methyl)amino)pyrimidine-4-carboxylate (900 mg, yield: N.D.) was obtained as a light yellow oily substance. LCMS (ESI) [M+1] ⁺ = 293.3.

### Step II: preparation of 6-((1-acetylpiperidin-4-yl)(methyl)amino)pyrimidine-4-carboxylic acid:

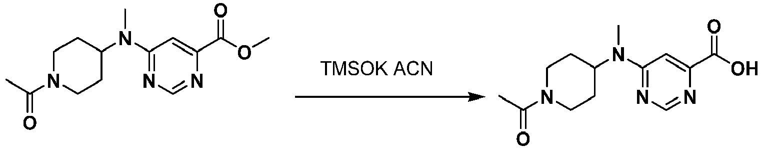

The raw material methyl 6-((1-acetylpiperidin-4-yl) (methyl)amino)pyrimidine-4-carboxylate (850 mg, 2.908 mmol, 1 equiv.) was dissolved in acetonitrile (15 ml), followed by addition of TMSOK (potassium trimethylsilanolate) (450 mg, 3.4876 mmol, 1.2 equiv.), and stirred at room temperature (25°C) for 1h. The reaction was complete as monitored by TLC. The reaction mixture was filtrated, the filter cake was collected and dissolved in water, and 1M (molar concentration) HCl was added to adjust to pH 5-6. The solution was rotary evaporated, dissolved in dichloromethane and methanol, solid impurities were removed by filtration, and the filtrate was concentrated to obtain 6-((1-acetylpiperidin-4-yl)(methyl)amino)pyrimidine-4-carboxylic acid (900 mg crude product) as a white solid, which was directly used in the following step. LCMS (ESI) [M+1] ⁺ = 279.3.

### Step III: preparation of trans-1-(4-((6-(4-(3,4-dihydroisoquinolin-2(1H)-yl)-3-hydroxypiperidine-1-carbonyl)pyrimidin-4-yl)(methyl)amino)piperidin-1-ylethanone:

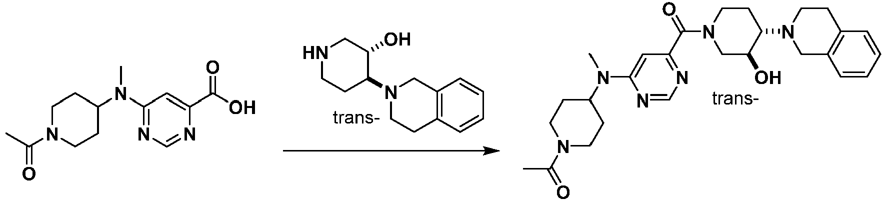

The reactant 6-((1-acetylpiperidin-4-yl)(methyl)amino)pyrimidine-4-carboxylic acid (100 mg, 0.359 mmol, 1 equiv.) was dissolved in DMF (N,N-dimethylformamide) (2 ml), followed by addition of HATU (2-(7-azabenzotriazole)-N,N,N',N'-tetramethyluronium hexafluorophosphate) (205 mg, 0.539 mmol, 1.5 equiv.) and DIPEA (N,N-diisopropylethylamine) (140 mg, 1.077 mmol, 3 equiv.), and stirred for 5 min. Then trans-4-(3,4-dihydroisoquinolin-2(1H)-yl)piperidin-3-ol (100 mg, 0.4308 mmol, 1.2 equiv.) was added. The reaction mixture was stirred at room temperature (25°C) for 1h. The reaction was monitored by LCMS until the raw materials disappeared. The reaction mixture was quenched by adding water, extracted several times with dichloromethane: methanol=10: 1, and then concentrated to obtain a crude product. The crude product was subjected to preparative TLC purification (1 mm, DCM:MeOH=10:1, TIV:Acetone=1:1) twice, and then purified with a preparative HPLC reverse phase column (C18, 10 mmol/L aqueous NH₄HCO₃, acetonitrile) to obtain the product (60 mg, 100% purity, yield: 33.9%). LCMS (ESI) [M+1] ⁺ = 493.3; ¹H NMR (400 MHz, DMSO-d₆) δ 8.51 (dd, J = 9.5, 1.1 Hz, 1H), 7.11-7.06 (m, 3H), 7.05-7.01 (m, 1H), 6.80-6.65 (m, 1H), 4.88-4.69 (m, 1H), 4.44-4.29 (m, 2H), 3.98- 3.80 (m, 1H), 3.79-3.74(m,2H), 3.67-3.60(m, 2H), 3.30-3.27 (m, 1H), 3.19- 3.17 (m, 1H), 3.04-2.95 (m, 1H), 2.93-2.75 (m, 8H), 2.69-2.56 (m, 2H), 2.03 (s, 3H), 1.90 -1.67 (m, 2H), 1.67-1.45 (m, 4H).

### Example 115

### Preparation of trans-1-(4-(2-((6-(4-(3,4-dihydroisoquinolin-2(1H)-yl)-3-hydroxypiperidine-1-carbonyl)pyrimidin-4- yl)amino)ethyl)piperidin-1-yl)ethanone:

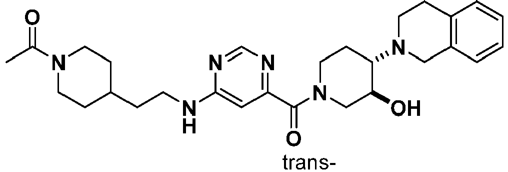

### Step I: preparation of t-butyl ((2-acetylpiperidin-4-yl)ethyl)carbamate:

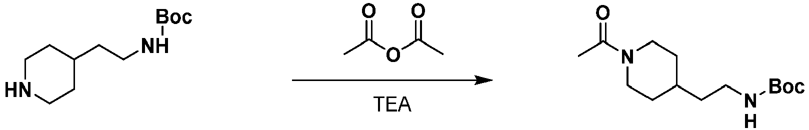

The raw material t-butyl N-(4-piperidinylethyl)carbamate (500 mg, 2.19 mmol, 1 equiv.) was dissolved in dichloromethane (15 ml), and TEA (triethylamine) (670 mg, 6.57 mmol, 3 equiv.) and acetic anhydride (223 mg, 2.19 mmol, 1 equiv.) were added at 25°C. A reaction was stirred at room temperature (25°C) overnight and quenched by addition of water. After extraction with dichloromethane, the organic phase was dried and concentrated to obtain a crude product of t-butyl ((2-acetylpiperidin-4-yl)ethyl)carbamate (650 mg, yield not calculated for the crude product) as a light yellow oily substance. LCMS (ESI) [M+1]⁺ = 271.3.

### Step II: preparation of 1-(4-(2-aminoethyl)piperidin-1-yl)ethanone trifluoroacetate:

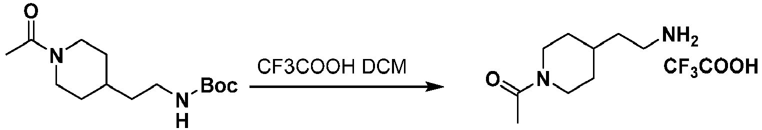

The raw material t-butyl ((2-acetylpiperidin-4-yl)ethyl)carbamate (600 mg crude product, 2.219 mmol, 1 equiv.) was dissolved in dichloromethane (9 ml), followed by addition of trifluoroacetic acid (3 ml), and a reaction was stirred under stirring at room temperature (25°C) for 1 h. The reaction was complete as monitored by TLC. The raction mixture was directly concentrated to obtain the product 1-(4-(2-aminoethyl)piperidin-1-yl)ethanone trifluoroacetate (700 mg crude product) as a white solid. LCMS (ESI) [M+1] ⁺ = 171.2.

### Step III: preparation of methyl 6-((2-(1-acetylpiperidin-4-yl)ethyl)amino)pyrimidine-4-carboxylate:

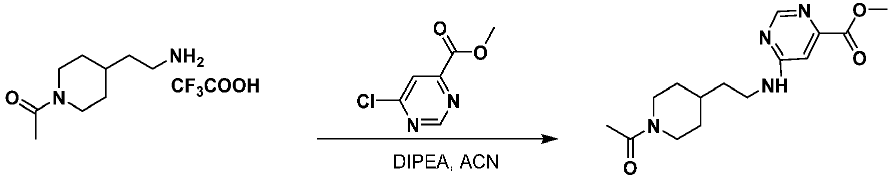

1-(4-(2-aminoethyl)piperidin-1-yl)ethanone trifluoroacetate (650 mg crude product, 2.288 mmol, 1 equiv.) and methyl 6-chloropyrimidine-4-carboxylate (474 mg, 2.746 mmol, 1.2 equiv.) were dissolved in acetonitrile (12 ml), followed by addition of DIPEA (N,N-diisopropylethylamine) (1.18 g, 9.154 mmol, 4 equiv.), and stirred at 90°C for 18 h. After the reaction was complete as monitored by LCMS, the solvent was rotary evaporated, and the resultant was separated and purified by flash chromatography (silica gel, MeOH: DCM = 0-4%) to obtain the product methyl 6-((2-(1-acetylpiperidin-4-yl)ethyl)amino)pyrimidine-4-carboxylate (1 g, yield: N.D.) as a light yellow oily substance. LCMS (ESI) [M+1] ⁺ = 307.2;

### Step IV: preparation of 6-((2-(1-acetylpiperidin-4-yl)ethyl)amino)pyrimidine-4-carboxylic acid:

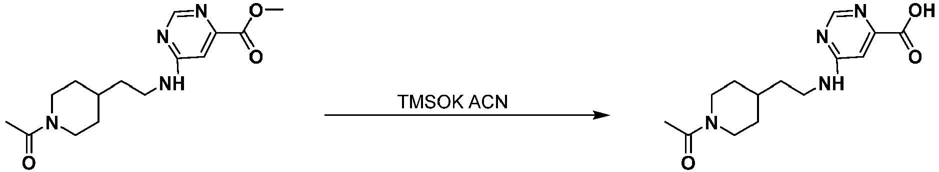

The raw material methyl 6-((2-(1-acetylpiperidin-4-yl)ethyl)amino)pyrimidine-4-carboxylate (900 mg, 2.928 mmol, 1 equiv.) was dissolved in acetonitrile (15 ml), followed by addition of TMSOK (potassium trimethylsilanolate) (450 mg, 3.514 mmol, 1.2 equiv.), and stirred at room temperature (25°C) for 1 h. The reaction was complete as monitored by TLC. The reaction mixture was filtrated, the filter cacke was collected and dissolved in water, and 1M (molar concentration) hydrochloride acid was added to adjust to pH 5-6. The solution was rotary evaporated, dissolved in dichloromethane and methanol, and filtrated to remove solid impurities. The filtrate was concentrated to obtain 6-((2-(1-acetylpiperidin-4-yl)ethyl)amino)pyrimidine-4-carboxylic acid (580 mg crude product) as a light yellow solid, which was directly used in the following step.

### Step V: preparation of trans-1-(4-(2-((6-(4-(3,4-dihydroisoquinolin-2(1H)-yl)-3-hydroxypiperidine-1-carbonyl)pyrimidin-4-yl)amino)ethyl)piperidin-1-yl)ethanone:

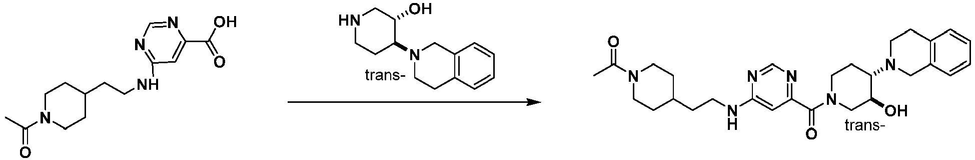

The reactant 6-((2-(1-acetylpiperidin-4-yl)ethyl)amino)pyrimidine-4-carboxylic acid (100 mg, 0.341 mmol, 1 equiv.) was dissolved in DMF (N,N-dimethylformamide) (2 ml), followed by addition of HATU (2-(7-azabenzotriazole)-N,N,N',N'-tetramethyluronium hexafluorophosphate) (195 mg, 0.515 mmol, 1.5 equiv.) and DIPEA (N,N-diisopropylethylamine) (130 mg, 1.023 mmol, 3 equiv.), and stirred for 5 minutes. Then trans-4-(3,4-dihydroisoquinolin-2(1H)-yl)piperidin-3-ol (96 mg, 0.4092 mmol, 1.2 equiv.) was added, and a reaction was stirred under stirring at room temperature (20-25°C) for 1 h. The reaction was monitored by LCMS until the raw materials disappeared. The reaction mixture was quenched by adding water, extracted several times with dichloromethane: methanol=10: 1, and then concentrated to obtain a crude product. The crude product was subjected to purification with preparative TLC (1 mm, DCM:MeOH=10:1, TIV: Acetone= 1: 1) twice, and then purified with a preparative HPLC reverse phase column (C18, 10 mmol/L aqueous NH₄HCO₃, acetonitrile) to obtain the title compound (52 mg, yield: 30%). LCMS (ESI) [M+1] ⁺ = 507.8; ¹H NMR (400 MHz, DMSO-d₆) δ 8.51-8.47 (m,, 1H), 7.71-7.63 (m, 1H), 7.11 - 7.01 (m, 4H), 6.59-6.53 (m, 1H), 4.76 (dd, J = 35.9, 3.8 Hz, 1H), 4.56-4.41 (m, 2H), 3.89- 3.55 (m, 5H), 3.37-3.36 (m, 1H), 3.04-2.88 (m, 3H), 2.86-2.76 (m, 4H), 2.69-2.53 (m, 2H), 1.97 (s, 3H), 1.84-1.70 (m, 3H), 1.62-1.51(m, 1H), 1.50-1.45 (m, 3H),1.08-1.01 (m, 1H), 0.98-0.94 (m, 1H).

### Example 116

### Preparation of trans-(6-((1-(cyclopropanecarbonyl)piperidin-4-yl)amino)pyrimidin-4-yl)(4-(3,4-dihydroisoquinolin-2(1H)-yl)-3-hydroxypiperidin-1-yl)ketone:

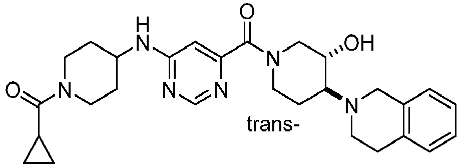

### Step I: preparation of trans-(6-((1-(cyclopropanecarbonyl)piperidin-4-yl)amino)pyrimidin-4-yl)(4-(3,4-dihydroisoquinolin-2(1H)-yl)-3-hydroxypiperidin-1-yl)ketone:

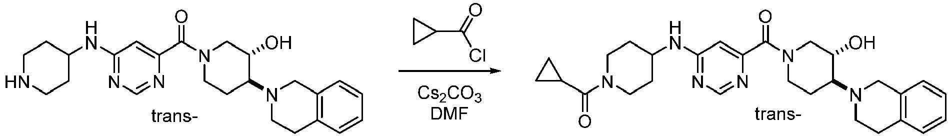

Trans-(4-(3,4-dihydroisoquinolin-2(1*H*)-yl)-3-hydroxypiperidin-1-yl)(6-(piperidin-4-ylamino )pyrimidin-4-yl)ketone (150 mg, 0.07 mmol, 1.0 equiv.) was dissolved in DMF (N,N-dimethylformamide) (1.5 mL), followed by addition of Cs₂CO₃ (cesium carbonate) (45 mg, 0.14 mmol, 2.0 equiv.) and cyclopropylformyl chloride (7 mg, 0.07 mmol, 1.0 equiv.), and a reaction was stirred at 20°C for 0.5 h. The reaction mixture was filtrated, the filtrate was concentrated, and the crud product was separated and purified by preparative HPLC (C18, 10 mmol/L aqueous NH₄HCO₃, acetonitrile) to obtain the target compound (22.07 mg, 63.6%). LCMS (ESI) [M+H]⁺ = 505.6; ¹H NMR (400 MHz, DMSO-d₆) δ 8.49 - 8.38 (m, 1H), 7.70 - 7.61 (m, 1H), 7.12 - 7.01 (m, 4H), 6.53 (s, 1H), 4.86 - 4.72 (m, 1H), 4.53 - 4.05 (m, 4H), 3.89 - 3.56 (m, 4H), 3.31 - 3.20 (m, 1H), 3.08 - 2.72 (m, 7H), 2.68 - 2.55 (m, 1H), 2.07 - 1.69 (m, 4H), 1.56 - 1.19 (m, 3H), 0.81 - 0.63 (m, 4H).

### Example 117

### Preparation of trans-1-(4-((2-chloro-6-(4-(3,4-dihydroisoquinolin-2(1H)-yl)-3-hydroxypiperidine-1-carbonyl)pyrimidin-4-yl)amino)piperidin-1-yl)ethan-1-one:

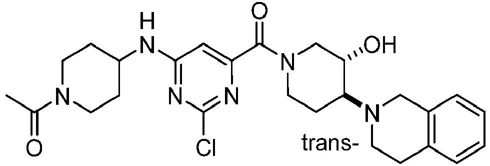

### Step I: preparation of 6-((1-acetylpiperidin-4-yl)amino)-2-chloropyrimidine-4-carboxylic acid:

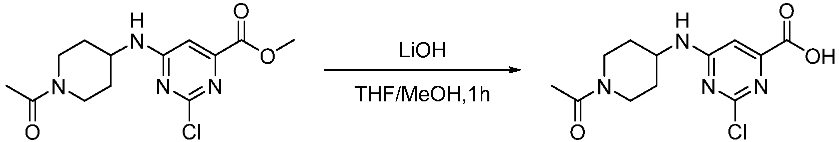

At room temperature (25°C), the raw material methyl 6-((1-acetylpiperidin-4-yl)amino)-2-chloropyrimidine-4-carboxylate (500 mg, 1.60 mmol, 1.0 equiv.) was added to water (1.6 mL), tetrahydrofuran (3.0 mL) and methanol (3.0 mL), followed by addition of lithium hydroxide (76.6 mg, 3.20 mmol, 2.0 equiv.), and stirred at room temperature (25-30°C) for 1h. After the reaction was complete as indicated by LCMS, the reaction mixture was concentrated to remove the organic solvents, and adjusted to pH = 3 with 1M (molar concentration) HCl. The mixture was extracted three times with dichloromethane:methanol (V/V= 10/1) (30 mL×5), and the organic phases were combined, dried, filtrated and concentrated to obtain a crude product of 6-((1-acetylpiperidin-4-yl)amino)-2-chloropyrimidine-4-carboxylic acid (1.5 g, crude) as a yellow solid. LCMS (ESI) [M+H]⁺= 299.2.

### Step II: preparation of trans-1-(4-((2-chloro-6-(4-(3,4-dihydroisoquinolin-2(1H)-yl)-3-hydroxypiperidine-1-carbonyl)pyrimidin-4-yl)amino)piperidin-1-yl)ethan-1-one:

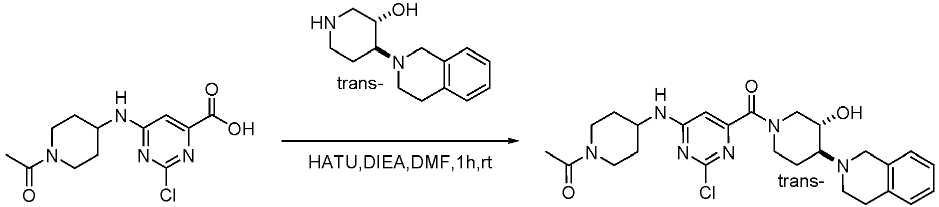

The raw material 6-((1-acetylpiperidin-4-yl)amino)-2-chloropyrimidine-4-carboxylic acid (1.4 g, 7.34 mmol, 1.0 equiv.) was added into DMF (N,N-dimethylformamide) (10 mL), followed by addition of HATU (CAS: 148893-10-1) (1.78 g, 4.69 mmol, 1.0 equiv.) and N,N-diisopropylethylamine (1.82 g, 14.06 mmol, 3.0 equiv.), and stirred at room temperature (25°C) for 2h. After the reaction was complete as indicated by LCMS, solvent was removed from the mixture, dichloromethane was added, and solid was precipitated. Upon filtration and drying, 4 g crude product was obtained. 180 mg of the crude product was purified by preparative HPLC (C18, 10 mmol/L aqueous NH₄HCO₃, acetonitrile), and lyophilized to obtain the compound trans-1-(4-((2-chloro-6-(4-(3,4-dihydro-isoquinolin-2(1H)-yl)-3-hydroxypiperidine-1-carbonyl)pyrimidin-4-yl)amino)piperidin-1-yl)-ethan-1-one (18 mg). LCMS (ESI) [M+H]+ = 513.5; ¹H NMR (400 MHz, DMSO-d₆) *δ* 8.28-8.12 (m, 1H), 7.10-7.02 (m, 4H), 6.78-6.47 (m, 1H), 4.83-4.74 (m, 1H), 4.46-4.21 (m, 2H), 4.05 (s, 1H), 3.86-3.77 (m, 3H), 3.72-3.58 (m, 2H), 3.23-3.17 (m, 1H), 3.03 (t, *J=* 12.0 Hz, 1H), 2.91-2.78 (m, 6H), 2.68-2.58 (m, 1H), 2.01 (s, 3H), 1.94-1.73 (m, 3H), 1.55-1.34 (m, 2H), 1.31-1.22 (m, 1H).

### Example 118

### Preparation of trans-1-(4-((6-(4-(3,4-dihydroisoquinolin-2(1H)-yl)-3-hydroxypiperidine-1-carbonyl)-2-(dimethylamino)pyrimidin-4-yl)amino)piperidin-1-yl)ethan-1-one:

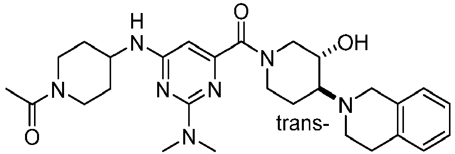

### Step I: preparation of trans-1-(4-((6-(4-(3,4-dihydroisoquinolin-2(1H)-yl)-3-hydroxypiperidine-1-carbonyl)-2-(dimethylamino)pyrimidin-4-yl)amino)piperidin-1-yl)ethan -1-one:

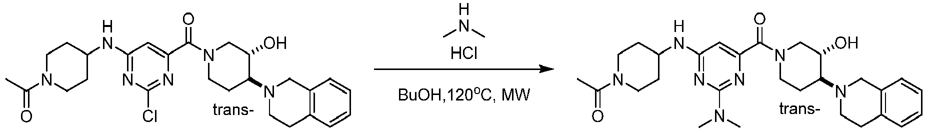

The raw material trans-1-(4-((2-chloro-6-(4-(3,4-dihydroisoquinolin-2(1*H*)-yl)-3-hydroxypiperidine-1-carbonyl)pyrimidin-4-yl)amino)piperidin-1-yl)ethan-1-one (2 g, 3.9 mmol, 1 equiv.) was added to n-butanol (10 mL), followed by addition of dimethylamine hydrochloride (3.5 g, 42.92 mmol, 11 equiv.), and stirred under microwave at 120°C for 1h. After the reaction was complete as indicated by LCMS, the crude product was separated and purified by preparative HPLC (C18, 10 mmol/L aqueous NH₄HCO₃, acetonitrile), and lyophilized to obtain the title compound. (7.16 mg, yield: 0.4%). LCMS (ESI) [M+H]+ = 522.6; ¹H NMR (400 MHz, CD₃OD) *δ* 7.11-7.08 (m, 3H), 7.06-7.03 (m, 1H), 5.83 (d, *J=* 8.0 Hz, 1H), 4.76-4.52 (m, 3H), 4.38 (d, *J* = 13.6 Hz, 1H), 4.07-3.76 (m, 5H), 3.13-3.11 (m, 6H), 3.07-2.64 (m, 8H), 2.12-2.11 (m, 4H), 2.04-1.87 (m, 2H), 1.72-1.65 (m, 1H), 1.51-1.40 (m, 2H).

### Example 119

### Preparation of trans-1-(4-((6-(4-(3,4-dihydroisoquinolin-2(1H)-yl)-3-hydroxypiperidine-1-carbonyl)pyrimidin-4-yl)thio)piperidin-1-yl)ethan-1-one

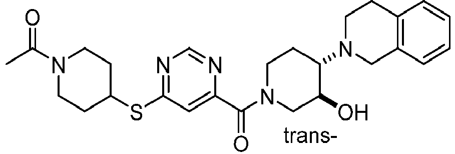

### Step I: preparation of methyl 6-((1-(t-butoxycarbonyl)piperidin-4-yl)thio)pyrimidine-4-carboxylate:

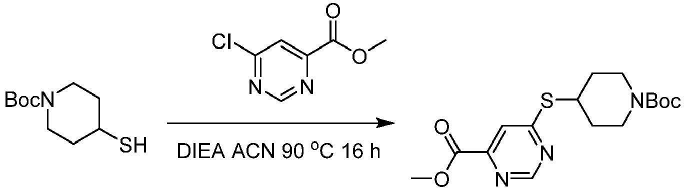

The compound t-butyl 4-mercaptopiperidine-1-carboxylate (300 mg, 1.38 mmol, 1.0 equiv.) and methyl 6-chloropyrimidine-4-carboxylate (285.8 mg, 1.66 mmol, 1.2 equiv.) were dissolved in acetonitrile (7.0 mL), purged three times with nitrogen, followed by addition of DIEA (N,N-diisopropylethylamine) (535 mg, 4.14 mmol, 3.0 equiv.). The reaction mixture was stirred at 90°C for 16 h. The reaction was complete as indicated by TLC. The reaction mixture was mixed with water (30 mL), and extracted three times with ethyl acetate (3×20 mL). The organic phases were combined, washed once with saturated sodium chloride (20 mL), dried over anhydrous sodium sulfate, filtrated, concentrated, and subjected to purification by column chromatography (PE:EA=70:30) to obtain 430 mg methyl 6-((1-(t-butoxycarbonyl)piperidin-4-yl)thio)pyrimidine-4-carboxylate as a gray solid, yield: 88.3%. LCMS(ESI): m/z=354.29; ¹H NMR (400 MHz, ) δ 9.10 (d, J = 1.3 Hz, 1H), 7.88 (d, J = 1.3 Hz, 1H), 4.21 - 4.04 (m, 1H), 3.89 (s, 3H), 3.86-3.76 (m, 2H), 3.07 (s, 2H), 2.10-1.97 (m, 2H), 1.61 - 1.48 (m, 2H), 1.40 (s, 9H).

### Step II: preparation of methyl 6-(piperidin-4-ylthio)pyrimidine-4-carboxylate:

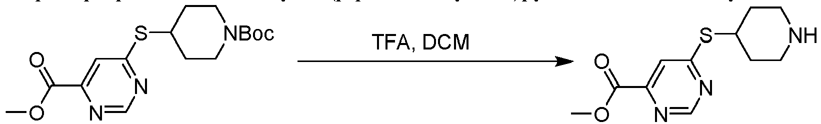

The compound methyl 6-((1-(t-butoxycarbonyl)piperidin-4-yl)thio)pyrimidine-4-carboxylate (405 mg, 1.15 mmol, 1.0 equiv.) was dissolved in dichloromethane (6.0 mL), followed by addition of trifluoroacetic acid (1.5 mL), and stirred at 16°C for 1 h. The reaction was complete as indicated by TLC, and the solvent was rotary evaporated to obtain 280 mg yellow oily methyl 6-(piperidin-4-ylthio)pyrimidine-4-carboxylate, yield: 96.5%. LCMS(ESI): m/z=254.16; ¹H NMR (400 MHz, DMSO) δ 9.11 (d, J = 1.2 Hz, 1H), 7.93 (d, J = 1.3 Hz, 1H), 4.24-4.13 (m, 1H), 3.90 (s, 3H), 3.37-3.27 (m, 2H), 3.19-3.06 (m, 2H), 2.29-2.18 (m, 2H), 1.88-1.77 (m, 2H).

### Step III: preparation of methyl 6-((1-acetylpiperidin-4-yl)thio)pyrimidine-4-carboxylate:

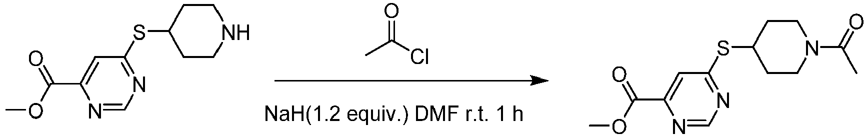

The compound methyl 6-(piperidin-4-ylthio)pyrimidine-4-carboxylate (300 mg, 1.18 mmol, 1 equiv.) was added dropwise to a solution of sodium hydride (56.8 mg, 1.42 mmol, 1.2 equiv., 60%) in N,N-dimethylformamide (6 mL), and stirred on an ice bath for 0.5 h. At this temperature, acetyl chloride (111.5 mg, 1.42 mmol, 1.2 equiv.) was added, and the stirring was continued at 16°C for 0.5 h. The reaction was complete as indicated by TLC. On an ice bath, saturated ammonium chloride (1 mL) was slowly added into the reaction mixture, which was extracted three times with ethyl acetate (3×20 mL). The organic phases were combined, washed once with saturated sodium chloride (20 mL), dried over anhydrous sodium sulfate, filtrated, concentrated, and purified by column chromatography (PE:EA=70:30) to obtain 45 mg methyl 6-((1-acetylpiperidin-4-yl)thio)-pyrimidine-4-carboxylate as a light yellow solid, yield: (12.9%). LCMS(ESI): m/z=296.15.

### Step IV: preparation of 6-((1-acetylpiperidin-4-yl)thio)pyrimidine-4-carboxylic acid:

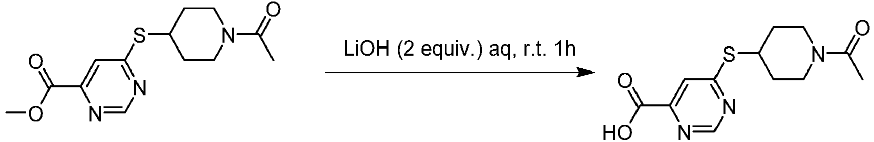

The compound methyl 6-((1-acetylpiperidin-4-yl)thio)pyrimidine-4-carboxylate (45 mg, 0.152 mmol, 1.0 equiv.) was dissolved in tetrahydrofuran (0.1 mL), followed by dropwise addition of lithium hydroxide (7.3 mg, 0.304 mmol, 2.0 equiv., prepared into a 1 M (molar concentration) aqueous solution with 0.3 mL H₂O), and stirred at 16°C for 1 h. The reaction was complete as indicated by TLC, adjusted to about pH=5 with 1 M (molar concentration) HCl, and extracted three times with ethyl acetate (3×20 mL). The organic phases were combined, washed once with saturated sodium chloride (20 mL), dried over anhydrous sodium sulfate, filtrated, and concentrated to obtain 35 mg 6-((1-acetylpiperidin-4-yl)thio)pyrimidine-4-carboxylic acid as a light yellow solid, yield: (81.9%). LCMS(ESI): m/z=282.2.

### Step V: preparation of trans-1-(4-((6-4-(3,4-dihydroisoquinolin-2(1H)-yl)-3-hydroxypiperidine-1-carbonyl)pyrimidin-4-yl)thio)piperidin-1-yl)ethan-1-one:

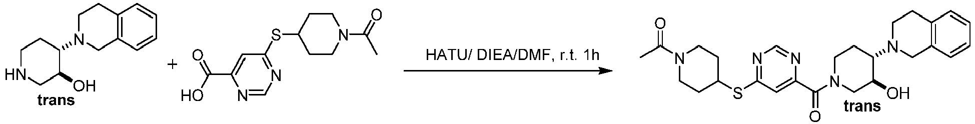

The compounds 6-((1-acetylpiperidin-4-yl)thio)pyrimidine-4-carboxylic acid (35 mg, 0.124 mmol, 1.0 equiv.), trans-4-(3,4-dihydroisoquinolin-2(1H)-yl)piperidin-3-ol (28.8 mg, 0.124 mmol, 1.0 equiv.) and HATU (2-(7-azabenzotriazol-1-yl)-*N*,*N*,*N*'*,N*'-tetramethylurea hexafluorophosphate) (70.7 mg, 0.186 mmol, 1.5 equiv.) were added to DMF (N,N-dimethylformamide) (0.4 mL), followed by addition of DIEA (N,N-diisopropylethylamine) (48 mg, 0.372 mmol, 3.0 equiv.), and stirred at 16°C for 1 h. The reaction was complete as indicated by TLC. The reaction mixture was mixed with water (15 mL), extracted three times with ethyl acetate (3×15 mL), and the organic phases were combined, washed once with saturated sodium chloride (20 mL), dried over anhydrous sodium sulfate, filtrated, concentrated, and separated and purified by preparative HPLC (C18, 10 mmol/L aqueous NH₄HCO₃, acetonitrile) to obtain 4.7 mg of the title compound, yield: 7.7 %. LCMS(ESI): m/z=496.5; ¹H NMR (400 MHz, CDCl₃) δ 8.91 (dd, J = 6.8, 1.3 Hz, 1H), 7.41-7.33 (m, 1H), 7.18-7.01 (m, 4H), 5.08-4.73 (m, 1H), 4.37-4.27 (m, 1H), 4.26-4.04 (m, 2H), 4.02-3.91 (m, 1H), 3.85-3.66 (m, 4H), 3.41-3.29 (m, 1H), 3.17-2.91 (m, 5H), 2.85-2.61 (m, 3H), 2.22-2.09 (m, 5H), 2.04-1.95 (m, 1H), 1.72-1.68 (m, 3H).

### Example 120

### 1-(4-((6-(trans-4-(3,4-dihydroisoquinolin-2(1H)-yl)-3-hydroxypiperidine-1-carbonyl)-2-methoxypyrimidin-4-yl)amino)piperidin-1-yl)ethan-1-one

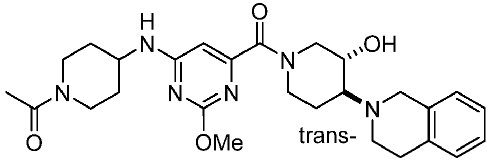

### Step I: preparation of methyl 6-((1-acetylpiperidin-4-yl)amino)-2-chloropyrimidine-4-carboxylate:

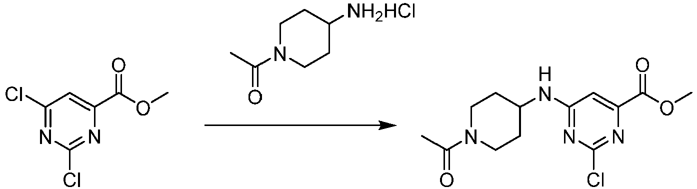

Methyl 2,6-dichloropyrimidine-4-carboxylate (2 g, 9.66 mmol), 1-acetylpiperidin-4-amine hydrochloride (1.9 g, 10.63 mmol) and N,N-diisopropylethylamine (4.99 g, 38.65 mmol) were dissolved in acetonitrile (50 ml), and stirred at 25°C (2 h). The reaction mixture was extracted three times with ethyl acetate (50 mL each), and the ethyl acetate phases were combined, washed once with 20 mL water and once with 20 mL saturated sodium chloride, dried over anhydrous sodium sulfate for 10 min, and filtrated. The crude product was separated and purified on a column with DCM:MeOH=100:1 to obtain methyl 6-((1-acetylpiperidin-4-yl)amino)-2-chloropyrimidine-4-carboxylate (2.8 g, 92%) as a white solid. LCMS (ESI) [M+H]⁺ =313.2.

### Step II: preparation of 6-((1-acetylpiperidin-4-yl)amino)-2-chloropyrimidine-4-carboxylic acid:

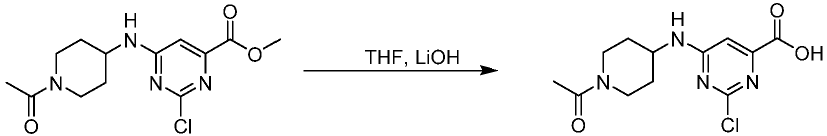

Methyl 6-((1-acetylpiperidin-4-yl)amino)-2-methylpyrimidine-4-carboxylate (200 mg, 0.639 mmol) was dissolved in THF (4 mL), followed by addition of 2M (molar concentration) aqueous lithium hydroxide (0.64 mL, 1.28 mmol, 2 equiv.), and the reaction mixture was stirred at room temperature (25°C) for 2 h. The reaction was complete as indicated by TLC. After 1M (molar concentration) HCl was added to adjust to PH=6-7, the mixture was directly rotary evaporated to obtain a crude product of 6-((1-acetylpiperidin-4-yl)amino)-2-chloropyrimidine-4-carboxylic acid (400 mg) as a white solid. LCMS (ESI) [M+H]⁺ =299.2.

### Step III: preparation of 1-(4-((2-((3H-[1,2,3]triazolo[4,5-b]pyridin-3-yl)oxy)-6-(trans-4-(3,4-dihydroisoquinolin-2(1H)-yl)-3-hydroxypiperidine-1-carbonyl)pyrimidin-4-yl)amino)-piperidin-1 -yl)ethan-1 -one:

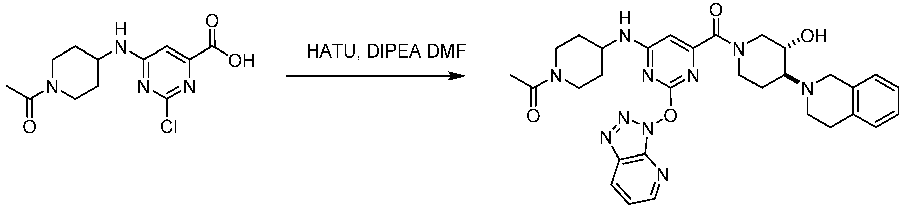

6-((1-acetylpiperidin-4-yl)amino)-2-chloropyrimidine-4-carboxylic acid (380 mg, 1.27 mmol), HATU (2-(7-azabenzotriazol-1-yl)-*N*,*N*,*N'*,*N'*-tetramethylurea hexafluorophosphate) (725.5 mg, 1.91 mmol), trans-4-(3,4-dihydroisoquinolin-2(1H)-yl)piperidin-3-ol (265 mg, 1.14 mmol) and DIPEA (N,N-diisopropylethylamine) (0.822 g, 6.36 mmol) were dissolved in DMF (N,N-dimethylformamide) (5 mL), and the reaction mixture was stirred at room temperature (25°C) for 2 h. The reaction was complete as indicated by LCMS. Upon extraction three times with ethyl acetate (20 mL each), the ethyl acetate phases were combined, washed once with 10 mL water and once with 10 mL saturated sodium chloride, dried over anhydrous sodium sulfate for 10 min, and filtrated. The crude product was separated and purified on a column with DCM:MeOH=20:1 to obtain 1-(4-((2-((3H-[1,2,3]triazolo[4,5-b]pyridin-3-yl)oxy)-6-(trans-4-(3,4-dihydroisoquinolin-2(1 H)-yl)-3 -hydroxypiperidine-1 -carbonyl)-pyrimidin-4-yl)amino)piperidin-1 -yl)ethan-1 -one (200 mg), as a white solid. LCMS (ESI) [M+H]⁺ =613.3.

### Step IV: preparation of 1-(4-((6-(trans-4-(3,4-dihydroisoquinolin-2(1H)-yl)-3-hydroxypiperidine-1 -carbonyl)-2-methoxypyrimidin-4-yl)amino)piperidin-1 -yl)ethan-1 -one:

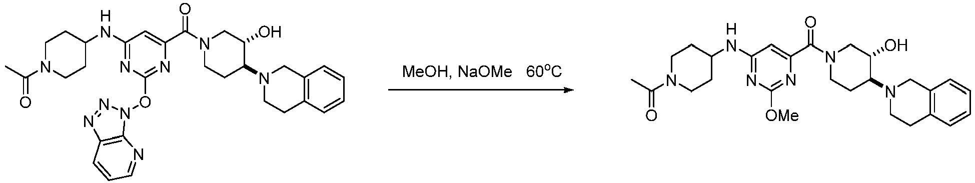

1-(4-((2-((3H-[1,2,3]triazolo[4,5-b]pyridin-3-yl)oxy)-6-(trans-4-(3,4-dihydroisoquinolin-2(1H)-yl)-3-hydroxypiperidine-1-carbonyl)pyrimidin-4-yl)amino)piperidin-1-yl)ethan-1-one (130 mg, 0.212 mmol) was dissolved in methanol (4 mL), followed by addition of sodium methoxide (114.6 mg, 0.84 mmol,), and the reaction mixture was stirred at 60°C for 4 h. The reaction was complete as indicated by LCMS. Upon extraction three times with ethyl acetate (10 mL each), the ethyl acetate phases were combined, washed once with 10 mL water and once with 10 mL saturated sodium chloride, dried over anhydrous sodium sulfate for 10 min, and filtrated. The crude product was separated and purified by preparative HPLC (C18) to obtain 1-(4-((6-(trans-4-(3,4-dihydroisoquinolin-2(1H)-yl)-3-hydroxypiperidine-1-carbonyl)-2-methoxypyrimidin-4-yl)amino)piperidin-1-yl)ethan-1-one (42.61 mg, 39%). LCMS (ESI) [M+H]+ =509.3; 1H NMR (400 MHz, CDCl₃) δ 7.16 - 7.11 (m, 3H), 7.04 - 7.02 (m, 1H), 6.28 (d, J = 18.1 Hz, 1H), 5.10 (d, J = 13.1 Hz, 0.3H), 5.03 - 4.99 (m, 1H), 4.77 (d, J = 13.2 Hz, 0.7H), 4.56 (d, J = 13.7 Hz, 1H), 4.33 - 4.18 (m, 1H), 3.95 (d, J = 10.5 Hz, 4H), 3.92-3.71 (m, 4H), 3.25 (t, J = 12.9 Hz, 1H), 3.07-3.05 (m, 1H), 2.97-2.93 (m, 3H), 2.84 (t, J = 12.7 Hz, 2H), 2.72 - 2.58 (m, 2H), 2.11-2.08 (d, 4H), 2.05 - 1.85 (m, 2H), 1.79-1.73 (m, 1H), 1.44 - 1.34 (m, 2H).

### Example 121

### Preparation of trans-(4-(3,4-dihydroisoquinolin-2(1H)-yl)-3-hydroxypiperidin-1-yl)(6-((1-(2,2,2-trifluoroethyl)piperidin-4-yl)amino)pyrimidin-4-yl)ketone:

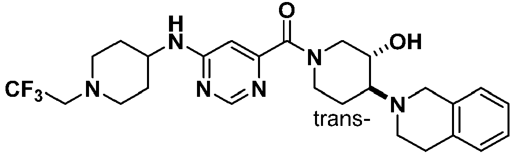

### Step I: preparation of trans-(4-(3,4-dihydroisoquinolin-2(1H)-yl)-3-hydroxypiperidin-1-yl)(6-((1-(2,2,2-trifluoroethyl)piperidin-4-yl)amino)pyrimidin-4-yl)ketone:

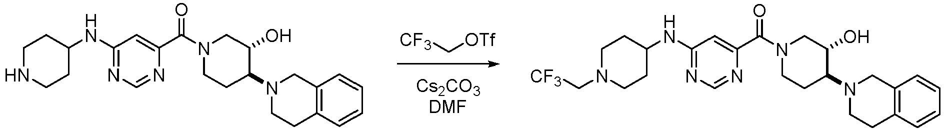

Trans-(4-(3,4-dihydroisoquinolin-2(1H)-yl)-3-hydroxypiperidin-1-yl)(6-(piperidin-4-ylamino )pyrimidin-4-yl)ketone (200 mg, 0.09 mmol, 1.0 equiv.) was dissolved in DMF (N,N-dimethylformamide) (2 mL), followed by addition of Cs₂CO₃ (cesium carbonate) (36 mg, 0.11 mmol, 1.2 equiv.) and 2,2,2-trifluoroethyl trifluoromethanesulfonate (21 mg, 0.09 mmol, 1.0 equiv.), and a reaction was stirred at 20°C for 0.5 h. The reaction mixture was filtrated, the filtrate was concentrated, and the crude product was separated and purified by preparative HPLC (C18, 0.1% aqueous formic acid, acetonitrile) to obtain a monoformate of the title compound (7.63 mg, 16.1%). LCMS (ESI) [M+H]⁺ = 519.7; ¹H NMR (400 MHz, CDCl₃) δ 8.57 - 8.46 (m, 1H), 8.17 (s, 1H), 7.22 - 7.10 (m, 3H), 7.08 - 7.01 (m, 1H), 6.68 - 6.54 (m, 1H), 5.06 - 4.66 (m, 1H), 4.26 - 4.05 (m, 2H), 3.92 - 3.83 (m, 3H), 3.20 - 3.11 (m, 1H), 3.10 - 2.81 (m, 10H), 2.71 - 2.48 (m, 2H), 2.09 - 1.87 (m, 3H), 1.82 - 1.68 (m, 1H), 1.66 - 1.54 (m, 2H).

### Example 122

### Preparation of trans-(4-(3,4-dihydroisoquinolin-2(1H)-yl)-3-hydroxypiperidin-1-yl)(6-((1-(2-hydroxyethyl)piperidin-4-yl)amino)pyrimidin-4-yl)ketone:

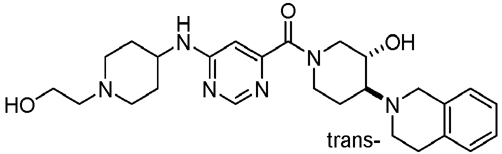

### Step I: preparation of trans-(4-(3,4-dihydroisoquinolin-2(1H)-yl)-3-hydroxypiperidin-1-yl)(6-((1-(2-hydroxyethyl)piperidin-4-yl)amino)pyrimidin-4-yl)ketone:

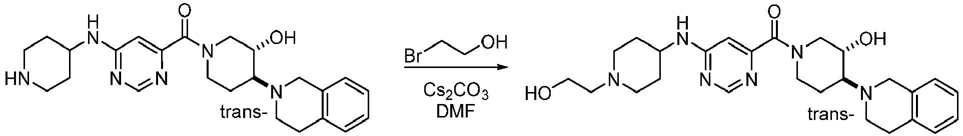

Trans-(4-(3,4-dihydroisoquinolin-2(1H)-yl)-3-hydroxypiperidin-1-yl)(6-(piperidin-4-ylamino )pyrimidin-4-yl)ketone (150 mg, 0.07 mmol, 1.0 equiv.) was dissolved in DMF (N,N-dimethylformamide) (1.5 mL), followed by addition of Cs₂CO₃ (cesium carbonate) (45 mg, 0.14 mmol, 2.0 equiv.) and 2-bromoethanol (17 mg, 0.14 mmol, 2.0 equiv.), and a reaction was stirred at 20°C for 2 days. The reaction mixture was filtrated, the filtrate was concentrated, and the crude product was separated and purified by preparative HPLC (C18, 10 mmol/L aqueous NH₄HCO₃, acetonitrile) to obtain the title compound (13.89 mg, 42.1%). LCMS (ESI) [M+H]⁺ = 481.5; ¹H NMR (400 MHz, CDCl₃) δ 8.54 - 8.50 (m, 1H), 7.17 - 7.10 (m, 3H), 7.05 - 7.01 (m, 1H), 6.68 - 6.54 (m, 1H), 5.53 - 5.28 (m, 1H), 5.08 - 4.66 (m, 1H), 4.32 - 4.10 (m, 1H), 4.00 - 3.89 (m, 2H), 3.73 - 3.68 (m, 2H), 3.65 - 3.60 (m, 2H), 3.09 - 2.83 (m, 7H), 2.74 - 2.62 (m, 3H), 2.60 - 2.53 (m, 2H), 2.32 - 2.23 (m, 2H), 2.09 - 1.97 (m, 3H), 1.74 - 1.64 (m, 1H), 1.62 - 1.50 (m, 2H).

### Example 123

### Preparation of methyl trans-4-((6-(4-(3,4-dihydroisoquinolin-2(1H)-yl)-3-hydroxypiperidine-1-carbonyl)pyrimidin-4-yl)amino)piperidine-1-carboxylate

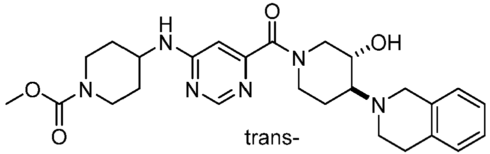

### Step I: preparation of methyl trans-4-((6-(4-(3,4-dihydroisoquinolin-2(1H)-yl)-3-hydroxypiperidine-1-carbonyl)pyrimidin-4-yl)amino)piperidine-1-carboxylate:

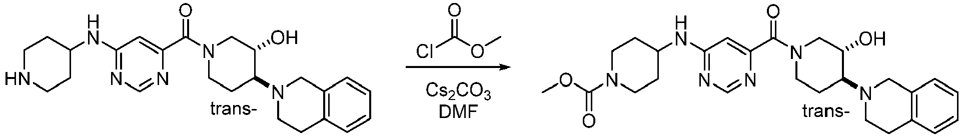

Trans-(4-(3,4-dihydroisoquinolin-2(1H)-yl)-3-hydroxypiperidin-1-yl)(6-(piperidin-4-ylamino )pyrimidin-4-yl)ketone (40 mg, 0.09 mmol, 1.0 equiv.) was dissolved in DMF (N,N-dimethylformamide) (1.5 mL), followed by addition of Cs₂CO₃ (cesium carbonate) (60 mg, 0.18 mmol, 2.0 equiv.) and methyl chloroformate (9 mg, 0.09 mmol, 1.0 equiv.), and a reaction was stirred at 20°C for 0.5 h. The reaction mixture was filtrated, the filtrate was concentrated, and the crude product was separated and purified by preparative HPLC (C18, 10 mmol/L aqueous NH₄HCO₃, acetonitrile) to obtain the target compound (26.97 mg, 59.5%). LCMS (ESI) [M+H]⁺ = 495.5; ¹H NMR (400 MHz, CDCl₃) δ 8.57 - 8.50 (m, 1H), 7.19 - 7.09 (m, 3H), 7.07 - 7.00 (m, 1H), 6.65 - 6.55 (m, 1H), 5.22 - 5.09 (m, 1H), 5.05 - 4.66 (m, 1H), 4.29 - 3.93 (m, 5H), 3.81 - 3.73 (m, 2H), 3.71 (s, 3H), 3.14 - 3.04 (m, 1H), 3.05 - 2.93 (m, 5H), 2.93 - 2.83 (m, 1H), 2.81 - 2.70 (m, 2H), 2.08 - 1.97 (m, 3H), 1.92 - 1.85 (m, 1H), 1.51 - 1.35 (m, 2H).

### Example 124

### Preparation of 1-(4-((6-((4S,SS)-4-(3,4-dihydroisoquinolin-2(1H)-yl)-5-hydroxyazepane-1-carbonyl)pyrimidin-4-yl)amino)piperidin-1-yl)ethan-1-one:

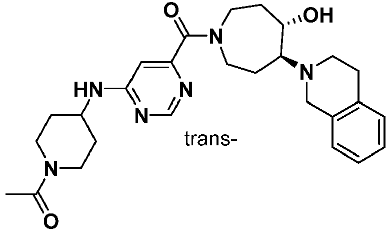

### Step I: preparation of t-butyl (1R,7S)-8-oxa-4-azabicyclo[5.1.0]octane-4-carboxylate:

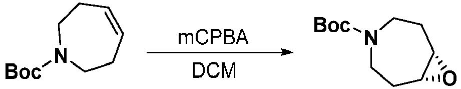

T-butyl 2,3,6,7-tetrahydro-*1H*-azepine-1-carboxylate (1 g, 5.00 mmol, 1.0 eq) was dissolved in DCM (dichloromethane) (25 mL), followed by addition of mCPBA (chloroperbenzoic acid) (1.10 g, 6.60 mmol, 1.3 eq), and stirred at 25°C for 3 h. The reaction mixture was quenched with saturated sodium sulfite, and extracted three times with dichloromethane (15 mL each). The dichloromethane phases were combined, washed once with 20 mL water and once with 20 mL saturated sodium chloride, dried over anhydrous sodium sulfate for 10 min, filtrated and concentrated to obtain t-butyl (1R,7S)-8-oxo-4-azabicyclo[5.1.0]octane-4-carboxylate (1.05 g, 97.2%) as a colorless oily substance. ¹H NMR (400 MHz, CDCl₃) δ 3.94 - 3.57 (m, 2H), 3.19 (s, 2H), 2.85 - 2.60 (m, 2H), 2.28 - 1.99 (m, 4H), 1.45 (s, 9H).

### Step II: preparation of t-butyl trans-4-(3,4-dihydroisoquinolin-2(1H)-yl)-5-hydroxyazepane-1-carboxylate:

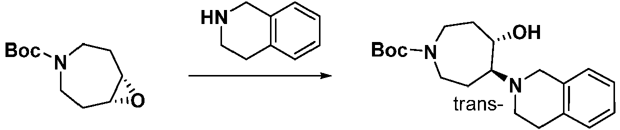

T-butyl (1R,7S)-8-oxo-4-azabicyclo[5.1.0]octane-4-carboxylate (1 g, 4.69 mmol, 1.0 eq) was dissolved in water (25 mL), followed by addition of 1,2,3,4-tetrahydroisoquinoline (811 mg, 6.10 mmol, 1.3 eq), and stirred at 100°C for 16 h. The reaction mixture was extracted three times with ethyl acetate (15 mL each), and the ethyl acetate phases were combined, washed once with 20 mL water and once with 20 mL saturated sodium chloride, dried over anhydrous sodium sulfate for 10 min, filtrated and concentrated. Upon separation and purification by flash chromatography (silica gel, DCM: MeOH = 49: 1), t-butyl trans-4-(3,4-dihydroisoquinolin-2(1*H*)-yl)-5-hydroxyazepane-1-carboxylate (1.13 g, 69.8%) was obtained as a yellow oily substance. LCMS (ESI) [M+H]⁺ =347.32; ¹H NMR (400 MHz, CDCl₃) δ 7.19 - 6.99 (m, 4H), 3.91 (d, *J =* 14.4 Hz, 1H), 3.73 - 3.49 (m, 4H), 3.45 - 3.07 (m, 2H), 3.04 - 2.85 (m, 3H), 2.65-2.45 (m, 2H), 2.29 - 2.15 (m, 1H), 2.11 - 1.94 (m, 1H), 1.59 (ddd, *J=* 18.6, 11.5, 6.2 Hz, 2H), 1.48 (s, 9H), 1.27 (d, *J=* 12.2 Hz, 1H).

### Step III: preparation of trans-5-(3,4-dihydroisoquinolin-2(1H)-yl)azepan-4-ol:

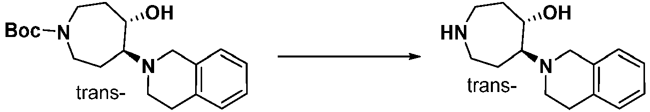

TFA (trifluoroacetic acid) (1.5 mL) was slowly added dropwise to a solution of the compound t-butyl trans-4-(3,4-dihydroisoquinolin-2(1*H*)-yl)-5-hydroxyazepane-1-carboxylate (420 mg, 1.21 mmol, 1.0 eq) in DCM (dichloromethane) (6 mL), and then stirred at room temperature (15°C) for 1 h. The reaction mixture was adjusted to an alkaline pH with saturated sodium bicarbonate, and extracted three times with dichloromethone (5 mL each). The dichloromethone phases were combined, washed once with 5 mL water and once with 5 mL saturated sodium chloride, dried over anhydrous sodium sulfate for 10 min, filtrated and concentrated to obtain a crude product of trans-5-(3,4-dihydroisoquinolin-2(1H)-yl)azepan-4-ol (280 mg, 97.1%) as a yellow oily substance. LCMS (ESI) [M+H]⁺ =247.24.

### Step IV: preparation of trans-1-(4-((6-(4-(3,4-dihydroisoquinolin-2(1H)-yl)-5-hydroxyazepane-1-carbonyl)pyrimidin-4-yl)amino)piperidin-1-yl)ethan-1-one:

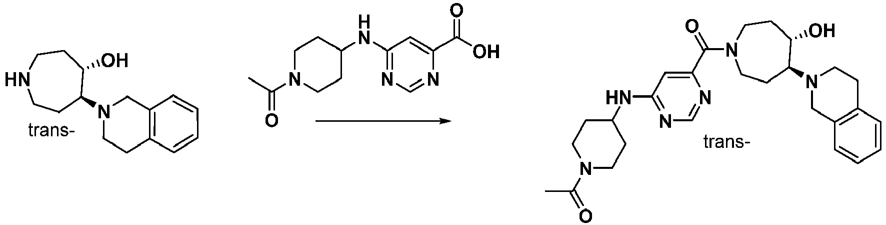

The compound trans-5-(3,4-dihydroisoquinolin-2(1H)-yl)azepan-4-ol (150 mg, 0.61 mmol, 1.0 eq), 6-((1-acetylpiperidin-4-yl)amino)pyrimidine-4-carboxylic acid (161 mg, 0.61 mmol, 1.0 eq) and HATU (2-(7-azabenzotriazole)-N,N,N',N'-tetramethyluronium hexafluorophosphate) (348 mg, 0.91 mmol, 1.5 eq) were dissolved in DMF (N,N-dimethylformamide) (3 mL), followed by addition of DIPEA (N,N-diisopropylethylamine) (236 mg, 1.83 mmol, 3.0 eq), and stirred at room temperature (10-15°C) for 1 h. The reaction mixture was poured into 10 mL water, extracted three times with ethyl acetate (6 mL each), and the ethyl acetate phases were combined, washed once with 10 mL water and once with 10 mL saturated sodium chloride, dried over anhydrous sodium sulfate for 5 min, filtrated, and concentrated. The crude product was seperatred and purified by Prep-HPLC (C18, 10 mmol/L aqueous NH₄HCO₃, acetonitrile) to obtain the target compound (14.2 mg, 4.7%). LCMS (ESI) [M+H]⁺ = 493.47; ¹H NMR (400 MHz, CDCl₃) *δ* 8.54 (d, *J=* 5.5 Hz, 1H), 7.14 (d, *J=* 6.1 Hz, 3H), 7.03 (t, *J* = 6.6 Hz, 1H), 6.58 (d, *J* = 18.3 Hz, 1H), 5.29 - 5.07 (m, 1H), 4.56 (d, *J* = 13.0 Hz, 2H), 3.93 (dt, *J* = 19.6, 10.6 Hz, 3H), 3.87 - 3.76 (m, 2H), 3.52-3.75 (m, 3H), 3.48 - 3.11 (m, 3H), 3.09 - 2.96 (m, 1H), 2.92 (s, 2H), 2.82 (t, *J* = 12.3 Hz, 1H), 2.73 - 2.53 (m, 2H), 2.40 - 2.18 (m, 1H), 2.17-2.13 (m, 1 H), 2.12 (s, 3H), 2.05 (d, *J* = 13.1 Hz, 1H), 1.87 - 1.71 (m, 2H), 1.42 (dd, *J* = 21.0, 10.3 Hz, 2H).

### Example 125

### Preparation of trans-5-(3,4-dihydroisoquinolin-2(1H)-yl)-1-(6-((3-fluorophenyl)amino)-pyrimidin-4-yl)azepan-4-ol:

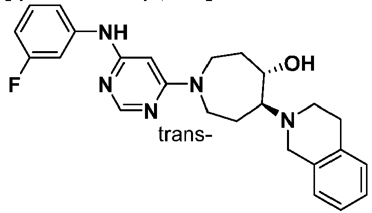

### Step I: preparation of trans-1-(6-chloropyrimidin-4-yl)-5-(3,4-dihydroisoquinolin-2(1H)-yl)-azepan-4-ol:

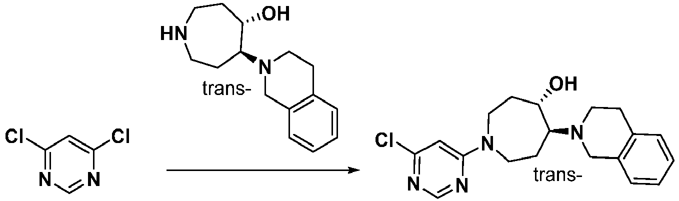

4,6-dichloropyrimidine (50 mg, 0.347 mmol, 1.0 eq) and trans-5-(3,4-dihydroisoquinolin-2(1*H*)-yl)azepan-4-ol (83 mg, 0.34 mmol, 1.0 eq) were dissolved in isopropanol (1.5 mL), followed by addition of N,N-diisopropylethylamine (88 mg, 0.68 mmol, 2.0 eq), and stirred at 100°C for 1 h. The reaction was complete as monitored by LCMS. The solvent was rotary evaporated to dryness, and upon purification by preparative HPLC (3% methanol/dichloromethane), trans-1-(6-chloropyrimidin-4-yl)-5-(3,4-dihydroisoquinolin-2(1*H*)-yl)azepan-4-ol (72 mg, 59.8%) was obtained as a white solid. LCMS (ESI) [M+H]⁺ =359.28.

### Step II: preparation of trans-5-(3,4-dihydroisoquinolin-2(1H)-yl)-1-(6-((3-fluorophenyl)-amino)-pyrimidin-4-yl)azepan-4-ol:

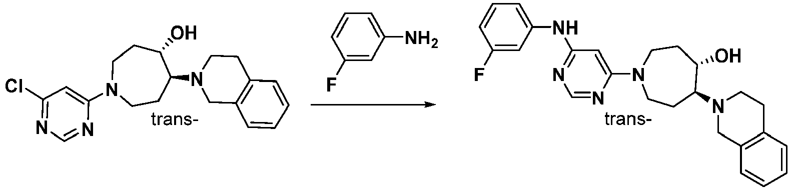

Trans-1-(6-chloropyrimidin-4-yl)-5-(3,4-dihydroisoquinolin-2(1*H*)-yl)azepan-4-ol (37 mg, 0.1 mmol, 1.0 eq) was dissolved in isopropanol (1 mL), followed by addition of 3-fluoroaniline (33 mg, 0.1mmol, 3.0 eq) and concentrated hydrochloric acid (0.1 mL), and heated at 100°C under stirring overnight (16 h). The mixture was adjusted to pH 7 with 1N aqueous sodium hydroxide, and extracted three times with ethyl acetate (5 mL each). The ethyl acetate phases were combined, washed once with 10 mL water and once with 10 mL saturated sodium chloride, dried over anhydrous sodium sulfate for 10 min, and filtrated. The crude product was separated and purified by preparative HPLC (C18, 10 mmol/L aqueous NH₄HCO₃, acetonitrile) to obtain trans-5-(3,4-dihydroisoquinolin-2(1*H*)-yl)-1-(6-((3-fluorophenyl)amino)pyrimidin-4-yl)azepan-4-ol (2.7 mg, 6.0%). LCMS (ESI) [M+H]⁺ =434.34; ¹H NMR (400 MHz, CDCl₃) *δ* 8.28 (s, 1H), 7.38 - 7.27 (m, 1H), 7.15 (d, *J* = 8.6 Hz, 4H), 7.02 (dd, J = 16.7, 8.6 Hz, 3H), 6.80 (t, J = 8.1 Hz, 1H), 5.85 (s, 1H), 3.91 (d, *J* = 14.4 Hz, 2H), 3.67 (d, J = 14.7 Hz, 1H), 3.60 (t, J = 8.2 Hz, 1H), 3.44 (s, 1H), 3.31 (t, *J* = 12.5 Hz, 1H), 2.99 (dd, *J =* 10.6, 5.3 Hz, 1H), 2.90 (s, 2H), 2.64-2.61 (m, 1H), 2.54 (t, J = 10.5 Hz, 1H), 2.34-2.31 (m, 1H), 2.14 (d, *J* = 13.2 Hz, 1H), 1.79 - 1.76 (m, 1H), 1.66-1.64 (m, 1H).

### Example 126

### Preparation of trans-5-(3,4-dihydroisoquinolin-2(1H)-yl)-1-(6-(phenylamino)-pyrimidin-4-yl)-azepan-4-ol:

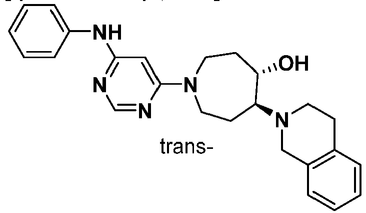

### Step I: preparation of trans-5-(3,4-dihydroisoquinolin-2(1H)-yl)-1-(6-(phenylamino)-pyrimidin-4-yl)azepan-4-ol:

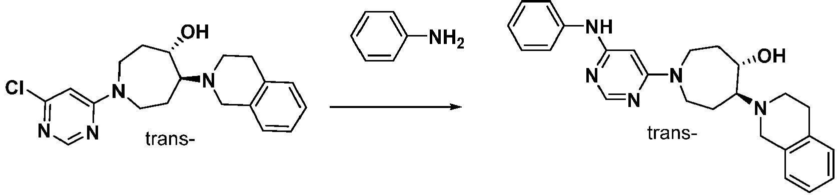

Trans-1-(6-chloropyrimidin-4-yl)-5-(3,4-dihydroisoquinolin-2(1*H*)-yl)azepan-4-ol (35 mg, 0.1 mmol, 1.0 eq) was dissolved in isopropanol (1 mL), followed by addition of aniline (28 mg, 0.1 mmol, 3.0 eq) and concentrated hydrochloric acid (0.1 mL), and heated at 100°C under stirring overnight (16 h). The mixture was adjusted to pH 7 with 1N aqueous sodium hydroxide, and extracted three times with ethyl acetate (5 mL each). The ethyl acetate phases were combined, washed once with 10 mL water and once with 10 mL saturated sodium chloride, dried over anhydrous sodium sulfate for 10 min, and filtrated. The crude product was separated and purified by Prep-HPLC (C18, 10 mmol/L aqueous NH₄HCO₃, acetonitrile) to obtain trans-5-(3,4-dihydroisoquinolin-2(1H)-yl)-1-(6-(phenylamino)pyrimidin-4-yl)-azepan-4-ol (3.2 mg, 7.9%). LCMS (ESI) [M+H]⁺ =416.36; ¹H NMR (400 MHz, CDCl₃) δ 8.27 (s, 1H), 7.38 (t, *J* = 7.5 Hz, 2H), 7.30 (d, *J* = 7.8 Hz, 2H), 7.15-7.12 (m, 4H), 7.02-7.01 (m, 1H), 6.87 (s, 1H), 5.82 (s, 1H), 4.62 (br s, 1H), 3.91 (d, *J* = 14.5 Hz, 2H), 3.67 (d, *J* = 14.4 Hz, 1H), 3.59 (t, *J* = 8.6 Hz, 1H), 3.46-3.42 (m, 1H), 3.29 (t, *J* = 12.3 Hz, 1H), 2.99 (dd, *J* = 10.6, 4.7 Hz, 1H), 2.91 (s, 2H), 2.64-2.61 (m, 1H), 2.55 (t, *J* = 9.8 Hz, 1H), 2.30 (d, *J*= 13.4 Hz, 1H), 2.13 (d, *J* = 13.1 Hz, 1H), 1.82-1.76 (m, 1H), 1.68-1.60 (m, 1H).

### Example 127

### Preparation of trans-4-(3,4-dihydroisoquinolin-2(1H)-yl)-5-hydroxyazepan-1-yl)(6-((1-(2,2,2-trifluoroethyl)piperidin-4-yl)amino)pyrimidin-4-yl)ketone:

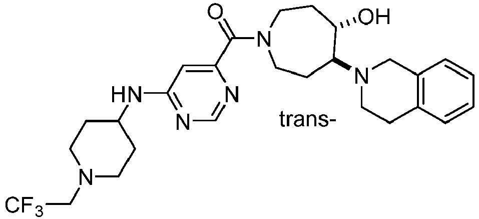

### Step I: preparation of t-butyl (1-(2,2,2-trifluoroethyl)piperidin-4-yl)carbamate:

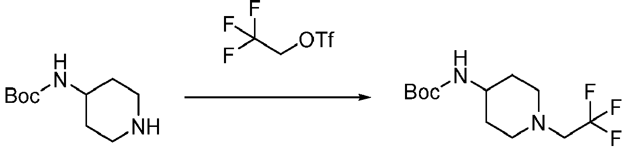

4-Boc-aminopiperidine (200 mg, 1.00 mmol, 1.0 eq) and triethylamine (404 mg, 3.99 mmol, 4.0 eq) were dissolved in tetrahydrofuran (2.5 mL), followed by addition of 2,2,2-trifluoroethyl trifluoromethanesulfonate (278 mg, 1.20 mmol, 1.2 eq), and a reaction was stirred at 75°C for 16 h. The reaction mixture was concentrated, and the crude product was separated and purified by flash chromatography (silica gel, DCM: MeOH = 15: 1) to obtain the target compound (267 mg, 94.7%), as a white solid. LCMS (ESI) [M+H]⁺ = 283.

### Step II: preparation of 1-(2,2,2-trifluoroethyl)piperidin-4-amine:

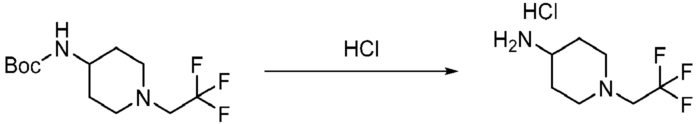

T-butyl (1-(2,2,2-trifluoroethyl)piperidin-4-yl)carbamate (224 mg, 0.79 mmol, 1.0 eq) was dissolved in methanol (1 mL), followed by addition of a hydrochloric acid solution in 1,4-dioxane (3 mL, 4 M), and a reaction was stirred at 20°C for 40 min. The reaction mixture was concentrated to obtain a crude product of the target compound (216 mg) as a white solid. LCMS (ESI) [M+H]⁺ = 183.10.

### Step III: preparation of trans-(4-(3,4-dihydroisoquinolin-2(1H)-yl)-5-hydroxyazepan-1-yl)(6-((1-(2,2,2-trifluoroethyl)piperidin-4-yl)amino)pyrimidin-4-yl)ketone:

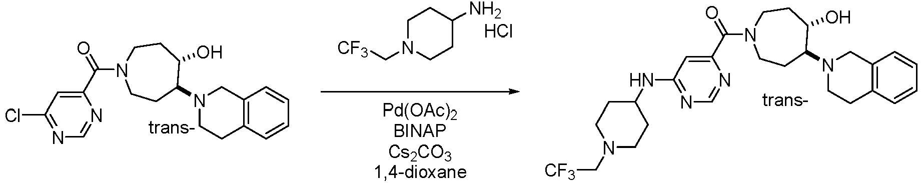

Trans-(6-chloropyrimidin-4-yl)(4-(3,4-dihydroisoquinolin-2(1*H*)-yl)-5-hydroxyazepan-1-yl)ketone (100 mg, 0.26 mmol, 1.0 equiv.), 1-(2,2,2-trifluoroethyl)piperidin-4-amine (141 mg, 0.78 mmol, 3.0 equiv.), Pd(OAc)₂ (palladium acetate) (12 mg, 0.05 mmol, 0.2 equiv.), BINAP (1,1'-binaphthyl-2,2'-bis(diphenylphosphine)) (64 mg, 0.10 mmol, 0.4 equiv.) and Cs₂CO₃ (cesium carbonate) (421 mg, 1.29 mmol, 5.0 equiv.) were dissolved in 1,4-dioxane (2 mL), and a reaction was stirred at 110°C under nitrogen for 16 h. The reaction mixture was filtrated, the filtrate was concentrated, and the crude product was separated and purified by preparative HPLC (C18, 10 mmol/L aqueous NH₄HCO₃, acetonitrile) to obtain trans-(4-(3,4-dihydroisoquinolin-2(1*H*)-yl)-5-hydroxyazepan-1-yl)(6-((1-(2,2,2-trifluoroethyl )piperidin-4-yl)amino)pyrimidin-4-yl)ketone (26.6 mg, 19.4%). LCMS (ESI) [M+H]⁺ = 533.7; ¹H NMR (400 MHz, CDCl₃) δ 8.53 (d, *J* = 5.2 Hz, 1H), 7.19 - 7.10 (m, 3H), 7.07 - 7.00 (m, 1H), 6.55 (d, *J=* 18.8 Hz, 1H), 5.07 (br s, 1H), 4.63 (br s, 1H), 4.03 - 3.86 (m, 2H), 3.84 - 3.30 (m, 6H), 3.09 - 2.87 (m, 7H), 2.74 - 2.48 (m, 4H), 2.38 - 2.12 (m, 2H), 2.08 - 1.96 (m, 2H), 1.89 - 1.71 (m, 3H), 1.61 - 1.54 (m, 1H).

### Example 128

### Preparation of trans-(4-(3,4-dihydroisoquinolin-2(1H)-yl)-5-hydroxyazepan-1-yl)(6-((tetrahydro-2H-pyran-4-yl)amino)pyrimidin-4-yl)ketone:

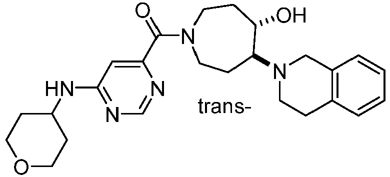

### Step I: preparation of trans-(4-(3,4-dihydroisoquinolin-2(1H)-yl)-5-hydroxyazepan-1-yl)-(6-((tetrahydro-2H-pyran-4-yl)amino)pyrimidin-4-yl)ketone:

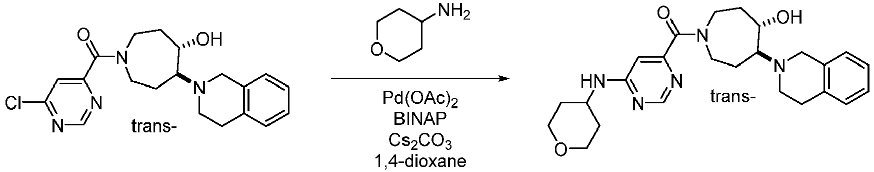

Trans-(6-chloropyrimidin-4-yl)(4-(3,4-dihydroisoquinolin-2(1*H*)-yl)-5-hydroxyazepan-1-yl)ketone (50 mg, 0.13 mmol, 1.0 equiv.), 4-aminotetrahydropyran (52 mg, 0.52 mmol, 4.0 equiv.), Pd(OAc)₂ (palladium acetate) (6 mg, 0.03 mmol, 0.2 equiv.), BINAP (1,1'-binaphthyl-2,2'-bis(diphenylphosphine)) (32 mg, 0.05 mmol, 0.4 equiv.) and Cs₂CO₃ (cesium carbonate) (210 mg, 0.65 mmol, 5.0 equiv.) were dissolved in 1,4-dioxane (1 mL), and a reaction was stirred at 110°C under nitrogen for 16 h. The reaction mixture was filtrated, the filtrate was concentrated, and the crude product was separated and purified by preparative HPLC (C18, 10 mmol/L aqueous NH₄HCO₃, acetonitrile) to obtain trans-(4-(3,4-dihydroisoquinolin-2(1*H*)-yl)-5-hydroxyazepan-1-yl)(6-((tetrahydro-2H-pyran-4-yl)amino)pyrimidin-4-yl)ketone (30.1 mg, 51.7%). LCMS (ESI) [M+H]⁺ = 452.5; ¹H NMR (400 MHz, CDCl₃) *δ* 8.54 (s, 1H), 7.20 - 7.10 (m, 3H), 7.07 - 6.98 (m, 1H), 6.58 (d, *J=* 19.4 Hz, 1H), 5.06 (br s, 1H), 4.65 (br s, 1H), 4.05 - 3.87 (m, 4H), 3.84 - 3.31 (m, 8H), 3.09 - 2.88 (m, 3H), 2.67 (s, 2H), 2.40 - 2.12 (m, 2H), 2.07 - 1.97 (m, 2H), 1.92 - 1.68 (m, 3H), 1.59 - 1.50 (m, 1H).

### Example 129

### Trans-(4-(3,4-dihydroisoquinolin-2(1H)-yl)-5-hydroxyazepan-1-yl)(6-((1-methylpiperidi n-4-yl)amino)pyrimidin-4-yl)ketone:

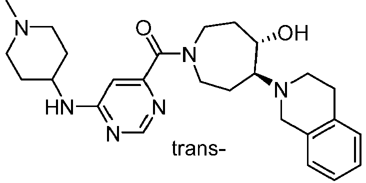

### Step I: trans-(4-(3,4-dihydroisoquinolin-2(1H)-yl)-5-hydroxyazepan-1-yl)(6-((1-methylpiperidin-4-yl)amino)pyrimidin-4-yl)ketone:

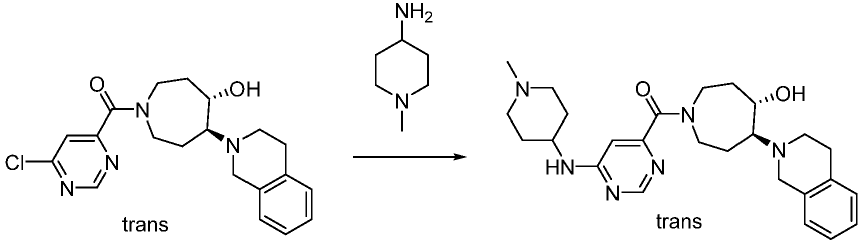

At room temperature (20°C) and under nitrogen, the raw material trans-(6-chloropyrimidin-4-yl)(4-(3,4-dihydroisoquinolin-2(1*H*)-yl)-5-hydroxyazepan-1-yl)ketone (racemic) (100 mg, 0.26 mmol, 1.0 eq) was dissolved in 1,4-dioxane (2 mL), followed by addition of 1-methylpiperidin-4-amine hydrochloride (117 mg, 0.77 mmol, 3.0 eq), palladium acetate (12 mg, 0.05 mmol, 0.2 eq), BINAP (1,1'-binaphthyl-2,2'-bis(diphenylphosphine)) (65 mg, 0.1 mmol, 0.4 eq) and cesium carbonate (420 mg, 1.3 mmol, 5.0 eq), and heated at 100 °C under stirring for 16 h. After the reaction was complete as indicated by LCMS, the reaction mixture was cooled to room temperature, diluted with dichloromethane (5 mL), filtrated and concentrated, and the crude product was separated and purified by preparative HPLC (C18, 10 mmol/L aqueous NH₄HCO₃, acetonitrile) to obtain the compound trans-(4-(3,4-dihydroisoquinolin-2(1*H*)-yl)-5-hydroxyazepin-1-yl)(6-((1-methylpiperidin-4-yl )amino)pyrimidin-4-yl)ketone (4.2 mg, 3.5%). LCMS: [M+H]⁺ =1.50; ¹H NMR (400 MHz, CDCl₃) *δ* 8.52 (d, *J=* 4.6 Hz, 1H), 7.18 - 7.09 (m, 3H), 7.07 - 6.98 (m, 1H), 6.55 (d, *J* = 18.3 Hz, 1H), 5.12 (br s, 1H), 4.02 - 3.93 (m, 1H), 3.92 - 3.87 (m, 1H), 3.83 - 3.30 (m, 6H), 3.06 - 2.90 (m, 3H), 2.84 (d, *J* = 11.1 Hz, 2H), 2.70 - 2.56 (m, 2H), 2.32 (s, 3H), 2.25 - 2.13 (m, 3H), 2.04 (d, *J=* 13.6 Hz, 3H), 1.84 - 1.72 (m, 3H), 1.60 - 1.54 (m, 1H).

### Example 130

### Preparation of trans-(4-(3,4-dihydroisoquinolin-2(1H)-yl)-5-hydroxyazepan-1-yl)(6-((1-(methylsulfonyl)piperidin-4-yl)amino)pyrimidin-4-yl)ketone:

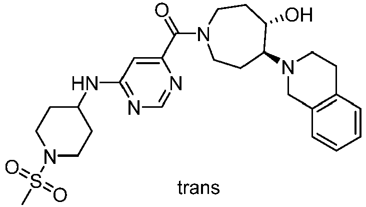

### Step I: preparation of trans-(4-(3,4-dihydroisoquinolin-2(1H)-yl)-5-hydroxyazepan-1-yl)(6-((1-(methylsulfonyl)piperidin-4-yl)amino)pyrimidin-4-yl)ketone:

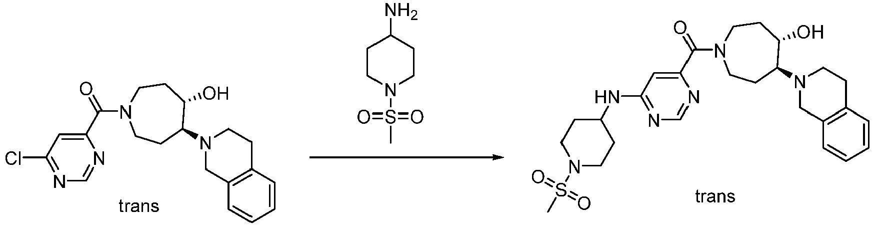

At room temperature (20°C) and under nitrogen, the raw material (6-chloropyrimidin-4-yl)(trans-(4-(3,4-dihydroisoquinolin-2(1H)-yl)-5-hydroxyazepan-1-yl) methanone (50 mg, 0.13 mmol, 1.0 eq) was dissolved in 1,4-dioxane (1 mL), followed by addition of 1-methanesulfonylpiperidin-4-amine hydrochloride (CAS: 651057-01-1) (83 mg, 0.39 mmol, 3.0 eq), palladium acetate (CAS: 462-08-8) (6 mg, 0.03 mmol, 0.2 eq), BINAP (1,1'-binaphthyl-2,2'-bis(diphenylphosphine) CAS: 98327-87-8) (32 mg, 0.05 mmol, 0.4 eq) and cesium carbonate (CAS: 534-17-8) (210 mg, 0.65 mmol, 5.0 eq), and heated at 100°C under stirring for 16 h. After the reaction was complete as indicated by LCMS, the reaction mixture was cooled to room temperature, diluted with dichloromethane (5 mL), filtrated and concentrated, and the crude product was separated and purified by preparative HPLC (C18, 10 mmol/L aqueous NH₄HCO₃, acetonitrile) to obtain the title compound trans-(4-(3,4-dihydroisoquinolin-2(1H)-yl)-5-hydroxyazepan-1-yl)(6-((1-(methylsulfonyl)pip eridin-4-yl)amino)pyrimidin-4-yl)ketone (14.9 mg, 21.8%). LCMS: [M+H]⁺ =1.56; ¹H NMR (400 MHz, CDCl₃) *δ* 8.53 (d, *J=* 6.7 Hz, 1H), 7.21 - 7.09 (m, 3H), 7.08 - 7.00 (m, 1H), 6.59 (d, *J=* 18.6 Hz, 1H), 5.33 (dd, *J=* 41.0, 7.6 Hz, 1H), 4.68 (s, 1H), 4.07 - 3.86 (m, 3H), 3.83 - 3.32 (m, 7H), 3.07 - 2.98 (m, 1H), 2.98 - 2.86 (m, 4H), 2.82 (s, 3H), 2.66 (d, *J=* 6.5 Hz, 2H), 2.37 - 2.03 (m, 4H), 1.92 - 1.70 (m, 3H), 1.62 - 1.60 (m, 1H).

### Example 131

### Preparation of trans-4-((6-(4-(3,4-dihydroisoquinolin-2(1H)-yl)-3-hydroxypiperidine-1-carbonyl)pyrimidin-4-yl)amino)-N-methylbenzamide:

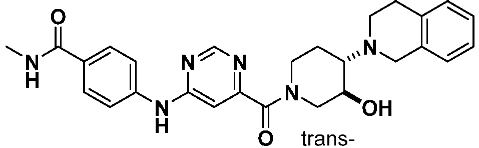

### Step I: preparation of trans-4-((6-(4-(3,4-dihydroisoquinolin-2(1H)-yl)-3-hydroxypiperidine-1-carbonyl)pyrimidin-4-yl)amino)-N-methylbenzamide:

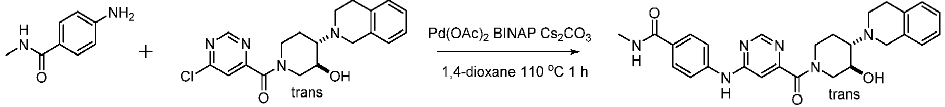

The compounds trans-1-(6-chloropyrimidin-4-yl)-4-(3,4-dihydroisoquinolin-2(1H)-yl)-piperidin-3-ol (50 mg, 0.13 mmol, 1.0 equiv.), 4-amino-N-methylbenzamide(24 mg, 0.16 mmol, 1.2 equiv.), Pd (OAc)₂ (palladium acetate) (3 mg, 0.013 mmol, 0.1 equiv.), BINAP(1,1'-binaphthyl-2,2'-bis(diphenylphosphine)) (17.0 mg, 0.0266 mmol, 0.2 equiv.) and Cs₂CO₃ (cesium carbonate) (87 mg, 0.26 mmol, 2.0 equiv.) were placed in a microwave tube, purged with nitrogen, followed by addition of anhydrous dioxane (0.5 mL), and a reaction was stirred under heating at 110°C for 1 h. The reaction was complete as monitored by LCMS, and the reaction mixture was cooled to room temperature and extracted with ethyl acetate (20 mL). The organic phase was washed once with saturated sodium chloride (20 mL), dried over anhydrous sodium sulfate, filtrated, and concentrated. The crude product was separated and purified by Prep-HPLC (C18, 10mmol/L aqueous NH₄HCO₃, acetonitrile) to obain 18.93 mg of the title compound, yield: 29.0%. LCMS(ESI):m/z=487.5; ¹H NMR (400 MHz, CDCl₃) δ 8.72 - 8.71 (m, 1H), 7.78 - 7.76 (m, 2H), 7.58 - 7.41 (m, 3H), 7.20 - 7.10 (m, 3H), 7.08 - 6.97 (m, 2H), 6.18 (d, J = 6.1 Hz, 1H), 5.05 - 4.73 (m, 1H), 4.20 -4.13 (m, 1H), 3.96 (d, J = 14.9 Hz, 1H), 3.72 (d, J = 13.7 Hz, 2H), 3.08 - 2.99 (m, 5H), 2.99 - 2.78 (m, 3H), 2.77 - 2.59 (m, 2H), 2.02 -1.67 (m, 1H), 1.67 - 1.48 (m, 1H).

### Example 132

### Preparation of (trans-4-(3,4-dihydroisoquinolin-2(1H)-yl)-3-hydroxypiperidin-1-yl)(6-((4-(methanesulfonyl)phenyl)amino)pyrimidin-4-yl)ketone:

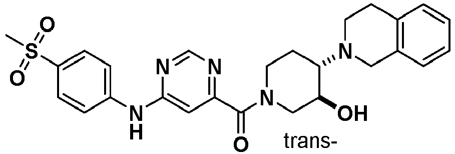

### Step I: preparation of (trans-4-(3,4-dihydroisoquinolin-2(1H)-yl)-3-hydroxypiperidin-1-yl)(6-((4-(methanesulfonyl)phenyl)amino)pyrimidin-4-yl)ketone:

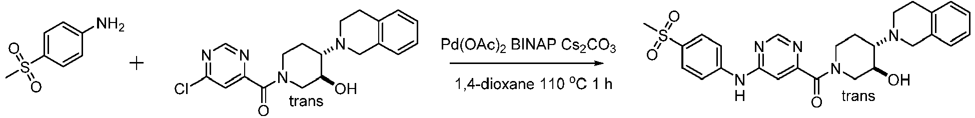

The compounds trans-1-(6-chloropyrimidin-4-yl)-4-(3,4-dihydroisoquinolin-2(1H)-yl)-piperidin-3-ol (50 mg, 0.134 mmol, 1.0 equiv.), 4-(methanesulfonyl)aniline (27.55 mg, 0.161 mmol, 1.2 equiv.), Pd(OAc)₂ (palladium acetate) (3 mg, 0.013 mmol, 0.1 equiv.), BINAP (1,1'-binaphthyl-2,2'-bis(diphenylphosphine)) (16.7 mg, 0.026 mmol, 0.2 equiv.) and Cs₂CO₃ (cesium carbonate) (87 mg, 0.26 mmol, 2.0 equiv.) were placed in a microwave tube, purged with nitrogen, followed by addition of anhydrous dioxane (0.5 mL), and a reaction was stirred under heating at 110°C for 1 h. The reaction was complete as monitored by LCMS, and the reaction mixture was cooled to room temperature and extracted with ethyl acetate (20 mL). The organic phase was washed once with saturated sodium chloride (20 mL), dried over anhydrous sodium sulfate, filtrated, and concentrated. The crude product was separated and purified by Prep-HPLC (C18, 10mmol/L aqueous NH₄HCO₃, acetonitrile) to obtain 21.41 mg of the title compound, yield: 31.5%. LCMS(ESI): m/z=508.2; ¹H NMR (400 MHz, DMSO-d₆) δ 10.31 (d, J = 7.5 Hz, 1H), 8.78 (dd, J = 9.2, 1.2 Hz, 1H), 8.03 - 7.94 (m, 2H), 7.96 - 7.87 (m, 2H), 7.14 - 7.01 (m, 4H), 6.99 (dd, J = 7.1, 1.2 Hz, 1H), 4.87 - 4.74 (m, 1H), 4.54 - 4.30 (m, 1H), 3.89 - 3.60 (m, 4H), 3.18 (d, J = 1.8 Hz, 3H), 3.06 (t, J = 12.2 Hz, 0.5H), 2.96 - 2.73 (m, 5H), 2.70 - 2.61 (m, 1.5H), 1.92 - 1.72 (m, 1H), 1.63 - 1.47 (m, 1H).

### Example 133

### Preparation of (trans-4-(3,4-dihydroisoquinolin-2(1H)-yl)-3-hydroxypiperidin-1-yl)(6-((3-(methanesulfonyl)phenyl)amino)pyrimidin-4-yl)ketone:

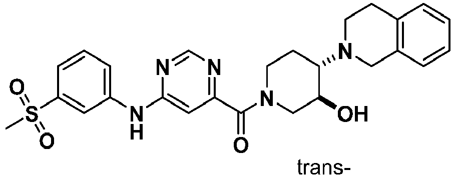

### Step I: preparation of (trans-4-(3,4-dihydroisoquinolin-2(1H)-yl)-3-hydroxypiperidin-1-yl)(6-((3-(methylsulfonyl)phenyl)amino)pyrimidin-4-yl)ketone:

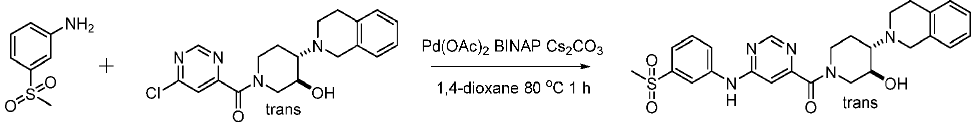

The compounds trans-1-(6-chloropyrimidin-4-yl)-4-(3,4-dihydroisoquinolin-2(1H)-yl)-piperidin-3-ol (83.0 mg, 0.223 mmol, 1.0 equiv.), 3-(methanesulfonyl)aniline (46.0 mg, 0.268 mmol, 1.5 equiv.), Pd(OAc)₂ (palladium acetate) (5.0 mg, 0.0223 mmol, 0.1 equiv.), BINAP (1,1'-binaphthyl-2,2'-bis(diphenylphosphine)) (14.0 mg, 0.0223 mmol, 0.1 equiv.) and Cs₂CO₃ (cesium carbonate) (145.0 mg, 0.446 mmol, 2.0 equiv.) were placed in a microwave tube, purged with nitrogen, followed by addition of anhydrous dioxane (1.3 mL), and a reaction was stirred under heating at 80°C for 1 h. The reaction was complete as indicated by LCMS, and the reaction mixture was cooled to room temperature and extracted with ethyl acetate (20 mL). The organic phase was washed once with saturated sodium chloride (20 mL), dried over anhydrous sodium sulfate, filtrated, and concentrated. The crude product was separated and purified by preparative HPLC (C18, 10mmol/L aqueous NH₄HCO₃, acetonitrile) to obtain 14.91 mg of the target product, yield: 13.2%. LCMS(ESI):m/z=508.4; ¹H NMR (400 MHz, DMSO-d₆) δ 10.22 (d, J = 7.5 Hz, 1H), 8.74 (dd, J = 9.2, 0.9 Hz, 1H), 8.36-8.26 (m, 1H), 8.10-7.99 (m, 1H), 7.70-7.53 (m, 2H), 7.13-7.00 (m, 4H), 6.93 (dd, J = 6.5, 1.1 Hz, 1H), 4.86-4.74 (m, 1H), 4.55-4.30 (m, 1H), 3.91-3.59 (m, 4H), 3.22 (d, J = 2.0 Hz, 3H), 3.11-2.84 (m, 3H), 2.82-2.61 (m, 4H), 1.92-1.70 (m, 1H), 1.64-1.43 (m, 1H).

### Example 134

### Preparation of trans-3-((6-(4-(3,4-dihydroisoquinolin-2(1H)-yl)-3-hydroxypiperidine-1-carbonyl)pyrimidin-4-yl)amino)-N-methylbenzamide:

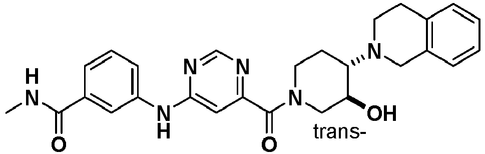

### Step I: preparation of trans-3-((6-(4-(3,4-dihydroisoquinolin-2(1H)-yl)-3-hydroxypiperidine-1-carbonyl)pyrimidin-4-yl)amino)-N-methylbenzamide:

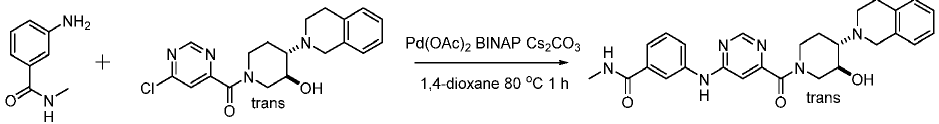

The compounds trans-1-(6-chloropyrimidin-4-yl)-4-(3,4-dihydroisoquinolin-2(1H)-yl)-piperidin-3-ol (99.0 mg, 0.266 mmol, 1.0 equiv.), 3-amino-N-methylbenzamide (48.0 mg, 0.319 mmol, 1.5 equiv.), Pd(OAc)₂ (palladium acetate) (6.0 mg, 0.0266 mmol, 0.1 equiv.), BINAP (1,1'-binaphthyl-2,2'-bis(diphenylphosphine)) (17.0 mg, 0.0266 mmol, 0.1 equiv.) and Cs₂CO₃ (cesium carbonate) (173.3 mg, 0.532 mmol, 2.0 equiv.) were placed in a microwave tube, purged with nitrogen, followed by addition of anhydrous dioxane (1.6 mL), and a reaction was stirred under heating at 80°C for 1 h. The reaction was complete as indicated by LCMS, and the reaction mixture was cooled to room temperature and extracted with ethyl acetate (20 mL). The organic phase was washed once with saturated sodium chloride (20 mL), dried over anhydrous sodium sulfate, filtrated, and concentrated. The crude product was separated and purified by preparative HPLC (C18, 10mmol/L aqueous NH₄HCO₃, acetonitrile) to obtain 25.03 mg of the title compound, yield: 19.4%. LCMS(ESI):m/z=487.5; ¹H NMR (400 MHz, DMSO) δ 9.96 (d, J = 7.4 Hz, 1H), 8.67 (dd, J = 9.1, 0.9 Hz, 1H), 8.48-8.36 (m, 1H), 8.12-8.03 (m, 1H), 7.95-7.82 (m, 1H), 7.54-7.38 (m, 2H), 7.14-7.00 (m, 4H), 6.88 (dd, J = 6.7, 1.1 Hz, 1H), 4.80 (dd, J = 41.7, 3.9 Hz, 1H), 4.55-4.29 (m, 1H), 3.92-3.58 (m, 4H), 3.09-2.73 (m, 9H), 2.69-2.63 (m, 1H), 1.93-1.69 (m, 1H), 1.62 -1.43 (m, 1H).

### Example 135

### Preparation of trans-(4-(3,4-dihydroisoquinolin-2(1H)-yl)-3-hydroxypiperidin-1-yl)(6-((1,1-dihydroxytetrahydro-2H-thiapyran-4-yl)amino)pyrimidin-4-yl)ketone

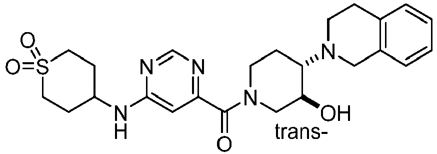

### Step I: preparation of methyl 6-((1,1-dihydroxytetrahydro-2H-thiapyran-4-yl)amino)-pyrimidine-4-carboxylate:

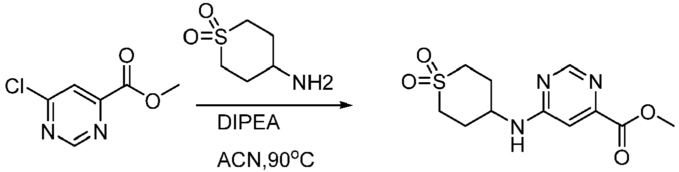

The raw material methyl 6-chloropyrimidine-4-carboxylate (200 mg, 1.22 mmol, 1.0 equiv.) was added to acetonitrile (3 mL), followed by addition of 4-aminotetrahydro-2H-thiapyran-1,1-dioxide (200 mg, 1.34 mmol, 1.1 equiv) and DIPEA (N,N-diisopropylethylamine) (315 mg, 2.44 mmol, 2.0 equiv.), and stirred at 90°C for 12 h. After the reaction was complete as indicated by LCMS, the reaction mixture was cooled to room temperature (28°C), mixed with water (5 mL), and extracted three times with dichloromethane:methanol (V/V=10/1) (5 mL×3). The organic phases were combined, filtrated and concentrated, and the crude prorduct was separated and purified by flash chromatography (silica gel, dichloromethane:methanol (V/V= 10/1) to obtain the title compound methyl 6-((1,1-dihydroxytetrahydro-2H-thiapyran-4-yl)amino)pyrimidine-4-carboxylate (190 mg), yield: 60%, as a yellow solid. LCMS (ESI) [M+H]+ = 286.2; ¹H NMR (400 MHz, CD₃OD) δ 8.53 (d, J = 1.2 Hz, 1H), 7.18 (d, J = 1.2 Hz, 1H), 4.33 (s, 1H), 3.95 (s, 3H), 3.33-3.31 (m, 3H), 3.18-3.12(m, 2H), 2.41-2.37(m, 2H), 2.22-2.12 (m, 2H).

### Step II: preparation of 6-((1,1-dihydroxytetrahydro-2H-thiapyran-4-yl)amino)pyrimidine-4-carboxylic acid:

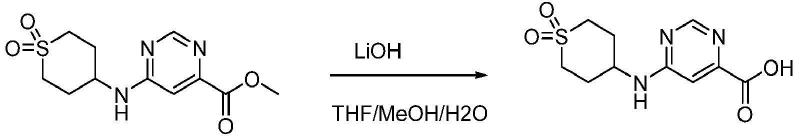

At room temperature (28°C), the raw material methyl 6-((1,1-dihydroxytetrahydro-2H-thiapyran-4-yl)amino)pyrimidine-4-carboxylate (200 mg, 0.70 mmol, 1.0 equiv.) was added to water (1 mL), methanol (1.0 mL) and tetrahydrofuran (1.0 mL), followed by addition of lithium hydroxide (67 mg, 2.8 mmol, 4.0 equiv.), and stirred at room temperature (28°C) for 2h. After the reaction was complete as indicated by TLC analysis (dichloromethane:methanol (V/V= 10/1)), the reaction mixture was concentrated to 1mL, and adjusted to pH=1-2 with 1M (molar concentration) HCl. The aqueous phase was lyophilized to obtain the title compound (250 mg) as a white solid. LCMS (ESI) [M+H]⁺ =272.13.

### Step III: preparation of trans-(4-(3,4-dihydroisoquinolin-2(1H)-yl)-3-hydroxypiperidin-1-yl) (6-((1,1-dihydroxytetrahydro-2H-thiapyran-4-yl)amino)pyrimidin-4-yl)ketone:

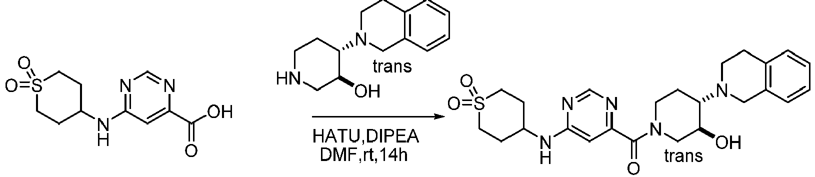

The raw material 6-((1,1-dihydroxytetrahydro-2H-thiapyran-4-yl)amino)pyrimidine-4-carboxylic acid (120 mg, 0.44 mmol, 1.0 equiv.) was added to DMF (N,N-dimethylformamide) (3 mL), followed by addition of HATU (2-(7-azabenzotriazol-1-yl)-*N*,*N*,*N*',*N*'-tetramethylurea hexafluorophosphate) (201 mg, 0.53 mmol, 1.2 equiv.) and DIPEA (N,N-diisopropylethylamine) (114 mg, 0.22 mmol, 2.0 equiv.), and stirred for 5 minutes. Then trans-4-(3,4-dihydroisoquinolin-2(1H)-yl)piperidin-3-ol (113 mg, 0.48 mmol, 1.20 equiv.) in DMF (1 mL) was added, and stirred at room temperature (28°C) for 3 h. After the reaction was complete as indicated by LCMS, the crude product was separated and purified by preparative HPLC (C18, 10 aqueous mmol/L NH₄HCO₃, acetonitrile), and lyophilized to obtain the title compound (25 mg, yield: 11.71%). LCMS (ESI) [M+H]+ =486.2; ¹H NMR (400 MHz, MeOD-d4) δ 8.50 (d, J = 8.8 Hz, 1H), 7.15-7.08 (m, 4H), 6.65 (d, J = 9.6 Hz, 1H), 4.76-4.56 (m, 1H), 4.33 (s, 1H),4.07-3.96 (m, 2H), 3.90-3.76 (m, 2H), 3.33-3.26 (m, 2H), 3.18-3.09 (m, 4H), 3.02-2.88(m, 6H), 2.41-2.37 (m, 2H), 2.22-2.06 (m, 3H), 1.96-1.71 (m, 2H).

### Example 136

### Preparation of trans-1-(4-(3,4-dihydroisoquinolin-2(1H)-yl)-3-hydroxypiperidin-1-yl) (2-(pyridin-4-yl)pyrimidin-4-yl)ketone

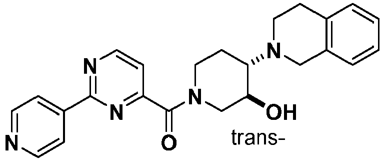

### Step I: preparation of trans-1-(2-chloropyrimidin-4-yl)(4-(3,4-dihydroisoquinolin-2(1H)-yl)-3-hydroxypiperidin-1-yl)ketone:

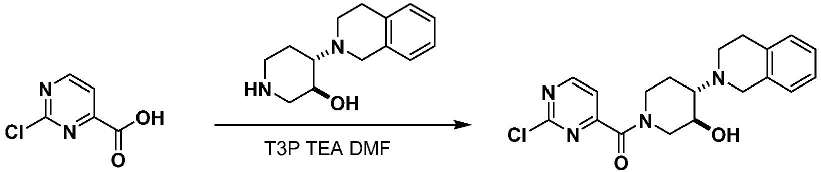

The raw materials 2-chloropyrimidine-4-carboxylic acid (300 mg, 1.8942 mmol, 1.1 equiv.) and trans-4-(3,4-dihydroisoquinolin-2(1H)-yl)piperidin-3-ol (400 mg, 1.722 mmol, 1 equiv.) were dissolved in DMF (N,N-dimethylformamide) (4 ml), followed by addition of TEA (triethylamine) (878 mg, 8.61 mmol, 5 equiv.) at 25°C, and stirred evenly. Then T3P (CAS: 68957-94-8) (1.643 g, 5.166 mmol, 3 equiv.) was added. The reaction was stirred at room temperature for an hour, and was quenched by adding water. The mixture was extracted with ethyl acetate, and the organic phase was dried and concentrated to obtain trans-1-(2-chloropyrimidin-4-yl)(4-(3,4-dihydroisoquinolin-2(1H)-yl)-3-hydroxypiperidin-1-yl)ketone (120 mg, yield of the crude product not calculated) as a light yellow oily substance. LCMS (ESI) [M+1]⁺ = 373.2.

### Step II: preparation of trans-1-(4-(3,4-dihydroisoquinolin-2(1H)-yl)-3-hydroxypiperidin-1-yl)(2-(pyridin-4-yl)pyrimidin-4-yl)ketone:

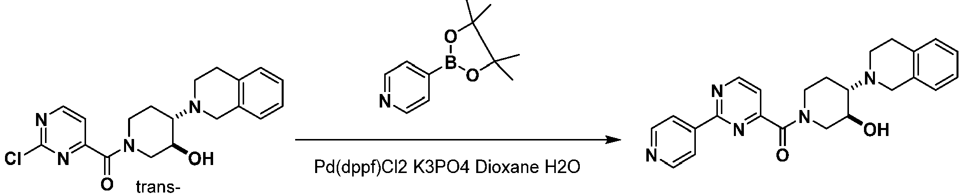

Trans-1-(2-chloropyrimidin-4-yl)(4-(3,4-dihydroisoquinolin-2(1H)-yl)-3-hydroxypiperidin-1-yl)-ketone (120 mg crude product, 0.322 mmol, 1 equiv.) and 4-pyridine boronic acid pinacol ester (79 mg, 0.385 mmol, 1.2 equiv.) were dissolved in 1,4-dioxane (1.2 ml) and water (0.3 ml), followed by addition of Pd(dppf)Cl₂ (24 mg, 0.0355 mmol, 0.1 equiv.) and potassium phosphate (205 mg, 0.966 mmol, 3 equiv.), and the reaction mixture was stirred at 90°C overnight. The reaction was complete as indicated by LCMS. The reaction mixture was quenched by adding water, and then extracted with dichloromethone. The organic phase was dried, and concentrated to obtain a crude product, which was purified by a preparative HPLC reverse phase column (C18, 10 mmol/L aqueous NH₄HCO₃, acetonitrile) to obtain the title compound (33.55 mg, purity 94.6%, yield: 25%). LCMS (ESI) [M+1] ⁺ = 416.2,; ¹H NMR (400 MHz, DMSO-d₆) δ 9.17-9.14 (m, 1H), 8.92-8.73 (m, 2H), 8.27-8.24 (m, 2H), 7.77-7.74 (m, 1H), 7.14-6.94 (m, 4H), 4.92-4.72 (m, 1H), 4.55-4.36 (m, 1H), 3.84-3.76 (m, 2H), 3.72-3.66 (m, 2H), 3.18-3.08 (m, 1H), 3.05-2.89 (m, 2H), 2.88 -2.73 (m, 4H), 1.92-1.74 (m, 1H), 1.65-1.57 (m, 1H).

### Example 137

### Preparation of trans-1-(4-(3,4-dihydroisoquinolin-2(1H)-yl)-3-hydroxypiperidin-1-yl) (2-phenylpyrimidin-4-yl)ketone

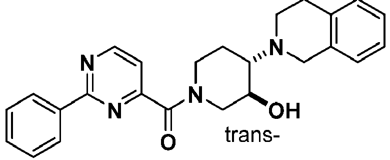

### Step I: preparation of 2-phenylpyrimidine-4-carboxylic acid:

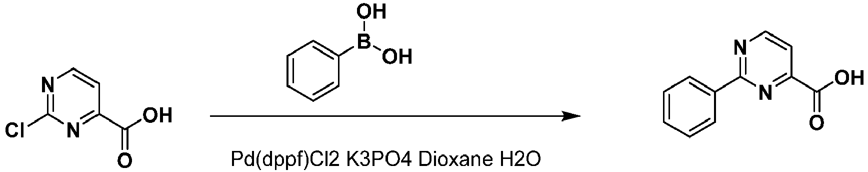

The raw material 2-chloropyrimidine-4-carboxylic acid (500 mg, 3.154 mmol, 1 equiv.) and phenylboronic acid (577 mg, 4.731 mmol, 1.5 equiv.) were dissolved in 1,4-dioxane (12 mL) and water (3 mL), followed by addition of Pd(dppf)Cl₂ (232 mg, 0.3154 mmol, 0.1 equiv.) and potassium phosphate (2.008 g, 9.462 mmol, 3 equiv.), and a reaction was stirred under stirring at 90°C overnight. The reaction was complete as indicated by LCMS. The reaction mixture was quenched by adding water, and then extracted with ethyl acetate. The aqueous phase was concentrated to obtain a crude product which was dissolved in dichloromethane and methanol and then filtrated, and the filtrate was concentrated to obtain 2-phenylpyrimidine-4-carboxylic acid (750 mg crude product, yield not calculated) as a black solid. LCMS (ESI) [M+1]⁺ = 201.2.

### Step II: preparation of trans-1-(4-(3,4-dihydroisoquinolin-2(1H)-yl)-3-hydroxypiperidin-1-yl)(2-phenylpyrimidin-4-yl)ketone:

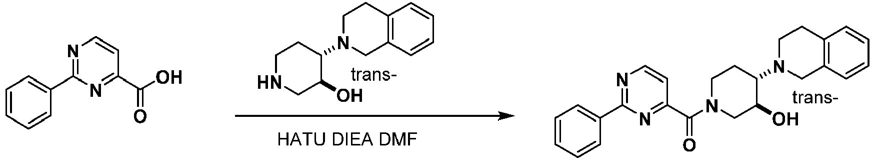

The raw materials 2-phenylpyrimidine-4-carboxylic acid (130 mg, 0.646 mmol, 1.5 equiv.) and trans-4-(3,4-dihydroisoquinolin-2(1H)-yl)piperidin-3-ol (100 mg, 0.430 mmol, 1 equiv.) were dissolved in DMF (N,N-dimethylformamide) (2.5 mL), followed by addition of HATU (2-(7-azabenzotriazole)-N,N,N',N'-tetramethyluronium hexafluorophosphate) (245 mg, 0.646 mmol, 5 equiv.) at 25°C, and stirred evenly. Then DIEA (N,N-diisopropylethylamine) (167 mg, 1.291 mmol, 3 equiv.) was added, and a reaction was stirred at room temperature (25°C) for 1 h. The reaction was quenched by adding water, and then extracted with dichloromethone. The organic phase was dried, and the crude product obtained upon concentration was purified with a preparative HPLC reverse phase column (C18, 10 mmol/L aqueous NH₄HCO₃, acetonitrile) to obtain the title compound (37.89 mg, purity 98.8%, yield: 21%). LCMS (ESI) [M+1] ⁺ = 415.4; ¹H NMR (400 MHz, DMSO) δ 9.07-9.05 (m, 1H), 8.42-8.38 (m, 2H), 7.67-7.47 (m, 4H), 7.15-6.93 (m, 4H), 4.91-4.71(m, 1H), 4.62-4.31 (m, 1H), 3.84-3.75 (m, 2H), 3.78-3.62 (m, 2H), 3.20-2.90 (m, 3H), 2.87-2.65 (m, 4H), 1.93-1.73 (m, 1H),1.67-1.60 (m, 1H).

### Example 138

### Preparation of 4-((6-(trans-4-(3,4-dihydroisoquinolin-2(1H)-yl)-3-hydroxypiperidine-1-carbonyl)pyrimidin-4-yl)amino)cyclohexane-1-carbonitrile:

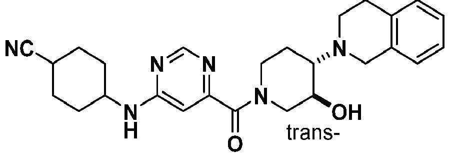

### Step I: preparation of methyl 6-((4-cyanocyclohexyl)amino)pyrimidine-4-carboxylate:

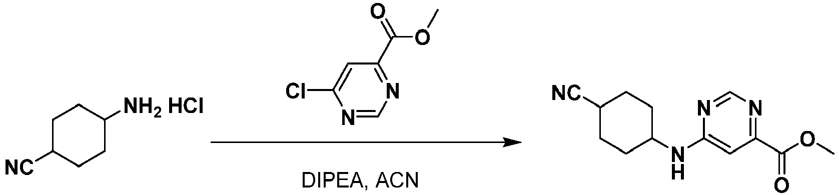

The raw materials 4-aminocyclohexanecarbonitrile hydrochloride (200 mg, 1.245 mmol, 1 equiv.) and methyl 6-chloropyrimidine-4-carboxylate (258 mg, 1.494mmol, 1.2 equiv.) were dissolved in acetonitrile (6.5 ml), followed by addition of DIPEA (N,N-diisopropylethylamine) (644 mg, 4.980 mmol, 4 equiv.) at 25°C, and stirred at 90°C for 18 h. After the reaction was complete as monitored by LCMS, the solvent was rotary evaporated, and the resultant was separated and purified by flash chromatography (silica gel, MeOH: DCM = 0-4%) to obtain the product methyl 6-((4-cyanocyclohexyl)amino)pyrimidine-4-carboxylate (300 mg, yield: 92%), as a light yellow oily substance. LCMS (ESI) [M+1] ⁺ = 261.2.

### Step II: preparation of 6-((4-cyanocyclohexyl)amino)pyrimidine-4-carboxylic acid:

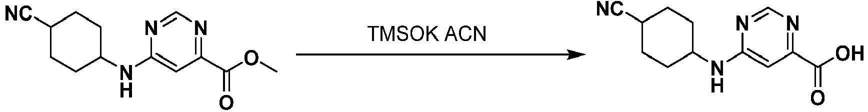

The raw material methyl 6-((4-cyanocyclohexyl)amino)pyrimidine-4-carboxylate (280 mg, 1.075 mmol, 1 equiv.) was dissolved in acetonitrile (15 mL), followed by addition of TMSOK (potassium trimethylsilanolate) (165 mg, 1.291 mmol, 1.2 equiv.), and stirred at room temperature (25°C) for 1 h. The reaction was complete as monitored by TLC. The reaction mixture was filtrated, and the filter cake was collected and then dissolved in water. 1M (molar concentration) HCl was added to adjust to pH 5-6, the solvent was rotary evaporated, and dichloromethane and methanol were added to dissolve the resultant. Solid impurities were removed by filtration, and the filtrate was concentrated to obtain 6-((4-cyanocyclohexyl)amino)pyrimidine-4-carboxylic acid (340 mg crude product, yield not calculated) as a white solid, which was directly used in the following step. LCMS (ESI) [M+1] ⁺ = 247.1.

### Step III: preparation of 4-((6-(trans-4-(3,4-dihydroisoquinolin-2(1H)-yl)-3-hydroxypiperidine-1-carbonyl)pyrimidin-4-yl)amino)cyclohexane-1-carbonitrile:

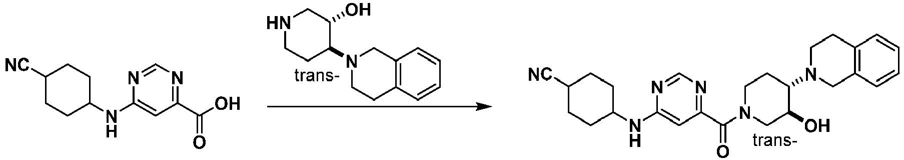

6-((4-cyanocyclohexyl)amino)pyrimidine-4-carboxylic acid (100 mg, 0.406 mmol, 1 equiv.) was dissolved in DMF (N,N-dimethylformamide) (2 mL), followed by addition of HATU (2-(7-azabenzotriazole)-N,N,N',N'-tetramethyluronium hexafluorophosphate) (231 mg, 0.609 mmol, 1.5 equiv.) and DIPEA (N,N-diisopropylethylamine) (160 mg, 1.218 mmol, 3 equiv.), and stirred for 5 min. Then trans-4-(3,4-dihydroisoquinolin-2(1H)-yl)piperidin-3-ol (94 mg, 0.406 mmol, 1 equiv.) was added, and the reaction was stirred at room temperature (25°C) for 1 h. The reaction was monitored by LCMS until the raw materials disappeared. The reaction mixture was quenched by adding water, extracted several times with dichloromethane: methanol=10: 1, and then concentrated to obtain a crude product. The crude product was purified by preparative TLC (silica gel plate, thickness: 1mm, THF:Acetone=1:1), and then purified by a Prep-HPLC reverse phase column (C18, 10 mmol/L aqueous NH₄HCO₃, MeCN) to obtain the product (75.13 mg, purity 100%, yield: 40%). LCMS (ESI) [M+1] ⁺ = 461.5; ¹H NMR (400 MHz, DMSO-d₆) δ 8.43-8.41 (m, 1H), 7.70-7.56 (m, 1H), 7.22-6.92 (m, 4H), 6.52 (s, 1H), 4.82-4.72 (m, 1H), 4.48-4.31 (m, 1H), 3.99-3.67 (m, 4H), 3.65 -3.61 (m, 1H), 3.18-2.70 (m, 7H), 2.68-2.55 (m, 1H), 2.10-1.80 (m, 5H), 1.78-1.64 (m, 2H), 1.51-1.27 (m, 3H).

### Example 139

### Preparation of 1-(4-((6-((trans-4-(3,4-dihydroisoquinolin-2(1H)-yl)-3-hydroxy-piperidine-1-carbonyl)-2-(pyridin-4-yl)pyrimidin-4-yl)amino)piperidin-1-yl)ethan-1-one

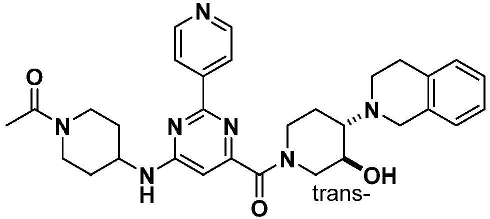

### Step I: preparation of 1-(4-((6-((trans-4-(3,4-dihydroisoquinolin-2(1H)-yl)-3-hydroxypiperidine-1-carbonyl)-2-(pyridin-4-yl)pyrimidin-4-yl)amino)piperidin-1-yl)ethan-1-one:

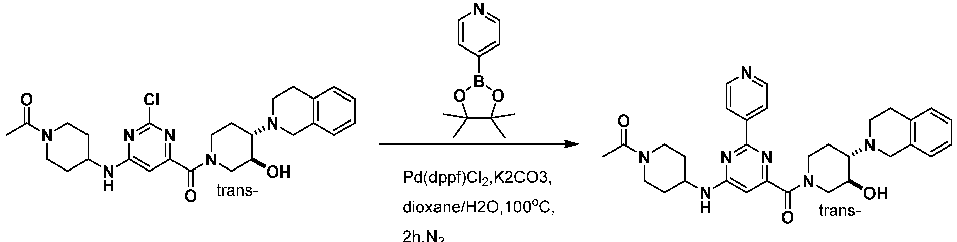

At room temperature (25°C), the raw material 1-(4-((2-chloro-6-(trans-4-(3,4-dihydroisoquinolin-2(1H)-yl)-3-hydroxypiperidine-1-carbonyl )pyrimidin-4-yl)amino)piperidin-1-yl)ethan-1-one (90 mg, 0.175 mmol, 1.0 equiv.) was added to dioxane (6.0 mL) and water (1.5 mL), followed by addition of 4-(4,4,5,5-tetramethyl-1,3,2-dioxaborolan-2-yl)pyridine (72.7 mg, 0.35 mmol, 2.0 equiv.), [1,1'-bis(diphenylphosphino)ferrocene]palladium dichloride (12.8 mg, 0.0175mmol, 0.1 equiv.) and potassium carbonate (48.5 mg, 0.35 mmol, 2.0 equiv.) under nitrogen, and stirred at 100°C for 2 h. After the reaction was complete as indicated by LCMS, the crude product was separated and purified by preparative HPLC (C18, 10 mmol/L aqueous NH₄HCO₃, acetonitrile), and lyophilized to obtain the title compound (7.61 mg, 8%). LCMS (ESI) [M+H]+ = 556.5; ¹H NMR (400 MHz, MeOH) δ 8.66 (td, J = 5.2 Hz, 1.6 Hz, 2H), 8.31 (td, J = 5.2 Hz, 1.6 Hz, 2H), 7.10-7.05 (m, 4H), 6.63 (d, J = 8.8 Hz, 1H), 4.75 (dd, J = 12.8 Hz, 3.2 Hz, 0.5H), 4.60 (d, J = 11.2 Hz, 1H), 4.44 (d, J = 12.4 Hz, 2H), 4.07 (dd, J = 12.4 Hz, 2.4 Hz, 0.5H), 3.98-3.81 (m, 5H), 3.38 (t, J = 12.4 Hz, 1H), 3.20-3.00 (m, 3H), 2.97-2.74 (m, 5H), 2.19-1.90 (m, 6H), 1.78-1.67 (m, 1H), 1.61-1.45 (m, 2H).

### Example 140 Synthesis of 1-(4-((6-((3R,4R)-4-(3,4-dihydro-4,4-dideuterium-isoquinolin-2(1H)-yl)-3-hydroxypiperidine-1-carbonyl)-2-isobutoxypyrimidin-4-yl)amino)piperidin-1-yl)ethan-1 -one:

### Step I: t-butyl (3R,4R)-3-((t-butoxycarbonyl)oxy)-4-(3,4-dihydroisoquinolin-2(1H)-yl)-piperidine-1-carboxylate:

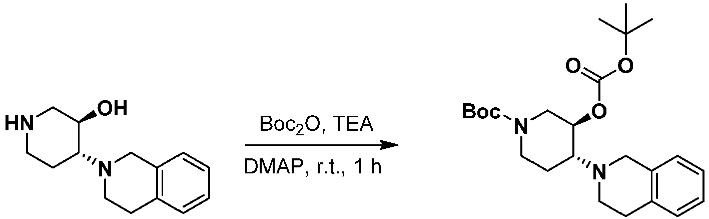

The compounds (3R,4R)-4-(3,4-dihydroisoquinolin-2(1H)-yl)piperidin-3-ol (1 g, 4.3 mmol, 1.0 equiv.), TEA (1.3 g, 12.9 mmol, 3.0 equiv.) and DMAP (105 mg, 0.86 mmol, 0.2 equiv.) were dissolved in THF (20 mL), followed by addition of BoczO (1.88 g, 8.6 mmol, 2.0 equiv.) under stirring, and a reaction was stirred at 20°C for 1 h. The reaction mixture was concentrated, and the crude product was separated and purified by column chromatography (Silica gel, PE: EA = 20: 1) to obtain the target compound (1.12 g, yield: 60%). LCMS (ESI) [M+H]⁺ = 433.3.

### Step II: t-butyl (3R,4R)-3-((t-butoxycarbonyl)oxy)-4-(4-oxo-3,4-dihydroisoquinolin-2(1H)-yl)piperidine-1-carboxylate:

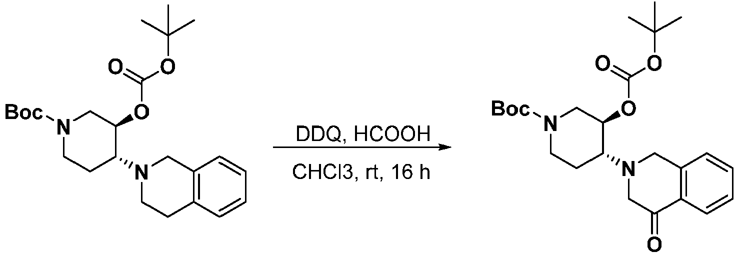

The compound t-butyl (3R,4R)-3-((t-butoxycarbonyl)oxy)-4-(3,4-dihydroisoquinolin-2(1H)-yl)piperidine-1-carboxylate (300 mg, 0.49 mmol, 1.0 equiv.) was dissolved in CHCl₃ (10 mL), followed by addition of formic acid (451 mg, 9.8 mmol, 20.0 equiv.) and DDQ (473 mg, 2.08 mmol, 3.0 equiv.), and a reaction was stirred at 20°C for 16 h. The reaction mixture was mixed with saturated sodium carbonate (5 mL), and extracted with water and DCM. The organic phase was concentrated, and the crude product was separated and purified by column chromatography (Silica gel, DCM: MeOH = 50: 1) to obtain the target compound (46 mg, yield: 18%). LCMS (ESI) [M+Na]⁺ = 469.25.

### Step III: 2-((3R,4R)-3-hydroxypiperidin-4-yl)-2,3-dihydroisoquinolin-4(1H)-one:

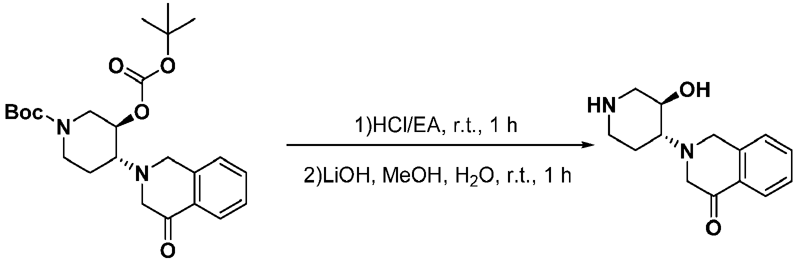

The compound t-butyl (3R,4R)-3-((t-butoxycarbonyl)oxy)-4-(4-oxo-3,4-dihydroisoquinolin-2(1H)-yl)piperidine-1-carboxylate (46 mg, 0.1 mmol, 1.0 equiv.) was dissolved in EA (0.5 mL), followed by addition of ethyl acetate-HCl (5 mL, 4M), and a reaction was stirred at 20°C for 1 h. The reaction mixture was concentrated, and the crude product was dissolved in MeOH:HzO (1 mL: 1 mL), followed by addition of lithium hydroxide (7 mg, 0.3 mmol, 3.0 equiv.), and a reaction was stirred at 20°C for 1 h. The reaction mixture was concentrated, and purified by pre-TL (Silica gel, DCM: MeOH = 5: 1) to obtain the target compound (12 mg, yield: 48%). LCMS (ESI) [M+H]⁺ = 247.

### Step IV: (3R,4R)-4-(3,4-dihydro-4,4-dideuterium-isoquinolin-2(1H)-yl)piperidin-3-ol:

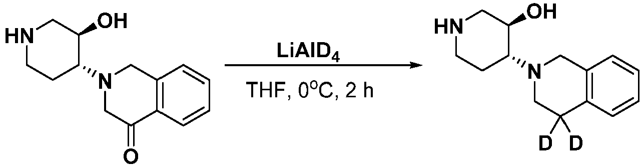

The compound 2-((3R,4R)-3-hydroxypiperidin-4-yl)-2,3-dihydroisoquinolin-4(1H)-one (4 mg, 0.016 mmol, 1.0 equiv.) was dissolved in dry THF (1 mL), followed by addition of LiAlD₄ (1.3 mg, 0.032 mmol, 2.0 equiv.), and a reaction was stirred at 20°C for 2 h. The reaction mixture was quenched with deuterated water, and concentrated to obtain the title compound (6 mg, crude product). LCMS (ESI) [M+1]⁺ =235.1.

### Step V: preparation of methyl 6-((1-acetylpiperidin-4-yl)amino)-2-chloropyrimidine-4-carboxylate

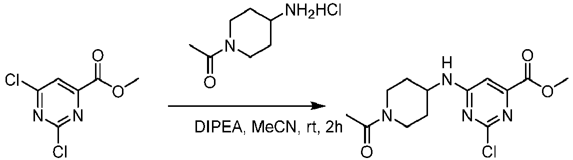

Methyl 2,6-dichloropyrimidine-4-carboxylate (6 g, 28.98 mmol), 1-acetylpiperidin-4-amine hydrochloride (5.7 g, 31.88 mmol) and DIPEA (14.98 g, 115.94 mmol) were dissolved in acetonitrile (50 mL), and stirred at room temperature (25-30°C) (2 h). The reaction mixture was extracted three times with ethyl acetate (100 mL each), and the ethyl acetate phases were combined, washed once with 50 mL water and once with 50 mL saturated sodium chloride, dried over anhydrous sodium sulfate for 10 min, and filtrated. The crude product was separated and purified on a column with DCM:MeOH=100:1 to obtain the product (5.8 g, yield: 64%). LCMS (ESI) [M+H]⁺ =313.2.

### Step VI: preparation of 6-((1-acetylpiperidin-4-yl)amino)-2-isobutoxypyrimidine-4-carboxylic acid:

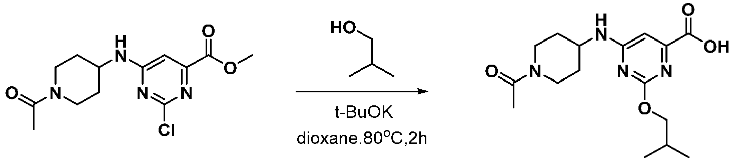

At 20°C, methyl 6-((1-acetylpiperidin-4-yl)amino)-2-chloropyrimidine-4-carboxylate (250 mg, 0.80 mmol, 1.0 equiv.) was dissolved in anhydrous dioxane (3 mL), followed by addition of 2-methylpropan-1-ol (178 mg, 2.4 mmol, 1.1 equiv.) and potassium t-butoxide (180 mg, 1.6 mmol, 2 equiv.), and stirred at 80°C for 2h. After the reaction was complete as indicated by LCMS, the resultant was concentrated, and the crude product was separated and purified by preparative HPLC (C18, 10 mmol/L aqueous NH₄HCO₃, acetonitrile) and then lyophilized to obtain the title compound (35 mg, yield: 13%). LCMS (ESI) [M+H]+ =337.2.

### Step VII: Synthesis of 1-(4-((6-((3R,4R)-4-(3,4-dihydro-4,4-dideuterium-isoquinolin-2(1H)-yl)-3-hydroxypiperidine-1-carbonyl)-2-isobutoxypyrimidin-4-yl)amino)-piperidin-1 -yl)ethan-1 -one:

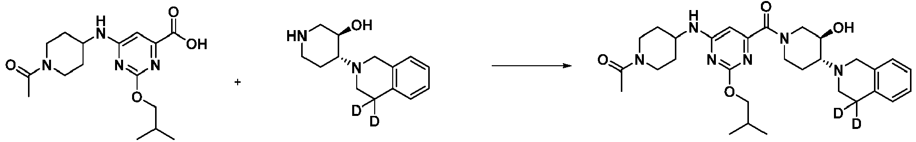

6-((1-Acetylpiperidin-4-yl)amino)-2-isobutoxypyrimidine-4-carboxylic acid (150 mg, 0.45 mmol, 1 equiv.), (3R, 4R)-4-(4,4-dideuterium-3,4-dihydroisoquinolin-2(1H)-yl)-piperidin-3-ol (104 mg, 0.45 mmol, 1 equiv.), HATU (203 mg, 0.54 mmol, 1.2 equiv.) and trimethylamine (135 mg, 1.34 mmol, 3 equiv.) were dissolved in DMF (5 mL), and the reaction mixture was stirred at room temperature for 2 h. The reaction was complete as indicated by LCMS, and extracted by adding water and ethyl acetate. The organic phase was dried and concentrated, and purified by a preparative plate (dichloromethane: methanol=10: 1), and the resultant crude product was purified by prep-HPLC to obtain the title compound (57 mg, yield: 23.1%). LCMS (ESI) [M+H]⁺ = 553.45; ¹H NMR (400 MHz, CD₃OD) δ 7.34 - 7.22 (m, 4H), 6.69 (dd, J = 25.6, 6.0 Hz, 1H), 4.68 (dd, J = 62.3, 27.8 Hz, 1H), 4.46 - 4.28 (m, 4H), 4.23 - 3.94 (m, 3H), 3.90 - 3.78 (m, 1H), 3.74 - 3.45 (m, 2H), 3.37 (ddd, J = 11.7, 5.6, 3.1 Hz, 2H), 3.20 - 3.12 (m, 1H), 3.05 - 2.76 (m, 2H), 2.35 - 1.95 (m, 8H), 1.74 - 1.50 (m, 2H), 1.38 - 1.26 (m, 1H), 1.10 - 1.03 (m, 6H).

### Example 141

### Synthesis of 1-(4-((6-((3R,4R)-4-(3,4-dihydro-4,4-dideuterium-isoquinolin-2(1H)-yl)-3-hydroxypiperidine-1-carbonyl)-2-(pentane-3-oxy)pyrimidin-4-yl)amino)piperidin-1-yl )- ethan-1-one:

### Step I: 6-((1-acetylpiperidin-4-yl)amino)-2-(pentane-3-oxy)pyrimidine-4-carboxylic acid:

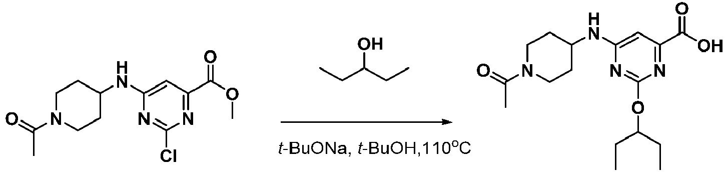

Methyl 6-((1-acetylpiperidin-4-yl)amino)-2-chloropyrimidine-4-carboxylate (300 mg, 0.96 mmol, 1.0 equiv.) and 3-pentanol (845 mg, 9.6 mmol, 10 equiv.) were dissolved in anhydrous t-butanol (5 mL), followed by addition of sodium t-butoxide (369 mg, 3.84 mmol, 4.0 equiv.), and a reaction was stirred at 100°C for 16 h. After the reaction was complete as indicated by LCMS, the reaction mixture was adjusted to pH 4 with 1M diluted hydrochloric acid, and the solvent was rotary evaporated to obtain a crude product (700 mg). LCMS: [M+H]⁺ =351.2.

### Step II: synthesis of 1-(4-((6-((3R,4R)-4-(3,4-dihydro-4,4-dideuterium-isoquinolin-2(1H)-yl)-3-hydroxypiperidine-1-carbonyl)-2-(pentane-3-oxy)pyrimidin-4-yl)ami no)-piperidin-1-yl)-ethan-1-one:

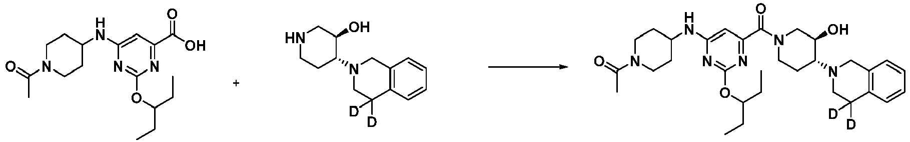

6-((1-Acetylpiperidin-4-yl)amino)-2-(pentane-3-oxy)pyrimidine-4-carboxylic acid (100 mg, 0.28 mmol, 1.0 equiv.) and (3R, 4R)-4-(4,4-dideuterium-3,4-dihydroisoquinolin-2(1H)-yl)piperidin-3-ol (67 mg, 0.28 mmol, 1.0 equiv.) were dissolved in N,N-dimethylformamide (3 mL), followed by addition of triethylamine (141 mg, 1.40 mmol, 5 equiv.) and 2-(7-azabenzotriazole)-N,N,N',N'-tetramethyluronium hexafluorophosphate (159 mg, 0.42 mmol, 1.5 eq.) to the reaction mixture, and the reaction mixture was stirred at 25°C for 2 h. The reaction was quenched by adding water (10 mL) and extracted with ethyl acetate (3 ^{∗} 15 mL), and the organic phases were combined and washed with saturated sodium chloride (3 ^{∗}20 mL), dried over anhydrous sodium sulfate, filtrated, and concentrated to obtain a crude product. The crude product was separated and purified by thin layer chromatography (dichloromethane: methanol = 12:1) to obtain the target compound (65 mg), and prepared by reverse phase prep-HPLC to obtain the title compound (53 mg, yield: 31.4%). LCMS (ESI) [M+H]⁺ = 567.50; ¹H NMR (400 MHz, CD₃OD): δ 7.29 (dd, J = 8.7, 6.7 Hz, 3H), 7.22 (d, J = 6.0 Hz, 1H), 6.48 (s, 1H), 5.17 (d, J = 6.0 Hz, 1H), 4.77 - 4.60 (m, 2H), 4.51 - 4.20 (m, 2H), 4.16 - 3.90 (m, 3H), 3.85 - 3.44 (m, 2H), 3.14 (d, J = 25.1 Hz, 3H), 2.85 (d, J = 55.8 Hz, 3H), 2.21 (s, 1H), 2.11 (s, 6H), 1.87 - 1.70 (m, 4H), 1.61 (s, 2H), 0.99 (s, 6H).

### Example 142

### Preparation of trans-1-(4-((6-(4-(3,4-dihydroisoquinolin-2(1H)-yl)-3-hydroxypiperidine-1-carbonyl)pyrimidin-4-yl)oxy)piperidin-1-yl)ethan-1-one:

### Step I: preparation of the compound 6-chloropyrimidine-4-carbonyl chloride:

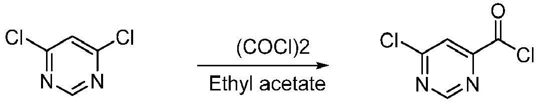

The compound 4,6-dichloropyrimidine (570 mg, 3.83 mmol, 1.0 eq) was dissolved in EA (18 mL), followed by addition of oxalyl chloride (2.43 g, 19.13 mmol, 5.0 eq) and *N*,*N*-dimethylformamide (1.8 mL), and a reaction was stirred at 85°C for 2 h. After the reaction was complete as monitored by a TLC and LCMS, the reaction mixture was rapidly evaporated on a rotary evaporator to dryness, and sealed to be directly used in the following step.

### Step II: preparation of t-butyl trans-4-(3,4-dihydroisoquinolin-2(1H)-yl)-3-hydroxypiperidine-1-carboxylate:

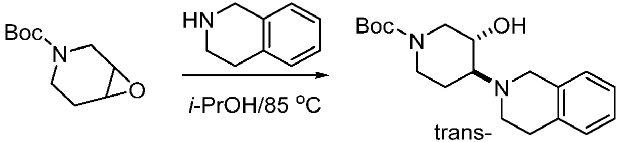

The compound t-butyl 7-oxa-3-azabicyclo[4.1.0]heptane-3-carboxylate (2.50 g, 12.56 mmol, 1.0 eq) was dissolved in *i*-PrOH (isopropanol, 63 mL), followed by addition of the compound 1,2,3,4-tetrahydroisoquinoline (1.67 g, 12.56 mmol, 1.0 eq), and a reaction was stirred under nitrogen at 85°C for 18h. After the reaction was complete as monitored by a TLCand LCMS, the solvent was rotary evaporated, water (200 mL) was added, and the mixture was extracted with dichloromethone (three time in total, 200 mL each), dried over anhydrous sodium sulfate, filtrated, and rotary evaporated. Regioisomers were present in this step, including the compound t-butyl trans-4-(3,4-dihydroisoquinolin-2(1*H*)-yl)-3-hydroxypiperidin-1-carboxylate and the compound t-butyl trans-3-(3,4-dihydroisoquinolin-2(1H)-yl)-4-hydroxypiperidine-1-carboxylate in a mixed crude product (3.40 g, yield: 81.5%). The crude product was seperated 2-3 time by chromatography (silica gel, ethyl acetate:petroleum ether = 15: 85) and purified to obtain the title compound (1.7 g, yield: 41%). LCMS (ESI) [M+H]⁺= 333.3; ¹H NMR (400 MHz, CDCl₃) δ 7.20 - 7.08 (m, 3H), 7.05 - 6.98 (m, 1H), 4.59 - 4.17 (m, 2H), 3.94 (d, *J* = 14.6 Hz, 1H), 3.68 (d, *J* = 14.5 Hz, 2H), 3.54 (td, *J* = 10.0, 5.0 Hz, 1H), 3.03 (dt, *J* = 10.9, 5.3 Hz, 1H), 2.91 (t, *J* = 5.6 Hz, 2H), 2.80 - 2.47 (m, 4H), 1.82 (dd, *J* = 12.7, 2.5 Hz, 1H), 1.59 - 1.38 (m, 10H).

### Step III: preparation of trans-4-(3,4-dihydroisoquinolin-2(1H)-yl)piperidin-3-ol:

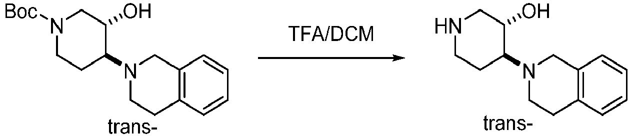

The compound t-butyl trans-4-(3,4-dihydroisoquinolin-2(1*H*)-yl)-3-hydroxypiperidine-1-carboxylate (1.00 g, 3.01 mmol, 1.0 eq) was dissolved in DCM (dichloromethane) (15mL) and TFA (trifluoroacetic acid) (3.75 mL), and a reaction was stirred at room temperature (20-25°C) under stirring for 2 h. After the reaction was complete as monitored by a TLC plate and LCMS, the reaction mixture was evaporated on a rotary evaporator, and 1,2-dichloroethane was added three times to remove extra TFA. Upon evaporation to dryness, the product was sealed and stored, and directed used in Step IV LCMS (ESI) [M+H]⁺ = 233.2.

### Step IV: preparation of trans-(6-chloropyrimidin-4-yl)(4-(3,4-dihydroisoquinolin-2(1H)-yl)-3-hydroxypiperidin-1-yl)ketone:

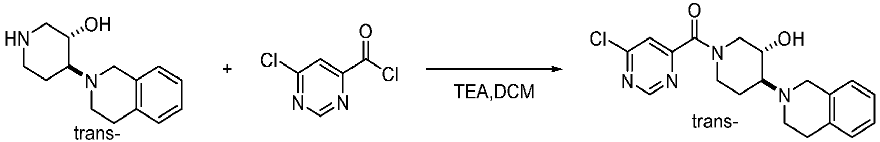

The compound trans-4-(3,4-dihydroisoquinolin-2(1*H*)-yl)piperidin-3-ol (570 mg, 3.23 mmol, 1.0 eq) dissolved in DCM (dichloromethane) (8 ml) and TEA (triethylamine) (653 mg, 6.46 mmol, 2.0 eq), and the compound 6-chloropyrimidine-4-carbonyl chloride dissolved in dichloromethane (8 mL) were slowly added under nitrogen to the reaction mixture of trans-4-(3,4-dihydroisoquinolin-2(1*H*)-yl)piperidin-3-ol. The reaction mixture was allowed to react on ice bath for 2 h, and then stirred for 1 h after being gradually warmed to room temperature (20-25°C). After the reaction was complete as monitored by a TLC and LCMS, the reaction mixture was mixed with 100 ml water, extracted with ethyl acetate, and dried over anhydrous sodium sulfate. The organic phase was concentrated, and seperated and purified by reverse phase HPLC (C18, 0.08% aqueous NH₄HCO₃, acetonitrile) to obtain the title compound (900 mg, yield: 56.3%). LCMS (ESI) [M+H]⁺ = 373.

### Step V: preparation of trans-1-(4-((6-(4-(3,4-dihydroisoquinolin-2(1H)-yl)-3-hydroxypiperidine-1-carbonyl)pyrimidin-4-yl)oxy)piperidin-1-yl)ethan-1-one:

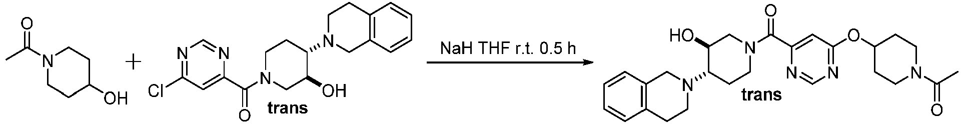

The compound 1-(4-hydroxypiperidin-1-yl)ethan-1-one (77 mg, 0.536 mmol, 2.0 equiv.) was added to a solution of NaH (60% by mass) (35.7 mg, 0.536 mmol, 2.0 equiv.) in THF (1.4 mL), and stirred at 0°C under nitrogen for 0.5 h. Trans-1-(6-chloropyrimidin-4-yl)-4-(3,4-dihydroisoquinolin-2(1H)-yl)piperidin-3-ol (100 mg, 0.268 mmol, 1.0 equiv.) was then added, and the reaction continued at 16°C for 0.5 h. The reaction was complete as monitored by LCMS. 10mL saturated ammonium chloride was added dropwise at 0°C, and the mixture was extracted once with EA (20 mL). The organic phase was washed once with saturated sodium chloride (20 mL), dried over anhydrous sodium sulfate, and filtrated, and the reaction mixture was concentrated. The crude product was separated and purified by reverse phase column (Cis, 10mmol/L aqueous ammonium bicarbonate/ acetonitrile) to obtain the title compound (2.32 mg, yield: 1.5%). LCMS(ESI): m/z = 480.5; ¹H NMR (400 MHz, CDCl₃) δ 8.76-8.74 (m, 1H), 7.21-7.10 (m, 3H), 7.06-7.01 (m, 1H), 7.00-6.93 (m, 1H), 5.49-5.37 (m, 1H), 5.09-4.76 (m, 1H), 4.18-4.06 (m, 1H), 4.03-3.92 (m, 2H), 3.79-3.68 (m, 3H), 3.56-3.39 (m, 2H), 3.13-2.92 (m, 4H), 2.83-2.72 (m, 2H), 2.14 (s, 3H), 2.10-2.02 (m, 2H), 1.86-1.67 (m, 6H).

### Example 143

### Preparation of (3-(4-((6-(trans-4-(3,4-dihydroisoquinolin-2(1H)-yl)-3-hydroxypiperidine -1-carbonyl)pyrimidin-4-yl)amino)piperidin-1-yl)-3-carbonylpropionitrile:

### Step I: preparation of methyl 6-((1-(t-butoxycarbonyl)piperidin-4-yl)amino)pyrimidine-4-carboxylate:

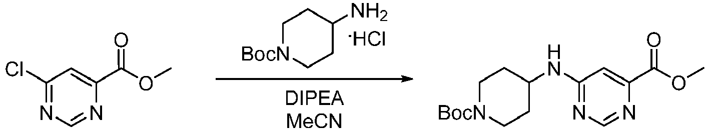

Methyl 6-chloro-pyrimidine-4-carboxylate (1 g, 5.80 mmol, 1.0 equiv.), 1-t-butoxycarbonyl-4-aminopiperidine hydrochloride (1.65 g, 6.95 mmol, 1.2 equiv.) and DIPEA (N,N-diisopropylethylamine) (3.00 g, 23.18 mmol, 4.0 equiv.) were dissolved in MeCN (acetonitrile) (30 mL), and a reaction was stirred at 90°C for 15 h. The reaction mixture was concentrated, and the crude product was separated and purified by flash chromatography (silica gel, PE: EA = 3: 2) to obtain the target compound (1.82 g, yield: 93.4%). LCMS (ESI) [M+H]⁺ = 337.31;

### Step II: preparation of 6-((1-(t-butoxycarbonyl)piperidin-4-yl)amino)pyrimidine-4-carboxylic acid:

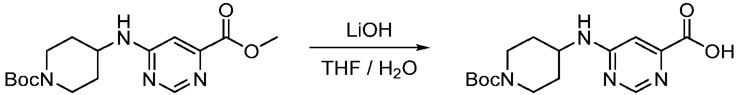

Methyl 6-((1-(t-butoxycarbonyl)piperidin-4-yl)amino)pyrimidine-4-carboxylate (1.8 g, 5.35 mmol, 1.0 equiv.) and LiOH (lithium hydroxide) (0.26 g, 10.70 mmol, 2.0 equiv.) were dissolved in THF (tetrahydrofuran) (27 mL) and H₂O (water) (9 mL), and a reaction was stirred at 20°C for 1 h. The reaction mixture was concentrated, diluted with dichloromethane/methanol (3:1), and filtrated, and the filtrate was concentrated to obtain a crude product of the target compound (2.14 g). LCMS (ESI) [M+H]⁺ = 323.2;

### Step III: preparation of t-butyl trans-4-((6-(4-(3,4-dihydroisoquinolin-2(1H)-yl)-3-hydroxypiperidine-1-carbonyl)pyrimidin-4-yl)amino)piperidine-1-carboxylate:

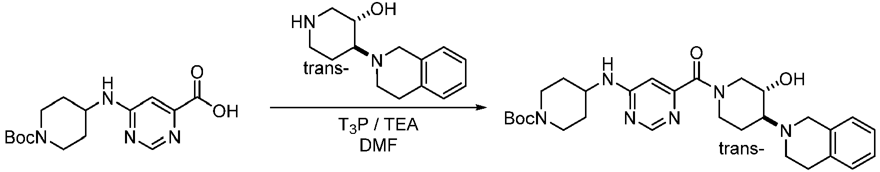

6-((1-(t-butoxycarbonyl)piperidin-4-yl)amino)pyrimidine-4-carboxylic acid (2.1 g, 6.51 mmol, 1.0 equiv.), trans-4-(3,4-dihydroisoquinolin-2(1*H*)-yl)piperidin-3-ol (2.09 g, 8.99 mmol, 1.38 equiv.), T₃P (1-N-propylphosphoric anhydride) in ethyl acetate (50% by mass) (8.29 g, 13.03 mmol, 2.0 equiv.) and TEA (triethylamine) (3.30 g, 32.57 mmol, 5.0 equiv.) were dissolved in DMF (*N,N*-dimethylformamide) (30 mL), and a reaction was stirred at 20°C for 17 h. The reaction mixture was extracted with ethyl acetate, the organic phase was concentrated, and the crude product was separated and purified by flash chromatography (silica gel, DCM: MeOH = 20: 1) to obtain a crude product of the title compound (0.98 g, yield: 28.0%). 30 mg of the crude product of the title compound was separated and purified by Prep-HPLC (C18, 10 mmol/L aqueous NH₄HCO₃, acetonitrile) to obtain the target compound (19.4 mg). LCMS (ESI) [M+H]⁺ = 537.6; ¹H NMR (400 MHz, CDCl₃) δ 8.57 - 8.51 (m, 1H), 7.19 - 7.08 (m, 3H), 7.06 - 6.99 (m, 1H), 6.63 - 6.54 (m, 1H), 5.17 - 4.66 (m, 2H), 4.32 - 3.84 (m, 6H), 3.77 - 3.62 (m, 2H), 3.17 - 2.80 (m, 7H), 2.79 - 2.54 (m, 2H), 2.08 - 1.81 (m, 3H), 1.76 - 1.65 (m, 1H), 1.47 (s, 9H), 1.44 - 1.35 (m, 2H).

### Step IV: preparation of trans-(4-(3,4-dihydroisoquinolin-2(1H)-yl)-3-hydroxy-piperidin-1-yl)-(6-(piperidin-4-ylamino)pyrimidin-4-yl)ketone:

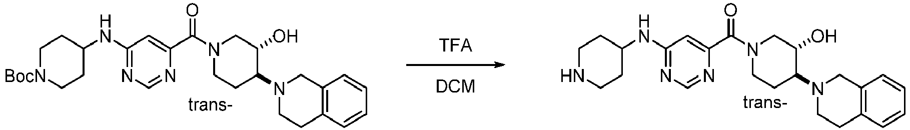

T-butyl trans-4-((6-(4-(3,4-dihydroisoquinolin-2(1H)-yl)-3-hydroxypiperidine-1-carbonyl)pyrimidin-4-yl)amino)piperidine-1-carboxylate (30 mg, 0.06 mmol, 1.0 equiv.) was dissolved in DCM (dichloromethane) (0.4 mL), followed by addition of TFA (trifluoroacetic acid) (0.1 mL), and a reaction was stirred at 20°C for 0.5 h. The reaction mixture was quenched with saturated aqueous solution of sodium bicarbonate, and concentrated, and the crude product was separated and purified by preparative HPLC (Cis, 10 mmol/L aqueous NH₄HCO₃, acetonitrile) to obtain the title compound (17.42 mg, yield: 71.4%). LCMS (ESI) [M+H]⁺ = 437.5; ¹H NMR (400 MHz, DMSO-d₆) δ 8.46 - 8.32 (m, 1H), 7.61 (t, *J* = 7.7 Hz, 1H), 7.13 - 7.01 (m, 4H), 6.51 (s, 1H), 4.79 (dd, *J* = 35.1, 3.9 Hz, 1H), 4.40 (dd, *J* = 56.4, 12.7 Hz, 1H), 3.93 (m, 1H), 3.87 - 3.56 (m, 5H), 2.98 (d, *J* = 12.5 Hz, 2H), 2.93 - 2.87 (m, 1H), 2.85 - 2.75 (m, 4H), 2.69 - 2.53 (m, 4H), 1.92 - 1.69 (m, 3H), 1.57 - 1.43 (m, 1H), 1.41 - 1.27 (m, 2H).

### Step V: preparation of 2-cyanoacetyl chloride:

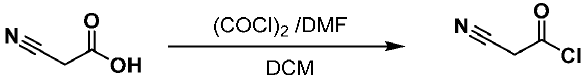

Cyanoacetic acid (1 g, 11.8 mmol, 1.0 equiv.) was dissolved in DCM (100 mL), followed by addition of (COCl)₂ (1.64 g, 12.9 mmol, 1.1 equiv.) and DMF (8 mg, 0.1 mmol, 0.01 equiv.) at 0°C, and stirred under nitrogen at 25°C for 1 h. The reaction was complete as indicated by TLC, and rotary evaporated to dryness to obtain a crude product of 2-cyanoacetyl chloride (1.1 g), which was directly used in the reaction of the following step.

### Step VI: preparation of (3-(4-((6-(trans-4-(3,4-dihydroisoquinolin-2(1H)-yl)-3-hydroxypiperidine-1-carbonyl)pyrimi din-4-yl)amino)piperidin-1-yl)-3-carbonylpropionitrile:

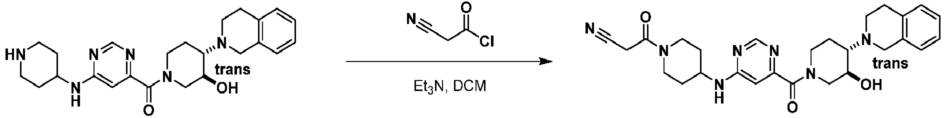

The crude product of 2-cyanoacetyl chloride (5 mg, 0.046 mmol, 1.0 equiv.) was dissolved in DCM (2mL), added dropwise to a solution of trans-4-(3,4-dihydroisoquinolin-2(1H)-yl)-3-hydroxypiperidin-1-yl)(6-(piperidin-4-ylamino)pyrimidin-4-yl)ketone (20 mg, 0.046 mmol, 1.0 equiv.) in DCM (1.5 mL) at 0°C, followed by addition of triethylamine (9 mg, 0.09 mmol, 2.0 equiv.), and stirred at 0°C for 1 h. After the reaction was complete as indicated by LCMS, the mixture was concentrated, and the crude product was separated and purified by reverse phase column (C₁₈, 10 mmol/L aqueous ammonium bicarbonate/acetonitrile) to obtain the target compound (5.8 mg, yield: 25.4%). LCMS: [M+H]⁺ = 504.56; ¹H NMR (400 MHz, CDCl₃) δ 8.60 - 8.45 (m, 1H), 7.20 - 7.09 (m, 3H), 7.08 - 6.99 (m, 1H), 6.71 - 6.58 (m, 1H), 6.02 - 5.60 (m, 1H), 5.07 - 4.65 (m, 1H), 4.47 (d, J = 13.3 Hz, 1H), 4.23 - 4.10 (m, 2H), 4.01 - 3.93 (m, 1H), 3.79 - 3.69 (m, 3H), 3.63 - 3.46 (m, 2H), 3.37 - 3.26 (m, 1H), 3.11 - 2.99 (m, 2H), 2.97 - 2.88 (m, 3H), 2.80 - 2.66 (m, 2H), 2.20 - 2.04 (m, 2H), 2.01 - 1.78 (m, 3H), 1.76 - 1.63 (m, 1H), 1.61 - 1.41 (m, 2H).

### Example 144

### Preparation of 1-(4-((6-(trans-4-(3,4-dihydroisoquinolin-2(1H)-yl)-3-hydroxypiperidine -1- carbonyl)-2-morpholinopyrimidin-4-yl)amino)piperidin-1-yl)ethan-1-one:

### Step I: preparation of methyl 6-((1-acetylpiperidin-4-yl)amino)-2-chloropyrimidine-4-carboxylate:

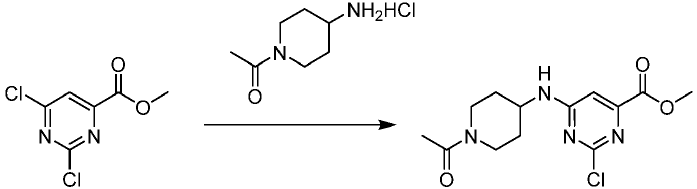

Methyl 2,6-dichloropyrimidine-4-carboxylate (2 g, 9.66 mmol), 1-acetylpiperidin-4-amine hydrochloride (1.9 g, 10.63 mmol) and N,N-diisopropylethylamine (4.99 g, 38.65mmol) were dissolved in acetonitrile (50 ml), and stirred at 25°C (2 h). The reaction mixture was extracted three times with ethyl acetate (50 mL each), and the ethyl acetate phases were combined, washed once with 20 mL water and once with 20 mL saturated sodium chloride, dried over anhydrous sodium sulfate for 10 min, and filtrated. The crude product was separated and purified on a column with DCM:MeOH=100:1 to obtain methyl 6-((1-acetylpiperidin-4-yl)amino)-2-chloropyrimidine-4-carboxylate (2.8 g, yield: 92%). LCMS (ESI) [M+H]⁺ =313.2.

### Step II: preparation of 6-((1-acetylpiperidin-4-yl)amino)-2-chloropyrimidine-4-carboxylic acid:

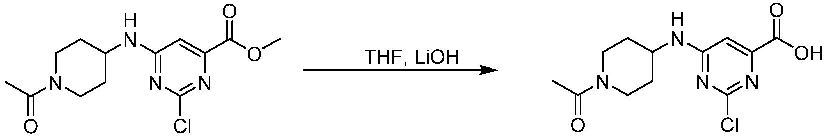

Methyl 6-((1-acetylpiperidin-4-yl)amino)-2-methylpyrimidine-4-carboxylate (200 mg, 0.639 mmol) was dissolved in THF (4 mL), followed by addition of an aqueous solution of 2M (molar concentration) lithium hydroxide (0.64 mL, 1.28 mmol, 2equiv.), and the reaction mixture was stirred at room temperature (25°C) for 2 h. The reaction was complete as indicated by TLC, adjusted to pH=6-7 with 1M (molar concentration) HCl, and directly rotary evaporated to obtain a crude product of 6-((1-acetylpiperidin-4-yl)amino)-2-chloropyrimidine-4-carboxylic acid (400 mg). LCMS (ESI) [M+H]⁺ =299.2.

### Step III: preparation of 1-(4-((2-((3H-[1,2,3]triazolo[4,5-b]pyridin-3-yl)oxy)-6-(trans-4-(3,4-dihydroisoquinolin-2(1H)-yl)-3-hydroxypiperidine-1-carbonyl)pyrimidin-4-yl) amino)piperidin-1-yl)ethan-1-one:

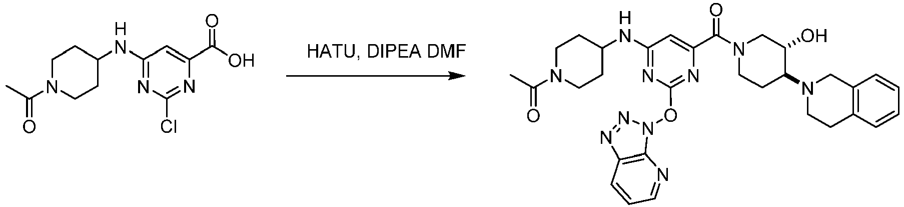

6-((1-acetylpiperidin-4-yl)amino)-2-chloropyrimidine-4-carboxylic acid (380 mg, 1.27 mmol), HATU (2-(7-azabenzotriazol-1-yl)-N,N,N',N'-tetramethyluronium hexafluorophosphate) (725.5 mg, 1.91 mmol), trans-4-(3,4-dihydroisoquinolin-2(1H)-yl) piperidin-3-ol (265 mg, 1.14 mmol) and DIPEA (N,N-diisopropylethylamine) (0.822 g, 6.36 mmol) were dissolved in DMF (N,N-dimethylformamide) (5 mL), and the reaction mixture was stirred at room temperature (25°C) for 2 h. The reaction was complete as indicated by LCMS, and extracted three times with ethyl acetate (20 mL each). The ethyl acetate phases were combined, washed once with 10 mL water and once with 10 mL saturated sodium chloride, dried over anhydrous sodium sulfate for 10 min, and filtrated. The crude product was separated and purified on a column with DCM:MeOH=20:1 to obtain the title compound (200 mg). LCMS (ESI) [M+H]⁺ =613.3.

### Step IV: 1-(4-((6-(trans-4-(3,4-dihydroisoquinolin-2(1H)-yl)-3-hydroxypiperidine-1-carbonyl)-2-morpholinopyrimidin-4-yl)amino)piperidin-1-yl)ethan-1-one:

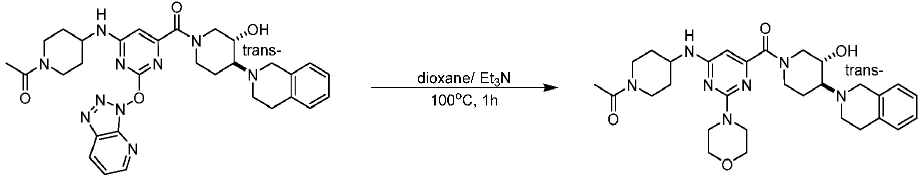

1-(4-((2-((3H-[1,2,3]triazolo[4,5-b]pyridin-3-yl)oxy)-6-(trans-4-(3,4-dihydroisoquinolin -2(1H)-yl)-3-hydroxypiperidine-1-carbonyl)pyrimidin-4-yl)amino)piperidin-1-yl)ethan-1-one (40 mg, 0. 64 mmol) was dissolved in 1,4-dioxane (1.5 mL), followed by addition of Et₃N (19.2 mg, 0.192 mmol) and morpholine (16 mg, 0.192 mmol), and stirred at 100°C for 1 h. The reaction was complete as indicated by LCMS, and extracted three times with EA (10 mL each). The ethyl acetate phases were combined, washed once with 10 mL water and once with 10 mL saturated sodium chloride solution, dried over anhydrous sodium sulfate, and filtrated, followed by purification of the crude product by reverse phase preparative HPLC (C18, 10 mmol/L aqueous NH₄HCO₃, acetonitrile) to obtain the title compound (25.59 mg, yield: 69%). LCMS (ESI) [M+H]⁺ =564.4; ¹H NMR (400 MHz, DMSO-d₆) δ 7.25 (s, 1H), 7.13 - 6.99 (m, 4H), 5.86 (s, 1H), 4.83 - 4.63 (m, 1H), 4.52 - 4.28 (m, 1H), 4.19 (d, J = 12.6 Hz, 1H), 4.02 (s, 1H), 3.89 - 3.70 (m, 4H), 3.70 - 3.51 (m, 9H), 3.17 (t, J = 12.4 Hz, 1H), 3.02 - 2.87 (m, 1.5H), 2.87 - 2.69 (m, 5H), 2.69 - 2.53 (m, 1.5H), 2.05 - 1.97 (m, 3H), 1.97 - 1.69 (m, 3H), 1.56 - 1.44 (m, 1H), 1.44 - 1.32 (m, 1H), 1.32 - 1.18 (m, 1H).

### Example 145

### Preparation of trans-1-(4-((6-((3R,4R)-4-(3,4-dihydroisoquinolin-2(1H)-yl)-3-hydroxy-piperidine-1-carbonyl)-2-(1H-pyrazol-4-yl)pyrimidin-4-yl)amino)piperidin-1-yl)ethan-1-one:

### Step I: preparation of 1-(4-((2-chloro-6-((trans)-4-(3,4-dihydroisoquinolin-2(1H)-yl)-3-hydroxypiperidine-1 -carbonyl)pyrimidin-4-yl)amino)piperidin-1 -yl)ethan-1 -one:

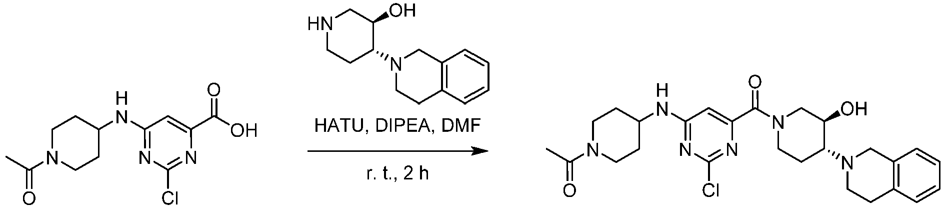

6-((1-acetylpiperidin-4-yl)amino)-2-chloropyrimidine-4-carboxylic acid hydrochloride (500 mg, 1.67 mmol), HATU (636.4 mg, 1.67 mmol), trans-4-(3,4-dihydroisoquinolin-2(1H)-yl)piperidin-3-ol (388.8 mg, 1.67 mmol) and DIPEA (1.08 g, 8.37 mmol) were dissolved in DMF (6.5 mL), and the reaction mixture was stirred at room temperature (25-30°C) for 2 h. The reaction was complete as indicated by LCMS, and extracted three times with ethyl acetate (20 mL each). The ethyl acetate phases were combined, washed once with 10 mL water and once with 10 mL saturated sodium chloride, dried over anhydrous sodium sulfate for 10 min, and filtrated. The crude product was separated and purified on a column with DCM:MeOH=20:1 to obtain the title compound (200 mg) and a crude product of the title compound (550 mg). LCMS (ESI) [M+H]⁺ =513.2.

### Step II: preparation of trans-1-(4-((6-((3R,4R)-4-(3,4-dihydroisoquinolin-2(1H)-yl)-3-hydroxypiperidine-1-carbonyl)-2-(1H-pyrazol-4-yl)pyrimidin-4-yl)amino)piperidin-1-yl)-ethan-1-one:

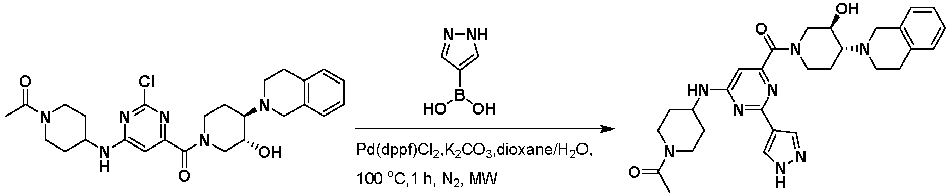

At room temperature (25°C), the raw material 1-(4-((2-chloro-6-((3S,4S)-4-(3,4-dihydroisoquinolin-2(1H)-yl)-3-hydroxypiperidine-1-carbonyl)pyrimidin-4-yl)amino)piperidi n-1-yl)ethan-1-one (100 mg, 0.195 mmol, 1.0 equiv.) was added to dioxane (2.0 mL) and water (0.5 mL), followed by addition of (1H-pyrazol-4-yl)boronic acid (21.8 mg, 0.39 mmol, 2.0 equiv.), [1,1'-bis(diphenylphosphino)ferrocene]palladium dichloride (14.2 mg, 0.0195mmol, 0.1 equiv.) and potassium carbonate (53.9 mg, 0.39mmol, 2.0 equiv.) under nitrogen, and stirred under a microwave condition at 100°C for 1h. After the reaction was complete as indicated by LCMS, the crude product was separated and purified by reverse phase Prep-HPLC (C18, 10 mmol/L NH₄HCO₃ in water, MeCN), and lyophilized to obtain the title compound (28.01 mg, yield: 26.2%). LCMS (ESI) [M+H]+ = 545.7; ¹H NMR (400 MHz, DMSO-d6) δ 13.08 (s, 1H), 8.21-8.01 (m, 2H), 7.53-7.49 (m, 1H), 7.11-7.04 (m, 4H), 6.34-6.32 (m, 1H), 4.83-4.76 (m, 1H), 4.51-4.15 (m, 3H), 3.88-3.79 (m, 4H), 3.71-3.65 (m, 1H), 3.28-3.21 (m, 1H), 3.15-2.75 (m, 7H), 2.68-2.57 (m, 1H) 2.07-1.74 (m, 6H), 1.61-1.17 (m, 3H).

### Example 146

### Preparation of trans-(4-(3,4-dihydroisoquinolin-2(1H)-yl)-3-hydroxypiperidin-1-yl)(6-(((1 S,3 S)-3-methoxycyclobutyl)amino)-2-phenylpyrimidin-4-yl)ketone:

### Step I: preparation of methyl 2-chloro-6-(((1S,3S)-3-methoxycyclobutyl)amino) pyrimidine-4-carboxylate:

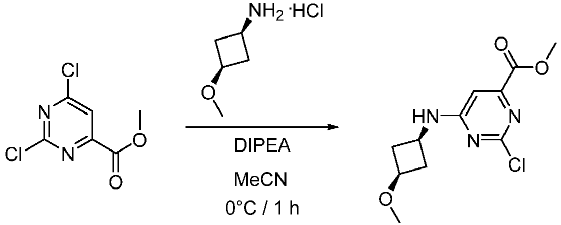

Methyl 2,6-dichloropyrimidine-4-carboxylate (200 mg, 0.97 mmol, 1.0 equiv.), cis-3-methoxycyclobutylamine hydrochloride (133 mg, 0.97 mmol, 1.0 equiv.) and DIPEA (375 mg, 2.90 mmol, 3.0 equiv.) were dissolved in MeCN (5 mL), and a reaction was stirred at 0°C for 1 h. The reaction mixture was concentrated, and the crude product was separated and purified by flash chromatography (Silica gel, PE: EA = 3: 1) to obtain the target compound (233 mg, yield: 88.8%). LCMS (ESI) [M+H]⁺ = 272.0.

### Step II: preparation of 6-(((1S,3S)-3-methoxycyclobutyl)amino)-2-phenylpyrimidine-4-carboxylic acid:

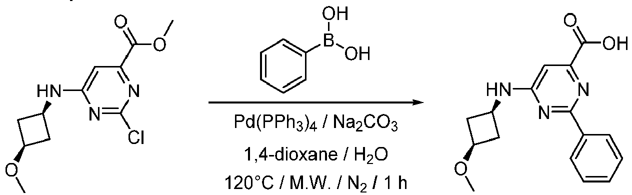

Methyl 2-chloro-6-(((1S,3S)-3-methoxycyclobutyl)amino)pyrimidine-4-carboxylate (100 mg, 0.37 mmol, 1.0 equiv.), phenylboronic acid (135 mg, 1.10 mmol, 3.0 equiv.), tetra(triphenylphosphine)palladium (43 mg, 0.04 mmol, 0.1 equiv.) and sodium carbonate (59 mg, 0.55 mmol, 1.5 equiv.) were dissolved in 1,4-dioxane (2 mL) and water (0.5 mL), and a reaction was stirred at 120°C under microwave with nitrogen for 1 h. The reaction mixture was extracted with ethyl acetate, and the aqueous phase was adjusted to pH 2-3 with diluted hydrochloric acid, and concentrated to obtain a crude product of the target compound (212 mg). LCMS (ESI) [M+H]⁺ = 300.2.

### Step III: preparation of trans-(4-(3,4-dihydroisoquinolin-2(1H)-yl)-3-hydroxy-piperidin-1-yl)(6-(((1S,3S)-3-methoxycyclobutyl)amino)-2-phenylpyrimidin-4-yl)ketone:

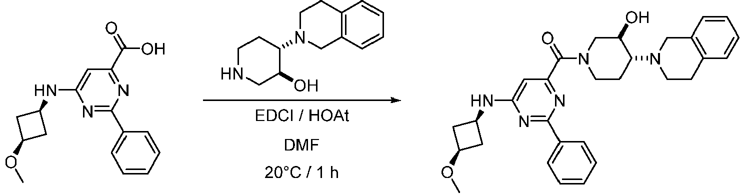

6-(((1S,3S)-3-methoxycyclobutyl)amino)-2-phenylpyrimidine-4-carboxylic acid (170 mg, 0.30 mmol, 1.0 equiv.), trans-4-(3,4-dihydroisoquinolin-2(1H)-yl)piperidin-3-ol (69 mg, 0.30 mmol, 1.0 equiv.), EDCI (85 mg, 0.44 mmol, 1.5 equiv.) and HOAt (60 mg, 0.44 mmol, 1.5 equiv.) were dissolved in DMF (3 mL), and a reaction was stirred at 20°C for 1 h. The reaction mixture was concentrated, the crude product was purified by reverse phase preparative HPLC (C18, 10 mmol/L aqueous NH₄HCO₃, acetonitrile) to obtain the target compound (37.71 mg, yield 24.9%). LCMS (ESI) [M+H]⁺ = 514.7; ¹H NMR (400 MHz, CDCl₃) δ 8.39 - 8.31 (m, 2H), 7.48 - 7.41 (m, 3H), 7.19 - 7.08 (m, 3H), 7.07 - 7.01 (m, 1H), 6.56 - 6.47 (m, 1H), 5.35 (s, 1H), 5.13 - 4.70 (m, 1H), 4.51 - 4.34 (m, 1H), 4.03 - 3.68 (m, 5H), 3.31 - 3.25 (m, 3H), 3.14 - 2.98 (m, 2H), 2.98 - 2.84 (m, 4H), 2.79 - 2.61 (m, 2H), 2.08 - 1.84 (m, 3H), 1.86 - 1.67 (m, 3H).

### Example 147

### Preparation of trans-1-(6-((2,2-difluoroethyl)amino)-2-(1H-imidazol-1-yl)pyrimidin-4-yl) (4-(3,4-dihydroisoquinolin-2(1H)-yl)-3-hydroxypiperidin-1-yl)ketone:

### Step I: preparation of methyl 2-chloro-6-((2,2-difluoroethyl)amino)pyrimidine-4-carboxylate:

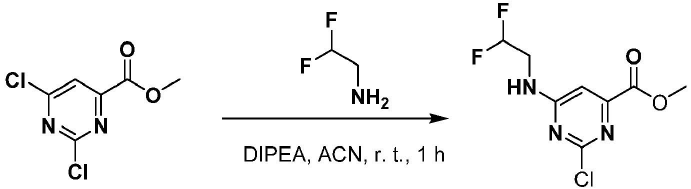

The raw materials methyl 2,6-dichloropyrimidine-4-carboxylate (5 g, 24.2 mmol, 1 equiv.) and 2,2-difluoroethane-1-amine (2.06 g, 25.4 mmol, 1.05 equiv.) were dissolved in acetonitrile (30 mL), followed by addition of DIPEA (9.4 g, 72.6 mmol, 3 equiv.), and the reaction mixture was stirred at 25°C for 5 h. The reaction was complete as indicated by LCMS. The reaction mixture was quenched by adding water, and then extracted with ethyl acetate. The organic phase was combined and washed with saturated sodium chloride, dried over anhydrous sodium sulfate, and then filtrated and concentrated to obtain a crude product, which was separated and purified by flash chromatography (Silica gel, petroleum ether: ethyl acetate = 0-15%) to obtain the title compound (5.6 g, yield: 92%). LCMS (ESI) [M+1]⁺ =252.0.

### Step II: preparation of 2-chloro-6-((2,2-difluoroethyl)amino)pyrimidine-4-carboxylic acid:

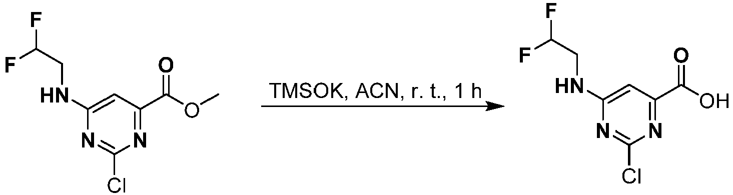

The raw material methyl 2-chloro-6-((2,2-difluoroethyl)amino)pyrimidine-4-carboxylate (2 g, 7.95 mmol, 1 equiv.) was dissolved in acetonitrile (30 mL), followed by addition of TMSOK (potassium trimethylsilanolate, CAS: 10519-96-7) (1.23 g, 9.54 mmol, 1.2 equiv.), and stirred at room temperature for 1 h. The reaction was complete as monitored by TLC. The reaction mixture was filtered, the filter cake was collected and dissolved in water, and adjusted to pH 5-6 with 1N HCl. The solution was rotary evaporated. and the resultant was dissolved in dichloromethane and methanol, solid impurities were removed by filtration, and the filtrate was concentrated to obtain the title compound (1.6 g, crude product), which was directly used in the following step. LCMS (ESI) [M+1]⁺ =238.0.

### Step III: preparation of trans-1-(2-chloro-6-((2,2-difluoroethyl)amino)pyrimidin-4-yl) (4-(3,4-dihydroisoquinolin-2(1H)-yl)-3-hydroxypiperidin-1-yl)ketone:

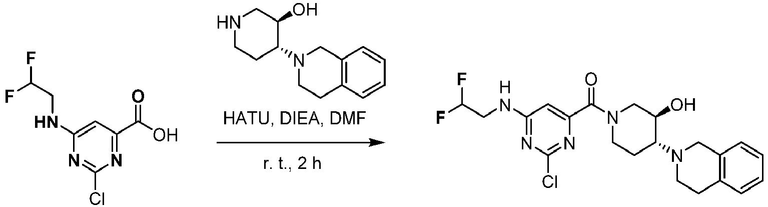

The raw materials 2-chloro-6-((2,2-difluoroethyl)amino)pyrimidine-4-carboxylic acid (1.55 g, 6.54 mmol, 1 equiv.), trans-4-(3,4-dihydroisoquinolin-2(1H)-yl)piperidin-3-ol (1.82 g, 7.85 mmol, 1.2 equiv.), HATU (2.74 g, 7.19 mmol, 1.1 equiv.) and DIEA (1.693 g, 13.08 mmol, 2 equiv.) were dissolved in DMF (5 mL), and the reaction mixture was stirred at 25°C for 1 h. The reaction was complete as indicated by LCMS, and the reaction mixture was quenched by adding water, and then extracted with dichloromethane. The organic phases were combined, and then dried, filtrated and concentrated to obtain a crude product, which was separated and purified by flash chromatography (Silica gel, dichloromethane: methanol= 0-9%) to obtain the title compound (900 mg, yield: 30.5%), LCMS (ESI) [M+H]⁺ = 452.2.

### Step IV: preparation of trans-1-(6-((2,2-difluoroethyl)amino)-2-(1H-imidazol-1-yl)pyrimidin-4-yl)(4-(3,4-dihydroisoquinolin -2(1H)-yl)-3-hydroxypiperidin-1-yl)ketone:

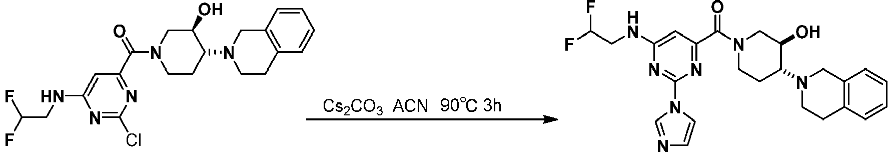

The raw materials trans-1-(2-chloro-6-((2,2-difluoroethyl)amino)pyrimidin-4-yl) (4-(3,4-dihydroisoquinone-2(1H)-yl)-3-hydroxypiperidin-1-yl)ketone (100 mg, 0.222 mmol, 1 equiv.) and imidazole (CAS: 288-32-4) (18 mg, 0.266 mmol, 1.2 equiv.) were dissolved in acetonitrile (0.5 ml), followed by addition of cesium carbonate (217 mg, 0.665 mmol, 3 equiv.), and a reaction was stirred under stirring at 90°C for 3 h. The reaction was complete as monitored by LCMS, and the reaction mixture was quenched by adding water, and then extracted with dichloromethone. The organic phases were combined and washed with saturated sodium chloride, dried over anhydrous sodium sulfate, and then filtrated and concentrated to obtain a crude product, which was separated and purified by reverse phase HPLC (C18, aqueous NH₄HCO₃, acetonitrile) to obtain the title compound (21.90 mg, yield: 20.4%). LCMS (ESI) [M+H]+ =484.4; ¹H NMR (400 MHz, (CD₃)₂SO) δ 8.54 (d, J=8.4 Hz, 1H), 8.48-8.39 (m, 1H), 7.88 (d, J=10.7 Hz, 1H), 7.12-7.06 (m, 4H), 7.05-7.02 (m, 1H), 6.62 (d, J=9.5 Hz, 1H), 6.40-6.05 (m, 1H), 4.79-4.72 (m, c1H), 4.51-4.29 (m, 1H), 4.03-3.87 (m, 2H), 3.85-3.81 (m, 2H), 3.77-3.62 (m, 2H), 3.11-2.79 (m, 5H), 2.77-2.60 (m, 2H), 1.90-1.71 (m, 1H), 1.62-1.49 (m, 1H).

### Example 148

### Preparation of trans-(4-(3,4-dihydroisoquinolin-2(1H)-yl)-3-hydroxypiperidin-1-yl) (6-((4-methoxybutyl)amino)-2-phenylpyrimidin-4-yl)ketone:

### Step I: preparation of N-(2,4-dimethoxybenzyl)-4-methoxybutane-1-amine:

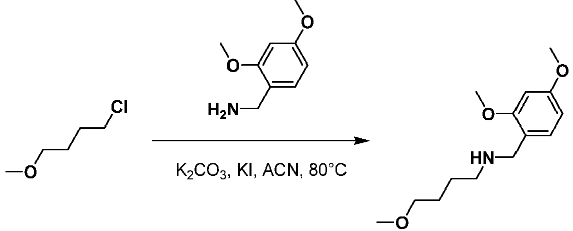

4-Chlorobutyl methyl ether (300 mg, 2.45 mmol, 1 equiv.) and 2,4-dimethoxybenzylamine (1.64 g, 9.81 mmol, 4.01 equiv.) were dissolved in acetonitrile (9 ml), followed by addition of potassium carbonate (676 mg, 4.89 mmol, 2 equiv.) and potassium iodide (812 mg, 4.89 mmol, 2 equiv.), and stirred at 80°C overnight (16 h). Products were produced as monitored by LCMS. The reaction mixture was filtrated, and the filtrate was rotary evaporated. The crude product was separated and purified by flash chromatography (Silica gel, MeOH: EA = 0-2%) to obtain the title compound (90 mg, yield: 11.8%). LCMS (ESI) [M+H]⁺ = 254.2.

### Step II: preparation of methyl 2-chloro-6-(((2,4-dimethoxybenzyl))(4-methoxybutyl)amino)pyrimidine-4-carboxylate:

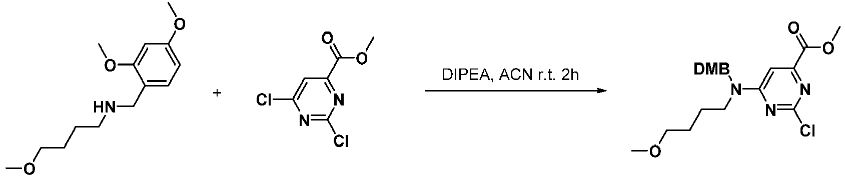

N-(2,4-dimethoxybenzyl)-4-methoxybutan-1-amine (90 mg, 0.356 mmol, 1 equiv.) and methyl 2,6-dichloropyrimidine-4-carboxylate (220 mg, 1.063 mmol, 3 equiv.) were dissolved in acetonitrile (3 mL), followed by addition of DIPEA (250 mg, 1.934 mmol, 5.4 equiv.), and stirred at room temperature (25°C) for 2h. After the reaction was complete as monitored by TLC, the solvent was rotary evaporated, and the crude product was separated and purified by flash chromatography (Silica gel, EA: PE = 0-20%) to obtain the title compound (134 mg, yield: 75.5%). LCMS (ESI) [M+H]⁺ = 424.2.

### Step III: preparation of 6-((2,4-dimethoxybenzyl)(4-methoxybutyl)amino)-2-phenylpyrimidine-4-carboxylic acid:

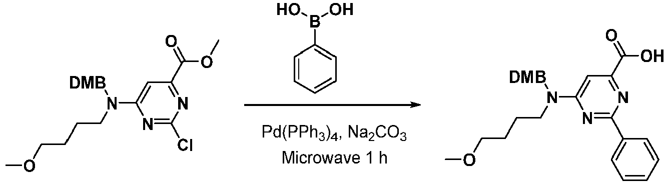

The raw materials methyl 2-chloro-6-(((2,4-dimethoxybenzyl))(4-methoxybutyl)amino)-pyrimidine-4-carboxylate (134 mg, 0.316 mmol, 1 equiv.), phenylboronic acid (77 mg, 0.632 mmol, 2 equiv.), tetra(triphenylphosphine)palladium (36 mg, 0.0312 mmol, 0.1 equiv.) and sodium carbonate (70 mg, 0.660 mmol, 2.09 equiv.) were weighed and put into a microwave tube, sealed with a cap and purged with nitrogen. After dioxane (1.6 ml) and water (0.4 ml) were added, a reaction was stirred at 120°C under microwave and nitrogen for 1 h. The reaction was complete as monitored by LCMS. 1 M aqueous HCl was added to adjust to pH 4-5. Upon filtration, the solvent was rotary evaporated to obtain a crude product of the title compound, which was directly used in the following step. LCMS (ESI) [M+H]⁺= 452.2.

### Step IV: preparation of trans-(4-(3,4-dihydroisoquinolin-2(1H)-yl)-3-hydroxy-piperidin-1-yl)-(6-((2,4-dimethoxybenzyl)(4-methoxybutyl)amino)-2-phenylpyrimidin-4-yl)ketone:

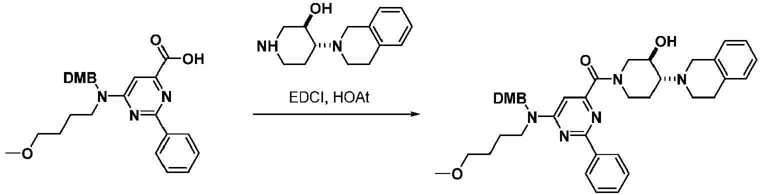

The reactant 6-((2,4-dimethoxybenzyl)(4-methoxybutyl)amino)-2-phenylpyrimidine-4-carboxylic acid (180 mg, 0.315 mmol, 1 equiv.), EDCI (91 mg, 0.475 mmol, 1.51 equiv.) and HOAt (65 mg, 0.478 mmol, 1.52 equiv.) were dissolved in DMF (1 ml), and stirred for 5 minutes. Then trans-4-(3,4-dihydroisoquinolin-2(1H)-yl)piperidin-3-ol (88 mg, 0.379 mmol, 1.2 equiv.) in DMF (0.6 ml) was added, and a reaction was stirred under stirring at room temperature for 2 h. The solvent was rotary evaporated, and the crude product was separated and purified by flash chromatography (Silica gel, MeOH:DCM = 0:20) to obtain the title compound (51 mg, yield: 24.4%). LCMS (ESI) [M+H]⁺ = 666.4.

### Step V: preparation of trans-(4-(3,4-dihydroisoquinolin-2(1H)-yl)-3-hydroxypiperidin-1-yl)(6-((4-methoxybutyl)amino)-2-phenylpyrimidin-4-yl)ketone:

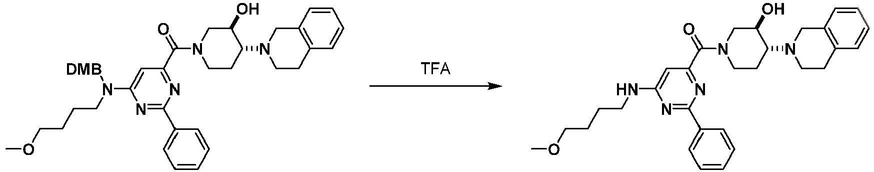

Trans-(4-(3,4-dihydroisoquinolin-2(1H)-yl)-3-hydroxypiperidin-1-yl)(6-((2,4-dimethoxybenzyl)(4-methoxybutyl)amino)-2-phenylpyrimidin-4-yl)ketone (50 mg, 0.0751 mmol, 1 equiv.) was dissolved in trifluoroacetic acid (3 mL), and stirred at 80°C for 20 minutes. After the reaction was complete as indicated by LCMS, the reaction mixture was concentrated under reduced pressure to obtain a crude product, and it was dissolved in methanol, adjusted to pH 7-8, and purified by a prep-HPLC reverse phase column (C18, 10 mmol/L NH₄HCO₃ in water, MeCN) to obtain the title compound (25 mg, yield: 64.6%). LCMS: [M+H]⁺ = 516.25; ¹H NMR (400 MHz, CD₃Cl) δ 8.43 - 8.29 (m, 2H), 7.49 - 7.39 (m, 3H), 7.20 - 7.09 (m, 3H), 7.08 - 7.01 (m, 1H), 6.60 - 6.47 (m, 1H), 5.39 (brs, 1H), 5.15 - 5.05 (m, 0.4H), 4.81 - 4.70 (m, 0.6H), 4.52 - 4.43 (m, 0.6H), 4.43 - 4.35 (m, 0.4H), 4.02 - 3.94 (m, 1H), 3.93 - 3.65 (m, 3H), 3.58 - 3.40 (m, 3H), 3.39 - 3.30 (m, 3H), 3.07 (ddd, J = 13.1, 9.2, 4.1 Hz, 2H), 2.98 - 2.60 (m, 5H), 2.07 - 1.65 (m, 6H).

### Example 149

### Preparation of 1-(4-((6-((3R,4R)-4-(3,4-dihydroisoquinolin-2(1H)-yl)-3-hydroxypiperidine-1-carbonyl)-2-(4-methylpiperazin-1-yl)pyrimidin-4-yl)amino)piperidin-1-yl)ethan-1-one:

### Step I: preparation of methyl 6-((1-acetylpiperidin-4-yl)amino)-2-chloropyrimidine-4-carboxylate:

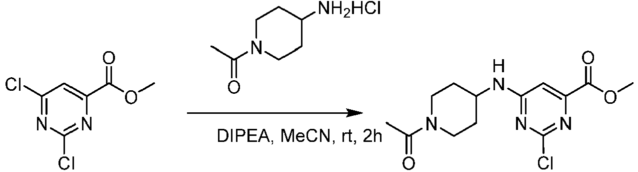

Methyl 2,6-dichloropyrimidine-4-carboxylate (6 g, 28.98 mmol), 1-acetylpiperidin-4-amine hydrochloride (5.7 g, 31.88 mmol) and DIPEA (14.98 g, 115.94 mmol) were dissolved in acetonitrile (50 mL), and stirred at room temperature (25-30°C) (2 h). The reaction mixture was extracted three times with ethyl acetate (100 mL each), and the ethyl acetate phases were combined, washed once with 50 mL water and once with 50 mL saturated sodium chloride, dried over anhydrous sodium sulfate for 10 min, and filtrated. The crude product was separated and purified on a column with DCM:MeOH=100:1 to obtain the title compound (5.8 g, yield: 64%). LCMS (ESI) [M+H]⁺ =313.2.

### Step II: preparation of methyl 6-((1-acetylpiperidin-4-yl)amino)-2-(4-methyl-piperazin-1-yl)-pyrimidine-4-carboxylate:

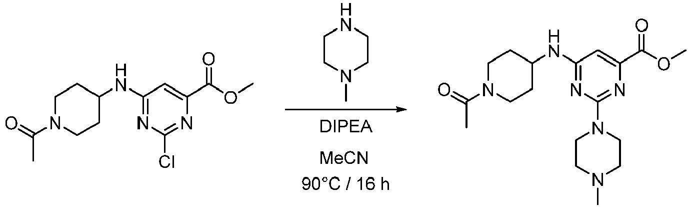

Methyl 6-((1-acetylpiperidin-4-yl)amino)-2-chloropyrimidine-4-carboxylate (100 mg, 0.32 mmol, 1.0 equiv.), N-methylpiperazine (35 mg, 0.35 mmol, 1.1 equiv.) and DIPEA (83 mg, 0.64 mmol, 2.0 equiv.) were dissolved in MeCN (1.6 mL), and a reaction was stirred at 90°C for 16 h. The reaction mixture was concentrated, and the crude product was separated and purified by flash chromatography (Silica gel, DCM: MeOH = 10: 1) to obtain the title compound (113 mg, yield: 93.9%). LCMS (ESI) [M+H]⁺ = 377.2.

### Step III: preparation of 6-((1-acetylpiperidin-4-yl)amino)-2-(4-methylpiperazin-1-yl)-pyrimidine-4-carboxylic acid:

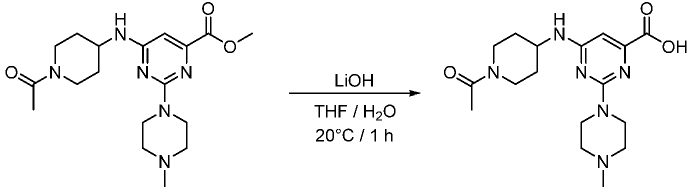

Methyl 6-((1-acetylpiperidin-4-yl)amino)-2-(4-methylpiperazin-1-yl)pyrimidine-4-carboxylate (113 mg, 0.30 mmol, 1.0 equiv.) and LiOH (14 mg, 0.60 mmol, 2.0 equiv.) were dissolved in THF (1.5 mL) and water (0.15 mL), and a reaction was stirred at 20°C for 1 h. The reaction mixture was adjusted to acidic with diluted HCl, and then concentrated to obtain a crude product of the title compound (454 mg), LCMS (ESI) [M+H]⁺ = 363.2.

### Step IV: preparation of 1-(4-((6-((3R,4R)-4-(3,4-dihydroisoquinolin-2(1H)-yl)-3-hydroxypiperidine-1 -carbonyl)-2-(4-methylpiperazin-1 -yl)pyrimidin-4-yl)amino)piperidin-1 - yl)ethan-1-one:

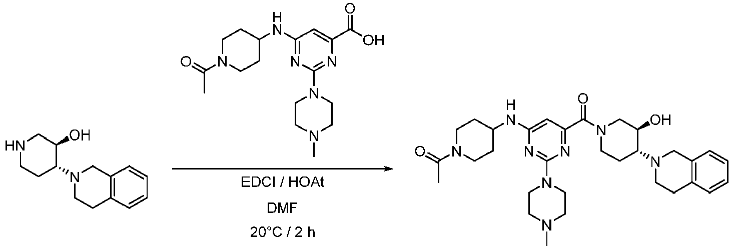

(3R,4R)-4-(3,4-dihydroisoquinolin-2(1H)-yl)piperidin-3-ol (50 mg, 0.22 mmol, 1.0 equiv.), 6-((1-acetylpiperidin-4-yl)amino)-2-(4-methylpiperazin-1-yl)pyrimidine-4-carboxylic acid (454 mg, 0.30 mmol, 1.4 equiv.), EDCI (62 mg, 0.32 mmol, 1.5 equiv.) and HOAt (44 mg, 0.32 mmol, 1.5 equiv.) were dissolved in DMF (2 mL), and a reaction was stirred at 20°C for 2 h. The reaction mixture was concentrated, and the crude product was purified by reverse phase preparative HPLC (C18, 10 mmol/L aqueous NH₄HCO₃, acetonitrile) to obtain the title compound (36.91 mg, yield: 29.7%). LCMS (ESI) [M+H]⁺ = 577.39; ¹H NMR (400 MHz, CDCl₃) δ 7.19 - 7.08 (m, 3H), 7.06 - 6.99 (m, 1H), 5.93 - 5.84 (m, 1H), 5.06 - 4.62 (m, 2H), 4.54 - 4.45 (m, 1H), 4.40 - 4.16 (m, 1H), 4.00 - 3.89 (m, 1H), 3.88 - 3.57 (m, 8H), 3.27 - 3.15 (m, 1H), 3.08 - 2.45 (m, 13H), 2.38 (s, 3H), 2.16 - 2.08 (m, 4H), 2.07 - 1.92 (m, 2H), 1.87 - 1.79 (m, 1H), 1.47 - 1.32 (m, 2H).

### Example 150

### Preparation of 1-(4-((6-((3R,4R)-4-(3,4-dihydroisoquinolin-2(1H)-yl)-3-hydroxypiperidine-1-carbonyl)-2-ethynylpyrimidin-4-yl)amino)piperidin-1-yl)ethan-1-one:

### Step I: preparation of methyl 6-((1-acetylpiperidin-4-yl)amino)-2-((trimethylsilyl)-ethynyl)pyrimidine-4-carboxylate:

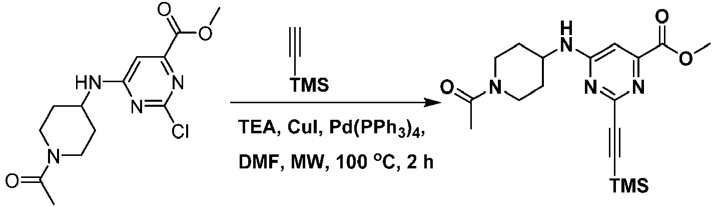

Methyl 6-[(1-acetylpiperidin-4-yl)amino]-2-chloropyrimidine-4-carboxylate (999 mg, 3.197 mmol, 1.0 eq), ethynyl(trimethyl)silane (1.8841 g, 19.182 mmol, 6.0 eq), copper iodide (0.0913 g, 0.48 mmol, 0.15 eq) and triethylamine (0.9705g, 9.591 mmol, 3.0 eq) were dissolved in DMF (8 mL), and Pd(PPh₃)₄ (0.5541g, 0.48 mmol, 0.15 eq) was then added at 25°C under nitrogen . The mixed solution was degassed with nitrogen for 2 minutes, and then reacted at 100°C for 2 h. The reaction was complete as indicated by TLC (DCM: MeOH=10: 1, Rf=0.6). The reaction mixture was filtrated, the filter cake was washed with DCM: MeOH=10: 1 (2 x 15 mL), and the filtrate was concentrated to obtain a crude product. The crude product was separated on a reverse phase column with a mixed solvent (DCM: MeOH=10: 1), and concentrated to obtain the title compound (0.5 g, yield: 41.8%). ¹H NMR (400 MHz, DMSO) δ 7.95 (d,J = 7.5 Hz, 1H), 7.06 (s, 1H), 4.25 - 4.07 (m, 2H), 3.84 (s, 3H), 3.76 (d,J = 13.7 Hz, 1H), 3.22 (t,J = 11.5 Hz, 1H), 2.90 - 2.81 (m, 1H), 2.01 (s, 3H), 1.87 (m, 2H), 1.44 - 1.25 (m, 2H), 0.25 (s, 9H).

### Step II: preparation of 6-((1-acetylpiperidin-4-yl)amino)-2-ethynylpyrimidine-4-carboxylic acid:

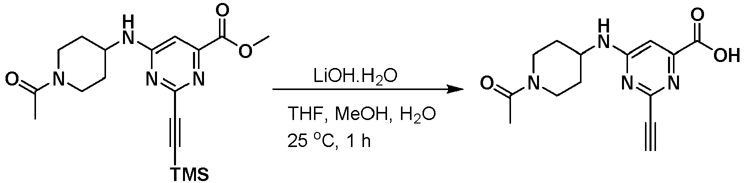

Lithium hydroxide (0.0896 g, 2.136 mmol, 2.5 equiv.) was dissolved in water (5 mL), and then added to a mixed solution of methyl 6-((1-acetylpiperidin-4-yl)amino)-2-((trimethylsilyl)ethynyl)pyrimidine-4-carboxylate (320 mg, 0.854 mmol, 1 equiv.) in tetrahydrofuran (5 ml, 33.33%) and methanol (5 mL, 33.33%), and a reaction was stirred at 25°C for 1 h. After the reaction was complete as indicated by LCMS, the mixture was concentrated, and the crude product was subjected to (C18, 10 mmol/L aqueous NH₄HCO₃, acetonitrile) to obtain the title compound (150 mg, yield: 60.9%), LCMS: [M+H]⁺ =289.2.

### Step III: preparation of 1-(4-((6-((3R,4R)-4-(3,4-dihydroisoquinolin-2(1H)-yl)-3-hydroxypiperidine-1-carbonyl)-2-ethynylpyrimidin-4-yl)amino)piperidin-1-yl)ethan-1-one:

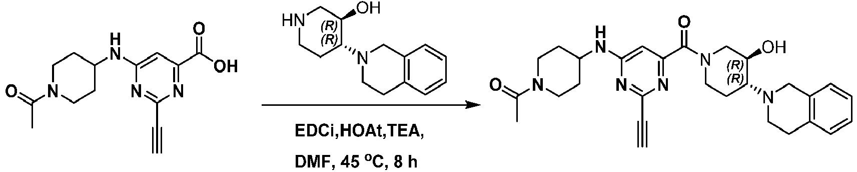

The reactant 6-((1-acetylpiperidin-4-yl)amino)-2-ethynylpyrimidine-4-carboxylic acid (0.1 g, 0.347 mmol, 1 equiv.), EDCI (0.0998 g, 0.52 mmol, 1.5 equiv.), HOAT (0.0331 g, 0.243 mmol, 0.7 equiv.), and triethylamine (0.1405 g, 1.388 mmol, 4 equiv.) were dissolved in DMF (8 mL), followed by addition of (3R,4R)-4-(3,4-dihydroisoquinolin-2(1H)-yl)-piperidin-3-ol (0.0887 g, 0.382 mmol, 1.1 equiv.), and a reaction was stirred at 45°C for 8 h. After the reaction was complete as indicated by LCMS, the mixture was concentrated and the crude product was subjected to (C18, 10 mmol/L aqueous NH₄HCO₃, acetonitrile) to obtain the title compound (50 mg, yield: 28.5%). LCMS: [M+H]⁺ =504.4; ¹HNMR(400 MHz, (CD3)2SO) δ 7.97-7.72 (m, 1H), 7.12-6.99 (m,4H), 6.75-6.47 (m, 1H), 4.79 (d, J=35.6 Hz, 1H), 4.50-4.17 (m, 2H), 4.16-4.01 (m, 2H), 3.90 -3.73 (m, 3H), 3.72-3.53 (m, 2H), 3.70-3.51 (m, 1H), 3.07-2.85 (m, 2H), 2.85-2.73 (m, 4H), 2.68-2.55 (m, 2H), 2.01 (s, 3H), 1.95-1.70 (m, 3H), 1.57-1.32 (m, 2H), 1.28-1.17 (m, 1H).

### Example 151

### Preparation of 1-(4-((6-((3R,4R)-4-(3,4-dihydroisoquinolin-2(1H)-yl)-3-hydroxypiperidine-1-carbonyl)-2-(trifluoromethoxy)pyrimidin-4-yl)amino)piperidin-1-yl)ethan-1-one :

### Step I: preparation of methyl 6-((1-acetylpiperidin-4-yl)amino)-2-hydroxypyrimidine-4-carboxylate:

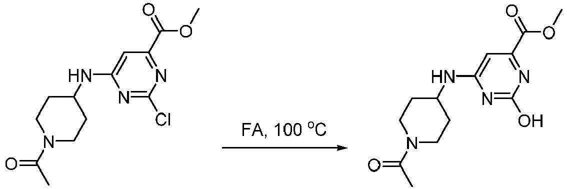

Methyl 6-[(1-acetylpiperidin-4-yl)amino]-2-chloropyrimidine-4-carboxylate (1 g, 0.0032 mol, 1 equiv.) was dissolved in formic acid (10 ml, 100%), and a reaction was stirred under heating at 100°C for 12 h. After the reaction was complete as indicated by LCMS, the mixture was concentrated and the crude product was subjected to (C18, 5 mmol/L aqueous NH₃, acetonitrile) to obtain the title compound (200 mg, yield: 21.3%). LCMS: [M+H]⁺ =295.1.

### Step II: preparation of methyl 6-((1-acetylpiperidin-4-yl)amino)-2-(trifluoromethoxy)-pyrimidine-4-carboxylate:

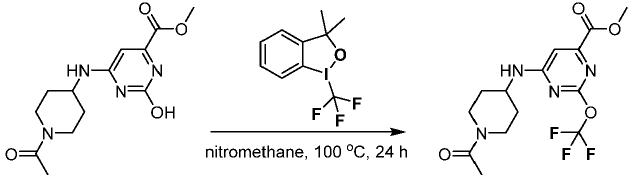

At room temperature, 3,3-dimethyl-1-(trifluoromethyl)-1lambda3,2-benziodoxole (0.22 g, 0.68 mmol, 1 equiv.) was added to a solution of methyl 6-((1-acetylpiperidin-4-yl)amino)-2-hydroxypyrimidine-4-carboxylate (200.13 mg, 0.68 mmol, 1 equiv.) in nitromethane (3 ml, 100%), which was purged with nitrogen, and a reaction was stirred under heating at 100°C for 12 h; then 3,3-dimethyl-1-(trifluoromethyl)-1lambda3,2-benziodoxole (0.011 g, 0.034 mmol, 0.05 equiv.) was supplemented, and the reaction was continued under heating at 100°C for 12 h. After the reaction was complete as indicated by LCMS, the mixture was concentrated to dryness and purified on a column with 10% DCM/MeOH to obtain the title compound (35 mg, yield: 14.2%). LCMS: [M+H]⁺ =363.1.

### Step III: preparation of 6-((1-acetylpiperidin-4-yl)amino)-2-(trifluoromethoxy)-pyrimidine-4-carboxylic acid:

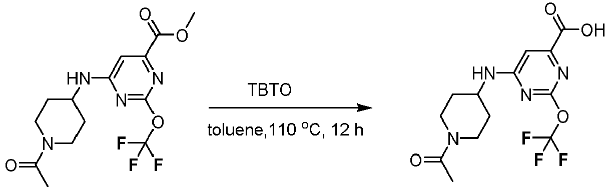

Tributyltin oxide (0.069 g, 0.116 mmol, 1.2 equiv.) was added to a mixed solution of methyl 6-((1-acetylpiperidin-4-yl)amino)-2-(trifluoromethoxy)pyrimidine-4-carboxylate (25 mg, 0.069 mmol, 1 equiv.) in acetonitrile (1 ml, 20%) and toluene (4 mL, 80%), and a reaction was stirred at 110°C for 12 h. After the reaction was complete as indicated by LCMS, the crude product was quenched with 1 mL aqueous KF and concentrated, and the crude product was made into a slurry with ethyl ether and dried to obtain the title compound (25 mg, yield: 74.3%). LCMS: [M+H]+ =349.0.

### Step IV: preparation of 1-(4-((6-((3R,4R)-4-(3,4-dihydroisoquinolin-2(1H)-yl)-3-hydroxypiperidine-1-carbonyl)-2-(trifluoromethoxy)pyrimidin-4-yl)amino)piperidin-1-yl)-ethan-1-one:

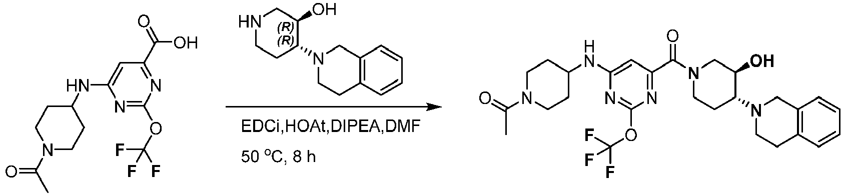

The reactant 6-((1-acetylpiperidin-4-yl)amino)-2-(trifluoromethoxy)pyrimidine-4-carboxylic acid (14.98 mg, 0.043 mmol, 1 equiv.), EDCI (12.36 mg, 0.064 mmol, 1.5 equiv.), HOAt (0.0044 g, 0.032 mmol, 0.75 equiv.) and diisopropylethylamine (0.022 g, 0.17 mmol, 4 equiv.) were dissolved in DMF (N,N-dimethylformamide) (3 mL), followed by addition of (3R,4R)-4-(3,4-dihydroisoquinolin-2(1H)-yl)piperidin-3-ol (10.99 mg, 0.047 mmol, 1.1 equiv.), and a reaction was stirred at 50°C for 8 h. After the reaction was complete as indicated by LCMS, the mixture was concentrated, and the crude product was subjected to (C18, 10 mmol/L aqueous NH₄HCO₃, acetonitrile) to obtain the title compound (6.1 mg, yield: 25.2%). LCMS: [M+H]⁺ =563.3; ¹H NMR (400 MHz, CD3OD) δ 7.12-7.06 (m, 3H), 7.05-7.00 (m, 1H), 6.51-6.48 (m, 1H), 4.72-4.49 (m, 1H), 4.42 (d, J = 13.2 Hz, 1H), 4.18-4.07 (m, 1H), 4.00-3.84 (m, 4H), 3.83-3.73 (m, 1H), 3.63-3.43 (m, 1H), 3.15-2.96 (m, 2H), 2.95-2.88 (m, 4H), 2.87-2.67 (m, 2H), 2.12 (s, 3H), 2.10-1.87 (m, 3H), 1.74-1.63 (m, 1H), 1.56-1.37 (m, 2H).

### Example 152

### Preparation of 1-(4-((6-((3R,4R)-4-(3,4-dihydroisoquinolin-2(1H)-yl)-3-hydroxypiperidine-1-carbonyl)-2-vinylpyrimidin-4-yl)amino)piperidin-1-yl)ethan-1-one:

### Step I: preparation of methyl 6-((1-acetylpiperidin-4-yl)amino)-2-vinylpyrimidine-4-carboxylate:

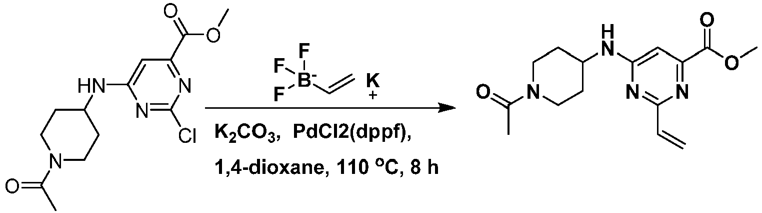

The reactant methyl 6-((1-acetylpiperidin-4-yl)amino)-2-chloropyrimidine-4-carboxylate (0.90 g, 2.9 mmol, 1 equiv.), potassium vinyl(trifluoro)borate (0.46 g, 3.5 mmol, 1.2 equiv.), potassium carbonate (0.99 g, 7.2 mmol, 2.5 equiv.) and [1,1-bis(diphenylphosphino)ferrocene]palladium dichloride (90 mg, 0.1 equiv.) were dissolved in 1,4-dioxane (30 mL), purged with nitrogen for 2 minutes, and a reaction was stirred under heating at 110°C for 8 h. After the reaction was complete as indicated by LCMS, the mixture was filtrated, and the filter cake was washed twice with 15 mL mixed solvent (DCM: MeOH=10:1). The filtrate was concentrated, and the crude product was separated on a reverse phase column with a mixed solvent (DCM: MeOH=10:1) and concentrated to obtain the title compound (0.85 g, yield: 97%). LCMS: [M+H]⁺ =305.2.

### Step II: preparation of 6-((1-acetylpiperidin-4-yl)amino)-2-vinylpyrimidine-4-carboxylic acid:

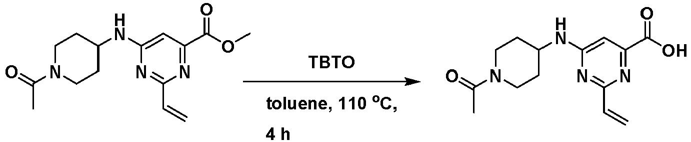

Tributyltin oxide (0.47 g, 0.79 mmol, 1.2 equiv.) was added to a mixed solution of methyl 6-((1-acetylpiperidin-4-yl)amino)-2-vinylpyrimidine-4-carboxylate (0.2 g, 0.66 mmol, 1 equiv.) in acetonitrile (4 mL, 20%) and tolune (16 mL, 80%), and a reaction was stirred at 110°C for 4 h. After the reaction was complete as indicated by LCMS, the crude product was quenched with 2 mL aqueous KF and concentrated, and the crude product was made into a slurry with ethyl ether and dried to obtain the title compound (0.15 g, yield: 78.6% ). LCMS: [M+H]⁺ =291.1.

### Step III: preparation of 1-(4-((6-((3R,4R)-4-(3,4-dihydroisoquinolin-2(1H)-yl)-3-hydroxypiperidine-1-carbonyl)-2-vinylpyrimidin-4-yl)amino)piperidin-1-yl)ethan-1-one:

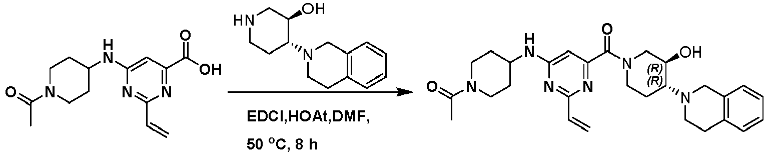

The reactants 6-((1-acetylpiperidin-4-yl)amino)-2-vinylpyrimidine-4-carboxylic acid (100 mg, 0.34 mmol, 1 equiv.), 3-(ethyliminomethylideneamino)propyl-dimethylazane hydrochloride (0.110 g, 0.55 mmol, 1.6 equiv.), 3-hydroxytriazolo[4,5-b]pyridine (0.038 g, 0.28 mmol, 0.8 equiv.) and diisopropylethylamine (0.180 g, 1.4 mmol, 4 equiv.) were dissolved in DMF (8 mL), followed by addition of (3R,4R)-4-(3,4-dihydroisoquinolin-2(1H)-yl)piperidin-3-ol (0.088 g, 0.38 mmol, 1.1 equiv.), and a reaction was stirred at 50°C for 8 h. After the reaction was complete as indicated by LCMS, the mixture was concentrated, and the crude product was subjected to (C18, 10 mmol/L aqueous NH₄HCO₃, acetonitrile) to obtain the title compound (21 mg, yield: 28.5%) . LCMS: [M+H]⁺ =505.3; ¹H NMR (400 MHz, CD₃OD) δ 7.13 - 7.01 (m, 4H), 6.70 - 6.59 (m, 1H), 6.57 - 6.48 (m, 1H), 6.47 - 6.35 (m, 1H), 5.73 - 5.61 (m, 1H), 4.76 - 4.49 (m,2H), 4.41 (d, J = 13.2 Hz, 1H), 4.31-4.20 (m, 1H), 4.02 - 3.87 (m, 3H), 3.86 - 3.73 (m, 2H), 3.16 - 3.00 (m, 2H), 2.99 - 2.83 (m, 5H), 2.83 - 2.67 (m, 1H), 2.12 (s, 3H), 2.10 - 1.84 (m, 3H), 1.76 - 1.63 (m, 1H), 1.58 - 1.38 (m, 2H).

### Example 153

### Preparation of 1-(4-((6-((3R,4R)-4-(3,4-dihydroisoquinolin-2(1H)-yl)-3-hydroxypiperidine-1-carbonyl)-2-(5 -methylthiophen-2-yl)pyrimidin-4-yl)amino)piperidin-1-yl)ethan-1-one:

### Step I: preparation of 6-((1-acetylpiperidin-4-yl)amino)-2-(5-methylthiophen-2-yl)pyrimidine-4-carboxylic acid:

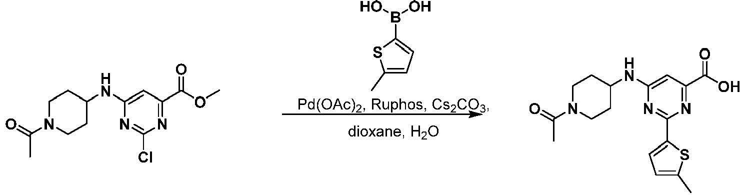

Methyl 6-((1-(pyrimidin-4-yl)piperidin-4-yl)amino)pyrimidine-4-carboxylate (150 mg, 0.48 mmol, 1.0 equiv.), 5-methylthiophene-2-boronic acid (102 mg, 0.72 mmol, 1.5 equiv.), Pd(OAc)₂ (10.8 mg, 0.048 mmol, 0.1 equiv.), RuPhos (44 mg, 0.096 mmol, 0.2 equiv.) and Cs₂CO₃ (468 mg, 1.4 mmol, 3.0 equiv.) were dissolved in 1,4-dioxane (2 mL) and water (0.5 mL), and the reaction mixture was stirred at 100°C for 3 h. The reaction was complete as indicated by LCMS, and the reaction mixture was quenched by adding water, and then extracted three times with ethyl acetate (30 mL each). The aqueous phase containing the product was adjusted to pH 7 with 1 M HCl, and then the aqueous phase was concentrated and rotary evaporated to obtain a crude product of the title compound (200 mg). LCMS (ESI) [M+H]⁺ =361.2.

### Step II: preparation of 1-(4-((6-((3R,4R)-4-(3,4-dihydroisoquinolin-2(1H)-yl)-3-hydroxypiperidine-1-carbonyl)-2-(5-methylthiophen-2-yl)pyrimidin-4-yl)amino)piperidin-1-yl)ethan-1-one:

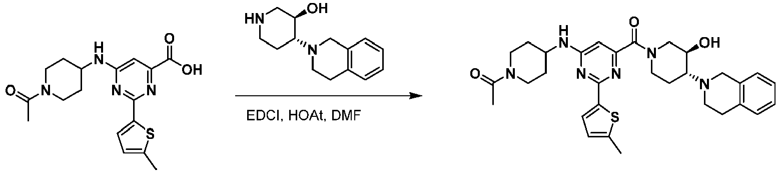

6-((1-acetylpiperidin-4-yl)amino)-2-(thiophen-3-yl)pyrimidine-4-carboxylic acid (100 mg, 0.28 mmol, 1.0 equiv.), (3R,4R)-4-(3,4-dihydroisoquinolin-2(1H)-yl)piperidin-3-ol (65 mg, 0.28 mmol, 1 equiv.), EDCI (80 mg, 0.42 mmol, 1.5 equiv.), and HOAt (56 mg, 0.42 mmol, 1.5 equiv.) were dissolved in DMF (3 mL), and the reaction mixture was stirred at 25°C for 1 h. The reaction was complete as indicated by LCMS, and the reaction mixture was quenched by adding water, and then concentrated, and the resultant crude product was separated and purified by reverse phase HPLC (C18, aqueous NH₄HCO₃, acetonitrile) to obtain the title compound (75.3 mg, yield: 47.2%). LCMS (ESI) [M+H]⁺ =575.2; ¹H NMR (400 MHz, (CD₃)₂SO) δ 10.51 - 10.32 (m, 1H), 8.14 - 8.13 (m, 1H), 7.83 - 7.79 (m, 1H), 7.30 - 7.19 (m, 4H), 6.89 (s, 1H), 6.51 (s, 1H), 4.74 - 4.43 (m, 3H), 4.25 - 4.15 (m, 2H), 4.06 - 4.04 (m, 2H), 3.93 - 3.72 (m, 5H), 3.45 - 3.10 (m, 5H), 3.00 - 2.84 (m, 3H), 2.74 - 2.67 (m, 1H), 2.37 - 2.14 (m, 1H), 2.01 - 1.86 (m, 6H), 1.47 - 1.23 (m, 2H).

### Example 154

### Preparation of 1-(4-((2-((4,4-difluorocyclohexyl)oxy)-6-((3R,4R)-4-(3,4-dihydroisoquinolin-2(1H)-yl)-3-hydroxypiperidine-1-carbonyl)pyrimidin-4-yl)amino)-piperidin-1 -yl)ethan-1 -one:

### Step I: preparation of 6-((1-acetylpiperidin-4-yl)amino)-2-((4,4-difluorocyclohexyl)oxy) pyrimidine-4-carboxylic acid:

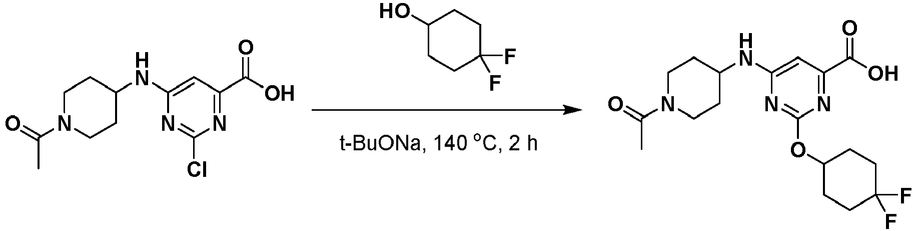

6-((1-acetylpiperidin-4-yl)amino)-2-chloropyrimidine-4-carboxylic acid (200 mg, 0.671 mmol, 1.0 equiv.) was dissolved in 4,4-difluorocyclohexane-1-ol (1 mL), followed by addition of sodium t-butoxide (1.343 mg, 1.343 mmol, 2.2 equiv.), and a reaction was stirred at 140°C for 2 h. After the reaction was complete as indicated by LCMS, the mixture was concentrated to obtain a crude product of the title compound (180 mg). LCMS: [M+H]⁺ =399.2.

### Step II: preparation of 1-(4-((2-((4,4-difluorocyclohexyl)oxy)-6-((3R,4R)-4-(3,4-dihydroisoquinolin-2(1H)-yl)-3-hydroxypiperidine-1-carbonyl)pyrimidin-4-yl)amino)-piperidin-1 -yl)ethan-1 -one:

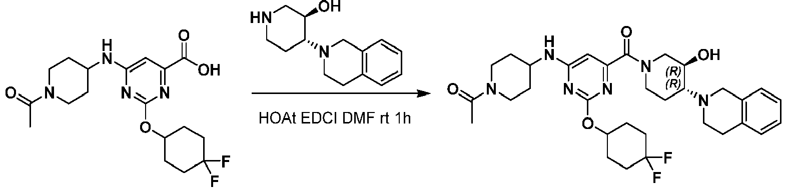

The reactants 6-((1-acetylpiperidin-4-yl)amino)-2-((4,4-difluorocyclohexyl)oxy)-pyrimidine-4-carboxylic acid (81.60 mg, 0.402 mmol, 1.0 equiv.), (3R,4R)-4-(3,4-dihydroisoquinolin-2(1H)-yl)piperidin-3-ol (103 mg, 0.442 mmol, 1.1 equiv.), EDCI (63 mg, 0.33 mmol, 1.5 equiv.) and HOAt (45 mg, 0.33 mmol, 1.5 equiv.) were dissolved in DMF (2 mL), and a reaction was stirred at 25°C for 1 h. After the reaction was complete as indicated by LCMS, the mixture was concentrated, and the crude product was separated and purified by reverse phase HPLC (C18, 10 mmol/L aqueous NH₄HCO₃, acetonitrile) to obtain the title compound (26.13 mg, yield: 20.5%). LCMS: [M+H]⁺ =613.4; ¹H NMR (400 MHz, (CD₃)₂SO) δ 7.71-7.57 (m, 1H), 7.14-6.96 (m, 4H), 6.42-6.10 (m, 1H), 5.09-4.97 (m, 1H), 4.82-4.67 (m, 1H), 4.51-4.18 (m, 2H), 4.12-3.99 (m, 1H), 3.89-3.70 (m, 4H), 3.66-3.56 (m, 1H), 3.27-3.09 (m, 1H), 3.05-2.76 (m, 6H), 2.70-2.54 (m, 2H), 2.10-1.97 (m, 8H), 1.94-1.71 (m, 6H), 1.57-1.44 (m, 1H), 1.40-1.18 (m, 2H).

### Example 155

### Preparation of 1-(4-((6-((3R,4R)-4-(3,4-dihydroisoquinolin-2(1H)-yl)-3-hydroxy-piperidine-1 -carbonyl)-2-(isopropylthio)pyrimidin-4-yl)amino)piperidin-1 -yl)ethan-1 -one hydrochloride:

### Step I: preparation of 6-((1-acetylpiperidin-4-yl)amino)-2-(isopropylthio)pyrimidine-4-carboxylic acid:

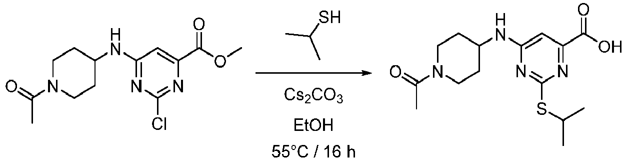

Methyl 6-((1-acetylpiperidin-4-yl)amino)-2-chloropyrimidine-4-carboxylate (100 mg, 0.32 mmol, 1.0 equiv.), isopropylthiol (122 mg, 1.60 mmol, 5.0 equiv.) and Cs₂CO₃ (208 mg, 0.64 mmol, 2.0 equiv.) were dissolved in EtOH (0.6 mL), and a reaction was stirred at 55°C for 16 h. The reaction mixture was filtrated, and the filtrate was concentrated to obtain a crude product of the target compound (255 mg), LCMS (ESI) [M+H]⁺ = 339.2.

### Step II: preparation of 1-(4-((6-((3R,4R)-4-(3,4-dihydroisoquinolin-2(1H)-yl)-3-hydroxypiperidine-1 -carbonyl)-2-(isopropylthio)pyrimidin-4-yl)amino)piperidin-1 -yl)ethan-1 -one:

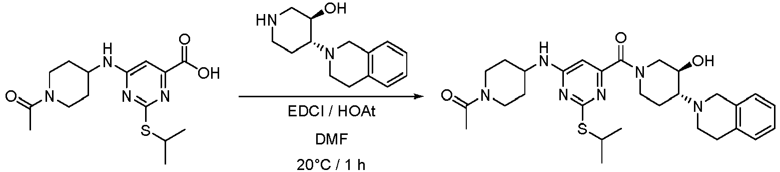

6-((1-acetylpiperidin-4-yl)amino)-2-(isopropylthio)pyrimidine-4-carboxylic acid (235 mg, 0.29 mmol, 1.0 equiv.), (3R,4R)-4-(3,4-dihydroisoquinolin-2(1H)-yl)piperidin-3-ol (102 mg, 0.44 mmol, 1.5 equiv.), EDCI (84 mg, 0.44 mmol, 1.5 equiv.) and HOAt (60 mg, 0.44 mmol, 1.5 equiv.) were dissolved in DMF (3.5 mL), and a reaction was stirred at 20°C for 1 h. The reaction mixture was concentrated, and the crude product was purified by reverse phase preparative HPLC (C18, 10 mmol/L aqueous NH₄HCO₃, acetonitrile) to obtain the target compound (63 mg, yield: 39.1%). LCMS (ESI) [M+H]⁺ = 553.19.

### Step III: preparation of 1-(4-((6-((3R,4R)-4-(3,4-dihydroisoquinolin-2(1H)-yl)-3-hydroxypiperidine-1 -carbonyl)-2-(isopropylthio)pyrimidin-4-yl)amino)piperidin-1 -yl)ethan-1 -one hydrochloride:

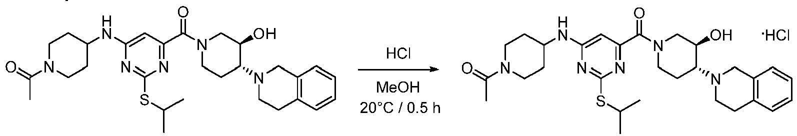

1-(4-((6-((3R,4R)-4-(3,4-dihydroisoquinolin-2(1H)-yl)-3-hydroxypiperidine-1-carbonyl) -2-(isopropylthio)pyrimidin-4-yl)amino)piperidin-1-yl)ethan-1-one (59 mg, 0.11 mmol, 1.0 equiv.) and hydrochloric acid (0.1 M) (1.17 mL, 0.12 mmol, 1.1 equiv.) were dissolved in MeOH (3 mL), and a reaction was stirred at 20°C for 0.5h. The reaction mixture was concentrated to obtain the target compound (52.1 mg, yield: 82.8%). LCMS (ESI) [M-Cl]⁺ = 553.14; ¹H NMR (400 MHz, DMSO-d₆) δ 10.50 - 10.00 (m, 1H), 8.13 - 7.70 (m, 1H), 7.40 - 7.01 (m, 4H), 6.57 - 5.89 (m, 2H), 4.77 - 4.35 (m, 3H), 4.30 - 4.01 (m, 2H), 3.99 - 3.74 (m, 6H), 3.45 - 3.24 (m, 2H), 3.23 - 2.93 (m, 3H), 2.89 - 2.62 (m, 2H), 2.38 - 2.08 (m, 1H), 2.06 - 1.98 (m, 3H), 1.95 - 1.68 (m, 3H), 1.47 - 1.24 (m, 8H).

### Example 156

### Preparation of (6-((1-(3,3-difluorocyclobutane-1-carbonyl)piperidin-4-yl)amino)-2-(pentan-3-oxy)pyrimidin-4-yl)((3R,4R)-4-(3,4-dihydroisoquinolin-2(1H)-yl)-3-hydroxy-piperidin-1-yl)ketone:

### Step I: preparation of methyl 6-((1-t-butoxycarbonylpiperidin-4-yl)amino)-2-chloropyrimidine-4-carboxylate:

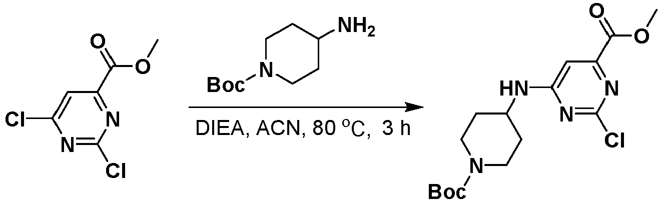

The raw material methyl 2,6-dichloropyrimidine-4-carboxylate (1.086 g, 5.24 mmol, 1.05 equiv.) and 1-t-butoxycarbonyl-4-aminopiperidine (1 g, 4.99 mmol, 1.05 equiv.) were dissolved in acetonitrile (40 mL), followed by addition of DIPEA (1.94 g, 14.97 mmol, 3 equiv.), and a reaction was stirred under stirring at 80°C for 3 h. The reaction was complete as monitored by LCMS. The reaction mixture was quenched by adding water, and then extracted with ethyl acetate. The organic phases were combined and washed with saturated sodium chloride, dried over anhydrous sodium sulfate, and then filtrated and concentrated to obtain a crude product, which was separated and purified by flash chromatography (Silica gel, petroleum ether: ethyl acetate = 1:5) to obtain the title compound (1.5 g, yield: 80%). LCMS (ESI) [M+1]⁺ =371.0.

### Step II: preparation of 6-((1-(1-butoxycarbonyl)piperidin-4-yl)amino)-2-pentan-3-yloxy-pyrimidine-4-carboxylic acid:

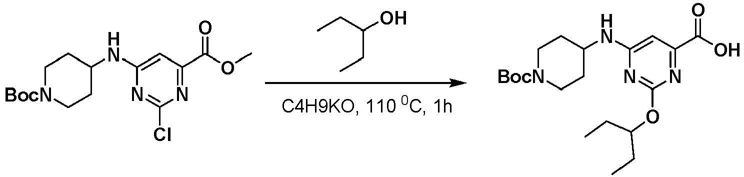

Methyl 6-((1-(1-butoxycarbonyl)piperidin-4-yl)amino)-2-chloropyrimidine-4-carboxylate (1 g, 2.70 mmol, 1.0 equiv.) was dissolved in 3-pentanol (10 mL), followed by addition of potassium t-butoxide (1.56 g, 16.21 mmol, 6.0 equiv), and stirred at 110 °C for 16h. After the reaction was complete as indicated by LCMS, the mixture was concentrated, and separated and purified by column chromatography (MeOH: DCM = 10%) to obtain the title compound (718 mg), LCMS: [M+H]⁺ =409.2

### Step III: preparation of t-butyl 4-((6-((3R,4R)-4-(3,4-dihydroisoquinolin-2(1H)-yl)-3-hydroxypiperidine-1 -carbonyl)-2-(pentan-3 -yloxy)pyrimidin-4-yl)amino)piperidine-1 - carboxylate:

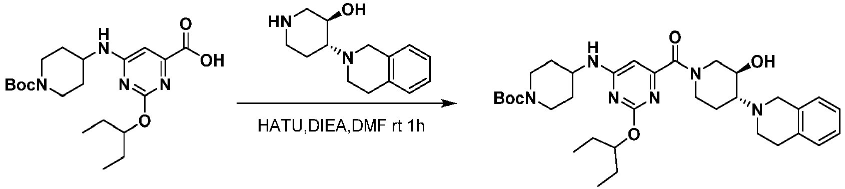

6-(((1-(1-butoxycarbonyl)piperidin-4-yl)amino)-2-pentan-3-yloxypyrimidine-4-carboxyl ic acid (698 mg, 1.71 mmol, 1.0 equiv.) was dissolved in N,N-dimethylformamide (10 mL), followed by addition of (3R,4R)-4-(3,4-dihydroisoquinolin-2(1H)-yl)piperidin-3-ol (396.9 mg, 1.71 mmol, 1.0 equiv.), HATU (649.7 mg, 1.709 mmol, 1.0 equiv.) and N,N-diisopropylethylamine (662.5 mg, 5.126 mmol, 3.0 equiv), and stirred at room temperature for 2h. After the reaction was complete as indicated by LCMS, the mixture was extracted and concentrated to obtain the title compound (580 mg, yield: 54.5%). LCMS: [M+H]⁺ =623.5.

### Step IV: preparation of ((3R,4R)-4-(3,4-dihydroisoquinolin-2(1H)-yl)-3-hydroxy-piperidin-1-yl)(2-(pentan-3-yloxy)-6-(piperidin-4-ylamino)pyrimidin-4-yl)ketone:

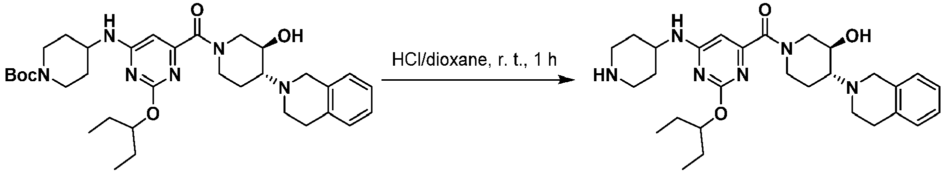

T-butyl 4-((6-((3R,4R)-4-(3,4-dihydroisoquinolin-2(1H)-yl)-3-hydroxypiperidine-1-carbonyl)-2-(pentan-3-yloxy)pyrimidin-4-yl)amino)piperidine-1-carboxylate (580 mg, 0.931 mmol, 1.0 equiv.) was dissolved in dioxane (10 mL), followed by addition of hydrochloric acid in dioxane (2 mL), and stirred at 25°C for 2h. After the reaction was complete as indicated by LCMS, the mixture was concentrated to obtain a crude product of the title compound (832 mg), LCMS: [M+H]⁺ =523.2.

### Step V: preparation of (6-((1-(3,3-difluorocyclobutane-1-carbonyl)piperidin-4-yl)-amino)-2-(pentan-3-yloxy)pyrimidin-4-yl)((3R,4R)-4-(3,4-dihydroisoquinolin-2(1H)-yl)-3-hydroxypiperidin-1-yl)ketone:

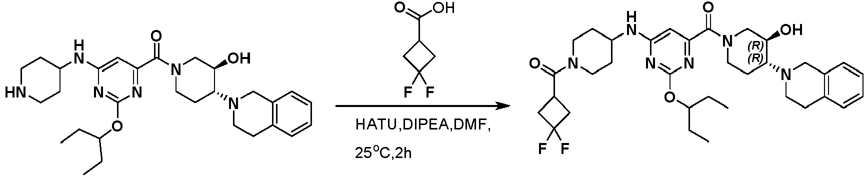

((3R,4R)-4-(3,4-dihydroisoquinolin-2(1H)-yl)-3-hydroxypiperidin-1-yl)(2-(pentan-3-yloxy)-6 -(piperidin-4-ylamino)pyrimidin-4-yl)ketone (100 mg, 0.191 mmol, 1.0 equiv.) was dissolved in N,N-dimethylformamide (5 mL), followed by addition of 3,3-difluorocyclobutane-1-carboxylic acid (20.0 mg, 0.191 mmol, 1.0 equiv.), HATU (72.7 mg, 0.191 mmol, 1.0 equiv.) and N,N-diisopropylethylamine (74.1 mg, 0.574 mmol, 3.0 equiv), and stirred at room temperature for 2h. After the reaction was complete as indicated by LCMS, the mixture was extracted and concentrated to obtain the title compound (58.55 mg, yield: 47.8%). LCMS: [M+H]⁺ =641.5; ¹H NMR (400 MHz, CD₃OD) δ 7.10 - 7.02 (m, 4H), 6.16 (d, J = 7.2 Hz, 1H), 5.01 - 4.96 (m, 1H), 4.69 - 4.52 (m, 1H), 4.41 (d, J = 13.6 Hz, 1H), 4.22 - 4.11 (m, 1H), 3.96 - 3.77 (m, 5H), 3.27 - 2.56 (m, 14H), 2.10 - 1.86 (m, 3H), 1.75 - 1.62 (m, 5H), 1.48 - 1.39 (m, 2H), 0.99 - 0.93 (m, 6H).

### Example 157

### Preparation of 1-(4-((6-((3R,4R)-4-(3,4-dihydroisoquinolin-2(1H)-yl)-3-hydroxypiperidine-1-carbonyl)-2-(isopropylsulfinyl)pyrimidin-4-yl)amino)piperidin-1-yl)ethan-1-one:

### Step I: preparation of 1-(4-((6-((3R,4R)-4-(3,4-dihydroisoquinolin-2(1H)-yl)-3-hydroxy-piperidine-1-carbonyl)-2-(isopropylsulfinyl)pyrimidin-4-yl)amino)piperidin-1-yl)ethan-1-one

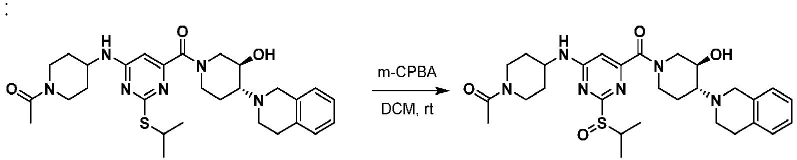

1-(4-((6-((3R,4R)-4-(3,4-dihydroisoquinolin-2(1H)-yl)-3-hydroxypiperidine-1-carbonyl)-2-(isopropylthio)pyrimidin-4-yl)amino)piperidin-1-yl)ethan-1-one (40 mg, 0.072368 mmol, 1 equiv.) and m-CPBA (13.7374 mg, 0.079605 mmol, 1.1 equiv.) were dissolved in dichloromethane (2 mL), and the reaction mixture was stirred at room temperature for 2 h. The reaction mixture was evaporated on a rotary evaporator to obtain a crude product, and the crude product was separated and purified by reverse phase HPLC (C18, aqueous NH₄HCO₃, acetonitrile) to obtain the title compound (15 mg, yield: 36%). LCMS (ESI) [M+H]⁺ =569.0; ¹H NMR (400 MHz, DMSO) δ 8.48 - 7.45 (m, 1H), 7.17 - 6.99 (m, 4H), 6.81 - 6.56 (m, 1H), 4.85 - 4.69 (m, 1H), 4.46 - 4.09 (m, 3H), 3.86 - 3.76 (m, 3H), 3.69 - 3.59 (m, 2H), 3.26 - 3.13 (m, 2H), 3.08 - 2.73 (m, 6H), 2.70 - 2.59 (m, 2H), 2.07 - 1.66 (m, 6H), 1.55 - 1.25 (m, 6H), 1.11 - 0.96 (m, 2H).

### Biological test and assessment

The present invention will be further described hereinafter in combination with test examples, but the examples are not intended to limit the scope of the present invention.

### Test Example 1. Test on the inhibitory activity of the compounds of the invention against PRMT5 enzyme by radioisotopic method

**Test method:** A 1x enzyme reaction buffer (10 mM Tris 8.0 (Sigma, Cat. No. T2694-1L), 0.01% Tween-20 (Sigma, Cat. No. P2287-100ML), 1 mM DTT (Sigma, Cat.No. D0632-10G)) was prepared. PRMT5 (Active Motif, Cat. No. 31921) and [3H]-SAM (PerkinElmer, Cat. No. NET155V001MC) were added to the 1x enzyme reaction buffer, to prepare a 25/15x mixed solution (PRMT5 final concentration: 5 nM, [3H]-SAM final concentration: 0.3 µM). 15µL of this solution was transferred into a 384-well microplate (Corning 384-well Polypropylene Storage Microplates, Cat. No. 3657) with various concentrations of the compounds (DMSO final concentration 1%), and incubated at room temperature for 60 minutes. A polypeptide substrate, GL-27 (Ac-SGRGKGGKGLGKGGAKRHRKVGG-K (Biotin) (GL Biochem, Cat. No. 342095)), was added into the 1x enzyme reaction buffer to prepare a 25/10x substrate solution. Then 10 µL of the polypeptide substrate solution (final concentration of the polypeptide substrate: 100 nM) was added, a reaction was stirred at room temperature for 120 minutes, and then 5 µL 6x ice cold SAM (Sigma, Cat. No. A7007-100MG) solution was added to stop the reaction (SAM final concentration: 0.125 mM). 25 µL of the reaction mixture was transferred into a FlashPlate (Streptavidin FlashPlate HTS PLUS, High Capacity, 384-well, Perkin Elmer, Cat. No. SMP410A001PK), and incubated at room temperature for 1h. After washed three times with distilled water containing 0.1% Tween-20, the microplate was read on a MicroBeta reader for CPM data (Counts Per Minute). After the CPM raw data of the compounds at various concentrations were obtained, the data were normalized according to Inh% = (Max-Sample) / (Max-Min)^{∗}100%, and the enzyme activity inhibition rate Inh% at each concentration point was obtained (wherein Max is the CPM value of a positive well with the enzyme, Min is the CPM value of a negative well without the enzyme, and Sample is the CPM value of the sample well treated with the compounds). Then the inhibition rate Inh% (Y) corresponding to each concentration (X) was input in EXCEL, and the IC₅₀ value (the half maximal inhibitory concentration) of each compound was calculated with the XLfit plug-in according to the built-in four-parameter fitting equation Y=Bottom + (Top-Bottom)/(1+(IC₅₀/X)^{∗}HillSlope).

Results: From the above procedure, the compounds of the examples according to the present invention showed the biological activities in the PRMT5 enzymatic activity inhibition assay as in Table 1 below.

**Table 1: IC₅₀ of compounds against PRMT5 enzyme activity**

| Compound of Example | PRMT5/IC₅₀ |
|---|---|
| 17 | A |
| 24 | A |
| 25 | A |
| 28 | A |
| 30 | A |
| 34 | A |
| 35 | A |
| 37 | A |
| 38 | A |
| 39 | A |
| 41 | A |
| 42 | A |
| 43 | A |
| 46 | A |
| 47 | A |
| 49 | A |
| 50 | A |
| 51 | A |
| 53 | A |
| 54 | A |
| 55 | A |
| 56 | A |
| 57 | A |
| 58 | A |
| 62 | A |
| 66 | A |
| 67 | A |
| 73 | A |
| 74 | A |
| 78 | A |
| 80 | A |
| 83 | A |
| 84 | A |
| 87 | A |
| 88 | A |
| 89 | A |
| 90 | A |
| 91 | A |
| 92 | A |
| 93 | A |
| 94 | A |
| 95 | A |
| 96 | A |
| 97 | A |
| 98 | A |
| 99 | A |
| 100 | A |
| 101 | A |
| 102 | A |
| 103 | A |
| 104 | A |
| 105 | A |
| 106 | A |
| 107 | A |
| 108 | A |
| 109 | A |
| 110 | A |
| 111 | A |
| 112 | A |
| 113 | A |
| 115 | A |
| 116 | A |
| 117 | A |
| 118 | A |
| 119 | A |
| 120 | A |
| 121 | A |
| 122 | A |
| 123 | A |
| 124 | A |
| 127 | A |
| 128 | A |
| 129 | A |
| 130 | A |
| 131 | A |
| 132 | A |
| 133 | A |
| 134 | A |
| 135 | A |
| 138 | A |
| 139 | A |
| 140 | A |
| 141 | A |
| 142 | A |
| 143 | A |
| 144 | A |
| 145 | A |
| 146 | A |
| 147 | A |
| 148 | A |
| 149 | A |
| 150 | A |
| 151 | A |
| 152 | A |
| 153 | A |
| 154 | A |
| 155 | A |
| 156 | A |
| 157 | A |

| | |
|---|---|
| Note: A represents IC₅₀ < 100 nM. | |

The results in the above table demonstrate that these compounds have potent inhibitory activity against the PRMT5 enzyme.

## Claims

1. A compound represented by formula (I), or stereoisomers, geometric isomers, tautomers, pharmaceutically acceptable salts, prodrugs, hydrates, solvates or isotope labeled analogues thereof: 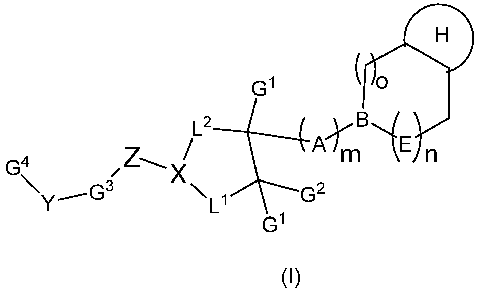
wherein L¹ and L² are each independently one selected from-C(R¹)(R²)-, -C(R¹)(R²)C(R¹)(R²)-, and -C(R¹)(R²)C(R¹)(R²)C(R¹)(R²)-; wherein R¹ and R² are each independently one selected from hydrogen, halogen, hydroxy, mercapto, amino, cyano, a C₁₋₆ alkyl, a C₃₋₆ cycloalkyl, a 4- to 6-membered heterocyclyl, -OR³, -NHR³, and -NR³R⁴; R³ and R⁴ are each independently one selected from a C₁₋₆ alkyl, a C₁₋₆ alkenyl, a C₁₋₆ alkynyl, a C₃₋₆ cycloalkyl, a 4- to 6-membered heterocyclyl, an aryl, and a 5- to 6-membered heteroaryl;
X is selected from C(R⁵) or N; wherein each R⁵ is independently one selected from hydrogen, halogen, hydroxyl, mercapto, amino, and cyano;
Y is one selected from— (chemical bond), -H, -OH, -NH₂, halogen, -O-, -S-, -CO-, -C(R⁶)F-, -CF₂-, -SO-, -SO₂-, -(CH₂)ₚN(R⁶)-, -N(R⁶)(CH₂)ₚ-, -S(O)N(R⁶)-, -S(O)₂N(R⁶)-, -N(R⁶)SO-, -N(R⁶)S(O)₂-, -C(O)N(R⁶)-, -N(R⁶)C(O)-, and -CH(R⁶)-; wherein p=0, 1, 2 or 3; R⁶ may be one selected from hydrogen, an optionally substituted C₁₋₆ alkyl, an optionally substituted C₃₋₆ cycloalkyl, and an optionally substituted 4- to 6-membered heterocyclyl; the term "optionally substituted" means that the hydrogen on the group to be substituted is unsubstituted or one or more substitutable sites of the group to be substituted are independently substituted with one selected from halogen, hydroxyl, mercapto, amino, cyano, a C₁₋₆ alkyl, a C₁₋₆ alkenyl, a C₁₋₆ alkynyl, a C₃₋₆ cycloalkyl, a 4- to 6-membered heterocyclyl, an aryl, and a 5- to 6-membered heteroaryl; when Y is one selected from -H, -OH, -NH₂ and halogen, G⁴ does not exist;
Z is one selected from— (chemical bond), -O-, -S-, -CO-, -N(R⁷)-, -S(O)N(R⁷)-, -S(O)₂N(R⁷)-, -N(R⁷)SO-, -N(R⁷)S(O)₂-, -C(O)N(R⁷)-, -N(R⁷)C(O)-, -N(R⁷)C(O)N(R⁷)-, and -CH(R⁷)-; wherein each R⁷ is independently one selected from hydrogen, an optionally substituted C₁₋₆ alkyl, an optionally substituted C₃₋₆ cycloalkyl and an optionally substituted 4- to 6-membered heterocyclyl; the term "optionally substituted" means that the hydrogen on the group to be substituted is unsubstituted or one or more substitutable sites of the group to be substituted are independently substituted with one selected from halogen, hydroxyl, mercapto, amino, cyano, a C₁₋₆ alkyl, a C₁₋₆ alkenyl, a C₁₋₆ alkynyl, a C₃₋₆ cycloalkyl, a 4- to 6-membered heterocyclyl, an aryl, and a 5- to 6-membered heteroaryl;
each G¹ is independently one selected from hydrogen, halogen, hydroxy, mercapto, amino, cyano, and a C₁₋₅ linear or branched alkyl;
G² is one selected from hydrogen, halogen, hydroxyl, mercapto, amino, cyano, optionally substituted R⁸, optionally substituted -O(R⁸), optionally substituted -S(R⁸), optionally substituted -NH(R⁸), and optionally substituted -N(R⁸)(R⁸); wherein each R⁸ is independently one selected from a C₁₋₆ alkyl, a C₃₋₆ cycloalkyl and a 4- to 6-membered heterocyclyl; the term "optionally substituted" means that the hydrogen on the group to be substituted is unsubstituted or one or more substitutable sites of the group to be substituted are independently substituted with one selected from halogen, hydroxyl, mercapto, amino, cyano, a C₁₋₆ alkyl, a C₁₋₆ alkenyl, a C₁₋₆ alkynyl, a C₃₋₆ cycloalkyl, a 4- to 6-membered heterocyclyl, an aryl, and a 5- to 6-membered heteroaryl;
G³ is one selected froman optionally substituted C₆₋₁₀ aryl, an optionally substituted 5- to 10-membered heteroaryl, an optionally substituted C₃₋₇ cycloalkyl, and an optionally substituted 4- to 10-membered heterocyclyl; the term "optionally substituted" means that the hydrogen on the group to be substituted is unsubstituted or one or more substitutable sites of the group to be substituted are independently substituted with R¹⁶, wherein each R¹⁶ is independently selected from hydrogen, halogen, hydroxy, mercapto, amino, cyano, -R⁹, -OR⁹, -SR⁹, -SO(R⁹), -SO₂(R⁹), -COOR⁹, -NH(R⁹), -N(R⁹)(R¹⁰), -CONHR⁹, -CON(R⁹)(R¹⁰), -SONH(R⁹), -SON(R⁹)(R¹⁰), -SO₂NH(R⁹), and -SO₂N(R⁹)(R¹⁰); wherein R⁹ and R¹⁰ are each independently selected from a C₁₋₆ alkyl, a C₁₋₆ alkenyl, a C₁₋₆ alkynyl, a C₃₋₆ cycloalkyl, a 4-to 6-membered heterocyclyl, an aryl, and a 5- to 6-membered heteroaryl, which may be substituted with one or more of hydrogen, halogen, hydroxy, mercapto, amino, cyano, a C₁₋₆ alkyl, a C₃₋₆ cycloalkyl, a 4- to 6-membered heterocyclyl, an aryl, and a 5- to 6-membered heteroaryl;
G⁴ is one selected from an optionally substituted C₁₋₁₂ alkyl, an optionally substituted C₁₋₁₂ alkenyl, an optionally substituted C₁₋₁₂ alkynyl, an optionally substituted C₃₋₁₂ cycloalkyl, an optionally substituted 4- to 10-membered heterocyclyl, an optionally substituted C₆₋₁₀ aryl, and an optionally substituted 5- to 10-membered heteroaryl; the term "optionally substituted" means that the hydrogen on the group to be substituted is unsubstituted or one or more substitutable sites of the group to be substituted are independently substituted with R¹⁷, where each R¹⁷ is independently selected from hydrogen, halogen, hydroxyl, mercapto, amino,
cyano, carbonyl, -R¹¹, -OR¹¹, -SR¹¹, -NH(R¹¹), -N(R¹¹)(R¹¹), 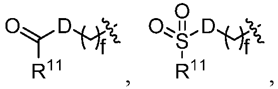 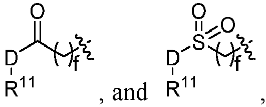 wherein each R¹¹ is independently selected from a C₁₋₆ alkyl, a C₁₋₆ alkenyl, a C₁₋₆ alkynyl, a C₃₋₆ cycloalkyl, a 4- to 6-membered heterocyclyl, an aryl, and a 5-to 6-membered heteroaryl, which may be substituted with one or more of hydrogen, halogen, hydroxy, mercapto, amino, cyano, a C₁₋₆ alkyl, a C₃₋₆ cycloalkyl, a 4- to 6-membered heterocyclyl, an aryl, and a 5- to 6-membered heteroaryl; each D is independently one selected from a bond, -CH₂-, -C(=O)-, -NH-, -N(CH₃)-, -O-, and -S-, and each f is independently selected from 0, 1, 2, 3, 4, 5, 6, 7 or 8;
m=0 or 1, and when m=1, A may be selected from -N(R¹²)-, -CH(R¹²)- or -CH(NHR¹²)-, where R¹² may be one selected from hydrogen, hydroxyl, halogen, C₁₋₆ alkyl, C₃₋₆ cycloalkyl and a 4- to 6-membered heterocyclyl; and when m=0, A does not exist;
B may be selected from -N- or -CH-;
n=0 or 1, and when n=1, E may be selected from -NR¹³- or -C(R¹³)R¹³-, where each R¹³ is independently one selected from hydrogen, hydroxyl, halogen, a C₁₋₆ alkyl, a C₃₋₆ cycloalkyl and a 4- to 6-membered heterocyclyl; and when n=0, E does not exist;
at least one of A, B, and E is an N atom that meets the respective definition;
o=0, 1, 2 or 3;
H ring is one selected from a C₆₋₁₀ aryl ring and a 5- to 10-membered heteroaryl ring; the aryl ring or heteroaryl ring may be independently substituted with one or more R¹⁵, where each R¹⁵ is independently selected from hydrogen, halogen, hydroxy, mercapto, amino, cyano, optionally substituted -R¹⁴, optionally substituted -OR¹⁴, optionally substituted -NHR¹⁴, and optionally substituted -N(R¹⁴)(R¹⁴); wherein each R¹⁴ is independently selected from a C₁₋₆ alkyl, a C₁₋₆ alkenyl, a C₁₋₆ alkynyl, a C₃₋₆ cycloalkyl, a 4- to 6-membered heterocyclyl, an aryl, and a 5- to 6-membered heteroaryl; the term "optionally substituted" means that the hydrogen on the group to be substituted is unsubstituted or one or more substitutable sites of the group to be substituted are independently substituted by one selected from halogen, hydroxyl, mercapto, amino, cyano, a C₁₋₆ alkyl, a C₁₋₆ alkenyl, a C₁₋₆ alkynyl, a C₃₋₆ cycloalkyl, a 4- to 6-membered heterocyclyl, an aryl, and a 5- to 6-membered heteroaryl.

2. The compound according to claim 1, or stereoisomers, geometric isomers, tautomers, pharmaceutically acceptable salts, prodrugs, hydrates, solvates or isotope labeled analogues thereof, wherein the compound represented by formula (I) has a structure represented by formula (II)A, (II)B or (II)C: 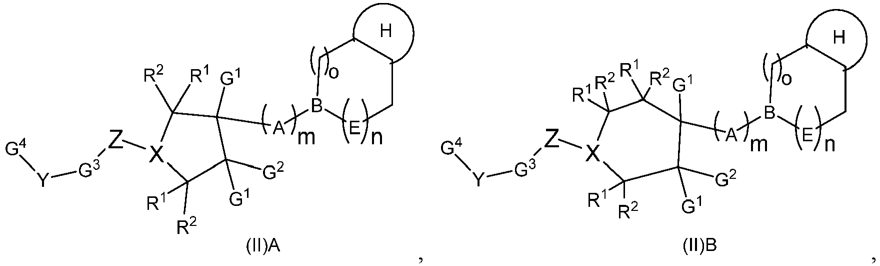 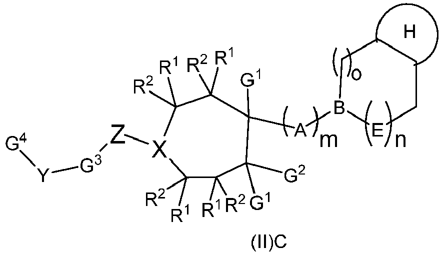 wherein each substituent in formula (II)A, (II)B, or (II)C is as defined in claim 1.

3. The compound according to claim 1 or 2, or stereoisomers, geometric isomers, tautomers, pharmaceutically acceptable salts, prodrugs, hydrates, solvates or isotope labeled analogues thereof, wherein R¹ and R² are each independently one selected from hydrogen, halogen, hydroxy, mercapto, amino, cyano, a C₁₋₆ alkyl, a C₃₋₆ cycloalkyl, a 4- to 6-membered heterocyclyl, -OR³, -NH(R³), and -NR³R⁴; R³ and R⁴ are each independently selected from a C₁₋₆ alkyl, a C₁₋₆ alkenyl, a C₁₋₆ alkynyl, a C₃₋₆ cycloalkyl, a 4- to 6-membered heterocyclyl, an aryl, and a 5- to 6-membered heteroaryl; preferably, R¹ and R² are each independently one of hydrogen, halogen, hydroxy, amino, methyl, methylamino, and dimethylamino; most preferably hydrogen.

4. The compound according to any of claims 1 to 3, or stereoisomers, geometric isomers, tautomers, pharmaceutically acceptable salts, prodrugs, hydrates, solvates or isotope labeled analogues thereof, wherein X is one selected fromCH, C(OH) and N.

5. The compound according to any of claims 1 to 4, or stereoisomers, geometric isomers, tautomers, pharmaceutically acceptable salts, prodrugs, hydrates, solvates or isotope labeled analogues thereof, wherein Y is one selected from— (chemical bond), -H, -OH, -NH₂, halogen, -O-, -S-, -CO-, -C(R⁶)F-, -CF₂-, -SO-, -SO₂-, -(CH₂)ₚN(R⁶)-, -N(R⁶)(CH₂)ₚ-, -S(O)N(R⁶)-, -S(O)₂N(R⁶)-, -N(R⁶)SO-, -N(R⁶)S(O)₂-, -C(O)N(R⁶)-, -N(R⁶)C(O)-, and -CH(R⁶)-, where p=0, 1, 2 or 3; R⁶ may be one selected from hydrogen, a C₁₋₆ alkyl, a C₃₋₆ cycloalkyl, and a 4- to 6-membered heterocyclyl, and when Y is one selected from -H, -OH, -NH₂ and halogen, G⁴ does not exist.

6. The compound according to any of claims 1 to 5, or stereoisomers, geometric isomers, tautomers, pharmaceutically acceptable salts, prodrugs, hydrates, solvates or isotope labeled analogues thereof, wherein Z is one selected from— (chemical bond), -O-, -S-, -CO-, -N(R⁷)-, -S(O)N(R⁷)-, -S(O)₂N(R⁷)-, -N(R⁷)SO-, -N(R⁷)S(O)₂-, -C(O)N(R⁷)-, -N(R⁷)C(O)-, -N(R⁷)C(O)N(R⁷)-, and -CH(R⁷)-, where each R⁷ is independently one selected from hydrogen, a C₁₋₆ alkyl, a C₃₋₆ cycloalkyl and a 4- to 6-membered heterocyclyl.

7. The compound according to any of claims 1 to 6, or stereoisomers, geometric isomers, tautomers, pharmaceutically acceptable salts, prodrugs, hydrates, solvates or isotope labeled analogues thereof, wherein each G¹ is independently one selected from hydrogen, halogen, hydroxyl, mercapto, amino, cyano and methyl.

8. The compound according to any of claims 1 to 7, or stereoisomers, geometric isomers, tautomers, pharmaceutically acceptable salts, prodrugs, hydrates, solvates or isotope labeled analogues thereof, wherein G² is one selected from hydrogen, halogen, hydroxyl, mercapto, amino, cyano, -R⁸, -O(R⁸), -S(R⁸), -NH(R⁸), and -N(R⁸)(R⁸), where each R⁸ is independently one selected froma C₁₋₆ alkyl, a C₃₋₆ cycloalkyl and a 4- to 6-membered heterocyclyl; more preferably, G² is one selected from hydrogen, halogen, hydroxyl, mercapto, amino, cyano, -CH₃, cyclopropyl, -OCH₃, -SCH₃, -NHCH₃, -N(CH₃)(CH₃), and -NH(CH₃); most preferably, G² is selected from hydrogen, fluorine, hydroxyl, and amino.

9. The compound according to any of claims 1 to 8, or stereoisomers, geometric isomers, tautomers, pharmaceutically acceptable salts, prodrugs, hydrates, solvates or isotope labeled analogues thereof, wherein G³ is selected from an optionally substituted C₆₋₁₀ aryl, and an optionally substituted 5- to 10-membered heteroaryl, wherein the 5- to 10-membered heteroaryl has 1, 2, 3 or 4 heteroatoms which are N, O, or S, wherein the term "optionally substituted" means that the hydrogen on the group to be substituted is unsubstituted or one or more substitutable sites of the group to be substituted are independently substituted with R¹⁶.

10. The compound according to any of claims 1 to 9, or stereoisomers, geometric isomers, tautomers, pharmaceutically acceptable salts, prodrugs, hydrates, solvates or isotope labeled analogues thereof, wherein G³ is one of an optionally substituted 6- to 10-membered aryl and an optionally substituted 5- to 10-membered heteroaryl, and the 6- to 10-membered aryl or 5-to 10-membered heteroaryl is one selected from: 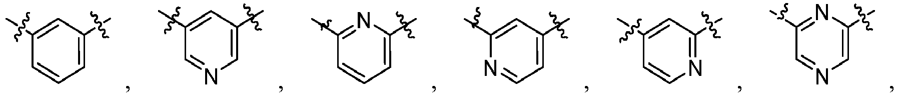 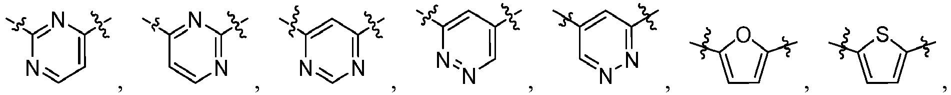 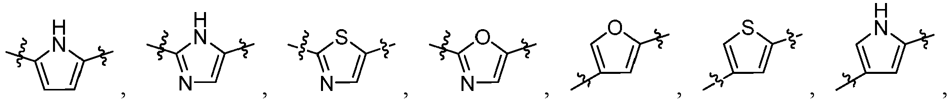 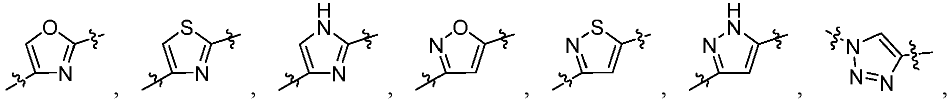 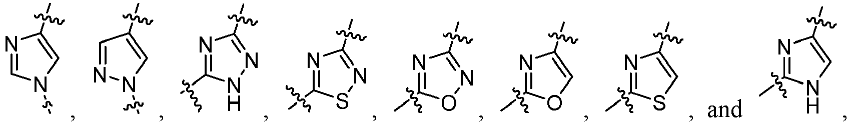 wherein the term "optionally substituted" means that the hydrogen on the group to be substituted is unsubstituted or one or more substitutable sites of the group to be substituted are independently substituted with R¹⁶.

11. The compound according to any of claims 1 to 10, or stereoisomers, geometric isomers, tautomers, pharmaceutically acceptable salts, prodrugs, hydrates, solvates or isotope labeled analogues thereof, wherein G⁴ is one selected from an optionally substituted C₃₋₁₂ cycloalkyl, an optionally substituted 4- to 10-membered heterocyclyl, an optionally substituted C₆₋₁₀ aryl, and an optionally substituted 5- to 10-membered heteroaryl, wherein the term "optionally substituted" means that the hydrogen on the group to be substituted is unsubstituted or one or more substitutable sites of the group to be substituted are independently substituted with R¹⁷, where each R¹⁷ is independently selected from hydrogen, halogen, hydroxyl, mercapto, amino, cyano, carbonyl, -R¹¹, -OR¹¹ , -SR¹¹, -NH(R¹¹), -N(R¹¹)(R¹¹), 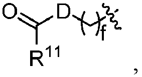 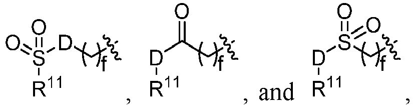 wherein each R¹¹ is independently selected from a C₁₋₆ alkyl, a C₁₋₆ alkenyl, a C₁₋₆ alkynyl, a C₃₋₆ cycloalkyl, a 4- to 6-membered heterocyclyl, an aryl, and a 5- to 6-membered heteroaryl, which may be substituted with one or more of hydrogen, halogen, hydroxy, mercapto, amino, cyano, a C₁₋₆ alkyl, a C₃₋₆ cycloalkyl, a 4- to 6-membered heterocyclyl, an aryl, and a 5- to 6-membered heteroaryl; each D is independently one selected from a bond, -CH₂-, -C(=O)-, -NH-, -N(CH₃)-, -O-, and -S-, and each f is independently selected from 0, 1, or 2.

12. The compound according to any of claims 1 to 11, or stereoisomers, geometric isomers, tautomers, pharmaceutically acceptable salts, prodrugs, hydrates, solvates or isotope labeled analogues thereof, wherein G⁴ is one selected from: 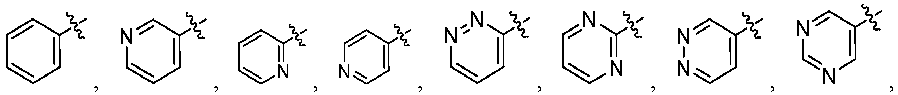 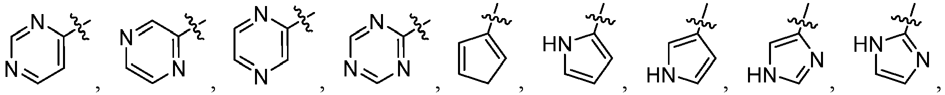 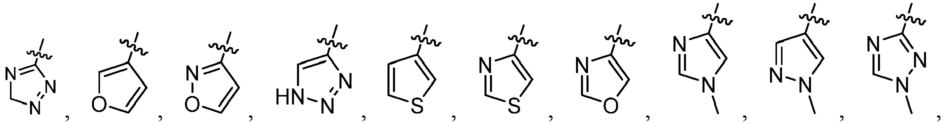 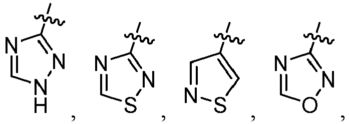 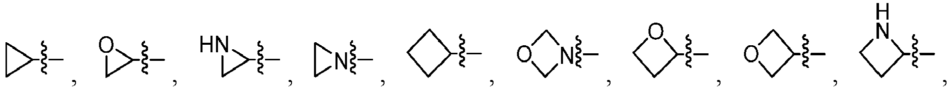 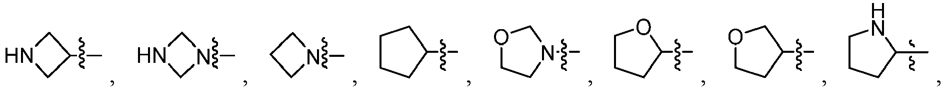 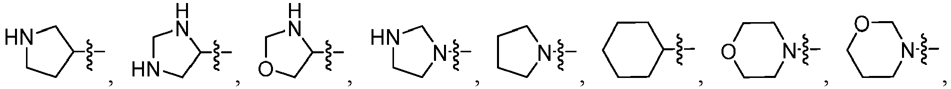 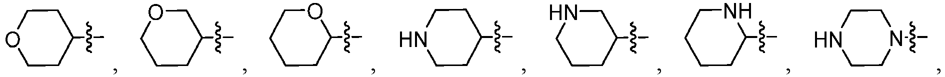 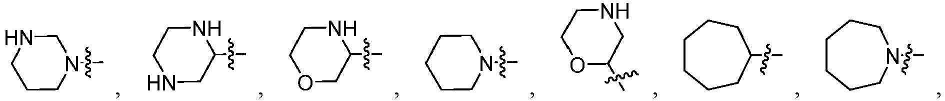 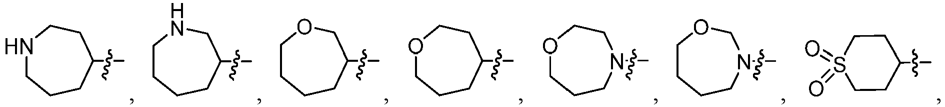 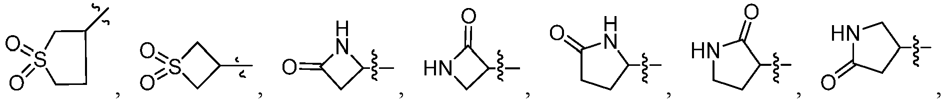 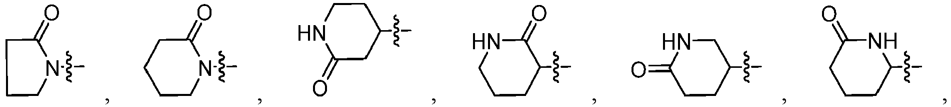 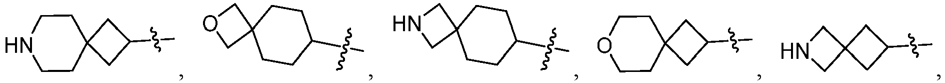 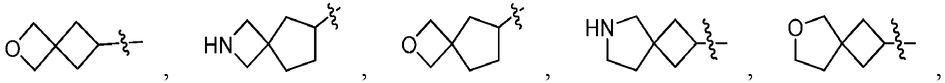 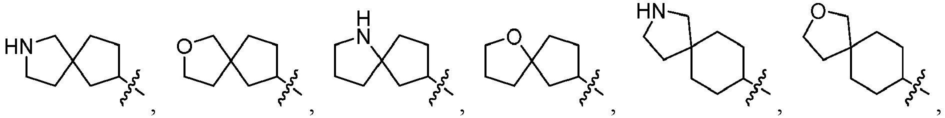 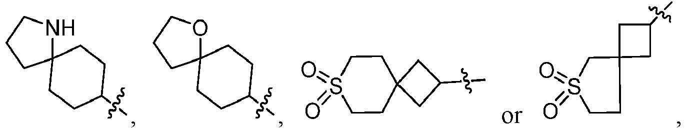 wherein the above groups are substituted with one or more R¹⁷ at any substitutable sites of the groups.

13. The compound according to any of claims 1 to 12, or stereoisomers, geometric isomers, tautomers, pharmaceutically acceptable salts, prodrugs, hydrates, solvates or isotope labeled analogues thereof, wherein the H ring is one selected from a benzene ring, a 5- to 6-membered heteroaryl ring, a 5-membered fused to 6-membered heteroaryl ring, and a 6-membered fused to 5-membered heteroaryl ring which may be independently substituted with one or more R¹⁵, wherein R¹⁵ is selected from hydrogen, halogen, hydroxyl, mercapto, amino, cyano, a C₁₋₆ alkyl, a C₁₋₆ alkenyl, a C₁₋₆ alkynyl, a C₃₋₆ cycloalkyl, a 4- to 6-membered heterocyclyl, an aryl, and a 5- to 6-membered heteroaryl.

14. The compound according to claim 13, or stereoisomers, geometric isomers, tautomers, pharmaceutically acceptable salts, prodrugs, hydrates, solvates or isotope labeled analogues thereof, wherein the H ring is a benzene ring which may be independently substituted with one or more R¹⁵.

15. The compound according to claim 13, or stereoisomers, geometric isomers, tautomers, pharmaceutically acceptable salts, prodrugs, hydrates, solvates or isotope labeled analogues thereof, wherein the H ring is a 5- to 6-membered heteroaryl ring selected from: 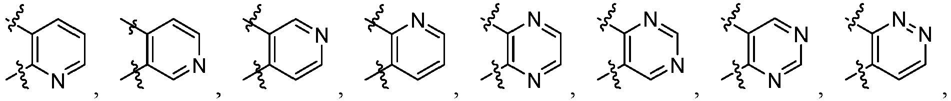 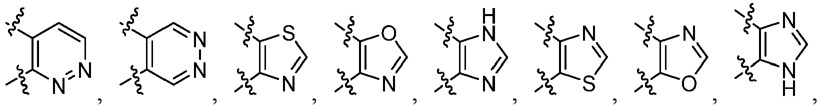 and the 5-to 6-membered heteroaryl ring may be independently substituted with one or more R¹⁵.

16. The compound according to claim 13, or stereoisomers, geometric isomers, tautomers, pharmaceutically acceptable salts, prodrugs, hydrates, solvates or isotope labeled analogues thereof, wherein the H ring is a 5-membered fused to 6-membered heteroaryl ring selected from: 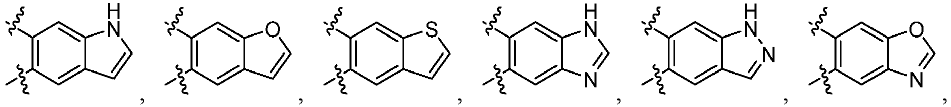 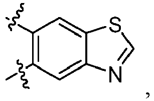 and the 5-membered fused to 6-membered heteroaryl ring may be independently substituted with one or more R¹⁵.

17. The compound according to claim 13, or stereoisomers, geometric isomers, tautomers, pharmaceutically acceptable salts, prodrugs, hydrates, solvates or isotope labeled analogues thereof, wherein the H ring is a 6-membered fused to 5-membered heteroaryl ring selected from: 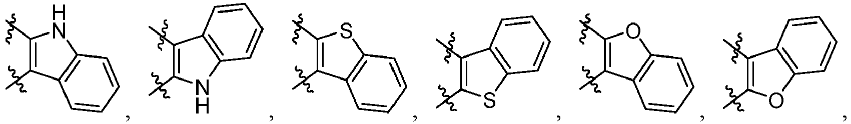 and the 6-membered fused to 5-membered heteroaryl ring may be independently substituted with one or more R ¹⁵.

18. The compound according to claim 1, or stereoisomers, geometric isomers, tautomers, pharmaceutically acceptable salts, prodrugs, hydrates, solvates or isotope labeled analogues thereof, wherein the compound has the following structure:
| | | | |
|---|---|---|---|
| 1 | 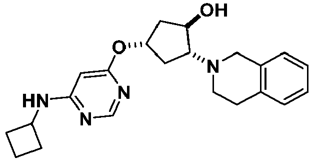 | 2 | 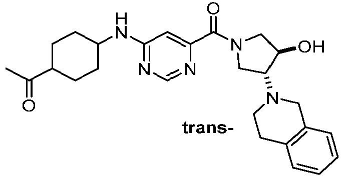 |
| 3 | 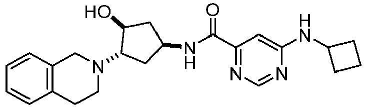 | 4 | 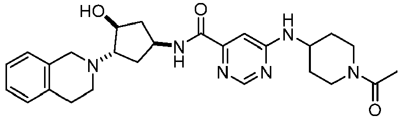 |
| 5 | 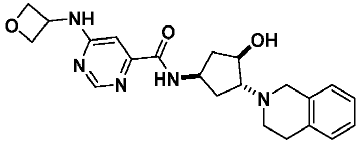 | 6 | 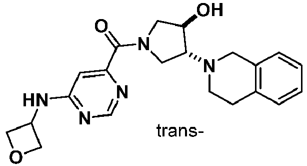 |
| 7 | 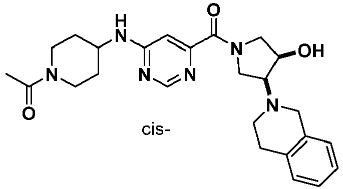 | 8 | 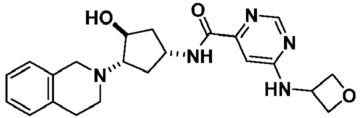 |
| 9 | 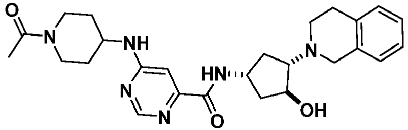 | 10 | 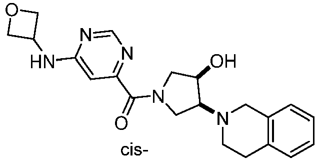 |
| 11 | 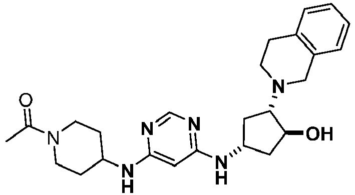 | 12 | 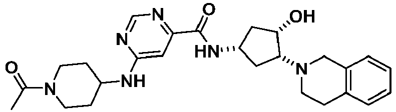 |
| 13 | 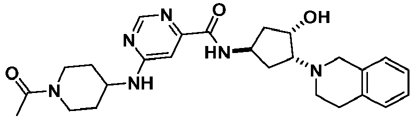 | 14 | 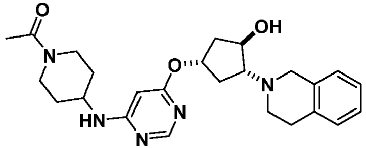 |
| 15 | 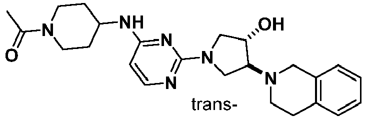 | 16 | 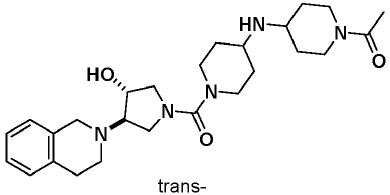 |
| 17 | 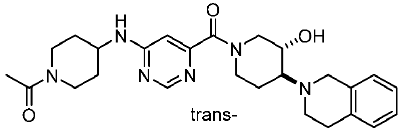 | 18 | 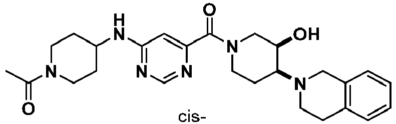 |
| 19 | 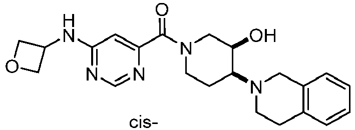 | 20 | 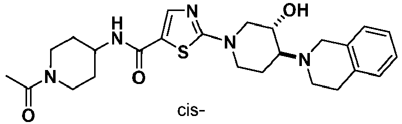 |
| 21 | 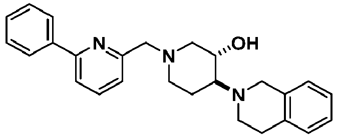 | 22 | 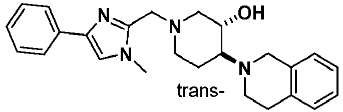 |
| 23 | 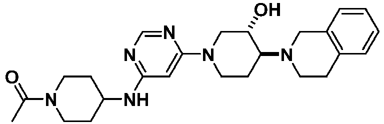 | 24 | 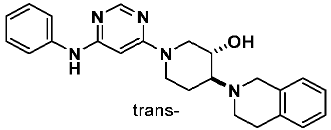 |
| 25 | 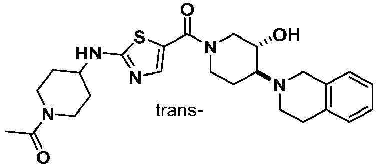 | 26 | 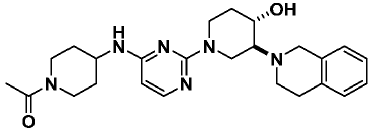 |
| 27 | 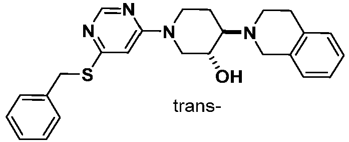 | 28 | 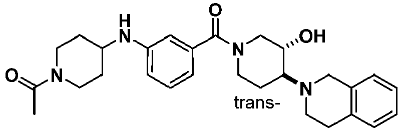 |
| 29 | 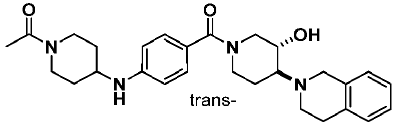 | 30 | 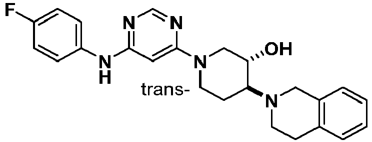 |
| 31 | 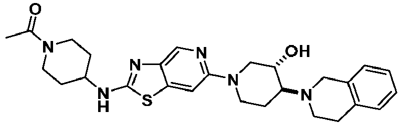 | 32 | 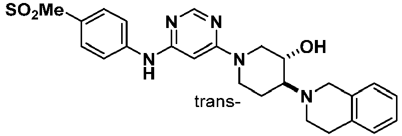 |
| 33 | 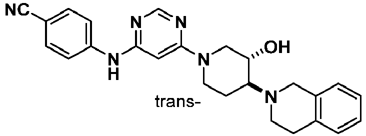 | 34 | 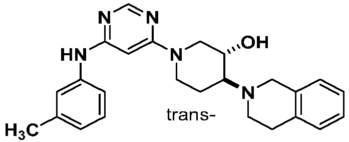 |
| 35 | 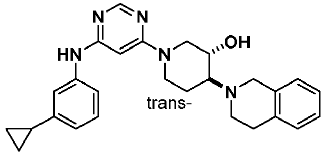 | 36 | 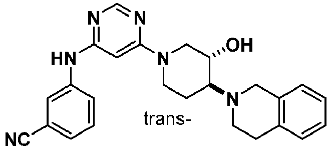 |
| 37 | 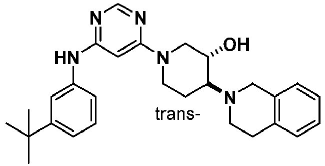 | 38 | 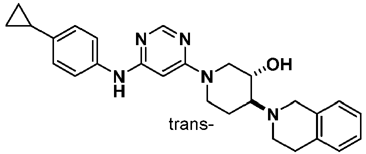 |
| 39 | 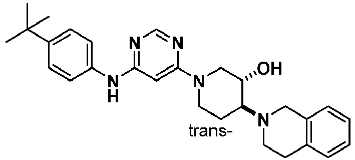 | 40 | 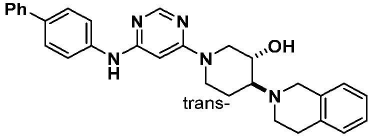 |
| 41 | 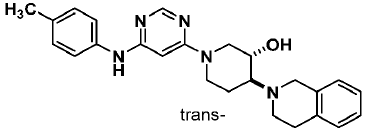 | 42 | 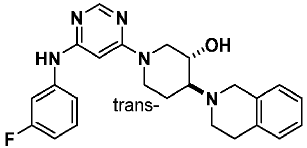 |
| 43 | 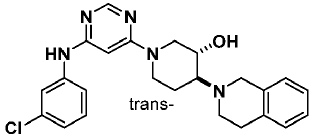 | 44 | 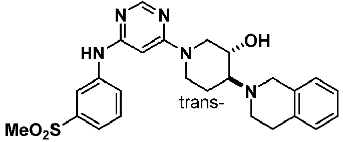 |
| 45 | 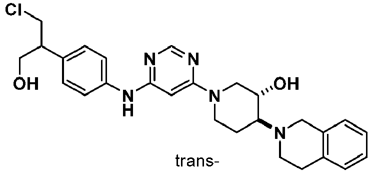 | 46 | 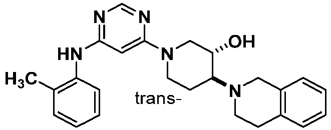 |
| 47 | 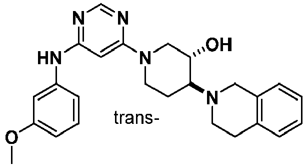 | 48 | 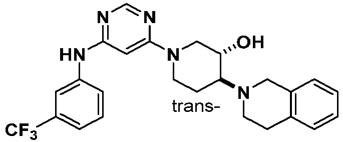 |
| 49 | 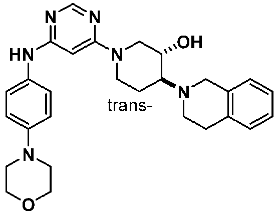 | 50 | 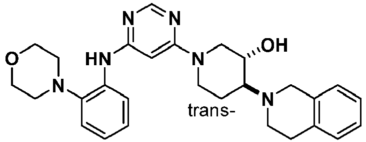 |
| 51 | 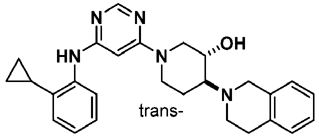 | 52 | 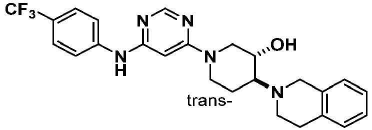 |
| 53 | 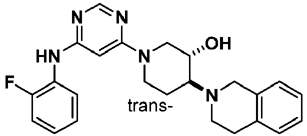 | 54 | 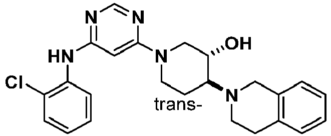 |
| 55 | 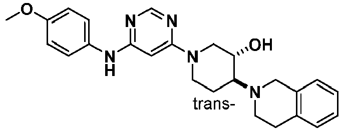 | 56 | 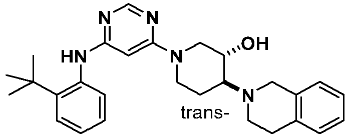 |
| 57 | 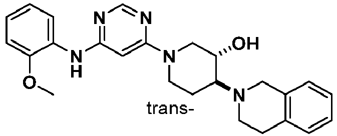 | 58 | 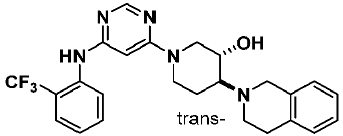 |
| 59 | 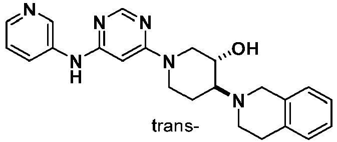 | 60 | 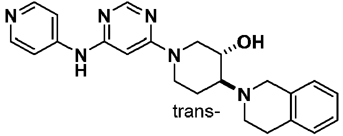 |
| 61 | 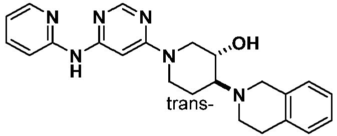 | 62 | 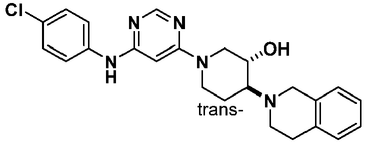 |
| 63 | 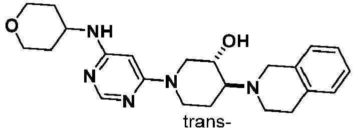 | 64 | 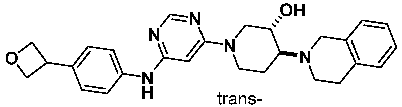 |
| 65 | 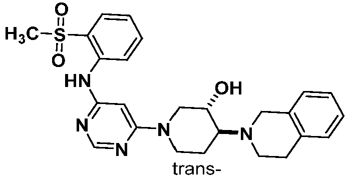 | 66 | 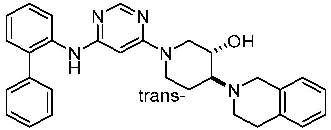 |
| 67 | 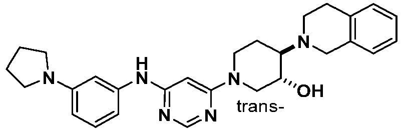 | 68 | 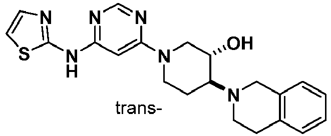 |
| 69 | 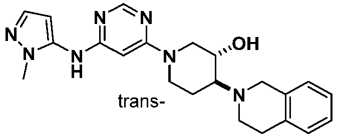 | 70 | 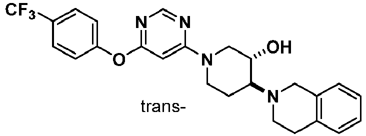 |
| 71 | 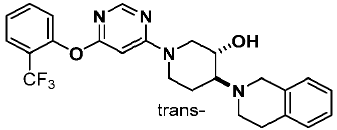 | 72 | 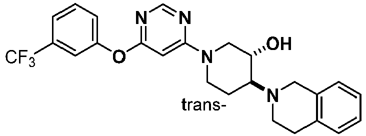 |
| 73 | 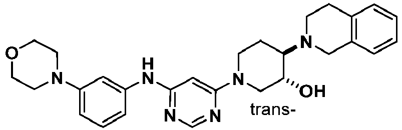 | 74 | 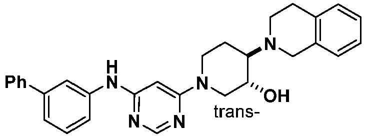 |
| 75 | 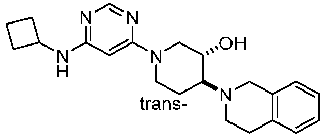 | 76 | 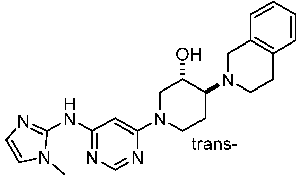 |
| 77 | 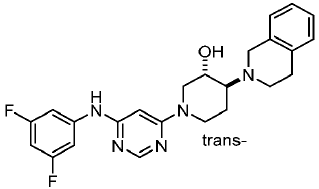 | 78 | 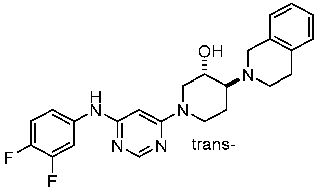 |
| 79 | 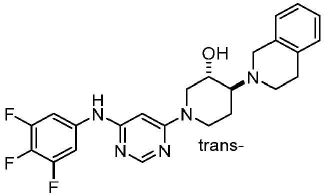 | 80 | 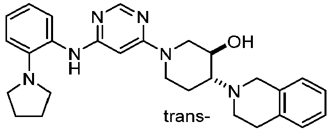 |
| 81 | 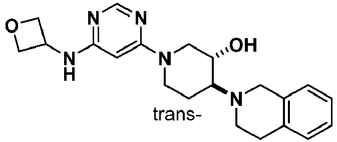 | 82 | 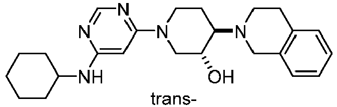 |
| 83 | 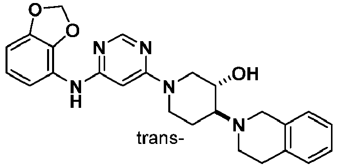 | 84 | 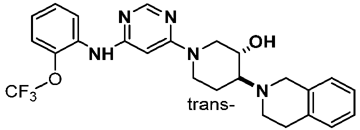 |
| 85 | 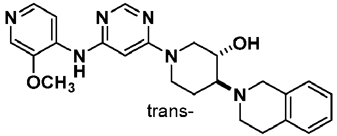 | 86 | 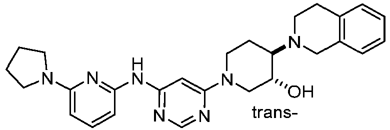 |
| 87 | 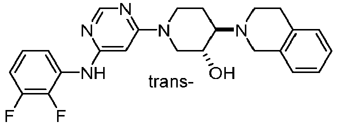 | 88 | 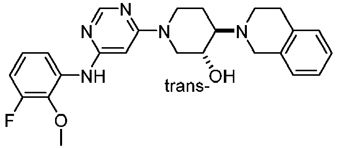 |
| 89 | 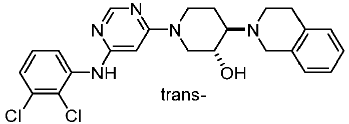 | 90 | 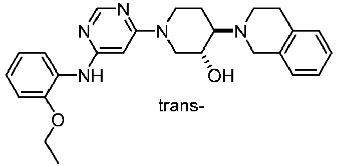 |
| 91 | 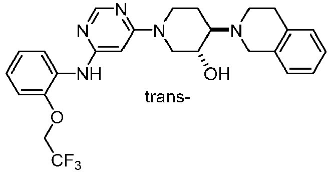 | 92 | 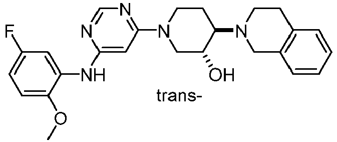 |
| 93 | 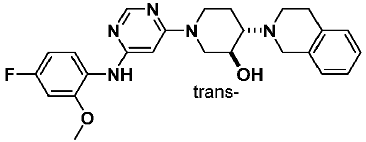 | 94 | 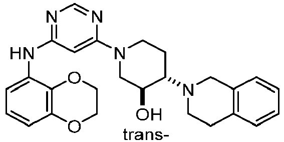 |
| 95 | 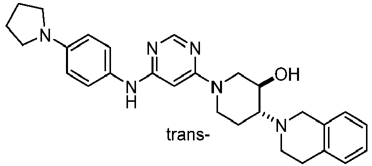 | 96 | 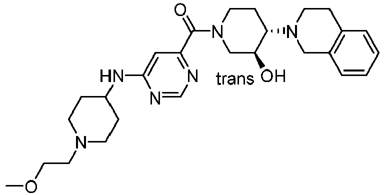 |
| 97 | 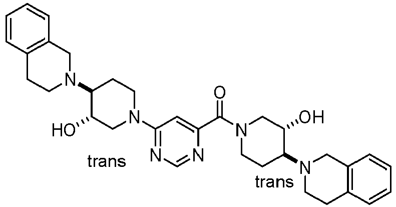 | 98 | 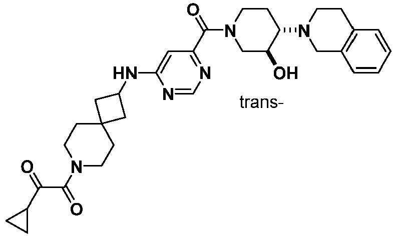 |
| 99 | 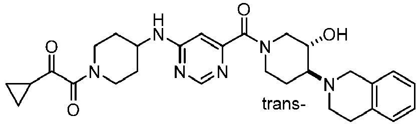 | 100 | 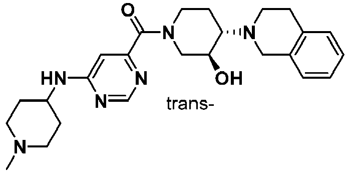 |
| 101 | 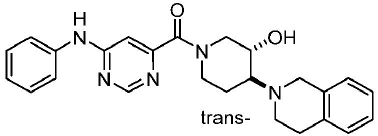 | 102 | 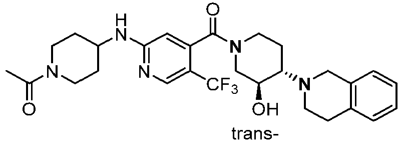 |
| 103 | 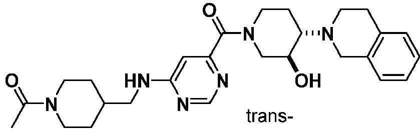 | 104 | 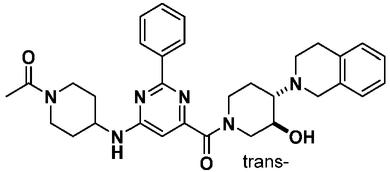 |
| 105 | 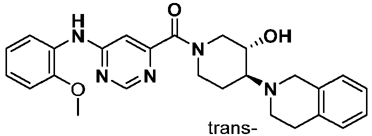 | 106 | 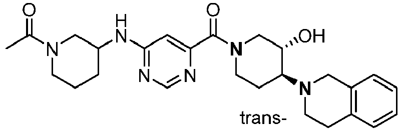 |
| 107 | 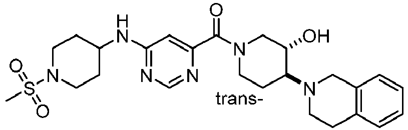 | 108 | 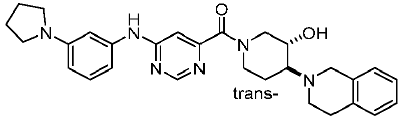 |
| 109 | 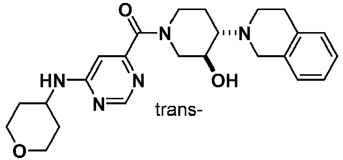 | 110 | 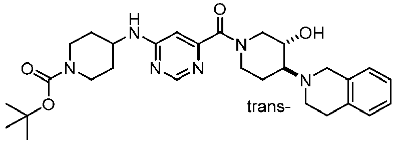 |
| 111 | 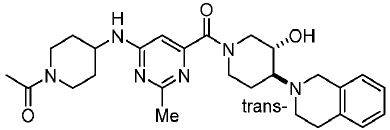 | 112 | 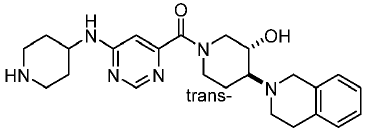 |
| 113 | 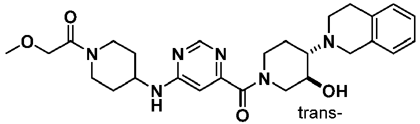 | 114 | 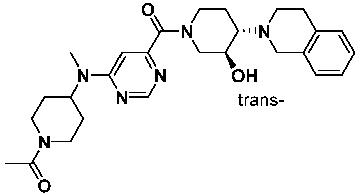 |
| 115 | 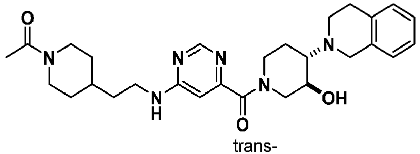 | 116 | 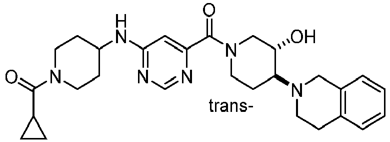 |
| 117 | 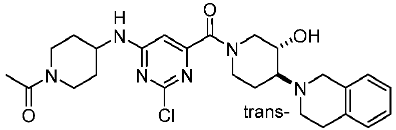 | 118 | 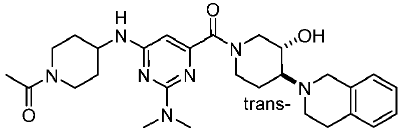 |
| 119 | 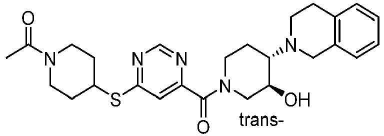 | 120 | 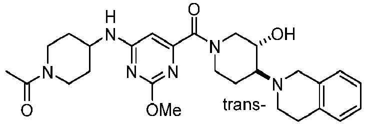 |
| 121 | 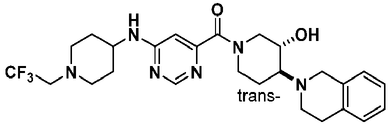 | 122 | 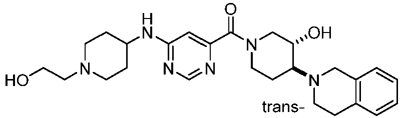 |
| 123 | 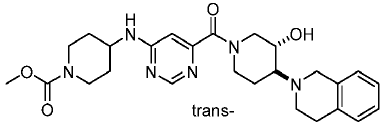 | 124 | 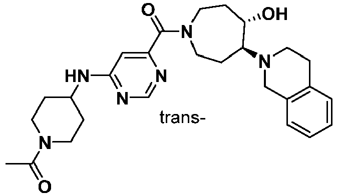 |
| 125 | 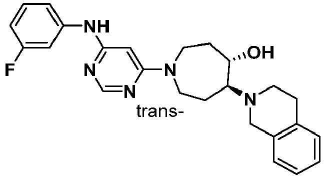 | 126 | 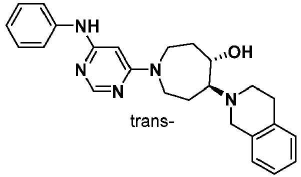 |
| 127 | 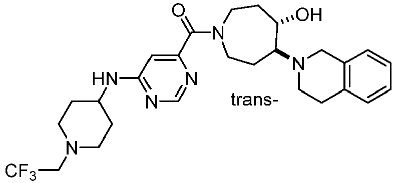 | 128 | 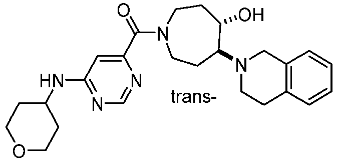 |
| 129 | 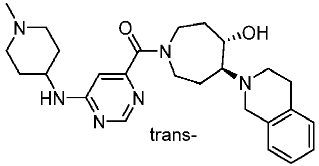 | 130 | 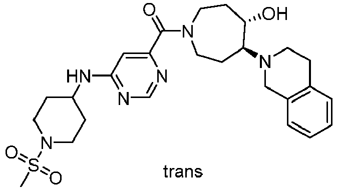 |
| 131 | 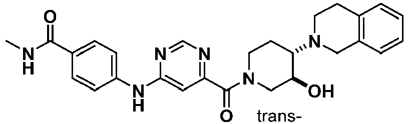 | 132 | 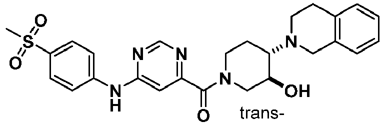 |
| 133 | 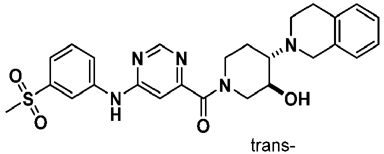 | 134 | 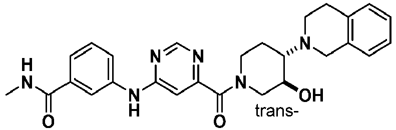 |
| 135 | 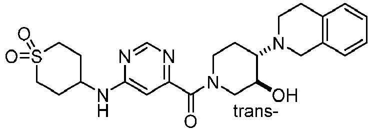 | 136 | 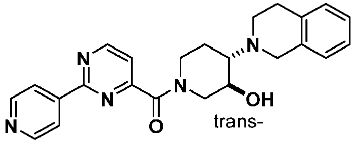 |
| 137 | 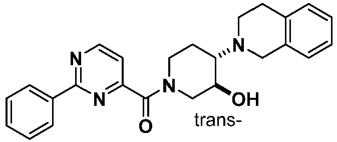 | 138 | 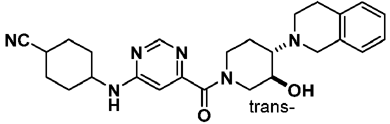 |
| 139 | 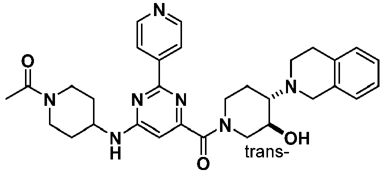 | 140 | 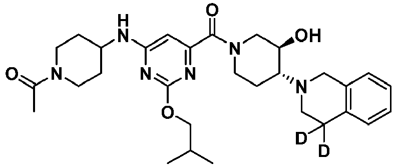 |
| 141 | 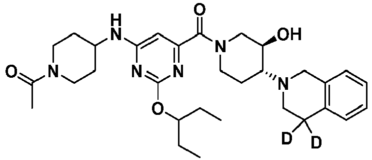 | 142 | 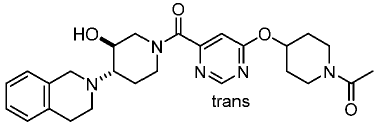 |
| 143 | 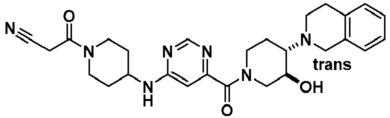 | 144 | 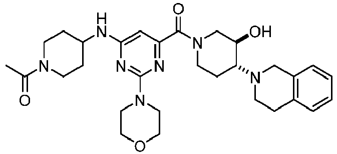 |
| 145 | 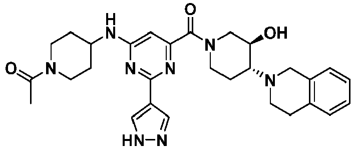 | 146 | 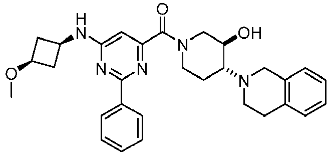 |
| 147 | 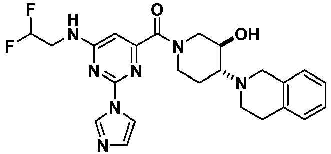 | 148 | 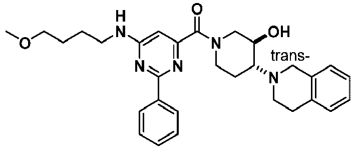 |
| 149 | 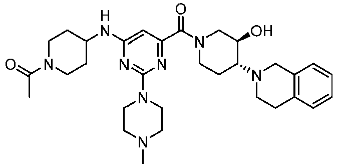 | 150 | 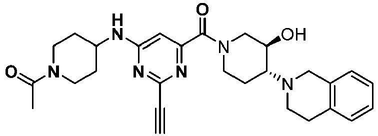 |
| 151 | 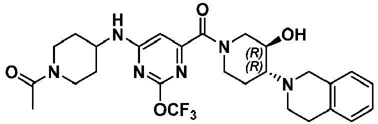 | 152 | 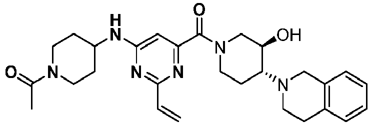 |
| 153 | 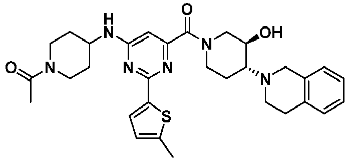 | 154 | 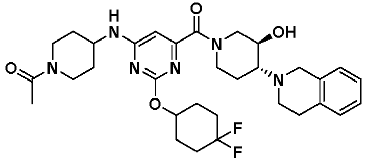 |
| 155 | 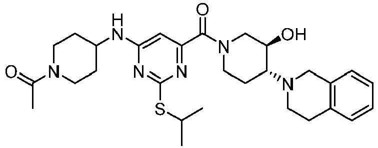 | 156 | 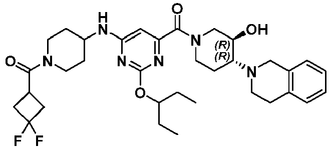 |
| 157 | 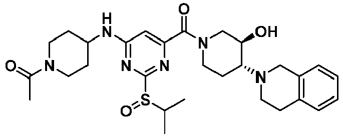 | | |

19. The compound according to claim 2, or stereoisomers, geometric isomers, tautomers, pharmaceutically acceptable salts, prodrugs, hydrates, solvates or isotope labeled analogues thereof,
wherein X is N, B is N, m=0, o=1, n=1, E is -C(R¹³)(R¹³)-, where each R¹³ is independently selected from hydrogen, halogen, a C₁₋₆ alkyl and a C₃₋₆ cycloalkyl; the H ring is a benzene ring which may be substituted with one or more R¹⁵, where R¹⁵ is selected from hydrogen, halogen, a C₁₋₆ alkyl and a C₃₋₆ cycloalkyl; each G¹ is independently selected from hydrogen, halogen, and a C₁₋₅ linear or branched alkyl; and G² is selected from hydroxy, mercapto and amino.

20. The compound according to claim 19, or stereoisomers, geometric isomers, tautomers, pharmaceutically acceptable salts, prodrugs, hydrates, solvates or isotope labeled analogues thereof, wherein each R¹³ is independently selected from hydrogen and halogen.

21. The compound according to claim 19, or stereoisomers, geometric isomers, tautomers, pharmaceutically acceptable salts, prodrugs, hydrates, solvates or isotope labeled analogues thereof, wherein R¹⁵ is selected from hydrogen and halogen.

22. The compound according to claim 19, or stereoisomers, geometric isomers, tautomers, pharmaceutically acceptable salts, prodrugs, hydrates, solvates or isotope labeled analogues thereof, wherein each G¹ is independently selected from hydrogen and halogen.

23. The compound according to claim 19, or stereoisomers, geometric isomers, tautomers, pharmaceutically acceptable salts, prodrugs, hydrates, solvates or isotope labeled analogues thereof, wherein G² is hydroxy.

24. The compound according to claim 19, or stereoisomers, geometric isomers, tautomers, pharmaceutically acceptable salts, prodrugs, hydrates, solvates or isotope labeled analogues thereof, wherein R¹ and R² are each independently selected from hydrogen, halogen, hydroxy, mercapto, amino, cyano, a C₁₋₆ alkyl, a C₃₋₆ cycloalkyl, a 4- to 6-membered heterocyclyl, -OR³, -NH(R³), and -NR³R⁴, wherein R³ and R⁴ are independently selected from a C₁₋₆ alkyl, a C₁₋₆ alkenyl, a C₁₋₆ alkynyl, a C₃₋₆ cycloalkyl, a 4- to 6-membered heterocyclyl, an aryl, and a 5- to 6-membered heteroaryl.

25. The compound according to claim 19, or stereoisomers, geometric isomers, tautomers, pharmaceutically acceptable salts, prodrugs, hydrates, solvates or isotope labeled analogues thereof, wherein R¹ and R² are each independently selected from hydrogen, halogen and methyl.

26. The compound according to claim 19, or stereoisomers, geometric isomers, tautomers, pharmaceutically acceptable salts, prodrugs, hydrates, solvates or isotope labeled analogues thereof, wherein Z is selected from - (chemical bond) and -CO-.

27. The compound according to claim 19, or stereoisomers, geometric isomers, tautomers, pharmaceutically acceptable salts, prodrugs, hydrates, solvates or isotope labeled analogues thereof, wherein G³ is selected from an optionally substituted C₆₋₁₀ aryl, and an optionally substituted 5- to 10-membered heteroaryl, wherein the 5- to 10-membered heteroaryl has 1, 2, 3 or 4 heteroatoms which are N, O, or S, wherein the term "optionally substituted" means that the C₆₋₁₀ aryl and 5- to 10-membered heteroaryl are unsubstituted or substituted with one or more R¹⁶.

28. The compound according to claim 19, or stereoisomers, geometric isomers, tautomers, pharmaceutically acceptable salts, prodrugs, hydrates, solvates or isotope labeled analogues thereof, wherein G³ is selected from optionally substituted 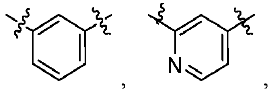 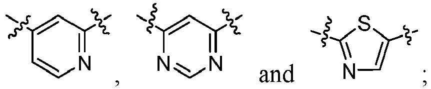 wherein the term "optionally substituted" means unsubstituted or substituted with one or more R¹⁶.

29. The compound according to claim 19, or stereoisomers, geometric isomers, tautomers, pharmaceutically acceptable salts, prodrugs, hydrates, solvates or isotope labeled analogues thereof, wherein G³ is optionally substituted 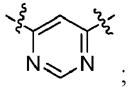 wherein the term "optionally substituted" means unsubstituted or substituted with one or more R¹⁶.

30. The compound according to any of claims 27 to 29, or stereoisomers, geometric isomers, tautomers, pharmaceutically acceptable salts, prodrugs, hydrates, solvates or isotope labeled analogues thereof, wherein each R¹⁶ is independently selected from hydrogen, halogen, -R⁹, -OR⁹, -SR⁹, -SO(R⁹), -NH(R⁹), and -N(R⁹)(R¹⁰), wherein R⁹ and R¹⁰ are each independently selected from a C₁₋₆ alkyl, a C₁₋₆ alkenyl, a C₁₋₆ alkynyl, a C₃₋₆ cycloalkyl, an aryl, and a 5- to 6-membered heteroaryl, which may be substituted with one or more of hydrogen, halogen, a C₁₋₆ alkyl, and a C₃₋₆ cycloalkyl.

31. The compound according to any of claims 27 to 29, or stereoisomers, geometric isomers, tautomers, pharmaceutically acceptable salts, prodrugs, hydrates, solvates or isotope labeled analogues thereof, wherein each R¹⁶ is independently selected from hydrogen, halogen, methyl and methoxy.

32. The compound according to any of claims 27 to 29, or stereoisomers, geometric isomers, tautomers, pharmaceutically acceptable salts, prodrugs, hydrates, solvates or isotope labeled analogues thereof, wherein each R¹⁶ is independently selected from hydrogen, halogen, methyl, ethyl, n-propyl, isopropyl, n-butyl, isobutyl, n-pentyl, isopentyl, ethoxy, n-propoxy, isopropoxy, n-butoxy, isobutoxy, n-pentyloxy, isopentyloxy, trifluoromethyl, methoxy, amino, methylamino, dimethylamino, phenyl, pyridyl, vinyl, ethynyl, -OCF₃, -SCH(CH₃)₂, -OCH(CH₂CH₃)₂, -S(O)CH(CH₃)₂,

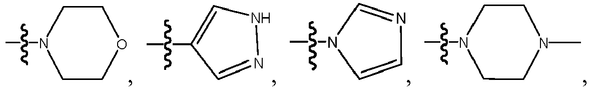

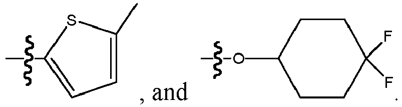

33. The compound according to claim 19, or stereoisomers, geometric isomers, tautomers, pharmaceutically acceptable salts, prodrugs, hydrates, solvates or isotope labeled analogues thereof, wherein Y is selected from - (chemical bond), -NH-, -O-, -S-, -SO-, -SO₂-, -N(CH₃)-, -S(O)NH-, -S(O)₂NH-, -NHSO- and -NHS(O)₂-.

34. The compound according to claim 19, or stereoisomers, geometric isomers, tautomers, pharmaceutically acceptable salts, prodrugs, hydrates, solvates or isotope labeled analogues thereof, wherein Y selected from - (chemical bond), -S-, -O- and -NH-.

35. The compound according to claim 19, or stereoisomers, geometric isomers, tautomers, pharmaceutically acceptable salts, prodrugs, hydrates, solvates or isotope labeled analogues thereof, wherein G⁴ is selected from 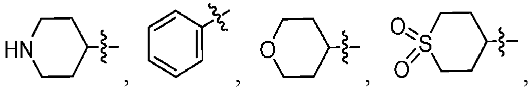 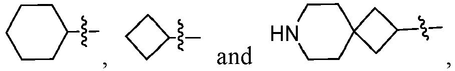 where the above groups are substituted with one or more R¹⁷ at any substitutable sites of the groups.

36. The compound according to claim 19, or stereoisomers, geometric isomers, tautomers, pharmaceutically acceptable salts, prodrugs, hydrates, solvates or isotope labeled analogues thereof, wherein G⁴ is 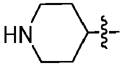 substituted with one or more R¹⁷, and at least one R¹⁷ is on the N atom.

37. The compound according to claim 35 or 36, or stereoisomers, geometric isomers, tautomers, pharmaceutically acceptable salts, prodrugs, hydrates, solvates or isotope labeled analogues thereof, wherein each R¹⁷ is independently selected from hydrogen, halogen, hydroxy, mercapto, amino, cyano, carbonyl, -R¹¹, -OR¹¹, -SR¹¹,

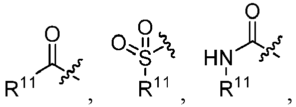

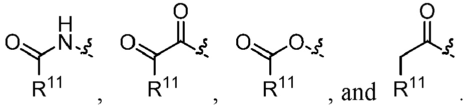

38. The compound according to claim 37, or stereoisomers, geometric isomers, tautomers, pharmaceutically acceptable salts, prodrugs, hydrates, solvates or isotope labeled analogues thereof, wherein R¹⁷ is

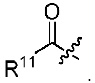

39. The compound according to claim 35, or stereoisomers, geometric isomers, tautomers, pharmaceutically acceptable salts, prodrugs, hydrates, solvates or isotope labeled analogues thereof, wherein G⁴ is 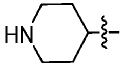 substituted with 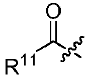 at the N atom.

40. The compound according to any of claims 37 to 39, or stereoisomers, geometric isomers, tautomers, pharmaceutically acceptable salts, prodrugs, hydrates, solvates or isotope labeled analogues thereof, wherein each R¹¹ is independently selected from a C₁₋₆ alkyl, a C₃₋₆ cycloalkyl, a 4- to 6-membered heterocyclyl, an aryl, and a 5- to 6-membered heteroaryl, which may be substituted with one or more of hydrogen, halogen, hydroxy, mercapto, amino, cyano, and a C₁₋₆ alkyl.

41. The compound according to claim 40, or stereoisomers, geometric isomers, tautomers, pharmaceutically acceptable salts, prodrugs, hydrates, solvates or isotope labeled analogues thereof, wherein each R¹¹ is independently selected from a C₁₋₆ alkyl or an aryl, which may be substituted with one or more of hydrogen, halogen, hydroxy, and C₁₋₆ alkyl.

42. The compound according to claim 38 or 39, or stereoisomers, geometric isomers, tautomers, pharmaceutically acceptable salts, prodrugs, hydrates, solvates or isotope labeled analogues thereof, wherein R¹¹ is selected from trifluoromethyl, difluoromethyl, trifluoroethyl, difluoroethyl, methyl, ethyl, n-propyl, isopropyl, cyclopropyl, n-butyl, isobutyl, tert-butyl, sec-butyl, methoxy, ethoxy, n-propoxy, isopropoxy, cyclopropoxy, n-butoxy, isobutoxy, tert-butoxy, sec-butoxy, difluorocyclobutyl, a 4- to 6-membered heterocyclyl and phenyl.

43. The compound according to claim 19, or stereoisomers, geometric isomers, tautomers, pharmaceutically acceptable salts, prodrugs, hydrates, solvates or isotope labeled analogues thereof, wherein G⁴ is a C₁₋₆ alkyl optionally substituted with R¹⁷, preferably a C₁₋₄ alkyl optionally substituted with R¹⁷, more preferably ethyl, n-propyl or n-butyl optionally substituted with R¹⁷, where R¹⁷ is selected from halogen and methoxy.

44. A pharmaceutical composition comprising the compound according to any of claims 1 to 43, or stereoisomers, geometric isomers, tautomers, pharmaceutically acceptable salts, prodrugs, hydrates, solvates or isotope labeled analogues thereof, and a pharmaceutically acceptable excipient.

45. Use of the compound according to any of claims 1 to 43, or stereoisomers, geometric isomers, tautomers, pharmaceutically acceptable salts, prodrugs, hydrates, solvates or isotope labeled analogues thereof, in the preparation of a medicament for treating a disease mediated by PRMT5 inhibitor.

46. The use according to claim 45, wherein the disease mediated by PRMT5 inhibitor is a disease associated with cancer or tumor, including skin cancer, bladder cancer, ovarian cancer, breast cancer, stomach cancer, pancreatic cancer, prostate cancer, colon cancer, lung cancer, bone cancer, brain cancer, neuroblastoma, rectal cancer, colon cancer, familial adenomatous polyposis cancer, hereditary nonpolyposis colorectal cancer, esophageal cancer, lip cancer, laryngeal cancer, hypopharyngeal cancer, tongue cancer, salivary gland cancer, gastric cancer, adenocarcinoma, medullary thyroid cancer, papillary thyroid cancer, kidney cancer, carcinoma of renal parenchyma, ovarian cancer, cervical cancer, corpus carcinoma, endometrial cancer, choriocarcinoma, pancreatic cancer, prostate cancer, testicular cancer, urinary cancer, melanoma, brain tumors such as glioblastoma, astrocytoma, meningioma, medulloblastoma and peripheral primitive neuroectodermal tumor, Hodgkin's lymphoma, non-Hodgkin's lymphoma, Burkitt's lymphoma, acute lymphoblastic leukemia (ALL), chronic lymphocytic leukemia (CLL), acute myelogenous leukemia (AML), chronic myelogenous leukemia (CML), adult T-cell leukemia lymphoma, diffuse large B-cell lymphoma (DLBCL), hepatocellular carcinoma, gallbladder carcinoma, bronchial carcinoma, small cell lung cancer, non-small cell lung cancer, multiple myeloma, basal cell tumor, teratoma, retinoblastoma, choroidal melanoma, seminoma, rhabdomyosarcoma, craniopharyngioma, osteosarcoma, chondrosarcoma, myoma, liposarcoma, fibrosarcoma, Ewing sarcoma, or plasmacytoma.
